# EUROPEAN PATENT SPECIFICATION

(11) **EP 4 565 584 B1**
(45) Date of publication and mention of the grant of the patent: **11.11.2026**
(21) Application number: 23753837.6
(22) Date of filing: 01.08.2023
(51) Int. Cl.: C07D 471/04, A61K 31/437, A61P 35/00, A61P 35/02, A61P 35/04

(54) **1H-PYRROLO[3,2-B]PYRIDINE DERIVATIVES AS IRREVERSIBLE INHIBITORS OF MUTANT EGFR FOR THE TREATMENT OF CANCER**
1H-PYRROLO[3,2-B]PYRIDIN-DERIVATE ALS IRREVERSIBLE INHIBITOREN VON MUTANT EGFR ZUR BEHANDLUNG VON KREBS
DÉRIVÉS DE 1H-PYRROLO[3,2-B]PYRIDINE EN TANT QU'INHIBITEURS IRREVERSIBLES DE EGFR MUTANT POUR LE TRAITEMENT DU CANCER

(30) Priority: 02.08.2022 US 202263394575 P
(43) Date of publication of application: 11.06.2025
(73) Proprietor: Bayer Aktiengesellschaft, 51373 Leverkusen (DE); The Broad Institute, Inc., Cambridge, MA 02142 (US); Dana-Farber Cancer Institute, Inc., Boston, Massachusetts 02215 (US)
(72) Inventor: SIEGEL, Stephan, 10779 Berlin (DE); HERBERT, Simon Anthony, 10117 Berlin (DE); SCHMECK, Carsten, 45472 Mülheim (DE); SIEGEL, Franziska, 10439 Berlin (DE); MORTIER, Jeremie Xavier G., 12055 Berlin (DE); ZIMMERMANN, Stefanie, 40233 Düsseldorf (DE); KORR, Daniel, 10427 Berlin (DE); BÖMER, Ulf, 16548 Glienicke (DE); SCHRÖDER, Jens, 99425 Weimar (DE); LECHNER, Christian, 10117 Berlin (DE); EVANS, Laura, Somerville, Massachusetts 02143 (US); ORSI, Douglas, Cambridge, Massachusetts 02142 (US); GREULICH, Heidi, Cambridge, Massachusetts 02142 (US); MEYERSON, Matthew, Boston, Massachusetts 02215 (US); GOULD, Alexandra, Cambridge, Massachusetts 02142 (US)
(74) Representative: BIP Patents
(86) International application number: PCT/EP2023/071279
(87) International publication number: WO 2024/028316

(56) References cited:
- WO-A1-2022/023340
- WO-A1-2022/101184

## Description

### Field of application of the invention

The present invention covers 1H-pyrrolo[3,2-b]pyridine derivatives of formula (I) as described and defined herein, methods of preparing said compounds, intermediate compounds useful for preparing said compounds, pharmaceutical compositions and combinations comprising said compounds, and the compounds for use in the treatment or prophylaxis of diseases, in particular cancer, as a sole agent or in combination with other active ingredients.

References in the present description to methods of medical treatment or medical uses have to be understood as references to the compounds of the present invention for use in methods of medical treatment. Methods of medical treatment or medical uses are not encompassed by the claimed invention.

### BACKGROUND OF THE INVENTION

The present invention covers 1H-pyrrolo[3,2-b]pyridine derivatives of formula (I) which inhibit EGFR.

The Epidermal Growth Factor Receptor (EGFR or EGF-receptor) receptor tyrosine kinase family consists of 4 members: EGFR (Erbb1, Her1), ERBB2 (Her2), ERBB3 (Her3), and ERBB4 (Her4). EGFR mediates activation of MAPK and PI3K signaling pathways and thereby regulates cell proliferation, differentiation, migration and survival (Pao et al., 2010). EGFR gene amplification, overexpression, and mutations are frequently observed in various cancer indications and are associated with a poor prognosis (Gridelli et al., 2015).

In lung adenocarcinoma, mutations of EGFR are prevalent in approximately 15% of Western patients and up to 50% of East Asian patients (Paez et al., 2004). These mutations typically occur in one of four exons, exons 18-21, in the kinase domain of EGFR (Paez et al., 2004). The most common activating mutations in EGFR are a point mutation in exon 21, substituting an arginine for a leucine (L858R), and a small in-frame deletion in exon 19 that removes four amino acids (del 19/del746-750) (Pao et al., 2010). The FDA-approved inhibitors gefitinib, erlotinib, and afatinib, targeting mutations in exons 18, 19, and 21 of EGFR, are effective in patients, but the response is often not durable (Mok et al., 2009; Sequist et al., 2013). Resistance frequently occurs in these patients in response to acquisition of a second mutation, T790M (Pao et al., 2005). Second generation inhibitors, e.g. afatinib, irreversibly target this mutation, but are still potent inhibitors of wild-type EGFR, leading to dose-limiting toxicity and lack of efficacy in patients.

Several irreversible EGFR inhibitors are published in CN 110857292, IN 201821027709, CN 110698461, WO 2020001351, WO 2020001350, WO 2019233459, CN 110407852, CN 110357863, WO 2019070167, WO20061470, and WO 2022/101184. WO2019/081486 describes 4H-Pyrrolo[3,2-c]pyridine-4-one derivatives.

A third-generation irreversible inhibitor, osimertinib, that maximizes activity towards T790M while minimizing activity towards wild-type EGFR, is effective in T790M mutant patients and is currently the standard treatment for T790M positive patients (Mok et al., 2017). Osimertinib is also approved as a front-line therapy for patients with mutations of EGFR exons 19 or 21 (Soria et al., 2018).

By contrast, and with the exception of A763_Y764insFQEA, small in-frame insertions of EGFR exon20 are resistant to the classical EGFR inhibitors at doses achievable in lung cancer patients and comprise an unmet medical need (Yasuda et. al., 2013).

Patients with EGFR exon20 insertions, such as V769_D770insASV, D770_N771insSVD, D770_N771insNPG, N771_P772insH, H773_V774insH, H773_V774insNPH, V774_C775insHV show particular low response rates to EGFR-targeted therapies, resulting in significantly reduced progression-free survival as well as overall survival (Chen et al., 2016). This has been shown for the first-generation inhibitors erlotinib and gefitinib as well as for the second-generation inhibitor afatinib (Chen et al., 2016; Yang et al., 2015).

The same resistance profile has been observed for exon20 insertion mutations in ERBB2 (e.g. ERBB2 A775_G776insYVMA with the highest prevalence), another member of the EGF-receptor family (Arcila et al., 2012) and some of the uncommon EGFR mutations like L681Q (Chiu et al., 2015).

The standard of care for EGFR exon20 insertion patients is currently chemotherapy. However, amivantamab and mobocertinib received accelerated approval for 2^{nd} line treatment post chemotherapy recently, and several other inhibitors are currently in clinical trials for the treatment of EGFR exon20 insertion mutation positive lung cancer patients (Friedlaender et al., 2022).

About 40% of patients with advanced EGFR mutant NSCLC develop brain metastases over the course of their disease (Rangachari et al., 2015). The 1^{st} and 2^{nd} generation EGFR inhibitors show only limited brain permeability. The 3^{rd} generation EGFR inhibitor Osimertinib shows clearly improved CNS activity and is currently the preferred treatment option for patients with classical activating EGFR mutations and brain metastasis (Reungwetwattana et al., 2018).

However, Osimertinib has only limited activity on EGFR exon20 insertion mutations.

Furthermore, the recently approved bispecific antibody amivantamab and also mobocertinib show only limited blood-brain-barrier permeability. So there still remains a high unmet medical need especially for lung cancer patients carrying EGFR exon20 insertion mutations and brain metastasis.

WO 2022/023340 A1 discloses e.g. 1H-pyrrolo[3,2-b]pyridine derivatives as EGFR inhibitors for the treatment of cancer.

In summary, mutant EGFR is a promising drug target for cancer therapy. In particular, patients with primary resistance to approved anti-EGFR therapies, due to EGFR exon20 insertions and with brain metastases, have only few treatment options to date and there is a great need for novel alternative and/or improved therapeutics to provide these patients with an efficacious, well-tolerable therapy. Therefore, potent inhibitors of mutant EGFR, particularly of mutant EGFR with exon20 insertion mutations that show improved permeability of the blood-brain-barrier and CNS activity, represent valuable compounds that should complement therapeutic options either as single agents or in combination with other drugs.

### SUMMARY OF THE INVENTION

The invention provides compounds that inhibit a mutant EGFR; specifically, an EGFR comprising one or more exon 20 insertion mutations, an L858R mutation, or a small in-frame deletion of exon 19, in the presence or absence of a T790M mutation and show brain permeability.

It has now been found that the compounds of the present invention have surprising and advantageous properties.

In particular, said compounds of the present invention have surprisingly been found to effectively inhibit mutant EGFR with exon 20 insertion mutations, particularly those harboring a D770_N771ins SVD exon 20 insertion. Furthermore it has been found that these compounds additionally show high cellular potency in EGFR V769_D770insASV, D770_N771insSVD, D770_N771insNPG, N771_P772insH, or H773_V774insNPH exon 20 insertion harboring BA/F3 cell lines.

Surprisingly, the here described compounds retain high cellular activity in BA/F3 cell lines harboring D770_N771insSVD and the T790M mutation.

In addition, the here described compounds potently inhibit proliferation of BA/F3 cell lines carrying EGFR activating mutations with or without T790M acquired resistance mutations (EGFR E746_A750del, L858R, E746_A750del T790M, L858R T790M).

Based on the described properties the here described compounds can therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses mediated by mutant EGFR with exon 20 insertion mutations, a L858R mutation, or a small in-frame deletion of exon 19 (e.g. EGFR E746_A750del) in the presence or absence of a T790M mutation and/or reduce (or block) proliferation in cells harboring EGFR with exon 20 insertion mutations, a L858R mutation, or a small in-frame deletion of exon 19 (e.g. EGFR E746_A750del) in the presence or absence of a T790M mutation, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

### Description of the invention

In accordance with a first aspect, the invention relates to compounds of formula (I): 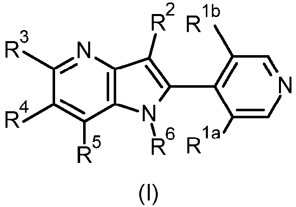 in which:
- R^{1a}: represents a group selected from the group: -O-(C₂-C₆-alkanediyl)-NR⁷R⁸, -O-CH₂-(C₁-C₅-haloalkanediyl)-NR⁷R⁸, -O-(C₁-C₅-alkanediyl)-R⁹, or -O-R⁹;
- R^{1b}: represents a hydrogen atom or fluoro;
- R²: represents phenyl or heteroaryl, wherein said groups are substituted, one or more times, independently of each other, with R¹⁰;
- R³: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁴: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁵: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁶: represents a hydrogen atom or methyl;
- R⁷: represents -(CO)-R¹¹, -(CO)-C≡C-R¹², -(SO₂)-CH=CH₂, or oxirane-2-carbonyl;
- R⁸: represents a hydrogen atom or C₁-C₃-alkyl;
- R⁹: represents a group selected from the group: 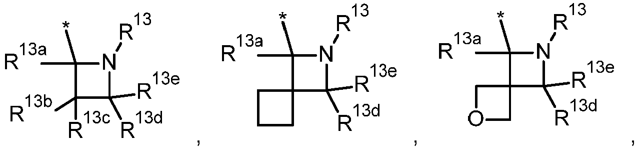 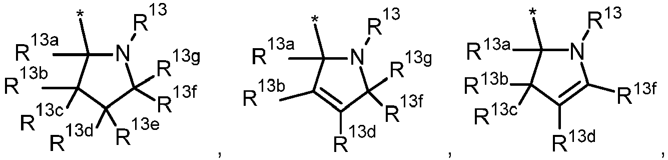 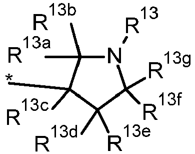 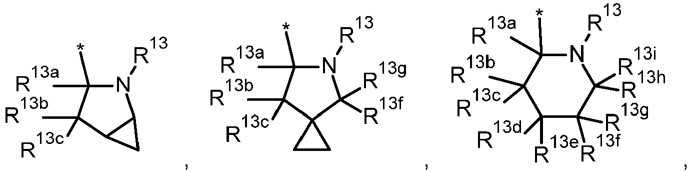 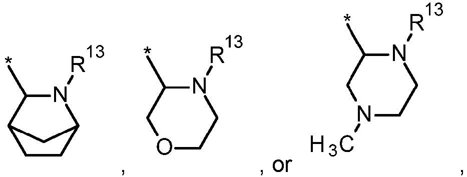 wherein * indicates the point of attachment of said group with the rest of the molecule;
- R¹⁰: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R¹¹: represents -CH₂-C≡CH, or -C(R¹⁴)-R¹⁵;
- R¹²: represents a hydrogen atom, methyl, -CH₂₋N(CH₃)-CHR¹⁶R¹⁷, or 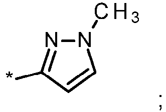
- R¹³: represents -(CO)-R¹¹, -(CO)-C≡C-R¹², -(SO₂)-CH=CH₂, or oxirane-2-carbonyl;
- R^{13a}: represents a hydrogen atom, methyl, or fluoro;
- R^{13b}: represents a hydrogen atom, methyl, or fluoro;
- R^{13c}: represents a hydrogen atom, methyl, or fluoro;
- R^{13d}: represents a hydrogen atom, methyl, fluoro, or -N(CH₃)-CH₂-CH₂-N(CH₃)-CHR¹⁶R¹⁷;
- R^{13e}: represents a hydrogen atom, methyl, or fluoro;
- R^{13f}: represents a hydrogen atom, methyl, or fluoro;
- R^{13g}: represents a hydrogen atom, methyl, or fluoro;
- R^{13h}: represents a hydrogen atom, methyl, or fluoro;
- R¹³ⁱ: represents a hydrogen atom, methyl, or fluoro;
- R¹⁴: represents =CH₂, =CH-CH₃, =CH-CH₂₋N(CH₃)-CHR¹⁶R¹⁷, or =CH-CH₂₋R¹⁸;
- R¹⁵: represents a hydrogen atom, C₁-C₃-alkyl, or fluoro;
- R¹⁶: represents a hydrogen atom or methyl;
- R¹⁷: represents a hydrogen atom or methyl;
- R¹⁸: represents a group selected from the group: 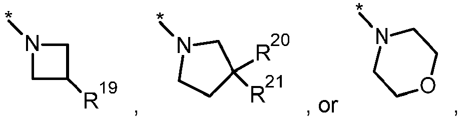 wherein * indicates the point of attachment of said group with the rest of the molecule;
- R¹⁹: represents a hydrogen atom, methyl, methoxy, or fluoro;
- R²⁰: represents a hydrogen atom, methyl, methoxy, or fluoro;
- R²¹: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In accordance with an embodiment of the first aspect, the invention relates to compounds of formula (I): 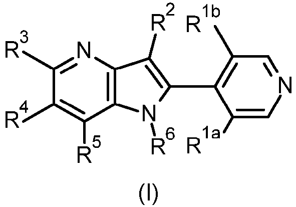 in which:
- R^{1a}: represents a group selected from the group: -Q-(C₂-C₆-alkanediyl)-NR⁷R⁸, -Q-CH₂-(C₁-C₅-haloalkanediyl)-NR⁷R⁸, or -Q-(C₁-C₅-alkanediyl)-R⁹;
- R^{1b}: represents a hydrogen atom;
- R²: represents phenyl or heteroaryl, wherein said groups are substituted, one or more times, independently of each other, with R¹⁰;
- R³: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁴: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁵: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁- C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁶: represents a hydrogen atom or methyl;
- R⁷: represents -(CO)-R¹¹, -(CO)-C≡C-R¹², -(SO₂)-CH=CH₂, or oxirane-2-carbonyl;
- R⁸: represents a hydrogen atom or C₁-C₃-alkyl;
- R⁹: represents a group selected from the group: 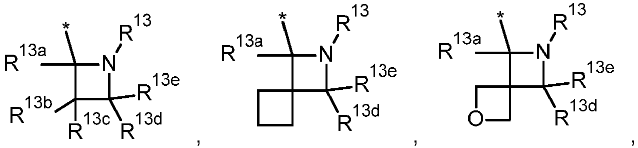 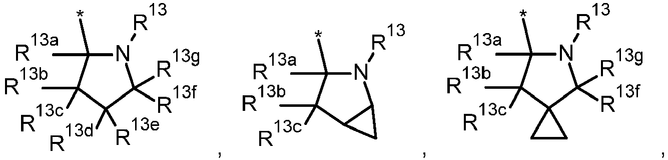 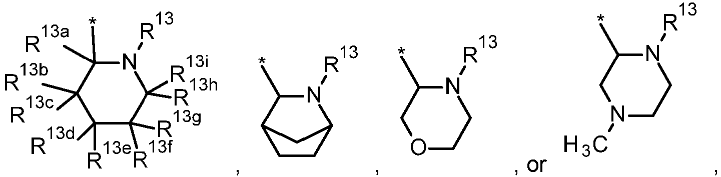 wherein * indicates the point of attachment of said group with the rest of the molecule;
- R¹⁰: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R¹¹: represents -CH₂-C≡CH, or -C(R¹⁴)-R¹⁵;
- R¹²: represents a hydrogen atom or methyl;
- R¹³: represents -(CO)-R¹¹, -(CO)-C≡C-R¹², -(SO₂)-CH=CH₂, or oxirane-2-carbonyl;
- R^{13a}: represents a hydrogen atom, methyl, or fluoro;
- R^{13b}: represents a hydrogen atom, methyl, or fluoro;
- R^{13c}: represents a hydrogen atom, methyl, or fluoro;
- R^{13d}: represents a hydrogen atom, methyl, or fluoro;
- R^{13e}: represents a hydrogen atom, methyl, or fluoro;
- R^{13f}: represents a hydrogen atom, methyl, or fluoro;
- R^{13g}: represents a hydrogen atom, methyl, or fluoro;
- R^{13h}: represents a hydrogen atom, methyl, or fluoro;
- R¹³ⁱ: represents a hydrogen atom, methyl, or fluoro;
- R¹⁴: represents =CH₂, =CH-CH₃, =CH-CH₂₋N(CH₃)-CHR¹⁶R¹⁷, or =CH-CH₂₋R¹⁸;
- R¹⁵: represents a hydrogen atom, C₁-C₃-alkyl, or fluoro;
- R¹⁶: represents a hydrogen atom or methyl;
- R¹⁷: represents a hydrogen atom or methyl;
- R¹⁸: represents a group selected from the group: 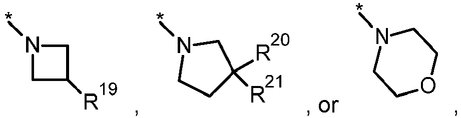 wherein * indicates the point of attachment of said group with the rest of the molecule;
- R¹⁹: represents a hydrogen atom, methyl, methoxy, or fluoro;
- R²⁰: represents a hydrogen atom, methyl, methoxy, or fluoro;
- R²¹: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In a second aspect, the invention relates to compounds of formula (I) as described *supra,* in which:
- R^{1a}: represents a group selected from the group: -O-(C₂-C₆-alkanediyl)-NR⁷R⁸, -O-CH₂-(C₁-C₅-haloalkanediyl)-NR⁷R⁸, -Q-(C₁-C₅-alkanediyl)-R⁹, or -O-R⁹;
- R^{1b}: represents a hydrogen atom or fluoro;
- R²: represents a group selected from the group: or 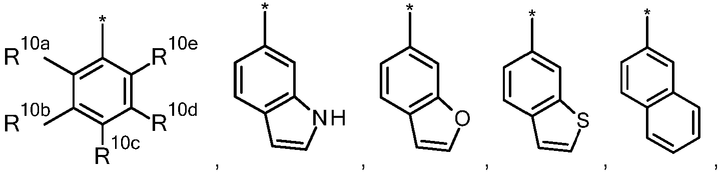 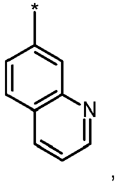 wherein * indicates the point of attachment of said group with the rest of the molecule;
- R³: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁴: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁵: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁶: represents a hydrogen atom or methyl;
- R⁷: represents -(CO)-R¹¹, -(CO)-C≡C-R¹², -(SO₂)-CH=CH₂, or oxirane-2-carbonyl;
- R⁸: represents a hydrogen atom or C₁-C₃-alkyl;
- R⁹: represents a group selected from the group: 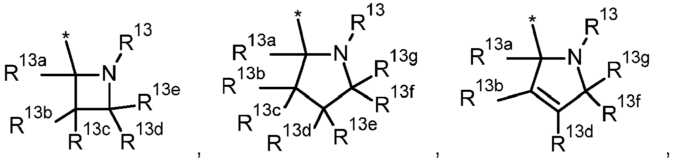 or 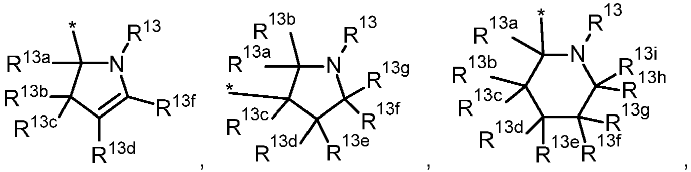 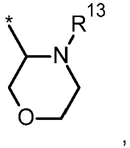 wherein * indicates the point of attachment of said group with the rest of the molecule;
- R^{10a}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10b}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10c}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10d}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10e}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R¹¹: represents -CH₂-C≡CH, or -C(R¹⁴)-R¹⁵; 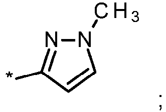
- R¹²: represents a hydrogen atom, methyl, -CH₂₋N(CH₃)-CHR¹⁶R¹⁷, or
- R¹³: represents -(CO)-R¹¹, -(CO)-C≡C-R¹², -(SO₂)-CH=CH₂, or oxirane-2-carbonyl;
- R^{13a}: represents a hydrogen atom, methyl, or fluoro;
- R^{13b}: represents a hydrogen atom, methyl, or fluoro;
- R^{13c}: represents a hydrogen atom, methyl, or fluoro;
- R^{13d}: represents a hydrogen atom, methyl, fluoro, or -N(CH₃)-CH₂-CH₂-N(CH₃)-CHR¹⁶R¹⁷;
- R^{13e}: represents a hydrogen atom, methyl, or fluoro;
- R^{13f}: represents a hydrogen atom, methyl, or fluoro;
- R^{13g}: represents a hydrogen atom, methyl, or fluoro;
- R^{13h}: represents a hydrogen atom, methyl, or fluoro;
- R¹³ⁱ: represents a hydrogen atom, methyl, or fluoro;
- R¹⁴: represents =CH₂, =CH-CH₃, =CH-CH₂₋N(CH₃)-CHR¹⁶R¹⁷, or =CH-CH₂₋R¹⁸;
- R¹⁵: represents a hydrogen atom, C₁-C₃-alkyl, or fluoro;
- R¹⁶: represents a hydrogen atom or methyl;
- R¹⁷: represents a hydrogen atom or methyl;
- R¹⁸: represents a group selected from the group: 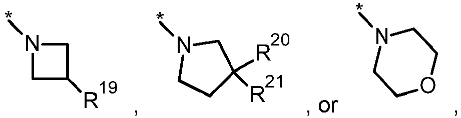 wherein * indicates the point of attachment of said group with the rest of the molecule;
- R¹⁹: represents a hydrogen atom, methyl, methoxy, or fluoro;
- R²⁰: represents a hydrogen atom, methyl, methoxy, or fluoro;
- R²¹: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In accordance with an embodiment of the second aspect, the invention relates to compounds of formula (I), in which:
- R^{1a}: represents a group selected from the group: -O-(C₂-C₆-alkanediyl)-NR⁷R⁸, -O-CH₂-(C₁-C₅-haloalkanediyl)-NR⁷R⁸, or -O-(C₁-C₅-alkanediyl)-R⁹;
- R^{1b}: represents a hydrogen atom;
- R²: represents a group selected from the group: 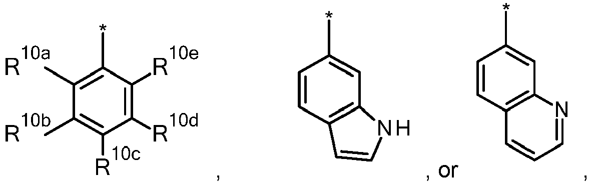 wherein * indicates the point of attachment of said group with the rest of the molecule;
- R³: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁- C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁴: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁵: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R⁶: represents a hydrogen atom or methyl;
- R⁷: represents -(CO)-R¹¹, -(CO)-C≡C-R¹², -(SO₂)-CH=CH₂, or oxirane-2-carbonyl;
- R⁸: represents a hydrogen atom or C₁-C₃-alkyl;
- R⁹: represents a group selected from the group: 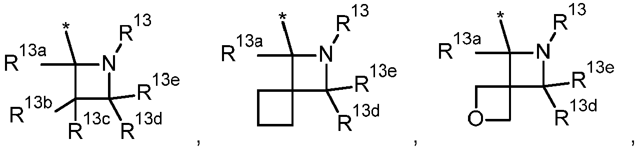 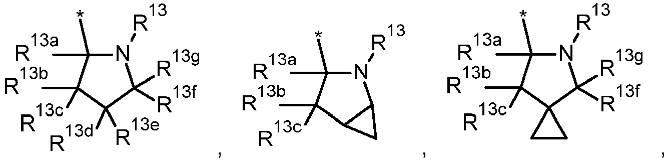 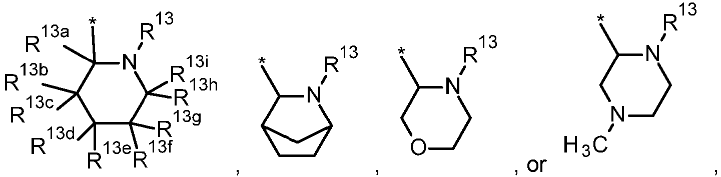 wherein * indicates the point of attachment of said group with the rest of the molecule;
- R^{10a}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10b}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10c}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10d}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R^{10e}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
- R¹¹: represents -CH₂-C≡CH, or -C(R¹⁴)-R¹⁵;
- R¹²: represents a hydrogen atom or methyl;
- R¹³: represents -(CO)-R¹¹, -(CO)-C≡C-R¹², -(SO₂)-CH=CH₂, or oxirane-2-carbonyl;
- R^{13a}: represents a hydrogen atom, methyl, or fluoro;
- R^{13b}: represents a hydrogen atom, methyl, or fluoro;
- R^{13c}: represents a hydrogen atom, methyl, or fluoro;
- R^{13d}: represents a hydrogen atom, methyl, or fluoro;
- R^{13e}: represents a hydrogen atom, methyl, or fluoro;
- R^{13f}: represents a hydrogen atom, methyl, or fluoro;
- R^{13g}: represents a hydrogen atom, methyl, or fluoro;
- R^{13h}: represents a hydrogen atom, methyl, or fluoro;
- R¹³ⁱ: represents a hydrogen atom, methyl, or fluoro;
- R¹⁴: represents =CH₂, =CH-CH₃, =CH-CH₂₋N(CH₃)-CHR¹⁶R¹⁷, or =CH-CH₂₋R¹⁸;
- R¹⁵: represents a hydrogen atom, C₁-C₃-alkyl, or fluoro;
- R¹⁶: represents a hydrogen atom or methyl;
- R¹⁷: represents a hydrogen atom or methyl;
- R¹⁸: represents a group selected from the group: 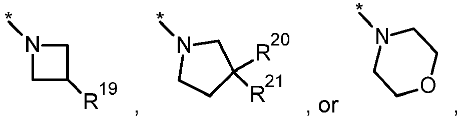 wherein * indicates the point of attachment of said group with the rest of the molecule;
- R¹⁹: represents a hydrogen atom, methyl, methoxy, or fluoro;
- R²⁰: represents a hydrogen atom, methyl, methoxy, or fluoro;
- R²¹: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In a third aspect, the invention relates to compounds of formula (I) as described *supra,* in which:
- R^{1a}: represents a group selected from the group: -O-(C₂-C₄-alkanediyl)-NR⁷R⁸, -O-CH₂-R⁹, or -O-R⁹;
- R^{1b}: represents a hydrogen atom;
- R²: represents a group selected from the group: or 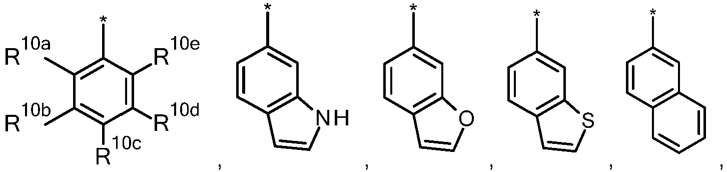 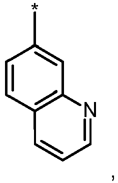 wherein * indicates the point of attachment of said group with the rest of the molecule;
- R³: represents a hydrogen atom, or methoxy;
- R⁴: represents a hydrogen atom, or fluoro;
- R⁵: represents a hydrogen atom or methyl;
- R⁶: represents a hydrogen atom;
- R⁷: represents -(CO)-R¹¹, -(CO)-C≡C-R¹², or -(SO₂)-CH=CH₂;
- R⁸: represents a hydrogen atom, methyl, or ethyl;
- R⁹: represents a group selected from the group: 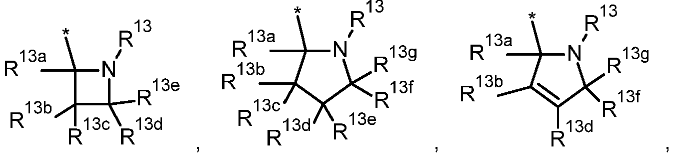 or 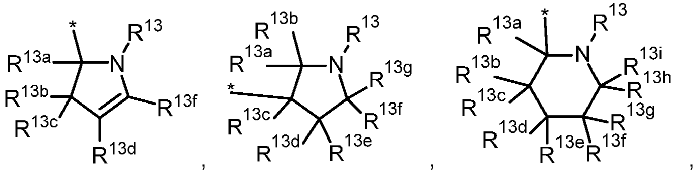 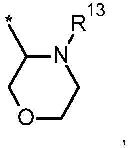 wherein * indicates the point of attachment of said group with the rest of the molecule;
- R^{10a}: represents a hydrogen atom, ethyl, methoxy, or fluoro;
- R^{10b}: represents a hydrogen atom, methyl, ethyl, iso-propyl, ethinyl, trifluoromethyl, methoxy, difluoromethoxy, trifluoromethoxy, methoxycarbonyl, cyano, fluoro, or chloro;
- R^{10c}: represents a hydrogen atom, fluoro, or chloro;
- R^{10d}: represents a hydrogen atom, methyl, ethyl, fluoro, or chloro;
- R^{10e}: represents a hydrogen atom, fluoro, or chloro;
- R¹¹: represents -C(R¹⁴)-R¹⁵;
- R¹²: represents a hydrogen atom, or methyl;
- R¹³: represents -(CO)-R¹¹, -(CO)-C≡C-R¹², or -(SO₂)-CH=CH₂;
- R^{13a}: represents a hydrogen atom, or methyl;
- R^{13b}: represents a hydrogen atom;
- R^{13c}: represents a hydrogen atom;
- R^{13d}: represents a hydrogen atom, methyl, fluoro, or -N(CH₃)-CH₂-CH₂-N(CH₃)-CHR¹⁶R¹⁷;
- R^{13e}: represents a hydrogen atom, or fluoro;
- R^{13f}: represents a hydrogen atom;
- R^{13g}: represents a hydrogen atom;
- R^{13h}: represents a hydrogen atom;
- R¹³ⁱ: represents a hydrogen atom;
- R¹⁴: represents =CH₂, or =CH-CH₂-N(CH₃)-CHR¹⁶R¹⁷;
- R¹⁵: represents a hydrogen atom;
- R¹⁶: represents a hydrogen atom;
- R¹⁷: represents a hydrogen atom;
or an N-oxide, a salt, a tautomer, a rotamer, or a stereoisomer of said compound, or a salt of said N-oxide, tautomer, rotamer, or stereoisomer.

In accordance with an embodiment of the third aspect, the invention relates to compounds of formula (I), in which:
- R^{1a}: represents a group selected from the group: -O-(C₂-C₃-alkanediyl)-NR⁷R⁸, or -O-CH₂-R⁹;
- R^{1b}: represents a hydrogen atom;
- R²: represents a group selected from the group: 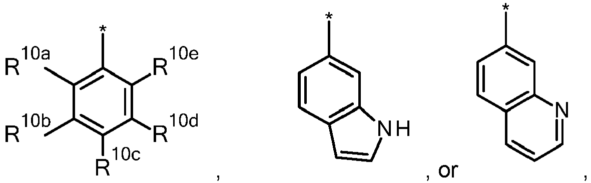 wherein * indicates the point of attachment of said group with the rest of the molecule;
- R³: represents a hydrogen atom, or methoxy;
- R⁴: represents a hydrogen atom, or fluoro;
- R⁵: represents a hydrogen atom;
- R⁶: represents a hydrogen atom;
- R⁷: represents -(CO)-R¹¹, -(CO)-C≡C-R¹², or -(SO₂)-CH=CH₂;
- R⁸: represents a hydrogen atom, methyl, or ethyl;
- R⁹: represents a group: 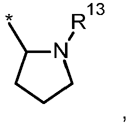 wherein * indicates the point of attachment of said group with the rest of the molecule;
- R^{10a}: represents a hydrogen atom, methoxy, or fluoro;
- R^{10b}: represents a hydrogen atom, methyl, ethyl, trifluoromethyl, methoxy, methoxycarbonyl, fluoro, or chloro;
- R^{10c}: represents a hydrogen atom, fluoro, or chloro;
- R^{10d}: represents a hydrogen atom, or chloro;
- R^{10e}: represents a hydrogen atom;
- R¹¹: represents -C(R¹⁴)-R¹⁵;
- R¹²: represents a hydrogen atom, or methyl;
- R¹³: represents -(CO)-CH=CH₂, -(CO)-CH=CH-CH₂-N(CH₃)₂, or -(SO₂)-CH=CH₂;
- R¹⁴: represents =CH₂, or =CH-CH₂₋N(CH₃)-CHR¹⁶R¹⁷;
- R¹⁵: represents a hydrogen atom;
- R¹⁶: represents a hydrogen atom;
- R¹⁷: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the third aspect, the present invention covers compounds of formula (I), supra, in which:
R1b represents a hydrogen atom.

In a further embodiment of the first aspect, the present invention covers compounds of formula (I), *supra,* in which:
- R^{1a}: represents a group selected from the group: -O-(C₂-C₆-alkanediyl)-NR⁷R⁸, -O-CH₂-(C₁-C₅-haloalkanediyl)-NR⁷R⁸, -O-(C₁-C₅-alkanediyl)-R⁹, or -O-R⁹;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{1a}: represents a group selected from the group: -O-(C₂-C₄-alkanediyl)-NR⁷R⁸, -O-CH₂-R⁹, or -O-R⁹;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{1b}: represents a hydrogen atom or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{1b}: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R²: represents phenyl or heteroaryl (*e.g*., monocyclic heteroaryl, bicyclic heteroaryl), wherein said groups are substituted, one or more times, independently of each other, with R¹⁰;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R²: represents a group selected from the group: or 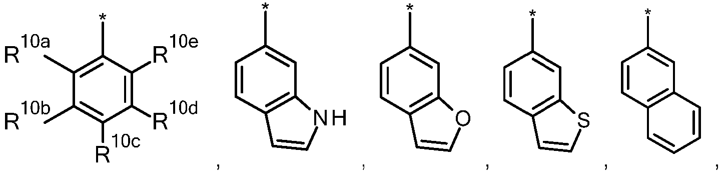 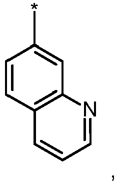 wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R²: represents a group selected from the group: 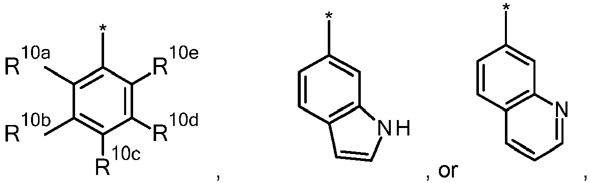 wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R³: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R³: represents a hydrogen atom, or methoxy;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁴: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁴: represents a hydrogen atom, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁵: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁵: represents a hydrogen atom or methyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁶: represents a hydrogen atom or methyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁶: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁷: represents -(CO)-R¹¹, -(CO)-C≡C-R¹², -(SO₂)-CH=CH₂, or oxirane-2-carbonyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁷: represents -(CO)-R¹¹, -(CO)-C≡C-R¹², or -(SO₂)-CH=CH₂;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁸: represents a hydrogen atom or C₁-C₃-alkyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁸: represents a hydrogen atom, methyl, or ethyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁹: represents a group selected from the group: 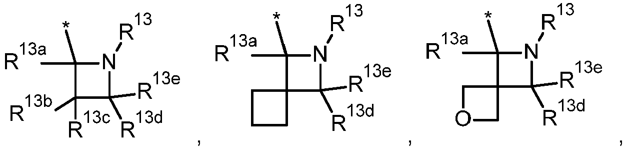 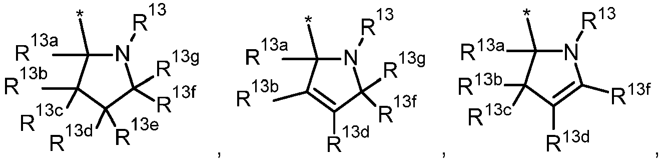 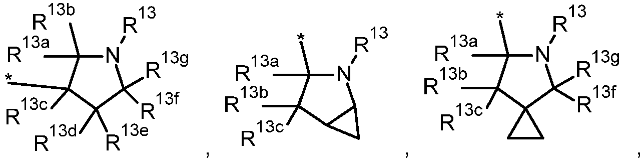 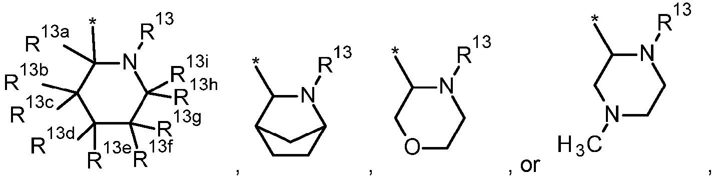 wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁹: represents a group selected from the group: 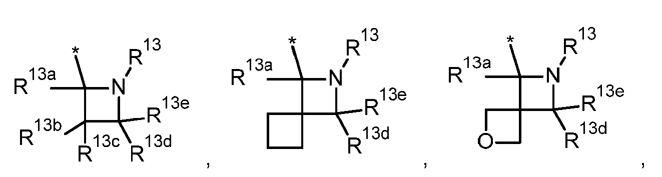 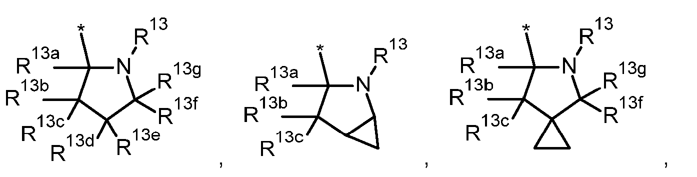 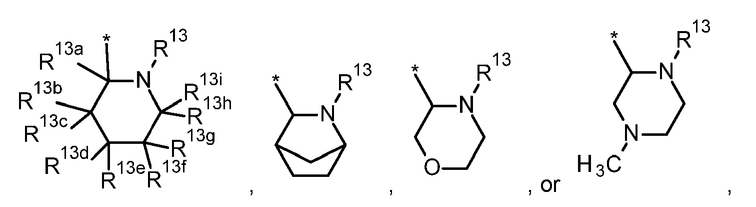
wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁹: represents a group selected from the group: 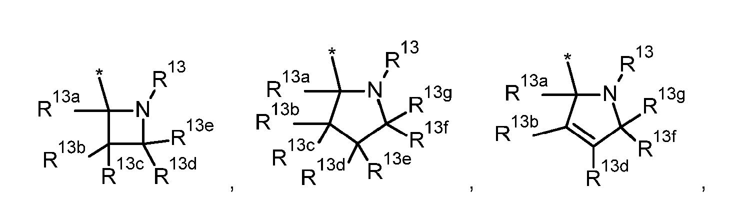 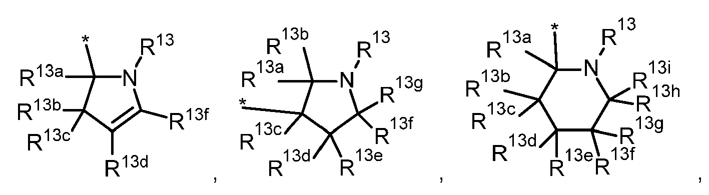 or 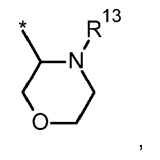
wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R⁹: represents a group: 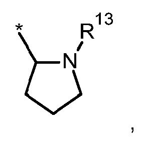 wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁰: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10a}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10a}: represents a hydrogen atom, ethyl, methoxy, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10b}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
In a further embodiment of the first aspect, the present invention covers compounds of formula (I), *supra,* in which:
- R^{10b}: represents a hydrogen atom, methyl, ethyl, iso-propyl, ethinyl, trifluoromethyl, methoxy, difluoromethoxy, trifluoromethoxy, methoxycarbonyl, cyano, fluoro, or chloro;
or an N-oxide, a salt, a tautomer, a rotamer, or a stereoisomer of said compound, or a salt of said N-oxide, tautomer, rotamer, or stereoisomer.
- R^{10b}: represents a hydrogen atom, methyl, ethyl, trifluoromethyl, methoxy, methoxycarbonyl, fluoro, or chloro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10c}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10c}: represents a hydrogen atom, fluoro, or chloro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10d}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10d}: represents a hydrogen atom, methyl, ethyl, fluoro, or chloro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10d}: represents a hydrogen atom, or chloro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10e}: represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10e}: represents a hydrogen atom, fluoro, or chloro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{10e}: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹¹: represents -CH₂-C≡CH, or -C(R¹⁴)-R¹⁵;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹¹: represents -C(R¹⁴)-R¹⁵;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹²: represents a hydrogen atom, methyl, -CH₂₋N(CH₃)-CHR¹⁶R¹⁷, or 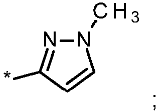
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹²: represents a hydrogen atom or methyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹³: represents -(CO)-R¹¹, -(CO)-C≡C-R¹², -(SO₂)-CH=CH₂, or oxirane-2-carbonyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹³: represents -(CO)-R¹¹, -(CO)-C≡C-R¹², or -(SO₂)-CH=CH₂;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹³: represents -(CO)-CH=CH₂, -(CO)-CH=CH-CH₂-N(CH₃)₂, or -(SO₂)-CH=CH₂;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13a}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13a}: represents a hydrogen atom, or methyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13b}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13b}: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13c}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13c}: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13d}: represents a hydrogen atom, methyl, fluoro, or -N(CH₃)-CH₂-CH₂-N(CH₃)-CHR¹⁶R¹⁷;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13d}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13e}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13e}: represents a hydrogen atom, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13f}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13f}: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R^{13g}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
In a further embodiment of the first aspect, the present invention covers compounds of formula (I), *supra,* in which:
- R^{13g}: represents a hydrogen atom;
or an N-oxide, a salt, a tautomer, a rotamer, or a stereoisomer of said compound, or a salt of said N-oxide, tautomer, rotamer, or stereoisomer.
- R^{13h}: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
In a further embodiment of the first aspect, the present invention covers compounds of formula (I), *supra,* in which:
- R^{13h}: represents a hydrogen atom;
or an N-oxide, a salt, a tautomer, a rotamer, or a stereoisomer of said compound, or a salt of said N-oxide, tautomer, rotamer, or stereoisomer.
- R¹³ⁱ: represents a hydrogen atom, methyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
In a further embodiment of the first aspect, the present invention covers compounds of formula (I), *supra,* in which:
- R¹³ⁱ: represents a hydrogen atom;
or an N-oxide, a salt, a tautomer, a rotamer, or a stereoisomer of said compound, or a salt of said N-oxide, tautomer, rotamer, or stereoisomer.
- R¹⁴: represents =CH₂, =CH-CH₃, =CH-CH₂₋N(CH3)-CHR¹⁶R¹⁷, or =CH-CH₂₋R¹⁸;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁴: represents =CH₂, or =CH-CH₂₋N(CH₃)-CHR¹⁶R¹⁷;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁵: represents a hydrogen atom, C₁-C₃-alkyl, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁵: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁶: represents a hydrogen atom or methyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁶: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁷: represents a hydrogen atom or methyl;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁷: represents a hydrogen atom;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁸: represents a group selected from the group: 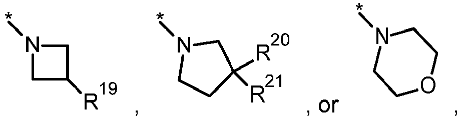 wherein * indicates the point of attachment of said group with the rest of the molecule;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R¹⁹: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R²⁰: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In a further embodiment of the first aspect, the present invention covers compounds of formula (I), *supra,* in which:
- R²¹: represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

In a particular further embodiment of the first aspect, the present invention covers combinations of two or more of the above mentioned embodiments of the first aspect.

A further aspect of the invention relates to compounds of formula (I), which are present as their salts, such as pharmaceutically acceptable salts.

It is to be understood that the present invention relates to any sub-combination within any embodiment or aspect of the present invention of compounds of formula (I), supra.

More particularly still, the present invention covers compounds of formula (I) which are disclosed in the Example section of this text, infra.

In a further embodiment, the present disclosure provides the compound of formula (I), or an N-oxide, a salt, a tautomer, a rotamer, or a stereoisomer of said compound, or a salt of said N-oxide, tautomer, rotamer, or stereoisomer thereof, for use in the treatment or prophylaxis of diseases.

Pharmaceutical compositions comprising a compound of formula (I) are also provided. The pharmaceutical composition may comprise a compound of formula (I), or an N-oxide, a salt, a tautomer, a rotamer, or a stereoisomer of said compound, or a salt of said N-oxide, tautomer, rotamer, or stereoisomer thereof, and at least one pharmaceutically acceptable auxiliary.

In a further aspect, combinations are provided comprising a compound of formula (I) , or an N-oxide, a salt, a tautomer, a rotamer, or a stereoisomer of said compound, or a salt of said N-oxide, tautomer, rotamer, or stereoisomer thereof, and one or more second active ingredients typically selected from chemotherapeutic anti-cancer agents and target-specific anti-cancer agents.

Methods of use are also provided. In some embodiments, the method may be for inhibition EGF-receptor kinase activity in a cancer cell, the method comprising contacting the cancer cell with a compound of formula (I) , or an N-oxide, a salt, a tautomer, a rotamer, or a stereoisomer of said compound, or a salt of said N-oxide, tautomer, rotamer, or stereoisomer thereof. In some embodiments, the method may be for reducing the survival cancer cell or inducing death in a cancer cell, the method comprising contacting a cancer cell comprising a mutation in an EGF-receptor with a compound of formula (I) , or an N-oxide, a salt, a tautomer, a rotamer, or a stereoisomer of said compound, or a salt of said N-oxide, tautomer, rotamer, or stereoisomer thereof. The disclosed methods also include methods of treating cancer in subject, the method comprising administering to the subject and effective amount of a compound of formula (I) , or an N-oxide, a salt, a tautomer, a rotamer, or a stereoisomer of said compound, or a salt of said N-oxide, tautomer, rotamer, or stereoisomer thereof.

In a further embodiment, methods for selecting a patient for cancer treatment with a compound of formula (I) , or an N-oxide, a salt, a tautomer, a rotamer, or a stereoisomer of said compound, or a salt of said N-oxide, tautomer, rotamer, or stereoisomer thereof, are provided, the method comprising detecting the presence of a mutation in exon 20 of the EGF-receptor in a biological sample of the subject, thereby determining that the patient should be treated with the compound of formula (I) , or an N-oxide, a salt, a tautomer, a rotamer, or a stereoisomer of said compound, or a salt of said N-oxide, tautomer, rotamer, or stereoisomer thereof.

In accordance with another aspect, the present invention covers methods of preparing compounds of the present invention, said methods comprising the steps as described in the Experimental Section herein.

Another embodiment of the invention are compounds according as disclosed in the Claims section or disclosed analogs of the exemplified compounds and subcombinations thereof.

### Definitions

It is to be understood that embodiments disclosed herein are not meant to be understood as individual embodiments which would not relate to one another. Features discussed with one embodiment or aspect of the invention are meant to be disclosed also in connection with other embodiments or aspects of the invention shown herein. If, in one case, a specific feature is not disclosed with one embodiment or aspect of the invention, but with another, the skilled person would understand that does not necessarily mean that said feature is not meant to be disclosed with said other embodiment or aspect of the invention. The skilled person would understand that it is the gist of this application to disclose said feature also for the other embodiment or aspect of the invention, but that just for purposes of clarity and to keep the length of this specification manageable. For example, it is to be understood that all aspects, embodiments, pharmaceutical compositions, combinations, uses and/or methods of the present invention defined herein for the compounds of formula (I) also relate to more specific embodiments of the compounds of formula (I), such as, but not limited to, the compounds of formula (la) and vice-versa, for example.

It is further to be understood that the content of the documents referred to herein is incorporated by reference in their entirety, namely when e.g. a method is discussed details of which are described in said document. This approach serves to keep the length of this specification manageable.

The term "comprising" when used in the specification includes "consisting of".

If it is referred to "as mentioned above" or "mentioned above", *"supra"* within the description it is referred to any of the disclosures made within the specification in any of the preceding pages.

If it is referred to "as mentioned herein", "described herein", "provided herein," or "as mentioned in the present text," or "stated herein" within the description it is referred to any of the disclosures made within the specification in any of the preceding or subsequent pages.

By "subject" is meant a mammal, including, but not limited to, a human or non-human mammal, such as a bovine, equine, canine, ovine, or feline.

"Suitable" within the sense of the invention means chemically possible to be made by methods within the knowledge of a skilled person.

The terms as mentioned in the present text may have the following meanings:
The term "C₁-C₆-alkyl" means a linear or branched, saturated, monovalent hydrocarbon group having 1, 2, 3, 4, 5 or 6 carbon atoms, e.g. a methyl, ethyl, propyl, isopropyl, butyl, sec-butyl, isobutyl, *tert*-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("C₁-C₄-alkyl"), e.g. a methyl, ethyl, propyl, isopropyl, butyl, sec-butyl isobutyl, or *tert-*butyl group, more particularly 1, 2 or 3 carbon atoms ("C₁-C₃-alkyl"), e.g. a methyl, ethyl, *n-*propyl or isopropyl group.

The term "C₁-C₆-haloalkyl" means a linear or branched, saturated, monovalent hydrocarbon group in which the term "C₁-C₆-alkyl" is as defined *supra,* and in which one or more of the hydrogen atoms are replaced, identically or differently, with a halogen atom. Particularly, said halogen atom is a fluorine atom. Said C₁-C₆-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl.

The term "C₁-C₆-alkanediyl" means a diradical of a C₁-C₆-alkyl group with radical centers on different skeletal atoms, formally derived by removal of one hydrogen atom from each of two skeletal atoms.

The term "C₁-C₆-haloalkanediyl" means a diradical of a C₁-C₆-haloalkyl group with radical centers on different skeletal atoms, formally derived by removal of one hydrogen atom from each of two skeletal atoms.

The term "heteroaryl" means a monovalent, monocyclic, bicyclic or tricyclic aromatic ring having 5, 6, 8, 9, 10, 11, 12, 13 or 14 ring atoms (a "5 to 14 membered heteroaryl" group), particularly 5, 6, 9 or 10 ring atoms, which contains at least one ring heteroatom and optionally one, two or three further ring heteroatoms from the series: N, O and/or S, and which is bound via a ring carbon atom or optionally via a ring nitrogen atom (if allowed by valency).

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl, pyridazinyl, pyrimidinyl, pyrazinyl or triazinyl; or a tricyclic heteroaryl group, such as, for example, carbazolyl, acridinyl or phenazinyl; or a 9-membered heteroaryl group, such as, for example, benzofuranyl, benzothienyl, benzoxazolyl, benzisoxazolyl, benzimidazolyl, benzothiazolyl, benzotriazolyl, indazolyl, indolyl, isoindolyl, indolizinyl or purinyl; or a 10-membered heteroaryl group, such as, for example, quinolinyl, quinazolinyl, isoquinolinyl, cinnolinyl, phthalazinyl, quinoxalinyl or pteridinyl.

In general, and unless otherwise mentioned, the heteroaryl or heteroarylene groups include all possible isomeric forms thereof, e.g.: tautomers and positional isomers with respect to the point of linkage to the rest of the molecule. Thus, for some illustrative non-restricting examples, the term pyridinyl includes pyridin 2 yl, pyridin 3 yl and pyridin 4 yl; or the term thienyl includes thien 2 yl and thien 3 yl.

Further, as used herein, the term "C₁-C₆", as used throughout this text, e.g. in the context of the definition of "C₁-C₆-alkyl", is to be understood as meaning an alkyl group having a finite number of carbon atoms of 1 to 6, *i.e.* 1, 2, 3, 4, 5, or 6 carbon atoms. It is to be understood further that the term "C₃-C₆" is to be interpreted as any sub-range comprised therein, *e*.*g*. C₃-C₆, C₄-C₅, C₃-C₅, C₃-C₄, C₄-C₆, C₅-C₆; particularly C₃-C₆.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

As used herein, the term "one or more", *e*.*g*. in the definition of the substituents of the compounds of the formulae of the present invention, is understood as meaning "one, two, three, four, five, etc. particularly one, two, three or four, more particularly one, two or three, even more particularly one or two".

The compounds of formula (I) may exist as isotopic variants. The invention therefore includes one or more isotopic variant(s) of the compounds of formula (I), particularly deuterium-containing compounds of formula (I).

The term "isotopic variant" of a compound or a reagent is defined as a compound exhibiting an unnatural proportion of one or more of the isotopes that constitute such a compound.

The term "isotopic variant of the compound of formula (I)" is defined as a compound of formula (I) exhibiting an unnatural proportion of one or more of the isotopes that constitute such a compound.

The expression "unnatural proportion" is to be understood as meaning a proportion of such isotope which is higher than its natural abundance. The natural abundances of isotopes to be applied in this context are described in "Isotopic Compositions of the Elements 1997", Pure Appl. Chem., 70(1), 217-235, 1998. Examples of such isotopes include stable and radioactive isotopes of hydrogen, carbon, nitrogen, oxygen, phosphorus, sulfur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, respectively.

With respect to the treatment and/or prophylaxis of the disorders specified herein the isotopic variant(s) of the compounds of formula (I) in one embodiment contain deuterium ("deuterium-containing compounds of formula (I)"). Isotopic variants of the compounds of formula (I) in which one or more radioactive isotopes, such as ³H or ¹⁴C, are incorporated are useful *e.g*. in drug and/or substrate tissue distribution studies. These isotopes are particularly suited for the ease of their incorporation and detectability. Positron emitting isotopes such as ¹⁸F or ¹¹C may be incorporated into a compound of formula (I). These isotopic variants of the compounds of formula (I) are useful for *in vivo* imaging applications. Deuterium-containing and ¹³C-containing compounds of formula (I) can be used in mass spectrometry analyses (H. J. Leis et al., Curr. Org. Chem., 1998, 2, 131) in the context of preclinical or clinical studies.

Isotopic variants of the compounds of formula (I) can generally be prepared by methods known to a person skilled in the art, such as those described in the schemes and/or examples herein, by substituting a reagent for an isotopic variant of said reagent, in one embodiment for a deuterium-containing reagent. Depending on the desired sites of deuteration, in some cases deuterium from D₂O can be incorporated either directly into the compounds or into reagents that are useful for synthesizing such compounds (Esaki et al., Tetrahedron, 2006, 62, 10954; Esaki et al., Chem. Eur. J., 2007, 13, 4052). Deuterium gas is also a useful reagent for incorporating deuterium into molecules. Catalytic deuteration of olefinic bonds (H. J. Leis et al., Curr. Org. Chem., 1998, 2, 131; J. R. Morandi et al., J. Org. Chem., 1969, 34 (6), 1889) and acetylenic bonds (N. H. Khan, J. Am. Chem. Soc., 1952, 74 (12), 3018; S. Chandrasekhar et al., Tetrahedron, 2011, 52, 3865) is a rapid route for incorporation of deuterium. Metal catalysts (i.e. Pd, Pt, and Rh) in the presence of deuterium gas can be used to directly exchange deuterium for hydrogen in functional groups containing hydrocarbons (J. G. Atkinson et al., US Patent 3966781). A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA. Further information on the state of the art with respect to deuterium-hydrogen exchange is given for example in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1993; J. Atzrodt et al., Angew. Chem., Int. Ed. 2007, 46, 7744; K. Matoishi et al., J. Chem. Soc, Chem. Commun. 2000, 1519-1520; K. Kassahun et al., WO2012/112363.

The term "deuterium-containing compound of formula (I)" is defined as a compound of formula (I), in which one or more hydrogen atom(s) is/are replaced by one or more deuterium atom(s) and in which the abundance of deuterium at each deuterated position of the compound of formula (I) is higher than the natural abundance of deuterium, which is about 0.015%. Particularly, in a deuterium-containing compound of formula (I) the abundance of deuterium at each deuterated position of the compound of formula (I) is higher than 10%, 20%, 30%, 40%, 50%, 60%, 70% or 80%, in one embodiment higher than 90%, 95%, 96% or 97%, in other embodiments higher than 98% or 99% at said position(s). It is understood that the abundance of deuterium at each deuterated position is independent of the abundance of deuterium at other deuterated position(s).

The selective incorporation of one or more deuterium atom(s) into a compound of formula (I) may alter the physicochemical properties (such as for example acidity [A. Streitwieser et al., J. Am. Chem. Soc., 1963, 85, 2759; C. L. Perrin, et al., J. Am. Chem. Soc., 2007, 129, 4490], basicity [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (D. J. Kushner et al., Can. J. Physiol. Pharmacol., 1999, 77, 79; A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of formula (I). In some cases deuterium substitution reduces or eliminates the formation of an undesired or toxic metabolite and enhances the formation of a desired metabolite (*e*.*g*. Nevirapine: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. Indiplon (A. J. Morales et al., Abstract 285, The 15th North American Meeting of the International Society of Xenobiotics, San Diego, CA, October 12-16, 2008), ML-337 (C. J. Wenthur et al., J. Med. Chem., 2013, 56, 5208), and Odanacatib (K. Kassahun et al., WO2012/112363) are examples for this deuterium effect. Still other cases have been reported in which reduced rates of metabolism result in an increase in exposure of the drug without changing the rate of systemic clearance (*e*.*g*. Rofecoxib: F. Schneider et al., Arzneim. Forsch. Drug. Res., 2006, 56, 295; Telaprevir: F. Maltais et al., J. Med. Chem., 2009, 52, 7993). Deuterated drugs showing this effect may have reduced dosing requirements (*e*.*g*. lower number of doses or lower dosage to achieve the desired effect) and/or may produce lower metabolite loads.

A compound of formula (I) may have multiple potential sites of attack for metabolism. To optimize the above-described effects on physicochemical properties and metabolic profile, deuterium-containing compounds of formula (I) having a certain pattern of one or more deuterium-hydrogen exchange(s) can be selected. Particularly, the deuterium atom(s) of deuterium- containing compound(s) of formula (I) is/are attached to a carbon atom and/or is/are located at those positions of the compound of formula (I), which are sites of attack for metabolizing enzymes such as *e*.*g*. cytochrome P₄₅₀.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

The compounds of this invention may contain one or more asymmetric centre, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds.

Substituents on a ring may also be present in either cis or trans form. It is intended that all such configurations (including enantiomers and diastereomers), are included within the scope of the present invention.

Typically, compounds of the present disclosure are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e*.*g*., chiral HPLC columns), with or without conventional derivatisation, optimally chosen to maximise the separation of the enantiomers. Suitable chiral HPLC columns are manufactured by Daicel, *e*.*g*., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e*.*g*. R- or S-isomers, or E- or Z-isomers, in any ratio. Isolation of a single stereoisomer, *e*.*g*. a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, namely :

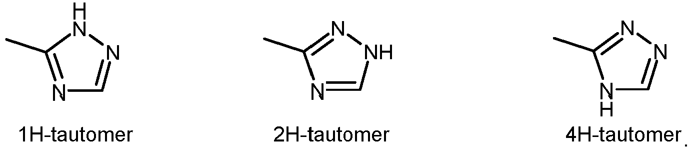

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also relates to useful forms of the compounds as disclosed herein, such as hydrates, solvates, salts, in particular pharmaceutically acceptable salts, and co-precipitates.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e*.*g*. a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in free form, *e*.*g*. as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, paratoluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x CF₃COOH", "x Na⁺", for example, are to be understood as not a stoichiometric specification, but solely as a salt form.

This applies analogously to cases in which synthesis intermediates or example compounds or salts thereof have been obtained, by the preparation and/or purification processes described, as solvates, such as hydrates with (if defined) unknown stoichiometric composition.

The salts include water-insoluble and, particularly, water-soluble salts.

Furthermore, derivatives of the compounds of formula (I) and the salts thereof which are converted into a compound of formula (I) or a salt thereof in a biological system (bioprecursors or pro-drugs) are covered by the invention. Said biological system is e.g. a mammalian organism, particularly a human subject. The bioprecursor is, for example, converted into the compound of formula (I) or a salt thereof by metabolic processes.

As used herein, the term *"in vivo* hydrolysable ester" is understood as meaning an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, *e*.*g*. methoxymethyl, C₁-C₆ alkanoyloxymethyl esters, *e*.*g*. pivaloyloxymethyl, phthalidyl esters, C₃-C₈ cycloalkoxy-carbonyloxy-C₁-C₆ alkyl esters, *e*.*g*. 1-cyclohexylcarbonyloxyethyl, 1,3-dioxolen-2-onylmethyl esters, *e.g.* 5-methyl-1,3-dioxolen-2-onylmethyl, and C₁-C₆-alkoxycarbonyloxyethyl esters, *e*.*g*. 1-methoxycarbonyloxyethyl, and may be formed at any carboxy group in the compounds of this invention.

An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.

Furthermore, the present invention includes all possible crystalline forms, or polymorphs, of the compounds of the present invention, either as single polymorphs, or as a mixture of more than one polymorphs, in any ratio.

In the context of the properties of the compounds of the present invention the term "pharmacokinetic profile" means one single parameter or a combination thereof including permeability, bioavailability, exposure, and pharmacodynamic parameters such as duration, or magnitude of pharmacological effect, as measured in a suitable experiment. Compounds with improved pharmacokinetic profiles can, for example, be used in lower doses to achieve the same effect, may achieve a longer duration of action, or a may achieve a combination of both effects.

The term "combination" in the present invention is used as known to persons skilled in the art and may be present as a fixed combination, a non-fixed combination or kit-of-parts.

A "fixed combination" in the present invention is used as known to persons skilled in the art and is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A non-fixed combination or "kit-of-parts" in the present invention is used as known to persons skilled in the art and is defined as a combination wherein the said first active ingredient and the said second active ingredient are present in more than one unit. One example of a non-fixed combination or kit-of-parts is a combination wherein the said first active ingredient and the said second active ingredient are present separately. The components of the non-fixed combination or kit-of-parts may be administered separately, sequentially, simultaneously, concurrently or chronologically staggered. Any such combination of a compound of formula (I) of the present invention with an anti-cancer agent as defined below is an embodiment of the invention.

The term "(chemotherapeutic) anti-cancer agents" relates to any agent that reduces the survival or proliferation of a cancer cell, and includes but is not limited to 131I-chTNT, abarelix, abiraterone, aclarubicin, ado-trastuzumab emtansine, afatinib, aflibercept, aldesleukin, alemtuzumab, Alendronic acid, alitretinoin, altretamine, amifostine, aminoglutethimide, Hexyl aminolevulinate, amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, basiliximab, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, copanlisib, crisantaspase, cyclophosphamide, cyproterone, cytarabine, dacarbazine, dactinomycin, darbepoetin alfa, dabrafenib, dasatinib, daunorubicin, decitabine, degarelix, denileukin diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, eculizumab, edrecolomab, elliptinium acetate, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (123I), iomeprol, ipilimumab, irinotecan, Itraconazole, ixabepilone, lanreotide, lapatinib, lasocholine, lenalidomide, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumabpentetreotide, nilotinib, nilutamide, nimorazole, nimotuzumab, nimustine, nitracrine, nivolumab, obinutuzumab, octreotide, ofatumumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palifermin, palladium-103 seed, palonosetron, pamidronic acid, panitumumab, pantoprazole, pazopanib, pegaspargase, PEG-epoetin beta (methoxy PEG-epoetin beta), pembrolizumab, pegfilgrastim, peginterferon alfa-2b, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, poziotinib, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib, regorafenib, risedronic acid, rhenium-186 etidronate, rituximab, romidepsin, romiplostim, romurtide, roniciclib, samarium (153Sm) lexidronam, sargramostim, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

By "Epidermal Growth Factor Receptor (EGFR) Polypeptide" is meant a polypeptide having at least about 95% amino acid sequence identity to the sequence provided at UniProt Accession No. P00533-1 (SEQ ID No. 1) or a fragment thereof. In some embodiments, the EGFR fragment binds an EFGR ligand and/or has kinase activity. Mutant EGFR polypeptides include those having an insertion between, for example, amino acids V769 and D770 or between D770 and N771. In other embodiments, the amino acid sequence identity is 96, 97, 98, 99, or 100% to UniProt Accession No. P00533-1 (SEQ ID No. 1).

An exemplary full length sequence of human EGFR, which indicates V769, D770, and N771 in bold, is provided at UniProt Accession No. P00533-1 (SEQ ID No. 1), which is reproduced below:

10 20 30 40 50

60 70 80 90 100

110 120 130 140 150

160 170 180 190 200

210 220 230 240 250

260 270 280 290 300

310 320 330 340 350

360 370 380 390 400

410 420 430 440 450

460 470 480 490 500


510 520 530 540 550

560 570 580 590 600

610 620 630 640 650

660 670 680 690 700

710 720 730 740 750

760 770 780 790 800

810 820 830 840 850

860 870 880 890 900

910 920 930 940 950

960 970 980 990 1000

1010 1020 1030 1040 1050

1060 1070 1080 1090 1100

1110 1120 1130 1140 1150

1160 1170 1180 1190 1200

1210


By "Epidermal Growth Factor Receptor (EGFR) Polynucleotide" is meant a nucleic acid molecule encoding an EGFR polypeptide or fragment thereof. An exemplary polynucleotide encoding EGFR is provided at NCBI Reference Sequence: NM_001346897.1 (SEQ ID No. 2), which is reproduced below:

1 gtccgggcag cccccggcgc agcgcggccg cagcagcctc cgcccccgc
acggtgtgag
61 cgcccgacgc ggccgaggcg gccggagtcc cgagctagcc ccggcggccg
ccgccgccca
121 gaccggacga caggccacct cgtcggcgtc cgcccgagtc cccgcctcgc
cgccaacgcc
181 acaaccaccg cgcacggccc cctgactccg tccagtattg atcgggagag
ccggagcgag
241 ctcttcgggg agcagcgatg cgaccctccg ggacggccgg ggcagcgctc
ctggcgctgc
301 tggctgcgct ctgcccggcg agtcgggctc tggaggaaaa gaaagtttgc
caaggcacga
361 gtaacaagct cacgcagttg ggcacttttg aagatcattt tctcagcctc
cagaggatgt
421 tcaataactg tgaggtggtc cttgggaatt tggaaattac ctatgtgcag
aggaattatg
481 atctttcctt cttaaagacc atccaggagg tggctggtta tgtcctcatt
gccctcaaca
541 cagtggagcg aattcctttg gaaaacctgc agatcatcag aggaaatatg
tactacgaaa
601 attcctatgc cttagcagtc ttatctaact atgatgcaaa taaaaccgga
ctgaaggagc
661 tgcccatgag aaatttacag ggccaaaagt gtgatccaag ctgtcccaat
gggagctgct
721 ggggtgcagg agaggagaac tgccagaaac tgaccaaaat catctgtgcc
cagcagtgct
781 ccgggcgctg ccgtggcaag tcccccagtg actgctgcca caaccagtgt
gctgcaggct
841 gcacaggccc ccgggagagc gactgcctgg tctgccgcaa attccgagac
gaagccacgt
901 gcaaggacac ctgcccccca ctcatgctct acaaccccac cacgtaccag
atggatgtga
961 accccgaggg caaatacagc tttggtgcca cctgcgtgaa gaagtgtccc
cgtaattatg
1021 tggtgacaga tcacggctcg tgcgtccgag cctgtggggc cgacagctat
gagatggagg

1081 aagacggcgt ccgcaagtgt aagaagtgcg aagggccttg ccgcaaagtg
tgtaacggaa
1141 taggtattgg tgaatttaaa gactcactct ccataaatgc tacgaatatt
aaacacttca
1201 aaaactgcac ctccatcagt ggcgatctcc acatcctgcc ggtggcattt
aggggtgact
1261 ccttcacaca tactcctcct ctggatccac aggaactgga tattctgaaa
accgtaaagg
1321 aaatcacagg gtttttgctg attcaggctt ggcctgaaaa caggacggac
ctccatgcct
1381 ttgagaacct agaaatcata cgcggcagga ccaagcaaca tggtcagttt
tctcttgcag
1441 tcgtcagcct gaacataaca tccttgggat tacgctccct caaggagata
agtgatggag
1501 atgtgataat ttcaggaaac aaaaatttgt gctatgcaaa tacaataaac
tggaaaaaac
1561 tgtttgggac ctccggtcag aaaaccaaaa ttataagcaa cagaggtgaa
aacagctgca
1621 aggccacagg ccaggtctgc catgccttgt gctcccccga gggctgctgg
ggcccggagc
1681 ccagggactg cgtctcttgc cggaatgtca gccgaggcag ggaatgcgtg
gacaagtgca
1741 accttctgga gggtgagcca agggagtttg tggagaactc tgagtgcata
cagtgccacc
1801 cagagtgcct gcctcaggcc atgaacatca cctgcacagg acggggacca
gacaactgta
1861 tccagtgtgc ccactacatt gacggccccc actgcgtcaa gacctgcccg
gcaggagtca
1921 tgggagaaaa caacaccctg gtctggaagt acgcagacgc cggccatgtg
tgccacctgt
1981 gccatccaaa ctgcacctac ggatgcactg ggccaggtct tgaaggctgt
ccaacgaatg
2041 ggcctaagat cccgtccatc gccactggga tggtgggggc cctcctcttg
ctgctggtgg
2101 tggccctggg gatcggcctc ttcatgcgaa ggcgccacat cgttcggaag
cgcacgctgc

2161 ggaggctgct gcaggagagg gagcttgtgg agcctcttac acccagtgga
gaagctccca
2221 accaagctct cttgaggatc ttgaaggaaa ctgaattcaa aaagatcaaa
gtgctgggct
2281 ccggtgcgtt cggcacggtg tataagggac tctggatccc agaaggtgag
aaagttaaaa
2341 ttcccgtcgc tatcaaggaa ttaagagaag caacatctcc gaaagccaac
aaggaaatcc
2401 tcgatgaagc ctacgtgatg gccagcgtgg acaaccccca cgtgtgccgc
ctgctgggca
2461 tctgcctcac ctccaccgtg cagctcatca cgcagctcat gcccttcggc
tgcctcctgg
2521 actatgtccg ggaacacaaa gacaatattg gctcccagta cctgctcaac
tggtgtgtgc
2581 agatcgcaaa gggcatgaac tacttggagg accgtcgctt ggtgcaccgc
gacctggcag
2641 ccaggaacgt actggtgaaa acaccgcagc atgtcaagat cacagatttt
gggctggcca
2701 aactgctggg tgcggaagag aaagaatacc atgcagaagg aggcaaagtg
cctatcaagt
2761 ggatggcatt ggaatcaatt ttacacagaa tctataccca ccagagtgat
gtctggagct
2821 acggggtgac tgtttgggag ttgatgacct ttggatccaa gccatatgac
ggaatccctg
2881 ccagcgagat ctcctccatc ctggagaaag gagaacgcct ccctcagcca
cccatatgta
2941 ccatcgatgt ctacatgatc atggtcaagt gctggatgat agacgcagat
agtcgcccaa
3001 agttccgtga gttgatcatc gaattctcca aaatggcccg agacccccag
cgctaccttg
3061 tcattcaggg ggatgaaaga atgcatttgc caagtcctac agactccaac
ttctaccgtg
3121 ccctgatgga tgaagaagac atggacgacg tggtggatgc cgacgagtac
ctcatcccac
3181 agcagggctt cttcagcagc ccctccacgt cacggactcc cctcctgagc
tctctgagtg

3241 caaccagcaa caattccacc gtggcttgca ttgatagaaa tgggctgcaa
agctgtccca
3301 tcaaggaaga cagcttcttg cagcgataca gctcagaccc cacaggcgcc
ttgactgagg
3361 acagcataga cgacaccttc ctcccagtgc ctggtgagtg gcttgtctgg
aaacagtcct
3421 gctcctcaac ctcctcgacc cactcagcag cagccagtct ccagtgtcca
agccaggtgc
3481 tccctccagc atctccagag ggggaaacag tggcagattt gcagacacag
tgaagggcgt
3541 aaggagcaga taaacacatg accgagcctg cacaagctct ttgttgtgtc
tggttgtttg
3601 ctgtacctct gttgtaagaa tgaatctgca aaatttctag cttatgaagc
aaatcacgga
3661 catacacatc tgtgtgtgtg agtgttcatg atgtgtgtac atctgtgtat
gtgtgtgtgt
3721 gtatgtgtgt gtttgtgaca gatttgatcc ctgttctctc tgctggctct
atcttgacct
3781 gtgaaacgta tatttaacta attaaatatt agttaatatt aataaatttt
aagctttatc
3841 cagaaaaaaa aaaaaaaaa

By "fragment" is meant a portion of a polypeptide or nucleic acid molecule. This portion contains, for example, at least 10%, 20%, 30%, 40%, 50%, 60%, 70%, 80%, or 90% of the entire length of the reference nucleic acid molecule or polypeptide. A fragment may contain 10, 20, 30, 40, 50, 60, 70, 80, 90, or 100, 200, 300, 400, 500, 600, 700, 800, 900, or 1000 nucleotides or amino acids.

The intermediates used for the synthesis of the compounds of claims 1-4 as described below, as well as their use for the synthesis of the compounds of claims 1-4, are one further aspect of the present invention. Certain intermediates are the Intermediate Examples as disclosed below.

### General Procedures

The compounds according to the invention can be prepared according to the following schemes 1 - 9.

The schemes and procedures described below illustrate synthetic routes to the compounds of formula **(I)** of the invention and are not intended to be limiting. It is obvious to the person skilled in the art that the order of transformations as exemplified in the schemes can be modified in various ways. The order of transformations exemplified in the schemes is therefore not intended to be limiting. In addition, interconversion of any of the substituents R², R³, R⁴, R⁵, R⁷, R⁸, R¹³ (*e.g*., R^{13a}, R^{13b}, R^{13c}, R^{13d}, R^{13e}, R^{13f}, R^{13g}), and PG **can** be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art. Specific examples are described in the subsequent paragraphs.

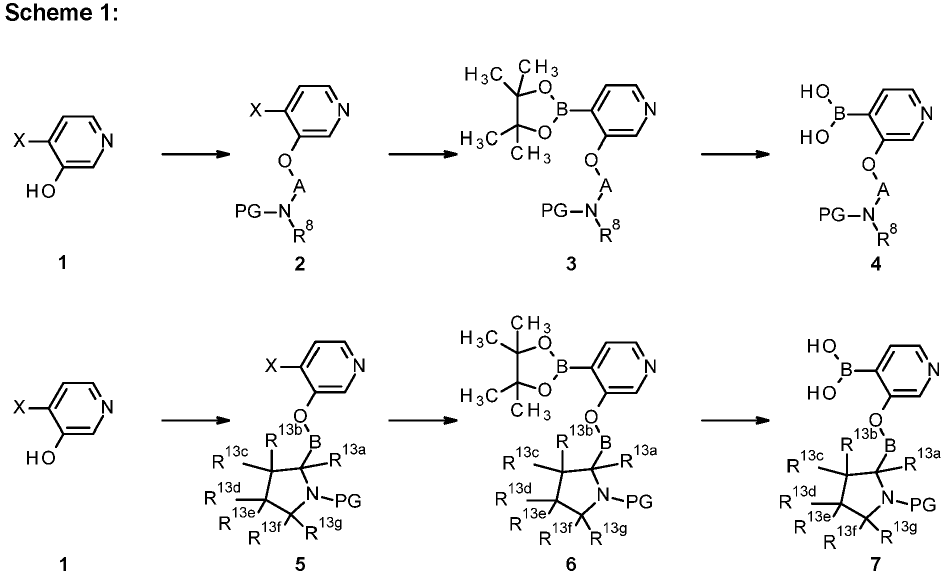

*Scheme* 1: Route for the preparation of intermediates of the general formulas 4 and 7, wherein R⁸, R^{13a}, R^{13b}, R^{13c}, R^{13d}, R^{13e}, R^{13f}, R^{13g} have the meaning as given for general formula (I). A represents a group selected from the group: (C₂-C₆-alkanediyl) or CH₂-(C₁-C₅-haloalkanediyl) as described for the general formula (I). B represents a group (C₁-C₅-alkanediyl) as described for the general formula (I). Compound 5 stands exemplary for a group of 4- to 6- membered rings according to the description for R⁹ as given in the description of general formula (I). PG can be hydrogen or optionally a suitable protecting group, e.g. tert-butoxycarbonyl (Boc) or any other suitable protecting group as known to one skilled in the art (or as generally described in the chemical literature such as in *Greens Protecting Group in Organic Chemistry*)*.* X represents a halogen, such as Cl, Br or I. Compounds of the general formula **1** can be converted to compounds of the general formula **2** and **5** by reacting for example suitable alcohols in the presence of triphenylphosphine with azo compounds (Mitsonubu type reactions). Optional, compounds of the general formulas **2** and **5** can be obtained by reacting **1** with corresponding elctrophiles such as bromides or chlorides in the presence of bases such as cesiumcarbonate, sodium hydride or any other base known to persons skilled in the art. Suitable electrophiles can also been generated for example from corresponding trichloroacetimidates under the influence of lewis acids such as BF₃OEt₂ or of acids such as trifluoromethanesulfonic acid or any other suitable acid known to those skilled in the art. O-alkylations of compound **1** of this type are conducted in solvents such as dichloromethane, pentane or cyclohexane without employing additional bases. Introduction of boronic acid esters like for example (but not limited to) those depicted in **3** and **6** can be synthezised from **2** and **5** by palladium catalyzed reactions with octamethyl-2,2'-bi-1,3,2-dioxaborolane in the presence of potassium acetate or other suitable bases knows to those skilled in the art. A suitable catalyst is for example 1-1'-bis(diphenylphosphino)ferrocenepalladium(ll)chloride. The hydrolytic transformation from **3** and **6** to the boronic acids **4** and **7** can be performed for example by employing water or slightly basic aqueous buffer solutions know to a person skilled in the art.

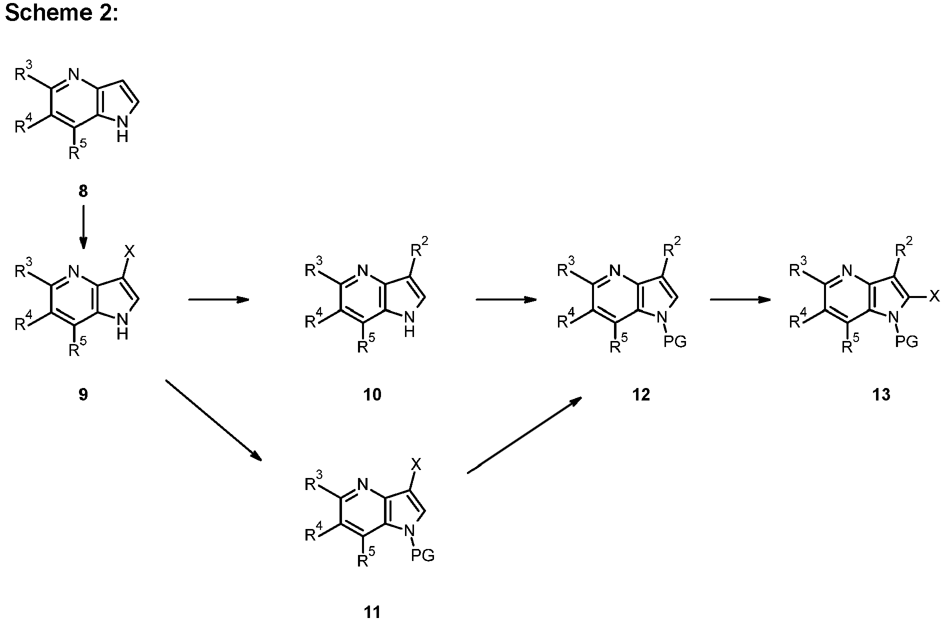

*Scheme 2:* Route for the preparation of intermediates of the general formula **13** wherein R², R³, R⁴, R⁵ have the meaning as given for general formula (I) and PG can be a suitable protecting group, *e*.*g*. tert-butoxycarbonyl (Boc), 2-(trimethylsilyl)-ethoxymethyl (SEM) or any other suitable protecting groups as known to one skilled in the art (or as generally described in the chemical literature such as in *Greens Protecting Group in Organic Chemistry*)*.* X represents a halogen, such as Cl, Br or I. Compounds of the general formula **8** can be converted to compounds of the general formula **9** by reactions to introduce halogen known to a person skilled in the art. Introduction of Br may occur by using N-Bromsuccinimid (NBS) in solvents such as DMF or acetonitrile in a temperature range from -30°C to the boiling point of the respective solvent. Compounds of the general formula **9** can be converted to compounds of the general formula **10** by reacting for example suitable boronic acids in a Suzuki-type reaction, employing palladium catalysts such as bis(triphenylphosphine)palladium(II) dichloride (but not limited to) in the presence of a base for example potassium carbonate in solvents such as propanol, water and mixtures thereof, 1,4-dioxane or any other solvent known to those skilled in the art at temperatures ranging from 0°C to the boling point of the respective solvent. Alternatively, compounds of the general formula **9** can be converted to compounds of the general formula **11** by using protection reactions known to those skilled in the art (or as generally described in the chemical literature such as in *Greens Protecting Group in Organic Chemistry).* For example, the tert-butoxycarbonyl (Boc) group can be introduced by reacting **9** with di-tert-butyl dicarbonate in suitable solvents such as dichloromethane in the presence of a base such as triethylamine at a temperature ranging from -30°C to the boiling point of the solvent used. SEM can be introduced for example (and not limited to) by reacting **9** with sodium hydride in tetrahydrofuran with subsequent addition of [2-(chloromethoxy)ethyl](trimethyl)silane at a temperature ranging from - 30°C to the boiling point of the respective solvent. Transformations of compounds of the general formula **10** to compounds of the general formula **12** can be performed by employing reactions in an analogous manner than those described for transformations of **9** to **11.** Alternatively, compounds of the general formula **12** can be prepared from compounds of the general formula **11** by employing reactions in an analogous manner than those described for transformations of **9** to **11.** Compounds of the general formula **12** can be converted to compounds of the general formula **13** by reactions to introduce halogen known to a person skilled in the art. Introduction of Br may occur by using N-Bromsuccinimid (NBS) in solvents such as DMF or acetonitrile in a temperature range from -30°C to the boiling point of the respective solvent.

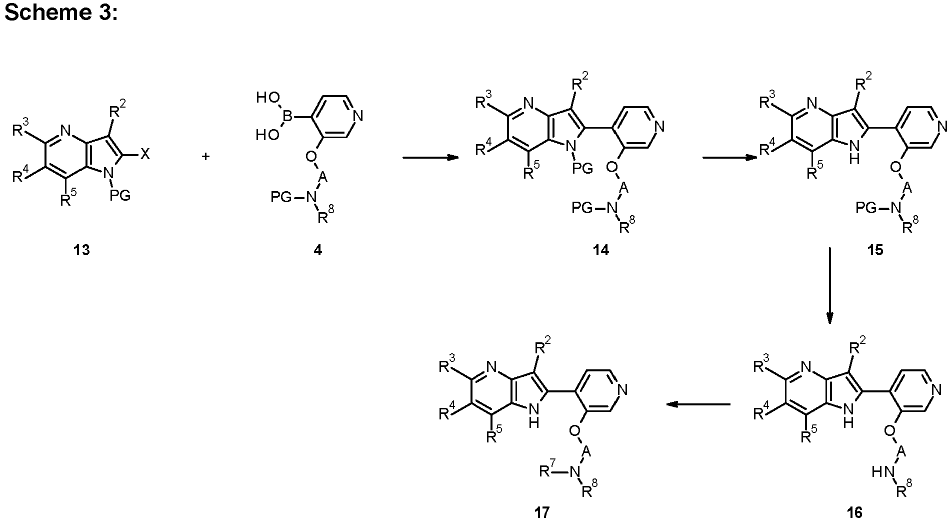

*Scheme 3:* Route for the preparation of compounds of general formula **17,** wherein, R², R³, R⁴, R⁵, R⁷, R⁸ have the meaning as given for general formula (I). A represents a group selected from the group: (C₂-C₆-alkanediyl) or CH₂-(C₁-C₅-haloalkanediyl) as described for the general formula (I). PG can be a suitable protecting group, *e*.*g*. tert-butoxycarbonyl (Boc), 2-(trimethylsilyl)-ethoxymethyl (SEM) or any other suitable protecting groups as known to one skilled in the art (or as generally described in the chemical literature such as in *Greens Protecting Group in Organic Chemistry).* X represents a halogen such as Cl, Br or I. Compounds of the general formula **13** can react with compounds of the general formula **4** in a Suzuki-type reaction to generate compounds of the general formula **14,** employing palladium catalysts such as bis(triphenylphosphine)palladium(ll) dichloride or tetrakis(triphenylphosphine)palladium(0) (but not limited to) in the presence of a base for example potassium carbonate in solvents such as propanol, water and mixtures thereof, 1,4-dioxane, DMF or any other solvent known to those skilled in the art at temperatures ranging from 0°C to the boling point of the respective solvent by conventional heating or by heating in a microwave apparatus. In cases in which PG represents tert-butoxycarbonyl, compounds of the general formula **15** can be prepared from compounds of the general formula **14** by deprotection reactions using acids like trifluoroacetic acid in dichloromethane or HCl in 1,4-dioxane, known to those skilled in the art (or as generally described in the chemical literature such as in *Greens Protecting Group in Organic Chemistry).* In cases where PG represents 2-(trimethylsilyl)-ethoxymethyl (SEM), removal of the protecting group can be accomplished by employing reagents such as tetrabutylammonium fluoride in tetrahydrofuran (or by any other method as described in the chemical literature such as in *Greens Protecting Group in Organic Chemistry).* In cases in which PG represents tert-butoxycarbonyl, compounds of the general formula **15** can be converted to compounds of the general formula **16** by deprotection reactions using acids like trifluoroacetic acid in dichloromethane or HCl in 1,4-dioxane, known to those skilled in the art (or as generally described in the chemical literature such as in *Greens Protecting Group in Organic Chemistry*)*.* Compounds of the general formula **16** can be converted to compounds of the general formula **17** using various methods known to those skilled in the art. Depending on the nature of R⁷ corresponding acid chlorides, sulfonylchlorides or other electrophiles can be used under basic conditions in a suitable solvent. If carboxylic acids are employed the corresponding reaction can be facilitated by the use coupling reagents such as HATU, EDC -HOBt or T3P. These reactions can be performed in a temperature range from -30°C to the boiling point of the respective solvent.

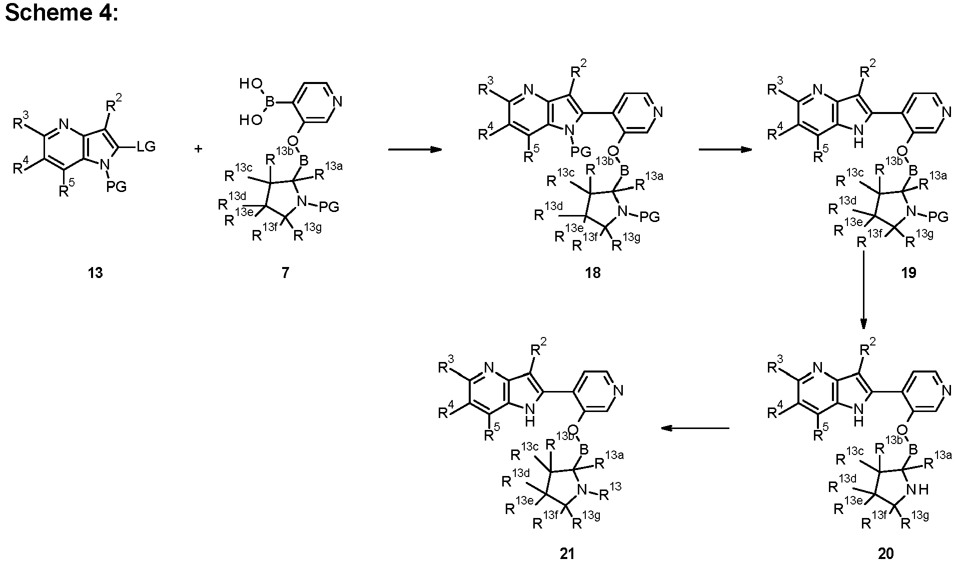

*Scheme 4:* Route for the preparation of compounds of general formula **21,** wherein, R², R³, R⁴, R⁵, R¹³, R^{13a}, R^{13b}, R^{13c}, R^{13d}, R^{13e}, R^{13f}, R^{13g} have the meaning as given for general formula (I). B represents a group (C₁-C₅-alkanediyl) as described for the general formula (I). Compound **7** stands exemplary for a group of 4- to 6- membered rings according to the description for R⁹ as given in the description of general formula (I). PG can be a suitable protecting group, e.g. tert-butoxycarbonyl (Boc), 2-(trimethylsilyl)-ethoxymethyl (SEM) or any other suitable protecting groups as known to one skilled in the art (or as generally described in the chemical literature such as in *Greens Protecting Group in Organic Chemistry*)*.* X represents a halogen such as Cl, Br or I. Compounds of the general formula **13** can react with compounds of the general formula 7 in a Suzuki-type reaction to generate compounds of the general formula **18,** employing palladium catalysts such as bis(triphenylphosphine)palladium(II) dichloride or tetrakis(triphenylphosphine)palladium(0) (but not limited to) in the presence of a base for example potassium carbonate in solvents such as propanol, water and mixtures thereof, 1,4-dioxane, DMF or any other solvent known to those skilled in the art at temperatures ranging from 0°C to the boling point of the respective solvent by conventional heating or by heating in a microwave apparatus. In cases in which PG represents tert-butoxycarbonyl, compounds of the general formula **19** can be prepared from compounds of the general formula **18** by deprotection reactions using acids like trifluoroacetic acid in dichloromethane or HCl in 1,4-dioxane, known to those skilled in the art (or as generally described in the chemical literature such as in *Greens Protecting Group in Organic Chemistry*)*.* In cases where PG represents 2-(trimethylsilyl)-ethoxymethyl (SEM), removal of the protecting group can be accomplished by employing reagents such as tetrabutylammonium fluoride in tetrahydrofuran (or by any other method as described in the chemical literature such as in *Greens Protecting Group in Organic Chemistry).* In cases in which PG represents tert-butoxycarbonyl, compounds of the general formula **19** can be converted to compounds of the general formula **20** by deprotection reactions using acids like trifluoroacetic acid in dichloromethane or HCl in 1,4-dioxane, known to those skilled in the art (or as generally described in the chemical literature such as in *Greens Protecting Group in Organic Chemistry*)*.* Compounds of the general formula **20** can be converted to compounds of the general formula **21** using various methods known to those skilled in the art. Depending on the nature of R⁷ corresponding acid chlorides, sulfonylchlorides or other electrophiles can be used under basic conditions in a suitable solvent. If carboxylic acids are employed the corresponding reaction can be facilitated by the use coupling reagents such as HATU, EDC -HOBt or T3P. These reactions can be performed in a temperature range from -30°C to the boiling point of the respective solvent.

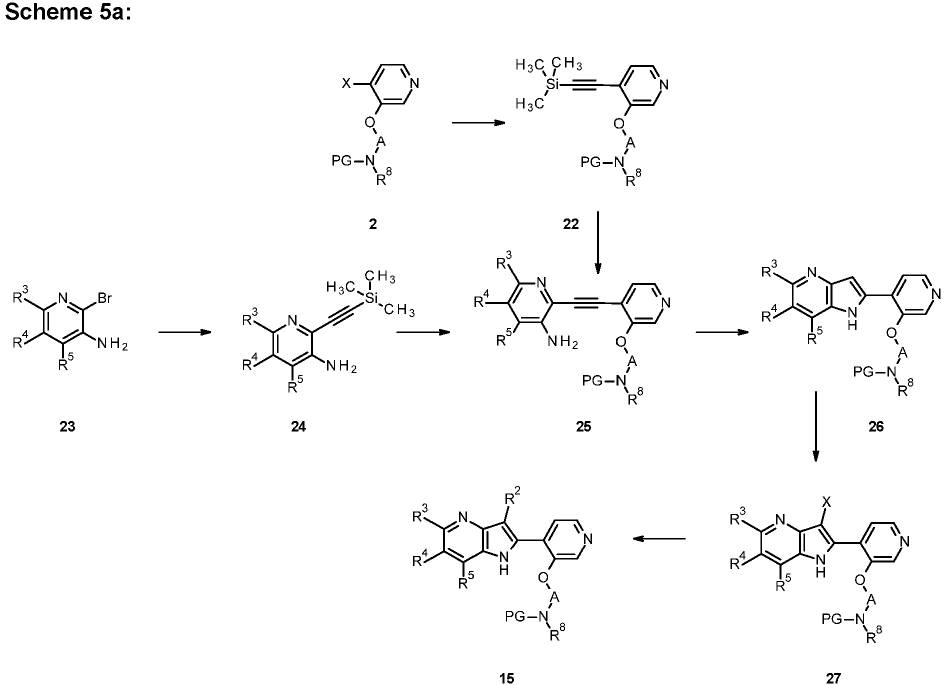

*Scheme* 5a: Route for the preparation of compounds of general formula **15,** wherein R², R³, R⁴, R⁵ and R⁸ have the meaning as given for general formula (I). A represents a group selected from the group: (C₂-C₆-alkanediyl) or CH₂-(C₁-C₅-haloalkanediyl) as described for the general formula (I). PG can be a protecting group, e.g. tert-butoxycarbonyl (Boc) or any other suitable protecting group as known to one skilled in the art (or as generally described in the chemical literature such as in *Greens Protecting Group in Organic Chemistry). X* represents a halogen such as Cl, Br or I. Compounds of the general formula **22** can be prepared by reacting compounds of the general formula **2** with ethynyl(trimethyl)silane in a Sonogashira type reaction, catalyzed by a mixture of a palladium (II) catalyst such as (but not limited to) dichlorobis(triphenylphosphine)palladium(II) and copper(I) iodide in polar aprotic solvents such as for example DMF under the influence of a base such as (but not limited to) triethylamine in a temperature range from 0°C up to the boiling point of the solvent used. In an analogous manner, compounds of the general formula **24** can be prepared from compounds of the general formula **23.** Compounds of the general formula **25** can be prepared by either reacting compounds of the general formula **22** with compounds of the general formula **23** or by reacting compounds of the general formula **24** with compounds of the general formula **2** in a reaction employing a mixture of a palladium (II) catalyst such as (but not limited to) dichlorobis(triphenylphosphine)palladium(II) and copper(I) iodide in polar aprotic solvents such as for example DMF under the influence of a base such as (but not limited to) triethylamine and a fluoride source such as (but not limited to) tetrabutylammonium fluoride in a temperature range from 0°C up to the boiling point of the solvent used. **25** can be converted to compounds of the general formula **26** in aprotic polar solvents such as (but not limited to) N-methylpyrollidine by employing a strong base such as potassium t-butoxide or other strong bases known to those skilled in the art in a temperature range from 0°C to 100°C. The transformation of **26** to compounds of the general formula **27** can be accomplished by reactions to introduce halogen known to a person skilled in the art. Introduction of Br may occur by using N-Bromsuccinimid (NBS) in solvents such as DMF or acetonitrile in a temperature range from -30°C to the boiling point of the respective solvent. Compounds of the general formula **27** can be converted to compounds of the general formula **15** by reacting **27** with suitable boronic acids in a Suzuki-type reaction, employing palladium catalysts such as bis(triphenylphosphine)palladium(II) dichloride (but not limited to) in the presence of a base for example potassium carbonate in solvents such as propanol, water and mixtures thereof, 1,4-dioxane or any other solvent known to those skilled in the art at temperatures ranging from 0°C to the boling point of the respective solvent.

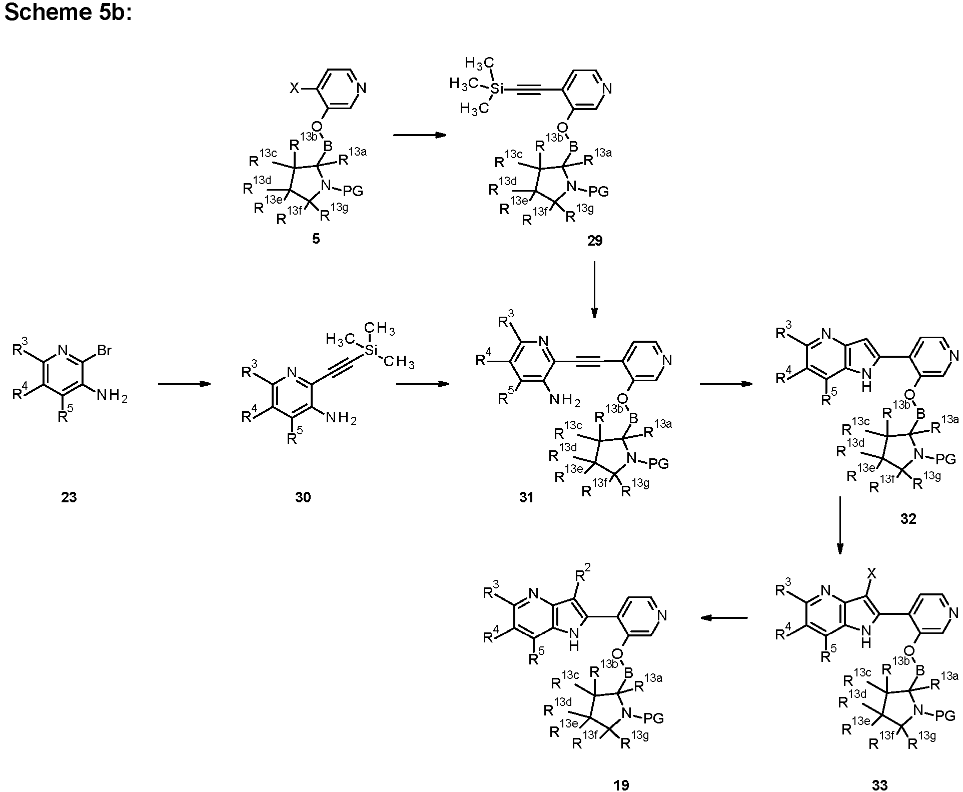

*Scheme 5b:* Route for the preparation of compounds of general formula **19,** wherein R², R³, R⁴, R⁵, R¹³, R^{13a}, R^{13b}, R^{13c}, R^{13d}, R^{13e}, R^{13f}, R^{13g} have the meaning as given for general formula (I). B represents a group (C₁-C₅-alkanediyl) as described for the general formula (I). Compound **5** stands exemplary for a group of 4- to 6- membered rings according to the description for R⁹ as given in the description of general formula (I). PG can be a protecting group, e.g. tert-butoxycarbonyl (Boc) or any other suitable protecting group as known to one skilled in the art (or as generally described in the chemical literature such as in *Greens Protecting Group in Organic Chemistry*)*.* X represents a halogen such as Cl, Br or I. Compound **5** stands exemplary for a group of 4- to 6- membered rings according to the description for R⁹ as given in the description of general formula (I). Compounds of the general formula **29** can be prepared by reacting compounds of the general formula **5** with ethynyl(trimethyl)silane in a Sonogashira type reaction, catalyzed by a mixture of a palladium (II) catalyst such as (but not limited to) dichlorobis(triphenylphosphine)palladium(II) and copper(I) iodide in polar aprotic solvents such as for example DMF under the influence of a base such as (but not limited to) triethylamine in a temperature range from 0°C up to the boiling point of the solvent used. In an analogous manner, compounds of the general formula **30** can be prepared from compounds of the general formula **23.** Compounds of the general formula **31** can be prepared by either reacting compounds of the general formula **29** with compounds of the general formula **23** or by reacting compounds of the general formula **30** with compounds of the general formula **5** in a reaction employing a mixture of a palladium (II) catalyst such as (but not limited to) dichlorobis(triphenylphosphine)palladium(II) and copper(I) iodide in polar aprotic solvents such as for example DMF under the influence of a base such as (but not limited to) triethylamine and a fluoride source such as (but not limited to) tetrabutylammonium fluoride in a temperature range from 0°C up to the boiling point of the solvent used. **31** can be converted to compounds of the general formula **32** in aprotic polar solvents such as (but not limited to) N-methylpyrollidine by employing a strong base such as potassium t-butoxide or other strong bases known to those skilled in the art in a temperature range from 0°C to 100°C. The transformation of **32** to compounds of the general formula **33** can be accomplished by reactions to introduce halogen known to a person skilled in the art. Introduction of Br may occur by using N-Bromsuccinimid (NBS) in solvents such as DMF or acetonitrile in a temperature range from -30°C to the boiling point of the respective solvent. Compounds of the general formula **33** can be converted to compounds of the general formula **19** by reacting **33** with suitable boronic acids in a Suzuki-type reaction, employing palladium catalysts such as bis(triphenylphosphine)palladium(II) dichloride (but not limited to) in the presence of a base for example potassium carbonate in solvents such as propanol, water and mixtures thereof, 1,4-dioxane or any other solvent known to those skilled in the art at temperatures ranging from 0°C to the boling point of the respective solvent.

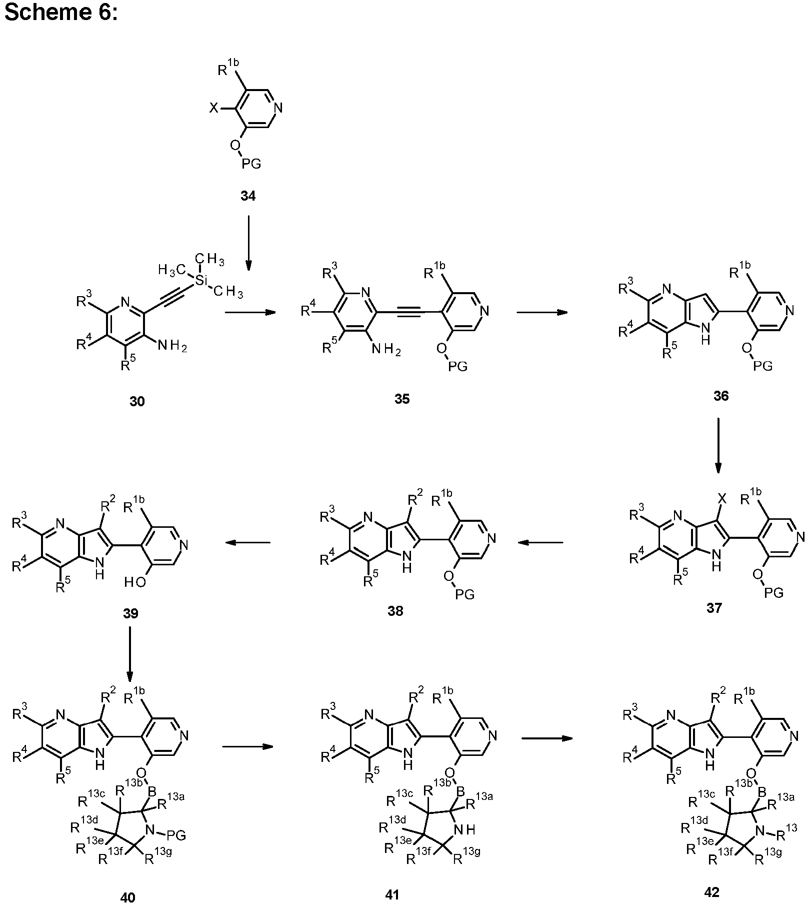

*Scheme 6:* Route for the preparation of compounds of general formula **42,** wherein R^{1a}, R^{1b}, R², R³, R⁴, R⁵, R¹³, R^{13a}, R^{13b}, R^{13c}, R^{13d}, R^{13e}, R^{13f}, R^{13g} have the meaning as given for general formula (I). B represents a group (C₁-C₅-alkanediyl) as described for the general formula (I). Compounds of type **30** and **34** (where PG may be, but not must be a suitable protecting group such as a methoxy ether), may be coupled together using a Sonogashira type reaction, (for instance but not limited to) a reaction catalyzed by Tetrakis(triphenylphosphin)palladium(0) and copper(I) iodide, in the presence of a suitable base such as triethylamine and reagent such as Tetra-n-butylammonium fluoride and a suitable solvent such as tetrahydrofuran, at a temperature of for instance of 70°C, as known to one skilled in the art, to produce formula **35.** Compounds of type **35,** may be cyclized to formula **36,** using for instance (but not limited to), trifluoracetic anhydride in the presence of triethylamine, in a suitable solvent such as dichloromethane. Halogenation, using for instance but not limited to N-Bromo succinimide, in a solvent such as dichloromethane, yield formula type **37,** which can be conveniently converted to formula **38** using, but not limited to, the so called Suzuki reaction, with a catalyst-ligand system such as XPhos Pd G2, with a base such as potassium phosphate and a suitable aryl boronic acid or ester, in a solvent system such as dioxane and water, as known to one skilled in the art. Deprotection, if required, of for instance a methoxy ether, using hydrobromic acid, yields formula **39,** which may be reacted, with for instance but not limited to, an alcohol in the presence of triphenyl phosphine and an azo compound and a suitable solvent such as THF (a so called Mitsunobu type reaction), to furnish formula type **40.** If required, deprotection may be performed of compounds of type **40,** of for instance a tert-butyloxycarbonyl group using for instance acidic conditions, with an acid such as hydrochloric acid in for instance a solvent such as dioxane to yield formula **41,** which in turn may be reacted with a suitable acid chloride or other suitable electrophile (also produced in situ by use of a so called coupling reagent such as T3P, in a solvent such as DMF, in the presence of a base such as DIPEA to produce formula type 42, depending on the nature of group R¹³, as known to one skilled in the art.

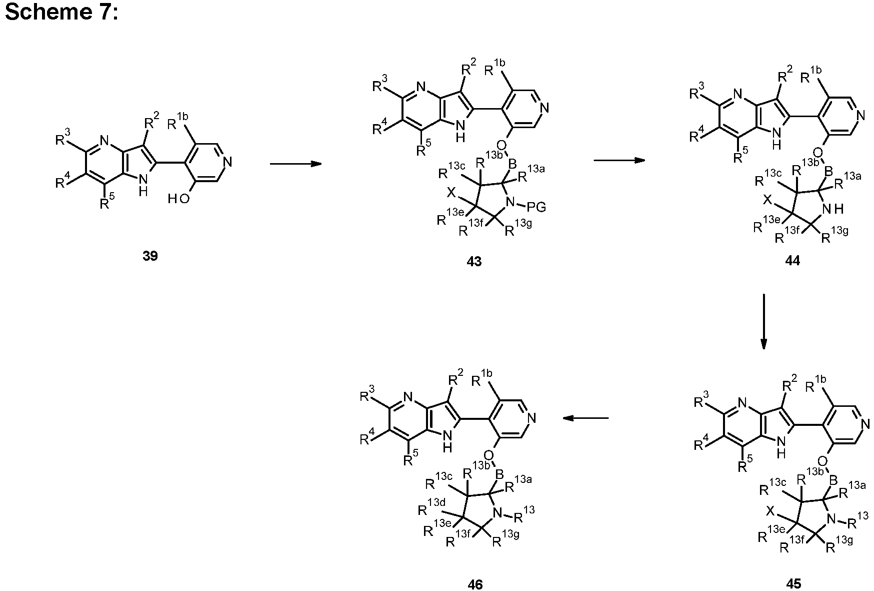

*Scheme* 7: Route for the preparation of compounds of general formula **46,** wherein R^{1a}, R^{1b}, R², R³, R⁴, R⁵, R¹³, R^{13a}, R^{13b}, R^{13c}, R^{13d}, R^{13e}, R^{13f}, R^{13g} have the meaning as given for general formula (I). B represents a group (C₁-C₅-alkanediyl) as described for the general formula (I). Formula type **39** (see Scheme 6), may be reacted with for instance but not limited to, an alcohol in the presence of triphenyl phosphine and an azo compound (such as DIAD) and a suitable solvent such as THF (so called Mitsunobu type reaction), to furnish formula type **43.** If required, deprotection of for instance a tert-butyloxycarbonyl group, using for example an acid such as HCl in a solvent such as dioxane can produce compounds of formula **44.** Compounds of the general formula **44** can be converted to compounds of the general formula **45** using various methods known to those skilled in the art. Depending on the nature of R¹³, corresponding acid chlorides, sulfonylchlorides or other electrophiles can be used under basic conditions in a suitable solvent. If carboxylic acids are employed the corresponding reaction can be facilitated by the use coupling reagents such as HATU, EDC -HOBt or T3P. These reactions can be performed in a temperature range from -30°C to the boiling point of the respective solvent. Formula **45,** may be converted to compounds of type **46,** by for instance but not limited to, reaction with a suitable nucleophile such as N,N,N'-Trimethylethylenediamine, in a solvent such as ethanol, at a temperature between 0°C and reflux of said solvent, depending on the nature of the substituents, as known to one skilled in the art.

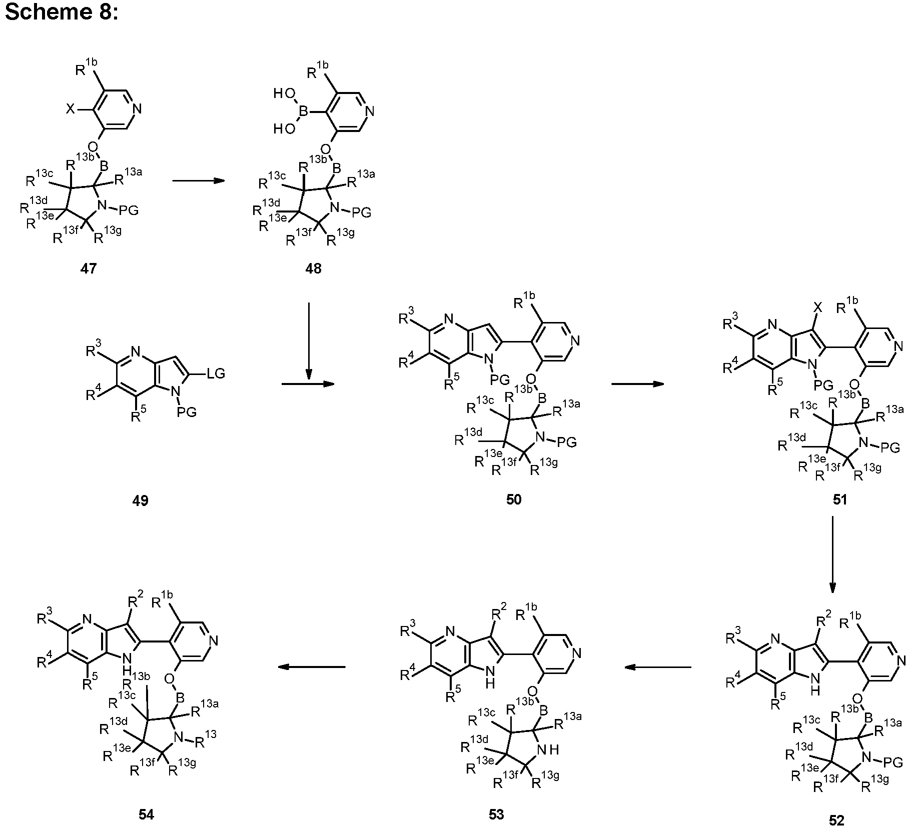

*Scheme* 8: Route for the preparation of compounds of general formula **54,** wherein R^{1a}, R^{1b}, R², R³, R⁴, R⁵, R¹³, R^{13a}, R^{13b}, R^{13c}, R^{13d}, R^{13e}, R^{13f}, R^{13g} have the meaning as given for general formula (I). B represents a group (C₁-C₅-alkanediyl) as described for the general formula (I). Formula **47,** may be converted to formula **48,** using for instance a suitable catalyst such as palladium dichloride, ligated by a ligand such as DPPF, in the presence of for instance potassium acetate, and for instance 4,4,4',4',5,5,5',5'-octamethyl-2,2'-bi-1,3,2-dioxaborolane in a solvent such as dioxane at for example a temperature of 90°C. Reaction of formula **48,** with type **49** (where a protecting group such as a tosyl group may be, but not necessarily must be employed), where LG may be an atom such as but not limited to iodide, in a so called Suzuki reaction, where for instance a catalyst such as PdCl2(dppf), in conjunction with a base such as sodium carbonate, in a solvent system such as a mixture of dioxane and water, at a temperature such as 80°C, may be used to produce compounds of formula **50.** Introduction of a halide, for instance by reaction with NBS in a suitable solvent such as but not limited to DMF, may be used to furnish formula type **51,** which may duly be converted to formula **52** by, for instance, a so called Suzuki reaction with a suitable boronic acid and catalyst such as PdCl2(dppf) and a base such as sodium carbonate, which can duly be deprotected if required by for instance cleavage of a tosyl group, with for example potassium carbonate in a solvent such as methanol at a temperature such as 50°C, to produce formula type **52.** Alternatively, as known to one skilled in the art, compounds of type **50,** may first be deprotected, if required, and then halogenated, and then subjected to a Suzuki type reaction, presenting an alternative sequence for production of formula type **52,** as desirable depending on the nature of the groups present in formula type **52.** If required, deprotection of **52,** when for instance the protecting group is tert-butyloxycarbonyl, may be accomplished using a variety of conditions, for example by reaction with hydrochloric acid in a solvent such as ethyl acetate producing formula **53.** Compounds of the general formula **53** can be converted to compounds of the general formula **54** using various methods known to those skilled in the art. Depending on the nature of R¹³, corresponding acid chlorides, sulfonylchlorides or other electrophiles can be used under basic conditions in a suitable solvent. If carboxylic acids are employed the corresponding reaction can be facilitated by the use coupling reagents such as HATU, EDC -HOBt or T3P.

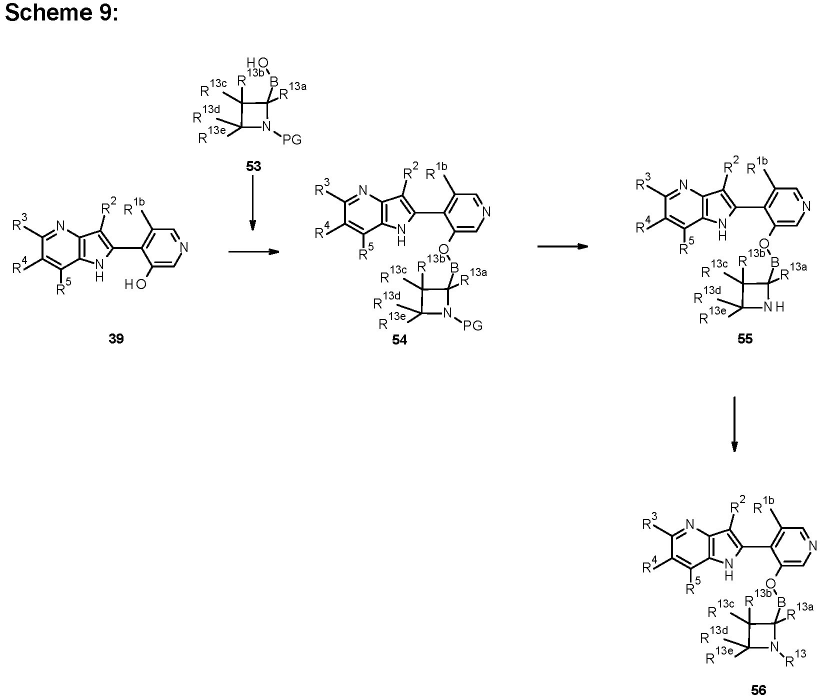

*Scheme 9:* Route for the preparation of compounds of general formula **56,** wherein R^{1a}, R^{1b}, R², R³, R⁴, R⁵, R¹³, R^{13a}, R^{13b}, R^{13c}, R^{13d}, R^{13e}, have the meaning as given for general formula (I). B represents a group (C₁-C₅-alkanediyl) as described for the general formula (I). Analogously to **Scheme 7,** reaction of formula **39** (as can be found in **Scheme 6**), with for instance, but not limited to, an alcohol of type **53** using so called Mitsonobu type conditions (an azo compound such as DIAD, triphenyl phosphine in a suitable solvent such as THF), can be used to produce formula **54.** If required, removal of a protecting group, such as for instance but not limited to benzyl, may be accomplished using a solvent system such as trifluoroethanol in the presence of a suitable catalyst such as palladium on carbon under an atmosphere of dihydrogen at a suitable pressure, for example, but not limited to 1 atm, producing formula **55.** Compounds of the general formula **55** can be converted to compounds of the general formula **56** using various methods known to those skilled in the art. Depending on the nature of R¹³, corresponding acid chlorides, sulfonylchlorides or other electrophiles can be used under basic conditions in a suitable solvent. If carboxylic acids are employed the corresponding reaction can be facilitated by the use coupling reagents such as HATU, EDC -HOBt or T3P.

It is known to the person skilled in the art that, if there are a number of reactive centers on a starting or intermediate compound, it may be necessary to block one or more reactive centers temporarily by protective groups in order to allow a reaction to proceed specifically at the desired reaction center.

The compounds according to the invention are isolated and purified in a manner known per se, *e.g.* by distilling off the solvent *in vacuo* and recrystallizing the residue obtained from a suitable solvent or subjecting it to one of the customary purification methods, such as chromatography on a suitable support material. Furthermore, reverse phase preparative HPLC may be applied. The compounds of the present invention which possess a sufficiently basic or acidic functionality, may result as a salt, such as, in the case of a compound of the present invention which is sufficiently basic, a trifluoroacetate or formate salt for example, or, in the case of a compound of the present invention which is sufficiently acidic, an ammonium salt for example. Salts of this type can either be transformed into its free base or free acid form, respectively, by various methods known to the person skilled in the art, or be used as salts in subsequent biological assays. Additionally, the drying process during the isolation of the compounds of the present invention may not fully remove traces of cosolvents, especially such as formic acid or trifluoroacetic acid, to give solvates or inclusion complexes. The person skilled in the art will recognise which solvates or inclusion complexes are acceptable to be used in subsequent biological assays. It is to be understood that the specific form (e.g. salt, free base, free acid, solvate, inclusion complex) of a compound of the present invention as isolated and described herein is not necessarily the only form in which said compound can be applied to a biological assay in order to quantify the specific biological activity.

Salts of the compounds of formula (I) according to the invention can be obtained by dissolving the free compound in a suitable solvent (for example a ketone such as acetone, methylethylketone or methylisobutylketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending on whether a mono- or polybasic acid or base is concerned and depending on which salt is desired, in an equimolar ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art. Certain salts are hydrochlorides and the process used in the example section.

Pure diastereomers and pure enantiomers of the compounds and salts according to the invention can be obtained e.g. by asymmetric synthesis, by using chiral starting compounds in synthesis or by splitting up enantiomeric and diasteriomeric mixtures obtained in synthesis.

Enantiomeric and diastereomeric mixtures can be split up into the pure enantiomers and pure diastereomers by methods known to the person skilled in the art. In one embodiment, diastereomeric mixtures are separated by crystallization, in particular fractional crystallization, or chromatography. Enantiomeric mixtures can be separated *e*.*g*. by forming diastereomers with a chiral auxiliary agent, resolving the diastereomers obtained and removing the chiral auxiliary agent. As chiral auxiliary agents, for example, chiral acids can be used to separate enantiomeric bases such as *e*.*g*. mandelic acid and chiral bases can be used to separate enantiomeric acids by formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

One aspect of the invention is the process for the preparation of the compounds of claims 1-4 according to the examples as well as the intermediates used for their preparation.

Optionally, compounds of the formula (I) can be converted into their salts, or, optionally, salts of the compounds of the formula (I) can be converted into the free compounds. Corresponding processes are customary for the skilled person.

### Commercial utility

As mentioned supra, the compounds of the present invention have surprisingly been found to effectively inhibit mutant EGFR in a cell (e.g., a cancer cell) contacted with the compound, thereby inducing cell death (*e*.*g*., apoptosis) and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by mutant EGFR, such as, for example, benign and malignant neoplasia, more specifically haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof, especially haematological tumours, solid tumours, and/or metastases of breast, bladder, bone, brain, central and peripheral nervous system, cervix, colon, endocrine glands (*e*.*g*., thyroid and adrenal cortex), endocrine tumours, endometrium, esophagus, gastrointestinal tumours, germ cells, kidney, liver, lung, larynx and hypopharynx, mesothelioma, ovary, pancreas, prostate, rectum, renal, small intestine, soft tissue, stomach, skin, testis, ureter, vagina and vulva as well as malignant neoplasias including primary tumours in said organs and corresponding secondary tumours in distant organs ("tumour metastases"). Haematological tumours can, e.g., be exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, and cancers of unknown primary site, as well as AIDS related malignancies.

A further aspect of the invention are the compounds according to formula (I) for use in the treatment of lung cancer, particularly lung cancer harboring mutant EGFR with exon 20 insertion mutations, more particularly lung cancer harboring V769_770ins ASV and/or D770_N771ins SVD exon 20 insertions, and/or metastases thereof, comprising administering an effective amount of a compound of formula (I).

A further aspect of the invention are the compounds according to formula (I) for use in the treatment of lung cancer, particularly lung cancer harboring a mutant EGFR with in-frame deletions in exon 19 (such as EGFR E746_A750del) or point mutations in exon 21 (e.g. L858R), and/or metastases thereof.

A further aspect of the invention are the compounds according to formula (I) for use in the treatment of lung cancer, particularly lung cancer harboring a mutant EGFR with a D770_N771insSVD C797S, E746_A750del C797S, or L858R C797S acquired resistance mutation, and/or metastases thereof.

A further aspect of the invention are the compounds according to formula (I) for use in the treatment of lung cancer, particularly lung cancer harboring a mutant ERBB2 with exon 20 insertion mutations (such as ERBB2 A775_G776insYVMA), and/or metastases thereof. In accordance with an aspect of the present invention therefore the invention relates to a compound of formula I, or an N-oxide, a salt, a tautomer or a stereoisomer of said compound, or a salt of said N-oxide, tautomer or stereoisomer particularly a pharmaceutically acceptable salt thereof, or a mixture of same, as described and defined herein, for use in the treatment or prophylaxis of a disease, especially for use in the treatment of a disease.

Another particular aspect of the present invention is therefore the compound of formula I, described *supra,* or a stereoisomer, a tautomer, an N-oxide, a hydrate, a solvate, or a salt thereof, particularly a pharmaceutically acceptable salt thereof, or a mixture of same, for use in the prophylaxis or treatment of hyperproliferative disorders or disorders responsive to induction of cell death, i.e., apoptosis.

By "hyperproliferative disease" is meant a disease, such as cancer, associated with inappropriately high levels of cell division, inappropriately low levels of apoptosis, or both. The term "inappropriate" within the context of the present invention, in particular in the context of "inappropriate cellular immune responses, or inappropriate cellular inflammatory responses", as used herein, is to be understood as generally meaning a response, which is less than, or greater than normal, and which is associated with, responsible for, or results in, the pathology of said diseases.

In particular embodiments, the compound for use is for the treatment or prophylaxis of diseases, especially the treatment, wherein the diseases are haematological tumours, solid tumours and/or metastases thereof.

Another aspect is the compound of formula (I) for use in the prophylaxis and/or treatment of lung cancer, particularly lung cancer harboring mutant EGFR with exon 20 insertion mutations, more particularly lung cancer harboring V769_770ins ASV and/or D770_N771ins SVD exon 20 insertions, and/or metastases thereof, especially for the treatment thereof.

Another aspect of the present invention is the compound of formula (I) or a stereoisomer, a tautomer, an N-oxide, a hydrate, a solvate, or a salt thereof, particularly a pharmaceutically acceptable salt thereof, or a mixture of same, as described herein, for use in the treatment or prophylaxis of a disease, wherein such disease is a hyperproliferative disorder or a disorder responsive to induction of cell death *e*.*g*., apoptosis. In an embodiment the disease is a haematological tumour, a solid tumour and/or metastases thereof. In another embodiment the disease is lung cancer, particularly lung cancer harboring mutant EGFR with exon 20 insertion mutations, more particularly lung cancer harboring V769_770ins ASV and/or D770_N771 ins SVD exon 20 insertions, and/or metastases thereof.

### Method of treating hyper-proliferative disorders

The present invention relates to the compounds of the present invention and compositions thereof, for use in the treatment of mammalian hyper-proliferative disorders. Compounds can be utilized to inhibit, block, reduce, decrease, etc., cell proliferation and/or cell division, and/or produce cell death *e*.*g*. apoptosis. This method comprises administering to a mammal in need thereof, including a human, an amount of a compound of this invention, or a pharmaceutically acceptable salt, polymorph, hydrate, solvate thereof; etc. which is effective to treat the disorder. Hyper-proliferative disorders include but are not limited, *e*.*g*., psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancer include, but are not limited to invasive ductal carcinoma, invasive lobular carcinoma, ductal carcinoma in situ, and lobular carcinoma *in situ.*

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypothalmic glioma, cerebellar and cerebral astrocytoma, medulloblastoma, ependymoma, as well as neuroectodermal and pineal tumour.

Tumours of the male reproductive organs include, but are not limited to prostate and testicular cancer. Tumours of the female reproductive organs include, but are not limited to endometrial, cervical, ovarian, vaginal, and vulvar cancer, as well as sarcoma of the uterus.

Tumours of the digestive tract include, but are not limited to anal, colon, colorectal, oesophageal, gallbladder, gastric, pancreatic, rectal, small-intestine, and salivary gland cancers.

Tumours of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, inverted sinonasal papilloma, inverted sinonasal papilloma-associated sinonasal squamous cell carcinoma, Merkel cell skin cancer, and non-melanoma skin cancer.

Head-and-neck cancers include, but are not limited to laryngeal, hypopharyngeal, nasopharyngeal, oropharyngeal cancer, inverted sinonasal papilloma, inverted sinonasal papilloma-associated sinonasal squamous cell carcinoma, lip and oral cavity cancer and squamous cell. Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, e.g., the management or care of a subject for the purpose of combating, alleviating, reducing, relieving, improving the condition of, *etc.,* of a disease or disorder, such as a carcinoma.

The present invention relates to compounds for use in a method of treating cancer in a subject, the method comprising administering to the subject an effective amount of a compound of formula (I) as defined herein.

The present invention relates to compounds for use in a method of treating cancer in a subject, wherein the cancer is or has acquired resistance to an anti-EGF receptor therapy, the method comprising administering to the subject an effective amount of a compound of formula (I) as defined herein.

The present invention relates to compounds for use in a method of enhancing the efficacy of an anti-EGF-receptor therapy, the method comprising administering to the subject an anti-EGF receptor therapy in combination with a a compound of formula (I) as defined herein.

In a further embodiment, the present invention relates to compounds for use in a method of treating cancer in a subject, wherein the cancer is selected from the group consisting of leukemia, myelodysplastic syndrome, malignant lymphoma, head and neck tumours, tumours of the thorax, gastrointestinal tumours, endocrine tumours, mammary and other gynaecological tumours, urological tumours, skin tumours, and sarcomas, the method comprising administering to the subject an effective amount of a compound of formula (I) as defined herein.

In a further embodiment, the present invention relates to compounds for use in a method of treating cancer in a subject, wherein the cancer is selected from the group consisting of inverted sinonasal papilloma or inverted sinonasal papilloma associated sinanonasal squamous cell carcinoma, the method comprising administering to the subject an effective amount of a compound of formula (I) as defined herein.

In a further embodiment, the present invention relates to compounds for use in a method of treating cancer in a subject, wherein the tumour of the thorax is non-small cell lung cancer, the method comprising administering to the subject an effective amount of a compound of formula (I) as defined herein.

In a further embodiment, the present invention relates to compounds for use in a method of treating cancer in a subject, wherein the cancer is lung cancer, particularly lung cancer harboring mutant EGFR with exon 20 insertion mutations, more particularly lung cancer harboring V769_770ins ASV and/or D770_N771ins SVD exon 20 insertions, and/or metastases thereof, comprising administering an effective amount of a compound of formula (I) as defined herein.

In a further embodiment, the present invention relates to a method of treating cancer in a subject, wherein the cancer is lung cancer, particularly lung cancer harboring a mutant EGFR with in-frame deletions in exon 19 (such as EGFR E746_A750del) or point mutations in exon 21 (e.g. L858R), and/or metastases thereof, the method comprising administering to the subject an effective amount of a compound of formula (I) as defined herein.

In a further embodiment, the present invention relates to compounds for use in a method of treating cancer in a subject, wherein the cancer is lung cancer, particularly lung cancer harboring a mutant EGFR with a D770_N771insSVD C797S, E746_A750del C797S, or L858R C797S acquired resistance mutation, and/or metastases thereof, the method comprising administering to the subject an effective amount of a compound of formula (I) as defined herein.

In a further embodiment, the present invention relates to compounds for use in a method of treating cancer in a subject, wherein the cancer is lung cancer, particularly lung cancer harboring a mutant ERBB2 with exon 20 insertion mutations (such as ERBB2 A775_G776insYVMA), and/or metastases thereof, the method comprising administering to the subject an effective amount of a compound of formula (I) as defined herein.

The present disclosure is also related to compounds for use in a method of selecting a patient for cancer treatment with a compound of formula (I) comprising detecting the presence of a mutation in exon 20 of the gene encoding the EGF-receptor in a biological sample of the subject, thereby determining that the patient should be treated with said compound. In some embodiments, the EGFR comprises aD770_N771insSVD C797S, E746_A750del C797S, or L858R C797S acquired resistance mutation, and/or metastases thereof. In some embodiments, the method of selecting a patient for cancer treatment with a compound of formula (I) may comprise detecting the presence of in-frame deletions in exon 19 or point mutations in exon 21 of the gene encoding EGF-receptor in a biological sample of the subject, thereby determining that the patient should be treated with said compound. For example, the in-frame deletion in exon 19 may be EGFR E746_A750del or the point mutation in exon 21 may be L858R. In some embodiments, the method of selecting a patient for cancer treatment with a compound of formula (I) may comprise detecting the presence of a mutation in exon 20 of the gene encoding ERBB2 in a biological sample of the subject, thereby determining that the patient should be treated with said compound. In some embodiments, the ERBB2 comprises an ERBB2 A775 or_G776insYVMA insertion mutation, and/or metastases thereof. Furthermore, methods of treating a patient with cancer may comprise administering to the subject a compound of formula (I) (*e*.*g*., in combination with anti-EGF receptor therapy), wherein the subject is selected for therapy by detecting the presence of a mutation in EGFR in a biological sample of the subject. In some embodiments, the method may comprise obtaining a biological sample from a subject and detecting a mutation in exon 19, 20, or 21 of the gene encoding EGF-receptor in the biological sample obtained from the subject. Detection of the presence of a mutation in exon 20 is within the skill of one of the art.

In embodiments, the disclosure provides compounds for use in a method of treating a selected subject, the method comprising administering to the selected subject a compound described herein, wherein the subject is selected by detecting a mutant EGFR comprising an in-frame deletion in exon 19 (e.g., EGFR E746_A750del) or a point mutations in exon 21 (e.g. L858R).

In some embodiments, the detection of a mutation (*e*.*g*., in an EGFR or a mutaton in exon 20 of the gene encoding EGFR) may be performed by sequencing (e.g., Sanger, Next Generation Sequencing) or a method selected from the group consisting of immunoblotting, mass spectrometry, immunoprecipitation quantitative PCR, Northern Blot, microarray, enzyme-linked immunosorbent assay (ELISA), in situ hybridization, and combinations thereof.

### Methods of treating kinase disorders

The present invention also provides compounds for use in methods for the treatment of disorders associated with aberrant mitogen extracellular kinase activity, including, but not limited to stroke, heart failure, hepatomegaly, cardiomegaly, diabetes, Alzheimer's disease, cystic fibrosis, symptoms of xenograft rejections, septic shock or asthma.

Effective amounts of compounds of the present invention can be used to treat such disorders, including those diseases (e.g., cancer) mentioned in the Background section above. Nonetheless, such cancers and other diseases can be treated with compounds of the present invention, regardless of the mechanism of action and/or the relationship between the kinase and the disorder.

The phrase "aberrant kinase activity" or "aberrant tyrosine kinase activity," includes any abnormal expression or activity of the gene encoding the kinase or of the polypeptide it encodes. Examples of such aberrant activity, include, but are not limited to, over-expression of the gene or polypeptide; gene amplification; mutations which produce constitutively-active or hyperactive kinase activity; gene mutations, deletions, substitutions, additions, etc.

The present invention also provides compounds for use in methods of inhibiting kinase activity, especially of mitogen extracellular kinase, comprising administering an effective amount of a compound of the present invention, including salts, polymorphs, hydrates, solvates thereof, and diastereoisomeric forms thereof. Kinase activity can be inhibited in cells *(e.g., in vitro),* or in the cells of a mammalian subject, especially a human patient in need of treatment.

### Methods of treating angiogenic disorders

The present invention also provides compounds for use in methods of treating disorders and diseases associated with excessive and/or abnormal angiogenesis.

Inappropriate and ectopic expression of angiogenesis can be deleterious to an organism. A number of pathological conditions are associated with the growth of extraneous blood vessels. These include, e.g., diabetic retinopathy, ischemic retinal-vein occlusion, and retinopathy of prematurity [Aiello et al. New Engl. J. Med. 1994, 331, 1480; Peer et al. Lab. Invest. 1995, 72, 638], age-related macular degeneration [AMD; see, Lopez et al. Invest. Opththalmol. Vis. Sci. 1996, 37, 855], neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of the present invention can be utilized to treat and/or prevent any of the aforementioned angiogenesis disorders, e.g., by inhibiting and/or reducing blood vessel formation; by inhibiting, blocking, reducing, decreasing, etc. endothelial cell proliferation or other types involved in angiogenesis, as well as causing cell death e.g. apoptosis of such cell types.

In various embodiments, the diseases of said method are haematological tumours, solid tumour and/or metastases thereof.

The compounds of the present invention can be used in particular in therapy and prevention i.e. prophylaxis, especially in therapy of tumour growth and metastases, especially in solid tumours of all indications and stages with or without pre-treatment of the tumour growth.

### Pharmaceutical compositions of the compounds of the invention

This invention also relates to pharmaceutical compositions containing one or more compounds of the present invention. These compositions can be utilised to achieve the desired pharmacological effect by administration to a patient in need thereof. A patient, for the purpose of this invention, is a mammal, including a human, in need of treatment for the particular condition, disorder, or disease.

Therefore, the present invention includes pharmaceutical compositions that are comprised of a pharmaceutically acceptable carrier or auxiliary and a pharmaceutically effective amount of a compound, or salt thereof, of the present invention.

Another aspect of the invention is a pharmaceutical composition comprising a pharmaceutically effective amount of a compound of formula (I) and a pharmaceutically acceptable auxiliary for the treatment of a disease mentioned supra, especially for the treatment of haematological tumours, solid tumours and/or metastases thereof.

A pharmaceutically acceptable carrier or auxiliary may be a carrier that is non-toxic and innocuous to a patient at concentrations consistent with effective activity of the active ingredient so that any side effects ascribable to the carrier do not vitiate the beneficial effects of the active ingredient. Carriers and auxiliaries are all kinds of additives assisting to the composition to be suitable for administration.

A pharmaceutically effective amount of compound may be that amount which produces a result or exerts the intended influence on the particular condition being treated.

The compounds of the present invention can be administered with pharmaceutically-acceptable carriers or auxiliaries well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatine type containing auxiliaries, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatine, disintegrating agents intended to assist the break-up and dissolution of the tablet following administration, such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, colouring agents, and flavouring agents such as peppermint, oil of wintergreen, or cherry flavouring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavouring and colouring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavouring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more colouring agents; one or more flavouring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavouring and colouring agents.

The compounds of this invention may also be administered parenterally, that is, subcutaneously, intravenously, intraocularly, intrasynovially, intramuscularly, or interperitoneally, as injectable dosages of the compound in, for example, a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimise or eliminate irritation at the site of injection, such compositions may contain a non-ionic surfactant having a hydrophile-lipophile balance (HLB) in one embodiment of from about 12 to about 17. The quantity of surfactant in such formulation in one embodiment ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for administration, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized.

Such ingredients and procedures include those described in the following references, each of which is incorporated herein by reference: Powell, M.F. et al., "Compendium of Excipients for Parenteral Formulations" PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:
acidifying agents (examples include but are not limited to acetic acid, citric acid, fumaric acid, hydrochloric acid, nitric acid);
alkalinizing agents (examples include but are not limited to ammonia solution, ammonium carbonate, diethanolamine, monoethanolamine, potassium hydroxide, sodium borate,
sodium carbonate, sodium hydroxide, triethanolamine, trolamine);
adsorbents (examples include but are not limited to powdered cellulose and activated charcoal);
aerosol propellants (examples include but are not limited to carbon dioxide, CCl₂F₂, F₂ClC-CClF₂ and CClF₃);
air displacement agents (examples include but are not limited to nitrogen and argon);
antifungal preservatives (examples include but are not limited to benzoic acid, butylparaben, ethylparaben, methylparaben, propylparaben, sodium benzoate);
antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
binding materials (examples include but are not limited to block polymers, natural and synthetic rubber, polyacrylates, polyurethanes, silicones, polysiloxanes and styrenebutadiene copolymers);
buffering agents (examples include but are not limited to potassium metaphosphate, dipotassium phosphate, sodium acetate, sodium citrate anhydrous and sodium citrate dihydrate);
carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection);
chelating agents (examples include but are not limited to edetate disodium and edetic acid); colourants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
clarifying agents (examples include but are not limited to bentonite);
emulsifying agents (examples include but are not limited to acacia, cetomacrogol, cetyl alcohol, glyceryl monostearate, lecithin, sorbitan monooleate, polyoxyethylene 50 monostearate);
encapsulating agents (examples include but are not limited to gelatin and cellulose acetate phthalate);
flavourants (examples include but are not limited to anise oil, cinnamon oil, cocoa, menthol, orange oil, peppermint oil and vanillin);
humectants (examples include but are not limited to glycerol, propylene glycol and sorbitol); levigating agents (examples include but are not limited to mineral oil and glycerin);
oils (examples include but are not limited to arachis oil, mineral oil, olive oil, peanut oil, sesame oil and vegetable oil);
ointment bases (examples include but are not limited to lanolin, hydrophilic ointment, polyethylene glycol ointment, petrolatum, hydrophilic petrolatum, white ointment, yellow ointment, and rose water ointment);
penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas);
plasticizers (examples include but are not limited to diethyl phthalate and glycerol);
solvents (examples include but are not limited to ethanol, corn oil, cottonseed oil, glycerol, isopropanol, mineral oil, oleic acid, peanut oil, purified water, water for injection, sterile water for injection and sterile water for irrigation);
stiffening agents (examples include but are not limited to cetyl alcohol, cetyl esters wax, microcrystalline wax, paraffin, stearyl alcohol, white wax and yellow wax);
suppository bases (examples include but are not limited to cocoa butter and polyethylene glycols (mixtures));
surfactants (examples include but are not limited to benzalkonium chloride, nonoxynol 10, oxtoxynol 9, polysorbate 80, sodium lauryl sulfate and sorbitan mono-palmitate);
suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
sweetening agents (examples include but are not limited to aspartame, dextrose, glycerol, mannitol, propylene glycol, saccharin sodium, sorbitol and sucrose);
tablet anti-adherents (examples include but are not limited to magnesium stearate and talc);
tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
tablet direct compression excipients (examples include but are not limited to dibasic calcium phosphate);
tablet disintegrants (examples include but are not limited to alginic acid, carboxymethylcellulose calcium, microcrystalline cellulose, polacrillin potassium, crosslinked polyvinylpyrrolidone, sodium alginate, sodium starch glycollate and starch);
tablet glidants (examples include but are not limited to colloidal silica, corn starch and talc);
tablet lubricants (examples include but are not limited to calcium stearate, magnesium stearate, mineral oil, stearic acid and zinc stearate);
tablet/capsule opaquants (examples include but are not limited to titanium dioxide);
tablet polishing agents (examples include but are not limited to carnuba wax and white wax);
thickening agents (examples include but are not limited to beeswax, cetyl alcohol and paraffin);
tonicity agents (examples include but are not limited to dextrose and sodium chloride);
viscosity increasing agents (examples include but are not limited to alginic acid, bentonite, carbomers, carboxymethylcellulose sodium, methylcellulose, polyvinyl pyrrolidone, sodium alginate and tragacanth); and
wetting agents (examples include but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:
Sterile i.v. solution: A 5 mg/ml solution of the desired compound of this invention can be made using sterile, injectable water, and the pH is adjusted if necessary. The solution is diluted for administration to 1 - 2 mg/ml with sterile 5% dextrose and is administered as an i.v. infusion over about 60 minutes.
Lyophilised powder for i.v. administration: A sterile preparation can be prepared with (i) 100 - 1000 mg of the desired compound of this invention as a lyophilised powder, (ii) 32- 327 mg/ml sodium citrate, and (iii) 300 - 3000 mg Dextran 40. The formulation is reconstituted with sterile, injectable saline or dextrose 5% to a concentration of 10 to 20 mg/ml, which is further diluted with saline or dextrose 5% to 0.2 - 0.4 mg/ml, and is administered either IV bolus or by IV infusion over 15 - 60 minutes.
Intramuscular suspension: The following solution or suspension can be prepared, for intramuscular injection:
   50 mg/ml of the desired, water-insoluble compound of this invention
   5 mg/ml sodium carboxymethylcellulose
   4 mg/ml TWEEN 80
   9 mg/ml sodium chloride
   9 mg/ml benzyl alcohol
Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.
Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.
Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.
Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

### Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and in particular embodiments from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will in other embodiments be from 0.01 to 200 mg/kg of total body weight. The average daily rectal dosage regimen will in particular embodiments be from 0.01 to 200 mg/kg of total body weight. The average daily vaginal dosage regimen will in other embodiments be from 0.01 to 200 mg/kg of total body weight. The average daily topical dosage regimen will in still other embodiments be from 0.1 to 200 mg administered between one to four times daily. The transdermal concentration will in other embodiments be that required to maintain a daily dose of from 0.01 to 200 mg/kg. The average daily inhalation dosage regimen will in other embodiments be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### Combination Therapies

The compounds of this invention can be administered as the sole pharmaceutical agent or in combination with one or more other pharmaceutical agents where the combination causes no unacceptable adverse effects. Those combined pharmaceutical agents can be other agents having antiproliferative effects such as for example for the treatment of haematological tumours, solid tumours and/or metastases thereof and/or agents for the treatment of undesired side effects. The present invention relates also to such combinations.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), especially (chemotherapeutic) anticancer agents as defined supra. The combination can be a non-fixed combination or a fixed-dose combination as the case may be.

Methods of testing for a particular pharmacological or pharmaceutical property are well known to persons skilled in the art.

The example testing experiments described herein serve to illustrate the present invention and the invention is not limited to the examples given.

The following examples illustrate the invention in greater detail, without restricting it. Further compounds according to the invention, of which the preparation is not explicitly described, can be prepared in an analogous way.

The compounds, which are mentioned in the examples and the salts thereof represent specific embodiments of the invention as well as a claim covering all subcombinations of the residues of the compound of formula (I) as disclosed by the specific examples.

The term "according to" within the experimental section is used in the sense that the procedure referred to is to be used "analogously to".

### EXPERIMENTAL SECTION

Chemical names were generated using the ACD/Name software from ACD/Labs. In some cases, generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1: Abbreviations**

| **Abbreviation** | **Meaning** |
|---|---|
| AcOH | acetic acid |
| aq. | Aqueous |
| br | broad signal (NMR) |
| d | doublet (NMR) |
| DAD | diode array detector |
| DCM | Dichloromethane |
| dd | doublet of doublet (NMR) |
| DMF | N,N-dimethylformamide |
| DMSO | Dimethylsulfoxide |
| EDC.HCl | N-(3-Dimethylaminopropyl)-N'-ethylcarbodiimide hydrochloride salt |
| ESI | electrospray (ES) ionization |
| EtOAc | ethyl acetate |
| EtOH | Ethanol |
| h, hr (hrs) | hour(s) |
| HATU | (1-[Bis(dimethylamino)methylene]-1H-1,2,3-triazolo[4,5-b]pyridinium 3-oxide hexafluorophosphate |
| HCl | hydrogen chloride, hydrochloric acid |
| HPLC | high performance liquid chromatography |
| LC-MS | liquid chromatography-mass spectrometry |
| m | multiplet (NMR) |
| MeCN | Acetonitrile |
| MeOH | Methanol |
| min | minute(s) |
| MS | mass spectrometry |
| NaH | sodium hydride |
| NMP | N-methylpyrollidone |
| NMR | nuclear magnetic resonance spectroscopy: chemical shifts (δ) are given in ppm. The chemical shifts were corrected by setting the DMSO signal to 2.50 ppm using unless otherwise stated. |
| q | quartet (NMR) |
| Rt or rt | room temperature |
| Rₜ | retention time |
| s | singulet (NMR) |
| sat. | Saturated |
| t | triplet (NMR) |
| td | triplet of doublet (NMR) |
| TBAF | Tetrabutylammoniumfluorid |
| TEA | Triethylamine |
| TFA | trifluoroacetic acid |
| THF | Tetrahydrofuran |
| T3P | propanephosphonic acid anhydride |
| δ | chemical shift |

Other abbreviations have their meanings customary *per se* to the skilled person.

The various aspects of the invention described in this application are illustrated by the following examples which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, e.g. Biotage SNAP cartridges KP-Sil^{®} or KP-NH^{®} in combination with a Biotage autopurifier system (SP4^{®} or Isolera Four^{®}) and eluents such as gradients of hexane/ethyl acetate or DCM/methanol. In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### Analytical LC-MS methods:

### Method 1:

System MS: Thermo Scientific FT-MS; System UHPLC+: Thermo Scientific UltiMate 3000; Column: Waters, HSST3, 2.1 x 75 mm, C18 1.8 µm; Eluent A: 1 l Water + 0.01% Formic acid; Eluent B: 1 l Acetonitrile + 0.01% Formic acid; Gradient: 0.0 min 10% B → 2.5 min 95% B → 3.5 min 95% B; Oven: 50°C; Flow: 0.90 ml/min; UV-Detection: 210 nm/ Optimum Integration Path 210-300 nm

### Method 2:

System MS: Thermo Scientific FT-MS; System UHPLC+: Thermo Scientific Vanquish; Column: Waters, HSST3, 2.1 x 75 mm, C18 1.8 µm; eluent A: 1 l water + 0.01% formic acid; Eluent B: 1 l acetonitrile + 0.01% formic acid; gradient: 0.0 min 10% B → 2.5 min 95% B → 3.5 min 95% B; Oven: 50°C; Flow: 0.90 ml/min; UV-Detection: 210 nm

### Method 9:

System MS: Thermo Scientific FT-MS; System UHPLC+: Thermo Scientific UltiMate 3000; Column: Waters, BEH c18 1.7µ, 2.1 x 50 mm, Eluent A: 1 l Water + 1.0 mL (25% Ammonia); Eluent B: 1 l Acetonitrile; Gradient: 0.0 min 5% B → 2.5 min 95% B → 3.5 min 95% B; Oven: 50°C; Flow: 0.90 ml/min; UV-Detection: 210 nm/ Optimum Integration Path 210-300 nm

### Method 10:

System MS: Waters TOF instrument; System UPLC: Waters Acquity I-CLASS; Column: Waters, HSST3, 2.1 x 50 mm, C18 1.8 µm; Eluent A: 1 l Water + 0.01% Formic acid; Eluent B: 1 I Acetonitrile + 0.01% Formic acid; Gradient: 0.0 min 2% B → 0.5 min 2% B → 7.5 min 95% B → 10.0 min 95% B; Oven: 50°C; Flow: 1.00 ml/min; UV-Detection: 210 nm

### Method A:0-60AB, Shimadzu

Instrument: SHIMADZU LCMS-2020 SingleQuad; Column: Chromolith@Flash RP-18E 25-2 MM; eluent A: water + 0.0375 vol% trifluoroacetic acid, eluent B: acetonitrile + 0.01875 vol% trifluoroacetic acid; gradient: 0-0.8 min 0-60% B, 0.8-1.2 min 60% B; flow 1.5 ml/min; temperature: 50 °C; PDA: 220 nm & 254 nm.

### Method C:5-95AB, Shimadzu

Instrument: SHIMADZU LCMS-2020 SingleQuad; Column: Chromolith@Flash RP-18E 25-2 MM; eluent A: water + 0.0375 vol% trifluoroacetic acid, eluent B: acetonitrile + 0.01875 vol% trifluoroacetic acid; gradient: 0-0.8 min, 5-95% B, 0.8-1.2 min 95% B; flow 1.5 ml/min; temperature: 50 °C; PDA: 220 nm & 254 nm.

### Method G:5-95CD, Shimadzu

Instrument: SHIMADZU LCMS-2020 SingleQuad; Column: Kinetex EVO C18 2.1*30 mm, 5 µm; eluent A: water + 0.025 vol% ammonium hydroxide, eluent B: acetonitrile; gradient: 0-0.8 min, 5-95% B, 0.8-1.2 min 95% B; flow 1.5 ml/min; temperature: 40 °C; PDA: 220 nm & 254 nm.

### Preparative LC-MS methods:

### Method 3:

Instrument: Knauer P2.1L, Knauer UV detector Azura UVD 2.1S, Prepcon 5 software. Column: Chromatorex C18 10 µm, 125 mm x 30 mm; eluent A: water, eluent B: acetonitrile; gradient: 0-5 min 20% B; 5-18 min 20%-90% B, 18-25 min 90% B; column temperature: rt; flow rate: 50 mL/min; UV detection: 210 nm.

### Method 4:

Instrument: Agilent 1260 with fraction collector. Column: Phenomenex Luna C18(2), 5 µm, 100 mm x 21.2 mm; Eluent A: water +0,1% formic acid, Eluent B: acetonitrile; gradient: 0-25 min 5% B; 25-27 min 95% B, column temperature: rt; flow rate: 25 mL/min; UV detection: 210 nm.

### Method 5:

Instrument: Agilent 1260 with fraction collector. Column: Phenomenex Luna C18(2), 5 µm, 100 mm x 21.2 mm; Eluent A: water +0,1% formic acid, Eluent B: acetonitrile; gradient: 0-12 min 5% B; 12-15 min 40 % B; 15-17 min 95% B, column temperature: rt; flow rate: 25 mL/min; UV detection: 210 nm.

### Method 6:

Instrument: Waters Prep LC/MS System. Column: Phenomenex Kinetex C18, 5 µm, 100 mm x 30 mm; Eluent A: water, Eluent B: acetonitrile; Eluent C: water +2% formic acid; Eluent D: acetonitrile/water 80Vol:20Vol%; column temperature: rt; flow rate: 80 mL/min; UV detection: 200-400 nm. Gradientprofile: 0 - 2 min: A 63 ml, B 7 ml; 2 -10 min: A 63 ml - > 39 ml, B 7ml - 31 ml; 10 - 12 min: B 70 ml Eluent B, C and D constant flow of 5 ml/min each.

### Method 7:

Instrument: Waters Prep LC/MS System. Column: Phenomenex Kinetex C18, 5 µm, 100 mm x 30 mm; Eluent A: water, Eluent B: acetonitrile; Eluent C: water +2% formic acid; Eluent D: acetonitrile/water 80Vol:20Vol%; column temperature: rt; flow rate: 80 mL/min; UV detection: 200-400 nm. Gradientprofile: 0 - 2 min: A 70 ml; 2 -10 min: A 70 ml -> 55 ml, B 0 ml - 15 ml; 10 - 12 min: B 70 ml Eluent B, C and D constant flow of 5 ml/min each.

### Method 8:

Instrument: Waters Prep LC/MS System, Column: XBridge C18 5µm 100x30 mm, eluent A: water, eluent B : acetonitrile, eluent C : 2% ammonia in water, eluent D : acetonitrile/water ( 80vol.%/20vol%), flowrate: 80 ml/min , temperature: rt, UV detection: 210 nm, gradient profile: A 0 - 2 min 55 ml, B 0 - 2min 15 ml, A 2 - 10 min from 55 ml to 31 ml and B from 15 ml to39 ml, 10 - 12 min 0 ml A and 70 ml B. C and D constant flow of 5 ml/min each.

### NMR Spectra:

The multiplicities of proton signals in ¹H NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - e.g. caused by rapid rotation of molecular moieties or by interchanging protons - have also been assigned tentatively (often referred to as a broad multiplet or broad singlet) or are not shown.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ, (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

### Syntheses of Intermediate Compounds

### Intermediate 1

### 3-(4-fluorophenyl)-1H-pyrrolo[3,2-b]pyridine

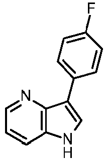

A suspension of 3-bromo-1H-pyrrolo[3,2-b]pyridine (500 mg, 2.54 mmol) in dimethoxyethane (10 ml) was carefully degassed and purged with argon. The palladium catalyst 1-1'-bis(diphenylphosphino)ferrocenepalladium(ll)chloride (186 mg, 254 µmol; CAS-RN:[72287-26-4]), (4-fluorophenyl)boronic acid (1.07 g, 7.61 mmol), potassium carbonate (1.75 g, 12.7 mmol) and water (4 ml) were added. The mixture was heated to 100°C and stirred at this temperature for 8 h. After cooling to rt, water (5 ml) was added and the the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) to give the desired product with insufficient purity. The material was subjected to reverse phase preparative HPLC (method 3) yielding 179 mg (99 % purity, 33 % yield) of the desired product.
LC-MS (method 1): Rₜ = 0.73 min; MS (ESlpos): m/z = 213 [M+H]⁺

### Intermediate 1

### tert-butyl 3-(4-fluorophenyl)-1H-pyrrolo[3,2-b]pyridine-1-carboxylate

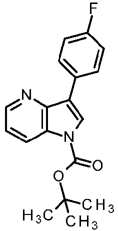

To a solution of 3-(4-fluorophenyl)-1H-pyrrolo[3,2-b]pyridine (500 mg, 2.36 mmol) in DCM (10 ml), triethylamine (660 µl, 4.7 mmol) and 4-dimethylaminopyridine (28.8 mg, 236 µmol) were added at rt. di-tert-butyl dicarbonate (617 mg, 2.83 mmol) was added and stirring at rt was continued for 2 h. Water (5 ml) was added and the mixture was extracted with DCM twice. The combined organic layers were dried over sodium sulfate, filtered and concentrated under reduced pressure. The residue was purified by reverse phase preparative HPLC (method 3) yielding 720 mg (100 % purity, 98 % yield) of the desired product.
LC-MS (method 1): Rₜ = 2.50 min; MS (ESlpos): m/z = 313 [M+H]⁺

### Intermediate 2

### tert-butyl {2-[(4-bromopyridin-3-yl)oxy]ethyl}carbamate

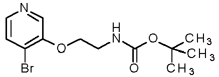

To a solution of triphenylphosphin (678 mg, 2.59 mmol]) in THF (6 ml), diethyl (E)-diazene-1,2-dicarboxylate (1.0 ml, 40 % purity in toluene, 2.6 mmol) was added at rt. After 30 min, a solution of tert-butyl (2-hydroxyethyl)carbamate (334 mg, 2.07 mmol) in THF (2 ml) was added followed by addition of 4-bromopyridin-3-ol (300 mg, 1.72 mmol). The mixture was stirred at 40°C for 16 h. Water (3 ml) was added and the mixture was extracted with EtOAc three times. The combined organic layers were washed with saturated sodium chloride solution, dried over sodium sulfate, filtered and concentrated under reduced pressure. The residue was purified by reverse phase preparative HPLC (method 3) yielding the desired product containing triphenylphosphine oxide as impurity. Thus, the material was further purified by silica gel column chromatography (eluent: cyclohexane/EtOAc 1:1) yielding 318 mg (97 % purity, 56 % yield) of the title compound.
LC-MS (method 1): Rₜ = 1.57 min; MS(ESlpos): m/z = 317 [M+H]⁺

### Intermediate 3

### (3-{2-[(tert-butoxycarbonyl)amino]ethoxy}pyridin-4-yl)boronic acid

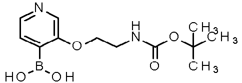

A suspension of tert-butyl {2-[(4-bromopyridin-3-yl)oxy]ethyl}carbamate (300 mg, 946 µmol), 4,4,4',4',5,5,5',5'-octamethyl-2,2'-bi-1,3,2-dioxaborolane (368 mg, 98 % purity, 1.42 mmol) and potassium acetate (278 mg, 2.84 mmol) in 3 ml 1,4-1,4-dioxane was carefully degassed and purged with argon. At rt, 1-1'-bis(diphenylphosphino)ferrocenepalladium(II)chloride (dichloromethane adduct) (77.2 mg, 94.6 µmol) was added and the mixture was heated to reflux. After 6 h at 100°C, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 94 mg (98 % purity, 35 % yield) of the title compound.
LC-MS (method 1): Rₜ = 0.76 min; MS(ESlpos): m/z = 293 [M+H]⁺

### Intermediate 4

### tert-butyl 2-bromo-3-(4-fluorophenyl)-1H-pyrrolo[3,2-b]pyridine-1-carboxylate

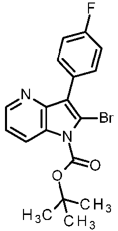

To a solution of tert-butyl 3-(4-fluorophenyl)-1H-pyrrolo[3,2-b]pyridine-1-carboxylate (405 mg, 1.30 mmol) in 1,2-dichloroethane (5 ml), 1-bromopyrrolidine-2,5-dione (231 mg, 1.30 mmol) was added at 0°C. Stirring at this temperature was continued for 30 min. Saturated sodium hydrogencarbonate solution (aqueous, 1 mL) was added and the mixture was dried over a water repellent filter. After concentration under reduced pressure the residue was purified by reverse phase preparative HPLC (method 3) yielding 471 mg (98 % purity, 91 % yield) of the title compound.
LC-MS (method 1): Rₜ = 2.49 min; MS(ESlpos): m/z = 391 [M+H]⁺

### Intermediate 5

### tert-butyl 2-(3-{2-[(tert-butoxycarbonyl)amino]ethoxy}pyridin-4-yl)-3-(4-fluorophenyl)-1H-pyrrolo[3,2-b]pyridine-1-carboxylate

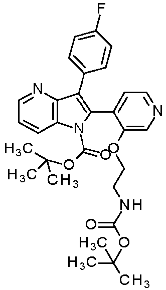

tert-butyl-2-bromo-3-(4-fluorophenyl)-1H-pyrrolo[3,2-b]pyridine-1-carboxylate (117 mg, 299 µmol) and (3-{2-[(tert-butoxycarbonyl)amino]ethoxy}pyridin-4-yl)boronic acid (92.8 mg, 329 µmol) were dissolved in a mixture of 1-propanole and water (5:1, 2 ml). The solution was carefully degassed and purged with argon. Subsequently, triphenylphosphine (7.84 mg, 29.9 µmol), potassium carbonate (124 mg, 897 µmol) and bis(triphenylphosphine)palladium(ll) dichloride (21.0 mg, 29.9 µmol; CAS-RN:[13965-03-2]) were added at rt. The reaction mixture was stirred at 100°C for 12.5 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 17.5 mg (100 % purity, 11 % yield) of the title compound.
LC-MS (method 1): Rₜ = 2.22 min; MS (ESlpos): m/z = 549 [M+H]⁺
¹H-NMR (600 MHz, DMSO-d6) δ [ppm]: 1.257 (16.00), 1.263 (15.20), 3.148 (0.66), 3.158 (0.65), 4.075 (0.55), 4.085 (1.06), 4.095 (0.53), 6.742 (0.40), 7.116 (0.73), 7.131 (1.52), 7.145 (0.84), 7.162 (0.57), 7.169 (0.60), 7.351 (0.63), 7.360 (0.77), 7.365 (0.74), 7.374 (0.55), 7.434 (0.54), 7.442 (0.55), 7.448 (0.56), 7.456 (0.55), 8.180 (0.67), 8.187 (0.68), 8.468 (0.72), 8.482 (0.69), 8.495 (1.74), 8.546 (0.69), 8.548 (0.70), 8.554 (0.69), 8.556 (0.66).

### Intermediate 6

### 2-({4-[3-(4-fluorophenyl)-1H-pyrrolo[3,2-b]pyridin-2-yl]pyridin-3-yl}oxy)ethan-1-amine

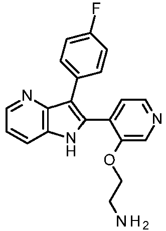

To a solution of tert-butyl 2-(3-{2-[(tert-butoxycarbonyl)amino]ethoxy}pyridin-4-yl)-3-(4-fluorophenyl)-1H-pyrrolo[3,2-b]pyridine-1-carboxylate (17.0 mg, 31.0 µmol) in DCM (1 ml) TFA (24 µl, 310 µmol) was added at rt. After 18 h, an additional amount of TFA (24 µl, 310 µmol) was added and stirring was continued for 72 h. The solvent was evaporated under reduced pressure until dryness. The remaining residue was taken up in DCM (10 ml) and washed with a saturated sodium hydrogencarbonate solution (aqueous, 5 mL) and a saturated NaCl solution (aqueous, 5 mL). The organic layer was dried over sodium sulfate, filtered and concentrated under reduced pressure. After drying under vacuum, 10.0 mg (98 % purity, 91 % yield) of the title compound were obtained that were used without further purification.
LC-MS (method 1): Rₜ = 0.49 min; MS (ESlpos): m/z = 349 [M+H]⁺
¹H-NMR (600 MHz, DMSO-d6) δ [ppm]: 0.545 (0.80), 0.557 (0.46), 1.234 (1.92), 1.905 (1.17), 2.424 (1.09), 2.519 (1.38), 2.522 (1.32), 2.653 (1.05), 2.731 (1.10), 2.740 (6.01), 2.749 (11.44), 2.758 (6.27), 2.890 (0.99), 3.168 (11.67), 4.100 (6.74), 4.109 (12.37), 4.118 (6.49), 7.162 (6.20), 7.177 (12.18), 7.192 (6.53), 7.210 (5.00), 7.218 (5.13), 7.224 (5.18), 7.231 (5.25), 7.259 (8.87), 7.267 (8.97), 7.312 (0.47), 7.537 (6.25), 7.541 (3.27), 7.547 (7.15), 7.552 (7.47), 7.558 (3.01), 7.561 (6.37), 7.863 (5.44), 7.865 (5.90), 7.877 (5.37), 7.879 (5.51), 8.228 (9.43), 8.236 (9.28), 8.262 (1.77), 8.404 (5.40), 8.407 (5.74), 8.412 (5.66), 8.414 (5.57), 8.495 (0.51), 8.548 (16.00).

### Intermediate 7

### 3-bromo-1-{[2-(trimethylsilyl)ethoxy]methyl}-1H-pyrrolo[3,2-b]pyridine

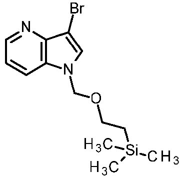

To a suspension of 3-bromo-1H-pyrrolo[3,2-b]pyridine (1.75 g, 8.88 mmol) in THF (25 ml), NaH (710 mg, 60 % purity in mineral oil, 17.8 mmol) was added under argon at 0°C. Stirring at this temperature was continued for 1 h. [2-(chloromethoxy)ethyl](trimethyl)silane (2.4 ml, 13 mmol) was added slowly, the mixture was allowed to warm to rt and stirring was continued for 1 h. Saturated ammonium chloride solution (aqueous, 10 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 1.11 g (86 % purity, 33 % yield) of the desired product.
LC-MS (method 1): Rₜ = 2.04 min; MS (ESlpos): m/z = 327 [M+H]⁺

### Intermediate 8

### 3-phenyl-1-{[2-(trimethylsilyl)ethoxy]methyl}-1H-pyrrolo[3,2-b]pyridine

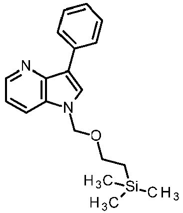

A solution of 3-bromo-1-{[2-(trimethylsilyl)ethoxy]methyl}-1H-pyrrolo[3,2-b]pyridine (600 mg, 1.83 mmol) and phenylboronic acid (335 mg, 2.75 mmol) in a mixture of 1-propanole and water (5:1,10 ml) was carefully degassed and purged with argon. Triphenylphosphine (48.1 mg, 183 µmol), potassium carbonate (760 mg, 5.50 mmol) and the palladium catalyst bis(triphenylphosphine)palladium(ll) dichloride(129 mg, 183 µmol; CAS-RN:[13965-03-2]) were added. The mixture was heated to 100°C and stirred for 2 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 345 mg (98 % purity, 57 % yield) of the desired product.
LC-MS (method 1): Rₜ = 2.27 min; MS (ESlpos): m/z = 325 [M+H]⁺

### Intermediate 9

### 2-bromo-3-phenyl-1-{[2-(trimethylsilyl)ethoxy]methyl}-1H-pyrrolo[3,2-b]pyridine

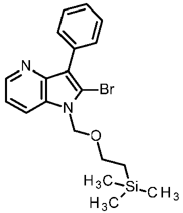

To a solution of 3-phenyl-1-{[2-(trimethylsilyl)ethoxy]methyl}-1H-pyrrolo[3,2-b]pyridine (320 mg, 986 µmol) in 1,2-dichloroethane (4 ml), 1-bromopyrrolidine-2,5-dione (176 mg, 986 µmol) was added at 0°C. After warming to rt within 30 min stirring at rt was continued for 18 h. Saturated sodium hydrogencarbonate solution (aqueous, 1 mL) was added, and the mixture was dried over a water repellent filter. After concentration under reduced pressure the residue was purified by reverse phase preparative HPLC (method 3) yielding 302 mg (97 % purity, 74 % yield) of the title compound.
LC-MS (method 1): Rₜ = 2.53 min; MS (ESlpos): m/z = 403 [M+H]⁺

### Intermediate 10

### tert-butyl (2-{[4-(3-phenyl-1-{[2-(trimethylsilyl)ethoxy]methyl}-1H-pyrrolo[3,2-b]pyridin-2-yl)pyridin-3-yl]oxy}ethyl)carbamate

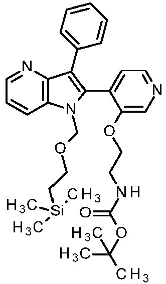

A solution of 2-bromo-3-phenyl-1-{[2-(trimethylsilyl)ethoxy]methyl}-1H-pyrrolo[3,2-b]pyridine (124 mg, 308 µmol) and (3-{2-[(tert-butoxycarbonyl)amino]ethoxy}pyridin-4-yl)boronic acid (113 mg, 401 µmol) in DMF (1.5 ml) in a microwave vial was treated with aqueous sodium carbonate solution (460 µl, 2.0 M, 920 µmol). The mixture was purged with argon for 10 min. Tetrakis(triphenylphosphine)palladium (29.6 mg, 25.6 µmol) was added, the vial was sealed and the mixture was stirred in a microwave apparatus at 110°C for 1.5 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 55.0 mg (99 % purity, 32 % yield) of the title compound.
LC-MS (method 1): Rₜ = 2.19 min; MS (ESlpos): m/z = 561 [M+H]⁺

### Intermediate 11

### tert-butyl (2-{[4-(3-phenyl-1H-pyrrolo[3,2-b]pyridin-2-yl)pyridin-3-yl]oxy}ethyl)carbamate

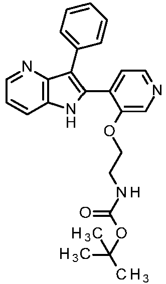

To a solution of tert-butyl (2-{[4-(3-phenyl-1-{[2-(trimethylsilyl)ethoxy]methyl}-1H-pyrrolo[3,2-b]pyridin-2-yl)pyridin-3-yl]oxy}ethyl)carbamate (55.0 mg, 98.1 µmol) in THF (2 ml) TBAF (980 µl, 1.0 M, 980 µmol) was added at rt. The mixture was stirred at 60°C for 18 h. After cooling to rt, water (1 ml) and saturated sodium hydrogen carbonate solution (1 ml) were added. The mixture was extracted with DCM and was dried over a water repellent filter. After concentration under reduced pressure the residue was dried in vacuo to yield 45.0 mg (45 % purity, 48 % yield) of the title compound that was used without further purification.
LC-MS (method 1): Rₜ = 1.14 min; MS (ESlneg): m/z = 429 [M-H]⁻

### Intermediate 12

### 2-{[4-(3-phenyl-1H-pyrrolo[3,2-b]pyridin-2-yl)pyridin-3-yl]oxy}ethan-1-amine

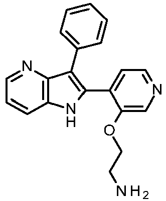

To a solution of tert-butyl (2-{[4-(3-phenyl-1H-pyrrolo[3,2-b]pyridin-2-yl)pyridin-3-yl]oxy}ethyl)carbamate (45.0 mg, 105 µmol) in DCM (1ml) TFA (81 µl, 1.0 mmol) was added at rt. After stirring for 18 h an additional portion of TFA (81 µl, 1.0 mmol) was added and stirring was continued for another 24 h. Saturated aquous sodium hydrogencarbonate solution (1 ml) was added and the mixture was dried over a water repellent filter. After concentration under reduced pressure and drying in vacuo 37.0 mg (88 % purity, 94 % yield) of the title compound were obtained.
LC-MS (method 1): Rₜ = 0.52 min; MS (ESlpos): m/z = 331 [M+H]⁺
¹H-NMR (500 MHz, DMSO-d6) δ [ppm]: 0.921 (6.68), 0.935 (16.00), 0.950 (7.81), 1.082 (1.02), 1.272 (0.54), 1.287 (2.05), 1.301 (3.75), 1.316 (3.79), 1.330 (2.09), 1.345 (0.55), 1.534 (0.77), 1.550 (1.82), 1.566 (2.29), 1.582 (1.60), 1.597 (0.66), 2.074 (0.62), 2.520 (0.42), 2.695 (0.87), 2.706 (1.63), 2.717 (0.89), 3.143 (2.75), 3.160 (2.46), 3.168 (2.37), 3.177 (2.81), 3.400 (0.68), 4.087 (0.98), 4.098 (1.89), 4.109 (1.14), 7.194 (0.91), 7.203 (1.03), 7.211 (1.24), 7.214 (1.75), 7.220 (1.65), 7.224 (1.75), 7.234 (1.16), 7.248 (0.71), 7.301 (0.48), 7.318 (1.60), 7.333 (2.04), 7.348 (0.91), 7.495 (0.49), 7.509 (0.82), 7.522 (1.87), 7.536 (1.50), 7.793 (0.44), 7.866 (0.88), 7.869 (0.92), 7.883 (0.85), 7.885 (0.84), 8.176 (1.46), 8.186 (1.42), 8.392 (1.01), 8.395 (1.05), 8.401 (1.05), 8.404 (1.03), 8.526 (0.71), 8.542 (2.31).

### Intermediate 13

### tert-butyl {3-[(4-bromopyridin-3-yl)oxy]propyl}methylcarbamate

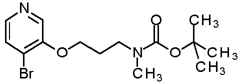

To a solution of 4-bromopyridin-3-ol (500 mg, 2.87 mmol) in THF (10 ml), tert-butyl (3-hydroxypropyl)methylcarbamate (640 µl, 3.4 mmol) and triphenylphosphine (1.13 g, 4.31 mmol) were added. After 30 min, the mixture was cooled to 0°C and dipropan-2-yl (E)-diazene-1,2-dicarboxylate (870 µl, 98 % purity, 4.3 mmol) was added slowly. The mixture was stirred at rt for 1 h. Water (3 ml) was added and the mixture was extracted with EtOAc three times. The combined organic layers were washed with saturated sodium chloride solution, dried over sodium sulfate, filtered and concentrated under reduced pressure. The residue was purified by reverse phase preparative HPLC (method 3) yielding the 298 mg (100 % purity, 30 % yield) of the desired product.
LC-MS (method 1): Rₜ = 1.88 min; MS (ESlpos): m/z = 345 [M+H]⁺

### Intermediate 14

### (3-{3-[(tert-butoxycarbonyl)(methyl)amino]propoxy}pyridin-4-yl)boronic acid

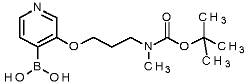

In a microwave vial, a suspension of tert-butyl {3-[(4-bromopyridin-3-yl)oxy]propyl}methylcarbamate (298 mg, 863 µmol) and 4,4,4',4',5,5,5',5'-octamethyl-2,2'-bi-1,3,2-dioxaborolane (336 mg, 98 % purity, 1.29 mmol) and potassium acetate (278 mg, 2.84 mmol) in 1,4-1,4-dioxane (3 ml) was carefully degassed and purged with argon. At rt, trans-dichlorobis(tricyclohexylphosphine)palladium(ll) (63.7 mg, 86.3 µmol; CAS-RN:[29934-17-6]) and potassium acetate (254 mg, 2.59 mmol) were added, the vial was sealed and stirred in a microwave oven at 110°C for 18 h. After cooling to rt, water (5 ml) was added and the mixture was extracted with EtOAc twice. The combined organic layers were dried over sodium sulfate, filtered and concentrated under reduced pressure. The residue was purified by reverse phase preparative HPLC (method 3) yielding 110 mg (100 % purity, 41 % yield) of the title compound.
LC-MS (method 1): Rₜ = 0.97 min; MS(ESlpos): m/z = 311 [M+H]⁺

### Intermediate 15

### tert-butyl methyl(3-{[4-(3-phenyl-1-{[2-(trimethylsilyl)ethoxy]methyl}-1H-pyrrolo[3,2-b]pyridin-2-yl)pyridin-3-yl]oxy}propyl)carbamate

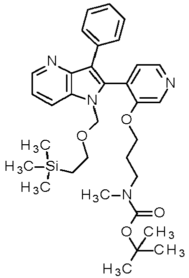

A solution of 2-bromo-3-phenyl-1-{[2-(trimethylsilyl)ethoxy]methyl}-1H-pyrrolo[3,2-b]pyridine (110 mg, 273 µmol) and (3-{3-[(tert-butoxycarbonyl)(methyl)amino]propoxy}pyridin-4-yl)boronic acid (110 mg, 355 µmol) and in DMF (1.5 ml) in a microwave vial was treated with aqueous sodium carbonate solution (410 µl, 2.0 M, 820 µmol). The mixture was purged with argon for 10 min. Tetrakis(triphenylphosphine)palladium (26.2 mg, 22.6 µmol) was added, the vial was sealed and the mixture was stirred in a microwave apparatus at 110°C for 1 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 72.0 mg (96 % purity, 43 % yield) of the title compound.
LC-MS (method 1): Rₜ = 2.41 min; MS (ESlpos): m/z = 589 [M+H]⁺
¹H-NMR (600 MHz, DMSO-d6) δ [ppm]: -0.168 (16.00), -0.096 (1.37), 0.637 (0.70), 0.651 (1.13), 0.665 (0.72), 1.175 (1.39), 1.342 (0.83), 1.597 (0.44), 1.609 (0.68), 1.619 (0.65), 3.222 (0.66), 3.236 (0.71), 3.249 (0.43), 7.179 (0.90), 7.191 (0.59), 7.250 (1.12), 7.263 (1.87), 7.276 (0.95), 7.302 (0.87), 7.310 (0.87), 7.316 (0.87), 7.324 (0.90), 7.462 (1.59), 7.474 (1.39), 8.116 (0.71), 8.130 (0.67), 8.482 (0.87), 8.484 (0.92), 8.490 (0.88), 8.492 (0.87), 8.527 (1.95).

### Intermediate 16

### tert-butyl methyl(3-{[4-(3-phenyl-1H-pyrrolo[3,2-b]pyridin-2-yl)pyridin-3-yl]oxy}propyl)carbamate

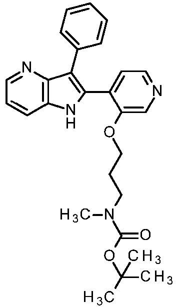

To a solution of tert-butyl methyl(3-{[4-(3-phenyl-1-{[2-(trimethylsilyl)ethoxy]methyl}-1H-pyrrolo[3,2-b]pyridin-2-yl)pyridin-3-yl]oxy}propyl)carbamate (72.0 mg, 122 µmol) in THF (2 ml), TBAF (1.2 ml, 1.0 M in THF, 1.2 mmol) was added at rt. The mixture was stirred at 60°C for 72 h. After cooling to rt, water (1 ml) and saturated sodium hydrogen carbonate solution (1 ml) were added. The mixture was extracted with EtOAc twice. The combined organic layers were dried over sodium sulfate, filtered and concentrated under reduced pressure. The residue was dried in vacuo to yield 66.0 mg (93 % purity, 109 % yield) of the title compound that was used without further purification.
LC-MS (method 1): Rₜ = 1.37 min; MS (ESlpos): m/z = 459 [M+H]⁺

### Intermediate 17

### N-methyl-3-{[4-(3-phenyl-1H-pyrrolo[3,2-b]pyridin-2-yl)pyridin-3-yl]oxy}propan-1-amine

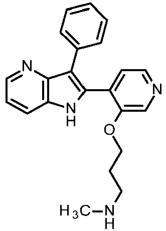

To a solution of tert-butyl methyl(3-{[4-(3-phenyl-1H-pyrrolo[3,2-b]pyridin-2-yl)pyridin-3-yl]oxy}propyl)carbamate (66.0 mg, 144 µmol) in DCM (1ml) TFA (110 µl, 1.4 mmol) was added at rt. After stirring for 18 h, saturated aquous sodium hydrogencarbonate solution (1 ml) was added. The mixture was diluted with DCM (5 ml) and dried over a water repellent filter. After concentration under reduced pressure and drying in vacuo 57.0 mg (88 % purity, 97 % yield) of the title compound were obtained that were used without further purification.
LC-MS (method 1): Rₜ = 0.53 min; MS (ESlneg): m/z = 357 [M-H]⁻

### Intermediate 18

### tert-butyl {2-[(4-bromopyridin-3-yl)oxy]ethyl}methylcarbamate

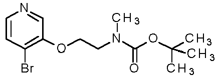

To a solution of 4-bromopyridin-3-ol (500 mg, 2.87 mmol) in THF (10 ml), tert-butyl (2-hydroxyethyl)methylcarbamate (604 mg, 3.45 mmol) and triphenylphosphine (1.13 g, 4.31 mmol) were added. After 30 min, the mixture was cooled to 0°C and dipropan-2-yl (E)-diazene-1,2-dicarboxylate (870 µl, 98 % purity, 4.3 mmol) was added slowly. The mixture was stirred at rt for 1 h. Water (3 ml) was added and the mixture was extracted with EtOAc twice. The combined organic layers were dried over sodium sulfate, filtered and concentrated under reduced pressure. The residue was purified by reverse phase preparative HPLC (method 3) yielding the 644 mg (97 % purity, 66 % yield) of the desired product.
LC-MS (method 1): Rₜ = 1.78 min; MS (ESlpos): m/z = 331 [M+H]⁺

### Intermediate 19

### 2-[(trimethylsilyl)ethynyl]pyridin-3-amine

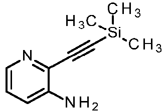

To a solution of 2-bromopyridin-3-amine (2.00 g, 11.6 mmol) in a mixture of THF and triethylamine (1:1, 20 ml) ethynyl(trimethyl)silane (1.9 ml, 14 mmol), copper(I)iodide (220 mg, 1.16 mmol) and tetrakis(triphenylphosphine)palladium(0) (668 mg, 578 µmol) were added at rt under argon. The solution was stirred at 60°C for 3 h. After cooling to rt, saturated aqueous ammonium chloride solution (10 ml) were added. The layers were separated and the aqueous layer was extracted with EtOAc. The combined organic layers were dried over sodium sulfate, filtered and concentrated under reduced pressure. The residue was purified by reverse phase preparative HPLC (method 3) yielding the 1.91 g (99 % purity, 86 % yield) of the desired product.
LC-MS (method 1): Rₜ = 1.34 min; MS (ESlpos): m/z = 191 [M+H]⁺

### Intermediate 20

### tert-butyl [2-({4-[(3-aminopyridin-2-yl)ethynyl]pyridin-3-yl}oxy)ethyl]methylcarbamate

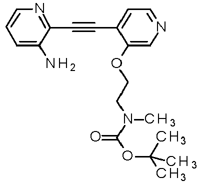

A solution of 2-[(trimethylsilyl)ethynyl]pyridin-3-amine (2.33 g, 12.2 mmol), tert-butyl {2-[(4-bromopyridin-3-yl)oxy]ethyl}methylcarbamate (4.50 g, 12.2 mmol), copper(i)iodide (117 mg, 612 µmol) and bis(triphenylphosphine)palladium(ll)dichloride(430 mg, 612 µmol; CAS-RN:[13965-03-2]) in DMF (20 ml) was carefully degassed and purged with argon. At rt, triethylamine (14 ml, 98 mmol) and TBAF (12 ml, 1.0 M in THF, 12 mmol) were added and the reaction mixture was stirred at 100 °C for 30 min. After cooling to rt, water (10 ml) and saturated aqueous ammonium chloride solution (10 ml) were added, and the mixture was extracted with EtOAc twice. The combined organic layers were dried over sodium sulfate, filtered, and concentrated under reduced pressure. The residue was purified by silica gel column chromatography (eluent: cyclohexane/EtOAc 1:1, EtOAc, EtOAc/MeOH 96:4) yielding 3.75 g (82 % purity, 68 % yield) of the desired product.
LC-MS (method 2): Rₜ = 1.46 min; MS (ESlpos): m/z = 369 [M+H]⁺

### Intermediate 21

### tert-butyl methyl(2-{[4-(1H-pyrrolo[3,2-b]pyridin-2-yl)pyridin-3-yl]oxy}ethyl)carbamate

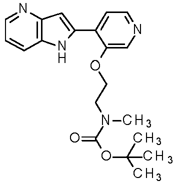

A solution of tert-butyl [2-({4-[(3-aminopyridin-2-yl)ethynyl]pyridin-3-yl}oxy)ethyl]methylcarbamate (3.75 g, 10.2 mmol) in NMP (10 ml) was carefully degassed and purged with argon. Potassium tert-butoxide (2.28 g, 20.4 mmol) was added at rt and the mixture was heated to 90°C for 1 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 2.32 g (96 % purity, 59 % yield) of the desired product.
LC-MS (method 1): Rₜ = 1.03 min; MS (ESlpos): m/z = 369 [M+H]⁺

### Intermediate 22

### tert-butyl (2-{[4-(3-bromo-1H-pyrrolo[3,2-b]pyridin-2-yl)pyridin-3-yl]oxy}ethyl)methylcarbamate

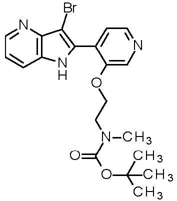

To a solution of tert-butyl methyl(2-{[4-(1H-pyrrolo[3,2-b]pyridin-2-yl)pyridin-3-yl]oxy}ethyl)carbamate (2.78 g, 7.55 mmol) in DCM (10 ml), 1-bromopyrrolidine-2,5-dione (1.34 g, 7.55 mmol; CAS-RN:[128-08-5]) was added at 0°C. After warming to rt within 30 min stirring at rt was continued for 1 h. Saturated sodium hydrogencarbonate solution (aqueous, 10 mL) was added and the mixture was extracted with DCM twice. The combined organic layers were dried over sodium sulfate, filtered, and concentrated under reduced pressure. The residue was purified by silica gel column chromatography (eluent: cyclohexane/EtOAc 1:1) yielding 2.67 g (100 % purity, 79 % yield) of the desired product.
LC-MS (method 1): Rₜ = 1.44 min; MS (ESlpos): m/z = 447 [M+H]⁺

### Intermediate 23

### tert-butyl methyl(2-{[4-(3-phenyl-1H-pyrrolo[3,2-b]pyridin-2-yl)pyridin-3-yl]oxy}ethyl)carbamate

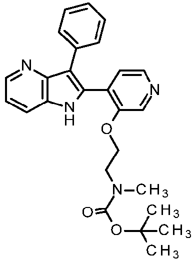

A solution of tert-butyl (2-{[4-(3-bromo-1H-pyrrolo[3,2-b]pyridin-2-yl)pyridin-3-yl]oxy}ethyl)methylcarbamate (800 mg, 1.79 mmol) and phenylboronic acid (327 mg, 2.68 mmol; CAS-RN:[98-80-6]) in a mixture of 1-propanole and water (5:1, 7 ml) was carefully degassed and purged with argon. Triphenylphosphine (46.9 mg, 179 µmol), potassium carbonate (741 mg, 5.37 mmol) and the palladium catalyst bis(triphenylphosphine)palladium(ll) dichloride(126 mg, 179 µmol; CAS-RN:[13965-03-2]) were added. The mixture was heated to 100°C and stirred for 2 h. After cooling to rt, water (5 ml) was added, and the mixture was extracted with EtOAc twice. The combined organic layers were dried over sodium sulfate, filtered, and concentrated under reduced pressure. The residue was purified by silica gel column chromatography (eluent: EtOAc/ MeOH 5:1) yielding 668 mg (85 % purity, 71 % yield) of the desired product.
LC-MS (method 1): Rₜ = 1.29 min; MS (ESlpos): m/z = 445 [M+H]⁺

### Intermediate 24

### N-methyl-2-{[4-(3-phenyl-1H-pyrrolo[3,2-b]pyridin-2-yl)pyridin-3-yl]oxy}ethan-1-amine

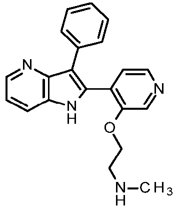

To a solution of tert-butyl methyl(2-{[4-(3-phenyl-1H-pyrrolo[3,2-b]pyridin-2-yl)pyridin-3-yl]oxy}ethyl)carbamate (668 mg, 1.50 mmol) in DCM (10 ml) TFA (1.2 ml, 15 mmol) was added at rt. After stirring for 18 h, saturated aqueous sodium hydrogencarbonate solution (1 ml) was added. The mixture was diluted with DCM (5 ml) and the layers were separated. The aqueous layer was extracted with DCM twice. The combined organic layers were dried over sodium sulfate, filtered, and concentrated under reduced pressure. After drying of the residue in vacuo, 389 mg (84 % purity, 63 % yield) were obtained that were used without further purification.
LC-MS (method 1): Rₜ = 0.52 min; MS (ESlneg): m/z = 343 [M-H]⁻

### Intermediate 25

### tert-butyl {3-[(4-bromopyridin-3-yl)oxy]propyl}carbamate

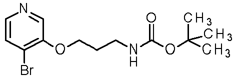

To a solution of 4-bromopyridin-3-ol (500 mg, 2.87 mmol) in THF (10 ml), tert-butyl (3-hydroxypropyl)carbamate (604 mg, 3.45 mmol) and triphenylphosphine (1.13 g, 4.31 mmol) were added. After 30 min, the mixture was cooled to 0°C and dipropan-2-yl (E)-diazene-1,2-dicarboxylate (870 µl, 98 % purity, 4.3 mmol) was added slowly. The mixture was stirred at rt for 1 h. Water (3 ml) was added and the mixture was extracted with EtOAc twice. The combined organic layers were dried over sodium sulfate, filtered and concentrated under reduced pressure. The residue was purified by reverse phase preparative HPLC (method 3) yielding the 853 mg (98 % purity, 88 % yield) of the desired product.
LC-MS (method 2): Rₜ = 1.74 min; MS (ESlpos): m/z = 331 [M+H]⁺

### Intermediate 26

### (3-{3-[(tert-butoxycarbonyl)amino]propoxy}pyridin-4-yl)boronic acid

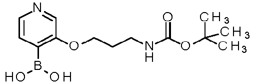

In a microwave vial, a suspension of tert-butyl {3-[(4-bromopyridin-3-yl)oxy]propyl}carbamate (853 mg, 2.58 mmol) and 4,4,4',4',5,5,5',5'-octamethyl-2,2'-bi-1,3,2-dioxaborolane (1.00 g, 98 % purity, 3.86 mmol) and potassium acetate (758 mg, 7.73 mmol) in 1,4-1,4-dioxane (10 ml) was carefully degassed and purged with argon. At rt, trans-dichlorobis(tricyclohexylphosphine)palladium(ll) (190 mg, 258 µmol; CAS-RN:[29934-17-6]) was added, the vial was sealed and stirred in a microwave oven at 110°C for 18 h. After cooling to rt, water (5 ml) was added and the mixture was extracted with EtOAc twice. The combined organic layers were dried over sodium sulfate, filtered and concentrated under reduced pressure. The residue was purified by reverse phase preparative HPLC (method 3) yielding 157 mg (100 % purity, 21 % yield) of the title compound.
LC-MS (method 1): Rₜ = 0.79 min; MS (ESlpos): m/z = 297 [M-H]*

### Intermediate 27

### tert-butyl (3-{[4-(3-phenyl-1-{[2-(trimethylsilyl)ethoxy]methyl}-1H-pyrrolo[3,2-b]pyridin-2-yl)pyridin-3-yl]oxy}propyl)carbamate

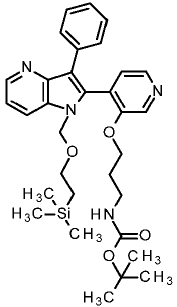

A solution of 2-bromo-3-phenyl-1-{[2-(trimethylsilyl)ethoxy]methyl}-1H-pyrrolo[3,2-b]pyridine (165 mg, 408 µmol) and (3-{3-[(tert-butoxycarbonyl)amino]propoxy}pyridin-4-yl)boronic acid (157 mg, 530 µmol) in DMF (1.5 ml) in a microwave vial was treated with aqueous sodium carbonate solution (610 µl, 2.0 M, 1.2 mmol). The mixture was purged with argon for 10 min. Tetrakis(triphenylphosphine)palladium(0) (39.1 mg, 33.8 µmol) was added, the vial was sealed and the mixture was stirred in a microwave apparatus at 110°C for 1.5 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 59.7 mg (97 % purity, 25 % yield) of the title compound.
LC-MS (method 1): Rₜ = 2.22 min; MS (ESlpos): m/z = 575 [M+H]⁺
¹H-NMR (500 MHz, DMSO-d6) δ [ppm]: -0.179 (0.91), -0.173 (16.00), -0.167 (0.73), 0.638 (0.69), 1.346 (5.57), 3.164 (1.12), 3.174 (1.17), 5.225 (0.48), 5.248 (0.52), 7.176 (0.54), 7.240 (0.44), 7.246 (0.72), 7.262 (1.12), 7.277 (0.56), 7.301 (0.53), 7.310 (0.52), 7.318 (0.51), 7.327 (0.52), 7.439 (0.93), 7.453 (0.82), 7.456 (0.62), 8.096 (0.53), 8.099 (0.53), 8.113 (0.52), 8.115 (0.48), 8.261 (0.51), 8.271 (0.50), 8.480 (0.60), 8.483 (0.59), 8.489 (0.59), 8.492 (0.53), 8.550 (1.31).

### Intermediate 28

### tert-butyl (3-{[4-(3-phenyl-1H-pyrrolo[3,2-b]pyridin-2-yl)pyridin-3-yl]oxylpropyl)carbamate

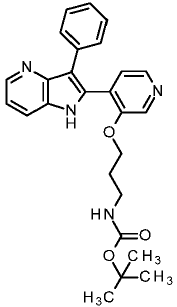

To a solution of tert-butyl (3-{[4-(3-phenyl-1-{[2-(trimethylsilyl)ethoxy]methyl}-1H-pyrrolo[3,2-b]pyridin-2-yl)pyridin-3-yl]oxy}propyl)carbamate (55.0 mg, 95.7 µmol) in THF (2 ml), TBAF (960 µl, 1.0 M in THF, 960 µmol) was added at rt. The mixture was stirred at 60°C for 18 h. After cooling to rt, water (1 ml) and saturated sodium hydrogen carbonate solution (1 ml) were added. The mixture was extracted with EtOAc twice. The combined organic layers were dried over sodium sulfate, filtered and concentrated under reduced pressure. The residue was purified by reverse phase preparative HPLC (method 3) yielding 17.0 mg (97 % purity, 39 % yield) of the title compound.
LC-MS (method 1): Rₜ = 1.24 min; MS (ESlpos): m/z = 445 [M+H]⁺

### Intermediate 29

### 3-{[4-(3-phenyl-1H-pyrrolo[3,2-b]pyridin-2-yl)pyridin-3-yl]oxy}propan-1-amine

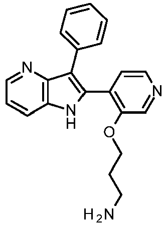

To a solution of tert-butyl (3-{[4-(3-phenyl-1H-pyrrolo[3,2-b]pyridin-2-yl)pyridin-3-yl]oxy}propyl)carbamate (17.0 mg, 38.2 µmol) in DCM (1 ml), TFA (29 µl, 380 µmol) was added at rt. After stirring for 18 h, saturated aqueous sodium hydrogencarbonate solution (1 ml) was added. The mixture was diluted with DCM (5 ml) and dried over a water repellent filter. After concentration under reduced pressure and drying in vacuo 14.0 mg (98 % purity, 104 % yield) of the title compound were obtained that were used without further purification.
LC-MS (method 1): Rₜ = 0.48 min; MS (ESlpos): m/z = 345 [M+H]⁺

### Intermediate 30

### 3-bromo-6-fluoro-1H-pyrrolo[3,2-b]pyridine

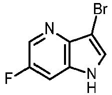

To a suspension of 6-fluoro-1H-pyrrolo[3,2-b]pyridine (500 mg, 3.67 mmol) in DCM (5 ml), 1-bromopyrrolidine-2,5-dione (654 mg, 3.67 mmol) were added at rt in small portions. To improve stirring, additional DCM (5 ml) was added. After 1 h, the precipitate was filtered off. The white solid was washed with DCM and dried in vacuo. 702 mg (100 % purity, 89 % yield) of the desired compound were obtained.
LC-MS (method 2): Rₜ = 1.13 min; MS (ESlpos): m/z = 215 [M+H]⁺
¹H-NMR (500 MHz, DMSO-d6) δ [ppm]: 2.430 (1.00), 6.587 (0.70), 7.673 (0.88), 7.687 (0.57), 7.709 (0.52), 7.756 (8.50), 7.759 (8.85), 7.775 (8.69), 7.778 (8.65), 7.870 (15.70), 7.874 (15.78), 8.337 (0.82), 8.413 (16.00), 11.799 (7.10).

### Intermediate 31

### 3-bromo-6-fluoro-1-{[2-(trimethylsilyl)ethoxy]methyl}-1H-pyrrolo[3,2-b]pyridine

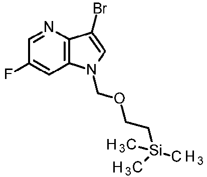

To a suspension of 3-bromo-6-fluoro-1H-pyrrolo[3,2-b]pyridine (700 mg, 3.26 mmol) in THF (25 ml), NaH (260 mg, 60 % purity in mineral oil, 6.51 mmol) was added under argon at 0°C. Stirring at this temperature was continued for 1 h. [2-(chloromethoxy)ethyl](trimethyl)silane (860 µl, 4.9 mmol) was added slowly, the mixture was allowed to warm to rt and stirring was continued for 1 h. Saturated ammonium chloride solution (aqueous, 10 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 5:1) yielding 830 mg (100 % purity, 74 % yield) of the desired product.
LC-MS (method 2): Rₜ = 2.33 min; MS (ESlpos): m/z = 345 [M+H]⁺
¹H-NMR (600 MHz, DMSO-d6) δ [ppm]: -0.100 (0.47), -0.023 (0.41), 0.005 (3.45), 0.029 (0.74), 0.098 (0.74), 0.889 (4.16), 0.902 (6.50), 0.916 (4.19), 2.642 (15.66), 3.542 (4.37), 3.555 (6.45), 3.569 (4.20), 5.660 (16.00), 8.174 (10.07), 8.190 (2.61), 8.195 (2.59), 8.207 (2.61), 8.211 (2.50), 8.572 (3.48), 8.574 (3.92), 8.576 (3.67), 8.578 (2.64).

### Intermediate 32

### 6-fluoro-3-phenyl-1-{[2-(trimethylsilyl)ethoxy]methyl}-1H-pyrrolo[3,2-b]pyridine

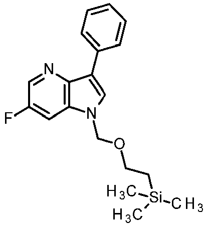

A solution of 3-bromo-6-fluoro-1-{[2-(trimethylsilyl)ethoxy]methyl}-1H-pyrrolo[3,2-b]pyridine (830 mg, 2.40 mmol) and phenylboronic acid (440 mg, 3.61 mmol) in a mixture of 1-propanole and water (5:1,10 ml) was carefully degassed and purged with argon. Triphenylphosphine (63.0 mg, 240 µmol), potassium carbonate (997 mg, 7.21 mmol) and the palladium catalyst bis(triphenylphosphine)palladium(ll) dichloride(169 mg, 240 µmol; CAS-RN:[13965-03-2]) were added. The mixture was heated to 100°C and stirred for 2 h. After cooling to rt, water (5 ml) was added, and the mixture was extracted with EtOAc twice. The combined organic layers were dried over sodium sulfate, filtered, and concentrated under reduced pressure. The residue was purified by silica gel column chromatography (eluent: cyclohexane/EtOAc 2:1) yielding 451 mg (100 % purity, 55 % yield) of the desired product.
LC-MS (method 1): Rₜ = 2.58 min; MS (ESlpos): m/z = 343 [M+H]⁺
¹H-NMR (600 MHz, DMSO-d6) δ [ppm]: -0.014 (0.65), 0.093 (10.08), 0.914 (2.68), 0.927 (3.96), 0.941 (2.63), 1.162 (0.88), 2.636 (16.00), 3.588 (2.74), 3.601 (3.91), 3.615 (2.65), 5.709 (9.43), 7.322 (0.92), 7.334 (2.05), 7.347 (1.21), 7.503 (2.52), 7.516 (3.92), 7.529 (2.21), 8.141 (1.54), 8.145 (1.70), 8.157 (1.54), 8.161 (1.63), 8.296 (3.21), 8.298 (3.56), 8.310 (3.39), 8.311 (2.91), 8.430 (6.30), 8.604 (2.08), 8.606 (2.37), 8.608 (2.38), 8.610 (1.94).

### Intermediate 33

### 2-bromo-6-fluoro-3-phenyl-1-{[2-(trimethylsilyl)ethoxy]methyl}-1H-pyrrolo[3,2-b]pyridine

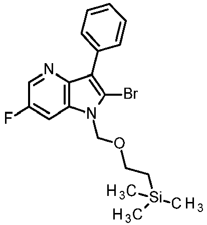

A solution of 6-fluoro-3-phenyl-1-{[2-(trimethylsilyl)ethoxy]methyl}-1H-pyrrolo[3,2-b]pyridine (614 mg, 96 % purity, 1.72 mmol) in DCM (5 ml) was treated at rt with 1-bromopyrrolidine-2,5-dione (322 mg, 1.81 mmol; CAS-RN:[128-08-5]). After stirring for 3 h, water (3 ml) was added. The layers were separated and the aqueous layer was extracted with DCM twice. The combined organic layers were dried over sodium sulfate, filtered and concentrated under reduced pressure. The residue was purified by silica gel column chromatography (eluent: cyclohexane/EtOAc 4:1) yielding 484.0 mg (93 % purity, 62 % yield) of the desired product.
LC-MS (method 1): Rₜ = 2.69 min; MS (ESlpos): m/z = 421 [M+H]⁺
¹H-NMR (500 MHz, DMSO-d6) δ [ppm]: 0.078 (0.60), 0.085 (16.00), 0.092 (0.52), 0.912 (0.98), 0.928 (1.55), 0.944 (1.00), 2.164 (0.46), 3.650 (1.04), 3.666 (1.56), 3.682 (1.02), 5.802 (3.40), 7.468 (0.77), 7.483 (0.52), 7.569 (0.95), 7.584 (1.57), 7.600 (0.80), 7.859 (0.54), 7.861 (1.25), 7.864 (1.38), 7.878 (1.28), 7.880 (1.01), 8.269 (0.60), 8.274 (0.68), 8.289 (0.60), 8.294 (0.65), 8.544 (0.80), 8.547 (0.89), 8.549 (0.89), 8.552 (0.72).

### Intermediate 34

### tert-butyl (2-{[4-(6-fluoro-3-phenyl-1-{[2-(trimethylsilyl)ethoxy]methyl}-1H-pyrrolo[3,2-b]pyridin-2-yl)pyridin-3-yl]oxy}ethyl)carbamate

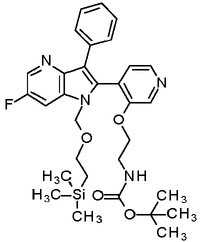

A solution of 2-bromo-6-fluoro-3-phenyl-1-{[2-(trimethylsilyl)ethoxy]methyl}-1H-pyrrolo[3,2-b]pyridine (132 mg, 314 µmol) and (3-{2-[(tert-butoxycarbonyl)amino]ethoxy}pyridin-4-yl)boronic acid (115 mg, 408 µmol) in DMF (1.5 ml) in a microwave vial was treated with aqueous sodium carbonate solution (470 µl, 2.0 M, 940 µmol). The mixture was purged with argon for 10 min. Tetrakis(triphenylphosphine)palladium(0) (30.1 mg, 26.0 µmol) was added, the vial was sealed and the mixture was stirred in a microwave apparatus at 110°C for 2 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 40.0 mg (100 % purity, 22 % yield) of the title compound.
LC-MS (method 1): Rₜ = 2.54 min; MS (ESlpos): m/z = 579 [M+H]⁺
¹H-NMR (600 MHz, DMSO-d6) δ [ppm]: 0.167 (0.92), 0.800 (0.45), 0.810 (0.46), 1.455 (6.74), 2.666 (11.61), 2.669 (16.00), 2.672 (12.34), 2.708 (14.05), 3.332 (0.92), 3.341 (1.04), 5.354 (0.40), 5.373 (0.42), 7.363 (0.48), 7.375 (0.61), 7.381 (0.40), 7.426 (0.58), 7.439 (0.99), 7.451 (0.49), 7.564 (0.93), 7.576 (0.77), 8.423 (0.41), 8.431 (0.42), 8.663 (0.59), 8.757 (0.91).

### Intermediate 35

### tert-butyl (2-{[4-(6-fluoro-3-phenyl-1H-pyrrolo[3,2-b]pyridin-2-yl)pyridin-3-yl]oxy}ethyl)carbamate

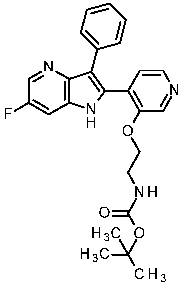

To a solution of tert-butyl (2-{[4-(6-fluoro-3-phenyl-1-{[2-(trimethylsilyl)ethoxy]methyl}-1H-pyrrolo[3,2-b]pyridin-2-yl)pyridin-3-yl]oxy}ethyl)carbamate (40.0 mg, 69.1 µmol) in THF (2 ml), TBAF (690 µl, 1.0 M, 690 µmol) was added at rt. The mixture was stirred at 60°C for 18 h. After cooling to rt, water (1 ml) and saturated sodium hydrogen carbonate solution (1 ml) were added. The mixture was diluted with DCM (5 ml) and dried over a water repellent filter. After concentration under reduced pressure and drying in vacuo 38.8 mg (83 % purity, 104 % yield) of the title compound were obtained that were used without further purification.
LC-MS (method 1): Rₜ = 1.87 min; MS (ESlpos): m/z = 449 [M+H]⁺

### Intermediate 36

### 2-{[4-(6-fluoro-3-phenyl-1H-pyrrolo[3,2-b]pyridin-2-yl)pyridin-3-yl]oxy}ethan-1-amine

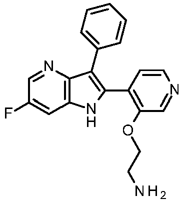

To a solution of tert-butyl (2-{[4-(6-fluoro-3-phenyl-1H-pyrrolo[3,2-b]pyridin-2-yl)pyridin-3-yl]oxy}ethyl)carbamate (38.0 mg, 84.7 µmol) in DCM (1 ml), TFA (65 µl, 850 µmol) was added at rt. After stirring for 48 h and additional amount of TFA (65 µl, 850 µmol) was added and stirring was continued for 5h. For work-up, saturated aqueous sodium hydrogencarbonate solution (1 ml) was added. The mixture was diluted with DCM (5 ml) and dried over a water repellent filter. After concentration under reduced pressure and drying in vacuo 27.0 mg (60 % purity, 55 % yield) of the title compound were obtained that were used without further purification.
LC-MS (method 1): Rₜ = 0.91 min; MS (ESlpos): m/z = 349 [M+H]⁺

### Intermediate 37

### (3-{2-[(tert-butoxycarbonyl)(methyl)amino]ethoxy}pyridin-4-yl)boronic acid

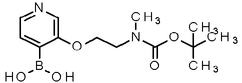

In a microwave vial, a suspension of tert-butyl {2-[(4-bromopyridin-3-yl)oxy]ethyl}methylcarbamate (644 mg, 1.94 mmol) and 4,4,4',4',5,5,5',5'-octamethyl-2,2'-bi-1,3,2-dioxaborolane (756 mg, 98 % purity, 2.92 mmol) and potassium acetate (572 mg, 5.83 mmol) in 1,4-1,4-dioxane (10 ml) was carefully degassed and purged with argon. At rt, trans-dichlorobis(tricyclohexylphosphine)palladium(ll) (159 mg, 194 µmol) was added, the vial was sealed and stirred in a microwave oven at 110°C for 18 h. After cooling to rt, water (5 ml) was added and the mixture was extracted with EtOAc twice. The combined organic layers were dried over sodium sulfate, filtered and concentrated under reduced pressure. The residue was purified by reverse phase preparative HPLC (method 3) yielding 229 mg (99 % purity, 39 % yield) of the title compound.
LC-MS (method 1): Rₜ = 0.92 min; MS (ESlpos): m/z = 297 [M+H]⁺

### Intermediate 38

### tert-butyl (2-{[4-(6-fluoro-3-phenyl-1-{[2-(trimethylsilyl)ethoxy]methyl}-1H-pyrrolo[3,2-b]pyridin-2-yl)pyridin-3-yl]oxy}ethyl)methylcarbamate

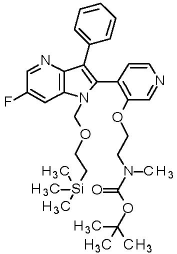

A solution of 2-bromo-6-fluoro-3-phenyl-1-{[2-(trimethylsilyl)ethoxy]methyl}-1H-pyrrolo[3,2-b]pyridine (167 mg, 397 µmol) and (3-{2-[(tert-butoxycarbonyl)(methyl)amino]ethoxy}pyridin-4-yl)boronic acid (153 mg, 517 µmol) in DMF (1.5 ml) in a microwave vial was treated with aqueous sodium carbonate solution (600 µl, 2.0 M, 1.2 mmol). The mixture was purged with argon for 10 min. Tetrakis(triphenylphosphine)palladium(0) (38.1 mg, 33.0 µmol) was added, the vial was sealed and the mixture was stirred in a microwave apparatus at 110°C for 2 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 53.0 mg (100 % purity, 22 % yield) of the title compound.
LC-MS (method 1): Rₜ = 2.63 min; MS (ESlpos): m/z = 593 [M+H]⁺
¹H-NMR (600 MHz, DMSO-d6) δ [ppm]: -0.164 (16.00), 0.632 (0.48), 0.648 (0.47), 1.266 (5.54), 1.907 (3.29), 2.072 (1.43), 2.422 (2.23), 3.249 (0.40), 3.260 (0.45), 7.197 (0.54), 7.258 (0.60), 7.271 (1.03), 7.283 (0.62), 7.416 (0.60), 7.428 (0.52), 8.488 (0.70), 8.490 (0.71).

### Intermediate 39

### tert-butyl (2-{[4-(6-fluoro-3-phenyl-1H-pyrrolo[3,2-b]pyridin-2-yl)pyridin-3-yl]oxy}ethyl)methylcarbamate

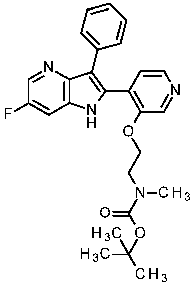

To a solution of tert-butyl (2-{[4-(6-fluoro-3-phenyl-1-{[2-(trimethylsilyl)ethoxy]methyl}-1H-pyrrolo[3,2-b]pyridin-2-yl)pyridin-3-yl]oxy}ethyl)methylcarbamate (53.0 mg, 89.4 µmol) in THF (1 ml), TBAF (890 µl, 1.0 M, 890 µmol) was added at rt. The mixture was stirred at 60°C for 18 h. After cooling to rt, water (1 ml) and saturated sodium hydrogen carbonate solution (1 ml) were added and the mixture was extracted with EtOAc twice. The combined organic layers were dried over sodium sulfate, filtered and concentrated under reduced pressure. After drying the remaining material in vacuo 44.0 mg (100 % purity, 106 % yield) of the title compound were obtained that were used without further purification.
LC-MS (method 1): Rₜ = 2.04 min; MS (ESlpos): m/z = 463 [M+H]⁺

### Intermediate 40

### 2-{[4-(6-fluoro-3-phenyl-1H-pyrrolo[3,2-b]pyridin-2-yl)pyridin-3-yl]oxy}-N-methylethan-1-amine

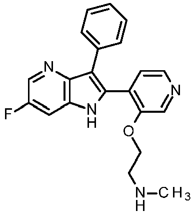

To a solution of tert-butyl (2-{[4-(6-fluoro-3-phenyl-1H-pyrrolo[3,2-b]pyridin-2-yl)pyridin-3-yl]oxy}ethyl)methylcarbamate (44.0 mg, 95.1 µmol) in DCM (1 ml), TFA (73 µl, 950 µmol; CAS-RN:[76-05-1]) was added at rt. After stirring for 24 h, saturated aqueous sodium hydrogencarbonate solution (1 ml) was added. The mixture was diluted with DCM (5 ml) and dried over a water repellent filter. After concentration under reduced pressure and drying in vacuo 44.0 mg (95 % purity, 121 % yield) of the title compound were obtained that were used without further purification.
LC-MS (method 1): Rₜ = 0.91 min; MS (ESIneg): m/z = 361 [M-H]⁻

### Intermediate 41

### 3-bromo-5-methoxy-1H-pyrrolo[3,2-b]pyridine

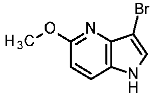

To a solution of 5-methoxy-1H-pyrrolo[3,2-b]pyridine (500 mg, 3.37 mmol) in DCM (5 ml), 1-bromopyrrolidine-2,5-dione (601 mg, 3.37 mmol) was added at rt in small portions. After 1 h, water (1 ml) and saturated sodium hydrogencarbonate solution (aqueous, 1 mL) were added. The mixture was diluted with DCM and dried over a water repellent filter. The solvent was evaporated under reduced pressure and the remaining residue was purified by silica gel column chromatography (eluent: cyclohexane/EtOAc 2:1) yielding 592 mg (96 % purity, 74 % yield) of the desired product.
LC-MS (method 1): Rₜ = 1.41 min; MS (ESlpos): m/z = 226 [M+H]⁺
¹H-NMR (600 MHz, DMSO-d6) δ [ppm]: 2.073 (0.74), 3.892 (16.00), 6.619 (2.56), 6.634 (2.66), 7.653 (2.43), 7.658 (2.40), 7.735 (2.70), 7.749 (2.66), 11.529 (0.88).

### Intermediate 42

### 3-bromo-5-methoxy-1-{[2-(trimethylsilyl)ethoxy]methyl}-1H-pyrrolo[3,2-b]pyridine

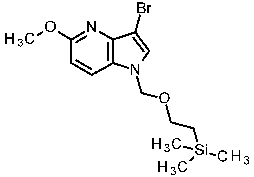

To a solution of 3-bromo-5-methoxy-1H-pyrrolo[3,2-b]pyridine (573 mg, 2.52 mmol) in THF (5 ml), NaH (121 mg, 60% in mineral oil, 5.05 mmol) was added under argon at 0°C. Stirring at this temperature was continued for 1 h. [2-(chloromethoxy)ethyl](trimethyl)silane (670 µl, 3.8 mmol) was added slowly, the mixture was allowed to warm to rt and stirring was continued for 5 h. Saturated ammonium chloride solution (aqueous, 2 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 534 mg (91 % purity, 54 % yield) of the desired product.
LC-MS (method 1): Rₜ = 2.49 min; MS (ESlpos): m/z = 357 [M+H]⁺
¹H-NMR (600 MHz, DMSO-d6) δ [ppm]: -0.090 (1.06), -0.088 (1.15), -0.037 (0.90), 0.095 (3.34), 0.098 (4.60), 0.880 (1.97), 0.893 (2.69), 0.906 (2.05), 2.640 (6.83), 3.511 (2.14), 3.524 (2.72), 3.537 (2.08), 4.007 (16.00), 5.619 (7.36), 6.809 (2.40), 6.824 (2.43), 7.961 (4.52), 8.062 (2.89), 8.076 (2.84).

### Intermediate 43

### 5-methoxy-3-phenyl-1-{[2-(trimethylsilyl)ethoxy]methyl}-1H-pyrrolo[3,2-b]pyridine

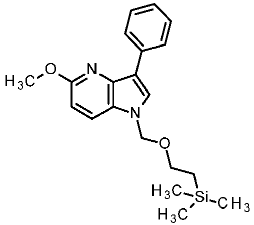

A solution of 3-bromo-5-methoxy-1-{[2-(trimethylsilyl)ethoxy]methyl}-1H-pyrrolo[3,2-b]pyridine (534 mg, 1.49 mmol) and phenylboronic acid (273 mg, 2.24 mmol) in a mixture of 1-propanole and water (5:1,10 ml) was carefully degassed and purged with argon. Triphenylphosphine (39.2 mg, 149 µmol), potassium carbonate (620 mg, 4.48 mmol) and the palladium catalyst bis(triphenylphosphine)palladium(ll) dichloride(105 mg, 149 µmol; CAS-RN:[13965-03-2]) were added. The mixture was heated to 100°C and stirred for 18 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 287 mg (98 % purity, 53 % yield) of the title compound.
LC-MS (method 1): Rₜ = 2.68 min; MS (ESlpos): m/z = 355 [M+H]⁺
¹H-NMR (600 MHz, DMSO-d6) δ [ppm]: -0.027 (0.60), -0.021 (0.44), 0.092 (2.82), 0.905 (1.37), 0.918 (1.94), 0.931 (1.41), 2.633 (16.00), 3.557 (1.46), 3.571 (1.98), 3.584 (1.40), 4.055 (10.36), 5.665 (4.77), 6.792 (1.74), 6.807 (1.76), 7.280 (0.50), 7.293 (1.04), 7.305 (0.60), 7.488 (1.28), 7.502 (1.91), 7.511 (0.46), 7.514 (1.15), 8.052 (2.00), 8.067 (1.95), 8.264 (3.09), 8.313 (1.57), 8.315 (1.78), 8.327 (1.71), 8.329 (1.45).

### Intermediate 44

### 2-bromo-5-methoxy-3-phenyl-1-{[2-(trimethylsilyl)ethoxy]methyl}-1H-pyrrolo[3,2-b]pyridine

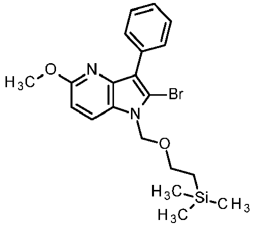

A solution of 5-methoxy-3-phenyl-1-{[2-(trimethylsilyl)ethoxy]methyl}-1H-pyrrolo[3,2-b]pyridine (287 mg, 810 µmol) in DCM (5 ml) was treated at rt with 1-bromopyrrolidine-2,5-dione (151 mg, 850 µmol). After stirring for 1 h, water (1 ml) and saturated sodium hydrogencarbonate solution (aqueous, 1 mL) were added. The mixture was diluted with DCM and dried over a water repellent filter. The solvent was evaporated under reduced pressure and the remaining residue was purified by silica gel column chromatography (eluent: cyclohexane/EtOAc 4:1) yielding 224 mg (92 % purity, 59 % yield) of the desired product.
LC-MS (method 1): Rₜ = 2.81 min; MS (ESlpos): m/z = 433 [M+H]⁺
¹H-NMR (500 MHz, DMSO-d6) δ [ppm]: 0.078 (0.58), 0.085 (16.00), 0.092 (0.48), 0.904 (0.62), 0.919 (0.93), 0.935 (0.64), 3.623 (0.67), 3.639 (0.97), 3.655 (0.68), 3.937 (4.77), 5.764 (2.10), 6.813 (1.00), 6.830 (0.99), 7.431 (0.50), 7.557 (0.59), 7.573 (0.92), 7.589 (0.51), 7.930 (0.79), 7.932 (0.83), 7.946 (0.77), 7.948 (0.62), 8.130 (0.93), 8.148 (0.90).

### Intermediate 45

### tert-butyl (2-{[4-(5-methoxy-3-phenyl-1-{[2-(trimethylsilyl)ethoxy]methyl}-1H-pyrrolo[3,2-b]pyridin-2-yl)pyridin-3-yl]oxy}ethyl)methylcarbamate

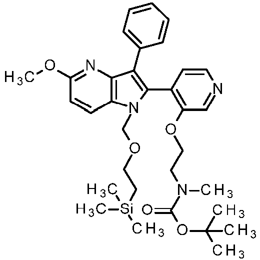

A solution of 2-bromo-5-methoxy-3-phenyl-1-{[2-(trimethylsilyl)ethoxy]methyl}-1H-pyrrolo[3,2-b]pyridine (92.7 mg, 214 µmol) and (3-{2-[(tert-butoxycarbonyl)(methyl)amino]ethoxy}pyridin-4-yl)boronic acid (190 mg, 50 % purity, 321 µmol) in DMF (3 ml) in a microwave vial was treated with aqueous sodium carbonate solution (320 µl, 2.0 M, 640 µmol). The mixture was purged with argon for 10 min. Tetrakis(triphenylphosphine)palladium(0) (20.5 mg, 17.8 µmol) was added, the vial was sealed and the mixture was stirred in a microwave apparatus at 110°C for 2 h. After cooling to rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 30.0 mg (98 % purity, 23 % yield) of the title compound. An additional fraction was obtained containig triphenylphosphine oxide as impurity (69.0 mg, 80% purity, 43% yield)
LC-MS (method 1): Rₜ = 2.73 min; MS (ESlpos): m/z = 605 [M+H]⁺

### Intermediate 46

### tert-butyl (2-{[4-(5-methoxy-3-phenyl-1H-pyrrolo[3,2-b]pyridin-2-yl)pyridin-3-yl]oxy}ethyl)methylcarbamate

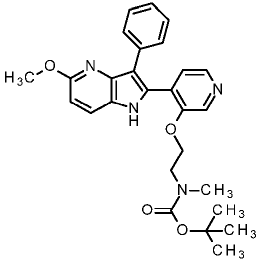

To a solution of tert-butyl (2-{[4-(5-methoxy-3-phenyl-1-{[2-(trimethylsilyl)ethoxy]methyl}-1H-pyrrolo[3,2-b]pyridin-2-yl)pyridin-3-yl]oxy}ethyl)methylcarbamate (92.7 mg, 153 µmol) in THF (1 ml), TBAF (150 µl, 1M solution in THF, 1.5 mmol) was added at rt. The mixture was stirred at 60°C for 18 h. After cooling to rt, water (1 ml) and saturated sodium hydrogen carbonate solution (1 ml) were added. The mixture was diluted with DCM and dried over a water repellent filter. The solvent was evaporated under reduced pressure After drying the remaining material in vacuo 68.0 mg (89 % purity, 83 % yield) of the title compound were obtained that were used without further purification.
LC-MS (method 1): Rₜ = 2.17 min; MS (ESlpos): m/z = 475 [M+H]⁺

### Intermediate 47

### 2-{[4-(5-methoxy-3-phenyl-1H-pyrrolo[3,2-b]pyridin-2-yl)pyridin-3-yl]oxy}-N-methylethan-1-amine

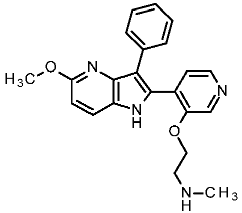

To a solution of tert-butyl (2-{[4-(5-methoxy-3-phenyl-1H-pyrrolo[3,2-b]pyridin-2-yl)pyridin-3-yl]oxy}ethyl)methylcarbamate (68.0 mg, 143 µmol) in DCM (1 ml), TFA (110 µl, 1.4 mmol) was added at rt. After stirring for 24 h, saturated aqueous sodium hydrogencarbonate solution (1 ml) was added. The mixture was diluted with DCM (5 ml) and dried over a water repellent filter. After concentration under reduced pressure and drying in vacuo 45.0 mg (87 % purity, 73 % yield) of the title compound were obtained that were used without further purification.
LC-MS (method 1): Rₜ = 1.07 min; MS (ESlpos): m/z = 375 [M+H]⁺

### Intermediate 48

### tert-butyl methyl[2-({4-[3-(3-methylphenyl)-1H-pyrrolo[3,2-b]pyridin-2-yl]pyridin-3-yl}oxy)ethyl]carbamate

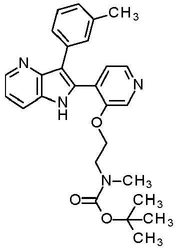

A solution of tert-butyl (2-{[4-(3-bromo-1H-pyrrolo[3,2-b]pyridin-2-yl)pyridin-3-yl]oxy}ethyl)methylcarbamate (80.0 mg, 179 µmol) and (3-methylphenyl)boronic acid (36.5 mg, 268 µmol) in a mixture of 1-propanole and water (5:1, 3 ml) was carefully degassed and purged with argon. Triphenylphosphine (4.69 mg, 17.9 µmol), potassium carbonate (74.1 mg, 537 µmol) and the palladium catalyst bis(triphenylphosphine)palladium(ll) dichloride(12.6 mg, 17.9 µmol; CAS-RN:[13965-03-2]) were added. The mixture was heated to 100°C and stirred for 1.5 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 56.0 mg (100 % purity, 68 % yield) of the title compound.
LC-MS (method 1): Rₜ = 1.38 min; MS (ESlpos): m/z = 459 [M+H]⁺
¹H-NMR (500 MHz, DMSO-d6) δ [ppm]: 1.281 (8.83), 1.309 (8.59), 2.266 (16.00), 2.520 (1.43), 2.524 (1.51), 2.582 (2.57), 2.584 (2.56), 2.637 (0.40), 3.128 (1.08), 3.164 (2.82), 3.175 (2.81), 3.254 (1.09), 3.997 (1.10), 4.080 (1.02), 4.090 (1.71), 4.101 (1.51), 4.111 (0.65), 7.029 (1.43), 7.042 (1.67), 7.156 (0.90), 7.171 (1.90), 7.186 (1.39), 7.194 (3.31), 7.203 (3.16), 7.210 (4.52), 7.215 (4.44), 7.219 (4.42), 7.230 (2.13), 7.306 (0.68), 7.361 (0.70), 7.426 (5.13), 7.821 (1.25), 8.238 (0.70), 8.280 (0.71), 8.406 (3.45), 8.408 (3.49), 8.415 (3.48), 8.417 (3.21), 8.513 (6.64), 11.645 (0.72), 11.699 (0.73).

The intermediates listed in the following table were prepared in an analogous manner to intermediate 49.

| **Intermediate** | **Structure** | **LC-MS method** |
|---|---|---|
| | **IUPAC-Name** | **Retention time** |
| | | **Mass found** |
| **50** | 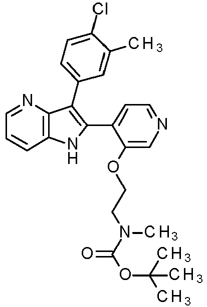 **tert-butyl [2-({4-[3-(4-chloro-3-methylphenyl)-1H-pyrrolo[3,2-b]pyridin-2-yl]pyridin-3-yl}oxy)ethyl]methylcarbamate** | method 2 |
| | | Rₜ = 1.70 min |
| | | m/z = 493 [M+H]⁺ |
| **51** | 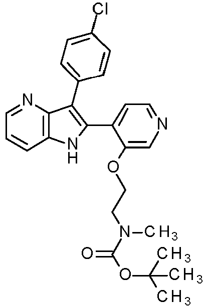 **tert-butyl [2-({4-[3-(4-chlorophenyl)-1H-pyrrolo[3,2-b]pyridin-2-yl]pyridin-3-yl}oxy)ethyl]methylcarbamate** | method 2 |
| | | Rₜ = 1.63 min |
| | | m/z = 479 [M+H]⁺ |
| **52** | 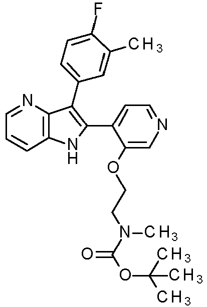 **tert-butyl [2-({4-[3-(4-fluoro-3-methylphenyl)-1H-pyrrolo[3,2-b]pyridin-2-yl]pyridin-3-yl}oxy)ethyl]methylcarbamate** | method 2 |
| | | Rₜ = 1.53 min |
| | | m/z = 477 [M+H]⁺ |
| **53** | 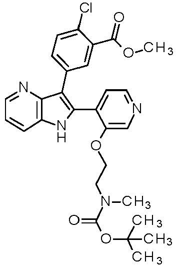 **methyl 5-[2-(3-{2-[(tert-butoxycarbonyl)(methyl)amino]ethoxy}pyridin-4-yl)-1H-pyrrolo[3,2-b]pyridin-3-yl]-2-chlorobenzoate** | method 2 |
| | | Rₜ = 1.70 min |
| | | m/z = 537 [M+H]⁺ |
| **54** | 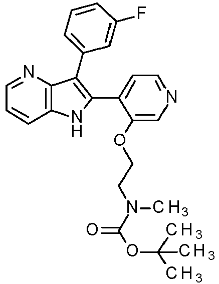 **tert-butyl [2-({4-[3-(3-fluorophenyl)-1H-pyrrolo[3,2-b]pyridin-2-yl]pyridin-3-yl}oxy)ethyl]methylcarbamate** | method 2 |
| | | Rₜ = 1.51 min |
| | | m/z = 463 [M+H]⁺ |
| **55** | 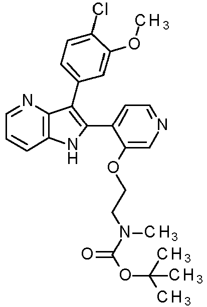 **tert-butyl [2-({4-[3-(4-chloro-3-methoxyphenyl)-1H-pyrrolo[3,2-b]pyridin-2-yl]pyridin-3-yl}oxy)ethyl]methylcarbamate** | method 1 |
| | | Rₜ = 1.53 min |
| | | m/z = 509 [M+H]⁺ |
| **56** | 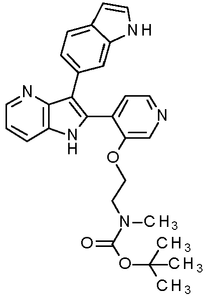 **tert-butyl [2-({4-[3-(1H-indol-6-yl)-1H-pyrrolo[3,2-b]pyridin-2-yl]pyridin-3-yl}oxy)ethyl]methylcarbamate** | method 2 |
| | | Rₜ = 1.29 min |
| | | m/z = 484 [M+H]⁺ |
| **57** | 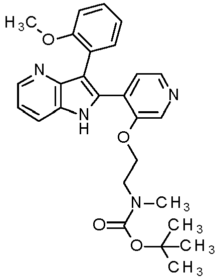 **tert-butyl [2-({4-[3-(2-methoxyphenyl)-1H-pyrrolo[3,2-b]pyridin-2-yl]pyridin-3-yl}oxy)ethyl]methylcarbamate** | method 2 |
| | | Rₜ = 1.24 min |
| | | m/z = 475 [M+H]⁺ |
| **58** | 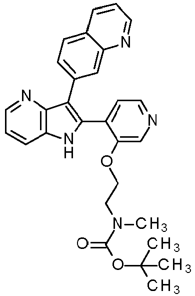 **tert-butyl methyl[2-({4-[3-(quinolin-7-yl)-1H-pyrrolo[3,2-b]pyridin-2-yl]pyridin-3-yl}oxy)ethyl]carbamate** | method 2 |
| | | Rₜ = 1.25 min |
| | | m/z = 496 [M+H]⁺ |
| **59** | 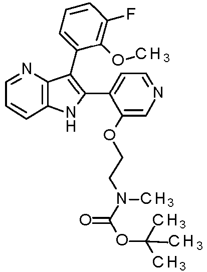 **tert-butyl [2-({4-[3-(3-fluoro-2-methoxyphenyl)-1H-pyrrolo[3,2-b]pyridin-2-yl]pyridin-3-yl}oxy)ethyl]methylcarbamate** | method 1 |
| | | Rₜ = 1.30 min |
| | | m/z = 493 [M+H]⁺ |
| **60** | 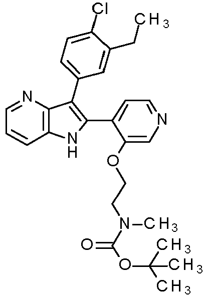 **tert-butyl [2-({4-[3-(4-chloro-3-ethylphenyl)-1H-pyrrolo[3,2-b]pyridin-2-yl]pyridin-3-yl}oxy)ethyl]methylcarbamate** | method 1 |
| | | Rₜ = 1.79 min |
| | | m/z = 505 [M-H]⁻ |
| **61** | 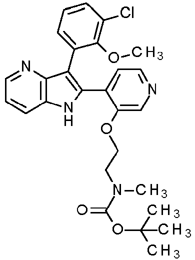 **tert-butyl [2-({4-[3-(3-chloro-2-methoxyphenyl)-1H-pyrrolo[3,2-b]pyridin-2-yl]pyridin-3-yl}oxy)ethyl]methylcarbamate** | method 1 |
| | | Rₜ = 1.37 min |
| | | m/z = 509 [M+H]⁺ |
| **62** | 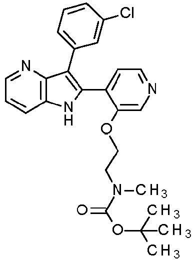 **tert-butyl [2-({4-[3-(3-chlorophenyl)-1H-pyrrolo[3,2-b]pyridin-2-yl]pyridin-3-yl}oxy)ethyl]methylcarbamate** | method 2 |
| | | Rₜ = 1.71 min |
| | | m/z = 479 [M+H]⁺ |
| **63** | 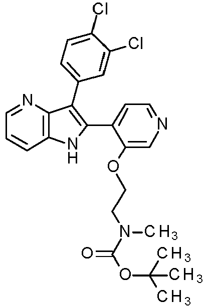 **tert-butyl [2-({4-[3-(3,4-dichlorophenyl)-1H-pyrrolo[3,2-b]pyridin-2-yl]pyridin-3-yl}oxy)ethyl]methylcarbamate** | method 2 |
| | | Rₜ = 1.97 min |
| | | m/z = 513 [M+H]⁺ |
| **64** | 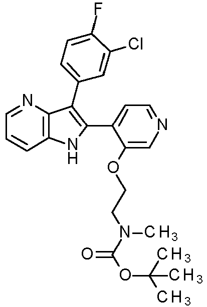 **tert-butyl [2-({4-[3-(3-chloro-4-fluorophenyl)-1H-pyrrolo[3,2-b]pyridin-2-yl]pyridin-3-yl}oxy)ethyl]methylcarbamate** | method 2 |
| | | Rₜ = 1.77 min |
| | | m/z = 497 [M+H]⁺ |

### Intermediate 65

### N-methyl-2-({4-[3-(3-methylphenyl)-1H-pyrrolo[3,2-b]pyridin-2-yl]pyridin-3-yl}oxy)ethan-1-amine

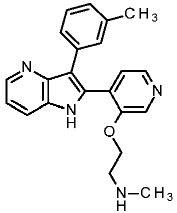

To a solution of tert-butyl methyl[2-({4-[3-(3-methylphenyl)-1H-pyrrolo[3,2-b]pyridin-2-yl]pyridin-3-yl}oxy)ethyl]carbamate (56.0 mg, 122 µmol) in DCM (1 ml), TFA (94 µl, 1.2 mmol) was added at rt. After stirring for 48 h, saturated aqueous sodium hydrogencarbonate solution (1 ml) was added. The mixture was diluted with DCM (5 ml) and dried over a water repellent filter. After concentration under reduced pressure and drying in vacuo 30.0 mg (100 % purity, 69 % yield) of the title compound were obtained that were used without further purification.
LC-MS (method 1): Rₜ = 0.55min; MS (ESlpos): m/z = 359 [M+H]⁺

The intermediates listed in the following table were prepared in an analogous manner to intermediate 65.

| **Intermediate** | **Structure IUPAC-Name** | **LC-MS method Retention time Mass found** |
|---|---|---|
| **66** | 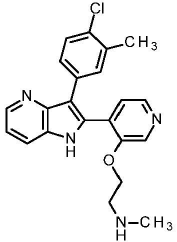 **2-({4-[3-(4-chloro-3-methylphenyl)-1H-pyrrolo[3,2-b]pyridin-2-yl]pyridin-3-yl}oxy)-N-methylethan-1-amine** | method 1 |
| | | Rₜ = 0.78 min |
| | | m/z = 391 [M-H]- |
| **67** | 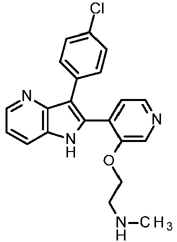 **2-({4-[3-(4-chlorophenyl)-1H-pyrrolo[3,2-b]pyridin-2-yl]pyridin-3-yl}oxy)-N-methylethan-1-amine** | method 1 |
| | | Rₜ = 0.69 min |
| | | m/z = 377 [M-H]- |
| **68** | 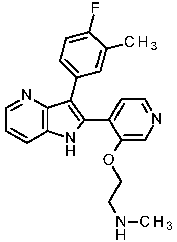 **2-({4-[3-(4-fluoro-3-methylphenyl)-1H-pyrrolo[3,2-b]pyridin-2-yl]pyridin-3-yl}oxy)-N-methylethan-1-amine** | method 1 |
| | | Rₜ = 0.63 min |
| | | m/z = 375 [M-H]- |
| **69** | 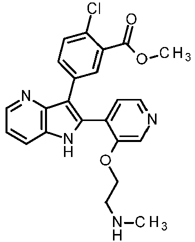 **methyl 2-chloro-5-(2-{3-[2-(methylamino)ethoxy]pyridin-4-yl}-1H-pyrrolo[3,2-b]pyridin-3-yl)benzoate** | method 1 |
| | | Rₜ = 0.70 min |
| | | m/z = 435 [M-H]- |
| **70** | 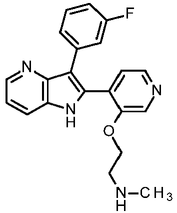 **2-({4-[3-(3-fluorophenyl)-1H-pyrrolo[3,2-b]pyridin-2-yl]pyridin-3-yl}oxy)-N-methylethan-1-amine** | method 1 |
| | | Rₜ = 0.59 min |
| | | m/z = 361 [M-H]- |
| **71** | 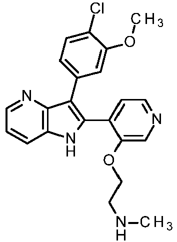 **2-({4-[3-(4-chloro-3-methoxyphenyl)-1H-pyrrolo[3,2-b]pyridin-2-yl]pyridin-3-yl}oxy)-N-methylethan-1-amine** | method 2 |
| | | Rₜ = 0.78 min |
| | | m/z = 407 [M-H]- |
| **72** | 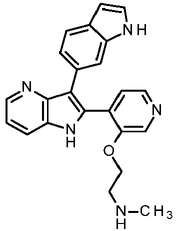 **2-({4-[3-(1H-indol-6-yl)-1H-pyrrolo[3,2-b]pyridin-2-yl]pyridin-3-yl}oxy)-N-methylethan-1-amine** | method 1 |
| | | Rₜ = 0.57 min |
| | | m/z = 382 [M-H]- |
| **73** | 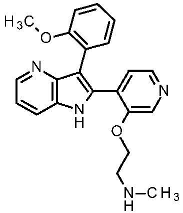 **2-({4-[3-(2-methoxyphenyl)-1H-pyrrolo[3,2-b]pyridin-2-yl]pyridin-3-yl}oxy)-N-methylethan-1-amine** | method 2 |
| | | Rₜ = 0.36 min |
| | | m/z = 375 [M+H]⁺ |
| **74** | 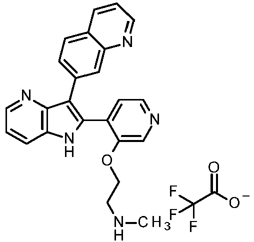 **N-methyl-2-({4-[3-(quinolin-7-yl)-1H-pyrrolo[3,2-b]pyridin-2-yl]pyridin-3-yl}oxy)ethanamine trifluoroacetate (1:1)** | method 1 |
| | | Rₜ = 0.53 min |
| | | m/z = 394 [M-H]⁻ |
| **75** | 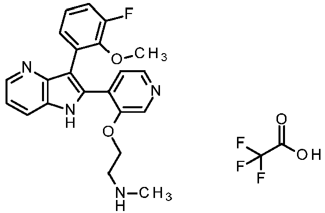 **2-({4-[3-(3-fluoro-2-methoxyphenyl)-1H-pyrrolo[3,2-b]pyridin-2-yl]pyridin-3-yl}oxy)-N-methylethanamine trifluoroacetate (1:1)** | method 1 |
| | | Rₜ = 0.53 min |
| | | m/z = 391 [M-H]⁻ |
| **76** | 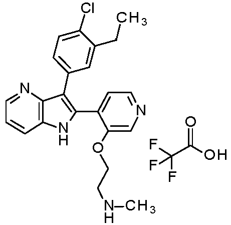 **2-({4-[3-(4-chloro-3-ethylphenyl)-1H-pyrrolo[3,2-b]pyridin-2-yl]pyridin-3-yl}oxy)-N-methylethanamine trifluoroacetate (1:1)** | method 2 |
| | | Rₜ = 0.95 min |
| | | m/z = 405 [M+H]⁺ |
| **77** | 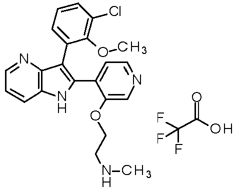 **2-({4-[3-(3-chloro-2-methoxyphenyl)-1H-pyrrolo[3,2-b]pyridin-2-yl]pyridin-3-yl}oxy)-N-methylethanamine trifluoroacetate (1:1)** | method 1 |
| | | Rₜ = 0.61 min |
| | | m/z = 407 [M-H]⁻ |
| **78** | 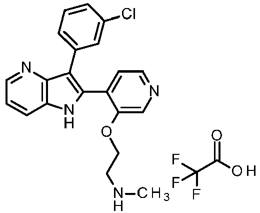 **2-({4-[3-(3-chlorophenyl)-1H-pyrrolo[3,2-b]pyridin-2-yl]pyridin-3-yl}oxy)-N-methylethanamine trifluoroacetate (1:1)** | method 1 |
| | | Rₜ = 0.68 min |
| | | m/z = 377 [M-H]⁻ |
| **79** | 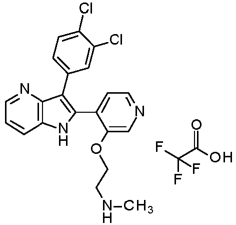 **2-((4-[3-(3,4-dichlorophenyl)-1H-pyrrolo[3,2-b]pyridin-2-yl]pyridin-3-yl}oxy)-N-methylethanamine trifluoroacetate (1:1)** | method 1 |
| | | Rₜ = 0.90 min |
| | | m/z = 411 [M-H]⁻ |
| **80** | 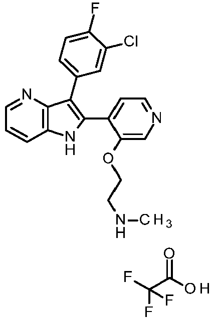 **2-({4-[3-(3-chloro-4-fluorophenyl)-1H-pyrrolo[3,2-b]pyridin-2-yl]pyridin-3-yl}oxy)-N-methylethanamine trifluoroacetate (1:1)** | method 1 |
| | | Rₜ = 0.76 min |
| | | m/z = 395 [M-H]⁻ |

### Intermediate 81

### 2-({4-[3-(1H-indol-6-yl)-1H-pyrrolo[3,2-b]pyridin-2-yl]pyridin-3-yl}oxy)-N-methylethanamine hydrochloride (1:1)

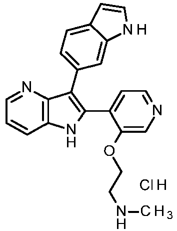

To a solution of tert-butyl [2-({4-[3-(1H-indol-6-yl)-1H-pyrrolo[3,2-b]pyridin-2-yl]pyridin-3-yl}oxy)ethyl]methylcarbamate (126 mg, 261 µmol) in DCM (2 ml) HCl (650 µl, 4.0 M in 1,4-dioxane, 2.6 mmol) was added at rt. Stirring was continued for 18 h. The solvent was removed under reduced pressure and the remaining residue was dried in vacuo. 151 mg (74 % purity, 102 % yield) of the title compound were obtained that were used without further purification.
LC-MS (method 1): Rₜ = 0.57 min; MS (ESlpos): m/z = 384 [M+H]⁺

### Intermediate 82

### 2-({4-[3-(3-chlorophenyl)-1H-pyrrolo[3,2-b]pyridin-2-yl]pyridin-3-yl}oxy)-N-methylethanamine hydrochloride (1:1)

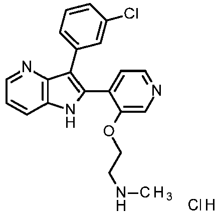

To a solution of tert-butyl [2-({4-[3-(3-chlorophenyl)-1H-pyrrolo[3,2-b]pyridin-2-yl]pyridin-3-yl}oxy)ethyl]methylcarbamate (140 mg, 292 µmol) in DCM (2 ml) HCl (730 µl, 4.0 M in 1,4-dioxane, 2.9 mmol) was added at rt. Stirring was continued for 18 h. The solvent was removed under reduced pressure and the remaining residue was dried in vacuo. 164 mg (84 % purity, 113 % yield) of the title compound were obtained that were used without further purification.
LC-MS (method 1): Rₜ = 0.72 min; MS (ESlneg): m/z = 377 [M-H]⁻

### Intermediate 83

### 2-{[4-(3-bromo-1H-pyrrolo[3,2-b]pyridin-2-yl)pyridin-3-yl]oxy}-N-methylethanamine hydrochloride (1:1)

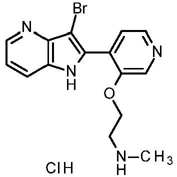

To a solution of tert-butyl (2-{[4-(3-bromo-1H-pyrrolo[3,2-b]pyridin-2-yl)pyridin-3-yl]oxy}ethyl)methylcarbamate (308 mg, 689 µmol) in DCM (5 ml) HCl (1.7 ml, 4.0 M in 1,4-dioxane, 6.9 mmol) was added at rt. To improve stirring of the thick suspension, MeOH (0.5 ml) were added. Stirring was continued for 1 h. The solvent was removed under reduced pressure and the remaining residue was dried in vacuo. 388 mg (92 % purity, 135 % yield) of the title compound were obtained that were used without further purification.
LC-MS (method 2): Rₜ = 0.39 min; MS (ESlpos): m/z = 347 [M+H]⁺

### Intermediate 84

### N-(2-{[4-(3-bromo-1H-pyrrolo[3,2-b]pyridin-2-yl)pyridin-3-yl]oxy}ethyl)-N-methylprop-2-enamide

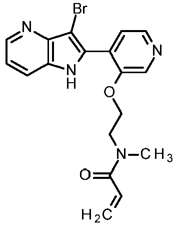

To a solution of 2-{[4-(3-bromo-1H-pyrrolo[3,2-b]pyridin-2-yl)pyridin-3-yl]oxy}-N-methylethanamine hydrochloride (1:1) (100 mg, 92 % purity, 240 µmol) in DMF (1 ml), prop-2-enoic acid (16 µl, 240 µmol) and N,N-diisopropylethylamine (260 µl, 1.4 mmol) were added at rt. T3P (210 µl, 50 % purity in DMF, 360 µmol; CAS-RN:[68957-94-8]) was added and stirring at rt was continued for 1 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 31.0 mg (100 % purity, 32 % yield) of the desired product.
LC-MS (method 1): Rₜ = 0.82 min; MS (ESlneg): m/z = 399 [M-H]⁻

### Intermediate 85

### tert-butyl {2-[(4-bromopyridin-3-yl)oxy]ethyl}ethylcarbamate

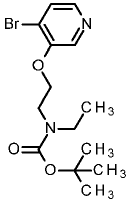

To a solution of 4-bromopyridin-3-ol (1.15 g, 6.60 mmol) in THF (17 ml), tert-butyl ethyl(2-hydroxyethyl)carbamate (1.50 g, 7.93 mmol) and triphenylphosphine (2.60 g, 9.91 mmol were added. The mixture was cooled to 0°C and dipropan-2-yl (E)-diazene-1,2-dicarboxylate (2.0 ml, 98 % purity, 9.9 mmol) was added slowly. The mixture was stirred at rt for 1 h. Water (3 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding the desired product in a mixture with triphenylphosphine oxide. This material was further purified by reverse phase preparative HPLC (method 3) yielding 1.15 g (100 % purity, 86 % yield) of the title compound.
LC-MS (method 2): Rₜ = 2.05 min; MS (ESlpos): m/z = 345 [M+H]⁺

### Intermediate 86

### tert-butyl ethyl[2-({4-[(trimethylsilyl)ethynyl]pyridin-3-yl}oxy)ethyl]carbamate

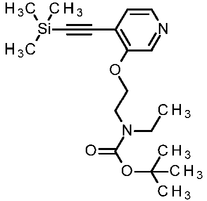

To a solution of tert-butyl {2-[(4-bromopyridin-3-yl)oxy]ethyl}ethylcarbamate (1.15 g, 3.33 mmol), ethynyl(trimethyl)silane (920 µl, 6.7 mmol) and Cu(I)I (46.8 mg, 133.2 µmol in DMF (6 ml) triethylamine (4.4 ml) was added at rt. The mixture was carefully degassed and purged with argon. Dichlorobis(triphenylphosphine)palladium(ll) (46.8 mg, 66.6 µmol; CAS-RN:[13965-03-2]) was added and the reaction mixture was heated to 80°C for 1h. After cooling to rt, water (10 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 1.05 g (94 % purity, 82 % yield) of the desired product.
LC-MS (method 2): Rₜ = 2.53 min; MS (ESlpos): m/z = 363 [M+H]⁺

### Intermediate 87

### tert-butyl [2-({4-[(3-aminopyridin-2-yl)ethynyl]pyridin-3-yl}oxy)ethyl]ethylcarbamate

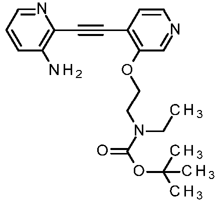

To a solution of tert-butyl ethyl[2-({4-[(trimethylsilyl)ethynyl]pyridin-3-yl}oxy)ethyl]carbamate (1.05 g, 2.90 mmol), 2-bromopyridin-3-amine (501 mg, 2.90 mmol), Cu(I)I (46.8 mg, 133.2 µmol and dichlorobis(triphenylphosphine)palladium(ll) (102 mg, 145 µmol; CAS-RN:[13965-03-2]) in DMF (4 ml) triethylamine (2.4 ml) and TBAF (2.9 ml, 1.0 M in THF, 2.9 mmol) were added at rt under argon. The reaction mixture was heated to 100°C for 40 min. After cooling to rt, water (10 ml) and EtOAc (10 ml) were added and the mixture was filtered through a pad of celite. The aqueous layer was extracted with EtOAc four times. The combined organic layers were dried over sodium sulfate, filtered and concentrated under reduced pressure. The residue was purified by silica gel column chromatography (eluent: DCM/MeOH 20:1) yielding 603 mg (100 % purity, 54 % yield) of the desired product.
LC-MS (method 2): Rₜ = 1.64 min; MS (ESlpos): m/z = 383 [M+H]⁺

### Intermediate 88

### tert-butyl ethyl(2-{[4-(1H-pyrrolo[3,2-b]pyridin-2-yl)pyridin-3-yl]oxy}ethyl)carbamate

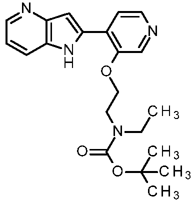

A solution of tert-butyl [2-({4-[(3-aminopyridin-2-yl)ethynyl]pyridin-3-yl}oxy)ethyl]ethylcarbamate (603 mg, 1.58 mmol) in NMP (4 ml) was carefully degassed and purged with argon. Potassium tert-butoxide (354 mg, 3.15 mmol) was added at rt and the mixture was heated to 90°C for 30 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 475 mg (100 % purity, 79 % yield) of the desired product.
LC-MS (method 1): Rₜ = 1.19 min; MS (ESlneg): m/z = 381 [M-H]⁻

### Intermediate 89

### tert-butyl (2-{[4-(3-bromo-1H-pyrrolo[3,2-b]pyridin-2-yl)pyridin-3-yl]oxy}ethyl)ethylcarbamate

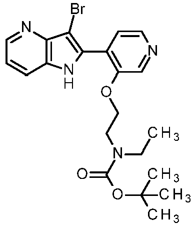

To a solution of tert-butyl ethyl(2-{[4-(1H-pyrrolo[3,2-b]pyridin-2-yl)pyridin-3-yl]oxy}ethyl)carbamate (470 mg, 1.23 mmol) in DCM (3 ml), 1-bromopyrrolidine-2,5-dione (219 mg, 1.23 mmol; CAS-RN:[128-08-5]) was added at 0°C. After warming to rt within 30 min stirring at rt was continued for 1 h. Saturated sodium hydrogencarbonate solution (aqueous, 1 mL) was added and the mixture diluted with DCM. The mixture was dried over a water repellent filter and concentrated under reduced pressure. The residue was dried in vacuo to yield 570 mg (97 % purity, 98 % yield) of the desired product which was used without further purification
LC-MS (method 2): Rₜ = 1.72 min; MS (ESlpos): m/z = 461 [M+H]⁺

### Intermediate 90

### tert-butyl ethyl(2-{[4-(3-phenyl-1H-pyrrolo[3,2-b]pyridin-2-yl)pyridin-3-yl]oxy}ethyl)carbamate

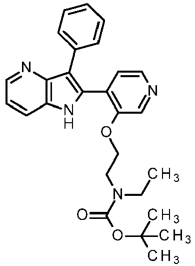

A solution of tert-butyl (2-{[4-(3-bromo-1H-pyrrolo[3,2-b]pyridin-2-yl)pyridin-3-yl]oxy}ethyl)ethylcarbamate (300 mg, 650 µmol) and phenylboronic acid (119 mg, 975 µmol) in a mixture of 1-propanole and water (5:1, 6 ml) was carefully degassed and purged with argon. Triphenylphosphine (17.1 mg, 65.0 µmol), potassium carbonate (270 mg, 1.95 mmol) and the palladium catalyst bis(triphenylphosphine)palladium(II) dichloride(45.6 mg, 65.0 µmol; CAS-RN:[13965-03-2]) were added. The mixture was heated to 100°C and stirred for 18 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 145 mg (97 % purity, 47 % yield) of the desired product.
LC-MS (method 1): Rₜ = 1.39 min; MS (ESlpos): m/z = 459 [M+H]⁺

### Intermediate 91

### N-ethyl-2-{[4-(3-phenyl-1H-pyrrolo[3,2-b]pyridin-2-yl)pyridin-3-yl]oxy}ethanamine hydrochloride (1:1)

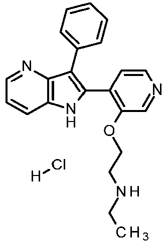

To a solution of tert-butyl ethyl(2-{[4-(3-phenyl-1H-pyrrolo[3,2-b]pyridin-2-yl)pyridin-3-yl]oxy}ethyl)carbamate (144 mg, 314 µmol) in DCM (1 ml), HCl (630 µl, 4.0 M in 1,4-dioxane, 2.5 mmol) was added at rt. Stirring was continued for 18 h. The solvent was removed under reduced pressure and the remaining residue was dried in vacuo. 166 mg (95 % purity, 127 % yield) of the title compound were obtained that were used without further purification.
LC-MS (method 1): Rₜ = 0.52 min; MS (ESlpos): m/z = 357 [M-H]⁻

### Intermediate 92

### tert-butyl (2S)-2-{[(4-bromopyridin-3-yl)oxy]methyl}pyrrolidine-1-carboxylate

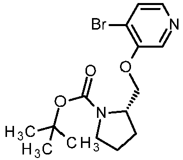

To a solution of 4-bromopyridin-3-ol (3.00 g, 17.2 mmol) in THF (50 ml), tert-butyl (2S)-2-(hydroxymethyl)pyrrolidine-1-carboxylate (4.16 g, 20.7 mmol) and triphenylphosphine (6.78 g, 25.9 mmol) were added. The mixture was cooled to 0°C and dipropan-2-yl (E)-diazene-1,2-dicarboxylate (5.2 ml, 98 % purity, 26 mmol) was added slowly. The mixture was stirred at rt for 3 h. Water (20 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 5.19 g (87 % purity, 73 % yield) of the desired product.
LC-MS (method 2): Rₜ = 2.06 min; MS (ESlpos): m/z = 357 [M+H]⁺

### Intermediate 93

### tert-butyl (2S)-2-[({4-[(trimethylsilyl)ethynyl]pyridin-3-yl}oxy)methyl]pyrrolidine-1-carboxylate

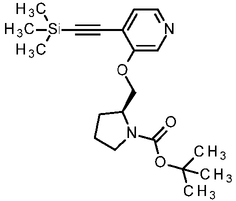

To a solution of tert-butyl (2S)-2-{[(4-bromopyridin-3-yl)oxy]methyl}pyrrolidine-1-carboxylate (5.10 g, 14.3 mmol), ethynyl(trimethyl)silane (4.0 ml, 29 mmol) and Cu(I)I (109 mg, 571 µmol) in DMF (18 ml) triethylamine (19 ml) was added at rt. The mixture was carefully degassed and purged with argon. Dichlorobis(triphenylphosphine)palladium(II) (200 mg, 286 µmol; CAS-RN:[13965-03-2]) was added and the reaction mixture was heated to 80°C for 3 h. After cooling to rt, water (10 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 4.65 g (87 % purity, 76 % yield) of the desired product.
LC-MS (method 2): Rₜ = 2.51 min; MS (ESlpos): m/z = 375 [M+H]⁺

### Intermediate 94

### tert-butyl (2S)-2-[({4-[(3-aminopyridin-2-yl)ethynyl]pyridin-3-yl}oxy)methyl]pyrrolidine-1-carboxylate

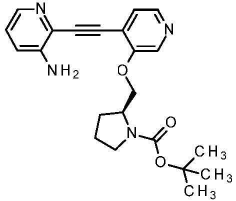

To a solution of tert-butoxy{(2S)-2-[({4-[(trimethylsilyl)ethynyl]pyridin-3-yl}oxy)methyl]pyrrolidin-1-yl}methanol (4.64 g, 12.3 mmol), 2-bromopyridin-3-amine (2.13 g, 12.3 mmol), Cu(I)I (117 mg, 616 µmol) and dichlorobis(triphenylphosphine)palladium(ll) (432 mg, 616 µmol; CAS-RN:[13965-03-2]) in DMF (10 ml) triethylamine (10 ml) and TBAF (12 ml, 1.0 M in THF, 12 mmol) were added at rt under argon. The reaction mixture was heated to 100°C for 40 min. After cooling to rt, water (10 ml) and EtOAc (10 ml) were added and the mixture was filtered through a pad of celite. The aqueous layer was extracted with EtOAc four times. The combined organic layers were dried over sodium sulfate, filtered and concentrated under reduced pressure. The residue was purified by silica gel column chromatography (eluent: DCM/MeOH 40:1) yielding 3.13 g (79 % purity, 51 % yield) of the desired product.
LC-MS (method 1): Rₜ = 1.56 min; MS (ESlpos): m/z = 395 [M+H]⁺

### Intermediate 95

### tert-butyl (2S)-2-({[4-(1H-pyrrolo[3,2-b]pyridin-2-yl)pyridin-3-yl]oxy}methyl)pyrrolidine-1-carboxylate

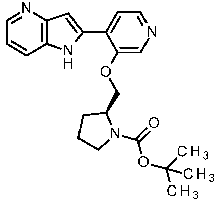

A solution of tert-butyl (2S)-2-[({4-[(3-aminopyridin-2-yl)ethynyl]pyridin-3-yl}oxy)methyl]pyrrolidine-1-carboxylate (3.13 g, 7.93 mmol) in NMP (20 ml) was carefully degassed and purged with argon. Potassium tert-butoxide (1.78 g, 15.9 mmol) was added at rt and the mixture was heated to 90°C for 30 min. After cooling to rt, water (5 ml) was added, and the mixture was extracted with EtOAc twice. The combined organic layers were dried over sodium sulfate, filtered, and concentrated under reduced pressure. The residue was purified by silica gel column chromatography (eluent: DCM/MeOH 20:1) yielding 2.19 g (98 % purity, 69 % yield) of the desired product.
LC-MS (method 2): Rₜ = 1.26 min; MS (ESlpos): m/z = 395 [M+H]⁺

### Intermediate 96

### tert-butyl (2S)-2-({[4-(3-bromo-1H-pyrrolo[3,2-b]pyridin-2-yl)pyridin-3-yl]oxy}methyl)pyrrolidine-1-carboxylate

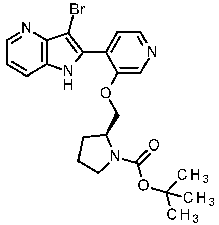

To a solution of tert-butyl (2S)-2-({[4-(1H-pyrrolo[3,2-b]pyridin-2-yl)pyridin-3-yl]oxy}methyl)pyrrolidine-1-carboxylate (2.19 g, 5.55 mmol) in DCM (10 ml), 1-bromopyrrolidine-2,5-dione (988 mg, 5.55 mmol) was added at 0°C. After warming to rt within 30 min stirring at rt was continued for 1 h. Saturated sodium hydrogencarbonate solution (aqueous, 1 mL) was added and the mixture diluted with DCM. The mixture was dried over a water repellent filter and concentrated under reduced pressure. The residue was purified by reverse phase preparative HPLC (method 3) yielding 1.94 g (90 % purity, 66 % yield) of the desired product.
LC-MS (method 2): Rₜ = 1.69 min; MS (ESlpos): m/z = 473 [M+H]⁺

### Intermediate 97

### tert-butyl (2S)-2-({[4-(3-phenyl-1H-pyrrolo[3,2-b]pyridin-2-yl)pyridin-3-yl]oxy}methyl)pyrrolidine-1-carboxylate

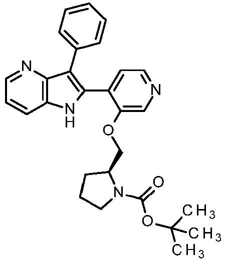

A solution of tert-butyl (2S)-2-({[4-(3-bromo-1H-pyrrolo[3,2-b]pyridin-2-yl)pyridin-3-yl]oxy}methyl)pyrrolidine-1-carboxylate (2.00 g, 4.22 mmol) and phenylboronic acid (773 mg, 6.34 mmol) in a mixture of 1-propanole and water (5:1, 42 ml) was carefully degassed and purged with argon. Triphenylphosphine (111 mg, 422 µmol), potassium carbonate (1.75 g, 12.7 mmol) and the palladium catalyst bis(triphenylphosphine)palladium(ll) dichloride(297 mg, 422 µmol; CAS-RN:[13965-03-2]) were added. The mixture was heated to 100°C and stirred for 4.5 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 1.29 g (100 % purity, 65 % yield) of the desired product.
LC-MS (method 2): Rₜ = 1.50 min; MS (ESlpos): m/z = 471 [M+H]⁺

### Intermediate 98

### 3-phenyl-2-(3-{[(2S)-pyrrolidin-2-yl]methoxy}pyridin-4-yl)-1H-pyrrolo[3,2-b]pyridine-hydrogen chloride (1/1)

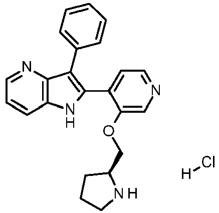

To a solution of tert-butyl (2S)-2-({[4-(3-phenyl-1H-pyrrolo[3,2-b]pyridin-2-yl)pyridin-3-yl]oxy}methyl)pyrrolidine-1-carboxylate (19.5 mg, 41.4 µmol) in DCM (1 ml), HCl (52 µl, 4.0 M in 1,4-dioxane, 210 µmol) was added at rt. Stirring was continued for 18 h. The solvent was removed under reduced pressure and the remaining residue was dried in vacuo. 23.6 mg (94 % purity, 132 % yield) of the title compound were obtained that were used without further purification.
LC-MS (method 2): Rₜ = 0.62min; MS (ESlpos): m/z = 369 [M+H]⁺

### Intermediate 99

### tert-butyl (2R)-2-{[(4-bromopyridin-3-yl)oxy]methyl}pyrrolidine-1-carboxylate

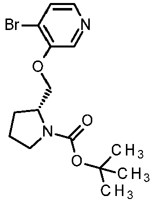

To a solution of 4-bromopyridin-3-ol (648 mg, 3.73 mmol) in THF (10 ml), tert-butyl (2R)-2-(hydroxymethyl)pyrrolidine-1-carboxylate (900 mg, 4.47 mmol) and triphenylphosphine (1.47 g, 5.59 mmol) were added. The mixture was cooled to 0°C and dipropan-2-yl (E)-diazene-1,2-dicarboxylate (1.1 ml, 98 % purity, 5.6 mmol) was added slowly. The mixture was stirred at rt for 1 h. Water (5 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 652 mg (100 % purity, 49 % yield) of the desired product.
LC-MS (method 1): Rₜ = 1.99 min; MS (ESlpos): m/z = 357 [M+H]⁺

### Intermediate 100

### tert-butyl (2R)-2-[({4-[(trimethylsilyl)ethynyl]pyridin-3-yl}oxy)methyl]pyrrolidine-1-carboxylate

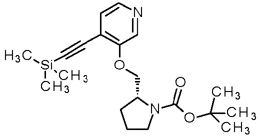

To a solution of tert-butyl (2R)-2-{[(4-bromopyridin-3-yl)oxy]methyl}pyrrolidine-1-carboxylate (650 mg, 1.82 mmol), ethynyl(trimethyl)silane (500 µl, 3.6 mmol) and Cu(I)I (13.9 mg, 73 µmol) in DMF (5 ml) triethylamine (2.4 ml) was added at rt. The mixture was carefully degassed and purged with argon. Dichlorobis(triphenylphosphine)palladium(ll) (25.5 mg, 36.4 µmol; CAS-RN:[13965-03-2]) was added and the reaction mixture was heated to 80°C for 1.5 h. After cooling to rt, water (10 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 415 mg (83 % purity, 51 % yield) of the desired product.
LC-MS (method 1): Rₜ = 2.46min; MS (ESlpos): m/z = 375 [M+H]⁺

### Intermediate 101

### tert-butyl (2R)-2-[({4-[(3-aminopyridin-2-yl)ethynyl]pyridin-3-yl}oxy)methyl]pyrrolidine-1-carboxylate

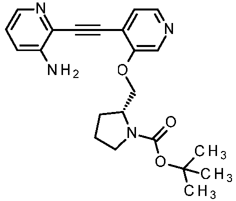

To a solution of tert-butyl (2R)-2-[({4-[(trimethylsilyl)ethynyl]pyridin-3-yl}oxy)methyl]pyrrolidine-1-carboxylate (415 mg, 1.11 mmol), 2-bromopyridin-3-amine (192 mg, 1.11 mmol), Cu(I)I (10.6 mg, 55 µmol) and dichlorobis(triphenylphosphine)palladium(ll) (38.9 mg, 55.4 µmol; CAS-RN:[13965-03-2]) in DMF (5 ml) triethylamine (10 ml) and TBAF (1.1 ml, 1.0 M in THF, 1.1 mmol]) were added at rt under argon. The reaction mixture was heated to 100°C for 30 min. After cooling to rt, water (10 ml) and EtOAc (10 ml) were added and the mixture was filtered through a pad of celite. The aqueous layer was extracted with EtOAc four times. The combined organic layers were dried over sodium sulfate, filtered and concentrated under reduced pressure. The residue was purified by silica gel column chromatography (eluent: DCM/MeOH 40:1) yielding 322 mg (76 % purity, 56 % yield) of the desired product.
LC-MS (method 2): Rₜ = 1.65 min; MS (ESlpos): m/z = 395 [M+H]⁺

### Intermediate 102

### tert-butyl (2R)-2-({[4-(1H-pyrrolo[3,2-b]pyridin-2-yl)pyridin-3-yl]oxy}methyl)pyrrolidine-1-carboxylate

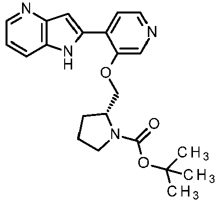

A solution of tert-butyl (2R)-2-[({4-[(3-aminopyridin-2-yl)ethynyl]pyridin-3-yl}oxy)methyl]pyrrolidine-1-carboxylate (320 mg, 811 µmol) in NMP (8 ml) was carefully degassed and purged with argon. Potassium tert-butoxide (182 mg, 1.62 mmol) was added at rt and the mixture was heated to 90°C for 45 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 157 mg (97 % purity, 48 % yield) of the desired product.
LC-MS (method 1): Rₜ = 1.17 min; MS (ESlpos): m/z = 395 [M+H]⁺

### Intermediate 103

### tert-butyl (2R)-2-({[4-(3-bromo-1H-pyrrolo[3,2-b]pyridin-2-yl)pyridin-3-yl]oxy}methyl)pyrrolidine-1-carboxylate

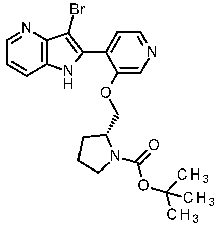

To a solution of tert-butyl (2R)-2-({[4-(1H-pyrrolo[3,2-b]pyridin-2-yl)pyridin-3-yl]oxy}methyl)pyrrolidine-1-carboxylate (157 mg, 398 µmol) in DCM (2 ml), 1-bromopyrrolidine-2,5-dione (70.8 mg, 398 µmol) was added at 0°C. After warming to rt within 30 min stirring at rt was continued for 1 h. Saturated sodium hydrogencarbonate solution (aqueous, 1 mL) was added and the mixture diluted with DCM. The mixture was dried over a water repellent filter and concentrated under reduced pressure. The residue was dried in vacuo yielding 188 mg (96 % purity, 96 % yield) of the desired product that was used without further purification.
LC-MS (method 1): Rₜ = 1.61 min; MS (ESlpos): m/z = 473 [M+H]⁺

### Intermediate 104

### tert-butyl (2R)-2-({[4-(3-phenyl-1H-pyrrolo[3,2-b]pyridin-2-yl)pyridin-3-yl]oxy}methyl)pyrrolidine-1-carboxylate

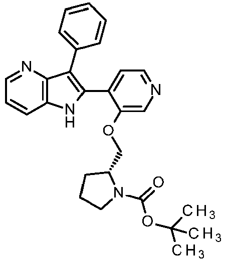

A solution of tert-butyl (2R)-2-({[4-(3-bromo-1H-pyrrolo[3,2-b]pyridin-2-yl)pyridin-3-yl]oxy}methyl)pyrrolidine-1-carboxylate (188 mg, 397 µmol) and phenylboronic acid (72.6 mg, 596 µmol) in a mixture of 1-propanole and water (5:1, 6 ml) was carefully degassed and purged with argon. Triphenylphosphine (10.4 mg, 39.7 µmol), potassium carbonate (165 mg, 1.19 mmol) and the palladium catalyst bis(triphenylphosphine)palladium(ll) dichloride (27.9 mg, 39.7 µmol; CAS-RN:[13965-03-2]) were added. The mixture was heated to 100°C and stirred for 3 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 136 mg (93 % purity, 68 % yield) of the desired product.
LC-MS (method 1): Rₜ = 1.41 min; MS (ESlpos): m/z = 471 [M+H]⁺
¹H-NMR (400 MHz, DMSO-d6) δ [ppm]: 1.347 (16.00), 1.399 (0.39), 1.481 (0.83), 1.634 (0.24), 2.936 (0.24), 3.057 (0.34), 3.296 (0.24), 3.373 (0.23), 3.851 (0.29), 3.970 (0.18), 4.067 (0.18), 4.103 (0.21), 5.751 (5.96), 7.168 (0.32), 7.187 (0.77), 7.198 (1.13), 7.209 (1.26), 7.219 (0.99), 7.230 (0.96), 7.270 (0.64), 7.288 (1.11), 7.364 (0.25), 7.538 (1.18), 7.557 (1.00), 7.808 (0.80), 7.826 (0.76), 8.251 (0.29), 8.287 (0.27), 8.411 (0.88), 8.414 (0.93), 8.422 (0.93), 8.425 (0.85), 8.540 (0.36), 8.566 (0.41), 11.711 (1.40).

### Intermediate 105

### 3-phenyl-2-{3-[(2R)-pyrrolidin-2-ylmethoxy]pyridin-4-yl}-1H-pyrrolo[3,2-b]pyridine hydrochloride (1:1)

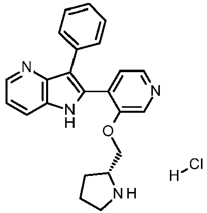

To a solution of tert-butyl (2R)-2-({[4-(3-phenyl-1H-pyrrolo[3,2-b]pyridin-2-yl)pyridin-3-yl]oxy}methyl)pyrrolidine-1-carboxylate (135 mg, 287 µmol) in DCM (1.5 ml), HCl (570 µl, 4.0 M in 1,4-dioxane, 2.3 mmol) was added at rt. Stirring was continued for 18 h. The solvent was removed under reduced pressure and the remaining residue was dried in vacuo. 135 mg (100 % purity, 116 % yield) of the title compound were obtained that were used without further purification.
LC-MS (method 1): Rₜ = 0.53 min; MS (ESlneg): m/z = 369 [M-H]⁻
¹H-NMR (400 MHz, DMSO-d6) δ [ppm]: 13.78 (s, 1H), 13.89-13.64 (m, 1H), 10.02-9.46 (m, 2H), 8.80-8.69 (m, 2H), 8.67-8.60 (m, 1H), 8.40-8.29 (m, 1H), 7.85-7.72 (m, 1H), 7.52-7.37 (m, 5H), 7.32-7.18 (m, 1H), 4.53-4.34 (m, 2H), 3.54-3.40 (m, 2H), 3.18-3.04 (m, 2H), 2.11-1.94 (m, 1H), 1.87-1.60 (m, 3H).

### Intermediate 106

### tert-butyl (2S)-2-{[(4-{3-[3-(trifluoromethyl)phenyl]-1H-pyrrolo[3,2-b]pyridin-2-yl}pyridin-3-yl)oxy]methyl}pyrrolidine-1-carboxylate

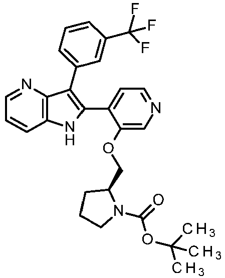

A solution of tert-butyl (2S)-2-({[4-(3-bromo-1H-pyrrolo[3,2-b]pyridin-2-yl)pyridin-3-yl]oxy}methyl)pyrrolidine-1-carboxylate (300 mg, 634 µmol) and [3-(trifluoromethyl)phenyl]boronic acid (181 mg, 951 µmol) in a mixture of 1-propanole and water (5:1, 4 ml) was carefully degassed and purged with argon. Triphenylphosphine (16.6 mg, 63.4 µmol, potassium carbonate (263 mg, 1.90 mmol) and the palladium catalyst Bis(triphenylphosphine)palladium(ll) dichloride(44.5 mg, 63.4 µmol; CAS-RN:[13965-03-2]) were added. The mixture was heated to 100°C and stirred for 1 h. After cooling to rt, water (2 ml) was added, and the mixture was extracted with EtOAc twice. The combined organic layers were dried over sodium sulfate, filtered, and concentrated under reduced pressure. The residue was purified by reverse phase preparative HPLC (method 3) yielding 238 mg (99 % purity, 69 % yield) of the desired product.
LC-MS (method 2): Rₜ = 2.02 min; MS (ESlpos): m/z = 539 [M+H]⁺
¹H-NMR (400 MHz, DMSO-d6) δ [ppm]: 11.94 (s, 1H), 8.65-8.52 (m, 1H), 8.50-8.44 (m, 1H), 8.41-8.30 (m, 1H), 8.08-7.93 (m, 1H), 7.89-7.83 (m, 1H), 7.83-7.75 (m, 1H), 7.56-7.39 (m, 3H), 7.31-7.21 (m, 1H), 4.12-3.97 (m, 1H), 3.96-3.85 (m, 1H), 3.82-3.67 (m, 1H), 3.64-3.49 (m, 1H), 3.10-2.97 (m, 1H), 2.95-2.83 (m, 1H), 1.64-1.39 (m, 3H), 1.37-1.21 (m, 10H).

### Intermediate 107

### tert-butyl (2S)-2-[({4-[3-(3-chlorophenyl)-1H-pyrrolo[3,2-b]pyridin-2-yl]pyridin-3-yl}oxy)methyl]pyrrolidine-1-carboxylate

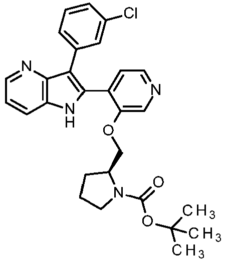

A solution of tert-butyl (2S)-2-({[4-(3-bromo-1H-pyrrolo[3,2-b]pyridin-2-yl)pyridin-3-yl]oxy}methyl)pyrrolidine-1-carboxylate (300 mg, 634 µmol) and (3-chlorophenyl)boronic acid (149 mg, 951 µmol) in a mixture of 1-propanole and water (5:1, 4 ml) was carefully degassed and purged with argon. Triphenylphosphine (16.6 mg, 63.4 µmol), potassium carbonate (263 mg, 1.90 mmol) and the palladium catalyst bis(triphenylphosphine)palladium(ll) dichloride (44.5 mg, 63.4 µmol; CAS-RN:[13965-03-2]) were added. The mixture was heated to 100°C and stirred for 1.5 h. After cooling to rt, water (2 ml) was added, and the mixture was extracted with EtOAc twice. The combined organic layers were dried over sodium sulfate, filtered, and concentrated under reduced pressure. The residue was purified by reverse phase preparative HPLC (method 3) yielding 235 mg (100 % purity, 73 % yield) of the desired product.
LC-MS (method 2): Rₜ = 1.86 min; MS (ESlpos): m/z = 505 [M+H]⁺
¹H-NMR (400 MHz, DMSO-d6) δ [ppm]: 11.85 (s, 1H), 8.67-8.51 (m, 1H), 8.49-8.41 (m, 1H), 8.38-8.26 (m, 1H), 7.94-7.69 (m, 2H), 7.53-7.33 (m, 2H), 7.32-7.18 (m, 3H), 4.18-3.91 (m, 2H), 3.89-3.56 (m, 2H), 3.13-2.98 (m, 1H), 2.97-2.79 (m, 1H), 1.74-1.55 (m, 1H), 1.53-1.27 (m, 12H).

### Intermediate 108

### tert-butyl (2S)-2-[({4-[3-(5-chloro-2-fluorophenyl)-1H-pyrrolo[3,2-b]pyridin-2-yl]pyridin-3-yl}oxy)methyl]pyrrolidine-1-carboxylate

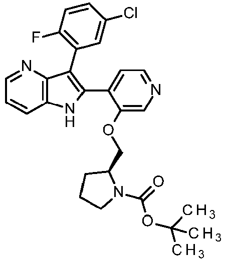

A solution of tert-butyl (2S)-2-({[4-(3-bromo-1H-pyrrolo[3,2-b]pyridin-2-yl)pyridin-3-yl]oxy}methyl)pyrrolidine-1-carboxylate (300 mg, 634 µmol) and (5-chloro-2-fluorophenyl)boronic acid (166 mg, 951 µmol) in a mixture of 1-propanole and water (5:1, 4 ml) was carefully degassed and purged with argon. Triphenylphosphine (16.6 mg, 63.4 µmol), potassium carbonate (263 mg, 1.90 mmol) and the palladium catalyst bis(triphenylphosphine)palladium(ll) dichloride (44.5 mg, 63.4 µmol; CAS-RN:[13965-03-2]) were added. The mixture was heated to 100°C and stirred for 2.5 h. After cooling to rt, water (2 ml) was added, and the mixture was extracted with EtOAc twice. The combined organic layers were dried over sodium sulfate, filtered, and concentrated under reduced pressure. The residue was purified by reverse phase preparative HPLC (method 3) yielding 113 mg (100 % purity, 34 % yield) of the desired product.
LC-MS (method 2): Rₜ = 1.84 min; MS (ESlpos): m/z = 523 [M+H]⁺
¹H-NMR (400 MHz, DMSO-d6) δ [ppm]: 11.96 (s, 1H), 8.61-8.47 (m, 1H), 8.46-8.39 (m, 1H), 8.33-8.20 (m, 1H), 7.94-7.82 (m, 1H), 7.77-7.67 (m, 1H), 7.43-7.32 (m, 2H), 7.30-7.13 (m, 3H), 4.20-4.00 (m, 1H), 3.98-3.70 (m, 2H), 3.67-3.49 (m, 1H), 3.21-2.92 (m, 2H), 1.81-1.47 (m, 4H), 1.44-1.27 (m, 10H).

### Intermediate 109

### 2-{3-[(2S)-pyrrolidin-2-ylmethoxy]pyridin-4-yl}-3-[3-(trifluoromethyl)phenyl]-1H-pyrrolo[3,2-b]pyridine hydrochloride (1:1)

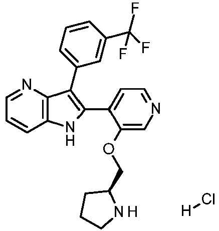

To a solution of tert-butyl (2S)-2-{[(4-{3-[3-(trifluoromethyl)phenyl]-1H-pyrrolo[3,2-b]pyridin-2-yl}pyridin-3-yl)oxy]methyl}pyrrolidine-1-carboxylate (238 mg, 442 µmol) in DCM (3 ml), HCl (880 µl, 4.0 M in 1,4-dioxane, 3.5 mmol) was added at rt. Stirring was continued for 18 h. The solvent was removed under reduced pressure and the remaining residue was dried in vacuo. 281 mg (99 % purity, 133 % yield) of the title compound were obtained that were used without further purification.
LC-MS (method 1): Rₜ = 0.86 min; MS (ESlneg): m/z = 437 [M-H]⁻
¹H-NMR (400 MHz, DMSO-d6) δ [ppm]: 13.73 (s, 1H), 9.97-9.76 (m, 1H), 9.70-9.51 (m, 1H), 8.78-8.72 (m, 1H), 8.70-8.63 (m, 2H), 8.40-8.32 (m, 1H), 7.89-7.80 (m, 1H), 7.79-7.70 (m, 3H), 7.70-7.61 (m, 1H), 7.40-7.31 (m, 1H), 4.53-4.29 (m, 3H), 3.53-3.42 (m, 2H), 3.17-3.03 (m, 2H), 2.08-1.94 (m, 1H), 1.88-1.56 (m, 3H).

### Intermediate 110

### 3-(3-chlorophenyl)-2-{3-[(2S)-pyrrolidin-2-ylmethoxy]pyridin-4-yl}-1H-pyrrolo[3,2-b]pyridine hydrochloride (1:1)

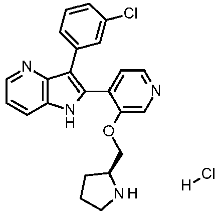

To a solution of tert-butyl (2S)-2-[({4-[3-(3-chlorophenyl)-1H-pyrrolo[3,2-b]pyridin-2-yl]pyridin-3-yl}oxy)methyl]pyrrolidine-1-carboxylate (235 mg, 465 µmol) in DCM (3 ml), HCl (930 µl, 4.0 M in 1,4-dioxane, 3.7 mmol) was added at rt. Stirring was continued for 18 h. The solvent was removed under reduced pressure and the remaining residue was dried in vacuo. 247 mg (100 % purity, 120 % yield) of the title compound were obtained that were used without further purification.
LC-MS (method 1): Rₜ = 0.75 min; MS (ESlneg): m/z = 403 [M-H]⁻
¹H-NMR (400 MHz, DMSO-d6) δ [ppm]: 13.76 (s, 1H), 9.95-9.77 (m, 1H), 9.70-9.53 (m, 1H), 8.78-8.73 (m, 1H), 8.73-8.68 (m, 1H), 8.68-8.64 (m, 1H), 8.40-8.35 (m, 1H), 7.80-7.72 (m, 1H), 7.67-7.62 (m, 1H), 7.51-7.40 (m, 2H), 7.38-7.29 (m, 2H), 4.52-4.39 (m, 2H), 3.54-3.43 (m, 2H), 3.20-3.03 (m, 3H), 2.10-1.95 (m, 1H), 1.88-1.61 (m, 3H).

### Intermediate 111

### 3-(5-chloro-2-fluorophenyl)-2-{3-[(2S)-pyrrolidin-2-ylmethoxy]pyridin-4-yl}-1H-pyrrolo[3,2-b]pyridine hydrochloride (1:1)

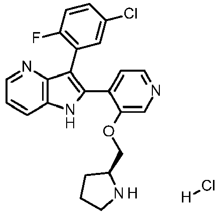

To a solution of tert-butyl (2S)-2-[({4-[3-(5-chloro-2-fluorophenyl)-1H-pyrrolo[3,2-b]pyridin-2-yl]pyridin-3-yl}oxy)methyl]pyrrolidine-1-carboxylate (113 mg, 216 µmol) in DCM (2 ml), HCl (430 µl, 4.0 M in 1,4-dioxane, 1.7 mmol) was added at rt. Stirring was continued for 18 h. The solvent was removed under reduced pressure and the remaining residue was dried in vacuo. 120 mg (99 % purity, 120 % yield) of the title compound were obtained that were used without further purification.
LC-MS (method 1): Rₜ = 0.73 min; MS (ESlneg): m/z = 421 [M-H]⁻
¹H-NMR (400 MHz, DMSO-d6) δ [ppm]: 13.86 (br s, 1H), 9.92-9.76 (m, 1H), 9.70-9.53 (m, 1H), 8.81-8.62 (m, 3H), 8.39-8.29 (m, 1H), 7.83-7.72 (m, 1H), 7.71-7.63 (m, 1H), 7.61-7.51 (m, 1H), 7.43-7.33 (m, 1H), 7.31-7.24 (m, 1H), 4.54-4.38 (m, 2H), 3.97-3.82 (m, 3H), 3.20-3.06 (m, 3H), 2.14-1.96 (m, 1H), 1.89-1.58 (m, 3H).

### Intermediate 112

### tert-butyl [2-({4-[3-(5-chloro-2-fluorophenyl)-1H-pyrrolo[3,2-b]pyridin-2-yl]pyridin-3-yl}oxy)ethyl]methylcarbamate

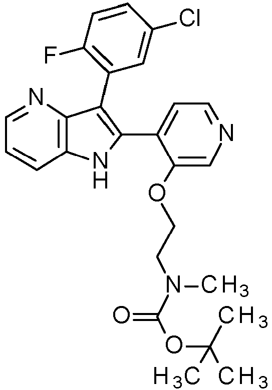

A solution of tert-butyl (2-{[4-(3-bromo-1H-pyrrolo[3,2-b]pyridin-2-yl)pyridin-3-yl]oxy}ethyl)methylcarbamate (250 mg, 559 µmol) and (5-chloro-2-fluorophenyl)boronic acid (146 mg, 838 µmol) in a mixture of 1-propanole and water (5:1,10 ml) was carefully degassed and purged with argon. Triphenylphosphine (14.7 mg, 55.9 µmol), potassium carbonate (232 mg, 1.68 mmol) and the palladium catalyst PdCl₂(PPh₃)₂ (39.2 mg, 55.9 µmol) were added. The mixture was heated to 100°C and stirred for 2 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 77.5 mg (100 % purity, 28 % yield) of the desired product.
LC-MS (method 1): Rₜ = 1.59 min; MS (ESlpos): m/z = 497 [M+H]⁺
¹H-NMR (400 MHz, DMSO-d6) δ [ppm]: 1.020 (1.01), 1.292 (12.51), 2.596 (2.43), 2.638 (2.49), 3.161 (15.66), 3.174 (16.00), 3.209 (1.01), 3.287 (0.51), 3.301 (0.84), 3.405 (0.62), 4.017 (0.99), 4.084 (1.60), 4.097 (3.86), 4.110 (4.03), 4.123 (2.12), 7.183 (0.99), 7.205 (2.02), 7.235 (3.31), 7.247 (3.12), 7.256 (3.36), 7.267 (3.53), 7.339 (0.64), 7.388 (1.42), 7.712 (2.31), 7.719 (2.36), 7.728 (2.43), 7.734 (2.15), 7.882 (1.26), 8.234 (0.80), 8.411 (3.53), 8.414 (3.50), 8.422 (3.58), 8.425 (3.22), 8.512 (5.76).

The intermediates listed in the following table were prepared in an analogous manner to intermediate 112.

| **Intermediate** | **Structure IUPAC-Name** | **LC-MS method Retention time Mass found** |
|---|---|---|
| **113** | 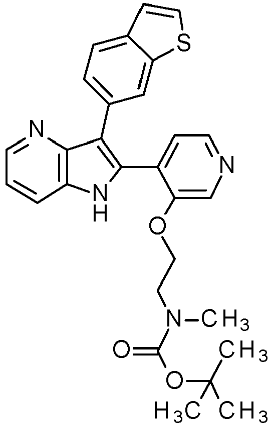 **tert-butyl [2-({4-[3-(1-benzothiophen-6-yl)-1H-pyrrolo[3,2-b]pyridin-2-yl]pyridin-3-yl}oxy)ethyl]methylcarbamate** | method 2 |
| | | Rₜ = 1.59 min |
| | | m/z = 501 [M+H]⁺ |
| **114** | 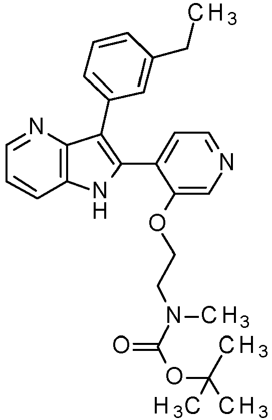 **tert-butyl [2-({4-[3-(3-ethylphenyl)-1H-pyrrolo[3,2-b]pyridin-2-yl]pyridin-3-yl}oxy)ethyl]methylcarbamate** | method 1 |
| | | Rₜ = 1.51 min |
| | | m/z = 473 [M+H]⁺ |
| **115** | 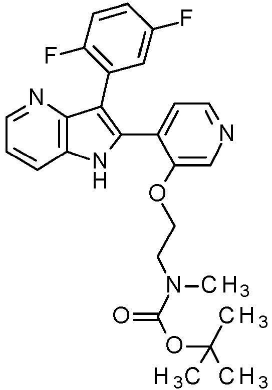 **tert-butyl [2-({4-[3-(2,5-difluorophenyl)-1H-pyrrolo[3,2-b]pyridin-2-yl]pyridin-3-yl}oxy)ethyl]methylcarbamate** | method 1 |
| | | Rₜ = 1.41 min |
| | | m/z = 479 [M-H]⁻ |
| **116** | 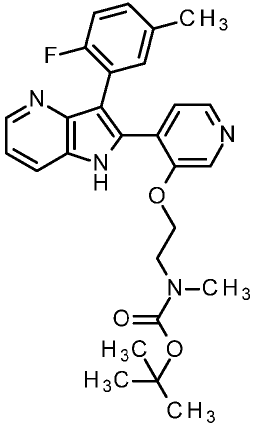 **tert-butyl [2-({4-[3-(2-fluoro-5-methylphenyl)-1H-pyrrolo[3,2-b]pyridin-2-yl]pyridin-3-yl}oxy)ethyl]methylcarbamate** | method 2 |
| | | Rₜ = 1.50 min |
| | | m/z = 477 [M+H]⁺ |
| **117** | 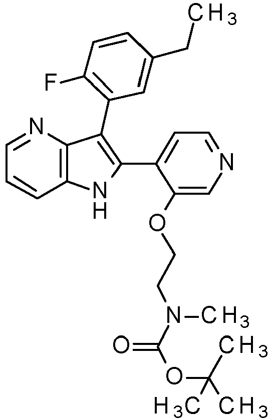 **tert-butyl [2-({4-[3-(5-ethyl-2-fluorophenyl)-1H-pyrrolo[3,2-b]pyridin-2-yl]pyridin-3-yl}oxy)ethyl]methylcarbamate** | method 1 |
| | | Rₜ = 1.52 min |
| | | m/z = 491 [M+H]⁺ |
| **118** | 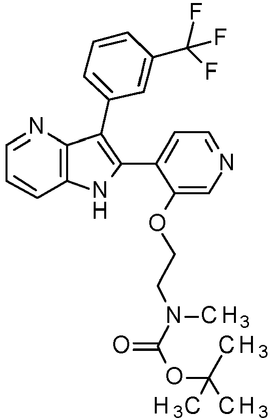 **tert-butyl methyl{2-[(4-{3-[3-(trifluoromethyl)phenyl]-1H-pyrrolo[3,2-b]lpyridin-2-yl}pyridin-3-yl)oxy]ethyl}carbamate** | method 2 |
| | | Rₜ = 1.87 min |
| | | m/z = 513 [M+H]⁺ |
| **119** | 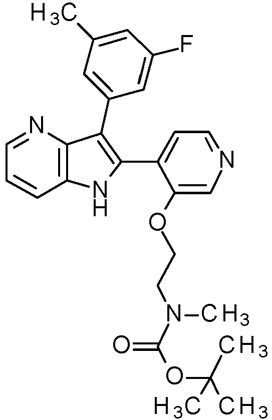 **tert-butyl [2-({4-[3-(3-fluoro-5-methylphenyl)-1H-pyrrolo[3,2-b]pyridin-2-yl]pyridin-3-yl}oxy)ethyl]methylcarbamate** | method 2 |
| | | Rₜ = 1.60 min |
| | | m/z = 477 [M+H]⁺ |
| **120** | 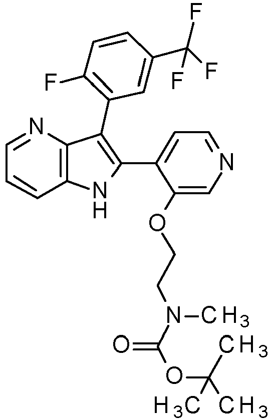 **tert-butyl {2-[(4-{3-[2-fluoro-5-(trifluoromethyl)phenyl]-1H-pyrrolo[3,2-b]pyridin-2-yl}pyridin-3-yl)oxy]ethyl}methylcarbamate** | method 1 |
| | | Rₜ = 1.74 min |
| | | m/z = 531 [M+H]⁺ |
| **121** | 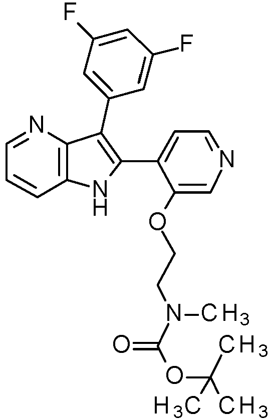 **tert-butyl [2-({4-[3-(3,5-difluorophenyl)-1H-pyrrolo[3,2-b]pyridin-2-yl]pyridin-3-yl}oxy)ethyl]methylcarbamate** | method 1 |
| | | Rₜ = 1.64 min |
| | | m/z = 481 [M+H]⁺ |
| **122** | 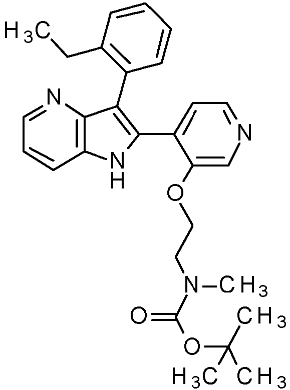 **tert-butyl [2-({4-[3-(2-ethylphenyl)-1H-pyrrolo[3,2-b]pyridin-2-yl]pyridin-3-yl}oxy)ethyl]methylcarbamate** | method 2 |
| | | Rₜ = 1.52 min |
| | | m/z = 473 [M+H]⁺ |
| **123** | 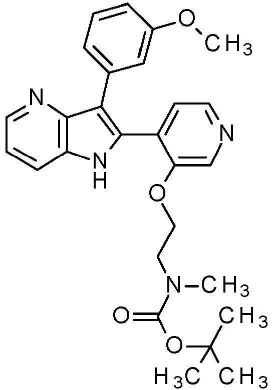 **tert-butyl [2-({4-[3-(3-methoxyphenyl)-1H-pyrrolo[3,2-b]pyridin-2-yl]pyridin-3-yl}oxy)ethyl]methylcarbamate** | method 1 |
| | | Rₜ = 1.35 min |
| | | m/z = 474 [M+H]⁺ |
| **124** | 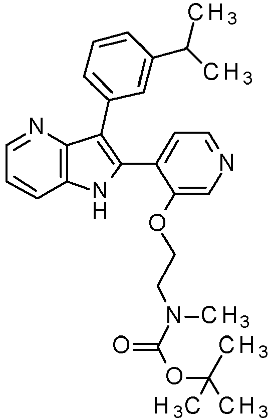 **tert-butyl methyl{2-[(4-{3-[3-(propan-2-yl)phenyl]-1H-pyrrolo[3,2-b]pyridin-2-yl}pyridin-3-yl)oxy]ethyl}carbamate** | method 1 |
| | | Rₜ = 1.63 min |
| | | m/z = 487 [M+H]⁺ |
| **125** | 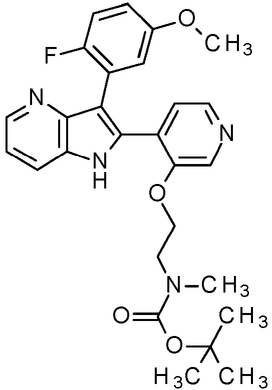 **tert-butyl [2-({4-[3-(2-fluoro-5-methoxyphenyl)-1H-pyrrolo[3,2-b]pyridin-2-yl]pyridin-3-yl}oxy)ethyl]methylcarbamate** | method 1 |
| | | Rₜ = 1.36 min |
| | | m/z = 493 [M+H]⁺ |
| **126** | 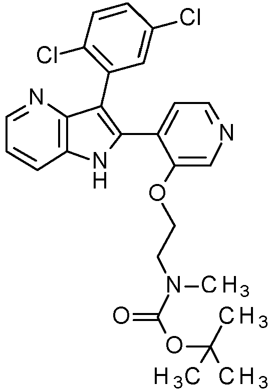 **tert-butyl [2-({4-[3-(2,5-dichlorophenyl)-1H-pyrrolo[3,2-b]pyridin-2-yl]pyridin-3-yl}oxy)ethyl]methylcarbamate** | method 1 |
| | | Rₜ = 1.59 min |
| | | m/z = 513 [M+H]⁺ |
| **127** | 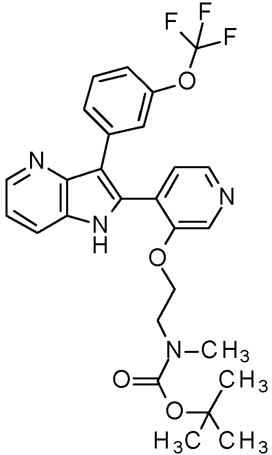 **tert-butyl methyl{2-[(4-{3-[3-(trifluoromethoxy)phenyl]-1H-pyrrolo[3,2-b]pyridin-2-yl}pyridin-3-yl)oxy]ethyl}carbamate** | method 1 |
| | | Rₜ = 1.78 min |
| | | m/z = 529 [M+H]⁺ |
| **128** | 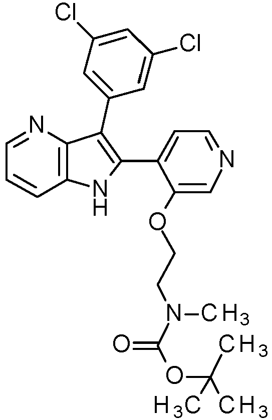 **tert-butyl [2-({4-[3-(3,5-dichlorophenyl)-1H-pyrrolo[3,2-b]pyridin-2-yl]pyridin-3-yl}oxy)ethyl]methylcarbamate** | method 1 |
| | | Rₜ = 2.00 min |
| | | m/z = 513 [M+H]⁺ |

### Intermediate 129

### 2-({4-[3-(5-chloro-2-fluorophenyl)-1H-pyrrolo[3,2-b]pyridin-2-yl]pyridin-3-yl}oxy)-N-methylethan-1-amine

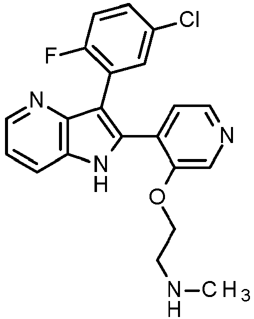

To a solution of tert-butyl [2-({4-[3-(5-chloro-2-fluorophenyl)-1H-pyrrolo[3,2-b]pyridin-2-yl]pyridin-3-yl}oxy)ethyl]methylcarbamate (77.5 mg, 156 µmol) in DCM (2 ml), hydrogen chloride (190 µl, 4.0 M in 1,4 dioxane, 780 µmol) was added at rt. After stirring for 16 h additional (190 µl, 4.0 M in 1,4 dioxane, 780 µmol) and MeOH (0.1 ml) were added and stirring was continued for 2 h. The solvents were evaporated under reduced pressure, the residue was dissolved in DCM (5 ml), washed with saturated aqueous sodium hydrogencarbonate solution (2.0 ml) and dried over a water repellent filter. After concentration under reduced pressure and drying in vacuo 63.0 mg (99 % purity, 101 % yield) of the title compound were obtained that were used without further purification.
LC-MS (method 2): Rₜ = 0.72 min; MS (ESlneg): m/z = 395 [M-H]⁻
¹H-NMR (600 MHz, DMSO-d6) δ [ppm]: 8.61-8.55 (m, 1H), 8.44-8.39 (m, 1H), 8.22-8.16 (m, 1H), 7.92-7.86 (m, 1H), 7.78-7.73 (m, 1H), 7.46-7.39 (m, 1H), 7.28-7.20 (m, 2H), 7.19-7.15 (m, 1H), 5.80-5.72 (m, 1H), 4.30-4.23 (m, 2H), 2.74-2.66 (m, 2H), 2.38-2.32 (m, 3H)

The intermediates listed in the following table were prepared in an analogous manner to intermediate 128.

| **Intermediate** | **Structure IUPAC-Name** | **LC-MS method Retention time Mass found** |
|---|---|---|
| **130** | 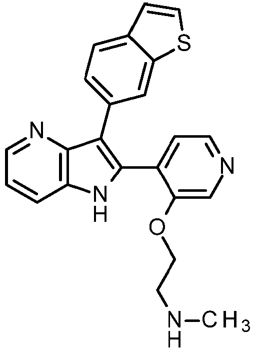 **2-({4-[3-(1-benzothiophen-6-yl)-1H-pyrrolo[3,2-b]pyridin-2-yl]pyridin-3-yl}oxy)-N-methylethan-1-amine** | method 2 |
| | | Rₜ = 0.74 min |
| | | m/z = 401 [M+H]⁺ |
| **131** | 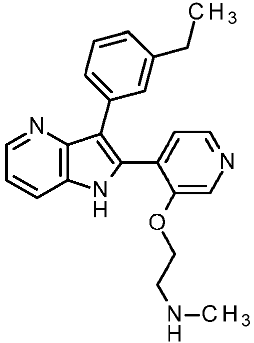 **2-({4-[3-(3-ethylphenyl)-1H-pyrrolo[3,2-b]pyridin-2-yl]pyridin-3-yl}oxy)-N-methylethan-1-amine** | method 1 |
| | | Rₜ = 0.76 min |
| | | m/z = 373 [M+H]⁺ |
| **132** | 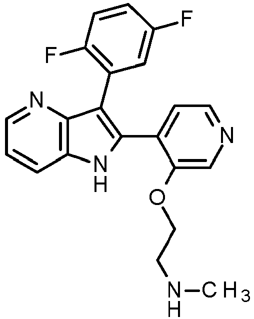 **2-({4-[3-(2,5-difluorophenyl)-1H-pyrrolo[3,2-b]pyridin-2-yl]pyridin-3-yl}oxy)-N-methylethan-1-amine** | method 2 |
| | | Rₜ = 0.58 min |
| | | m/z = 381 [M+H]⁺ |
| **133** | 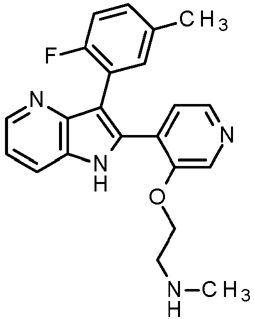 **2-({4-[3-(2-fluoro-5-methylphenyl)-1H-pyrrolo[3,2-b]pyridin-2-yl]pyridin-3-yl}oxy)-N-methylethan-1-amine** | method 1 |
| | | Rₜ = 0.54 min |
| | | m/z = 377 [M+H]⁺ |
| **134** | 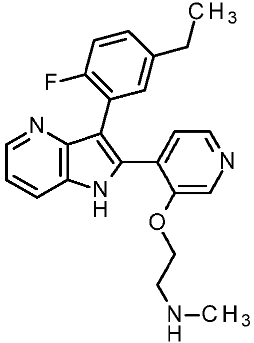 **2-({4-[3-(5-ethyl-2-fluorophenyl)-1H-pyrrolo[3,2-b]pyridin-2-yl]pyridin-3-yl}oxy)-N-methylethan-1-amine** | method 1 |
| | | Rₜ = 0.73 min |
| | | m/z = 389 [M-H]⁻ |
| **135** | 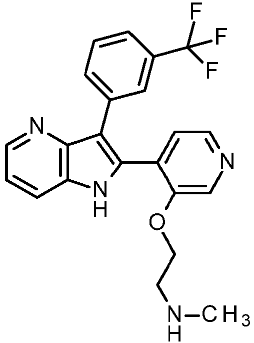 **N-methyl-2-[(4-{3-[3-(trifluoromethyl)phenyl]-1H-pyrrolo[3,2-b]pyridin-2-yl}pyridin-3-yl)oxy]ethan-1-amine** | method 2 |
| | | Rₜ = 0.90 min |
| | | m/z = 411 [M-H]⁻ |
| **136** | 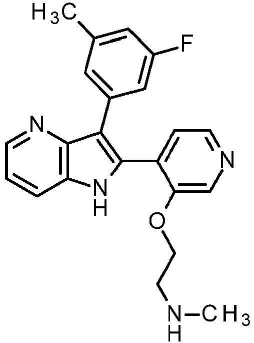 **2-({4-[3-(3-fluoro-5-methylphenyl)-1H-pyrrolo[3,2-b]pyridin-2-yl]pyridin-3-yl}oxy)-N-methylethan-1-amine** | method 2 |
| | | Rₜ = 0.73 min |
| | | m/z = 377 [M+H]⁺ |
| **137** | 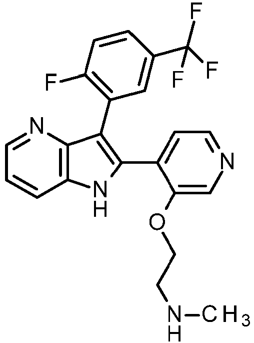 **2-[(4-{3-[2-fluoro-5-(trifluoromethyl)phenyl]-1H-pyrrolo[3,2-b]pyridin-2-yl}pyridin-3-yl)oxy]-N-methylethan-1-amine** | method 1 |
| | | Rₜ = 0.98 min |
| | | m/z = 429 [M-H]⁻ |
| **138** | 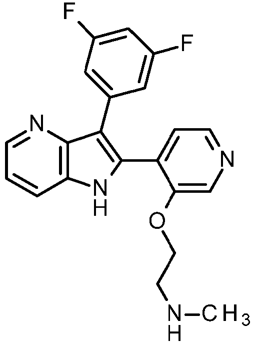 **2-({4-[3-(3,5-difluorophenyl)-1H-pyrrolo[3,2-b]pyridin-2-yl]pyridin-3-yl}oxy)-N-methylethan-1-amine** | method 2 |
| | | Rₜ = 0.74 min |
| | | m/z = 379 [M-H]⁻ |
| **139** | 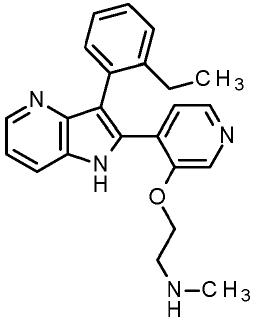 **2-({4-[3-(2-ethylphenyl)-1H-pyrrolo[3,2-b]pyridin-2-yl]pyridin-3-yl}oxy)-N-methylethan-1-amine** | method 2 |
| | | Rₜ = 0.63 min |
| | | m/z = 373 [M+H]⁺ |
| **140** | 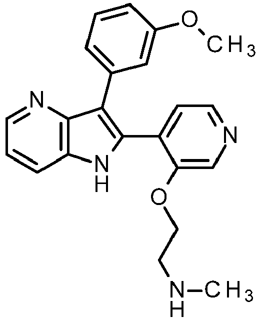 **2-({4-[3-(3-methoxyphenyl)-1H-pyrrolo[3,2-b]pyridin-2-yl]pyridin-3-yl}oxy)-N-methylethan-1-amine** | method 1 |
| | | Rₜ = 0.59 min |
| | | m/z = 373 [M-H]⁻ |
| **141** | 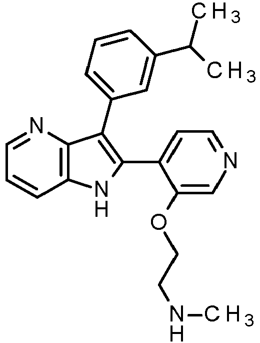 **N-methyl-2-[(4-{3-[3-(propan-2-yl)phenyl]-1H-pyrrolo[3,2-b]pyridin-2-yl}pyridin-3-yl)oxy]ethan-1-amine** | method 1 |
| | | Rₜ = 0.81 min |
| | | m/z = 387 [M+H]⁺ |
| **142** | 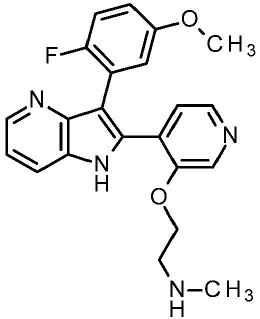 **2-({4-[3-(2-fluoro-5-methoxyphenyl)-1H-pyrrolo[3,2-b]pyridin-2-yl]pyridin-3-yl}oxy)-N-methylethan-1-amine** | method 1 |
| | | Rₜ = 0.56 min |
| | | m/z = 391 [M-H]⁻ |
| **143** | 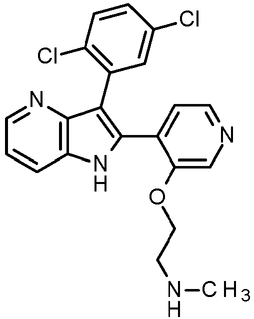 **2-({4-[3-(2,5-dichlorophenyl)-1H-pyrrolo[3,2-b]pyridin-2-yl]pyridin-3-yl}oxy)-N-methylethan-1-amine** | method 2 |
| | | Rₜ = 0.74 min |
| | | m/z = 413 [M+H]⁺ |
| **144** | 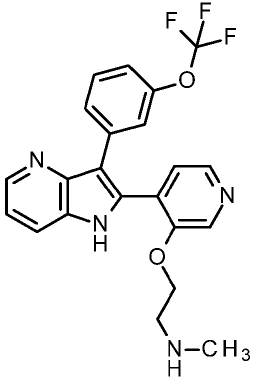 **N-methyl-2-[(4-{3-[3-(trifluoromethoxy)phenyl]-1H-pyrrolo[3,2-b]pyridin-2-yl)pyridin-3-yl)oxy]ethan-1-amine** | method 1 |
| | | Rₜ = 1.00 min |
| | | m/z = 427 [M-H]⁻ |
| **145** | 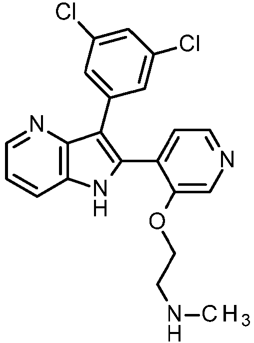 **2-({4-[3-(3,5-dichlorophenyl)-1H-pyrrolo[3,2-b]pyridin-2-yl]pyridin-3-yl}oxy)-N-methylethan-1-amine** | method 2 |
| | | Rₜ = 0.99 min |
| | | m/z = 411 [M-H]⁻ |

### Intermediate 146

### tert-butyl (2S)-2-{[(4-{3-[2-fluoro-5-(trifluoromethyl)phenyl]-1H-pyrrolo[3,2-b]pyridin-2-yl}pyridin-3-yl)oxy]methyl}pyrrolidine-1-carboxylate

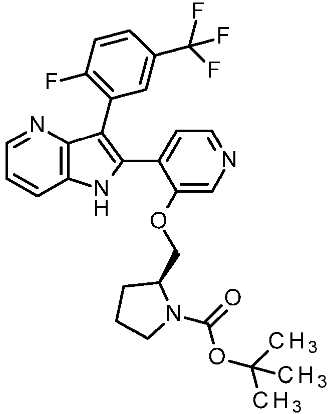

A solution of tert-butyl (2S)-2-({[4-(3-bromo-1H-pyrrolo[3,2-b]pyridin-2-yl)pyridin-3-yl]oxy}methyl)pyrrolidine-1-carboxylate (200 mg, 422 µmol) and [2-fluoro-5-(trifluoromethyl)phenyl]boronic acid (132 mg, 634 µmol) in a mixture of 1-propanole and water (5:1, 3.6 ml) was carefully degassed and purged with argon. Triphenylphosphine (11.1 mg, 42.2 µmol), potassium carbonate (175 mg, 1.27 mmol) and the palladium catalyst PdCl₂(PPh₃)₂ (29.7 mg, 42.2 µmol) were added. The mixture was heated to 100°C and stirred for 45 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 111 mg (100 % purity, 47 % yield) of the desired product.
LC-MS (method 1): Rₜ = 1.90 min; MS (ESlpos): m/z = 557 [M+H]⁺

The intermediates listed in the following table were prepared in an analogous manner to intermediate 146.

| **Intermediate** | **Structure IUPAC-Name** | **LC-MS method Retention time Mass found** |
|---|---|---|
| **147** | 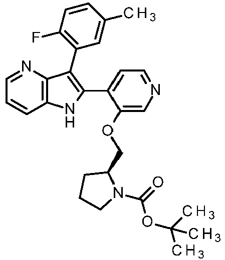 **tert-butyl (2S)-2-[({4-[3-(2-fluoro-5-methylphenyl)-1H-pyrrolo[3,2-b]pyridin-2-yl]pyridin-3-yl}oxy)methyl]pyrrolidine-1-carboxylate** | method 1 |
| | | Rₜ = 1.60 min |
| | | m/z = 503 [M+H]⁺ |
| **148** | 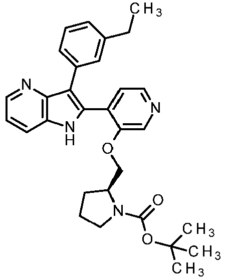 **tert-butyl (2S)-2-[({4-[3-(3-ethylphenyl)-1H-pyrrolo[3,2-b]pyridin-2-yl]pyridin-3-yl}oxy)methyl]pyrrolidine-1-carboxylate** | method 1 |
| | | Rₜ = 1.66 min |
| | | m/z = 499 [M+H]⁺ |
| **149** | 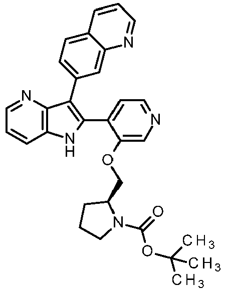 **tert-butyl (2S)-2-[({4-[3-(quinolin-7-yl)-1H-pyrrolo[3,2-b]pyridin-2-yl]pyridin-3-yl}oxy)methyl]pyrrolidine-1-carboxylate** | method 1 |
| | | Rₜ = 1.30 min |
| | | m/z = 522 [M+H]⁺ |
| **150** | 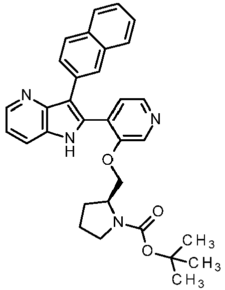 **tert-butyl (2S)-2-[({4-[3-(naphthalen-2-yl)-1H-pyrrolo[3,2-b]pyridin-2-yl]pyridin-3-yl}oxy)methyl]pyrrolidine-1-carboxylate** | method 1 |
| | | Rₜ = 1.68 min |
| | | m/z = 521 [M+H]⁺ |
| **151** | 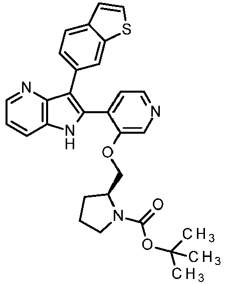 **tert-butyl (2S)-2-[({4-[3-(1-benzothiophen-6-yl)-1H-pyrrolo[3,2-b]pyridin-2-yl]pyridin-3-yl}oxy)methyl]pyrrolidine-1-carboxylate** | method 1 |
| | | Rₜ = 1.67 min |
| | | m/z = 527 [M+H]⁺ |
| **152** | 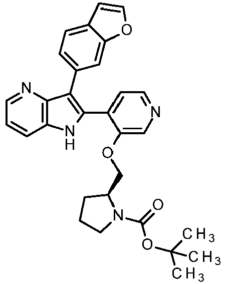 **tert-butyl (2S)-2-[({4-[3-(1-benzofuran-6-yl)-1H-pyrrolo[3,2-b]pyridin-2-yl]pyridin-3-yl}oxy)methyl]pyrrolidine-1-carboxylate** | method 1 |
| | | Rₜ = 1.54 min |
| | | m/z = 511 [M+H]⁺ |
| **153** | 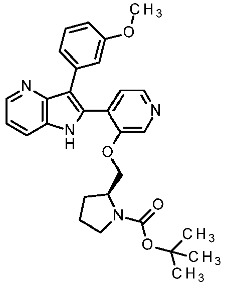 **tert-butyl (2S)-2-[({4-[3-(3-methoxyphenyl)-1H-pyrrolo[3,2-b]pyridin-2-yl]pyridin-3-yl}oxy)methyl]pyrrolidine-1-carboxylate** | method 1 |
| | | Rₜ = 1.49 min |
| | | m/z = 501 [M+H]⁺ |
| **154** | 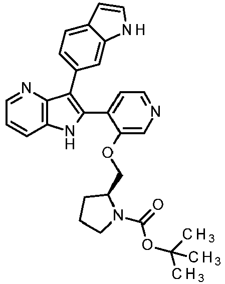 **tert-butyl (2S)-2-[({4-[3-(1H-indol-6-yl)-1H-pyrrolo[3,2-b]pyridin-2-yl]pyridin-3-yl}oxy)methyl]pyrrolidine-1-carboxylate** | method 1 |
| | | Rₜ = 1.46 min |
| | | m/z = 510 [M+H]⁺ |
| **155** | 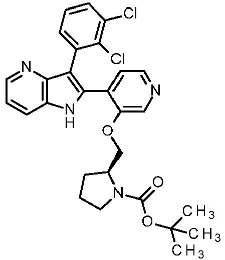 **tert-butyl (2S)-2-[({4-[3-(2,3-dichlorophenyl)-1H-pyrrolo[3,2-b]pyridin-2-yl]pyridin-3-yl}oxy)methyl]pyrrolidine-1-carboxylate** | method 1 |
| | | Rₜ = 1.72 min |
| | | m/z = 539 [M+H]⁺ |
| **156** | 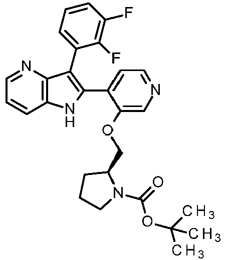 **tert-butyl (2S)-2-[({4-[3-(2,3-difluorophenyl)-1H-pyrrolo[3,2-b]pyridin-2-yl]pyridin-3-yl}oxy)methyl]pyrrolidine-1-carboxylate** | method 1 |
| | | Rₜ = 1.62 min |
| | | m/z = 507 [M+H]⁺ |
| **157** | 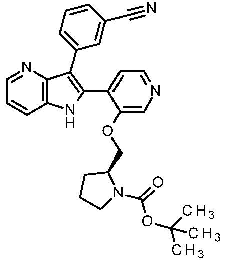 **tert-butyl (2S)-2-[({4-[3-(3-cyanophenyl)-1H-pyrrolo[3,2-b]pyridin-2-yl]pyridin-3-yl}oxy)methyl]pyrrolidine-1-carboxylate** | method 1 |
| | | Rₜ = 1.62 min |
| | | m/z = 496 [M+H]⁺ |
| **158** | 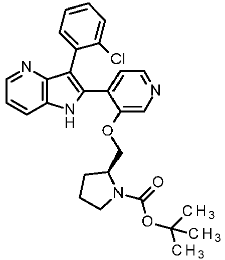 **tert-butyl (2S)-2-[({4-[3-(2-chlorophenyl)-1H-pyrrolo[3,2-b]pyridin-2-yl]pyridin-3-yl}oxy)methyl]pyrrolidine-1-carboxylate** | method 1 |
| | | Rₜ = 1.55 min |
| | | m/z = 505 [M+H]⁺ |
| **159** | 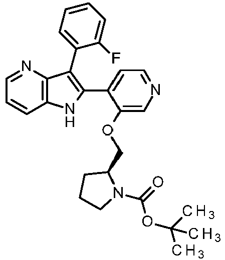 **tert-butyl (2S)-2-[({4-[3-(2-fluorophenyl)-1H-pyrrolo[3,2-b]pyridin-2-yl]pyridin-3-yl}oxy)methyl]pyrrolidine-1-carboxylate** | method 1 |
| | | Rₜ = 1.45 min |
| | | m/z = 489 [M+H]⁺ |
| **160** | 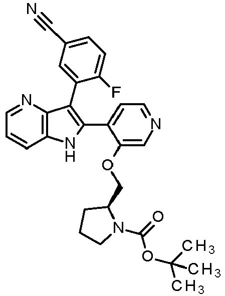 **tert-butyl (2S)-2-[({4-[3-(5-cyano-2-fluorophenyl)-1H-pyrrolo[3,2-b]pyridin-2-yl]pyridin-3-yl}oxy)methyl]pyrrolidine-1-carboxylate** | method 1 |
| | | Rₜ = 1.61 min |
| | | m/z = 514 [M+H]⁺ |
| **161** | 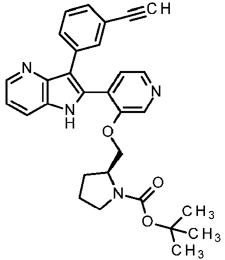 **tert-butyl (2S)-2-[({4-[3-(3-ethynylphenyl)-1H-pyrrolo[3,2-b]pyridin-2-yl]pyridin-3-yl}oxy)methyl]pyrrolidine-1-carboxylate** | method 1 |
| | | Rₜ = 1.63 min |
| | | m/z = 495 [M+H]⁺ |
| **162** | 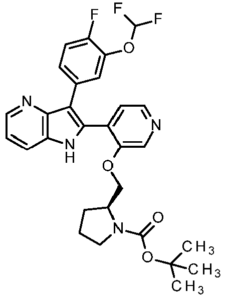 **tert-butyl (2S)-2-{[(4-{3-[3-(difluoromethoxy)-4-fluorophenyl]-1H-pyrrolo[3,2-b]pyridin-2-yl}pyridin-3-yl)oxy]methyl}pyrrolidine-1-carboxylate** | method 1 |
| | | Rₜ = 1.79 min |
| | | m/z = 555 [M+H]⁺ |
| **163** | 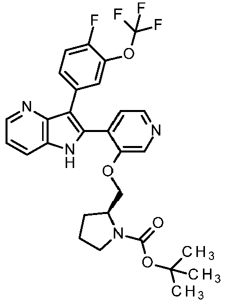 **tert-butyl (2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidine-1-carboxylate** | method 1 |
| | | Rₜ = 2.07 min |
| | | m/z = 573 [M+H]⁺ |

### Intermediate 164

### 3-[2-fluoro-5-(trifluoromethyl)phenyl]-2-(3-{[(2S)-pyrrolidin-2-yl]methoxy}pyridin-4-yl)-1H-pyrrolo[3,2-b]pyridine

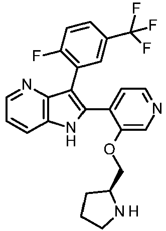

To a solution of tert-butyl (2S)-2-{[(4-{3-[2-fluoro-5-(trifluoromethyl)phenyl]-1H-pyrrolo[3,2-b]pyridin-2-yl}pyridin-3-yl)oxy]methyl}pyrrolidine-1-carboxylate (111 mg, 199 µmol) in DCM (2 ml), hydrogen chloride (400 µl, 4.0 M in 1,4 dioxane, 1.6 mmol) was added at rt. After stirring for 16 h, saturated aqueous sodium hydrogencarbonate solution (1 ml) was added. The mixture was diluted with DCM (5 ml) and dried over a water repellent filter. After concentration under reduced pressure and drying in vacuo 75.0 mg (100 % purity, 82 % yield) of the title compound were obtained that were used without further purification.
LC-MS (method 1): Rₜ = 0.82 min; MS (ESlneg): m/z = 455 [M-H]⁻

The intermediates listed in the following table were prepared in an analogous manner to intermediate 164.

| **Intermediate** | **Structure IUPAC-Name** | **LC-MS method Retention time Mass found** |
|---|---|---|
| **165** | 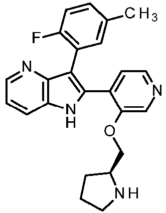 **3-(2-fluoro-5-methylphenyl)-2-(3-{[(2S)-pyrrolidin-2-yl]methoxy}pyridin-4-yl)-1H-pyrrolo[3,2-b]pyridine** | method 1 |
| | | Rₜ = 0.63 min |
| | | m/z = 401 [M-H]⁻ |
| **166** | 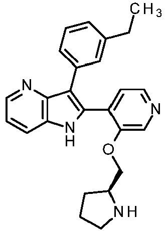 **3-(3-ethylphenyl)-2-(3-{[(2S)-pyrrolidin-2-yl]methoxy}pyridin-4-yl)-1H-pyrrolo[3,2-b]pyridine** | method 1 |
| | | Rₜ = 0.73 min |
| | | m/z = 397 [M-H]⁻ |
| **167** | 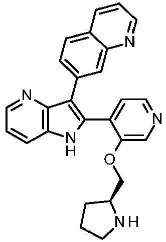 **7-[2-(3-{[(2S)-pyrrolidin-2-yl]methoxy}pyridin-4-yl)-1 H-pyrrolo[3,2-b]pyridin-3-yl]quinoline** | method 1 |
| | | Rₜ = 0.58 min |
| | | m/z = 420 [M-H]⁻ |
| **168** | 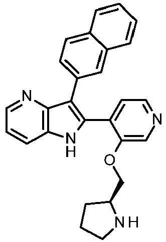 **3-(naphthalen-2-yl)-2-(3-{[(2S)-pyrrolidin-2-yl]methoxy}pyridin-4-yl)-1H-pyrrolo[3,2-b]pyridine** | method 1 |
| | | Rₜ = 0.79 min |
| | | m/z = 419 [M-H]⁻ |
| **169** | 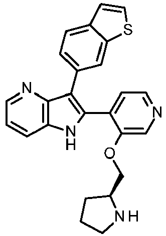 **3-(1-benzothiophen-6-yl)-2-(3-{[(2S)-pyrrolidin-2-yl]methoxy}pyridin-4-yl)-1H-pyrrolo[3,2-b]pyridine** | method 1 |
| | | Rₜ = 0.73 min |
| | | m/z = 425 [M-H]⁻ |
| **170** | 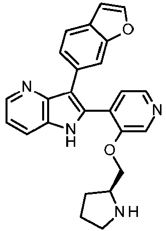 **3-(1-benzofuran-6-yl)-2-(3-{[(2S)-pyrrolidin-2-yl]methoxy}pyridin-4-yl)-1H-pyrrolo[3,2-b]pyridine** | method 1 |
| | | Rₜ = 0.79 min |
| | | m/z = 409 [M-H]⁻ |
| **171** | 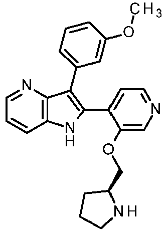 **3-(3-methoxyphenyl)-2-(3-{[(2S)-pyrrolidin-2-yl]methoxy}pyridin-4-yl)-1H-pyrrolo[3,2-b]pyridine** | method 1 |
| | | Rₜ = 0.63 min |
| | | m/z = 399 [M-H]⁻ |
| **172** | 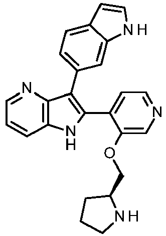 **3-(1H-indol-6-yl)-2-(3-{[(2S)-pyrrolidin-2-yl]methoxy}pyridin-4-yl)-1H-pyrrolo[3,2-b]pyridine** | method 1 |
| | | Rₜ = 0.60 min |
| | | m/z = 408 [M-H]⁻ |
| **173** | 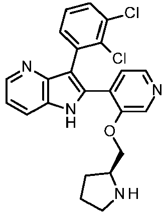 **3-(2,3-dichlorophenyl)-2-(3-{[(2S)-pyrrolidin-2-yl]methoxy}pyridin-4-yl)-1H-pyrrolo[3,2-b]pyridine** | method 8 |
| | | Rₜ = 1.56 min |
| | | m/z = 439 [M+H]⁺ |
| **174** | 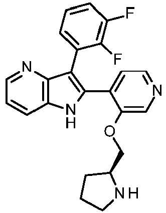 **3-(2,3-difluorophenyl)-2-(3-{[(2S)-pyrrolidin-2-yl]methoxy}pyridin-4-yl)-1H-pyrrolo[3,2-b]pyridine** | method 1 |
| | | Rₜ = 0.66 min |
| | | m/z = 405 [M-H]⁻ |
| **175** | 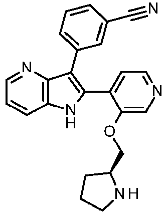 **3-[2-(3-{[(2S)-pyrrolidin-2-yl]methoxy}pyridin-4-yl)-1H-pyrrolo[3,2-b]pyridin-3-yl]benzonitrile** | method 1 |
| | | Rₜ = 0.67 min |
| | | m/z = 394 [M-H]⁻ |
| **176** | 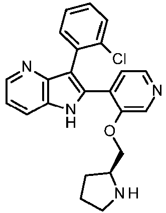 **3-(2-chlorophenyl)-2-(3-{[(2S)-pyrrolidin-2-yl]methoxy}pyridin-4-yl)-1H-pyrrolo[3,2-b]pyridine** | method 1 |
| | | Rₜ = 0.59 min |
| | | m/z = 403 [M-H]⁻ |
| **177** | 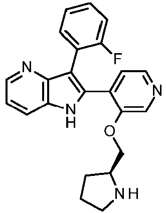 **3-(2-fluorophenyl)-2-(3-{[(2S)-pyrrolidin-2-yl]methoxy}pyridin-4-yl)-1H-pyrrolo[3,2-b]pyridine** | method 1 |
| | | Rₜ = 0.53 min |
| | | m/z = 387 [M-H]⁻ |
| **178** | 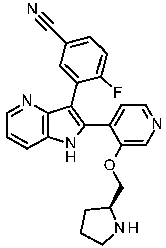 **4-fluoro-3-[2-(3-{[(2S)-pyrrolidin-2-yl]methoxy}pyridin-4-yl)-1H-pyrrolo[3,2-b]pyridin-3-yl]benzonitrile** | method 1 |
| | | Rₜ = 0.62 min |
| | | m/z = 412 [M-H]⁻ |
| **179** | 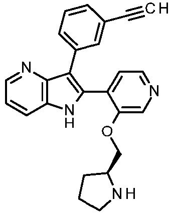 **3-(3-ethynylphenyl)-2-(3-{[(2S)-pyrrolidin-2-yl]methoxy}pyridin-4-yl)-1H-pyrrolo[3,2-b]pyridine** | method 1 |
| | | Rₜ = 0.72 min |
| | | m/z = 393 [M-H]⁻ |
| **180** | 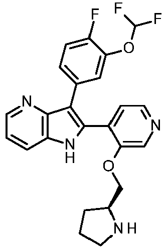 **3-[3-(difluoromethoxy)-4-fluorophenyl]-2-(3-{[(2S)-pyrrolidin-2-yl]methoxy}pyridin-4-yl)-1H-pyrrolo[3,2-b]pyridine** | method 1 |
| | | Rₜ = 0.84 min |
| | | m/z = 453 [M-H]⁻ |
| **181** | 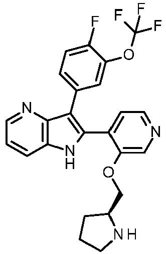 **3-[4-fluoro-3-(trifluoromethoxy)phenyl]-2-(3-{[(2S)-pyrrolidin-2-yl]methoxy}pyridin-4-yl)-**1**H-pyrrolo[3,2-b]pyridine** | method 1 |
| | | Rₜ = 1.02 min |
| | | m/z = 471 [M-H]⁻ |

### Intermediate 182

### 3-fluoro-4-iodo-5-methoxypyridine

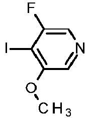

In a flame-dried flask, 3-fluoro-5-methoxypyridine (545 mg, 4.29 mmol) was dissolved in dry THF (5 ml) and cooled to -78°C. n-Butyllithium (3.2 ml, 1.6 M in hexane, 5.1 mmol) was added and stirring at -78°C was continued for 30 min. At this temperature iodine (1.20 g, 4.72 mmol) dissolved in THF (5 ml) was added and the mixture was allowed to warm to rt within 1 h. Water (2 ml) and an aqueous sodium thiosulfate solution (10%, 2 ml) were added. The mixture was extracted with EtOAc twice. The combined organic layers were dried over sodium sulfate, filtered and concentrated under reduced pressure. The residue was purified by reverse phase preparative HPLC (method 3) yielding 1.01 g (97 % purity, 90 % yield) of the desired product.
LC-MS (method 1): Rₜ = 1.42 min; MS (ESlpos): m/z = 253 [M+H]⁺
¹H-NMR (400 MHz, DMSO-d6) δ [ppm]: 8.14-8.12 (m, 1H), 8.09 (s, 1H), 3.99 (s, 3H)

### Intermediate 183

### 2-[(3-fluoro-5-methoxypyridin-4-yl)ethynyl]pyridin-3-amine

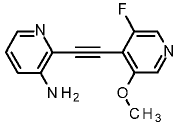

To a solution of 3-fluoro-4-iodo-5-methoxypyridine (803 mg, 3.17 mmol) in THF (15 ml), tetrakis(triphenylphosphine)palladium(0) (183 mg, 159 µmol), copper(I)iodide (181 mg, 952 µmol) and triethylamine (1.5 ml) were added at rt under argon. After 5 min, 2-[(trimethylsilyl)ethynyl]pyridin-3-amine (1.21 g, 6.35 mmol) and TBAF (6.3 ml, 1.0 M in THF, 6.3 mmol) were added. The solution was stirred at 70°C for 1 h. After cooling to rt, water (5 ml) was added and the mixture was filtered though a pad of Celite. The solution was extracted with EtOAc twice. The combined organic layers were washed with saturated aqueous sodium chloride solution, dried over sodium sulfate, filtered and concentrated under reduced pressure. The residue was purified by silica gel column chromatography (eluent: cyclohexane/EtOAc 1:1; EtOAc; EtOAc/MeOH 5:1) yielding 600 mg (85 % purity, 66 % yield) of the desired product.
LC-MS (method 1): Rₜ = 1.08 min; MS (ESlpos): m/z = 244 [M+H]⁺

### Intermediate 184

### 2,2,2-trifluoro-N-{2-[(3-fluoro-5-methoxypyridin-4-yl)ethynyl]pyridin-3-yl}acetamide

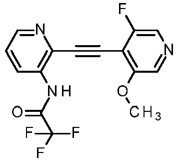

A solution of 2-[(3-fluoro-5-methoxypyridin-4-yl)ethynyl]pyridin-3-amine (730 mg, 3.00 mmol) in DCM (10 ml) was treated with triethylamine (1.0 ml, 7.5 mmol) and cooled to 0°C. Trifluoroacetic anhydride (640 µl, 4.5 mmol) was added and the mixture was allowed to warm to rt within 1 h. For work-up, all volatiles were removed under reduced pressure. The mixture was taken up in 10 ml of toluene and the mixture was evaporated to dryness under reduced pressure. MeOH was added to the residue and the precipitated solid was filtered off. The material obtained after filtration was further purified by reverse phase HPLC (method 3) yielding 345 mg (75 % purity, 25 % yield) of the desired compound.
LC-MS (method 1): Rₜ = 1.54 min; MS (ESlpos): m/z = 340 [M+H]⁺

### Intermediate 185

### 2-(3-fluoro-5-methoxypyridin-4-yl)-3-phenyl-1H-pyrrolo[3,2-b]pyridine

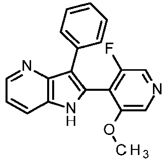

A solution of 2,2,2-trifluoro-N-{2-[(3-fluoro-5-methoxypyridin-4-yl)ethynyl]pyridin-3-yl}acetamide (395 mg, 1.16 mmol) in acetonitrile (12 ml) was carefully degassed and purged with argon. Iodobenzene (260 µl, 2.3 mmol), caesium carbonate (1.14 g, 3.49 mmol) and tetrakis(triphenylphosphine)palladium (67.3 mg, 58.2 µmol) were added and the mixture was stirred at 100 °C for 1.5 h. After cooling to rt, water (5 ml) was added and the mixture was extracted with EtOAc twice. The combined organic layers were dried over sodium sulfate, filtered and concentrated under reduced pressure. The residue was purified by reverse phase preparative HPLC (method 3) yielding 161 mg (100 % purity, 43 % yield) of the title compound.
LC-MS (method 1): Rₜ = 0.93 min; MS (ESlpos): m/z = 320 [M+H]⁺

### Intermediate 186

### 5-fluoro-4-(3-phenyl-1H-pyrrolo[3,2-b]pyridin-2-yl)pyridin-3-ol

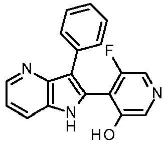

2-(3-fluoro-5-methoxypyridin-4-yl)-3-phenyl-1H-pyrrolo[3,2-b]pyridine (161 mg, 504 µmol) was dissolved in hydrobromic acid (5.7 ml, 48 % purity in water, 50 mmol) and stirred at 130 °C for 20 h. After cooling to rt, the reaction mixture was poured carefully into saturated NaHCO3 solution (aqueous, 10 mL). Upon addition of DCM, a solid precipitated and was filtered off. The solid was washed carefully with DCM and dried under vacuum to give 104 mg (100 % purity, 68 % yield) of the desired compound. The remaining aqueous solution was extracted with DCM twice, washed with aqueous sodium chloride solution, dried over sodium sulfate, filtered, and concentrated under reduced pressure. The remaining material was dried under vacuum to give additional 63.0 mg (90 % purity, 37 % yield) of the desired product.
LC-MS (method 1): Rₜ = 0.80 min; MS (ESlpos): m/z = 306 [M+H]⁺
¹H-NMR (400 MHz, DMSO-d6) δ [ppm]: 2.672 (0.40), 3.168 (16.00), 3.792 (1.00), 7.074 (2.72), 7.085 (2.84), 7.094 (2.92), 7.105 (3.74), 7.127 (3.42), 7.145 (2.25), 7.175 (3.88), 7.249 (4.29), 7.268 (6.99), 7.287 (3.56), 7.656 (4.43), 7.668 (6.58), 7.686 (5.77), 7.744 (3.31), 7.748 (3.28), 7.765 (3.18), 7.768 (2.96), 8.305 (3.34), 8.308 (3.38), 8.316 (3.45), 8.319 (3.11).

### Intermediate 187

### tert-butyl (2S)-2-({[5-fluoro-4-(3-phenyl-1H-pyrrolo[3,2-b]pyridin-2-yl)pyridin-3-yl]oxy}methyl)pyrrolidine-1-carboxylate

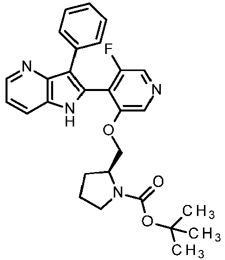

To a solution of 5-fluoro-4-(3-phenyl-1H-pyrrolo[3,2-b]pyridin-2-yl)pyridin-3-ol (61.0 mg, 200 µmol), tert-butyl (2S)-2-(hydroxymethyl)pyrrolidine-1-carboxylate (44.2 mg, 220 µmol) and triphenylphosphine (62.9 mg, 240 µmol) in THF (2 ml), dipropan-2-yl (E)-diazene-1,2-dicarboxylate (50 µl, 94 % purity, 240 µmol) was added at rt. After 30 min, water (2 ml) and saturated aqueous ammonium chloride solution (2 ml) were added and the mixture was extracted with EtOAc twice. The combined organic layers were dried over sodium sulfate, filtered, and concentrated under reduced pressure. The residue was purified by reverse phase preparative HPLC (method 3) yielding 63.0 mg (100 % purity, 65 % yield) of the desired product.
LC-MS (method 1): Rₜ = 1.50 min; MS (ESlpos): m/z = 489 [M+H]⁺
¹H-NMR (400 MHz, DMSO-d6) δ [ppm]: 11.83 (s br, 1H), 8.56-8.36 (m, 3H), 7.89-7.80 (m, 1H), 7.61-7.51 (m, 2H), 7.33-7.14 (m, 4H), 4.25-3.64 (m, 5H), 3.07-2.78 (m, 2H), 1.71-1.55 (m, 1H), 1.52-1.27 (m, 10 H)

### Intermediate 188

### 2-(3-fluoro-5-{[(2S)-pyrrolidin-2-yl]methoxy}pyridin-4-yl)-3-phenyl-1H-pyrrolo[3,2-b]pyridine hydrogen chloride (1/1)

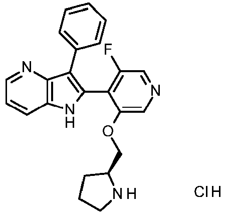

To a solution of tert-butyl (2S)-2-({[5-fluoro-4-(3-phenyl-1H-pyrrolo[3,2-b]pyridin-2-yl)pyridin-3-yl]oxy}methyl)pyrrolidine-1-carboxylate (60.0 mg, 123 µmol) in DCM (2 ml), hydrogen chloride (610 µl, 4.0 M in 1,4 dioxane, 2.5 mmol) was added at rt. A few drops of MeOH were added until a clear solution was obtained. After stirring for 36 h, all volatiles were removed under reduced pressure and the remaining material was dried in vacuo to give 62.5 mg (100 % purity, 120 % yield) of the title compound that was used without further purification.
LC-MS (method 10): Rₜ = 1.69 min; MS (ESlpos): m/z = 389 [M+H]⁺
¹H-NMR (400 MHz, DMSO-d6) δ [ppm]: 13.96 (s br, 1H), 10.00-9.49 (m, 2H), 8.80-8.63 (m, 2H), 8.60-8.52 (m, 1H), 8.41-8.32 (m, 1H), 7.85-7.75 (m, 1H), 7.50-7.33 (m, 5H), 4.51-4.41 (m, 2H), 3.53-3.43 (m, 1H), 3.14-2.97 (m, 2H), 2.08-1.93 (m, 1H), 1.82-1.59 (m, 3H)

### Intermediate 189

### tert-butyl (2S,4S)-2-{[(4-bromopyridin-3-yl)oxy]methyl}-4-methylpyrrolidine-1-carboxylate

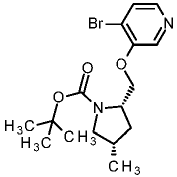

To a solution of 4-bromopyridin-3-ol (808 mg, 4.64 mmol) in THF (12 ml), tert-butyl (2S,4S)-2-(hydroxymethyl)-4-methylpyrrolidine-1-carboxylate (1.00 g, 4.64 mmol) and triphenylphosphine (1.83 g, 6.97 mmol) were added. The mixture was cooled to 0°C and dipropan-2-yl (E)-diazene-1,2-dicarboxylate (1.4 ml, 98 % purity, 7.0 mmol) was added slowly. The mixture was stirred at rt for 3 h. Water (5 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 986 mg (98 % purity, 56 % yield) of the desired product.
LC-MS (method 2): Rₜ = 2.23 min; MS (ESlpos): m/z = 371 [M+H]⁺

### Intermediate 190

### tert-butyl (2S,4S)-4-methyl-2-[({4-[(trimethylsilyl)ethynyl]pyridin-3-yl}oxy)methyl]pyrrolidine-1-carboxylate

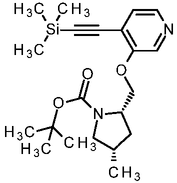

To a solution of tert-butyl (2S,4S)-2-{[(4-bromopyridin-3-yl)oxy]methyl}-4-methylpyrrolidine-1-carboxylate (986 mg, 2.66 mmol), ethynyl(trimethyl)silane (740 µl, 5.3 mmol) and Cu(I)I (20 mg, 106 µmol) in DMF (5 ml) triethylamine (3.5 ml) was added at rt. The mixture was carefully degassed and purged with argon. Dichlorobis(triphenylphosphine)palladium(ll) (37.3 mg, 53.1 µmol; CAS-RN:[13965-03-2]) was added and the reaction mixture was heated to 80°C for 2 h. After cooling to rt, water (5 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 815 mg (93 % purity, 73 % yield) of the desired product.
LC-MS (method 1): Rₜ = 2.59 min; MS (ESlpos): m/z = 389 [M+H]⁺

### Intermediate 191

### tert-butyl (2S,4S)-2-[({4-[(3-aminopyridin-2-yl)ethynyl]pyridin-3-yl}oxy)methyl]-4-methylpyrrolidine-1-carboxylate

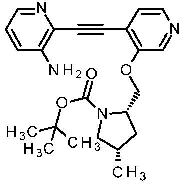

To a solution of tert-butyl (2S,4S)-4-methyl-2-[({4-[(trimethylsilyl)ethynyl]pyridin-3-yl}oxy)methyl]pyrrolidine-1-carboxylate (815 mg, 2.10 mmol), 2-bromopyridin-3-amine (363 mg, 2.10 mmol), Cu(I)I (20 mg, 105 µmol) and dichlorobis(triphenylphosphine)palladium(ll) (73.6 mg, 105 µmol; CAS-RN:[13965-03-2]) in DMF (6 ml) triethylamine (1.8 ml) and TBAF (2.1 ml, 1.0 M in THF, 2.1 mmol) were added at rt under argon. The reaction mixture was heated to 100°C for 40 min. After cooling to rt, water (10 ml) and EtOAc (10 ml) were added and the mixture was filtered through a pad of celite. The aqueous layer was extracted with EtOAc three times. The combined organic layers were dried over sodium sulfate, filtered and concentrated under reduced pressure. The residue was purified by silica gel column chromatography (eluent: DCM/MeOH 40:1) yielding 676 mg (83 % purity, 65 % yield) of the desired product.
LC-MS (method 2): Rₜ = 1.79 min; MS (ESlpos): m/z = 409 [M+H]⁺

### Intermediate 192

### tert-butyl (2S,4S)-4-methyl-2-({[4-(1H-pyrrolo[3,2-b]pyridin-2-yl)pyridin-3-yl]oxy}methyl)pyrrolidine-1-carboxylate

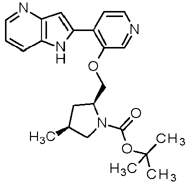

A solution of tert-butyl (2S,4S)-2-[({4-[(3-aminopyridin-2-yl)ethynyl]pyridin-3-yl}oxy)methyl]-4-methylpyrrolidine-1-carboxylate (676 mg, 1.65 mmol) in NMP (5 ml) was carefully degassed and purged with argon. Potassium tert-butoxide (371 mg, 3.31 mmol) was added at rt and the mixture was heated to 90°C for 30 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 436 mg (100 % purity, 64 % yield) of the desired product.
LC-MS (method 1): Rₜ = 1.27 min; MS (ESlpos): m/z = 409 [M+H]⁺
¹H-NMR (600 MHz, DMSO-d6) δ [ppm]: 0.990 (6.18), 1.000 (6.12), 1.358 (16.00), 1.517 (0.45), 1.533 (0.47), 2.130 (0.68), 2.279 (0.68), 2.291 (1.24), 2.301 (1.05), 2.312 (1.17), 2.324 (0.55), 2.518 (0.59), 2.521 (0.54), 2.524 (0.45), 2.804 (0.45), 2.822 (0.70), 3.167 (11.08), 3.175 (11.31), 3.727 (0.73), 3.740 (0.74), 4.082 (1.09), 4.091 (3.12), 4.100 (3.13), 4.109 (1.24), 4.259 (0.56), 4.439 (1.72), 7.163 (2.41), 7.171 (2.45), 7.177 (2.45), 7.184 (2.49), 7.298 (0.50), 7.336 (1.22), 7.820 (0.74), 7.853 (1.12), 7.866 (1.62), 8.320 (1.16), 8.370 (2.32), 8.377 (2.30), 8.552 (1.48), 11.745 (0.92).

### Intermediate 193

### tert-butyl (2S,4S)-2-({[4-(3-bromo-1H-pyrrolo[3,2-b]pyridin-2-yl)pyridin-3-yl]oxy}methyl)-4-methylpyrrolidine-1-carboxylate

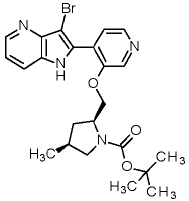

To a solution of tert-butyl (2S,4S)-4-methyl-2-({[4-(1H-pyrrolo[3,2-b]pyridin-2-yl)pyridin-3-yl]oxy}methyl)pyrrolidine-1-carboxylate (435 mg, 1.06 mmol) in DCM (5 ml), 1-bromopyrrolidine-2,5-dione (190 mg, 1.06 mmol) was added at 0°C. After warming to rt within 30 min stirring at rt was continued for 2 h. Saturated sodium hydrogencarbonatee solution (aqueous, 1 mL) was added and the mixture diluted with DCM. The mixture was dried over a water repellent filter and concentrated under reduced pressure. After drying in vacuo, 500 mg (99 % purity, 95 % yield) of the desired product were obtained that were used without further purification.
LC-MS (method 1): Rₜ = 1.72 min; MS (ESlpos): m/z = 487 [M+H]⁺
¹H-NMR (400 MHz, DMSO-d6) δ [ppm]: 0.608 (5.33), 0.622 (5.05), 1.292 (16.00), 1.366 (1.08), 1.885 (0.78), 1.978 (0.69), 1.996 (1.18), 2.009 (1.05), 2.026 (1.08), 2.174 (0.86), 2.200 (0.88), 2.564 (2.24), 3.268 (0.52), 3.291 (0.68), 3.307 (1.13), 3.386 (1.94), 3.406 (1.12), 3.429 (0.86), 3.942 (0.83), 4.232 (1.29), 4.337 (0.48), 4.445 (0.71), 5.751 (5.31), 7.235 (1.98), 7.247 (2.12), 7.256 (2.16), 7.267 (2.18), 7.569 (0.69), 7.632 (1.00), 7.809 (3.22), 7.829 (3.02), 8.375 (1.61), 8.426 (2.55), 8.436 (2.54), 8.600 (1.32), 11.986 (4.06).

### Intermediate 194

### tert-butyl (2S,4S)-4-methyl-2-({[4-(3-phenyl-1H-pyrrolo[3,2-b]pyridin-2-yl)pyridin-3-yl]oxy}methyl)pyrrolidine-1-carboxylate

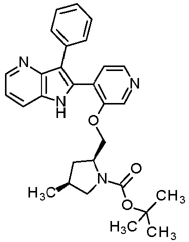

A solution of tert-butyl (2S,4S)-2-({[4-(3-bromo-1H-pyrrolo[3,2-b]pyridin-2-yl)pyridin-3-yl]oxy}methyl)-4-methylpyrrolidine-1-carboxylate (300 mg, 616 µmol) and phenylboronic acid (113 mg, 923 µmol) in a mixture of 1-propanole and water (5:1, 5 ml) was carefully degassed and purged with argon. Triphenylphosphine (16.1 mg, 61.6 µmol), potassium carbonate (255 mg, 1.85 mmol) and the palladium catalyst bis(triphenylphosphine)palladium(ll) dichloride (43 mg, 62 µmol; CAS-RN:[13965-03-2]) were added. The mixture was heated to 100°C and stirred for 4.5 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 192 mg (100 % purity, 64 % yield) of the desired product.
LC-MS (method 1): Rₜ = 1.48 min; MS (ESlpos): m/z = 485 [M+H]⁺
¹H-NMR (400 MHz, DMSO-d6) δ [ppm]: -0.017 (0.56), -0.008 (16.00), 0.556 (7.14), 0.571 (7.29), 1.185 (1.36), 1.209 (1.35), 1.237 (0.56), 1.304 (14.79), 1.842 (1.33), 2.090 (0.44), 2.188 (0.53), 3.277 (0.47), 3.305 (1.29), 3.318 (1.29), 3.383 (3.29), 3.391 (2.30), 3.416 (0.84), 3.630 (0.48), 3.825 (0.52), 4.124 (1.62), 4.274 (0.53), 5.743 (5.74), 7.186 (4.16), 7.197 (4.30), 7.206 (3.74), 7.218 (3.28), 7.264 (2.97), 7.530 (2.79), 7.787 (3.31), 7.790 (3.46), 7.808 (3.20), 7.811 (3.04), 8.218 (0.94), 8.247 (0.82), 8.396 (2.92), 8.399 (3.03), 8.407 (3.16), 8.410 (2.88), 8.522 (7.12), 11.700 (3.78).

### Intermediate 195

### 2-(3-{[(2S,4S)-4-methylpyrrolidin-2-yl]methoxy}pyridin-4-yl)-3-phenyl-1H-pyrrolo[3,2-b]pyridine

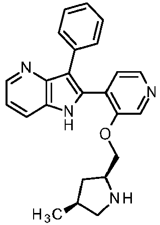

To a solution of tert-butyl (2S,4S)-4-methyl-2-({[4-(3-phenyl-1H-pyrrolo[3,2-b]pyridin-2-yl)pyridin-3-yl]oxy}methyl)pyrrolidine-1-carboxylate (191 mg, 394 µmol) in DCM (2.8 ml), hydrogen chloride (790 µl, 4.0 M in 1,4 dioxane, 3.2 mmol) was added at rt. After stirring for 16 h, all volatiles were removed under reduced pressure. The residue was dissolved in DCM (5 ml) and saturated aqueous sodium hydrogencarbonate solution (0.5 ml) was added. The mixture was diluted with DCM (5 ml) and dried over a water repellent filter. After concentration under reduced pressure and drying in vacuo 148 mg (100 % purity, 98 % yield) of the title compound were obtained that were used without further purification.
LC-MS (method 2): Rₜ = 0.67 min; MS (ESlneg): m/z = 383 [M-H]⁻
¹H-NMR (400 MHz, DMSO-d6) δ [ppm]: 0.798 (1.02), 0.820 (1.75), 0.829 (1.31), 0.852 (16.00), 0.868 (15.69), 1.232 (0.51), 1.847 (0.84), 1.866 (1.83), 1.877 (0.96), 1.885 (1.39), 1.897 (1.71), 1.915 (1.28), 1.928 (0.45), 1.947 (0.89), 1.966 (1.30), 1.985 (1.20), 2.003 (0.76), 2.206 (2.52), 2.227 (2.41), 2.232 (2.77), 2.253 (2.25), 2.369 (0.46), 2.713 (0.40), 2.905 (2.21), 2.923 (2.37), 2.931 (2.25), 2.949 (2.02), 3.218 (2.09), 3.237 (3.45), 3.248 (4.02), 3.256 (4.25), 3.267 (4.84), 3.568 (1.80), 3.667 (0.75), 3.679 (0.80), 3.699 (0.78), 3.713 (0.62), 3.914 (1.93), 3.933 (2.18), 3.939 (2.82), 3.957 (2.49), 4.034 (2.61), 4.045 (2.67), 4.058 (1.94), 4.069 (1.76), 5.752 (1.20), 7.198 (3.64), 7.211 (7.80), 7.219 (5.12), 7.223 (6.71), 7.230 (5.04), 7.234 (4.07), 7.253 (2.77), 7.315 (4.60), 7.335 (7.90), 7.353 (3.78), 7.532 (7.25), 7.549 (6.40), 7.553 (4.65), 7.824 (4.00), 7.827 (4.06), 7.844 (3.84), 7.848 (3.57), 8.171 (7.36), 8.183 (7.01), 8.395 (4.10), 8.398 (4.14), 8.406 (4.19), 8.409 (3.79), 8.560 (10.48).

### Intermediate 196

### tert-butyl (2S)-2-{[(4-bromopyridin-3-yl)oxy]methyl}-4,4-difluoropyrrolidine-1-carboxylate

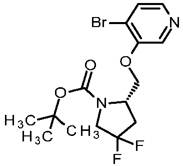

To a solution of 4-bromopyridin-3-ol (733 mg, 4.21 mmol) in THF (10 ml), tert-butyl (2S)-4,4-difluoro-2-(hydroxymethyl)pyrrolidine-1-carboxylate (1.00 g, 4.21 mmol) and triphenylphosphine (1.66 g, 6.32 mmol) were added. The mixture was cooled to 0°C and dipropan-2-yl (E)-diazene-1,2-dicarboxylate (1.3 ml, 98 % purity, 6.3 mmol) was added slowly. The mixture was stirred at rt for 1 h. Water (5 ml) was added and the mixture was extracted with EtOAc twice. The combined organic layers were dried over sodium sulfate, filtered and concentrated under reduced pressure. The residue was purified by reverse phase preparative HPLC (method 3) yielding 1.13 g (100 % purity, 68 % yield) of the desired product.
LC-MS (method 1): Rₜ = 2.00 min; MS (ESlpos): m/z = 393 [M+H]⁺

### Intermediate 197

### tert-butyl (2S)-4,4-difluoro-2-[({4-[(trimethylsilyl)ethynyl]pyridin-3-yl}oxy)methyl]pyrrolidine-1-carboxylate

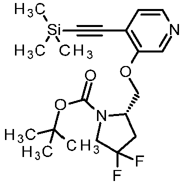

To a solution of tert-butyl (2S)-2-{[(4-bromopyridin-3-yl)oxy]methyl}-4,4-difluoropyrrolidine-1-carboxylate (1.13 g, 2.87 mmol), ethynyl(trimethyl)silane (800 µl, 5.7 mmol) and Cu(I)I (22 mg, 115 µmol) in DMF (5 ml) triethylamine (3.8 ml) was added at rt. The mixture was carefully degassed and purged with argon. Dichlorobis(triphenylphosphine)palladium(ll) (40 mg, 57 µmol; CAS-RN:[13965-03-2]) was added and the reaction mixture was heated to 80°C for 1 h. After cooling to rt, water (5 ml) was added, the mixture was filtered through a pad of Celite and was then extracted with EtOAc twice. The combined organic layers were dried over sodium sulfate, filtered and concentrated under reduced pressure. The residue was purified by silica gel column chromatography (eluent: cyclohexane/EtOAc 1:1) yielding 1.09 g (97 % purity, 90 % yield) of the desired product.
LC-MS (method 2): Rₜ = 2.53 min; MS (ESlpos): m/z = 411 [M+H]⁺

### Intermediate 198

### tert-butyl (2S)-2-[({4-[(3-aminopyridin-2-yl)ethynyl]pyridin-3-yl}oxy)methyl]-4,4-difluoropyrrolidine-1-carboxylate

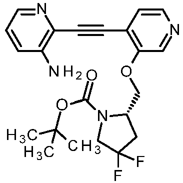

To a solution of tert-butyl (2S)-4,4-difluoro-2-[({4-[(trimethylsilyl)ethynyl]pyridin-3-yl}oxy)methyl]pyrrolidine-1-carboxylate (1.09 g, 2.66 mmol), 2-bromopyridin-3-amine (459 mg, 2.66 mmol), Cu(I)I (25 mg, 133 µmol) and dichlorobis(triphenylphosphine)palladium(ll) (93.2 mg, 133 µmol; CAS-RN:[13965-03-2]) in DMF (4 ml) triethylamine (2.2 ml) and TBAF (2.7 ml, 1.0 M in THF, 2.7 mmol) were added at rt under argon. The reaction mixture was heated to 100°C for 40 min. After cooling to rt, water (10 ml) and EtOAc (10 ml) were added and the mixture was filtered through a pad of celite. The aqueous layer was extracted with EtOAc four times. The combined organic layers were dried over sodium sulfate, filtered and concentrated under reduced pressure. The residue was purified by silica gel column chromatography (eluent: DCM/MeOH 20:1) yielding 590 mg (100 % purity, 52 % yield) of the desired product.
LC-MS (method 2): Rₜ = 1.74 min; MS (ESlpos): m/z = 431 [M+H]⁺

### Intermediate 199

### tert-butyl (2S)-4,4-difluoro-2-({[4-(1H-pyrrolo[3,2-b]pyridin-2-yl)pyridin-3-yl]oxy}methyl)pyrrolidine-1-carboxylate

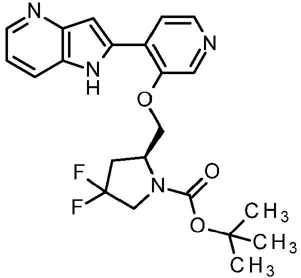

A solution of tert-butyl (2S)-2-[({4-[(3-aminopyridin-2-yl)ethynyl]pyridin-3-yl}oxy)methyl]-4,4-difluoropyrrolidine-1-carboxylate (570 mg, 1.32 mmol) in NMP (6 ml) was carefully degassed and purged with argon. Potassium tert-butoxide (297 mg, 2.65 mmol) was added at rt and the mixture was heated to 90°C for 1 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 417 mg (60 % purity, 44 % yield) of the desired product.

The title compound contained 40% of
tert-butyl (2S)-4-fluoro-2-({[4-(1H-pyrrolo[3,2-b]pyridin-2-yl)pyridin-3-yl]oxy}methyl)-2,3-dihydro-1H-pyrrole-1-carboxylate 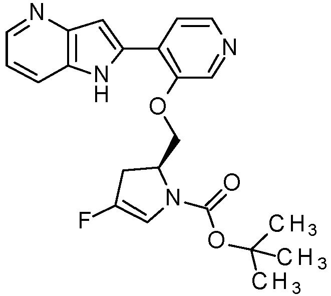
that could not be separated by reverse phase preparative HPLC (method 3). The proposed structure was confirmed by structure elucidation at the final step of the sequence (Examples 112 and 113).
LC-MS (method 1): Rₜ = 1.20 min; MS (ESlpos): m/z = 431 [M+H]⁺ and Rₜ = 1.13 min; MS (ESlpos): m/z = 411 [M+H]⁺

### Intermediate 200

### tert-butyl (2S)-2-({[4-(3-bromo-1H-pyrrolo[3,2-b]pyridin-2-yl)pyridin-3-yl]oxy}methyl)-4,4-difluoropyrrolidine-1-carboxylate

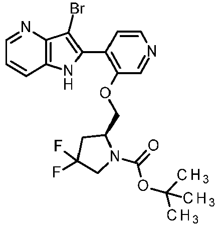

To a solution of a mixture of tert-butyl (2S)-4,4-difluoro-2-({[4-(1H-pyrrolo[3,2-b]pyridin-2-yl)pyridin-3-yl]oxy}methyl)pyrrolidine-1-carboxylate and tert-butyl (2S)-4-fluoro-2-({[4-(1H-pyrrolo[3,2-b]pyridin-2-yl)pyridin-3-yl]oxy}methyl)-2,3-dihydro-1H-pyrrole-1-carboxylate (60:40) (430 mg, 999 µmol) in DCM (4 ml), 1-bromopyrrolidine-2,5-dione (178 mg, 999 mmol) was added at 0°C. After warming to rt within 30 min stirring at rt was continued for 1.5 h. Saturated sodium hydrogencarbonate solution (aqueous, 1 mL) was added and the mixture diluted with DCM. The mixture was dried over a water repellent filter and concentrated under reduced pressure. The residue was purified by reverse phase preparative HPLC (method 3) yielding 419 mg (60 % purity, 49 % yield) of the desired product.

The title compound contained 40% of tert-butyl (2S)-2-({[4-(3-bromo-1H-pyrrolo[3,2-b]pyridin-2-yl)pyridin-3-yl]oxy}methyl)-4-fluoro-2,3-dihydro-1H-pyrrole-1-carboxylate 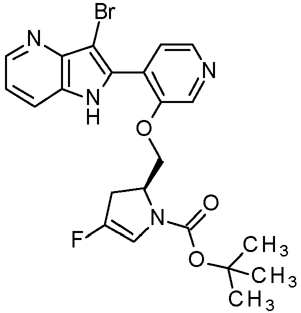 that could not be separated by reverse phase preparative HPLC (method 3). The proposed structure was confirmed by structure elucidation at the final step of the sequence (Examples 112 and 113).
LC-MS (method 1): Rₜ = 1.62 min; MS (ESlpos): m/z = 509 [M+H]⁺ and Rₜ = 1.56 min; MS (ESlpos): m/z = 489 [M+H]⁺

### Intermediate 201

### tert-butyl (2S)-4,4-difluoro-2-({[4-(3-phenyl-1H-pyrrolo[3,2-b]pyridin-2-yl)pyridin-3-yl]oxy}methyl)pyrrolidine-1-carboxylate

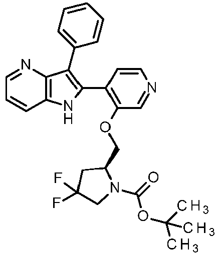

A solution of a mixture of tert-butyl (2S)-2-({[4-(3-bromo-1H-pyrrolo[3,2-b]pyridin-2-yl)pyridin-3-yl]oxy}methyl)-4,4-difluoropyrrolidine-1-carboxylate and tert-butyl (2S)-2-({[4-(3-bromo-1H-pyrrolo[3,2-b]pyridin-2-yl)pyridin-3-yl]oxy}methyl)-4-fluoro-2,3-dihydro-1H-pyrrole-1-carboxylate (60:40) (419 mg, 823 µmol) and phenylboronic acid (150 mg, 1.23 mmol) in a mixture of 1-propanole and water (5:1, 12 ml) was carefully degassed and purged with argon. Triphenylphosphine (21.6 mg, 82.3 µmol), potassium carbonate (341 mg, 2.47 mmol) and the palladium catalyst bis(triphenylphosphine)palladium(ll) dichloride (57.7 mg, 82.3 µmol; CAS-RN:[13965-03-2]) were added. The mixture was heated to 100°C and stirred for 4.5 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 297 mg (58 % purity, 41 % yield) of the desired product.

The title compound contained 42% of tert-butyl (2S)-4-fluoro-2-({[4-(3-phenyl-1H-pyrrolo[3,2-b]pyridin-2-yl)pyridin-3-yl]oxy}methyl)-2,3-dihydro-1H-pyrrole-1-carboxylate 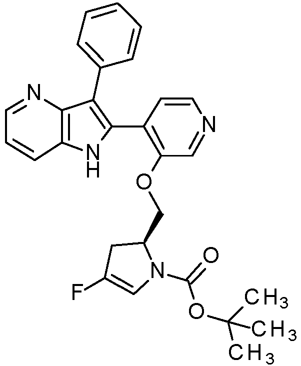 that could not be separated by reverse phase preparative HPLC (method 3). The proposed structure was confirmed by structure elucidation at the final step of the sequence (Examples 112 and 113).
LC-MS (method 1): Rₜ = 1.44 min; MS (ESlpos): m/z = 507 [M+H]⁺ and Rₜ = 1.39 min; MS (ESlpos): m/z = 487 [M+H]⁺
¹H-NMR (400 MHz, DMSO-d6) δ [ppm]: 1.313 (8.52), 1.327 (14.68), 1.340 (8.01), 2.327 (0.65), 2.523 (0.94), 3.284 (0.42), 3.295 (0.78), 3.303 (0.54), 3.311 (0.69), 3.323 (1.36), 3.331 (1.85), 3.336 (2.90), 3.385 (1.74), 3.389 (1.59), 3.398 (0.91), 3.402 (1.09), 3.409 (1.03), 3.416 (0.79), 3.437 (0.53), 3.493 (0.51), 3.526 (0.52), 3.560 (0.60), 3.584 (0.56), 3.881 (0.45), 4.080 (0.51), 4.168 (0.60), 4.195 (0.64), 4.230 (0.42), 4.890 (0.40), 4.985 (0.44), 5.747 (16.00), 7.176 (0.90), 7.195 (3.67), 7.201 (2.73), 7.207 (1.82), 7.213 (3.94), 7.216 (3.13), 7.222 (2.25), 7.227 (1.62), 7.233 (2.07), 7.269 (2.29), 7.288 (3.44), 7.307 (2.05), 7.528 (3.14), 7.547 (2.23), 7.786 (1.40), 7.792 (2.42), 7.795 (2.22), 7.807 (1.39), 7.812 (2.27), 7.816 (1.92), 8.226 (0.47), 8.238 (0.51), 8.274 (0.76), 8.404 (1.20), 8.408 (1.35), 8.412 (2.26), 8.416 (3.17), 8.423 (2.07), 8.427 (1.86), 8.537 (2.24), 8.560 (4.58), 11.652 (0.60), 11.727 (1.09).

### Intermediate 202

### 2-(3-{[(2S)-4,4-difluoropyrrolidin-2-yl]methoxy}pyridin-4-yl)-3-phenyl-1H-pyrrolo[3,2-b]pyridine

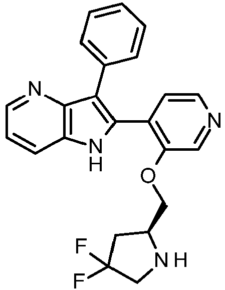

To a solution of a mixture of tert-butyl (2S,4S)-4-methyl-2-({[4-(3-phenyl-1H-pyrrolo[3,2-b]pyridin-2-yl)pyridin-3-yl]oxy}methyl)pyrrolidine-1-carboxylate and tert-butyl (2S)-4-fluoro-2-({[4-(3-phenyl-1H-pyrrolo[3,2-b]pyridin-2-yl)pyridin-3-yl]oxy}methyl)-2,3-dihydro-1H-pyrrole-1-carboxylate (58:42) (191 mg, 394 µmol) in DCM (3 ml), hydrogen chloride (1.2 ml, 4.0 M in 1,4 dioxane, 4.7 mmol) was added at rt. After stirring for 16 h, all volatiles were removed under reduced pressure. The residue was dissolved in DCM (5 ml) and saturated aqueous sodium hydrogencarbonate solution (0.5 ml) was added. The mixture was diluted with DCM (5 ml) and dried over a water repellent filter. After concentration under reduced pressure and drying in vacuo 224 mg (60 % purity, 56 % yield) of the title compound were obtained that were used without further purification.

The title compound contained 40% of 2-(3-{[(2S)-4-fluoro-2,3-dihydro-1H-pyrrol-2-yl]methoxy}pyridin-4-yl)-3-phenyl-1H-pyrrolo[3,2-b]pyridine

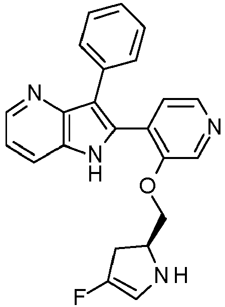

The proposed structure was confirmed by structure elucidation at the final step of the sequence (Examples 112 and 113).
LC-MS (method 1): Rₜ = 0.65 min; MS (ESlpos): m/z = 407 [M+H]⁺ and Rₜ = 0.54 min; MS (ESlpos): m/z = 387 [M+H]⁺

### Intermediate 203

### tert-butyl (2S,4S)-2-{[(4-bromopyridin-3-yl)oxy]methyl}-4-fluoropyrrolidine-1-carboxylate

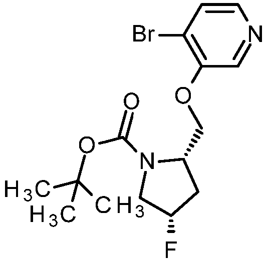

To a solution of 4-bromopyridin-3-ol (733 mg, 4.21 mmol) in THF (18 ml), tert-butyl (2S,4S)-4-fluoro-2-(hydroxymethyl)pyrrolidine-1-carboxylate (1.00 g, 4.56 mmol) and triphenylphosphine (1.79 g, 6.84 mmol) were added. The mixture was cooled to 0°C and dipropan-2-yl (E)-diazene-1,2-dicarboxylate (1.4 ml, 98 % purity, 6.8 mmol) was added slowly. The mixture was stirred at rt for 1 h. Water (5 ml) was added and the mixture was extracted with EtOAc twice. The combined organic layers were dried over sodium sulfate, filtered and concentrated under reduced pressure. The residue was purified by silica gel column chromatography (eluent: cyclohexane/EtOAc 1:1) yielding 1.00 g (99 % purity, 58 % yield) of the desired product.
LC-MS (method 2): Rₜ = 1.97 min; MS (ESlpos): m/z = 375 [M+H]⁺

### Intermediate 204

### tert-butyl (2S,4S)-4-fluoro-2-[({4-[(trimethylsilyl)ethynyl]pyridin-3-yl}oxy)methyl]pyrrolidine-1-carboxylate

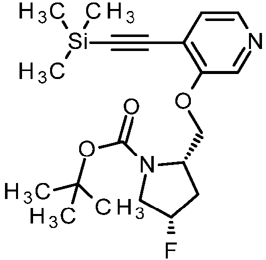

To a solution of tert-butyl (2S,4S)-2-{[(4-bromopyridin-3-yl)oxy]methyl}-4-fluoropyrrolidine-1-carboxylate (1.00 g, 2.66 mmol), ethynyl(trimethyl)silane (740 µl, 5.3 mmol) and Cu(I)I (20 mg, 107 µmol) in DMF (5 ml) triethylamine (3.5 ml) was added at rt. The mixture was carefully degassed and purged with argon. Dichlorobis(triphenylphosphine)palladium(ll) (37.4 mg, 53.3 µmol; CAS-RN:[13965-03-2]) was added and the reaction mixture was heated to 80°C for 1 h. After cooling to rt, water (5 ml) was added, the mixture was filtered through a pad of Celite and was then extracted with EtOAc twice. The combined organic layers were dried over sodium sulfate, filtered and concentrated under reduced pressure. The residue was purified by reverse phase preparative HPLC (method 3) yielding 601 mg (100 % purity, 57 % yield) of the desired product.
LC-MS (method 1): Rₜ = 2.35 min; MS (ESlpos): m/z = 393 [M+H]⁺

### Intermediate 205

### tert-butyl (2S,4S)-2-[({4-[(3-aminopyridin-2-yl)ethynyl]pyridin-3-yl}oxy)methyl]-4-fluoropyrrolidine-1-carboxylate

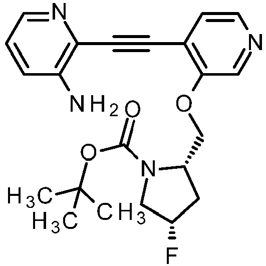

To a solution of tert-butyl (2S,4S)-4-fluoro-2-[({4-[(trimethylsilyl)ethynyl]pyridin-3-yl}oxy)methyl]pyrrolidine-1-carboxylate (600 mg, 1.53 mmol), 2-bromopyridin-3-amine (264 mg, 1.53 mmol), Cu(I)I (15 mg, 76 µmol) and dichlorobis(triphenylphosphine)palladium(ll) (53.6 mg, 76.4 µmol; CAS-RN:[13965-03-2]) in DMF (3 ml) triethylamine (1.3 ml) and TBAF (1.5 ml, 1.0 M in THF, 1.5 mmol) were added at rt under argon. The reaction mixture was heated to 100°C for 60 min. After cooling to rt, water (10 ml) and EtOAc (10 ml) were added and the mixture was filtered through a pad of celite. The aqueous layer was extracted with EtOAc four times. The combined organic layers were dried over sodium sulfate, filtered and concentrated under reduced pressure. The residue was purified by reverse phase preparative HPLC (method 3) yielding 413 mg (95 % purity, 62 % yield) of the desired product.
LC-MS (method 1): Rₜ = 1.50 min; MS (ESlpos): m/z = 413 [M+H]⁺

### Intermediate 206

### tert-butyl (2S,4S)-4-fluoro-2-({[4-(1H-pyrrolo[3,2-b]pyridin-2-yl)pyridin-3-yl]oxy}methyl)pyrrolidine-1-carboxylate

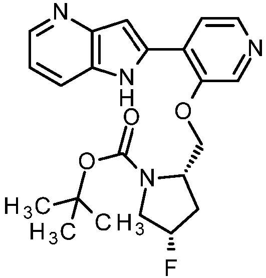

A solution of tert-butyl (2S,4S)-2-[({4-[(3-aminopyridin-2-yl)ethynyl]pyridin-3-yl}oxy)methyl]-4-fluoropyrrolidine-1-carboxylate (413 mg, 1.00 mmol) in NMP (3 ml) was carefully degassed and purged with argon. Potassium tert-butoxide (225 mg, 2.00 mmol) was added at rt and the mixture was heated to 90°C for 30 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 303 mg (75 % purity, 55 % yield) of the desired product.

The title compound contained 25 % of
tert-butyl (2S)-2-({[4-(1H-pyrrolo[3,2-b]pyridin-2-yl)pyridin-3-yl]oxy}methyl)-2,5-dihydro-1H-pyrrole-1-carboxylate 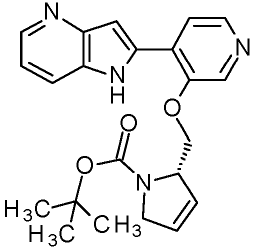
that could not be separated by reverse phase preparative HPLC (method 3). The proposed structure was confirmed by structure elucidation at the final step of the sequence (Examples 114 and 115).
LC-MS (method 1): Rₜ = 1.14 min; MS (ESlpos): m/z = 413 [M+H]⁺ and Rₜ = 1.10 min; MS (ESlpos): m/z = 393 [M+H]⁺

### Intermediate 207

### tert-butyl (2S,4S)-2-({[4-(3-bromo-1H-pyrrolo[3,2-b]pyridin-2-yl)pyridin-3-yl]oxy}methyl)-4-fluoropyrrolidine-1-carboxylate

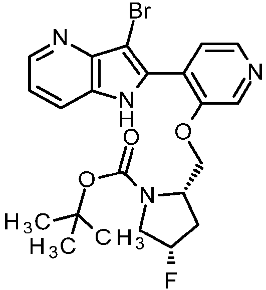

To a solution of a mixture of tert-butyl (2S,4S)-4-fluoro-2-({[4-(1H-pyrrolo[3,2-b]pyridin-2-yl)pyridin-3-yl]oxy}methyl)pyrrolidine-1-carboxylate and tert-butyl (2S)-2-({[4-(1H-pyrrolo[3,2-b]pyridin-2-yl)pyridin-3-yl]oxy}methyl)-2,5-dihydro-1H-pyrrole-1-carboxylate (75:25) (303 mg, 735 µmol) in DCM (2 ml), 1-bromopyrrolidine-2,5-dione (131 mg, 735 µmol) was added at 0°C. After warming to rt within 30 min stirring at rt was continued for 30 min. Saturated sodium hydrogencarbonate solution (aqueous, 1 mL) was added and the mixture diluted with DCM. The mixture was dried over a water repellent filter and concentrated under reduced pressure. After draying in vacuo 419 mg 326 mg (65 % purity, 59 % yield) of the desired product that were used without further purification.

The title compound contained 26% of tert-butyl (2S)-2-({[4-(3-bromo-1H-pyrrolo[3,2-b]pyridin-2-yl)pyridin-3-yl]oxy}methyl)-2,5-dihydro-1H-pyrrole-1-carboxylate.

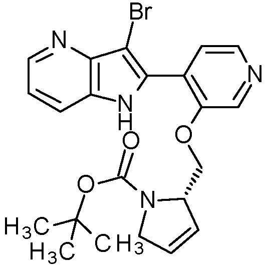

The proposed structure was confirmed by structure elucidation at the final step of the sequence (Examples 114 and 115).
LC-MS (method 1): Rₜ = 1.54 min; MS (ESlpos): m/z = 491 [M+H]⁺ and Rₜ = 1.48 min; MS (ESlpos): m/z = 471 [M+H]⁺

### Intermediate 208

### tert-butyl (2S,4S)-4-fluoro-2-({[4-(3-phenyl-1H-pyrrolo[3,2-b]pyridin-2-yl)pyridin-3-yl]oxy}methyl)pyrrolidine-1-carboxylate

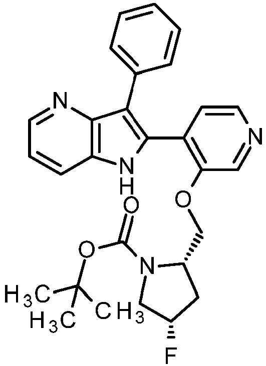

A solution of a mixture of tert-butyl (2S,4S)-2-({[4-(3-bromo-1H-pyrrolo[3,2-b]pyridin-2-yl)pyridin-3-yl]oxy}methyl)-4-fluoropyrrolidine-1-carboxylate and tert-butyl (2S)-2-({[4-(3-bromo-1H-pyrrolo[3,2-b]pyridin-2-yl)pyridin-3-yl]oxy}methyl)-2,5-dihydro-1H-pyrrole-1-carboxylate (71:29) (326 mg, 91 % purity, 604 µmol) and phenylboronic acid (110 mg, 906 µmol) in a mixture of 1-propanole and water (5:1, 8 ml) was carefully degassed and purged with argon. Triphenylphosphine (15.8 mg, 60.4 µmol), potassium carbonate (250 mg, 1.81 mmol) and the palladium catalyst bis(triphenylphosphine)palladium(ll) dichloride (42.4 mg, 60.4 µmol; CAS-RN:[13965-03-2]) were added. The mixture was heated to 100°C and stirred for 1 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 203 mg (74 % purity, 51 % yield) of the desired product.

The title compound contained 26% of tert-butyl (2S)-2-({[4-(3-phenyl-1H-pyrrolo[3,2-b]pyridin-2-yl)pyridin-3-yl]oxy}methyl)-2,5-dihydro-1H-pyrrole-1-carboxylate 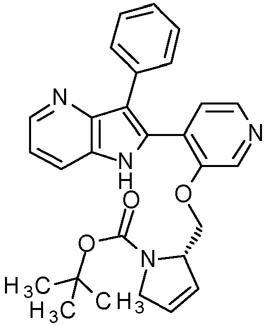
that could not be separated by reverse phase preparative HPLC (method 3). The proposed structure was confirmed by structure elucidation at the final step of the sequence (Examples 114 and 115).
LC-MS (method 1): Rₜ = 1.39 min; MS (ESlpos): m/z = 489 [M+H]⁺ and Rₜ = 1.36 min; MS (ESlpos): m/z = 469 [M+H]⁺

### Intermediate 209

### 2-(3-{[(2S,4S)-4-fluoropyrrolidin-2-yl]methoxy}pyridin-4-yl)-3-phenyl-1H-pyrrolo[3,2-b]pyridine

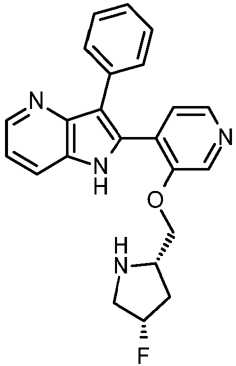

To a solution of a mixture of tert-butyl (2S,4S)-4-fluoro-2-({[4-(3-phenyl-1H-pyrrolo[3,2-b]pyridin-2-yl)pyridin-3-yl]oxy}methyl)pyrrolidine-1-carboxylate and tert-butyl (2S)-2-({[4-(3-phenyl-1H-pyrrolo[3,2-b]pyridin-2-yl)pyridin-3-yl]oxy}methyl)-2,5-dihydro-1H-pyrrole-1-carboxylate (74:26) (203 mg, 416 µmol) in DCM (2 ml), hydrogen chloride (830 µl, 4.0 M in 1,4-dioxane, 3.3 mmol) was added at rt. After stirring for 16 h, all volatiles were removed under reduced pressure. The residue was dissolved in DCM (5 ml) and saturated aqueous sodium hydrogencarbonate solution (0.5 ml) was added. The mixture was diluted with DCM (5 ml) and dried over a water repellent filter. After concentration under reduced pressure and drying in vacuo 160 mg (76 % purity, 75 % yield) of the title compound were obtained that were used without further purification.

The title compound contained 22 % of 2-{3-[(2S)-2,5-dihydro-1H-pyrrol-2-ylmethoxy]pyridin-4-yl}-3-phenyl-1H-pyrrolo[3,2-b]pyridine

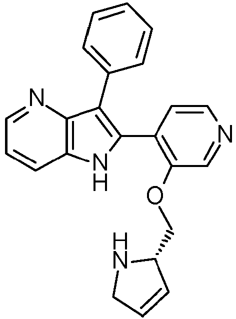

The proposed structure was confirmed by structure elucidation at the final step of the sequence (Examples 114 and 115).
LC-MS (method 8): Rₜ = 1.24 min; MS (ESlpos): m/z = 389 [M+H]⁺ and Rₜ = 1.29 min; MS (ESlpos): m/z = 369 [M+H]⁺

### Intermediate 210

### tert-butyl (3RS)-3-[(4-bromopyridin-3-yl)oxy]pyrrolidine-1-carboxylate

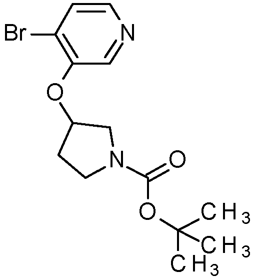

To a solution of 4-bromopyridin-3-ol (1.00 g, 5.75 mmol) in THF (8 ml), tert-butyl (3RS)-3-hydroxypyrrolidine-1-carboxylate (1.08 g, 5.75 mmol) and triphenylphosphine (1.81 g, 6.90 mmol) were added. The mixture was cooled to 0°C and dipropan-2-yl (E)-diazene-1,2-dicarboxylate (1.4 ml, 98 % purity, 6.8 mmol) was added slowly. The mixture was stirred at rt for 30 min. Water (5 ml) and saturated aqueous ammonium chloride solution (5 ml) were added and the mixture was extracted with EtOAc twice. The combined organic layers were dried over sodium sulfate, filtered and concentrated under reduced pressure. The residue was purified by reverse phase preparative HPLC (method 3) yielding 1.87 g (100 % purity, 95 % yield) of the desired product.
LC-MS (method 1): Rₜ = 1.79 min; MS (ESlpos): m/z = 343 [M+H]⁺

### Intermediate 211

### tert-butyl (3RS)-3-({4-[(trimethylsilyl)ethynyl]pyridin-3-yl}oxy)pyrrolidine-1-carboxylate

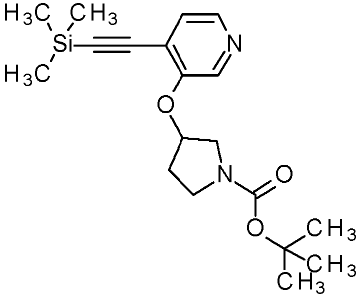

To a solution of tert-butyl (3RS)-3-[(4-bromopyridin-3-yl)oxy]pyrrolidine-1-carboxylate (1.87 g, 5.45 mmol), ethynyl(trimethyl)silane (1.5 ml, 11 mmol) and Cu(I)I (42 mg, 218 µmol) in DMF (8 ml) triethylamine (7.2 ml) was added at rt. The mixture was carefully degassed and purged with argon. Dichlorobis(triphenylphosphine)palladium(ll) (76.5 mg, 109 µmol; CAS-RN:[13965-03-2]) was added and the reaction mixture was heated to 80°C for 1 h. After cooling to rt, water (5 ml) was added, the mixture was filtered through a pad of Celite and was then extracted with EtOAc twice. The combined organic layers were dried over sodium sulfate, filtered and concentrated under reduced pressure. The residue was purified by reverse phase preparative HPLC (method 3) yielding 1.52 g (100 % purity, 77 % yield) of the desired product.
LC-MS (method 1): Rₜ = 2.28 min; MS (ESlpos): m/z = 361 [M+H]⁺
¹H-NMR (400 MHz, DMSO-d6) δ [ppm]: 0.227 (16.00), 1.379 (5.00), 1.399 (5.27), 2.065 (0.40), 3.424 (0.86), 3.455 (1.16), 5.221 (0.46), 5.751 (0.50), 7.371 (0.46), 7.381 (0.48).

### Intermediate 212

### tert-butyl (3RS)-3-({4-[(3-aminopyridin-2-yl)ethynyl]pyridin-3-yl}oxy)pyrrolidine-1-carboxylate

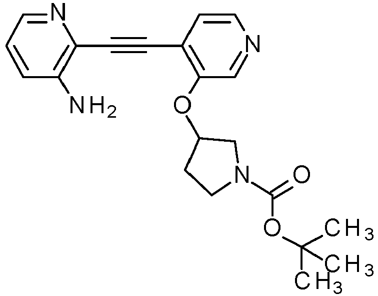

To a solution of tert-butyl (3RS)-3-({4-[(trimethylsilyl)ethynyl]pyridin-3-yl}oxy)pyrrolidine-1-carboxylate (1.52 g, 4.22 mmol), 2-bromopyridin-3-amine (729 mg, 4.22 mmol), Cu(I)I (40 mg, 211 µmol) and dichlorobis(triphenylphosphine)palladium(ll) (148 mg, 211 µmol; CAS-RN:[13965-03-2]) in DMF (20 ml) triethylamine (3.5 ml) and TBAF (4.2 ml, 1.0 M in THF, 4.2 mmol) were added at rt under argon. The reaction mixture was heated to 100°C for 40 min. After cooling to rt, water (20 ml) and EtOAc (20 ml) were added and the mixture was filtered through a pad of celite. The layers were separated and the aqueous layer was extracted with EtOAc two times. The combined organic layers were dried over sodium sulfate, filtered and concentrated under reduced pressure. The residue was purified by reverse phase preparative HPLC (method 3) yielding 0.81 g (95 % purity, 48 % yield) of the desired product.
LC-MS (method 2): Rₜ = 1.53 min; MS (ESlpos): m/z = 381 [M+H]⁺
¹H-NMR (400 MHz, DMSO-d6) δ [ppm]: 1.359 (16.00), 2.137 (0.53), 3.356 (0.67), 3.438 (1.58), 3.465 (1.55), 5.294 (1.01), 5.659 (1.86), 5.752 (0.66), 7.140 (4.20), 7.147 (4.04), 7.547 (0.40), 7.565 (0.45), 7.595 (0.64), 7.614 (0.87), 7.625 (1.16), 7.840 (0.75).

### Intermediate 213

### tert-butyl (3RS)-3-{[4-(3-bromo-1H-pyrrolo[3,2-b]pyridin-2-yl)pyridin-3-yl]oxy}pyrrolidine-1-carboxylate

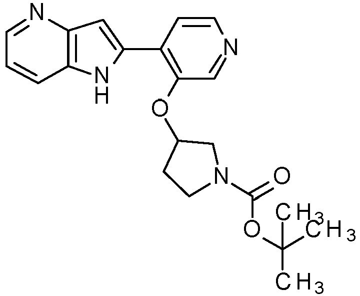

A solution of tert-butyl (3RS)-3-({4-[(3-aminopyridin-2-yl)ethynyl]pyridin-3-yl}oxy)pyrrolidine-1-carboxylate (700 mg, 1.84 mmol) in NMP (7 ml) was carefully degassed and purged with argon. Potassium tert-butoxide (413 mg, 3.68 mmol) was added at rt and the mixture was heated to 90°C for 30 min. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 559 mg (94 % purity, 75 % yield) of the desired product.
LC-MS (method 1): Rₜ = 0.89 min; MS (ESlpos): m/z = 381 [M+H]⁺
¹H-NMR (400 MHz, DMSO-d6) δ [ppm]: 1.330 (16.00), 1.365 (13.69), 1.986 (0.48), 2.243 (2.58), 2.522 (0.69), 3.161 (7.78), 3.174 (8.00), 3.291 (0.46), 3.367 (0.43), 3.375 (0.43), 3.389 (0.55), 3.448 (1.54), 3.462 (1.88), 3.476 (1.26), 3.556 (0.74), 3.586 (4.30), 3.642 (0.68), 4.079 (0.67), 4.092 (1.93), 4.105 (1.86), 4.118 (0.61), 5.375 (2.56), 7.146 (1.87), 7.157 (1.98), 7.167 (2.01), 7.178 (1.97), 7.217 (3.23), 7.546 (0.60), 7.548 (0.57), 7.554 (0.50), 7.564 (0.69), 7.571 (0.55), 7.596 (0.84), 7.613 (0.76), 7.625 (1.09), 7.630 (0.51), 7.643 (0.53), 7.798 (3.42), 7.819 (4.23), 7.836 (2.65), 8.354 (3.00), 8.572 (1.33), 11.681 (2.81).

### Intermediate 214

### tert-butyl (3RS)-3-{[4-(3-bromo-1H-pyrrolo[3,2-b]pyridin-2-yl)pyridin-3-yl]oxy}pyrrolidine-1-carboxylate

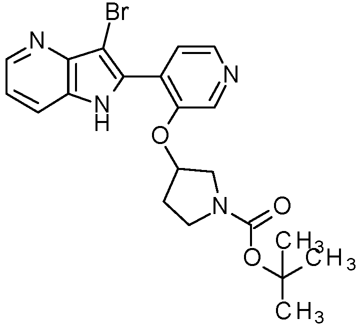

To a solution of tert-butyl (3RS)-3-{[4-(1H-pyrrolo[3,2-b]pyridin-2-yl)pyridin-3-yl]oxy}pyrrolidine-1-carboxylate (559 mg, 1.47 mmol) in DCM (1 ml), 1-bromopyrrolidine-2,5-dione (262 mg, 1.47 mmol) was added at 0°C. After warming to rt within 30 min stirring at rt was continued for 30 min. Saturated sodium hydrogencarbonate solution (aqueous, 1 mL) was added and the mixture diluted with DCM. The mixture was dried over a water repellent filter and concentrated under reduced pressure. The residue was purified by silica gel column chromatography (eluent: cyclohexane/EtOAc 1:1) yielding 524 mg (99 % purity, 77 % yield) of the desired product.
LC-MS (method 1): Rₜ = 1.33 min; MS (ESlpos): m/z = 459 [M+H]⁺
¹H-NMR (400 MHz, DMSO-d6) δ [ppm]: 1.154 (0.71), 1.172 (1.45), 1.190 (0.74), 1.297 (16.00), 1.986 (2.57), 2.040 (0.69), 2.080 (0.43), 2.091 (0.69), 2.102 (0.86), 2.114 (0.57), 2.125 (0.72), 2.522 (0.64), 3.160 (4.07), 3.174 (4.06), 3.288 (1.02), 3.296 (1.00), 3.441 (0.63), 3.481 (0.69), 3.516 (0.54), 3.547 (0.55), 4.017 (0.57), 4.036 (0.56), 4.089 (0.90), 4.102 (0.87), 5.160 (1.37), 5.752 (0.49), 7.237 (2.05), 7.248 (2.07), 7.258 (2.12), 7.269 (2.16), 7.585 (1.33), 7.595 (1.37), 7.817 (2.68), 7.821 (2.80), 7.838 (2.53), 7.841 (2.41), 8.399 (5.05), 8.411 (4.79), 8.426 (2.20), 8.429 (2.23), 8.437 (2.18), 8.440 (2.01), 8.619 (1.64), 11.957 (0.75), 11.982 (0.81).

### Intermediate 215

### tert-butyl (3RS)-3-{[4-(3-phenyl-1H-pyrrolo[3,2-b]pyridin-2-yl)pyridin-3-yl]oxy}pyrrolidine-1-carboxylate

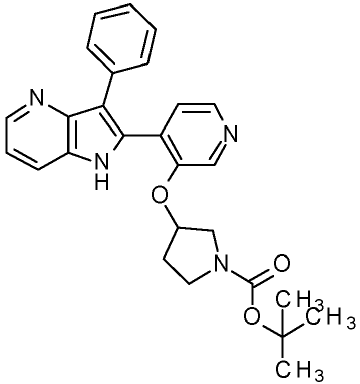

To a solution of tert-butyl (3RS)-3-{[4-(3-bromo-1H-pyrrolo[3,2-b]pyridin-2-yl)pyridin-3-yl]oxy}pyrrolidine-1-carboxylate (250 mg, 544 µmol) and phenylboronic acid (66 mg, 544 µmol) in 1,4-dioxane (4 ml), aqueous potassium phosphate solution (816 µl, 2M, 1.6 mmol) was added. The mixture was carefully degassed and purged with argon. The palladium catalyst chloro(2-dicyclohexylphosphino-2',4',6'-triisopropyl-1,1'-biphenyl)[2-(2'-amino-1,1'-biphenyl)]palladium(ll) (42.4 mg, 60.4 µmol; XPhos PD G2, CAS-RN:[1310584-14-5]) was added and the mixture was heated to 100°C and stirred for 4 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 176 mg (73 % purity, 52 % yield) of the desired product.
LC-MS (method 1): Rₜ = 1.20 min; MS (ESlpos): m/z = 457 [M+H]⁺
¹H-NMR (400 MHz, DMSO-d6) δ [ppm]: 1.154 (1.05), 1.172 (2.11), 1.190 (1.08), 1.298 (16.00), 1.986 (3.98), 2.041 (0.71), 2.080 (0.45), 2.092 (0.67), 2.103 (0.81), 2.114 (0.54), 2.125 (0.65), 3.157 (0.44), 3.219 (0.50), 3.409 (0.69), 3.441 (0.74), 3.480 (0.77), 3.516 (0.59), 3.565 (3.52), 4.017 (0.91), 4.036 (0.90), 5.161 (1.31), 7.242 (1.45), 7.253 (1.48), 7.262 (1.54), 7.274 (1.50), 7.596 (1.36), 7.824 (1.92), 7.827 (1.88), 7.845 (1.82), 8.401 (2.11), 8.412 (2.05), 8.428 (1.91), 8.439 (1.85), 8.619 (1.46), 11.970 (0.75), 11.993 (0.80).

### Intermediate 216

### 3-phenyl-2-(3-{[(3RS)-pyrrolidin-3-yl]oxy}pyridin-4-yl)-1H-pyrrolo[3,2-b]pyridine

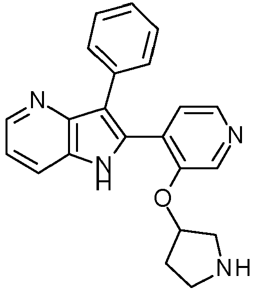

To a solution of tert-butyl (3RS)-3-{[4-(3-phenyl-1H-pyrrolo[3,2-b]pyridin-2-yl)pyridin-3-yl]oxy}pyrrolidine-1-carboxylate (176 mg, 386 µmol) in DCM (2 ml), hydrogen chloride (960 µl, 4.0 M in 1,4-dioxane, 3.9 mmol) was added at rt. A thick precipitate formed and MeOH was added dropwise until a clear solution was obtained. After stirring for 16 h, all volatiles were removed under reduced pressure. The residue was dissolved in DCM (5 ml) and saturated aqueous sodium hydrogencarbonate solution (1 ml) was added. The mixture was diluted with DCM (5 ml) and dried over a water repellent filter. After concentration under reduced pressure and drying in vacuo 120 mg (100 % purity, 87 % yield) of the title compound were obtained that were used without further purification.
LC-MS (method 1): Rₜ = 0.52 min; MS (ESlneg): m/z = 355 [M-H]⁻
¹H-NMR (400 MHz, DMSO-d6) δ [ppm]: 1.530 (0.45), 1.601 (2.31), 1.838 (0.46), 1.852 (0.64), 1.872 (0.71), 2.074 (16.00), 2.567 (0.90), 2.672 (0.53), 3.098 (1.55), 3.114 (2.07), 3.133 (1.61), 3.168 (4.88), 3.225 (1.74), 3.256 (2.87), 3.305 (5.05), 4.933 (0.82), 7.134 (1.24), 7.146 (1.40), 7.154 (1.89), 7.168 (1.87), 7.190 (1.09), 7.269 (3.36), 7.287 (4.48), 7.306 (2.02), 7.377 (0.55), 7.388 (0.55), 7.504 (3.01), 7.522 (2.53), 7.790 (0.42), 7.810 (0.41), 7.885 (1.17), 7.905 (1.07), 8.197 (1.79), 8.209 (1.78), 8.236 (0.63), 8.248 (0.54), 8.356 (2.03), 8.367 (2.01), 8.415 (0.84), 8.468 (2.40).

### Intermediate 217

### tert-butyl (3RS)-3-({4-[3-(3-chlorophenyl)-1H-pyrrolo[3,2-b]pyridin-2-yl]pyridin-3-yl}oxy)pyrrolidine-1-carboxylate

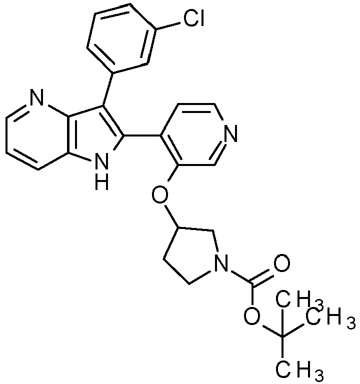

To a solution of tert-butyl (3RS)-3-{[4-(3-bromo-1H-pyrrolo[3,2-b]pyridin-2-yl)pyridin-3-yl]oxy}pyrrolidine-1-carboxylate (250 mg, 544 µmol) and 3-chlorophenyl)boronic acid (86.8 mg, 555 µmol) in 1,4-dioxane (5 ml), aqueous potassium phosphate solution (830 µl, 2M, 1.7 mmol) was added. The mixture was carefully degassed and purged with argon. The palladium catalyst chloro(2-dicyclohexylphosphino-2',4',6'-triisopropyl-1,1'-biphenyl)[2-(2'-amino-1,1'-biphenyl)]palladium(II) (65.5 mg, 83.3 µmol; XPhos PD G2, CAS-RN:[1310584-14-5]) was added and the mixture was heated to 100°C and stirred for 1 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 120 mg (93 % purity, 41 % yield) of the desired product.
LC-MS (method 1): Rₜ = 1.50 min; MS (ESlpos): m/z = 491 [M+H]⁺
¹H-NMR (400 MHz, DMSO-d6) δ [ppm]: 1.272 (15.20), 1.315 (16.00), 1.603 (0.95), 1.951 (0.94), 2.709 (0.51), 2.727 (0.58), 2.746 (0.61), 2.837 (0.65), 2.877 (0.96), 2.909 (0.82), 3.023 (0.73), 3.054 (0.85), 3.167 (6.73), 3.285 (1.22), 3.376 (2.15), 3.405 (1.56), 3.418 (1.40), 4.944 (0.95), 4.987 (0.97), 7.129 (0.57), 7.147 (1.14), 7.164 (0.65), 7.215 (1.97), 7.230 (4.79), 7.237 (4.26), 7.240 (5.29), 7.250 (3.41), 7.262 (3.98), 7.277 (1.52), 7.339 (1.05), 7.355 (1.25), 7.373 (1.41), 7.390 (1.68), 7.408 (0.78), 7.464 (1.45), 7.484 (2.30), 7.495 (2.48), 7.671 (1.51), 7.709 (1.47), 7.835 (3.26), 7.854 (3.01), 8.093 (1.00), 8.112 (0.92), 8.366 (3.47), 8.378 (3.32), 8.450 (3.83), 8.453 (3.92), 8.461 (3.88), 8.464 (3.62), 8.492 (1.96), 8.513 (1.84), 11.854 (4.32).

### Intermediate 218

### 3-(3-chlorophenyl)-2-(3-{[(3RS)-pyrrolidin-3-yl]oxy}pyridin-4-yl)-1H-pyrrolo[3,2-b]pyridine

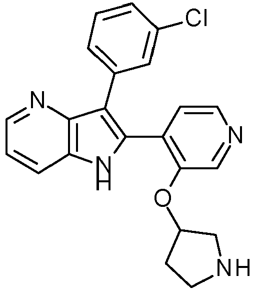

To a solution of tert-butyl (3RS)-3-({4-[3-(3-chlorophenyl)-1H-pyrrolo[3,2-b]pyridin-2-yl]pyridin-3-yl}oxy)pyrrolidine-1-carboxylate (120 mg, 244 µmol) in DCM (2 ml), hydrogen chloride (610 µl, 4.0 M in 1,4-dioxane, 2,4 mmol) was added at rt. A thick precipitate formed and MeOH was added dropwise until a clear solution was obtained. After stirring for 16 h, all volatiles were removed under reduced pressure. The residue was dissolved in DMSO (1 ml) and purified by reverse phase preparative HPLC (method 3) yielding 2 fractions of the desired product that contained considerable amounts of solvent
Fraction 1: 120 mg (93 % purity, 41 % yield)
LC-MS (method 1): Rₜ = 0.74 min; MS (ESlneg): m/z = 389 [M-H]⁻
¹H-NMR (400 MHz, DMSO-d6) δ [ppm]: -0.150 (1.04), 0.146 (1.07), 1.936 (1.28), 2.193 (1.26), 2.317 (3.52), 2.369 (3.32), 2.600 (1.14), 2.713 (3.32), 2.995 (8.34), 3.136 (2.09), 3.472 (16.00), 3.491 (10.67), 3.503 (8.38), 3.667 (2.74), 3.671 (2.36), 3.680 (2.99), 3.699 (3.04), 3.709 (2.03), 3.713 (2.48), 3.724 (1.26), 5.287 (1.71), 7.300 (2.52), 7.408 (3.87), 7.424 (8.06), 7.628 (6.11), 8.491 (1.19), 8.604 (1.46), 9.270 (1.26), 9.556 (1.11), 13.059 (0.47).
Fraction 2: 265 mg (99 % purity, 275 % yield)
LC-MS (method 1): Rₜ = 0.71 min; MS (ESlneg): m/z = 389 [M-H]⁻

### Intermediate 219

### tert-butyl (3RS)-3-[(4-{3-[3-(trifluoromethyl)phenyl]-1H-pyrrolo[3,2-b]pyridin-2-yl}pyridin-3-yl)oxy]pyrrolidine-1-carboxylate

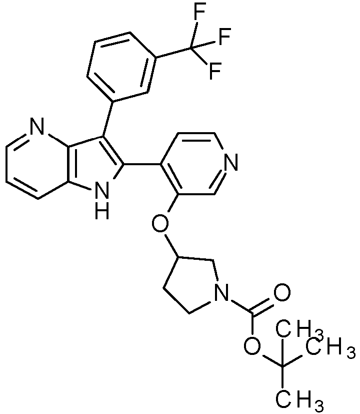

To a solution of tert-butyl (3RS)-3-{[4-(3-bromo-1H-pyrrolo[3,2-b]pyridin-2-yl)pyridin-3-yl]oxy}pyrrolidine-1-carboxylate (346 mg, 753 µmol) and 3-(trifluoromethyl)phenyl]boronic acid (143 mg, 753 µmol) in 1,4-dioxane (5 ml), aqueous potassium phosphate solution (1.1 ml, 2M, 2.3 mmol) was added. The mixture was carefully degassed and purged with argon. The palladium catalyst chloro(2-dicyclohexylphosphino-2',4',6'-triisopropyl-1,1'-biphenyl)[2-(2'-amino-1,1'-biphenyl)]palladium(II) (88.9 mg, 113 µmol µmol; XPhos PD G2, CAS-RN:[1310584-14-5]) was added and the mixture was heated to 100°C and stirred for 1 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 337 mg (100 % purity, 85 % yield) of the desired product.
LC-MS (method 1): Rₜ = 1.69 min; MS (ESlpos): m/z = 525 [M+H]⁺
¹H-NMR (400 MHz, DMSO-d6) δ [ppm]: 1.259 (3.82), 1.302 (4.01), 3.172 (16.00), 3.392 (1.36), 7.151 (0.46), 7.246 (1.04), 7.257 (1.01), 7.266 (1.04), 7.278 (1.06), 7.377 (0.46), 7.469 (0.59), 7.490 (0.46), 7.518 (1.49), 7.523 (1.50), 7.854 (1.08), 7.857 (1.17), 7.874 (0.94), 7.877 (0.88), 8.387 (0.76), 8.399 (0.71), 8.469 (1.29), 8.472 (1.34), 8.480 (1.44), 8.483 (1.44), 11.922 (1.11).

### Intermediate 220

### 2-(3-{[(3RS)-pyrrolidin-3-yl]oxy}pyridin-4-yl)-3-[3-(trifluoromethyl)phenyl]-1H-pyrrolo[3,2-b]pyridine

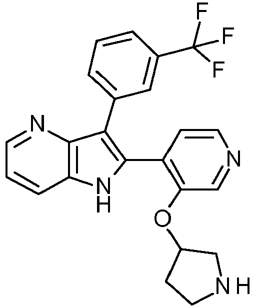

To a solution of tert-butyl (3RS)-3-[(4-{3-[3-(trifluoromethyl)phenyl]-1H-pyrrolo[3,2-b]pyridin-2-yl}pyridin-3-yl)oxy]pyrrolidine-1-carboxylate (337 mg, 642 µmol) in DCM (2 ml), hydrogen chloride (1.6 ml, 4.0 M in 1,4-dioxane, 6.4 mmol) was added at rt. A thick precipitate formed and MeOH was added dropwise until a clear solution was obtained. After stirring for 48 h, all volatiles were removed under reduced pressure. The residue was dissolved in DMSO (1 ml) and purified by reverse phase preparative HPLC (method 3) yielding 121 mg (100 % purity, 44 % yield) of the desired product.
LC-MS (method 1): Rₜ = 0.88 min; MS (ESlneg): m/z = 423 [M-H]⁻

### Intermediate 221

### tert-butyl (3RS)-3-({4-[3-(2-fluoro-5-methylphenyl)-1H-pyrrolo[3,2-b]pyridin-2-yl]pyridin-3-yl}oxy)pyrrolidine-1-carboxylate

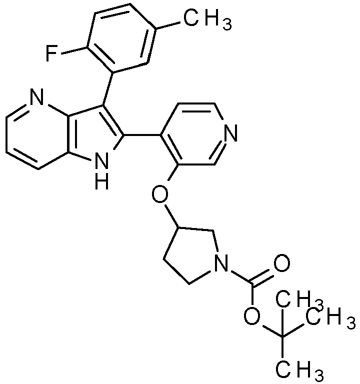

To a solution of tert-butyl (3RS)-3-{[4-(3-bromo-1H-pyrrolo[3,2-b]pyridin-2-yl)pyridin-3-yl]oxy}pyrrolidine-1-carboxylate (346 mg, 753 µmol) and 2-fluoro-5-methylphenyl)boronic acid (116 mg, 753 µmol) in 1,4-dioxane (5 ml), aqueous potassium phosphate solution (1.1 ml, 2M, 2.3 mmol) was added. The mixture was carefully degassed and purged with argon.

The palladium catalyst chloro(2-dicyclohexylphosphino-2',4',6'-triisopropyl-1,1'-biphenyl)[2-(2'-amino-1,1'-biphenyl)]palladium(II) (88.9 mg, 113 µmol µmol; XPhos PD G2, CAS-RN:[1310584-14-5]) was added and the mixture was heated to 100°C and stirred for 1 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 233 mg (100 % purity, 63 % yield) of the desired product.
LC-MS (method 1): Rₜ = 1.27 min; MS (ESlpos): m/z = 489 [M+H]⁺
¹H-NMR (400 MHz, DMSO-d6) δ [ppm]: 0.005 (0.41), 1.280 (16.00), 1.321 (15.91), 1.643 (1.09), 1.661 (1.10), 1.968 (1.04), 2.070 (0.71), 2.302 (14.09), 2.523 (1.09), 2.893 (0.61), 2.912 (0.63), 2.981 (0.99), 3.014 (1.28), 3.052 (0.85), 3.084 (0.84), 3.167 (15.35), 3.417 (1.06), 4.094 (0.83), 4.963 (2.07), 6.950 (1.80), 6.971 (2.97), 6.996 (2.41), 7.082 (1.65), 7.095 (2.02), 7.109 (1.17), 7.201 (3.96), 7.213 (3.90), 7.222 (3.97), 7.233 (4.06), 7.311 (4.90), 7.323 (4.93), 7.413 (1.88), 7.830 (4.45), 7.834 (4.41), 7.851 (4.23), 7.854 (3.81), 8.262 (5.24), 8.274 (5.00), 8.381 (4.43), 8.385 (4.34), 8.392 (4.44), 8.396 (3.94), 8.452 (1.98), 8.469 (1.94), 11.774 (5.01).

### Intermediate 222

### 3-(2-fluoro-5-methylphenyl)-2-(3-{[(3RS)-pyrrolidin-3-yl]oxy}pyridin-4-yl)-1H-pyrrolo[3,2-b]pyridine hydrogen chloride (1/2)

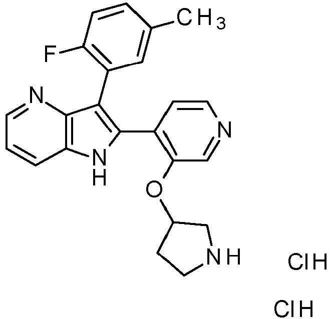

To a solution of tert-butyl (3RS)-3-({4-[3-(2-fluoro-5-methylphenyl)-1H-pyrrolo[3,2-b]pyridin-2-yl]pyridin-3-yl}oxy)pyrrolidine-1-carboxylate (230 mg, 471 µmol) in DCM (2 ml), hydrogen chloride (1.2 ml, 4.0 M in 1,4-dioxane, 4.7 mmol) was added at rt. A thick precipitate formed and MeOH was added dropwise until a clear solution was obtained. After stirring for 48 h, all volatiles were removed under reduced pressure and the residue was dried in vacuo, yielding 284 mg (98 % purity, 128 % yield) of the desired product that was used without further purification.
LC-MS (method 1): Rₜ = 0.62 min; MS (ESlneg): m/z = 387 [M-H]⁻
¹H-NMR (400 MHz, DMSO-d6) δ [ppm]: 13.66 (s br, 1H), 9.93-9.77 (m, 1H), 9.64-9.48 (m, 1H), 8.85-8.60 (m, 3H), 8.35-8.27 (m, 1H), 7.83-7.73 (m, 1H), 7.35-7.14 (m, 4H), 5.43-5.31 (m, 1H), 3.35-3.16 (m, 2H), 2.37-2.21 (m, 1H), 2.30 (s, 2H), 2.06-1.93 (m, 1H).

### Intermediate 223

### tert-butyl (3RS)-3-({4-[3-(5-chloro-2-fluorophenyl)-1H-pyrrolo[3,2-b]pyridin-2-yl]pyridin-3-yl}oxy)pyrrolidine-1-carboxylate

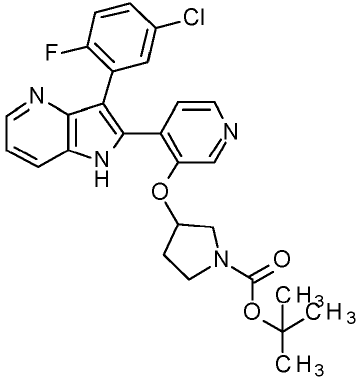

To a solution of tert-butyl (3RS)-3-{[4-(3-bromo-1H-pyrrolo[3,2-b]pyridin-2-yl)pyridin-3-yl]oxy}pyrrolidine-1-carboxylate (346 mg, 753 µmol) and 5-chloro-2-fluorophenyl)boronic acid (131 mg, 753 µmol) in 1,4-dioxane (5 ml), aqueous potassium phosphate solution (1.1 ml, 2M, 2.3 mmol) was added. The mixture was carefully degassed and purged with argon. The palladium catalyst chloro(2-dicyclohexylphosphino-2',4',6'-triisopropyl-1,1'-biphenyl)[2-(2'-amino-1,1'-biphenyl)]palladium(II) (88.9 mg, 113 µmol µmol; XPhos PD G2, CAS-RN:[1310584-14-5]) was added and the mixture was heated to 100°C and stirred for 1 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 195 mg (100 % purity, 51 % yield) of the desired product.
LC-MS (method 1): Rₜ = 1.45 min; MS (ESlpos): m/z = 509 [M+H]⁺
¹H-NMR (400 MHz, DMSO-d6) δ [ppm]: 1.261 (13.62), 1.314 (14.41), 1.615 (0.97), 1.974 (0.92), 2.070 (0.45), 2.883 (0.56), 2.904 (0.60), 2.946 (0.98), 2.979 (1.19), 3.056 (0.69), 3.085 (0.77), 3.167 (16.00), 3.199 (1.20), 3.261 (0.57), 3.410 (1.44), 4.098 (1.05), 4.988 (1.70), 7.145 (1.69), 7.167 (3.30), 7.191 (2.10), 7.240 (3.08), 7.251 (3.07), 7.261 (3.13), 7.272 (3.14), 7.337 (1.43), 7.344 (1.96), 7.354 (1.71), 7.366 (1.72), 7.376 (1.74), 7.393 (2.41), 7.714 (1.34), 7.862 (3.52), 7.865 (3.24), 7.883 (3.33), 8.308 (3.75), 8.320 (3.57), 8.422 (3.94), 8.425 (3.67), 8.433 (3.96), 8.436 (3.26), 8.482 (2.06), 11.955 (4.33).

### Intermediate 224

### 3-(5-chloro-2-fluorophenyl)-2-(3-{[(3RS)-pyrrolidin-3-yl]oxy}pyridin-4-yl)-1H-pyrrolo[3,2-b]pyridine hydrogen chloride (1/1)

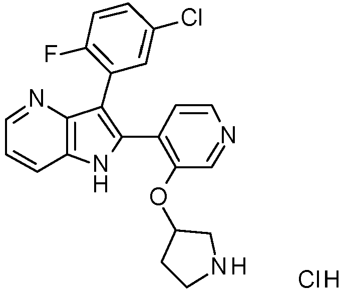

To a solution of tert-butyl (3RS)-3-({4-[3-(5-chloro-2-fluorophenyl)-1H-pyrrolo[3,2-b]pyridin-2-yl]pyridin-3-yl}oxy)pyrrolidine-1-carboxylate (195 mg, 383 µmol) in DCM (2 ml), hydrogen chloride (960 µl, 4.0 M in 1,4-dioxane, 3.8 mmol) was added at rt. A thick precipitate formed and MeOH was added dropwise until a clear solution was obtained. After stirring for 48 h, all volatiles were removed under reduced pressure and the residue was dried in vacuo, yielding 230 mg (97 % purity, 131 % yield) of the desired product that was used without further purification.
LC-MS (method 1): Rₜ = 0.73 min; MS (ESIneg): m/z = 407 [M-H]⁻
¹H-NMR (400 MHz, DMSO-d6) δ [ppm]: 2.010 (0.86), 2.028 (0.90), 2.045 (1.09), 2.263 (0.87), 2.276 (1.27), 2.287 (0.73), 2.298 (1.20), 2.311 (0.73), 2.334 (0.59), 3.168 (0.65), 3.280 (2.71), 3.377 (1.11), 3.399 (1.34), 3.464 (4.61), 3.494 (3.83), 3.504 (3.87), 3.644 (16.00), 5.400 (1.99), 7.315 (3.72), 7.327 (3.71), 7.356 (1.95), 7.378 (3.86), 7.401 (2.34), 7.548 (1.28), 7.555 (1.75), 7.559 (1.60), 7.566 (1.64), 7.570 (1.29), 7.577 (1.60), 7.581 (1.23), 7.588 (1.35), 7.614 (2.04), 7.620 (1.85), 7.629 (2.13), 7.636 (1.61), 7.771 (1.99), 7.786 (2.17), 7.792 (2.12), 7.806 (2.01), 8.354 (4.44), 8.367 (4.17), 8.693 (3.33), 8.705 (3.13), 8.749 (6.14), 8.766 (2.69), 8.787 (2.45), 9.694 (0.87), 9.894 (0.83), 13.771 (2.02).

### Intermediate 225

### 2-(3-fluoro-5-methoxypyridin-4-yl)-1H-pyrrolo[3,2-b]pyridine

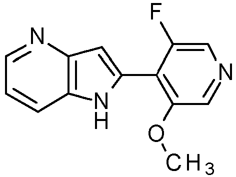

A solution of 2-[(3-fluoro-5-methoxypyridin-4-yl)ethynyl]pyridin-3-amine (650 mg, 2.67 mmol) in DCM (10 ml) was treated with triethylamine (0.93 ml, 6.7 mmol) and cooled to 0°C. Trifluoroacetic anhydride (570 µl, 4.0 mmol) was added and the mixture was allowed to warm to rt within 1 h. The mixture was stirred for 24 h. Additional trifluoroacetic anhydride (570 µl, 4.0 mmol) was added and stirring at rt was continued for 24 h. For work-up, saturated aqueous sodium hydrogencarbonate solution (5 ml) was added carefully. After separation of layers, the aqueous layer was extracted with DCM (10 ml) twice. The combined organic layers were washed with saturated aqueous sodium chloride solution, dried over sodium sulfate, filtered, and concentrated under reduced pressure. The residue was purified by silica gel column chromatography (eluent: cyclohexane/EtOAc 1:1) yielding 165 mg (100 % purity, 25 % yield) of the desired product.
LC-MS (method 1): Rₜ = 0.61 min; MS (ESlpos): m/z = 244 [M+H]⁺
¹H-NMR (400 MHz, DMSO-d6) δ [ppm]: 11.53 (s br, 1H), 8.48 (s, 1H), 8.45-8.38 (m, 2H), 7.94-7.87 (m, 1H), 7.24-7.17 (m, 1H), 7.15-7.10 (m, 1H), 4.10 (s, 3H).

### Intermediate 226

### 3-bromo-2-(3-fluoro-5-methoxypyridin-4-yl)-1H-pyrrolo[3,2-b]pyridine

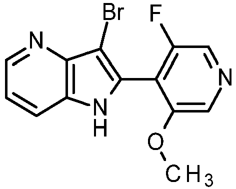

To a solution of 2-(3-fluoro-5-methoxypyridin-4-yl)-1H-pyrrolo[3,2-b]pyridine (178 mg, 732 µmol) in DCM (4 ml), 1-bromopyrrolidine-2,5-dione (156 mg, 878 µmol) was added at rt. After warming to rt within 30 min stirring at rt was continued for 1 h. Saturated sodium hydrogencarbonatee solution (aqueous, 1 mL) was added and the mixture diluted with DCM. The mixture was dried over a water repellent filter and concentrated under reduced pressure. The residue was purified by reverse phase preparative HPLC (method 3) yielding 169 mg (100 % purity, 72 % yield) of the desired product.
LC-MS (method 1): Rₜ = 0.94min; MS (ESlpos): m/z = 322 [M+H]⁺

### Intermediate 227

### 3-(5-chloro-2-fluorophenyl)-2-(3-fluoro-5-methoxypyridin-4-yl)-1H-pyrrolo[3,2-b]pyridine

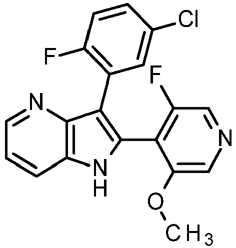

To a solution of 3-bromo-2-(3-fluoro-5-methoxypyridin-4-yl)-1H-pyrrolo[3,2-b]pyridine (169 mg, 525 µmol) and (5-chloro-2-fluorophenyl)boronic acid (91.5 mg, 525 µmol) in 1,4-dioxane (4 ml), aqueous potassium phosphate solution (790 µl, 2M, 1.6 mmol) was added. The mixture was carefully degassed and purged with argon. The palladium catalyst chloro(2-dicyclohexylphosphino-2',4',6'-triisopropyl-1,1'-biphenyl)[2-(2'-amino-1,1'-biphenyl)]palladium(ll) (61.9 mg, 78.7 µmol; XPhos PD G2, CAS-RN:[1310584-14-5]) was added and the mixture was heated to 100°C and stirred for 1 h. After cooling to rt, water (10 ml) was added, and the mixture was extracted with EtOAc twice. The combined organic layers were dried over sodium sulfate, filtered, and concentrated under reduced pressure. The residue was purified by reverse phase preparative HPLC (method 3) yielding 101 mg (100 % purity, 52 % yield) of the desired product.
LC-MS (method 1): Rₜ = 1.18 min; MS (ESlpos): m/z = 372 [M+H]⁺
¹H-NMR (400 MHz, DMSO-d6) δ [ppm]: 2.075 (0.87), 3.171 (1.19), 3.757 (16.00), 5.755 (3.41), 7.183 (1.19), 7.205 (1.84), 7.230 (1.48), 7.265 (1.56), 7.276 (1.55), 7.286 (1.59), 7.297 (1.63), 7.360 (0.77), 7.367 (0.91), 7.371 (0.93), 7.378 (0.87), 7.382 (0.76), 7.389 (0.81), 7.393 (0.73), 7.400 (0.69), 7.625 (1.39), 7.632 (1.37), 7.641 (1.40), 7.648 (1.29), 7.896 (1.57), 7.899 (1.65), 7.916 (1.51), 7.920 (1.46), 8.365 (5.62), 8.422 (4.86), 8.441 (1.84), 8.444 (1.91), 8.452 (1.85), 8.456 (1.71), 12.092 (1.50).

### Intermediate 228

### 4-[3-(5-chloro-2-fluorophenyl)-1H-pyrrolo[3,2-b]pyridin-2-yl]-5-fluoropyridin-3-ol

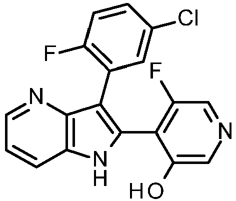

3-(5-chloro-2-fluorophenyl)-2-(3-fluoro-5-methoxypyridin-4-yl)-1H-pyrrolo[3,2-b]pyridine (101 mg, 272 µmol) was dissolved in hydrobromic acid (3.1 ml, 48 % purity, 27 mmol) and stirred at 130 °C for 20 h. After cooling to rt additional (3.1 ml, 48 % purity, 27 mmol) was added and the mixture was stirred additional 10 h at 130°C. After cooling to rt, the reaction mixture was poured carefully into saturated NaHCO3 solution (aqueous, 10 mL). Upon addition of DCM, a solid precipitated and was filtered off. The solid was washed carefully with DCM and dried under vacuum to give 73.5 mg (100 % purity, 76 % yield) of the desired compound.
LC-MS (method 1): Rₜ = 1.00 min; MS (ESlpos): m/z = 358 [M+H]⁺

### Intermediate 229

### tert-butyl (2S)-2-[({4-[3-(5-chloro-2-fluorophenyl)-1H-pyrrolo[3,2-b]pyridin-2-yl]-5-fluoropyridin-3-yl}oxy)methyl]pyrrolidine-1-carboxylate

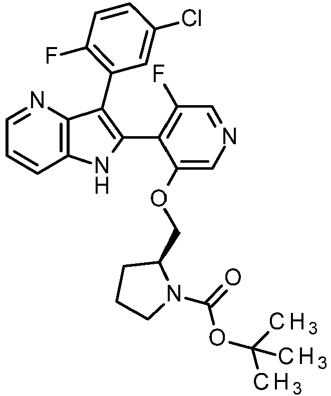

To a solution of 4-[3-(5-chloro-2-fluorophenyl)-1H-pyrrolo[3,2-b]pyridin-2-yl]-5-fluoropyridin-3-ol (73.0 mg, 204 µmol), tert-butyl (2S)-2-(hydroxymethyl)pyrrolidine-1-carboxylate (49.3 mg, 245 µmol) and triphenylphosphine (80.3 mg, 306 µmol) in THF (2 ml), dipropan-2-yl (E)-diazene-1,2-dicarboxylate (61 µl, 98 % purity, 310 µmol) was added at rt. Stirring was continued for 24h. In a separate flask, a solution of triphenylphosphine (80.3 mg, 306 µmol), dipropan-2-yl (E)-diazene-1,2-dicarboxylate (61 µl, 98 % purity, 310 µmol) and tert-butyl (2S)-2-(hydroxymethyl)pyrrolidine-1-carboxylate (49.3 mg, 245 µmol) was prepared at rt. This solution was added to the reaction mixture at rt and stirring was continued. After 18 h, water (3 ml) and saturated aqueous ammonium chloride solution (3 ml) were added and the mixture was extracted with EtOAc twice. The combined organic layers were dried over sodium sulfate, filtered, and concentrated under reduced pressure. The residue was purified by reverse phase preparative HPLC (method 3) yielding 7.70 mg (100 % purity, 7 % yield) of the desired product.
LC-MS (method 1): Rₜ = 1.83 min; MS (ESlpos): m/z = 541 [M+H]⁺

### Intermediate 230

### 3-(5-chloro-2-fluorophenyl)-2-(3-fluoro-5-{[(2S)-pyrrolidin-2-yl]methoxy}pyridin-4-yl)-1H-pyrrolo[3,2-b]pyridine / hydrogen chloride (1/1)

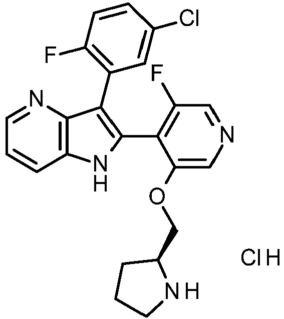

To a solution of tert-butyl (2S)-2-[({4-[3-(5-chloro-2-fluorophenyl)-1H-pyrrolo[3,2-b]pyridin-2-yl]-5-fluoropyridin-3-yl}oxy)methyl]pyrrolidine-1-carboxylate (7.70 mg, 14.2 µmol) in DCM (1 ml), hydrogen chloride (36 µl, 4.0 M in 1,4 dioxane, 140 µmol) was added at rt. A few drops of MeOH were added until a clear solution was obtained. After stirring for 24 h, all volatiles were removed under reduced pressure and the remaining material was dried in vacuo to give 8.50 mg (85 % purity, 106 % yield) of the title compound that was used without further purification.
LC-MS (method 1): Rₜ = 0.88 min; MS (ESlneg): m/z = 439 [M-H]⁻

### Intermediate 231

### 1-[(4-methylphenyl)sulfonyl]-1H-pyrrolo[3,2-b]pyridine

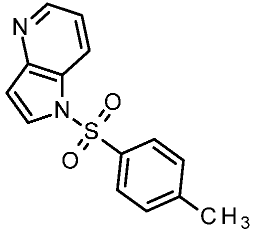

To a solution of 1H-pyrrolo[3,2-b]pyridine (2.00 g, 16.9 mmol) in tetrahydrofuran (50 ml) was added sodium hydride (1.02 g, 25.4 mmol) at 0 °C. After stirring at 0 °C for 0.5 hour, 4-toluenesulfonyl chloride (3.87 g, 20.3 mmol) was added and the resulting mixture was stirred at 0°C for another 1 hour. The reaction mixture was quenched with water, and then poured into water, extracted with ethyl acetate, combined the organic phase and concentrated to give a crude product. The crude product was purified by column chromatography on silica gel (petroleum ether: ethyl acetate = 30: 1 to 2: 1) to give 1-[(4-methylphenyl)sulfonyl]-1H-pyrrolo[3,2-b]pyridine (3.8 g, 14.0 mmol, 82% yield) as a yellow solid.

LC-MS (Method C): Rₜ = 0.828 min; MS (ESlpos): m/z = 273.1 [M+H]⁺.

### Intermediate 232

### 2-iodo-1-[(4-methylphenyl)sulfonyl]-1H-pyrrolo[3,2-b]pyridine

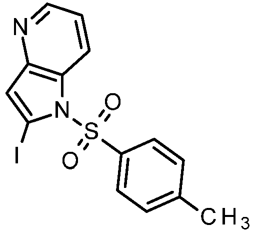

To a solution of 1-[(4-methylphenyl)sulfonyl]-1H-pyrrolo[3,2-b]pyridine (1 g, 3.67 mmol) in tetrahydrofuran (30 ml) was added butyllithium (0.282 g, 4.41 mmol, 2.5 M in tetrahydrofuran) at 20 °C. After stirring at -78 °C for 0.5 hour, iodine (0.932 g, 3.67 mmol) was added and the resulting mixture was stirred at 20 °C for 4 hours. The reaction was quenched with ammonium chloride solution, and the resulting mixture was extracted with ethyl acetate, combined the organic phase and cocentated in vacuo to give a crude product.

The crude product was purified by column chromatography on silica gel (petroleum ether: ethyl acetate = 30: 1 to 3: 1) to give 2-iodo-1-[(4-methylphenyl)sulfonyl]-1H-pyrrolo[3,2-b]pyridine (1.20 g, 3.01 mmol, 82% yield) as a yellow solid.

LC-MS (Method C): Rₜ = 0.883 min; MS (ESlpos): m/z = 399.0 [M+H]⁺.

### Intermediate 233

### 2-(3-methoxypyridin-4-yl)-1-(4-methylbenzene-1-sulfonyl)-1H-pyrrolo[3,2-b]pyridine

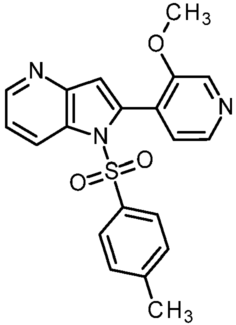

To a solution of 2-iodo-1-[(4-methylphenyl)sulfonyl]-1H-pyrrolo[3,2-b]pyridine (43.0 g, 108 mmol) and (3-methoxypyridin-4-yl)boronic acid (19.8 g, 130 mmol) in 1,4-dioxane (860 ml) and water (215 ml) were added (1,1'-bis(diphenylphosphino)ferrocene)palladium(II) dichloride (7.90 g, 10.8 mmol) and potassium carbonate (22.4 g, 162 mmol) at 20 °C. The mixture was stirred at 90 °C for 16 hours under nitrogen atmosphere. The reaction mixture was poured into water and extracted with ethyl acetate. The combined organic phase was washed with brine, dried over anhydrous sodium sulfate, filtered and concentrated in vacuo to give a crude product. The crude product was purified by column chromatography on silica gel (petroleum ether: ethyl acetate = 50: 1 to 1: 1) to give 2-(3-methoxypyridin-4-yl)-1-[(4-methylphenyl)sulfonyl]-1H-pyrrolo[3,2-b]pyridine (30.0 g, 79.1 mmol, 73% yield) as a yellow solid.

LCMS (Method C): Rₜ = 0.488 min; MS (ESlpos): m/z = 380.1 [M+H]⁺.

### Intermediate 234

### 3-bromo-2-(3-methoxypyridin-4-yl)-1-(4-methylbenzene-1-sulfonyl)-1H-pyrrolo[3,2-b]pyridine

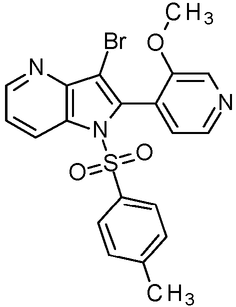

To a solution of 2-(3-methoxypyridin-4-yl)-1-[(4-methylphenyl)sulfonyl]-1H-pyrrolo[3,2-b]pyridine (12.1 g, 31.9 mmol) in N,N-dimethylformamide (100 ml) was added 1-bromopyrrolidine-2,5-dione (1.64 g, 9.22 mmol) at 20 °C. The mixture was stirred at 50 °C for 1 hour. The reaction mixture was poured into water and extracted with ethyl acetate. The combined organic layers were washed with brine, dried over anhydrous sodium sulfate, filtered and the filtrate was concentrated in vacuo to give a residue. The residue was purified by column chromatography on silica gel (petroleum ether: ethyl acetate = 1: 1) to give 3-bromo-2-(3-methoxypyridin-4-yl)-1-(4-methylbenzene-1-sulfonyl)-1H-pyrrolo[3,2-b]pyridine (14.5 g, 31.6 mmol, 99% yield) as a pale yellow oil.

LCMS (Method C): Rₜ = 0.862 min; MS (ESlpos): m/z = 459.8 [M+H]⁺.

### Intermediate 235

### 2-(3-methoxypyridin-4-yl)-1-(4-methylbenzene-1-sulfonyl)-3-phenyl-1H-pyrrolo[3,2-b]pyridine

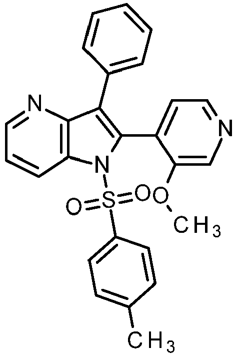

To a solution of 3-bromo-2-(3-methoxypyridin-4-yl)-1-[(4-methylphenyl)sulfonyl]-1H-pyrrolo[3,2-b]pyridine (14.5 g, 31.6 mmol) and phenylboronic acid (5.79 g, 47.5 mmol) in 1,4-dioxane (290 ml) and water (72.5 ml) were added sodium carbonate (5.03 g, 47.5 mmol) and (1,1'-bis(diphenylphosphino)ferrocene)palladium(II) dichloride (2.31 g, 3.16 mmol) at 20 °C. The mixture was stirred at 90 °C for 16 hours under nitrogen atmosphere. The reaction mixture was poured into water and extracted with ethyl acetate. The combined organic phase was washed with brine, dried over anhydrous sodium sulfate, filtered and the filtrate was concentrated in vacuo to give a residue. The residue was purified by column chromatography on silica gel (petroelum ether: ethyl acetate = 50: 1 to 1: 1) to give 2-(3-methoxypyridin-4-yl)-1-(4-methylbenzene-1-sulfonyl)-3-phenyl-1H-pyrrolo[3,2-b]pyridine (9.70 g, 21.3 mmol, 67% yield) as a yellow solid.

LCMS (Method C): Rₜ = 0.591 min; MS (ESlpos): m/z = 456.1 [M+H]⁺.

### Intermediate 236

### 4-(3-phenyl-1H-pyrrolo[3,2-b]pyridin-2-yl)pyridin-3-ol

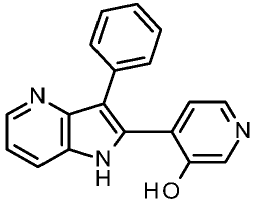

A solution of 2-(3-methoxypyridin-4-yl)-1-[(4-methylphenyl)sulfonyl]-3-phenyl-1H-pyrrolo[3,2-b]pyridine (9.70 g, 21.3 mmol) in hydrobromic acid (40% in water, 300 ml) at 20 °C. The mixture was stirred at 120 °C for 48 hours. after cooled to 20 °C, the pH of the reaction mixture was adjusted to 8~10 with sodium hydrogen carbonate aqueous solution. The resulting suspension was filtered and the solid cake was collected to give 4-(3-phenyl-1H-pyrrolo[3,2-b]pyridin-2-yl)pyridin-3-ol (5.20 g, 18.1 mmol, 85% yiled) as a yellow solid.

LCMS (Method A): Rₜ = 0.411 min; MS (ESlpos): m/z = 288.0 [M+H]⁺.

### Intermediate 237

### 2-(3-{[(2S)-1-benzylpiperidin-2-yl]methoxy}pyridin-4-yl)-3-phenyl-1H-pyrrolo[3,2-b]pyridine

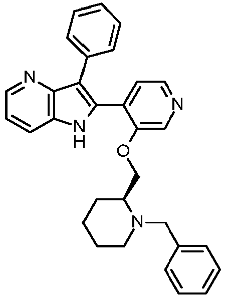

To a solution of 4-(3-phenyl-1H-pyrrolo[3,2-b]pyridin-2-yl)pyridin-3-ol (50.0 mg, 174 µmol) and [(2S)-1-benzylpiperidin-2-yl]methanol (53.6 mg, 261 µmol) in DCM (2 ml) polymer bound triphenylphosphine (220 mg, 31 % purity, 261 µmol) and dipropan-2-yl (E)-diazene-1,2-dicarboxylate (52 µl, 98 % purity, 260 µmol) were added at rt. The mixture was stirred for 20 h. For work-up, the mixture was filtered and the remaining solids were washed wit DCM. After evaporation of the solvent, the residue was purified by silica gel column chromatography (eluent: cyclohexane/EtOAc 1:1) yielding 33 mg (87 % purity, 35 % yield) of the desired product.
LC-MS (method 1): Rₜ = 1.00 min; MS (ESlneg): m/z = 473 [M-H]⁻

### Intermediate 238

### 3-phenyl-2-(3-{[(2S)-piperidin-2-yl]methoxy}pyridin-4-yl)-1H-pyrrolo[3,2-b]pyridine

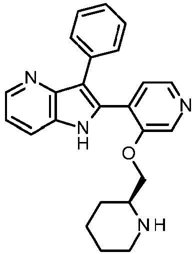

To a solution of 2-(3-{[(2S)-1-benzylpiperidin-2-yl]methoxy}pyridin-4-yl)-3-phenyl-1H-pyrrolo[3,2-b]pyridine (67.5 mg, 142 µmol) in ethanol (4.0 ml), palladiumhydroxide on carbon (9.99 mg, 20 % purity, 14.2 µmol) was added. The mixture was stirred for 24 under atmospheric pressure of hydrogen. The mixture was filtered through a pad of Celite and the solids were washed with methanol. The solvent was removed under reduced pressure and the remaining material was again dissolved in ethanol (4.0 ml). Palladiumhydroxide on carbon (20.0 mg, 20 % purity, 28.4 µmol) was added and stirring at rt under atmospheric pressure of hydrogen was continued for 36 h. Additional palladiumhydroxide on carbon (20.0 mg, 20 % purity, 28.4 µmol) was added and stirring at rt under atmospheric pressure of hydrogen was continued for 24 h. The mixture was filtered through a pad of Celite and the solids were washed with methanol. The solvent was removed under reduced pressure and the remaining material was purified by reverse phase preparative HPLC (method 3) yielding 42.0 mg (77 % purity, 59 % yield) of the desired product.
LC-MS (method 1): Rₜ = 1.00 min; MS (ESlneg): m/z = 383 [M-H]⁻

### Intermediate 239

### tert-butyl (2S,4S)-4-bromo-2-(hydroxymethyl)pyrrolidine-1-carboxylate

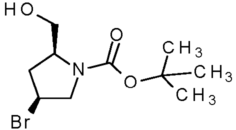

To a solution of 1-tert-butyl 2-methyl (2S,4S)-4-bromopyrrolidine-1,2-dicarboxylate (955 mg, 3.10 mmol) in dry THF (2.0 ml) lithium aluminium hydride (3.1 ml, 1.0 M in THF, 3.1 mmol) was added at 0°C. The mixture was allowed to warm to rt and strirring was continued for 1 h. Water (2 ml) was added and the mixture was extracted with EtOAc twice. The combined organic layers were dried over sodium sulfate, filtered, and concentrated under reduced pressure. After drying in vacuuo 523 mg (93 % purity, 56 % yield) of the desired product were obtained that were used without further purification.
LC-MS (method 1): Rₜ = 1.53 min; MS (ESlpos): m/z = 224 [M+H]-C4H8⁺

### Intermediate 240

### tert-butyl (2S,4S)-4-bromo-2-({[4-(3-phenyl-1H-pyrrolo[3,2-b]pyridin-2-yl)pyridin-3-yl]oxy}methyl)pyrrolidine-1-carboxylate

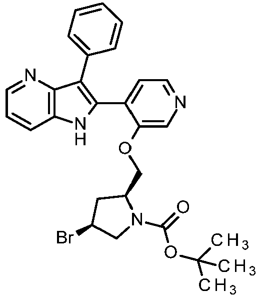

To a solution of 4-(3-phenyl-1H-pyrrolo[3,2-b]pyridin-2-yl)pyridin-3-ol (447 mg, 1.56 mmol), tert-butyl (2S,4S)-4-bromo-2-(hydroxymethyl)pyrrolidine-1-carboxylate (523 mg, 1.87 mmol) and triphenylphosphine (612 mg, 2.33 mmol) in THF (2 ml), dipropan-2-yl (E)-diazene-1,2-dicarboxylate (470 µl, 98 % purity, 2.3 mmol) was added at 0°C. The mixture was allowed to warm to rt and stirring was continued for 1 h. Water (3 ml) was added and the mixture was extracted with EtOAc twice. The combined organic layers were dried over sodium sulfate, filtered, and concentrated under reduced pressure. The residue was purified by reverse phase preparative HPLC (method 3) yielding 153 mg (100 % purity, 18 % yield) of the desired product.
LC-MS (method 1): Rₜ = 1.49 min; MS (ESlpos): m/z = 549 [M+H]⁺

### Intermediate 241

### 2-(3-{[(2S,4S)-4-bromopyrrolidin-2-yl]methoxy}pyridin-4-yl)-3-phenyl-1H-pyrrolo[3,2-b]pyridine hydrogen chloride (1/1)

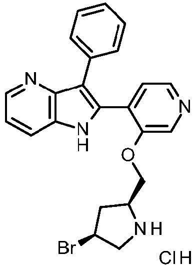

To a solution of tert-butyl (2S,4S)-4-bromo-2-({[4-(3-phenyl-1H-pyrrolo[3,2-b]pyridin-2-yl)pyridin-3-yl]oxy}methyl)pyrrolidine-1-carboxylate (153 mg, 278 µmol) in DCM (4.0 ml), hydrogen chloride (700 µl, 4.0 M in 1,4-dioxane, 2.8 mmol) was added at rt. A few drops of MeOH were added until a clear solution was obtained. After stirring for 24 h, all volatiles were removed under reduced pressure and the remaining material was dried in vacuo to give 188 mg (90 % purity, 125 % yield) of the title compound that was used without further purification.
LC-MS: Rₜ = 0.62 min; MS (ESlpos): m/z = 449 [M+H]⁺

### Intermediate 242

### 1-[(2S,4S)-4-bromo-2-({[4-(3-phenyl-1H-pyrrolo[3,2-b]pyridin-2-yl)pyridin-3-yl]oxy}methyl)pyrrolidin-1-yl]prop-2-en-1-one

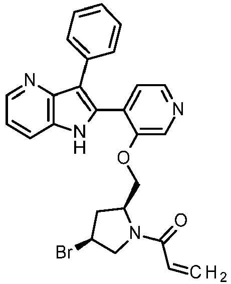

To a solution of 2-(3-{[(2S,4S)-4-bromopyrrolidin-2-yl]methoxy}pyridin-4-yl)-3-phenyl-1H-pyrrolo[3,2-b]pyridine hydrogen chloride (1/1) (150 mg, 309 µmol) in DMF (1 ml), prop-2-enoic acid (25 µl, 370 µmol) and N,N-diisopropylethylamine (320 µl, 1.9 mmol) were added at rt. T3P (270 µl, 50 % purity in DMF, 460 µmol) was added and stirring at rt was continued for 16 h. Water (0.5 ml) was added and the mixture was extracted with EtOAc twice. The combined organic layers were dried over sodium sulfate, filtered, and concentrated under reduced pressure. The residue was purified by reverse phase preparative HPLC (method 3) yielding 54.0 mg (100 % purity, 35 % yield) of the desired product.
LC-MS (method 1): Rₜ = 1.06 min; MS (ESlpos): m/z = 503 [M+H]⁺

### Intermediate 243

### tert-butyl methyl[2-{[4-(3-phenyl-1H-pyrrolo[3,2-b]pyridin-2-yl)pyridin-3-yl]oxy}propyl]carbamate

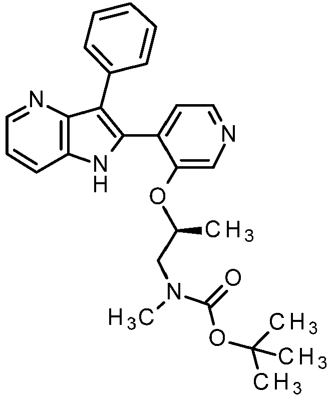

To a solution of 4-(3-phenyl-1H-pyrrolo[3,2-b]pyridin-2-yl)pyridin-3-ol hydrogen chloride (1/1) (300 mg, 0.927 mmol) and tert-butyl [2-hydroxypropyl]methylcarbamate (263 mg, 1.39 mmol, CAS 138373-85-0) in tetrahydrofuran (18 ml) were added triphenylphosphine (486 mg, 1.85 mmol) and diisopropyl azodicarboxylate (375 mg, 1.85 mmol) at 25 °C. The mixture was stirred at 25 °C for 16 hours. The reaction solution was poured into water and extracted with ethyl acetate. The combined organic layers were washed with brine, dried over anhydrous sodium sulfate, filtered and the filtrate was concentrated in vacuo to give a residue. The residue was purified by column chromatography on silica gel (petroleum ether: ethyl acetate = 3: 1) to give tert-butyl methyl[2-{[4-(3-phenyl-1H-pyrrolo[3,2-b]pyridin-2-yl)pyridin-3-yl]oxy}propyl]carbamate (400 mg, 0.872 mmol, 94% yield) as a pale yellow oil.

LCMS (Method C): Rₜ = 0.815 min; MS (ESlpos): m/z = 459.2 [M+H]⁺.

### Intermediate 244

### N-methyl-2-{[4-(3-phenyl-1H-pyrrolo[3,2-b]pyridin-2-yl)pyridin-3-yl]oxy}propan-1-amine hydrogen chloride (1/1)

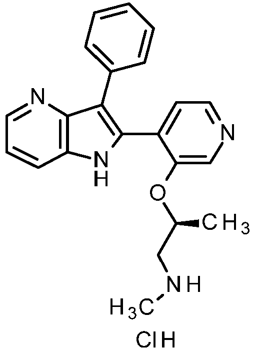

To a solution of tert-butyl methyl[2-{[4-(3-phenyl-1H-pyrrolo[3,2-b]pyridin-2-yl)pyridin-3-yl]oxy}propyl]carbamate (400 mg, 0.872 mmol) in ethyl acetate (10 ml) was added hydrochloric acid (3 ml, 12.0 mmol, 4M in ethyl acetate) at 20 °C. The mixture was stirred at 20 °C for 1 hour. The reaction mixture was filtered and the solid cake was collected to give N-methyl-2-{[4-(3-phenyl-1H-pyrrolo[3,2-b]pyridin-2-yl)pyridin-3-yl]oxy}propan-1-amine hydrogen chloride (1/1) (340 mg, 0.861 mmol, 99% yield) as a brown solid.

LCMS (Method C): Rₜ = 0.428 min; MS (ESlpos): m/z = 359.3 [(M-36)+H]⁺.

### Intermediate 245

### 7-chloro-1-(4-methylbenzene-1-sulfonyl)-1H-pyrrolo[3,2-b]pyridine

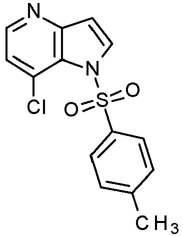

To a solution of 7-chloro-1H-pyrrolo[3,2-b]pyridine (20.0 g, 131 mmol) in tetrahydrofuran (200 ml) was added sodium hydride (13.1 g, 328 mmol, 60% purity in mineral oil) at 0 °C. After stirring at 20 °C for 1 hour, to the mixture was added 4-toluenesulfonyl chloride (37.5 g, 197 mmol) at 20 °C and the resulting mixture was stirred at 20 °C for 2 hours. The reaction was quenched with saturated ammonium chloride aqueous soution and the resulting solution was extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 3: 1 to 1: 1) to give 7-chloro-1-(4-methylbenzene-1-sulfonyl)-1H-pyrrolo[3,2-b]pyridine (34.0 g, 111 mmol, 85% yield) as a white solid.

LCMS (Method C): Rₜ = 0.955 min; MS (ESlpos): m/z = 307.0 [M+H]⁺.

### Intermediate 246

### 7-methyl-1-(4-methylbenzene-1-sulfonyl)-1H-pyrrolo[3,2-b]pyridine

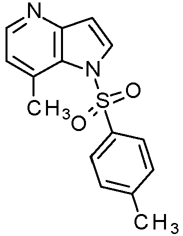

To a solution of 7-chloro-1-(4-methylbenzene-1-sulfonyl)-1H-pyrrolo[3,2-b]pyridine (10.0 g, 32.6 mmol) and 2,4,6-trimethyl-1,3,5,2,4,6-trioxatriborinane (19.0 ml, 65.2 mmol, 3.5 M in tetrahydrofuran) in 1,4-dioxane (150 ml) were added (1,1'-bis(diphenylphosphino)ferrocene)palladium(ll) dichloride (1.19 g, 1.63 mmol) and cesium carbonate (15.9 g, 48.9 mmol) at 20 °C. The mixture was stirred at 120 °C for 16 hours under nitrogen atmosphere. The reaction mixture was poured into water and extracted with ethyl acetate. The combined organic phase was concentrated in vacuo to give a crude product. The crude product was purified by column chromatography on silica gel (petroleum ether: ethyl acetate = 3: 2 to 1: 1) to give 7-methyl-1-(4-methylbenzene-1-sulfonyl)-1H-pyrrolo[3,2-b]pyridine (8.10 g, 28.3 mmol, 87% yield) as a yellow solid.

LCMS (Method C): Rₜ = 0.689 min; MS (ESlpos): m/z = 287.1 [M+H]⁺.

¹H NMR (400 MHz, DMSO-d₆), δ [ppm] = 8.33 (d, *J* = 4.8 Hz, 1H), 8.17 (d, *J* = 4.0 Hz, 1H), 7.67 (d, *J =* 8.4 Hz, 2H), 7.40 (t, *J* = 8.4 Hz, 2H), 7.10 (d, *J* = 4.8 Hz, 1H), 6.98 (d, *J* = 4.0 Hz, 1H), 2.54 (s, 3H), 2.34 (s, 3H).

### Intermediate 247

### 2-iodo-7-methyl-1-(4-methylbenzene-1-sulfonyl)-1H-pyrrolo[3,2-b]pyridine

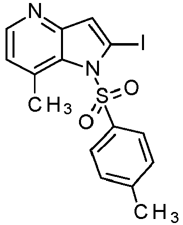

To a solution of 7-methyl-1-(4-methylbenzene-1-sulfonyl)-1H-pyrrolo[3,2-b]pyridine (8.10 g, 28.3 mmol) in tetrahydrofuran (95 ml) were added n-butyllithium (15 ml, 36.7 mmol, 2.5 M in hexane) at -78 °C. After stirring at -78 °C for 30 minutes under nitrogen atmosphere, to the mixture above was added iodine (9.33 g, 36.7 mmol) at -78 °C and the reaction mixture was stirred at 25 °C for 2 hours. The reaction solution was washed with saturated ammonium chloride aqueous solution and extracted with ethyl acetate. The combined organic layers were washed with brine, dried over anhydrous sodium sulfate, filtered and concentrated in vacuo to give a residue. The residue was purified by column chromatography on solica gel (petroleum ether: ethyl acetate = 4: 1 to 3: 1) to give 2-iodo-7-methyl-1-(4-methylbenzene-1-sulfonyl)-1H-pyrrolo[3,2-b]pyridine (9.30 g, 22.6 mmol, 80% yield) as a yellow solid.

¹H NMR (400 MHz, DMSO-d₆), δ [ppm] = 8.30 (d, *J =* 5.2 Hz, 1H), 7.44 (d, *J =* 8.4 Hz, 2H), 7.30-7.35 (m, 3H), 7.13 (d, *J =* 4.8 Hz, 1H), 2.63 (s, 3H), 2.33 (s, 3H).

### Intermediate 248

### 2-(3-methoxypyridin-4-yl)-7-methyl-1-(4-methylbenzene-1-sulfonyl)-1H-pyrrolo[3,2-b]pyridine

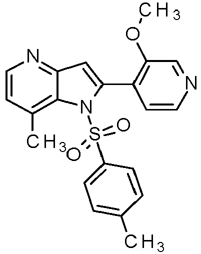

To a solution of 2-iodo-7-methyl-1-(4-methylbenzene-1-sulfonyl)-1H-pyrrolo[3,2-b]pyridine (2.00 g, 4.85 mmol) and (3-methoxypyridin-4-yl)boronic acid (1.11 g, 7.28 mmol) in 1,4-dioxane (20 ml) and water (4 ml) were added (1,1'-bis(diphenylphosphino)ferrocene)palladium(ll) dichloride (355 mg, 0.485 mmol) and sodium carbonate (1.03 g, 9.70 mmol) at 20 °C. The mixture was stirred at 90 °C for 16 hours under nitrogen atmosphere. The reaction solution was washed with water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 1: 0 to 6: 1) to give 2-(3-methoxypyridin-4-yl)-7-methyl-1-(4-methylbenzene-1-sulfonyl)-1H-pyrrolo[3,2-b]pyridine (1.70 g, 4.32 mmol, 89% yield) as a red solid.

LCMS (Method C): Rₜ = 0.682 min; MS (ESlpos): m/z = 394.2 [M+H]⁺.

### Intermediate 249

### 3-bromo-2-(3-methoxypyridin-4-yl)-7-methyl-1-(4-methylbenzene-1-sulfonyl)-1H-pyrrolo[3,2-b]pyridine

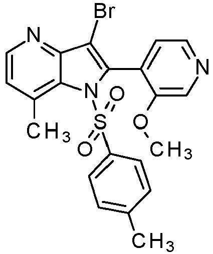

To a solution of 2-(3-methoxypyridin-4-yl)-7-methyl-1-(4-methylbenzene-1-sulfonyl)-1H-pyrrolo[3,2-b]pyridine (1.40 g, 3.56 mmol) in N,N-dimethylformamide (70 ml) was added N-bromosuccinimide (1.27 g, 7.12 mmol) at 20 °C. The mixture was stirred at 50 °C for 2 hours. The reaction mixture was washed with saturated sodium sulfite aqueous solution and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 1: 0 to 1: 1) to give 3-bromo-2-(3-methoxypyridin-4-yl)-7-methyl-1-(4-methylbenzene-1-sulfonyl)-1H-pyrrolo[3,2-b]pyridine (1.40 g, 2.96 mmol, 83% yield) as a pink solid.

LCMS (Method C): Rₜ = 0.563 min; MS (ESlpos): m/z = 474.1 [M+H]⁺.

### Intermediate 250

### 2-(3-methoxypyridin-4-yl)-7-methyl-1-(4-methylbenzene-1-sulfonyl)-3-phenyl-1H-pyrrolo[3,2-b]pyridine

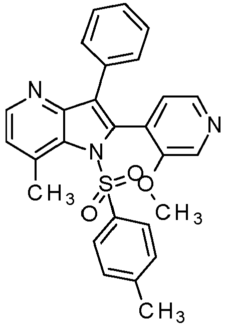

To a solution of 3-bromo-2-(3-methoxypyridin-4-yl)-7-methyl-1-(4-methylbenzene-1-sulfonyl)-1H-pyrrolo[3,2-b]pyridine (1.30 g, 2.75 mmol) and phenylboronic acid (503 mg, 4.13 mmol) in 1,4-dioxane (26 ml) and water (5 ml) were added (1,1'-bis(diphenylphosphino)ferrocene)palladium(ll) dichloride (201 mg, 0.275 mmol) and sodium carbonate (583 mg, 5.50 mmol) at 20 °C. The mixture was stirred at 90 °C for 16 hours under nitrogen atmosphere. The reaction solution was washed with water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 3: 2 to 1: 1) to give 2-(3-methoxypyridin-4-yl)-7-methyl-1-(4-methylbenzene-1-sulfonyl)-3-phenyl-1H-pyrrolo[3,2-b]pyridine (1.20 g, 2.56 mmol, 93% yield) as a white solid.

LCMS (Method C): Rₜ = 0.560 min; MS (ESlpos): m/z = 470.1 [M+H]⁺.

### Intermediate 251

### 4-(7-methyl-3-phenyl-1H-pyrrolo[3,2-b]pyridin-2-yl)pyridin-3-ol

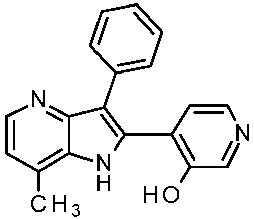

A solution of 2-(3-methoxypyridin-4-yl)-7-methyl-1-(4-methylbenzene-1-sulfonyl)-3-phenyl-1H-pyrrolo[3,2-b]pyridine (1.15 g, 2.45 mmol) in pyridine hydrochloride (5.66 g, 49.0 mmol) was stirred at 160 °C for 4 hours. The reaction mixture was cooled to room temperature and poured into water. The pH of the resulting solution was adjusted to 8 with saturated sodium hydrogen carbonate solution. The mixture above was filtered and the solid cake was collected and dried under reduced pressure to give a crude product. The crude product was triturated with ethyl acetate to give 4-(7-methyl-3-phenyl-1H-pyrrolo[3,2-b]pyridin-2-yl)pyridin-3-ol (0.700 g, 2.32 mmol, 95% yield) as a yellow solid.

LCMS (Method A): Rₜ = 0.764 min; MS (ESlpos): m/z = 302.1 [M+H]⁺.

### Intermediate 252

### tert-butyl (2S)-2-({[4-(7-methyl-3-phenyl-1H-pyrrolo[3,2-b]pyridin-2-yl)pyridin-3-yl]oxy}methyl)pyrrolidine-1-carboxylate

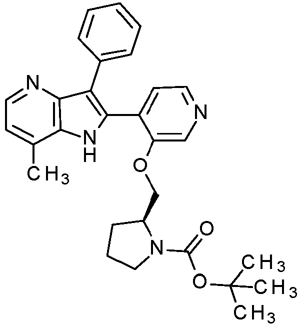

To a solution of 4-(7-methyl-3-phenyl-1H-pyrrolo[3,2-b]pyridin-2-yl)pyridin-3-ol (700 mg, 2.32 mmol) and tert-butyl (2S)-2-(hydroxymethyl)pyrrolidine-1-carboxylate (701 mg, 3.48 mmol) in tetrahydrofuran (10 ml) were added triphenylphosphine (1.83 g, 6.97 mmol) and diisopropyl azodicarboxylate (1.41 g, 6.97 mmol) at 0 °C. The mixture was stirred at 25 °C for 16 hours. The reaction solution was poured into water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 1: 1 to 2: 3) to give tert-butyl (2S)-2-({[4-(7-methyl-3-phenyl-1H-pyrrolo[3,2-b]pyridin-2-yl)pyridin-3-yl]oxy}methyl)pyrrolidine-1-carboxylate (400 mg, 0.825 mmol, 36% yield) as a red solid.

LCMS (Method A): Rₜ = 0.736 min; MS (ESlpos): m/z = 485.2 [M+H]⁺.

### Intermediate 253

### 7-methyl-3-phenyl-2-(3-{[(2S)-pyrrolidin-2-yl]methoxy}pyridin-4-yl)-1H-pyrrolo[3,2-b]pyridine hydrogen chloride (1/1)

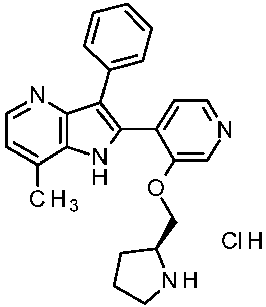

A solution of tert-butyl (2S)-2-({[4-(7-methyl-3-phenyl-1H-pyrrolo[3,2-b]pyridin-2-yl)pyridin-3-yl]oxy}methyl)pyrrolidine-1-carboxylate (400 mg, 0.825 mmol) in hydrochloric acid (10 ml, 40.0 mmol, 4M in ethyl acetate) was stirred at 25 °C for 1 hours. The reaction mixture was concentrated under reduced pressure to give 7-methyl-3-phenyl-2-(3-{[(2S)-pyrrolidin-2-yl]methoxy}pyridin-4-yl)-1H-pyrrolo[3,2-b]pyridine hydrogen chloride (1/1) (440 mg, 1.05 mmol, 127% yield) as a brown solid.

LCMS (Method C): Rₜ = 0.473 min; MS (ESlpos): m/z = 385.2 [(M-36)+H]⁺.

### Intermediate 254

### 3-(5-chloro-2-fluorophenyl)-2-(3-methoxypyridin-4-yl)-7-methyl-1-(4-methylbenzene-1-sulfonyl)-1H-pyrrolo[3,2-b]pyridine

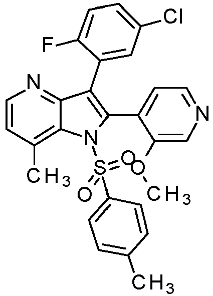

To a solution of 3-bromo-2-(3-methoxypyridin-4-yl)-7-methyl-1-(4-methylbenzene-1-sulfonyl)-1H-pyrrolo[3,2-b]pyridine (1.40 g, 2.96 mmol) and (5-chloro-2-fluorophenyl)boronic acid (0.775 g, 4.45 mmol) in 1,4-dioxane (10 ml) and water (2 ml) were added sodium carbonate (0.628 g, 5.93 mmol) and (1,1'-bis(diphenylphosphino)ferrocene)palladium(ll) dichloride (0.217 g, 0.296 mmol) at 20 °C. The mixture was stirred at 90 °C for 16 hours under nitrogen atmosphere. The reaction solution was poured into water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 3: 2 to 1: 1) to give 3-(5-chloro-2-fluorophenyl)-2-(3-methoxypyridin-4-yl)-7-methyl-1-(4-methylbenzene-1-sulfonyl)-1H-pyrrolo[3,2-b]pyridine (1.10 g, 2.11 mmol, 71% yield) as a white solid.

LCMS (Method C): Rₜ = 0.805 min; MS (ESlpos): m/z = 522.1 [M+H]⁺.

### Intermediate 255

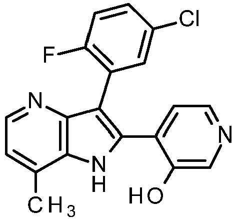

The mixture of 3-(5-chloro-2-fluorophenyl)-2-(3-methoxypyridin-4-yl)-7-methyl-1-(4-methylbenzene-1-sulfonyl)-1H-pyrrolo[3,2-b]pyridine (1.10 g, 2.11 mmol) and pyridine hydrochloride (2.44 g, 21.1 mmol) was stirred at 160 °C for 2 hours. After cooled to temperature, the reaction mixture was poured into water, and pH of the resulting mixture was adjusted to 8 with saturated sodium hydrogen carbonate aqueous solution. The suspension was filtered and the solid cake was collected to give a crude product. The crude product was triturated with ethyl acetate to give 4-[3-(5-chloro-2-fluorophenyl)-7-methyl-1 H-pyrrolo[3,2-b]pyridin-2-yl]pyridin-3-ol (0.700 g, 1.98 mmol, 94% yield) as a yellow solid.

LCMS (Method A): Rₜ = 0.698 min; MS (ESlpos): m/z = 354.0 [M+H]⁺.

### Intermediate 256

### tert-butyl (2S)-2-[({4-[3-(5-chloro-2-fluorophenyl)-7-methyl-1H-pyrrolo[3,2-b]pyridin-2-yl]pyridin-3-yl}oxy)methyl]pyrrolidine-1-carboxylate

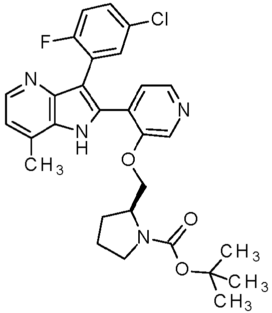

To a solution of 4-[3-(5-chloro-2-fluorophenyl)-7-methyl-1H-pyrrolo[3,2-b]pyridin-2-yl]pyridin-3-ol (700 mg, 1.98 mmol) and tert-butyl (2S)-2-(hydroxymethyl)pyrrolidine-1-carboxylate (796 mg, 3.96 mmol) in tetrahydrofuran (10 ml) were added triphenylphosphine (1.56 g, 5.94 mmol) and diisopropyl azodicarboxylate (1.20 g, 5.94 mmol) at 0 °C. The mixture was stirred at 25 °C for 16 hours. The reaction solution was poured into water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 1: 2 to 0: 1) to give tert-butyl (2S)-2-[({4-[3-(5-chloro-2-fluorophenyl)-7-methyl-1H-pyrrolo[3,2-b]pyridin-2-yl]pyridin-3-yl}oxy)methyl]pyrrolidine-1-carboxylate (700 mg, 1.30 mmol, 66% yield) as a yellow solid.

LCMS (Method C): Rₜ = 0.706 min; MS (ESlpos): m/z = 537.2 [M+H]⁺.

### Intermediate 257

### 3-(5-chloro-2-fluorophenyl)-7-methyl-2-(3-{[(2S)-pyrrolidin-2-yl]methoxy}pyridin-4-yl)-1H-pyrrolo[3,2-b]pyridine

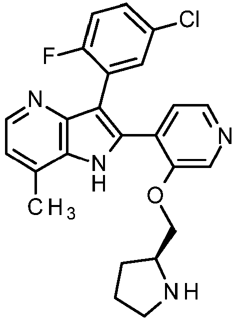

A solution of tert-butyl (2S)-2-[({4-[3-(5-chloro-2-fluorophenyl)-7-methyl-1H-pyrrolo[3,2-b]pyridin-2-yl]pyridin-3-yl}oxy)methyl]pyrrolidine-1-carboxylate (700 mg, 1.30 mmol) in hydrochloric acid (5 ml, 4M in ethyl acetate) was stirred at 25 °C for 1 hours. The raction mixture was concentrated under reduced pressure to give a residue. The residue was purified by flash reversed-phase MPLC (acetonitrile/water, 0.05% ammonia hydroxide, 20%~30%) to give 3-(5-chloro-2-fluorophenyl)-7-methyl-2-(3-{[(2S)-pyrrolidin-2-yl]methoxy}pyridin-4-yl)-1H-pyrrolo[3,2-b]pyridine (240 mg, 0.549 mmol, 42% yield) as a yellow solid.

LCMS (Method C): Rₜ = 0.576 min; MS (ESlpos): m/z = 437.1 [M+H]⁺.

### Intermediate 258

### tert-butyl (2RS)-2-{[(4-bromopyridin-3-yl)oxy]methyl}azetidine-1-carboxylate

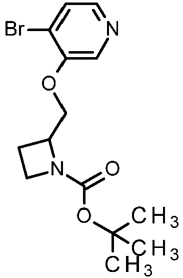

To a solution of 4-bromopyridin-3-ol (10.0 g, 57.5 mmol) and tert-butyl (2RS)-2-(hydroxymethyl)azetidine-1-carboxylate (12.9 g, 69.0 mmol) in tetrahydrofuran (200 ml) were added triphenylphosphine (45.2 g, 172 mmol) and diisopropyl azodicarboxylate (34.9 g, 172 mmol) at 25 °C. The mixture was stirred at 25 °C for 16 hours. The reaction mixture was poured into water and extracted with ethyl acetate. The combined organic phase was washed with brine, dried over anhydrous sodium sulfate, filtered and concentrated in vacuo to give a crude product, which was purified by column chromatography on silica gel (petroleum ether: ethyl acetate = 50: 1 to 3: 1) to give tert-butyl (2RS)-2-{[(4-bromopyridin-3-yl)oxy]methyl}azetidine-1-carboxylate (19.0 g, 55.4 mmol, 96% yield) as a yellow oil.

LC-MS (Method C): Rₜ = 0.824 min; MS (ESlpos): m/z = 345.1 [M+H]⁺.

### Intermediate 259

### (3-{[(2RS)-1-(tert-butoxycarbonyl)azetidin-2-yl]methoxy}pyridin-4-yl)boronic acid

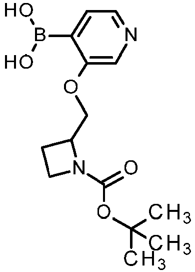

To a solution of tert-butyl (2RS)-2-{[(4-bromopyridin-3-yl)oxy]methyl}azetidine-1-carboxylate (19.0 g, 55.4 mmol) and 4,4,4',4',5,5,5',5'-octamethyl-2,2'-bi-1,3,2-dioxaborolane (28.1 g, 111 mmol) in 1,4-dioxane (300 ml) were added potassium acetate (10.9 g, 111 mmol) and (1,1'-bis(diphenylphosphino)ferrocene)palladium(II) dichloride (4.05 g, 5.54 mmol) at 20 °C. The mixture was stirred at 90 °C for 16 hours under nitrogen atmosphere. The reaction mixture was poured into water and extracted with ethyl acetate. The combined organic phase was washed with brine, dried over anhydrous sodium sulfate, filtered and concentrated in vacuo to give a crude product. The crude product was purified by column chromatography on silica gel (petroleum ether: ethyl acetate = 5: 1 to 0: 1, then ethyl acetate: methanol = 10: 1) to give (3-{[(2RS)-1-(tert-butoxycarbonyl)azetidin-2-yl]methoxy}pyridin-4-yl)boronic acid (10.0 g, 32.5 mmol, 59% yield) as a brown oil.

LC-MS (Method C): Rₜ = 0.424 min; MS (ESlpos): m/z = 309.2 [M+H]⁺.

### Intermediate 260

### tert-butyl (2RS)-2-{[(4-{1-[(4-methylphenyl)sulfonyl]-1H-pyrrolo[3,2-b]pyridin-2-yl}pyridin-3-yl)oxy]methyl}azetidine-1-carboxylate

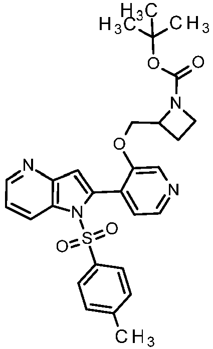

To a solution of 2-iodo-1-[(4-methylphenyl)sulfonyl]-1H-pyrrolo[3,2-b]pyridine (5.00 g, 12.6 mmol) and (3-{[(2RS)-1-(tert-butoxycarbonyl)azetidin-2-yl]methoxy}pyridin-4-yl)boronic acid (5.80 g, 18.8 mmol) in 1,4-dioxane (100 ml) and water (40 ml) were added tetrakis(triphenylphosphine)palladium(0) (1.45 g, 1.26 mmol) and cesium carbonate (8.18 g, 25.1 mmol) at 20 °C. The mixture was stirred at 90 °C for 16 hours under nitrogen atmosphere. The reaction mixture was poured into water and extracted with ethyl acetate. The combined organic phase was washed with brine, dried over anhydrous sodium sulfate, filtered and concentrated in vacuo to give a crude product. The crude product was purified by column chromatography on silica gel (petroleum ether: ethyl acetate = 50: 1 to 0: 1, then ethyl acetate: methanol = 20: 1) to give tert-butyl (2RS)-2-{[(4-{1-[(4-methylphenyl)sulfonyl]-1H-pyrrolo[3,2-b]pyridin-2-yl}pyridin-3-yl)oxy]methyl}azetidine-1-carboxylate (2.20 g, 4.11 mmol, 33% yield) as a yellow solid.

LC-MS (Method C): Rₜ = 0.914 min; MS (ESlpos): m/z = 535.2 [M+H]⁺.

### Intermediate 261

### tert-butyl (2RS)-2-({[4-(1H-pyrrolo[3,2-b]pyridin-2-yl)pyridin-3-yl]oxy}methyl)azetidine-1-carboxylate

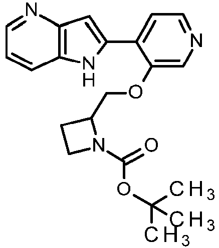

To a solution of tert-butyl (2RS)-2-{[(4-{1-[(4-methylphenyl)sulfonyl]-1H-pyrrolo[3,2-b]pyridin-2-yl}pyridin-3-yl)oxy]methyl}azetidine-1-carboxylate (2.20 g, 4.11 mmol) in methanol (70 ml) was added potassium carbonate (2.84 g, 20.6 mmol) at 25 °C. The mixture was stirred at 25 °C for 16 hours. The reaction mixture was poured into water and extracted with ethyl acetate. The combined organic phase was washed with brine, dried over anhydrous sodium sulfate, filtered and concentrated in vacuo to give tert-butyl (2RS)-2-({[4-(1H-pyrrolo[3,2-b]pyridin-2-yl)pyridin-3-yl]oxy}methyl)azetidine-1-carboxylate (1.50 g, 3.94 mmol, 96% yield) as a yellow solid.

LC-MS (Method C): Rₜ = 0.786 min; MS (ESlpos): m/z = 381.2 [M+H]⁺.

### Intermediate 262

### tert-butyl (2RS)-2-({[4-(3-bromo-1H-pyrrolo[3,2-b]pyridin-2-yl)pyridin-3-yl]oxy}methyl)azetidine-1-carboxylate

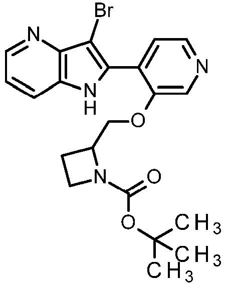

To a solution of tert-butyl (2RS)-2-({[4-(1H-pyrrolo[3,2-b]pyridin-2-yl)pyridin-3-yl]oxy}methyl)azetidine-1-carboxylate (1.5 g, 3.94 mmol) in N,N-dimethylformamide (20 ml) was added 1-bromopyrrolidine-2,5-dione (702 mg, 3.94 mmol) at 25 °C. The mixture was stirred at 25 °C for 16 hours. The reaction mixture was poured into water, and the resulting mixture was filtered. The solid cake was collected to give tert-butyl (2RS)-2-({[4-(3-bromo-1H-pyrrolo[3,2-b]pyridin-2-yl)pyridin-3-yl]oxy}methyl)azetidine-1-carboxylate (1.80 g, 3.92 mmol, 99% yield) as a yellow solid.

LC-MS (Method C): Rₜ = 0.803 min; MS (ESlpos): m/z = 461.1 [M+H]⁺.

### Intermediate 263

### tert-butyl (2RS)-2-({[4-(3-phenyl-1H-pyrrolo[3,2-b]pyridin-2-yl)pyridin-3-yl]oxy}methyl)azetidine-1-carboxylate

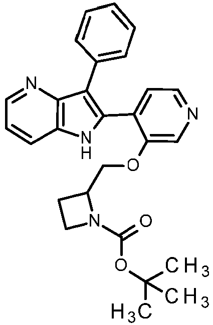

To a solution of tert-butyl (2RS)-2-({[4-(3-bromo-1H-pyrrolo[3,2-b]pyridin-2-yl)pyridin-3-yl]oxy}methyl)azetidine-1-carboxylate (750 mg, 1.63 mmol) and phenylboronic acid (299 mg, 2.45 mmol) in 1,4-dioxane (75 ml) and water (30 ml) were added sodium carbonate (346 mg, 3.27 mmol) and (1,1'-bis(diphenylphosphino)ferrocene)palladium(II) dichloride (119 mg, 0.163 mmol) at 20 °C. The mixture was stirred at 90 °C for 16 hours under nitrogen atmosphere. The reaction mixture was poured into water and extracted with ethyl acetate. The combined orgainc phase was washed with brine, dried over anhydrous sodium sulfate, filtered and concentrated in vacuo to give a residue. The residue was purified by column chromatography on silica gel (petroleum ether: ethyl acetate = 20: 1 to 0: 1) to give tert-butyl (2RS)-2-({[4-(3-phenyl-1H-pyrrolo[3,2-b]pyridin-2-yl)pyridin-3-yl]oxy}methyl)azetidine-1-carboxylate (340 mg, 0.744 mmol, 46% yield) as a yellow solid.

LC-MS (Method C): Rₜ = 0.489 min; MS (ESlpos): m/z = 457.5 [M+H]⁺.

### Intermediate 264

### (RS)-2-(3-(azetidin-2-ylmethoxy)pyridin-4-yl)-3-phenyl-1H-pyrrolo[3,2-b]pyridine

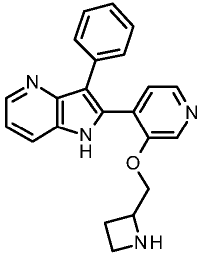

To a solution of tert-butyl (2RS)-2-({[4-(3-phenyl-1H-pyrrolo[3,2-b]pyridin-2-yl)pyridin-3-yl]oxy}methyl)azetidine-1-carboxylate (200 mg, 0.438 mmol) in dichloromethane (10 ml) was added trifluoroacetic acid (1.00 g, 8.76 mmol) at 0 °C. The mixture was stirred at 0 °C for 1 hour. The reaction mixture was concentrated in vacuo to give a residue. To this residue was added saturated sodium hydrogen carbonate aqueous solution and the resulting mixture was stirred at 0 °C for 1 hour. The reaction mixture was extracted with ethyl acetate. The combined organic phase was concentrated in vacuo to give (RS)-2-(3-(azetidin-2-ylmethoxy)pyridin-4-yl)-3-phenyl-1H-pyrrolo[3,2-b]pyridine (140 mg, 0.393 mmol, 90% yield) as a yellow solid.

LC-MS (Method C): Rₜ = 0.954 min; MS (ESlpos): m/z = 357.2 [M+H]⁺.

### Intermediate 265

### tert-butyl (2RS)-2-{[(4-{3-[3-(trifluoromethyl)phenyl]-1H-pyrrolo[3,2-b]pyridin-2-yl}pyridin-3-yl)oxy]methyl}azetidine-1-carboxylate

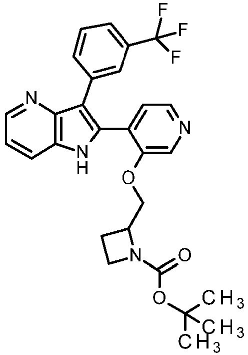

To a solution of tert-butyl (2RS)-2-({[4-(3-bromo-1H-pyrrolo[3,2-b]pyridin-2-yl)pyridin-3-yl]oxy}methyl)azetidine-1-carboxylate (750 mg, 1.63 mmol) and [3-(trifluoromethyl)phenyl]boronic acid (465 mg, 2.45 mmol) in 1,4-dioxane (20 ml) and water (5 ml) were added sodium carbonate (346 mg, 3.27 mmol) and (1,1'-bis(diphenylphosphino)ferrocene)palladium(ll) dichloride (119 mg, 0.163 mmol) at 20 °C. The mixture was stirred at 90 °C for 16 hours under nitrogen atmosphere. The reaction mixture was poured into water and extracted with ethyl acetate. The combined orgainc phase was washed with brine, dried over anhydrous sodium sulfate, filtered and concentrated in vacuo to give a residue. The residue was purified by column chromatography on silica gel (petroleum ether: ethyl acetate = 20: 1 to 0: 1) to give tert-butyl (2RS)-2-{[(4-{3-[3-(trifluoromethyl)phenyl]-1H-pyrrolo[3,2-b]pyridin-2-yl}pyridin-3-yl)oxy]methyl}azetidine-1-carboxylate (370 mg, 0.705 mmol, 43% yield) as a yellow solid.

LC-MS (Method C): Rₜ = 0.522 min; MS (ESlpos): m/z = 525.4 [M+H]⁺.

### Intermediate 266

### 2-{3-[(2RS)-azetidin-2-ylmethoxy]pyridin-4-yl}-3-[3-(trifluoromethyl)phenyl]-1H-pyrrolo[3,2-b]pyridine

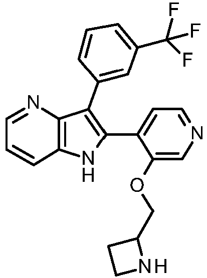

To a solution of tert-butyl (2RS)-2-{[(4-{3-[3-(trifluoromethyl)phenyl]-1H-pyrrolo[3,2-b]pyridin-2-yl}pyridin-3-yl)oxy]methyl}azetidine-1-carboxylate (350 mg, 0.667 mmol) in dichloromethane (10 ml) was added trifluoroacetic acid (1 ml, 13.3 mmol) at 0 °C. The mixture was stirred at 0 °C for 4 hours. The reaction mixture was concentrated in vacuo to give a residue. To this residue was added saturated sodium hydrogen carbonate aqueous solution and the resulting mixture was stirred at 0 °C for 1 hour. The reaction mixture was extracted with ethyl acetate. The combined organic phase was concentrated in vacuo to give 2-{3-[(2RS)-azetidin-2-ylmethoxy]pyridin-4-yl}-3-[3-(trifluoromethyl)phenyl]-1 H-pyrrolo[3,2-b]pyridine (270 mg, 0.636 mmol, 95% yield) as a yellow solid.

LC-MS (Method D): Rₜ = 1.021 min; MS (ESlpos): m/z = 425.2 [M+H]⁺.

### Intermediate 267

### 2-(3-{[(2RS)-1-benzyl-2-methylazetidin-2-yl]methoxy}pyridin-4-yl)-3-phenyl-1H-pyrrolo[3,2-b]pyridine

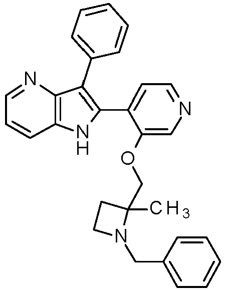

To a solution of 4-(3-phenyl-1H-pyrrolo[3,2-b]pyridin-2-yl)pyridin-3-ol (700 mg, 2.44 mmol) and [(2RS)-1-benzyl-2-methylazetidin-2-yl]methanol (559 mg, 2.92 mmol, CAS 850789-22-9) in tetrahydrofuran (21 ml) was added diisopropyl azodicarboxylate (985 mg, 4.87 mmol) and triphenylphosphine (1.28 g, 4.87 mmol) at 20 °C. The mixture was stirred at 20 °C for 16 hours. The reaction mixture was poured into water and extracted with ethyl acetate. The combined organic phase was washed with brine, dried over anhydrous sodium sulfate, filtered and cocentrated in vacuo to give a residue The residue was purified by column chromatography on silica gel (petroleum ether: ethyl acetate = 50: 1 to 1: 2) to give 2-(3-{[(2RS)-1-benzyl-2-methylazetidin-2-yl]methoxy}pyridin-4-yl)-3-phenyl-1H-pyrrolo[3,2-b]pyridine (740 mg, 1.61 mmol, 66% yield) as a yellow solid.

LC-MS (Method C): Rₜ = 0.705 min; MS (ESlpos): m/z = 461.2 [M+H]⁺.

### Intermediate 268

### 2-(3-{[(2RS)-2-methylazetidin-2-yl]methoxy}pyridin-4-yl)-3-phenyl-1H-pyrrolo[3,2-b]pyridine

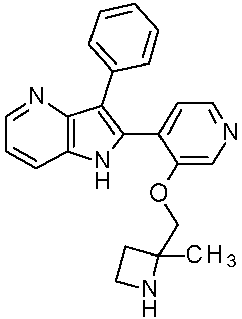

To a solution of 2-(3-{[(2RS)-1-benzyl-2-methylazetidin-2-yl]methoxy}pyridin-4-yl)-3-phenyl-1H-pyrrolo[3,2-b]pyridine (500 mg, 1.09 mmol) in 2,2,2-trifluoroethanol (20 ml) was added palladium/carbon (115 mg, 1.09 mmol) at 20 °C. After stirring at 20 °C for 16 hours under hydrogen atmosphere, to the mixture above was added palladium(ll) hydroxide (152 mg, 1.09 mmol) and the resulting mixture was stirred at 20 °C for 16 hours under hydrogen atmosphere. The reaction mixture was filtered and the filtrate was cocentrated in vacuo to give 2-(3-{[(2RS)-2-methylazetidin-2-yl]methoxy}pyridin-4-yl)-3-phenyl-1H-pyrrolo[3,2-b]pyridine (470 mg, 1.27 mmol, 117% yield) as a yellow solid.

LC-MS (Method C): Rₜ = 0.312 min; MS (ESlpos): m/z = 371.2 [M+H]⁺.

### Intermediate 269

### 2-(3-methoxypyridin-4-yl)-1-(4-methylbenzene-1-sulfonyl)-3-[3-(trifluoromethyl)phenyl]-1H-pyrrolo[3,2-b]pyridine

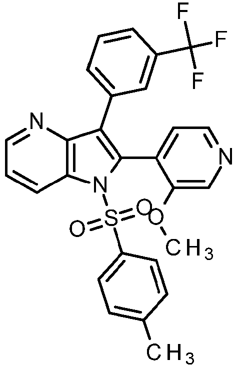

To a solution of 3-bromo-2-(3-methoxypyridin-4-yl)-1-(4-methylbenzene-1-sulfonyl)-1H-pyrrolo[3,2-b]pyridine (3.00 g, 6.55 mmol) and [3-(trifluoromethyl)phenyl]boronic acid (1.86 g, 9.82 mmol) in 1,4-dioxane (57 ml) and water (11 ml) were added (1,1'-bis(diphenylphosphino)ferrocene)palladium(ll) dichloride (479 mg, 0.655 mmol) and sodium carbonate (1.39 g, 13.1 mmol) at 20 °C. The mixture was stirred at 90 °C for 16 hours under nitrogen atmosphere. The reaction mixture was poured into water and extracted with ethyl acetate. The combined organic phase was washed with brine, dried over anhydrous sodium sulfate, filtered and the filtrate was concentrated in vacuo to give a residue. The residue was purified by column chromatography on silica gel (petroleum ether: ethyl acetate = 50: 1 to 2: 1) to give 2-(3-methoxypyridin-4-yl)-1-(4-methylbenzene-1-sulfonyl)-3-[3-(trifluoromethyl)phenyl]-1H-pyrrolo[3,2-b]pyridine (2.90 g, 5.54 mmol, 85% yield) as a yellow solid.

LC-MS (Method C): Rₜ = 0.990 min; MS (ESlpos): m/z = 524.2 [M+H]⁺.

### Intermediate 270

### 4-{3-[3-(trifluoromethyl)phenyl]-1H-pyrrolo[3,2-b]pyridin-2-yl}pyridin-3-ol

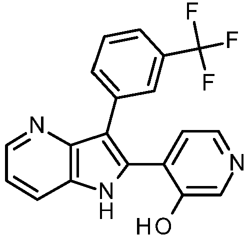

The solution of 2-(3-methoxypyridin-4-yl)-1-(4-methylbenzene-1-sulfonyl)-3-[3-(trifluoromethyl)phenyl]-1H-pyrrolo[3,2-b]pyridine (2.90 g, 5.54 mmol) in hydrobromic acid (40% purity in water, 40 ml) was stirred at 120 °C for 16 hours. pH of the reaction mixture was adjusted to 8~10 with sodium hydrogen carbonate aqueous solution. The resulting suspension was filtered and the solid cake was collected to give 4-{3-[3-(trifluoromethyl)phenyl]-1H-pyrrolo[3,2-b]pyridin-2-yl}pyridin-3-ol (1.90 g, 5.35 mmol, 97% yield) as a yellow solid.

LC-MS (Method A): Rₜ = 0.839 min; MS (ESlpos): m/z = 356.1 [M+H]⁺.

### Intermediate 271

### 2-(3-{[(2RS)-1-benzyl-2-methylazetidin-2-yl]methoxy}pyridin-4-yl)-3-[3-(trifluoromethyl)phenyl]-1H-pyrrolo[3,2-b]pyridine

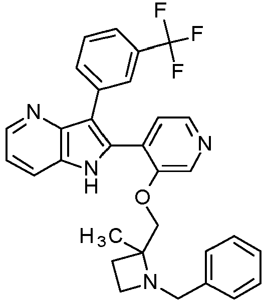

To a solution of 4-{3-[3-(trifluoromethyl)phenyl]-1H-pyrrolo[3,2-b]pyridin-2-yl}pyridin-3-ol (900 mg, 2.53 mmol) and [(2RS)-1-benzyl-2-methylazetidin-2-yl]methanol (533 mg, 2.79 mmol) in tetrahydrofuran (32 ml) were added diisopropyl azodicarboxylate (1.02 g, 5.07 mmol) and triphenylphosphine (1.33 g, 5.07 mmol) at 25 °C. The mixture was stirred at 25 °C for 16 hours. The reaction mixture was poured into water and extracted with ethyl acetate. The combined organic phase was washed with brine, dried over anhydrous sodium sulfate, filtered and the filtrate was concentrated in vacuo to give a residue. The residue was purified by column chromatography on silica gel (petroleum ether: ethyl acetate = 50: 1 to 0: 1) to give 2-(3-{[(2RS)-1-benzyl-2-methylazetidin-2-yl]methoxy}pyridin-4-yl)-3-[3-(trifluoromethyl)phenyl]-1H-pyrrolo[3,2-b]pyridine (260 mg, 0.492 mmol, 19% yield) as a yellow solid.

LC-MS (Method A): Rₜ = 0.758 min; MS (ESlpos): m/z = 529.2 [M+H]⁺.

### Intermediate 272

### 2-(3-{[(2RS)-2-methylazetidin-2-yl]methoxy}pyridin-4-yl)-3-[3-(trifluoromethyl)phenyl]-1H-pyrrolo[3,2-b]pyridine

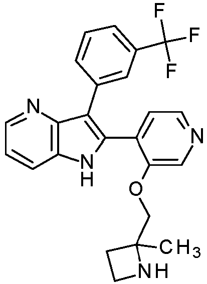

To a solution of 2-(3-{[(2RS)-1-benzyl-2-methylazetidin-2-yl]methoxy}pyridin-4-yl)-3-[3-(trifluoromethyl)phenyl]-1H-pyrrolo[3,2-b]pyridine (180 mg, 341 µmol) in 2,2,2-trifluoroethanol (36 ml) was added palladium/carbon (3.62 mg, 0.0341 mmol) at 20 °C. The mixture was stirred at 20 °C for 16 hours under hydrogen atmosphere. The reaction mixture was filtered through a pad of celite and the filtrate was concentrated in vacuo to give 2-(3-{[(2RS)-2-methylazetidin-2-yl]methoxy}pyridin-4-yl)-3-[3-(trifluoromethyl)phenyl]-1H-pyrrolo[3,2-b]pyridine (80.0 mg, 0.182 mmol, 54% yield) as a yellow solid.

LC-MS (Method C): Rₜ = 0.576 min; MS (ESlpos): m/z = 439.1 [M+H]⁺.

### Intermediate 273

### 2-(3-{[4-benzylmorpholin-3-yl]methoxy}pyridin-4-yl)-3-phenyl-1H-pyrrolo[3,2-b]pyridine

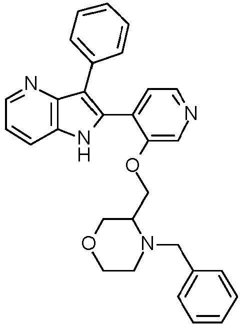

To a solution of 4-(3-phenyl-1H-pyrrolo[3,2-b]pyridin-2-yl)pyridin-3-ol (800 mg, 2.78 mmol) and [4-benzylmorpholin-3-yl]methanol (1.44 g, 6.96 mmol) in tetrahydrofuran (20 ml) were added triphenylphosphine (2.19 g, 8.35 mmol) and diisopropyl azodicarboxylate (0.54 ml, 8.35 mmol) at 0 °C. The mixture was stirred at 25 °C for 16 hours. The reaction solution was poured into water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 1: 2 to 0: 1) to give 2-(3-{[4-benzylmorpholin-3-yl]methoxy}pyridin-4-yl)-3-phenyl-1H-pyrrolo[3,2-b]pyridine (1.20 g, 2.52 mmol, 90% yield) as a yellow solid.

LCMS (Method C): Rₜ = 0.742 min; MS (ESlpos): m/z = 477.4 [M+H]⁺.

### Intermediate 274

### 2-(3-{[morpholin-3-yl]methoxy}pyridin-4-yl)-3-phenyl-1H-pyrrolo[3,2-b]pyridine

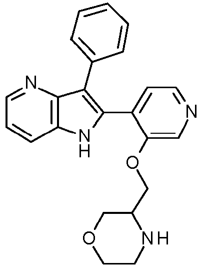

To a solution of 2-(3-{[4-benzylmorpholin-3-yl]methoxy}pyridin-4-yl)-3-phenyl-1H-pyrrolo[3,2-b]pyridine (200 mg, 0.420 mmol) in 2,2,2-trifluoroethanol (8 ml) was added palladium on active carbon (44.7 mg, 0.042 mmol) at 20 °C. The mixture was stirred at 20 °C for 16 hour under hydrogen atmosphere. The mixture was filtered and the solid cake was collected to give 2-(3-{[morpholin-3-yl]methoxy}pyridin-4-yl)-3-phenyl-1H-pyrrolo[3,2-b]pyridine (130 mg, 0.336 mmol, 80% yield) as a yellow oil.

LCMS (Method A): Rₜ = 0.215 min; MS (ESlpos): m/z = 387.3 [M+H]⁺.

### Intermediate 275

### 3-(5-chloro-2-fluorophenyl)-2-(3-methoxypyridin-4-yl)-1-(4-methylbenzene-1-sulfonyl)-1H-pyrrolo[3,2-b]pyridine

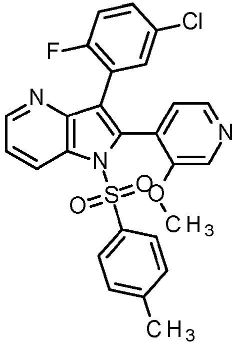

To a solution of 3-bromo-2-(3-methoxypyridin-4-yl)-1-(4-methylbenzene-1-sulfonyl)-1H-pyrrolo[3,2-b]pyridine (5.00 g, 10.9 mmol) and (5-chloro-2-fluorophenyl)boronic acid (2.85 g, 16.4 mmol) in 1,4-dioxane (100 ml) and water (20 ml) were added sodium carbonate (2.31 g, 21.8 mmol) and (1,1'-bis(diphenylphosphino)ferrocene)palladium(II) dichloride (0.798 g, 1.09 mmol) at 20 °C. The mixture was stirred at 90 °C for 16 hours under nitrogen atmosphere. The reaction mixture was poured into water and extracted with ethyl acetate. The combined organic phase was concentrated in vacuo to give a crude product. The crude product was purified by column chromatography on silica gel (petroleum ether: ethyl acetate = 2: 3 to 0: 1) to give 3-(5-chloro-2-fluorophenyl)-2-(3-methoxypyridin-4-yl)-1-(4-methylbenzene-1-sulfonyl)-1H-pyrrolo[3,2-b]pyridine (2.80 g, 5.51 mmol, 51% yield) as a yellow solid.

LCMS (Method C): Rₜ = 0.614 min; MS (ESlpos): m/z = 508.2 [M+H]⁺.

### Intermediate 276

### 4-[3-(5-chloro-2-fluorophenyl)-1H-pyrrolo[3,2-b]pyridin-2-yl]pyridin-3-ol

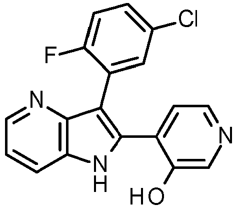

A solution of 3-(5-chloro-2-fluorophenyl)-2-(3-methoxypyridin-4-yl)-1-(4-methylbenzene-1-sulfonyl)-1 H-pyrrolo[3,2-b]pyridine (2.80 g, 5.51 mmol) in hydrobromic acid (34 ml, 40% in water) was stirred at 120 °C for 72 hours. After cooled to room temperature, pH of the reaction mixture was adjusted to 7~8 with saturated sodium hydrogen carbonate aqueous solution. The resulting mixture was filtered and the solid cake was collected to give 4-[3-(5-chloro-2-fluorophenyl)-1H-pyrrolo[3,2-b]pyridin-2-yl]pyridin-3-ol (1.60 g, 4.71 mmol, 85% yield) as a white solid.

LCMS (Method A): Rₜ = 0.789 min; MS (ESlpos): m/z = 340.1 [M+H]⁺.

### Intermediate 277

### tert-butyl -3-[({4-[3-(5-chloro-2-fluorophenyl)-1H-pyrrolo[3,2-b]pyridin-2-yl]pyridin-3-yl}oxy)methyl]morpholine-4-carboxylate

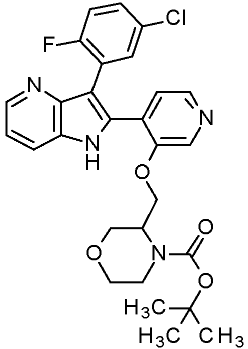

To a solution of 4-[3-(5-chloro-2-fluorophenyl)-1H-pyrrolo[3,2-b]pyridin-2-yl]pyridin-3-ol (1.00 g, 2.94 mmol) and tert-butyl 3-(hydroxymethyl)morpholine-4-carboxylate (1.92 g, 8.83 mmol) in tetrahydrofuran (20 ml) were added triphenylphosphine (2.32 g, 8.83 mmol) and diisopropyl azodicarboxylate (1.19 g, 5.89 mmol) at 0 °C. The mixture was stirred at 25 °C for 16 hours. The reaction solution was poured into water and extracted with ethyl acetate. The combined organic layers were washed with brine, dried over anhydrous sodium sulfate, filtered and concentrated in vacuo to give a residue. The residue was purified by column chromatography on silica gel (petroleum ether: ethyl acetate = 1: 4 to 1: 19) to give tert-butyl -3-[({4-[3-(5-chloro-2-fluorophenyl)-1H-pyrrolo[3,2-b]pyridin-2-yl]pyridin-3-yl}oxy)methyl]morpholine-4-carboxylate (1.30 g, 2.41 mmol, 82% yield) as a brown oil.

LCMS (Method C): Rₜ = 0.496 min; MS (ESlpos): m/z = 539.2 [M+H]⁺.

### Intermediate 278

### 3-(5-chloro-2-fluorophenyl)-2-(3-{[morpholin-3-yl]methoxy}pyridin-4-yl)-1H-pyrrolo[3,2-b]pyridine hydrogen chloride (1/1)

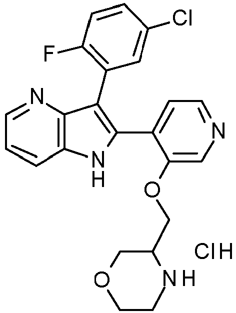

A solution of tert-butyl 3-[({4-[3-(5-chloro-2-fluorophenyl)-1H-pyrrolo[3,2-b]pyridin-2-yl]pyridin-3-yl}oxy)methyl]morpholine-4-carboxylate (1.00 g, 1.86 mmol) in hydrochloric acid (10 ml, 40.0 mmol, 4M in ethyl acetate) was stirred at 25 °C for 1 hour. The reaction mixture was concentrated under reduced pressure to give a residue. The residue was purified by flash reversed-phase MPLC (acetonitrile /water, 0.05% ammonia hydroxide, 0%~30%) to give 3-(5-chloro-2-fluorophenyl)-2-(3-{[morpholin-3-yl]methoxy}pyridin-4-yl)-1H-pyrrolo[3,2-b]pyridine hydrogen chloride (1/1) (240 mg, 0.505 mmol, 27% yield) as a yellow solid.

LCMS (Method C): Rₜ = 0.234 min; MS (ESlpos): m/z = 439.2 [(M-36)+H]⁺.

### Syntheses of Examples

### Example 1

### N-[2-({4-[3-(4-fluorophenyl)-1H-pyrrolo[3,2-b]pyridin-2-yl]pyridin-3-yl}oxy)ethyl]prop-2-enamide

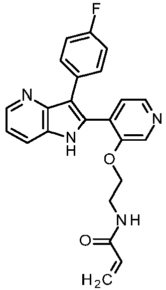

2-({4-[3-(4-fluorophenyl)-1H-pyrrolo[3,2-b]pyridin-2-yl]pyridin-3-yl}oxy)ethanamine trifluoroacetate (1:2) (9.00 mg, 15.6 µmol) and prop-2-enoic acid (1.2 µl, 17 µmol) were dissolved in 1 ml DMF. At rt, HATU (9.4 µl, 2.0 M, 19 µmol) and triethylamine (11 µl, 78 µmol) were added. After 16 h at rt, water (1 ml) was added and the mixture was extracted with EtOAc twice. The combined organic layers were dried over sodium sulfate, filtered and concentrated under reduced pressure. The crude product was purified by reverse phase preparative HPLC (method 4) yielding 3.00 mg (99 % purity, 47 % yield) of the title compound.
LC-MS (method 1): Rₜ = 0.87 min; MS (ESlpos): m/z = 403 [M+H]⁺
¹H-NMR (600 MHz, DMSO-d6) δ [ppm]: 1.235 (0.87), 1.754 (0.45), 2.424 (0.78), 2.653 (0.69), 3.229 (4.09), 3.239 (10.43), 3.248 (10.82), 3.258 (5.06), 4.036 (6.62), 4.046 (13.29), 4.055 (6.22), 5.562 (4.92), 5.565 (4.85), 5.579 (5.00), 5.582 (5.32), 5.754 (12.58), 6.050 (3.63), 6.053 (3.81), 6.078 (6.32), 6.081 (6.17), 6.155 (5.79), 6.172 (5.61), 6.183 (3.53), 6.200 (3.27), 7.149 (6.35), 7.164 (12.67), 7.179 (6.82), 7.216 (4.81), 7.224 (4.94), 7.230 (4.97), 7.238 (5.00), 7.309 (9.23), 7.316 (9.43), 7.519 (6.57), 7.529 (7.69), 7.533 (7.79), 7.543 (6.40), 7.850 (5.79), 7.852 (6.53), 7.864 (5.65), 7.865 (6.11), 8.146 (7.80), 8.179 (2.27), 8.188 (4.25), 8.197 (2.20), 8.252 (9.44), 8.260 (9.13), 8.411 (5.85), 8.413 (6.59), 8.419 (5.90), 8.420 (6.32), 8.537 (16.00), 11.662 (8.63).

### Example 2

### N-(2-{[4-(3-phenyl-1H-pyrrolo[3,2-b]pyridin-2-yl)pyridin-3-yl]oxy}ethyl)prop-2-enamide

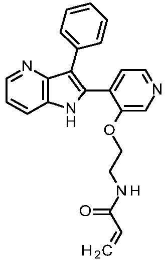

To a solution of 2-{[4-(3-phenyl-1H-pyrrolo[3,2-b]pyridin-2-yl)pyridin-3-yl]oxy}ethan-1-amine (37.0 mg, 88 % purity, 98.5 µmol) in 1 ml of DCM, triethylamine (27 µl, 200 µmol) was added. After cooling to 0°C, prop-2-enoyl chloride (7.2 µl, 89 µmol) was added and stirring was continued for additional 30 min at this temperature. The ice bath was removed and the mixture was allowed to warm to rt. The solution was diluted with DCM (5 ml) and saturated sodium hydrogencarbonate solution (aqueous, 2 mL) was added. The mixture was dried over a water repellent filter and concentrated under reduced pressure. The residue was purified by reverse phase preparative HPLC (method 3) yielding 4.50 mg (100 % purity, 12 % yield) of the desired product.
LC-MS (method 1): Rₜ = 0.81 min; MS (ESlpos): m/z = 385 [M+H]⁺
¹H-NMR (500 MHz, METHANOL-d4) δ [ppm]: -0.138 (1.06), -0.120 (0.62), -0.114 (2.75), - 0.095 (0.89), -0.084 (0.48), 0.977 (0.40), 1.007 (1.17), 1.022 (2.60), 1.037 (1.33), 1.054 (0.44), 1.279 (0.93), 1.389 (0.44), 1.404 (0.72), 1.419 (0.73), 1.434 (0.44), 1.655 (0.42), 1.833 (0.94), 1.888 (0.65), 1.933 (2.27), 3.211 (0.59), 3.229 (0.42), 3.246 (0.62), 3.345 (7.29), 3.484 (7.36), 3.495 (14.41), 3.505 (7.72), 4.081 (0.57), 4.092 (0.42), 4.122 (7.37), 4.132 (13.54), 4.143 (6.66), 5.562 (5.93), 5.570 (5.71), 5.578 (5.94), 5.586 (6.48), 5.645 (0.41), 6.000 (0.45), 6.096 (1.37), 6.130 (10.94), 6.140 (11.17), 6.147 (16.00), 6.174 (1.38), 7.243 (6.55), 7.253 (7.04), 7.260 (7.00), 7.269 (7.46), 7.278 (9.20), 7.283 (3.60), 7.288 (8.72), 7.295 (6.96), 7.299 (3.04), 7.307 (3.42), 7.310 (5.40), 7.312 (3.58), 7.325 (1.00), 7.336 (0.96), 7.350 (7.25), 7.365 (13.40), 7.376 (3.02), 7.380 (7.06), 7.430 (12.65), 7.444 (9.28), 7.447 (7.13), 7.465 (0.69), 7.483 (0.45), 7.490 (0.41), 7.967 (6.51), 7.969 (6.80), 7.983 (6.30), 7.986 (6.22), 8.000 (0.47), 8.086 (5.62), 8.096 (5.59), 8.333 (6.47), 8.336 (6.81), 8.342 (6.92), 8.345 (6.68), 8.372 (0.53), 8.400 (9.12).

### Example 3

### N-methyl-N-(3-{[4-(3-phenyl-1H-pyrrolo[3,2-b]pyridin-2-yl)pyridin-3-yl]oxy}propyl)prop-2-enamide

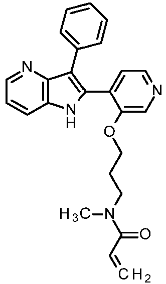

To a solution of N-methyl-3-{[4-(3-phenyl-1H-pyrrolo[3,2-b]pyridin-2-yl)pyridin-3-yl]oxy}propan-1-amine (57.0 mg, 159 µmol) in 1 ml of DCM, triethylamine (44 µl, 320 µmol) was added. At rt, prop-2-enoyl chloride (12 µl, 140 µmol) was added and stirring was continued for 1h. The solution was diluted with DCM (5 ml) and saturated sodium hydrogencarbonate solution (aqueous, 2 mL) was added. The mixture was dried over a water repellent filter and concentrated under reduced pressure. The residue was purified by reverse phase preparative HPLC (method 3) yielding 6.80 mg (100 % purity, 10 % yield) of the desired product.
LC-MS (method 1): Rₜ = 0.91 min; MS (ESlpos): m/z = 413 [M+H]⁺
¹H-NMR (600 MHz, DMSO-d6) δ [ppm]: 0.936 (0.42), 1.069 (1.57), 1.159 (16.00), 1.167 (12.80), 2.702 (1.30), 2.877 (0.92), 3.165 (0.44), 3.288 (0.41), 3.925 (0.41), 6.524 (0.61), 7.293 (0.54), 7.304 (0.44), 7.927 (1.14), 8.464 (0.44).

### Example 4

### N-methyl-N-(2-{[4-(3-phenyl-1H-pyrrolo[3,2-b]pyridin-2-yl)pyridin-3-yl]oxy}ethyl)prop-2-enamide

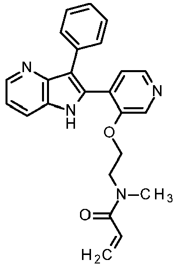

To a solution of N-methyl-2-{[4-(3-phenyl-1H-pyrrolo[3,2-b]pyridin-2-yl)pyridin-3-yl]oxy}ethan-1-amine (117 mg, 340 µmol) in DMF (1 ml) prop-2-enoic acid (35 µl, 510 µmol) and N,N-diisopropylethylamine (360 µl, 2.0 mmol) were added at rt. T3P (162 mg, 510 µmol) was added and stirring at rt was continued for 4 h. Water ( 1 ml) was added an the mixture was extracted with EtOAc twice. The combined organic layers were dried over sodium sulfate, filtered and concentrated under reduced pressure. The residue was purified by reverse phase preparative HPLC (method 3) yielding 70.0 mg (99 % purity, 51 % yield) of the desired product.
LC-MS (method 1): Rₜ = 0.84 min; MS (ESlpos): m/z = 399 [M+H]⁺
¹H-NMR (500 MHz, METHANOL-d4) δ [ppm]: -0.120 (0.91), 0.117 (0.94), 1.235 (0.47), 2.008 (0.86), 2.864 (14.42), 2.933 (16.00), 2.989 (0.52), 3.570 (1.68), 3.580 (3.20), 3.590 (1.80), 3.674 (1.91), 3.685 (3.57), 3.695 (2.03), 4.106 (1.86), 4.116 (3.35), 4.126 (1.69), 4.249 (2.10), 4.259 (3.71), 4.269 (1.92), 4.877 (1.97), 4.881 (1.75), 5.485 (2.64), 5.628 (1.52), 5.632 (1.48), 5.649 (1.53), 5.653 (1.58), 5.738 (1.26), 5.742 (1.24), 5.772 (1.41), 5.776 (1.38), 6.120 (1.40), 6.124 (1.39), 6.154 (1.65), 6.158 (1.60), 6.352 (1.18), 6.373 (1.22), 6.385 (1.13), 6.406 (1.04), 6.556 (1.45), 6.577 (1.45), 6.590 (1.32), 6.611 (1.19), 7.246 (0.53), 7.257 (1.88), 7.261 (1.74), 7.267 (2.80), 7.274 (2.53), 7.276 (2.80), 7.283 (4.94), 7.293 (3.94), 7.308 (0.81), 7.311 (1.22), 7.322 (1.80), 7.338 (3.19), 7.349 (2.99), 7.353 (3.51), 7.358 (2.75), 7.368 (3.33), 7.383 (1.69), 7.445 (3.22), 7.457 (3.98), 7.459 (4.01), 7.471 (2.48), 7.473 (1.81), 7.882 (1.48), 7.885 (1.51), 7.898 (1.45), 7.901 (1.39), 7.981 (1.54), 7.984 (1.54), 7.998 (1.51), 8.000 (1.42), 8.103 (2.07), 8.113 (2.01), 8.209 (2.11), 8.219 (2.02), 8.345 (1.57), 8.348 (1.62), 8.354 (1.64), 8.357 (1.53), 8.375 (1.62), 8.378 (1.64), 8.385 (2.05), 8.388 (3.79), 8.414 (3.27).

### Example 5

### N-(3-{[4-(3-phenyl-1H-pyrrolo[3,2-b]pyridin-2-yl)pyridin-3-yl]oxy}propyl)prop-2-enamide

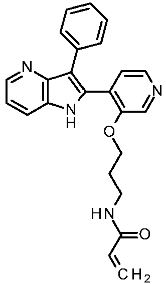

3-{[4-(3-phenyl-1H-pyrrolo[3,2-b]pyridin-2-yl)pyridin-3-yl]oxy}propan-1-amine (14.0 mg, 40.6 µmol) and prop-2-enoic acid (3.1 µl, 45 µmol) were dissolved in 1 ml DMF. At rt, HATU (18.5 mg, 48.8 µmol) and triethylamine (17 µl, 120 µmol) were added. After 16 h at rt, water (1 ml) was added and the mixture was extracted with EtOAc twice. The combined organic layers were dried over sodium sulfate, filtered and concentrated under reduced pressure. The crude product was purified by reverse phase preparative HPLC (method 7) yielding 1.00 mg (97 % purity, 6 % yield) of the title compound.
LC-MS (method 1): Rₜ = 0.82 min; MS (ESlpos): m/z = 399 [M+H]⁺
¹H-NMR (500 MHz, METHANOL-d4) δ [ppm]: -0.120 (10.97), 0.117 (11.56), 1.286 (1.36), 1.782 (1.96), 1.795 (6.27), 1.807 (9.18), 1.820 (6.32), 1.832 (1.87), 1.847 (2.19), 1.868 (0.71), 2.654 (0.99), 3.325 (7.99), 3.338 (14.70), 3.352 (7.24), 4.067 (7.92), 4.079 (16.00), 4.091 (7.66), 4.581 (2.79), 5.539 (7.61), 5.544 (7.08), 5.559 (7.79), 5.563 (8.01), 6.042 (4.06), 6.062 (3.25), 6.076 (10.86), 6.096 (10.12), 6.121 (10.99), 6.126 (11.45), 6.156 (4.33), 6.160 (3.48), 7.266 (5.65), 7.275 (6.50), 7.281 (12.30), 7.291 (6.33), 7.296 (5.99), 7.340 (6.75), 7.356 (12.04), 7.370 (5.96), 7.447 (11.37), 7.461 (8.65), 7.934 (4.88), 7.948 (4.43), 7.950 (4.48), 8.126 (1.40), 8.356 (4.70), 8.364 (4.77), 8.391 (1.43).

### Example 6

### N-(2-{[4-(6-fluoro-3-phenyl-1H-pyrrolo[3,2-b]pyridin-2-yl)pyridin-3-yl]oxy}ethyl)prop-2-enamide

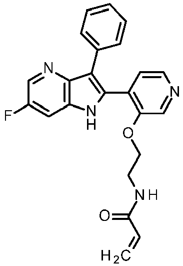

2-{[4-(6-fluoro-3-phenyl-1H-pyrrolo[3,2-b]pyridin-2-yl)pyridin-3-yl]oxy}ethan-1-amine (27.0 mg, 77.5 µmol) and prop-2-enoic acid (5.8 µl, 85 µmol) were dissolved in 1 ml DMF. At rt, HATU (35.4 mg, 93.0 µmol) and triethylamine (32 µl, 230 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 6.50 mg (93 % purity, 19 % yield) of the desired product.
LC-MS (method 1): Rₜ = 1.42 min; MS (ESlpos): m/z = 403 [M+H]⁺
¹H-NMR (500 MHz, DMSO-d6) δ [ppm]: -0.120 (1.27), 0.117 (1.36), 0.922 (3.72), 0.936 (8.72), 0.951 (4.35), 1.035 (0.44), 1.077 (6.97), 1.091 (14.11), 1.105 (7.19), 1.287 (1.20), 1.302 (2.17), 1.317 (2.20), 1.332 (1.21), 1.356 (1.44), 1.568 (1.39), 1.763 (1.43), 1.909 (0.65), 2.425 (0.60), 2.437 (1.21), 2.450 (0.69), 2.523 (3.28), 3.144 (1.81), 3.164 (3.32), 3.175 (3.19), 3.189 (0.96), 3.201 (0.91), 3.221 (3.74), 3.232 (10.50), 3.244 (10.61), 3.255 (3.94), 3.362 (2.76), 3.376 (7.09), 3.390 (6.90), 3.404 (2.28), 3.985 (0.87), 3.997 (1.27), 4.021 (7.37), 4.033 (14.80), 4.044 (6.89), 4.088 (0.64), 4.098 (0.59), 4.231 (0.61), 4.243 (1.18), 4.256 (0.58), 5.562 (6.44), 5.566 (6.15), 5.582 (6.38), 5.587 (6.90), 5.801 (0.49), 5.804 (0.45), 5.822 (0.57), 5.825 (0.63), 5.986 (0.41), 6.020 (0.59), 6.046 (4.66), 6.050 (4.83), 6.080 (8.40), 6.084 (8.15), 6.162 (7.41), 6.182 (7.79), 6.196 (4.44), 6.216 (4.32), 7.234 (3.04), 7.248 (7.79), 7.263 (5.86), 7.271 (2.03), 7.279 (11.04), 7.288 (10.76), 7.318 (9.57), 7.333 (16.00), 7.348 (7.84), 7.476 (14.92), 7.491 (12.88), 7.493 (9.84), 7.719 (5.86), 7.725 (6.74), 7.738 (5.99), 7.744 (6.32), 8.085 (0.45), 8.178 (2.71), 8.189 (4.94), 8.200 (2.72), 8.212 (1.42), 8.224 (9.57), 8.233 (8.81), 8.418 (8.28), 8.421 (9.31), 8.423 (9.26), 8.426 (7.43), 8.511 (1.32), 8.532 (14.37), 11.729 (0.78), 11.773 (10.12).

### Example 7

### N-(2-{[4-(6-fluoro-3-phenyl-1H-pyrrolo[3,2-b]pyridin-2-yl)pyridin-3-yl]oxy}ethyl)-N-methylprop-2-enamide

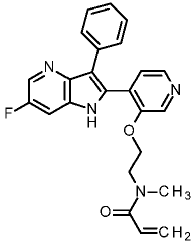

2-{[4-(6-fluoro-3-phenyl-1H-pyrrolo[3,2-b]pyridin-2-yl)pyridin-3-yl]oxy}-N-methylethan-1-amine (44.0 mg, 121 µmol) and prop-2-enoic acid (9.2 µl, 130 µmol) were dissolved in 1 ml DMF. At rt, HATU (55.4 mg, 146 µmol) and triethylamine (51 µl, 360 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 7.80 mg (92 % purity, 14 % yield) of the desired product.
LC-MS (method 1): Rₜ = 1.48 min; MS (ESlpos): m/z = 417 [M+H]⁺
¹H-NMR (500 MHz, DMSO-d6) δ [ppm]: -0.120 (1.59), 0.117 (1.66), 0.921 (6.47), 0.936 (16.00), 0.951 (7.58), 1.077 (4.51), 1.091 (9.20), 1.105 (4.64), 1.273 (0.52), 1.287 (1.89), 1.302 (3.41), 1.317 (3.39), 1.331 (1.85), 1.346 (0.51), 1.355 (1.58), 1.535 (0.69), 1.550 (1.52), 1.566 (2.00), 1.598 (0.61), 1.909 (1.45), 2.086 (4.26), 2.519 (3.84), 2.523 (2.91), 2.726 (15.41), 2.833 (13.90), 2.890 (0.64), 3.144 (2.47), 3.161 (2.17), 3.178 (2.46), 3.362 (2.14), 3.370 (1.98), 3.376 (5.92), 3.380 (3.72), 3.390 (6.17), 3.404 (1.69), 3.433 (1.64), 3.444 (3.25), 3.455 (1.68), 4.036 (1.85), 4.047 (3.54), 4.058 (1.72), 4.147 (1.73), 4.158 (3.25), 4.169 (1.61), 4.912 (1.43), 4.917 (1.33), 4.933 (1.36), 4.937 (1.48), 5.607 (1.38), 5.611 (1.32), 5.627 (1.35), 5.632 (1.45), 5.717 (1.32), 5.722 (1.34), 5.751 (1.53), 5.756 (1.79), 6.041 (1.27), 6.046 (1.28), 6.075 (1.45), 6.080 (1.47), 6.342 (1.26), 6.363 (1.32), 6.376 (1.21), 6.396 (1.12), 6.600 (1.29), 6.621 (1.32), 6.634 (1.24), 6.654 (1.09), 7.213 (0.96), 7.228 (2.43), 7.241 (2.56), 7.255 (1.26), 7.286 (2.61), 7.296 (2.82), 7.303 (3.01), 7.318 (4.73), 7.330 (4.30), 7.344 (1.92), 7.361 (2.74), 7.371 (2.76), 7.499 (3.52), 7.513 (6.19), 7.527 (3.21), 7.688 (1.53), 7.694 (1.68), 7.708 (1.54), 7.713 (1.63), 7.725 (1.41), 7.730 (1.41), 7.744 (1.26), 7.749 (1.32), 8.228 (2.05), 8.237 (2.01), 8.295 (2.45), 8.305 (2.38), 8.422 (2.24), 8.436 (2.05), 8.439 (2.25), 8.441 (2.31), 8.488 (3.76), 8.528 (3.32), 11.798 (2.15), 11.871 (2.44).

### Example 8

### N-(2-{[4-(5-methoxy-3-phenyl-1H-pyrrolo[3,2-b]pyridin-2-yl)pyridin-3-yl]oxy}ethyl)-N-methylprop-2-enamide

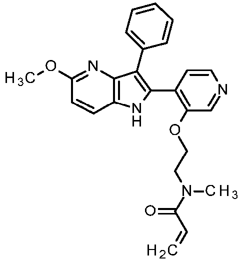

2-{[4-(5-methoxy-3-phenyl-1H-pyrrolo[3,2-b]pyridin-2-yl)pyridin-3-yl]oxy}-N-methylethan-1-amine (45.0 mg, 87 % purity, 105 µmol) and prop-2-enoic acid (7.9 µl, 120 µmol) were dissolved in 1 ml DMF. At rt, HATU (47.7 mg, 125 µmol) and triethylamine (44 µl, 310 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 4.10 mg (90 % purity, 8 % yield) of the desired product.
LC-MS (method 1): Rₜ = 1.64 min; MS (ESlpos): m/z = 429 [M+H]⁺
¹H-NMR (600 MHz, DMSO-d6) δ [ppm]: -0.100 (0.41), 0.097 (0.43), 1.846 (1.10), 2.424 (1.31), 2.516 (4.22), 2.519 (4.03), 2.522 (3.39), 2.583 (0.65), 2.653 (1.30), 2.726 (10.06), 2.843 (9.06), 3.171 (0.74), 3.343 (2.23), 3.398 (1.23), 3.407 (2.29), 3.417 (1.20), 3.864 (3.32), 3.869 (2.76), 3.873 (2.46), 3.881 (9.50), 3.901 (10.17), 3.947 (1.22), 3.969 (0.62), 3.978 (0.41), 4.002 (1.37), 4.011 (2.47), 4.020 (1.27), 4.120 (1.22), 4.129 (2.25), 4.138 (1.13), 5.016 (1.00), 5.020 (0.91), 5.033 (0.96), 5.037 (0.98), 5.616 (0.92), 5.620 (0.87), 5.633 (0.92), 5.637 (0.93), 5.756 (16.00), 5.771 (0.93), 5.776 (0.90), 5.799 (1.00), 5.804 (0.98), 6.059 (0.86), 6.063 (0.84), 6.087 (0.94), 6.091 (0.89), 6.384 (0.85), 6.401 (0.88), 6.412 (0.85), 6.429 (0.76), 6.611 (0.85), 6.629 (0.89), 6.639 (0.96), 6.647 (0.53), 6.657 (1.40), 6.662 (0.67), 6.673 (2.94), 6.687 (2.94), 7.180 (1.04), 7.193 (1.76), 7.203 (1.61), 7.215 (0.73), 7.275 (1.82), 7.283 (2.05), 7.296 (2.28), 7.308 (3.19), 7.317 (2.88), 7.330 (1.42), 7.352 (1.95), 7.360 (1.86), 7.549 (3.00), 7.562 (2.44), 7.567 (2.64), 7.581 (2.06), 7.705 (0.47), 7.720 (0.85), 7.733 (0.55), 7.751 (2.04), 7.759 (0.60), 7.765 (1.95), 7.773 (0.49), 7.796 (1.73), 7.810 (1.65), 7.994 (0.48), 8.214 (1.22), 8.221 (1.19), 8.285 (1.53), 8.293 (1.55), 8.424 (0.56), 8.458 (2.42), 8.476 (0.51), 8.496 (1.97), 8.510 (0.51), 11.517 (1.56), 11.589 (1.89).

### Example 9

### N-methyl-N-[2-({4-[3-(3-methylphenyl)-1H-pyrrolo[3,2-b]pyridin-2-yl]pyridin-3-yl}oxy)ethyl]prop-2-enamide

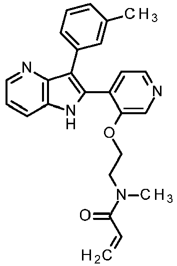

N-methyl-2-({4-[3-(3-methylphenyl)-1H-pyrrolo[3,2-b]pyridin-2-yl]pyridin-3-yl}oxy)ethan- 1-amine (30.0 mg, 83.7 µmol) and prop-2-enoic acid (6.3 µl, 92 µmol) were dissolved in 1 ml DMF. At rt, HATU (38.2 mg, 100 µmol) and triethylamine (35 µl, 250 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 5) yielding 6.40 mg (100 % purity, 19 % yield) of the desired product.
LC-MS (method 1): Rₜ = 0.90 min; MS (ESlpos): m/z = 413 [M+H]⁺
¹H-NMR (600 MHz, DMSO-d6) δ [ppm]: 1.078 (5.32), 1.090 (10.07), 1.101 (5.28), 1.882 (0.78), 2.255 (15.22), 2.267 (13.57), 2.423 (0.67), 2.652 (0.56), 2.728 (16.00), 2.831 (14.13), 3.364 (3.20), 3.376 (8.47), 3.387 (9.35), 3.455 (4.57), 4.036 (5.01), 4.152 (2.62), 4.160 (4.45), 4.889 (1.86), 4.909 (1.90), 5.614 (1.69), 5.632 (1.77), 5.729 (1.76), 5.755 (1.96), 6.058 (1.63), 6.086 (1.80), 6.353 (1.39), 6.371 (1.49), 6.381 (1.34), 6.399 (1.27), 6.599 (1.34), 6.617 (1.34), 6.627 (1.27), 6.645 (1.12), 7.026 (2.13), 7.037 (3.99), 7.048 (2.32), 7.156 (1.44), 7.169 (3.78), 7.181 (3.86), 7.193 (1.92), 7.212 (3.64), 7.224 (4.71), 7.236 (4.15), 7.250 (2.04), 7.286 (2.73), 7.293 (2.73), 7.359 (3.02), 7.367 (3.01), 7.422 (3.35), 7.448 (3.82), 7.804 (2.41), 7.817 (2.41), 7.852 (2.19), 7.864 (2.05), 8.219 (2.63), 8.227 (2.59), 8.290 (2.98), 8.298 (2.91), 8.407 (2.44), 8.413 (2.46), 8.428 (2.82), 8.434 (2.71), 8.478 (5.22), 8.520 (4.59), 11.631 (2.93), 11.707 (3.41).

### Example 10

### N-[2-({4-[3-(4-chloro-3-methylphenyl)-1H-pyrrolo[3,2-b]pyridin-2-yl]pyridin-3-yl}oxy)ethyl]-N-methylprop-2-enamide

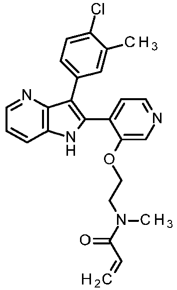

2-({4-[3-(4-chloro-3-methylphenyl)-1H-pyrrolo[3,2-b]pyridin-2-yl]pyridin-3-yl}oxy)-N-methylethan-1-amine (39.0 mg, 99.3 µmol) and prop-2-enoic acid (7.5 µl, 110 µmol) were dissolved in 1 ml DMF. At rt, HATU (45.3 mg, 119 µmol) and triethylamine (42 µl, 300 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 5) yielding 13.0 mg (100 % purity, 29 % yield) of the desired product.
LC-MS (method 2): Rₜ = 1.21 min; MS (ESlpos): m/z = 447 [M+H]⁺
¹H-NMR (600 MHz, DMSO-d6) δ [ppm]: 1.199 (0.70), 1.899 (1.24), 1.987 (0.49), 2.280 (16.00), 2.286 (15.37), 2.315 (1.59), 2.384 (2.40), 2.424 (1.74), 2.611 (2.29), 2.653 (0.86), 2.722 (13.64), 2.819 (11.73), 3.171 (0.79), 3.415 (5.41), 3.453 (4.56), 4.080 (4.94), 4.176 (4.26), 4.903 (1.85), 4.921 (1.84), 5.610 (1.69), 5.625 (1.80), 5.724 (1.67), 5.753 (1.86), 6.049 (1.52), 6.077 (1.69), 6.335 (1.19), 6.352 (1.34), 6.363 (1.24), 6.380 (1.07), 6.586 (1.11), 6.603 (1.15), 6.614 (1.11), 6.631 (0.90), 7.229 (3.43), 7.287 (3.85), 7.319 (5.88), 7.374 (2.69), 7.382 (2.77), 7.589 (3.37), 7.603 (3.85), 7.815 (2.24), 7.829 (2.25), 7.856 (2.08), 7.869 (1.99), 8.255 (2.21), 8.262 (2.34), 8.311 (2.62), 8.318 (2.67), 8.432 (2.71), 8.441 (3.05), 8.500 (4.36), 8.542 (3.95), 11.721 (2.79), 11.788 (3.15).

### Example 11

### N-[2-({4-[3-(4-chlorophenyl)-1H-pyrrolo[3,2-b]pyridin-2-yl]pyridin-3-yl}oxy)ethyl]-N-methylprop-2-enamide

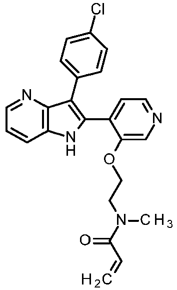

2-({4-[3-(4-chlorophenyl)-1H-pyrrolo[3,2-b]pyridin-2-yl]pyridin-3-yl}oxy)-N-methylethan-1-amine (23.0 mg, 60.7 µmol) and prop-2-enoic acid (4.6 µl, 67 µmol) were dissolved in 1 ml DMF. At rt, HATU (27.7 mg, 72.8 µmol) and triethylamine (25 µl, 180 µmol) were added. After 16 h at rt, water (1 ml) was added and the mixture was extracted with EtOAc twice. The combined organic layers were dried over sodium sulfate, filtered and concentrated under reduced pressure. The residue was purified by by reverse phase preparative HPLC (method 5) yielding 5.00 mg (100 % purity, 19 % yield) of the desired product.
LC-MS (method 1): Rₜ = 1.03 min; MS (ESlpos): m/z = 433 [M+H]⁺
¹H-NMR (600 MHz, DMSO-d6) δ [ppm]: 1.077 (4.20), 1.089 (8.43), 1.101 (4.19), 1.907 (1.33), 2.423 (0.86), 2.652 (0.78), 2.718 (16.00), 2.808 (13.65), 3.364 (1.91), 3.376 (6.08), 3.387 (8.04), 3.396 (2.70), 3.409 (2.26), 3.418 (3.93), 3.427 (2.01), 4.063 (2.37), 4.071 (4.24), 4.080 (2.14), 4.150 (2.15), 4.159 (3.78), 4.169 (1.92), 4.892 (1.63), 4.896 (1.51), 4.909 (1.61), 4.913 (1.66), 5.602 (1.48), 5.606 (1.40), 5.624 (1.48), 5.714 (1.50), 5.719 (1.52), 5.742 (1.73), 5.746 (1.70), 5.754 (1.13), 6.039 (1.42), 6.067 (1.50), 6.071 (1.51), 6.327 (1.39), 6.344 (1.45), 6.354 (1.36), 6.371 (1.17), 6.582 (1.25), 6.600 (1.29), 6.610 (1.30), 6.627 (1.13), 7.228 (2.11), 7.235 (2.75), 7.241 (2.27), 7.248 (1.97), 7.335 (2.44), 7.343 (2.48), 7.376 (7.57), 7.389 (8.89), 7.399 (3.26), 7.406 (2.96), 7.571 (5.68), 7.575 (6.81), 7.586 (5.67), 7.589 (5.34), 7.822 (2.09), 7.835 (2.03), 7.852 (1.78), 7.865 (1.66), 8.273 (2.51), 8.281 (2.45), 8.325 (3.05), 8.333 (2.88), 8.425 (1.99), 8.432 (2.05), 8.439 (2.42), 8.446 (2.20), 8.501 (5.05), 8.542 (4.18), 11.760 (2.53), 11.820 (3.07).

### Example 12

### N-[2-({4-[3-(4-fluoro-3-methylphenyl)-1H-pyrrolo[3,2-b]pyridin-2-yl]pyridin-3-yl}oxy)ethyl]-N-methylprop-2-enamide

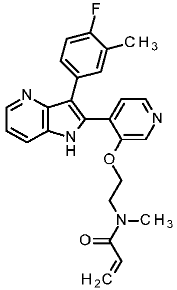

2-({4-[3-(4-fluoro-3-methylphenyl)-1H-pyrrolo[3,2-b]pyridin-2-yl]pyridin-3-yl}oxy)-N-methylethan-1-amine (29.0 mg, 77.0 µmol) and prop-2-enoic acid (5.8 µl, 85 µmol) were dissolved in 1 ml DMF. At rt, HATU (35.2 mg, 92.4 µmol) and triethylamine (32 µl, 230 µmol) were added. After 16 h at rt, water (1 ml) was added and the mixture was extracted with EtOAc twice. The combined organic layers were dried over sodium sulfate, filtered and concentrated under reduced pressure. The residue was purified by by reverse phase preparative HPLC (method 5) yielding 6.00 mg (91 % purity, 16 % yield) of the desired product.
LC-MS (method 1): Rₜ = 0.97 min; MS (ESlpos): m/z = 431 [M+H]⁺
¹H-NMR (500 MHz, DMSO-d6) δ [ppm]: -0.120 (0.46), 0.117 (0.46), 1.077 (4.94), 1.091 (10.11), 1.105 (5.07), 1.858 (0.51), 2.195 (9.05), 2.202 (8.14), 2.520 (0.49), 2.573 (0.65), 2.633 (0.51), 2.729 (16.00), 2.834 (14.53), 3.362 (1.99), 3.376 (5.17), 3.390 (5.07), 3.404 (1.73), 3.420 (1.72), 3.430 (3.45), 3.441 (1.84), 3.471 (1.67), 3.482 (3.36), 3.493 (1.68), 4.067 (1.85), 4.078 (3.56), 4.088 (1.72), 4.170 (1.73), 4.181 (3.35), 4.192 (1.60), 4.876 (1.51), 4.881 (1.41), 4.897 (1.44), 4.902 (1.54), 5.610 (1.43), 5.615 (1.39), 5.631 (1.42), 5.635 (1.52), 5.715 (1.40), 5.721 (1.42), 5.749 (1.57), 5.754 (1.56), 6.053 (1.33), 6.058 (1.33), 6.087 (1.52), 6.091 (1.49), 6.339 (1.33), 6.360 (1.37), 6.373 (1.28), 6.393 (1.17), 6.594 (1.36), 6.615 (1.38), 6.627 (1.29), 6.648 (1.17), 7.043 (1.28), 7.051 (1.16), 7.061 (1.94), 7.068 (1.80), 7.080 (1.43), 7.088 (1.24), 7.205 (1.81), 7.209 (2.08), 7.214 (2.00), 7.218 (2.17), 7.221 (2.17), 7.225 (2.26), 7.231 (2.12), 7.234 (2.40), 7.242 (1.71), 7.248 (1.71), 7.253 (2.18), 7.259 (1.49), 7.264 (1.36), 7.269 (1.31), 7.292 (2.49), 7.301 (2.52), 7.360 (2.80), 7.369 (2.84), 7.493 (1.12), 7.509 (2.12), 7.525 (1.21), 7.805 (1.92), 7.808 (1.95), 7.821 (1.84), 7.824 (1.77), 7.852 (1.73), 7.855 (1.70), 7.868 (1.64), 7.871 (1.52), 8.135 (6.59), 8.237 (2.61), 8.247 (2.56), 8.300 (3.00), 8.310 (2.89), 8.410 (1.69), 8.412 (1.80), 8.418 (1.88), 8.421 (1.86), 8.428 (2.11), 8.431 (2.06), 8.437 (1.95), 8.439 (1.79), 8.493 (4.65), 8.522 (0.57), 8.533 (4.10), 11.649 (2.23), 11.722 (2.48).

### Example 13

### Methyl 2-chloro-5-[2-(3-{2-[methyl(prop-2-enoyl)amino]ethoxy}pyridin-4-yl)-1H-pyrrolo[3,2-b]pyridin-3-yl]benzoate

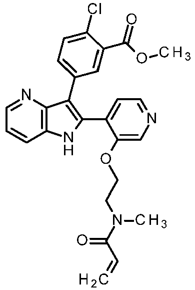

Methyl 2-chloro-5-(2-{3-[2-(methylamino)ethoxy]pyridin-4-yl}-1H-pyrrolo[3,2-b]pyridin-3-yl)benzoate (26.0 mg, 59.5 µmol) and prop-2-enoic acid (4.5 µl, 65 µmol) were dissolved in 1 ml DMF. At rt, HATU (27.2 mg, 71.4 µmol) and triethylamine (25 µl, 180 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 6) yielding 10.0 mg (99 % purity, 34 % yield) of the desired product.
LC-MS (method 1): Rₜ = 1.13 min; MS (ESlpos): m/z = 491 [M+H]⁺
¹H-NMR (600 MHz, DMSO-d6) δ [ppm]: 1.079 (7.92), 1.091 (16.00), 1.102 (7.88), 2.516 (1.02), 2.520 (0.91), 2.523 (0.74), 2.697 (11.96), 2.762 (10.12), 3.356 (1.54), 3.365 (5.59), 3.377 (9.34), 3.381 (3.12), 3.389 (8.88), 3.400 (2.66), 3.802 (14.56), 3.805 (12.44), 4.074 (1.53), 4.083 (2.90), 4.091 (1.48), 4.152 (1.34), 4.161 (2.56), 4.170 (1.27), 4.881 (1.16), 4.885 (1.11), 4.898 (1.13), 4.902 (1.19), 5.581 (1.03), 5.585 (0.99), 5.598 (1.01), 5.602 (1.07), 5.694 (1.09), 5.698 (1.08), 5.722 (1.20), 5.726 (1.17), 6.015 (0.97), 6.019 (0.96), 6.043 (1.08), 6.047 (1.05), 6.291 (1.02), 6.308 (1.03), 6.318 (0.98), 6.335 (0.90), 6.542 (0.94), 6.560 (0.95), 6.570 (0.90), 6.587 (0.83), 7.248 (1.17), 7.252 (1.44), 7.256 (1.32), 7.260 (1.75), 7.266 (1.51), 7.269 (1.32), 7.273 (1.37), 7.385 (1.75), 7.393 (1.81), 7.441 (2.09), 7.449 (2.11), 7.515 (1.95), 7.520 (2.31), 7.529 (2.23), 7.534 (2.48), 7.758 (1.09), 7.762 (1.12), 7.772 (1.03), 7.776 (1.13), 7.781 (1.43), 7.785 (1.37), 7.795 (1.18), 7.799 (1.17), 7.845 (1.45), 7.856 (1.41), 7.869 (1.30), 7.883 (1.18), 8.048 (2.33), 8.052 (2.33), 8.064 (2.08), 8.068 (1.94), 8.309 (1.83), 8.317 (1.80), 8.353 (2.23), 8.361 (2.15), 8.461 (1.32), 8.468 (1.39), 8.473 (1.66), 8.475 (1.58), 8.481 (1.52), 8.528 (3.66), 8.567 (3.03), 11.897 (1.79), 11.942 (2.12).

### Example 14

### N-[2-({4-[3-(3-fluorophenyl)-1H-pyrrolo[3,2-b]pyridin-2-yl]pyridin-3-yl}oxy)ethyl]-N-methylprop-2-enamide

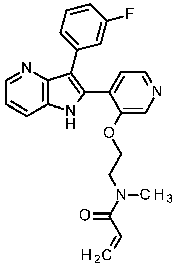

2-({4-[3-(3-fluorophenyl)-1H-pyrrolo[3,2-b]pyridin-2-yl]pyridin-3-yl}oxy)-N-methylethan-1-amine (29.0 mg, 80.0 µmol) and prop-2-enoic acid (6.0 µl, 88 µmol) were dissolved in 1 ml DMF. At rt, HATU (36.5 mg, 96.0 µmol) and triethylamine (33 µl, 240 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 6) yielding 8.00 mg (98 % purity, 24 % yield) of the desired product.
LC-MS (method 1): Rₜ = 0.93 min; MS (ESlpos): m/z = 417 [M+H]⁺
¹H-NMR (600 MHz, DMSO-d6) δ [ppm]: 1.079 (8.02), 1.091 (16.00), 1.102 (7.99), 2.345 (0.47), 2.520 (0.89), 2.699 (0.92), 2.713 (15.51), 2.792 (13.37), 3.365 (2.85), 3.377 (8.12), 3.389 (8.20), 3.396 (2.28), 3.401 (4.09), 3.405 (4.08), 3.414 (2.40), 3.421 (2.14), 3.430 (3.61), 3.439 (1.85), 4.078 (2.11), 4.087 (3.98), 4.096 (2.02), 4.159 (1.92), 4.168 (3.56), 4.177 (1.75), 4.850 (1.54), 4.855 (1.47), 4.868 (1.50), 4.872 (1.57), 5.596 (1.36), 5.600 (1.33), 5.613 (1.35), 5.617 (1.43), 5.691 (1.45), 5.695 (1.44), 5.719 (1.59), 5.722 (1.57), 6.033 (1.28), 6.037 (1.30), 6.060 (1.43), 6.064 (1.41), 6.308 (1.32), 6.325 (1.35), 6.336 (1.28), 6.353 (1.20), 6.568 (1.22), 6.585 (1.24), 6.596 (1.20), 6.613 (1.08), 7.022 (1.16), 7.036 (2.27), 7.050 (1.27), 7.233 (1.49), 7.238 (2.02), 7.246 (2.76), 7.251 (2.14), 7.259 (1.83), 7.282 (2.16), 7.295 (4.53), 7.305 (1.42), 7.318 (2.46), 7.331 (1.82), 7.342 (0.67), 7.357 (2.36), 7.365 (2.40), 7.415 (2.73), 7.423 (2.75), 7.484 (1.57), 7.502 (1.54), 7.829 (1.94), 7.840 (1.87), 7.860 (1.70), 7.872 (1.58), 8.287 (2.32), 8.295 (2.30), 8.338 (2.81), 8.346 (2.70), 8.446 (1.88), 8.453 (1.84), 8.460 (2.12), 8.467 (2.02), 8.520 (4.83), 8.559 (3.94), 11.802 (2.29), 11.858 (2.70).

### Example 15

### N-[2-({4-[3-(4-chloro-3-methoxyphenyl)-1 H-pyrrolo[3,2-b]pyridin-2-yl]pyridin-3-yl}oxy)ethyl]-N-methylprop-2-enamide

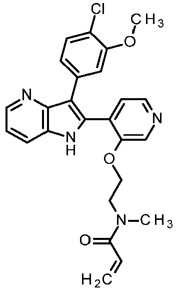

To a solution of 2-({4-[3-(4-chloro-3-methoxyphenyl)-1 H-pyrrolo[3,2-b]pyridin-2-yl]pyridin-3-yl}oxy)-N-methylethanamine trifluoroacetate (1/1) (65.0 mg, 124 µmol) in DMF (1 ml), prop-2-enoic acid (8.5 µl, 120 µmol) and N,N-diisopropylethylamine (130 µl, 750 µmol) were added at rt. T3P (110 µl, 50 % purity in DMF, 190 µmol) was added and stirring at rt was continued for 16 h. Water ( 1 ml) was added an the mixture was extracted with EtOAc twice. The combined organic layers were dried over sodium sulfate, filtered and concentrated under reduced pressure. The residue was purified by reverse phase preparative HPLC (method 3) yielding 21.0 mg (94 % purity, 34 % yield) of the desired product.
LC-MS (method 1): Rₜ = 1.05 min; MS (ESlpos): m/z = 463 [M+H]⁺
¹H NMR (600 MHz, DMSO-*d*₆) δ ppm 2.66 - 2.73 (m, 4 H) 2.75 - 2.84 (m, 3 H) 3.39 - 3.51 (m, 6 H) 4.04 - 4.13 (m, 3 H) 4.14 - 4.24 (m, 2 H) 4.83 - 4.90 (m, 1 H) 5.55 - 5.63 (m, 1 H) 5.66 - 5.73 (m, 1 H) 5.73 - 5.79 (m, 3 H) 5.98 - 6.08 (m, 1 H) 6.27 - 6.38 (m, 1 H) 6.52 - 6.62 (m, 1 H) 7.19 - 7.30 (m, 7 H) 7.31 - 7.39 (m, 4 H) 7.40 - 7.46 (m, 1 H) 7.83 - 7.95 (m, 2 H) 8.27 - 8.32 (m, 1 H) 8.32 - 8.39 (m, 1 H) 8.43 - 8.49 (m, 2 H) 8.50 - 8.54 (m, 1 H) 8.55 - 8.61 (m, 1 H) 11.73 - 12.05 (m, 2 H)

### Example 16

### N-[2-({4-[3-(1H-indol-6-yl)-1H-pyrrolo[3,2-b]pyridin-2-yl]pyridin-3-yl}oxy)ethyl]-N-methylprop-2-enamide

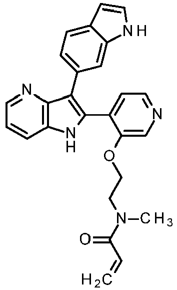

2-({4-[3-(1H-indol-6-yl)-1H-pyrrolo[3,2-b]pyridin-2-yl]pyridin-3-yl}oxy)-N-methylethan-1-amine (126 mg, 25 % purity, 63.4 µmol) and prop-2-enoic acid (4.8 µl, 70 µmol) were dissolved in 1 ml DMF. At rt, HATU (28.9 mg, 76.1 µmol) and triethylamine (53 µl, 380 µmol) were added. After 16 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 2 fractions still containing impurities. Therefore, these fractions were combined and purified by preparative thin layer chromatography (SiO₂, Eluent DCM:MeOH 10:1) yielding 2.30 mg (89 % purity, 7 % yield) of the desired product.
LC-MS (method 1): Rₜ = 0.88 min; MS (ESlpos): m/z = 438 [M+H]⁺
¹H-NMR (500 MHz, METHANOL-d4) δ [ppm]: 1.092 (0.55), 1.131 (0.94), 1.143 (0.76), 1.283 (2.02), 1.418 (0.87), 1.453 (0.66), 1.903 (2.15), 1.932 (0.96), 2.015 (0.55), 2.029 (0.73), 2.046 (0.57), 2.334 (0.92), 2.351 (0.98), 2.367 (0.58), 2.652 (0.40), 2.756 (0.56), 2.811 (15.33), 2.819 (4.99), 2.909 (16.00), 3.239 (2.15), 3.344 (6.77), 3.409 (0.49), 3.424 (0.95), 3.440 (2.98), 3.452 (4.79), 3.462 (2.41), 3.598 (0.42), 3.628 (2.75), 3.638 (5.24), 3.649 (2.87), 3.986 (2.30), 3.996 (4.20), 4.007 (2.23), 4.122 (0.57), 4.131 (0.41), 4.212 (2.97), 4.223 (4.76), 4.233 (2.48), 4.492 (0.41), 4.502 (0.63), 4.582 (0.42), 4.924 (1.58), 4.927 (1.58), 4.945 (1.56), 4.949 (1.56), 5.485 (1.96), 5.625 (1.71), 5.629 (1.70), 5.646 (1.76), 5.650 (1.77), 5.784 (1.42), 5.787 (1.41), 5.817 (1.59), 5.821 (1.56), 6.130 (1.58), 6.134 (1.56), 6.164 (1.85), 6.167 (1.89), 6.333 (1.25), 6.355 (1.29), 6.367 (1.18), 6.388 (1.10), 6.426 (2.57), 6.432 (2.62), 6.443 (2.81), 6.449 (2.79), 6.559 (1.48), 6.580 (1.48), 6.592 (1.33), 6.613 (1.20), 7.048 (1.97), 7.066 (3.84), 7.082 (1.99), 7.214 (3.05), 7.220 (3.38), 7.230 (3.72), 7.236 (3.88), 7.254 (2.60), 7.257 (2.41), 7.264 (2.36), 7.267 (2.50), 7.280 (1.71), 7.293 (0.92), 7.311 (2.53), 7.321 (2.51), 7.397 (2.27), 7.407 (2.34), 7.490 (2.43), 7.506 (2.36), 7.525 (6.34), 7.541 (2.73), 7.566 (3.44), 7.882 (1.99), 7.898 (1.93), 7.943 (0.43), 7.997 (2.06), 8.012 (2.03), 8.039 (1.77), 8.049 (1.79), 8.184 (1.72), 8.194 (1.67), 8.229 (0.54), 8.245 (0.49), 8.329 (3.08), 8.336 (4.81), 8.367 (2.54), 8.376 (3.04), 8.383 (3.16), 8.474 (0.44).

### Example 17

### N-[2-({4-[3-(2-methoxyphenyl)-1H-pyrrolo[3,2-b]pyridin-2-yl]pyridin-3-yl}oxy)ethyl]-N-methylprop-2-enamide

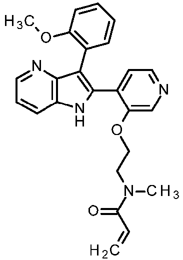

2-({4-[3-(2-methoxyphenyl)-1H-pyrrolo[3,2-b]pyridin-2-yl]pyridin-3-yl}oxy)-N-methylethan-1-amine (26.0 mg, 69.4 µmol) and prop-2-enoic acid (5.2 µl, 76 µmol) were dissolved in 1 ml acetonnitrile. At rt, HATU (31.7 mg, 83.3 µmol) and triethylamine (29 µl, 210 µmol) were added. After 2 h at rt, water (1 ml) was added and the mixture was extracted with EtOAc twice. The combined organic layers were dried over sodium sulfate, filtered and concentrated under reduced pressure. The residue was purified by silica gel column chromatography (eluent: DCM/methanol 10:1) yielding 7.50 mg (97 % purity, 24 % yield) of the title compound.
LC-MS (method 2): Rₜ = 0.86 min; MS (ESlpos): m/z = 429 [M+H]⁺
¹H-NMR (600 MHz, DMSO-d6) δ [ppm]: 1.175 (0.42), 1.235 (1.35), 1.909 (1.84), 1.946 (0.54), 2.073 (9.40), 2.516 (1.23), 2.519 (1.08), 2.522 (0.97), 2.689 (15.13), 2.717 (0.86), 2.751 (0.74), 2.775 (11.74), 2.855 (0.58), 2.887 (1.48), 2.963 (16.00), 3.287 (5.58), 3.333 (14.50), 3.522 (1.61), 3.531 (2.95), 3.540 (1.58), 3.658 (2.24), 3.666 (3.93), 3.675 (2.11), 4.083 (1.66), 4.092 (2.84), 4.101 (1.43), 4.213 (0.42), 4.244 (2.41), 4.253 (4.07), 4.262 (2.07), 4.286 (0.50), 5.009 (1.00), 5.013 (0.92), 5.026 (0.97), 5.030 (0.93), 5.657 (1.74), 5.661 (1.58), 5.674 (1.69), 5.678 (1.67), 5.784 (0.95), 5.787 (0.87), 5.811 (1.02), 5.815 (0.93), 6.127 (1.63), 6.131 (1.48), 6.154 (1.74), 6.158 (1.61), 6.462 (0.90), 6.480 (0.92), 6.490 (0.87), 6.508 (0.79), 6.688 (1.53), 6.705 (1.53), 6.716 (1.45), 6.733 (1.28), 6.938 (1.30), 6.952 (1.31), 6.981 (2.28), 6.987 (2.32), 6.995 (2.46), 7.002 (1.84), 7.015 (2.35), 7.027 (1.28), 7.107 (3.05), 7.115 (2.99), 7.153 (2.21), 7.161 (2.07), 7.215 (1.62), 7.271 (0.83), 7.283 (1.27), 7.294 (0.72), 7.309 (1.11), 7.311 (1.13), 7.322 (1.69), 7.335 (0.91), 7.448 (1.53), 7.460 (1.49), 7.487 (0.79), 7.499 (0.74), 7.862 (0.52), 7.977 (0.97), 7.991 (0.85), 8.084 (2.76), 8.092 (2.53), 8.180 (2.15), 8.188 (2.01), 8.333 (2.12), 8.340 (2.01), 8.356 (1.37), 8.363 (1.23), 8.445 (3.09), 8.485 (0.74), 8.497 (4.35), 11.574 (0.53).

### Example 18

### N-methyl-N-[2-({4-[3-(quinolin-7-yl)-1H-pyrrolo[3,2-b]pyridin-2-yl]pyridin-3-yl}oxy)ethyl]prop-2-enamide

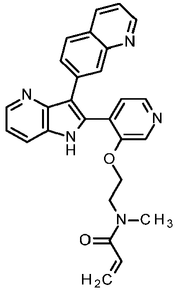

N-methyl-2-({4-[3-(quinolin-7-yl)-1H-pyrrolo[3,2-b]pyridin-2-yl]pyridin-3-yl}oxy)ethanamine trifluoroacetate (1:1) (95.0 mg, 187 µmol) and prop-2-enoic acid (6.4 µl, 93 µmol) were dissolved in 1 ml acetonitrile. At rt, HATU (42.6 mg, 112 µmol) and triethylamine (160 µl, 1.1 mmol) were added. After 2 h at rt, water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 6.60 mg (100 % purity, 8 % yield) of the desired product.
LC-MS (method 1): Rₜ = 0.76 min; MS (ESlpos): m/z = 450 [M+H]⁺
¹H-NMR (600 MHz, DMSO-d6) δ [ppm]: 2.385 (0.98), 2.424 (0.75), 2.516 (2.56), 2.519 (2.53), 2.596 (0.56), 2.626 (16.00), 2.653 (0.72), 2.715 (13.16), 3.164 (2.52), 3.173 (2.55), 3.183 (0.40), 3.239 (2.33), 3.248 (4.29), 3.257 (2.37), 3.333 (2.80), 3.989 (2.36), 3.998 (4.26), 4.007 (2.16), 4.084 (0.87), 4.092 (0.86), 4.101 (0.46), 4.114 (2.06), 4.123 (3.67), 4.133 (1.84), 4.853 (1.61), 4.857 (1.56), 4.870 (1.57), 4.875 (1.59), 5.559 (1.37), 5.562 (1.37), 5.576 (1.35), 5.580 (1.40), 5.685 (1.50), 5.689 (1.50), 5.712 (1.66), 5.716 (1.63), 6.001 (1.27), 6.005 (1.27), 6.029 (1.43), 6.032 (1.40), 6.218 (1.43), 6.235 (1.43), 6.245 (1.34), 6.262 (1.22), 6.521 (1.21), 6.539 (1.23), 6.549 (1.14), 6.567 (1.03), 7.264 (1.57), 7.270 (2.44), 7.277 (3.06), 7.283 (2.44), 7.290 (1.74), 7.385 (2.40), 7.394 (2.33), 7.450 (2.71), 7.458 (5.12), 7.466 (4.42), 7.780 (1.44), 7.793 (1.89), 7.813 (1.66), 7.827 (2.34), 7.867 (2.47), 7.880 (6.64), 7.895 (4.41), 7.911 (1.74), 8.266 (2.71), 8.274 (5.99), 8.293 (4.20), 8.307 (3.69), 8.334 (2.78), 8.341 (2.61), 8.483 (2.17), 8.491 (2.13), 8.502 (2.59), 8.512 (5.11), 8.562 (3.77), 8.827 (2.47), 8.834 (3.84), 11.862 (2.56), 11.924 (3.12).

### Example 19

### N-[2-({4-[3-(3-fluoro-2-methoxyphenyl)-1H-pyrrolo[3,2-b]pyridin-2-yl]pyridin-3-yl}oxy)ethyl]-N-methylprop-2-enamide

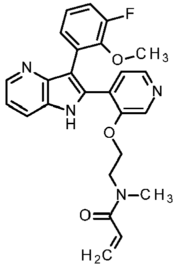

To a solution of 2-({4-[3-(3-fluoro-2-methoxyphenyl)-1H-pyrrolo[3,2-b]pyridin-2-yl]pyridin-3-yl}oxy)-N-methylethanamine trifluoroacetate (1:1) (68.0 mg, 91 % purity, 122 µmol) in DMF (1 ml), prop-2-enoic acid (8.4 µl, 120 µmol) and N,N-diisopropylethylamine (130 µl, 730 µmol) were added at rt. T3P (110 µl, 50 % purity in DMF, 180 µmol) was added and stirring at rt was continued for 16 h. Water ( 1 ml) was added an the mixture was extracted with EtOAc twice. The combined organic layers were dried over sodium sulfate, filtered and concentrated under reduced pressure. The residue was purified by reverse phase preparative HPLC (method 3) yielding 17.6 mg (100 % purity, 32 % yield) of the desired product.
LC-MS (method 2): Rₜ = 0.97 min; MS (ESlpos): m/z = 447 [M+H]⁺
¹H-NMR (400 MHz, DMSO-d6) δ [ppm]: 2.366 (0.48), 2.709 (0.47), 2.791 (14.01), 2.888 (0.56), 2.959 (16.00), 3.167 (5.57), 3.373 (14.12), 3.653 (3.95), 3.680 (2.84), 3.693 (4.62), 4.086 (0.48), 4.185 (3.91), 4.263 (2.71), 4.276 (4.54), 4.901 (1.48), 4.928 (1.51), 5.642 (1.85), 5.673 (1.86), 5.723 (1.41), 5.764 (1.60), 6.103 (1.70), 6.144 (1.93), 6.457 (1.22), 6.483 (1.28), 6.497 (1.13), 6.524 (1.05), 6.666 (1.53), 6.692 (1.57), 6.707 (1.44), 6.734 (1.27), 7.102 (6.05), 7.134 (2.68), 7.152 (1.43), 7.163 (1.45), 7.195 (4.92), 7.214 (6.96), 7.227 (6.03), 7.261 (2.54), 7.282 (1.74), 7.825 (2.19), 7.846 (2.03), 7.931 (2.40), 7.953 (2.32), 8.119 (3.01), 8.131 (2.87), 8.168 (2.65), 8.180 (2.41), 8.340 (2.87), 8.353 (4.45), 8.365 (2.36), 8.473 (4.24), 8.511 (4.89), 11.633 (3.15), 11.740 (2.75).

### Example 20

### N-[2-({4-[3-(4-chloro-3-ethylphenyl)-1H-pyrrolo[3,2-b]pyridin-2-yl]pyridin-3-yl}oxy)ethyl]-N-methylprop-2-enamide

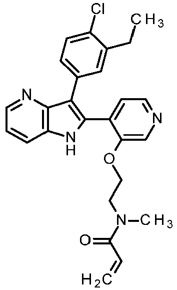

To a solution of 2-({4-[3-(4-chloro-3-ethylphenyl)-1H-pyrrolo[3,2-b]pyridin-2-yl]pyridin-3-yl}oxy)-N-methylethanamine trifluoroacetate (1:1) (130 mg, 250 µmol) in DMF (1 ml), prop-2-enoic acid (17 µl, 250 µmol) and N,N-diisopropylethylamine (260 µl, 1.5 mmol) were added at rt. T3P (220 µl, 50 % purity in DMF, 370 µmol) was added and stirring at rt was continued for 16 h. Water (1 ml) was added an the mixture was extracted with EtOAc twice. The combined organic layers were dried over sodium sulfate, filtered and concentrated under reduced pressure. The residue was purified by reverse phase preparative HPLC (method 3) yielding 36.0 mg (98 % purity, 31 % yield) of the desired product.
LC-MS (method 2): Rₜ = 1.35 min; MS (ESlpos): m/z = 461 [M+H]⁺
¹H-NMR (400 MHz, DMSO-d6) δ [ppm]: 1.002 (5.70), 1.020 (11.63), 1.038 (5.95), 2.084 (0.54), 2.327 (0.41), 2.577 (2.40), 2.596 (6.76), 2.615 (6.64), 2.633 (2.20), 2.670 (0.43), 2.711 (16.00), 2.794 (13.35), 3.168 (8.81), 3.360 (2.83), 3.373 (4.59), 3.387 (2.64), 3.400 (2.28), 3.414 (3.82), 3.427 (2.05), 4.032 (2.28), 4.045 (4.25), 4.058 (2.25), 4.137 (2.07), 4.151 (3.67), 4.164 (1.84), 4.884 (1.56), 4.890 (1.58), 4.916 (1.70), 5.596 (1.40), 5.622 (1.51), 5.718 (1.55), 5.759 (1.73), 6.027 (1.32), 6.032 (1.34), 6.074 (1.52), 6.310 (1.39), 6.336 (1.38), 6.352 (1.29), 6.378 (1.19), 6.556 (1.28), 6.581 (1.30), 6.597 (1.15), 6.623 (1.06), 7.215 (2.41), 7.227 (2.55), 7.235 (2.56), 7.247 (2.38), 7.340 (2.60), 7.352 (3.01), 7.359 (5.97), 7.379 (7.17), 7.412 (5.76), 7.420 (5.39), 7.511 (2.32), 7.524 (2.68), 7.538 (1.73), 7.817 (2.38), 7.837 (2.36), 7.850 (2.20), 7.870 (1.90), 8.273 (2.50), 8.284 (2.46), 8.329 (2.98), 8.341 (2.84), 8.431 (2.26), 8.444 (3.72), 8.458 (2.39), 8.497 (4.87), 8.542 (4.04), 11.751 (2.43), 11.813 (2.80).

### Example 21

### N-[2-({4-[3-(3-chloro-2-methoxyphenyl)-1H-pyrrolo[3,2-b]pyridin-2-yl]pyridin-3-yl}oxy)ethyl]-N-methylprop-2-enamide

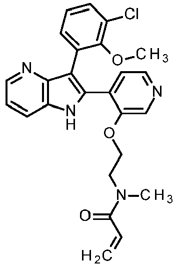

To a solution of 2-({4-[3-(3-chloro-2-methoxyphenyl)-1 H-pyrrolo[3,2-b]pyridin-2-yl]pyridin-3-yl}oxy)-N-methylethanamine trifluoroacetate (1:1) (82.0 mg, 157 µmol) in DMF (1 ml), prop-2-enoic acid (11 µl, 160 µmol) and N,N-diisopropylethylamine (160 µl, 940 µmol) were added at rt. T3P (140 µl, 50 % purity in DMF, 240 µmol) was added and stirring at rt was continued for 16 h. Water (1 ml) was added an the mixture was extracted with EtOAc twice. The combined organic layers were dried over sodium sulfate, filtered and concentrated under reduced pressure. The residue was purified by reverse phase preparative HPLC (method 3) yielding 25.0 mg (100 % purity, 34 % yield) of the desired product.
LC-MS (method 1): Rₜ = 0.96 min; MS (ESlpos): m/z = 463 [M+H]⁺
¹H-NMR (400 MHz, DMSO-d6) δ [ppm]: 2.084 (0.40), 2.794 (12.39), 2.958 (13.13), 3.172 (12.76), 3.249 (13.79), 3.273 (0.58), 3.648 (1.46), 3.661 (2.94), 3.674 (3.15), 3.687 (3.32), 3.699 (1.75), 4.197 (1.52), 4.210 (2.82), 4.222 (1.56), 4.266 (1.77), 4.279 (3.20), 4.292 (1.63), 4.873 (1.17), 4.879 (1.18), 4.899 (1.13), 4.904 (1.29), 5.634 (1.29), 5.640 (1.34), 5.660 (1.32), 5.666 (1.45), 5.699 (1.09), 5.706 (1.16), 5.741 (1.33), 5.754 (16.00), 6.091 (1.21), 6.097 (1.26), 6.133 (1.43), 6.139 (1.44), 6.460 (1.06), 6.486 (1.05), 6.502 (0.99), 6.528 (0.88), 6.659 (1.26), 6.685 (1.27), 6.701 (1.17), 6.727 (1.06), 7.076 (2.24), 7.088 (4.56), 7.100 (2.45), 7.165 (1.14), 7.176 (1.32), 7.184 (2.52), 7.196 (2.73), 7.209 (2.90), 7.215 (2.29), 7.220 (2.75), 7.230 (2.57), 7.241 (2.49), 7.402 (1.84), 7.424 (2.93), 7.447 (2.44), 7.467 (1.55), 7.512 (1.62), 7.528 (1.39), 7.849 (1.50), 7.869 (1.36), 7.950 (1.60), 7.970 (1.48), 8.111 (2.17), 8.123 (2.16), 8.153 (2.14), 8.165 (2.02), 8.349 (1.91), 8.360 (3.03), 8.373 (1.71), 8.480 (3.41), 8.511 (3.56), 11.687 (1.78), 11.787 (1.66).

### Example 22

### N-[2-({4-[3-(3-chlorophenyl)-1H-pyrrolo[3,2-b]pyridin-2-yl]pyridin-3-yl}oxy)ethyl]-N-methylprop-2-enamide

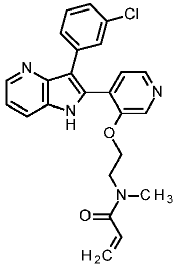

To a solution of 2-({4-[3-(3-chlorophenyl)-1H-pyrrolo[3,2-b]pyridin-2-yl]pyridin-3-yl}oxy)-N-methylethanamine trifluoroacetate (1:1) (123 mg, 250 µmol) in DMF (1 ml), prop-2-enoic acid (17 µl, 250 µmol) and N,N-diisopropylethylamine (260 µl, 1.5 mmol) were added at rt. T3P (220 µl, 50 % purity in DMF, 370 µmol) was added and stirring at rt was continued for 16 h. Water (1 ml) was added an the mixture was extracted with EtOAc twice. The combined organic layers were dried over sodium sulfate, filtered and concentrated under reduced pressure. The residue was purified by reverse phase preparative HPLC (method 3) yielding 33.0 mg (96 % purity, 29 % yield) of the desired product.
LC-MS (method 1): Rₜ = 1.05 min; MS (ESlpos): m/z = 433 [M+H]⁺
¹H-NMR (400 MHz, DMSO-d6) δ [ppm]: 2.327 (0.48), 2.648 (0.64), 2.717 (16.00), 2.795 (13.26), 3.168 (8.12), 3.388 (5.23), 3.401 (4.78), 3.415 (4.42), 4.063 (2.59), 4.075 (4.52), 4.145 (2.21), 4.158 (3.82), 4.172 (2.05), 4.859 (1.76), 4.885 (1.81), 5.591 (1.52), 5.616 (1.59), 5.687 (1.66), 5.729 (1.90), 6.024 (1.44), 6.065 (1.69), 6.295 (1.41), 6.321 (1.46), 6.337 (1.36), 6.363 (1.19), 6.559 (1.26), 6.585 (1.32), 6.601 (1.20), 6.627 (1.06), 7.238 (2.49), 7.258 (4.84), 7.278 (5.90), 7.294 (4.17), 7.313 (5.94), 7.332 (2.82), 7.360 (2.84), 7.371 (5.47), 7.419 (3.14), 7.431 (3.03), 7.752 (6.69), 7.832 (2.35), 7.855 (2.72), 7.881 (2.02), 8.287 (2.37), 8.299 (2.32), 8.337 (2.83), 8.350 (2.73), 8.447 (2.35), 8.463 (3.91), 8.475 (2.76), 8.519 (4.57), 8.556 (3.81), 11.827 (2.64), 11.886 (3.12).

### Example 23

### N-[2-({4-[3-(3,4-dichlorophenyl)-1H-pyrrolo[3,2-b]pyridin-2-yl]pyridin-3-yl}oxy)ethyl]-N-methylprop-2-enamide

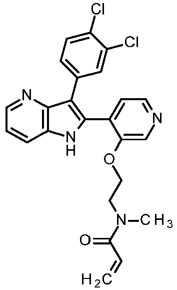

To a solution of 2-({4-[3-(3,4-dichlorophenyl)-1H-pyrrolo[3,2-b]pyridin-2-yl]pyridin-3-yl}oxy)-N-methylethanamine trifluoroacetate (1:1) (95.0 mg, 180 µmol) in DMF (1 ml), prop-2-enoic acid (12 µl, 180 µmol) and N,N-diisopropylethylamine (190 µl, 1.1 mmol) were added at rt. T3P (160 µl, 50 % purity in DMF, 270 µmol) was added and stirring at rt was continued for 16 h. Water (1 ml) was added an the mixture was extracted with EtOAc twice. The combined organic layers were dried over sodium sulfate, filtered and concentrated under reduced pressure. The residue was purified by reverse phase preparative HPLC (method 3) yielding 18.4 mg (97 % purity, 21 % yield) of the desired product.
LC-MS (method 2): Rₜ = 1.39 min; MS (ESlpos): m/z = 467 [M+H]⁺
¹H NMR (400 MHz, DMSO-d6) δ ppm 2.67 - 2.84 (m, 3 H) 3.39 - 3.52 (m, 4 H) 4.05 - 4.23 (m, 2 H) 4.83 - 4.94 (m, 1 H) 5.56 - 5.75 (m, 1 H) 5.98 - 6.10 (m, 1 H) 6.24 - 6.38 (m, 1 H) 6.50 - 6.64 (m, 1 H) 7.21 - 7.32 (m, 1 H) 7.35 - 7.48 (m, 2 H) 7.50 - 7.60 (m, 1 H) 7.80 - 7.91 (m, 1 H) 7.93 - 8.01 (m, 1 H) 8.26 - 8.40 (m, 1 H) 8.42 - 8.51 (m, 1 H) 8.52 - 8.64 (m, 1 H) 11.85 - 12.02 (m, 1 H).

### Example 24

### N-[2-({4-[3-(3-chloro-4-fluorophenyl)-1H-pyrrolo[3,2-b]pyridin-2-yl]pyridin-3-yl}oxy)ethyl]-N-methylprop-2-enamide

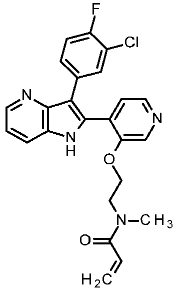

To a solution of 2-({4-[3-(3-chloro-4-fluorophenyl)-1H-pyrrolo[3,2-b]pyridin-2-yl]pyridin-3-yl}oxy)-N-methylethanamine trifluoroacetate (1:1) (96.0 mg, 188 µmol) in DMF (1 ml), prop-2-enoic acid (13 µl, 190 µmol) and N,N-diisopropylethylamine (200 µl, 1.1 mmol) were added at rt. T3P (170 µl, 50 % purity in DMF, 280 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 21.5 mg (95 % purity, 24 % yield) of the desired product as a mixture of rotamers.
LC-MS (method 1): Rₜ = 1.14 min; MS (ESlpos): m/z = 451 [M+H]⁺
¹H NMR (400 MHz, DMSO-*d*₆) δ ppm 2.67 - 2.85 (m, 3 H) 3.38 - 3.49 (m, 2 H) 4.02 - 4.25 (m, 2 H) 4.80 - 4.91 (m, 1 H) 5.54 - 5.75 (m, 1 H) 5.98 - 6.10 (m, 1 H) 6.26 - 6.39 (m, 1 H) 6.46 - 6.67 (m, 1 H) 7.19 - 7.28 (m, 1 H) 7.30 - 7.48 (m, 3 H) 7.79 - 7.96 (m, 2 H) 8.26 - 8.40 (m, 1 H) 8.42 - 8.50 (m, 1 H) 8.51 - 8.62 (m, 1 H) 11.75 - 11.96 (m, 1 H) ¹H NMR (500 MHz, DMSO-*d*₆) δ ppm 1.11 - 1.26 (m, 3 H) 1.47 - 1.99 (m, 5 H) 3.11 - 3.27 (m, 1 H) 3.75 (dd, J=9.31, 6.10 Hz, 1 H) 3.82 - 3.90 (m, 1 H) 3.92 - 4.00 (m, 1 H) 4.02 - 4.10 (m, 1 H) 4.13 - 4.24 (m, 1 H) 4.88 - 4.94 (m, 1 H) 5.61 - 5.66 (m, 1 H) 5.79 - 5.86 (m, 1 H) 6.23 - 6.32 (m, 1 H) 6.39 - 6.47 (m, 1 H) 7.16 - 7.25 (m, 2 H) 7.26 - 7.35 (m, 3 H) 7.38 - 7.42 (m, 1 H) 7.53 - 7.59 (m, 2 H) 7.80 - 7.86 (m, 1 H) 8.23 - 8.27 (m, 1 H) 8.30 - 8.33 (m, 1 H) 8.40 - 8.45 (m, 1 H) 8.46 - 8.49 (m, 1 H) 8.58 - 8.61 (m, 1 H) 11.72 - 11.80 (m, 1 H)

### Example 25

### N-methyl-N-(2-{[4-(3-phenyl-1H-pyrrolo[3,2-b]pyridin-2-yl)pyridin-3-yl]oxy}ethyl)ethenesulfonamide

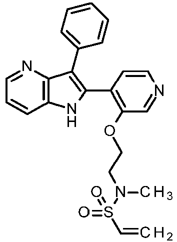

To a solution of N-methyl-2-{[4-(3-phenyl-1H-pyrrolo[3,2-b]pyridin-2-yl)pyridin-3-yl]oxy}ethan-1-amine (50.0 mg, 145 µmol) in DMF (1 ml), triethylamine (71 µl, 510 µmol) and 2-chloroethane-1-sulfonyl chloride (7.6 µl, 73 µmol) were added at rt. After stirring at rt for 16 h, water (1 ml) and saturated sodium hydrogencarbonate solution (aqueous, 2 mL) were added, and the mixture was extracted with EtOAc twice. The combined organic layers were dried over sodium sulfate, filtered and concentrated under reduced pressure. The residue was purified by reverse phase preparative HPLC (method 3) containing the desired product together with impurities. This material was further purified by silica gel column chromatography (eluent: DCM/methanol 10:1) yielding 9.60 mg (97 % purity, 15 % yield) of the title compound.
LC-MS (method 2): Rₜ = 1.04 min; MS (ESlpos): m/z = 435 [M+H]⁺
¹H-NMR (600 MHz, DMSO-d6) δ [ppm]: 2.074 (1.30), 2.512 (16.00), 3.048 (1.63), 3.058 (3.39), 3.067 (1.66), 4.082 (1.70), 4.092 (3.51), 4.101 (1.64), 5.756 (0.90), 5.891 (2.81), 5.908 (2.91), 5.942 (2.75), 5.969 (2.96), 6.553 (1.45), 6.569 (1.46), 6.580 (1.38), 6.597 (1.26), 7.203 (0.74), 7.207 (1.60), 7.214 (2.90), 7.220 (1.68), 7.228 (2.42), 7.301 (1.96), 7.314 (3.30), 7.327 (1.72), 7.367 (2.48), 7.375 (2.54), 7.529 (2.92), 7.541 (2.71), 7.820 (1.65), 7.823 (1.66), 7.834 (1.58), 7.836 (1.53), 8.290 (2.84), 8.297 (2.68), 8.412 (1.67), 8.414 (1.68), 8.420 (1.68), 8.422 (1.59), 8.518 (4.30), 11.701 (2.12).

### Example 26

### (2E)-4-(dimethylamino)-N-methyl-N-(2-{[4-(3-phenyl-1H-pyrrolo[3,2-b]pyridin-2-yl)pyridin-3-yl]oxy}ethyl)but-2-enamide

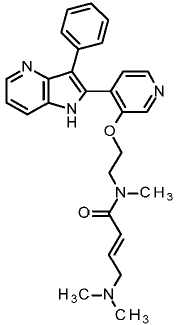

To a solution of N-methyl-2-{[4-(3-phenyl-1H-pyrrolo[3,2-b]pyridin-2-yl)pyridin-3-yl]oxy}ethan-1-amine (50.0 mg, 145 µmol) in DMF (1 ml), N,N-diisopropylethylamine (150 µl, 870 µmol) and (2E)-4-(dimethylamino)but-2-enoic acid hydrogen chloride (1/1) (36.1 mg, 218 µmol) were added at rt. T3P (130 µl, 50 % purity in DMF, 220 µmol) was added and stirring at rt was continued for 16 h. Water (1 ml) was added and the mixture was extracted with EtOAc twice. The combined organic layers were dried over sodium sulfate, filtered and concentrated under reduced pressure. The remaining residue did not contain the desired product as indicated by LCMS. Thus, the aqueous phase was concentrated and the remaining residue was purified by reverse phase preparative HPLC (method 3) yielding 2.20 mg (100 % purity, 3 % yield) of the desired product.
LC-MS (method 2): Rₜ = 0.70 min; MS (ESlneg): m/z = 454 [M-H]⁻
¹H-NMR (500 MHz, METHANOL-d4) δ [ppm]: 0.968 (0.57), 2.064 (13.98), 2.248 (16.00), 2.492 (1.65), 2.505 (1.68), 2.530 (0.47), 2.871 (0.47), 2.896 (8.13), 2.936 (9.28), 3.115 (1.56), 3.126 (1.56), 3.344 (0.47), 3.583 (0.90), 3.593 (1.72), 3.603 (0.96), 3.692 (1.09), 3.702 (2.02), 3.713 (1.16), 4.137 (1.01), 4.147 (1.83), 4.157 (0.96), 4.261 (1.20), 4.272 (2.11), 4.282 (1.12), 6.267 (0.48), 6.298 (1.28), 6.322 (0.48), 6.334 (0.91), 6.346 (0.41), 6.439 (0.79), 6.469 (1.02), 6.672 (0.83), 6.686 (0.42), 6.703 (0.64), 7.258 (1.27), 7.268 (2.32), 7.275 (2.85), 7.278 (2.39), 7.284 (2.25), 7.289 (1.42), 7.292 (1.77), 7.296 (1.42), 7.301 (1.62), 7.304 (1.67), 7.308 (0.91), 7.311 (1.19), 7.313 (1.69), 7.339 (1.19), 7.354 (2.95), 7.369 (3.16), 7.383 (1.23), 7.442 (2.16), 7.459 (3.12), 7.474 (1.65), 7.477 (1.24), 7.889 (0.93), 7.891 (0.96), 7.905 (0.90), 7.908 (0.89), 7.993 (1.03), 7.996 (1.06), 8.010 (1.00), 8.013 (0.98), 8.088 (1.15), 8.098 (1.12), 8.185 (1.01), 8.195 (0.98), 8.345 (1.09), 8.348 (1.17), 8.354 (1.14), 8.357 (1.10), 8.382 (0.95), 8.385 (0.96), 8.392 (0.98), 8.394 (0.91), 8.415 (1.90), 8.426 (1.70).

### Example 27

### N-methyl-N-(2-{[4-(3-phenyl-1H-pyrrolo[3,2-b]pyridin-2-yl)pyridin-3-yl]oxy}ethyl)prop-2-ynamide

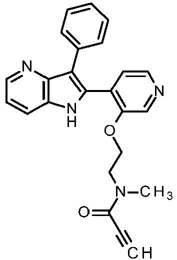

To a solution of N-methyl-2-{[4-(3-phenyl-1H-pyrrolo[3,2-b]pyridin-2-yl)pyridin-3-yl]oxy}ethan-1-amine (50.0 mg, 145 µmol) in DMF (1 ml), N,N-diisopropylethylamine (150 µl, 870 µmol) and prop-2-ynoic acid (13 µl, 220 µmol) were added at rt. T3P (130 µl, 50 % purity in DMF, 220 µmol) was added and stirring at rt was continued for 1 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 32.0 mg (100 % purity, 56 % yield) of the desired product.
LC-MS (method 2): Rₜ = 0.90 min; MS (ESlpos): m/z = 397 [M+H]⁺
¹H-NMR (400 MHz, DMSO-d6) δ [ppm]: 2.630 (8.64), 2.884 (9.78), 3.170 (16.00), 3.373 (1.40), 3.387 (2.53), 3.400 (1.49), 3.527 (0.97), 3.542 (2.01), 3.556 (1.08), 4.104 (1.39), 4.117 (2.80), 4.125 (2.79), 4.139 (1.27), 4.292 (3.36), 4.480 (3.79), 7.197 (1.87), 7.201 (1.85), 7.209 (1.85), 7.213 (2.88), 7.217 (2.84), 7.222 (2.20), 7.229 (2.09), 7.233 (2.62), 7.293 (1.75), 7.299 (2.12), 7.313 (2.95), 7.319 (3.51), 7.322 (3.40), 7.334 (3.03), 7.354 (1.65), 7.366 (1.59), 7.529 (2.55), 7.533 (3.10), 7.546 (2.35), 7.553 (2.71), 7.804 (1.05), 7.807 (1.19), 7.825 (1.98), 7.828 (2.16), 7.846 (1.12), 7.849 (1.18), 8.257 (1.98), 8.269 (1.90), 8.287 (1.76), 8.299 (1.69), 8.410 (2.11), 8.413 (2.32), 8.421 (2.29), 8.424 (2.20), 8.511 (2.88), 8.528 (2.56), 11.693 (0.61).

### Example 28

### N-methyl-N-(2-{[4-(3-phenyl-1H-pyrrolo[3,2-b]pyridin-2-yl)pyridin-3-yl]oxy}ethyl)but-2-ynamide

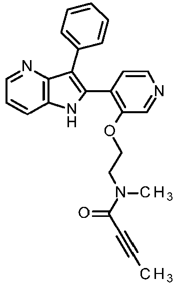

To a solution of N-methyl-2-{[4-(3-phenyl-1H-pyrrolo[3,2-b]pyridin-2-yl)pyridin-3-yl]oxy}ethan-1-amine (50.0 mg, 145 µmol) in DMF (1 ml), T3P (130 µl, 50 % purity in DMF, 220 µmol) and but-2-ynoic acid (19 µl, 220 µmol) were added at rt. N,N-diisopropylethylamine (150 µl, 870 µmol) was added and stirring at rt was continued for 1 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 14.0 mg (100 % purity, 23 % yield) of the desired product.
LC-MS (method 2): Rₜ = 0.97 min; MS (ESlpos): m/z = 411 [M+H]⁺
¹H-NMR (400 MHz, DMSO-d6) δ [ppm]: 1.157 (0.96), 1.175 (1.98), 1.192 (1.02), 1.759 (15.05), 1.948 (15.86), 1.989 (3.84), 2.627 (14.58), 2.857 (16.00), 3.169 (5.78), 3.357 (2.19), 3.371 (3.79), 3.384 (2.02), 3.513 (1.54), 3.528 (3.28), 3.542 (1.72), 4.020 (0.95), 4.038 (0.90), 4.107 (4.39), 4.114 (4.29), 7.199 (2.36), 7.211 (3.24), 7.219 (5.31), 7.231 (3.67), 7.240 (2.71), 7.300 (3.21), 7.316 (7.81), 7.328 (3.83), 7.334 (2.92), 7.339 (2.77), 7.344 (3.09), 7.356 (2.64), 7.532 (4.80), 7.546 (3.69), 7.551 (4.03), 7.801 (1.72), 7.804 (1.87), 7.821 (1.74), 7.824 (1.83), 7.830 (1.97), 7.834 (1.99), 7.851 (1.76), 7.854 (1.72), 8.250 (2.88), 8.262 (2.82), 8.280 (2.79), 8.292 (2.61), 8.410 (3.62), 8.421 (3.58), 8.507 (4.44), 8.541 (4.15), 11.663 (2.47), 11.692 (2.34).

### Example 29

### N-[2-({4-[3-(3-chlorophenyl)-1H-pyrrolo[3,2-b]pyridin-2-yl]pyridin-3-yl}oxy)ethyl]-N-methylethenesulfonamide

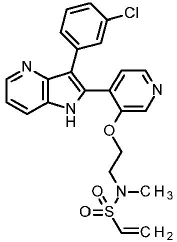

To a solution of 2-({4-[3-(3-chlorophenyl)-1H-pyrrolo[3,2-b]pyridin-2-yl]pyridin-3-yl}oxy)-N-methylethanamine hydrochloride (1:1) (164 mg, 84 % purity, 332 µmol) in DMF (3 ml), N,N'-diisopropylethylendiamine (180 µl, 1.3 mmol; CAS-RN:[121-44-8]) and 2-chloroethane-1-sulfonyl chloride (35 µl, 330 µmol) were added at rt. After stirring at rt for 16 h, water (1 ml) and saturated sodium hydrogencarbonate solution (aqueous, 2 mL) were added, and the mixture was extracted with EtOAc twice. The combined organic layers were dried over sodium sulfate, filtered and concentrated under reduced pressure. The residue was purified by reverse phase preparative HPLC (method 3) containing the desired product together with impurities. This material was further purified by silica gel column chromatography (eluent: DCM/methanol 10:1) yielding 22.0 mg (100 % purity, 14 % yield) of the title compound.
LC-MS (method 1): Rₜ = 1.21 min; MS (ESlpos): m/z = 469 [M+H]⁺
¹H-NMR (400 MHz, DMSO-d6) δ [ppm]: 3.036 (4.51), 3.050 (10.13), 3.064 (4.79), 3.269 (0.44), 3.303 (1.25), 3.308 (1.39), 3.381 (1.32), 3.390 (1.47), 3.414 (0.48), 3.430 (0.50), 4.092 (4.87), 4.107 (10.12), 4.121 (4.62), 5.885 (9.12), 5.910 (9.84), 5.935 (8.96), 5.976 (10.12), 6.525 (5.16), 6.550 (5.10), 6.566 (4.84), 6.591 (4.19), 7.234 (4.88), 7.246 (4.82), 7.255 (6.23), 7.266 (5.53), 7.273 (4.18), 7.278 (6.66), 7.283 (4.31), 7.290 (4.62), 7.309 (7.70), 7.328 (3.73), 7.344 (4.06), 7.348 (6.90), 7.352 (3.75), 7.363 (2.03), 7.367 (3.16), 7.428 (8.12), 7.440 (8.30), 7.759 (7.95), 7.764 (4.69), 7.841 (5.39), 7.845 (5.51), 7.862 (5.13), 7.865 (4.83), 8.339 (9.13), 8.352 (8.68), 8.452 (5.52), 8.456 (5.52), 8.463 (5.55), 8.467 (5.05), 8.547 (13.18), 11.866 (6.68).

### Example 30

### N-[2-({4-[3-(1H-indol-6-yl)-1H-pyrrolo[3,2-b]pyridin-2-yl]pyridin-3-yl}oxy)ethyl]-N-methylethenesulfonamide

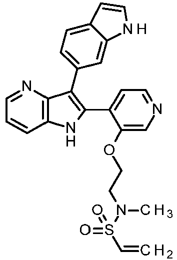

To a solution of 2-({4-[3-(1H-indol-6-yl)-1H-pyrrolo[3,2-b]pyridin-2-yl]pyridin-3-yl}oxy)-N-methylethanamine hydrochloride (1:1) (151 mg, 74 % purity, 266 µmol) in DMF (3 ml), N,N'-diisopropylethylendiamin (190 µl, 1.1 mmol; CAS-RN:[4013-94-9]) and 2-chloroethane-1-sulfonyl chloride (28 µl, 270 µmol) were added at rt. After stirring at rt for 16 h, water (1 ml) and saturated sodium hydrogencarbonate solution (aqueous, 2 mL) were added, and the mixture was extracted with EtOAc twice. The combined organic layers were dried over sodium sulfate, filtered, and concentrated under reduced pressure. The residue was purified by reverse phase preparative HPLC (method 3) yielding 4.60 mg (84 % purity, 3 % yield) of the title compound.
LC-MS (method 1): Rₜ = 0.99 min; MS (ESlpos): m/z = 474 [M+H]⁺
¹H-NMR (400 MHz, DMSO-d6) δ [ppm]: 11.54 (br s, 1H), 11.01 (br s, 1H), 8.61-8.46 (m, 2H), 8.44-8.31 (m, 2H), 8.30-8.20 (m, 1H), 7.88-7.88 (m, 1H), 7.86-7.74 (m, 3H), 7.46-7.38 (m, 2H), 7.37-7.28 (m, 3H), 7.25-7.14 (m, 2H), 7.08-6.98 (m, 1H), 6.57-6.45 (m, 1H), 6.42-6.33 (m, 1H), 5.97-5.84 (m, 2H), 4.16-4.03 (m, 3H), 3.05-2.96 (m, 2H), 2.46 (s, 3H).

### Example 31

### N-[2-({4-[3-(3,5-dichlorophenyl)-1H-pyrrolo[3,2-b]pyridin-2-yl]pyridin-3-yl}oxy)ethyl]-N-methylprop-2-enamide

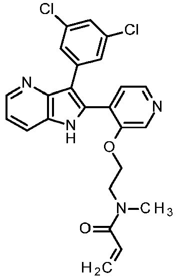

A solution of N-(2-{[4-(3-bromo-1H-pyrrolo[3,2-b]pyridin-2-yl)pyridin-3-yl]oxy}ethyl)-N-methylprop-2-enamide (30.0 mg, 74.8 µmol) and (3,5-dichlorophenyl)boronic acid (21.4 mg, 112 µmol) in a mixture of 1-propanole and water (5:1,1 ml) was carefully degassed and purged with argon. Triphenylphosphine (1.96 mg, 7.48 µmol), potassium carbonate (31.0 mg, 224 µmol) and the palladium catalyst bis(triphenylphosphine)palladium(ll) dichloride (5.25 mg, 7.48 µmol; CAS-RN:[13965-03-2]) were added. The mixture was heated to 100°C and stirred for 1 h. After cooling to rt, water (5 ml) was added, and the mixture was extracted with EtOAc twice. The combined organic layers were dried over sodium sulfate, filtered, and concentrated under reduced pressure. The residue was purified by reverse phase preparative HPLC (method 3) yielding 7.40 mg (100 % purity, 21 % yield) of the desired product.
LC-MS (method 2): Rₜ = 1.49 min; MS (ESlpos): m/z = 467 [M+H]⁺
¹H-NMR (400 MHz, DMSO-d6) δ [ppm]: 12.03 (br s, 1H), 8.64-8.54 (m, 1H), 8.53-8.45 (m, 1H), 8.43-8.30 (m, 1H), 7.94-7.82 (m, 1H), 7.66-7.57 (m, 2H), 7.52-7.37 (m, 2H), 7.33-7.22 (m, 1H), 6.64-6.50 (m, 1H), 6.37-6.23 (m, 1H), 6.11-5.97 (m, 1H), 5.76-5.54 (m, 1H), 4.91-4.81 (m, 1H), 4.23-4.07 (m, 2H), 3.50-3.40 (m, 2H), 2.81-2.69 (m, 3H).

### Example 32

### N-ethyl-N-(2-{[4-(3-phenyl-1H-pyrrolo[3,2-b]pyridin-2-yl)pyridin-3-yl]oxy}ethyl)prop-2-enamide

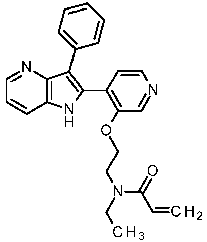

To a solution of N-ethyl-2-{[4-(3-phenyl-1H-pyrrolo[3,2-b]pyridin-2-yl)pyridin-3-yl]oxy}ethanamine hydrochloride (1:1) (70.0 mg, 177 µmol) in DMF (1 ml), prop-2-enoic acid (9.7 µl, 140 µmol) and N,N-diisopropylethylamine (120 µl, 710 µmol) were added at rt. T3P (160 µl, 50 % purity in DMF, 270 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 20.4 mg (100 % purity, 28 % yield) of the desired product.
LC-MS (method 1): Rₜ = 0.93 min; MS (ESlpos): m/z = 413 [M+H]⁺
¹H-NMR (400 MHz, DMSO-d6) δ [ppm]: 11.80-11.66 (m, 1H), 8.52-8.45 (m, 1H), 8.45-8.40 (m, 1H), 8.34-8.30 (m, 1H), 8.28-8.25 (m, 1H), 7.88-7.80 (m, 1H), 7.58-7.52 (m, 2H), 7.43-7.29 (m, 3H), 7.26-7.19 (m, 2H), 6.63-6.55 (m, 1H), 6.40-6.33 (m, 1H), 6.14-6.07 (m, 1H), 5.82-5.76 (m, 1H), 5.65-5.60 (m, 1H), 4.98-4.94 (m, 1H), 4.13-3.97 (m, 2H), 3.32-3.28 (m, 2H), 3.18-3.11 (m, 2H), 0.88-0.79 (m, 3H).

### Example 33

### N-ethyl-N-(2-{[4-(3-phenyl-1H-pyrrolo[3,2-b]pyridin-2-yl)pyridin-3-yl]oxy}ethyl)ethenesulfonamide

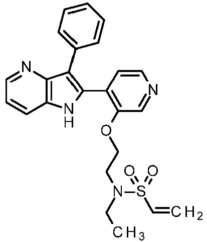

To a solution of N-ethyl-2-{[4-(3-phenyl-1H-pyrrolo[3,2-b]pyridin-2-yl)pyridin-3-yl]oxy}ethanamine hydrochloride (1:1) (96.0 mg, 95 % purity, 231 µmol) in DMF (2 ml), N,N'-diisopropylethylendiamine (130 µl, 920 µmol) and 2-chloroethane-1-sulfonyl chloride (22 µl, 210 µmol) were added at rt. After stirring at rt for 16 h, water (1 ml) and saturated sodium hydrogencarbonate solution (aqueous, 2 mL) were added, and the mixture was extracted with EtOAc twice. The combined organic layers were dried over sodium sulfate, filtered, and concentrated under reduced pressure. The residue was purified by reverse phase preparative HPLC (method 3) yielding 21.0 mg (100 % purity, 20 % yield) of the title compound.
LC-MS (method 1): Rₜ = 1.05 min; MS (ESlpos): m/z = 449 [M+H]⁺
¹H-NMR (400 MHz, DMSO-d6) δ [ppm]: 11.81-11.63 (m, 1H), 8.53-8.46 (m, 1H), 8.45-8.38 (m, 1H), 8.32-8.27 (m, 1H), 7.85-7.80 (m, 1H), 7.56-7.52 (m, 2H), 7.40-7.37 (m, 1H), 7.33-7.29 (m, 2H), 7.24-7.19 (m, 2H), 6.61-6.56 (m, 1H), 5.96-5.91 (m, 1H), 5.84-5.80 (m, 1H), 4.09-4.02 (m, 2H), 3.10-3.05 (m, 2H), 2.94-2.86 (m, 2H), 0.81-0.74 (m, 3H).

### Example 34

### 1-[(2S)-2-({[4-(3-phenyl-1H-pyrrolo[3,2-b]pyridin-2-yl)pyridin-3-yl]oxy}methyl)pyrrolidin-1-yl]prop-2-en-1-one

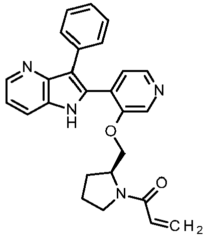

To a solution of 3-phenyl-2-{3-[(2S)-pyrrolidin-2-ylmethoxy]pyridin-4-yl}-1H-pyrrolo[3,2-b]pyridine hydrochloride (1:1) (150 mg, 369 µmol) in DMF (1 ml), prop-2-enoic acid (25 µl, 370 µmol) and N,N-diisopropylethylamine (260 µl, 1.5 mmol) were added at rt. T3P (330 µl, 50 % purity in DMF, 550 µmol) was added and stirring at rt was continued for 16 h. Water (1 ml) was added and the mixture was extracted with EtOAc twice. The combined layers were dried over sodium sulfate, filtered and concentrated under reduced pressure. Additionally, the aqueous layer was extracted three times with DCM. Combined DCM layers were dried over a water repellent filter and concentrated under reduced pressure. The combined residues were purified by reverse phase preparative HPLC (method 3) yielding 57.5 mg (94 % purity, 35 % yield) of the desired product as a mixture of rotamers.
LC-MS (method 1): Rₜ = 0.95 min; MS (ESlpos): m/z = 425 [M+H]⁺
¹H NMR (500 MHz, DMSO-*d*₆) δ ppm 1.11 - 1.26 (m, 3 H) 1.47 - 1.99 (m, 5 H) 3.11 - 3.27 (m, 1 H) 3.75 (dd, J=9.31, 6.10 Hz, 1 H) 3.82 - 3.90 (m, 1 H) 3.92 - 4.00 (m, 1 H) 4.02 - 4.10 (m, 1 H) 4.13 - 4.24 (m, 1 H) 4.88 - 4.94 (m, 1 H) 5.61 - 5.66 (m, 1 H) 5.79 - 5.86 (m, 1 H) 6.23 - 6.32 (m, 1 H) 6.39 - 6.47 (m, 1 H) 7.16 - 7.25 (m, 2 H) 7.26 - 7.35 (m, 3 H) 7.38 - 7.42 (m, 1 H) 7.53 - 7.59 (m, 2 H) 7.80 - 7.86 (m, 1 H) 8.23 - 8.27 (m, 1 H) 8.30 - 8.33 (m, 1 H) 8.40 - 8.45 (m, 1 H) 8.46 - 8.49 (m, 1 H) 8.58 - 8.61 (m, 1 H) 11.72 - 11.80 (m, 1 H)

### Example 35

### 1-[(2R)-2-({[4-(3-phenyl-1H-pyrrolo[3,2-b]pyridin-2-yl)pyridin-3-yl]oxy}methyl)pyrrolidin-1-yl]prop-2-en-1-one

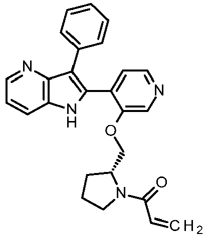

To a solution of 3-phenyl-2-{3-[(2R)-pyrrolidin-2-ylmethoxy]pyridin-4-yl}-1H-pyrrolo[3,2-b]pyridine hydrochloride (1:1) (70.0 mg, 172 µmol) in DMF (1 ml), prop-2-enoic acid (11 µl, 150 µmol) and N,N-diisopropylethylamine (120 µl, 690 µmol) were added at rt. T3P (150 µl, 50 % purity in DMF, 260 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 30.1 mg (100 % purity, 41 % yield) of the desired product as a mixture of rotamers.
LC-MS (method 2): Rₜ = 1.04 min; MS (ESlpos): m/z = 425 [M+H]⁺
¹H-NMR (400 MHz, DMSO-d6) δ [ppm]: 11.83-11.68 (m, 1H), 8.59 (s, 2H), 8.35-8.17 (m, 1H), 7.88-7.74 (m, 1H), 7.62-7.50 (m, 2H), 7.44-7.13 (m, 5H), 6.51-6.22 (m, 1H), 6.16-5.78 (m, 1H), 5.67-4.86 (m, 1H), 4.26-3.69 (m, 3H), 3.27-3.12 (m, 1H), 1.81-1.44 (m, 4H).

### Example 36

### 2-(3-{[(2S)-1-(ethenesulfonyl)pyrrolidin-2-yl]methoxy}pyridin-4-yl)-3-phenyl-1H-pyrrolo[3,2-b]pyridine

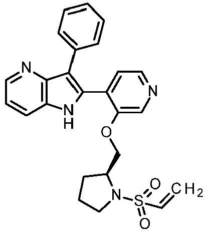

To a solution of 3-phenyl-2-{3-[(2S)-pyrrolidin-2-ylmethoxy]pyridin-4-yl}-1H-pyrrolo[3,2-b]pyridine hydrochloride (1:1) (100 mg, 246 µmol) and triethylamine (150 µl, 1.1 mmol) in DCM (2 ml), 2-chloroethane-1-sulfonyl chloride (31 µl, 290 µmol) was added at 0°C. After stirring at rt for 16 h, water (0.5 ml) was added. The reaction mixture was concentrated under reduced pressure. The residue was purified by reverse phase preparative HPLC (method 3) yielding 8.50 mg (100 % purity, 8 % yield) of the desired product.
LC-MS (method 2): Rₜ = 1.13 min; MS (ESlpos): m/z = 461 [M+H]⁺
¹H-NMR (400 MHz, DMSO-d6) δ [ppm]: 11.72 (s br, 1H), 8.57-8.48 (m, 1H), 8.47-8.37 (m, 1H), 8.33-8.26 (m, 1H), 7.88-7.75 (m, 1H), 7.62-7.49 (m, 2H), 7.42-7.34 (m, 1H), 7.33-7.14 (m, 5H), 6.85-6.72 (m, 1H), 6.11-6.00 (m, 2H), 4.12-3.86 (m, 2H), 3.64-3.53 (m, 1H), 3.03-2.92 (m, 2H), 1.63-1.40 (m, 4H).

### Example 37

### (2E)-4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one

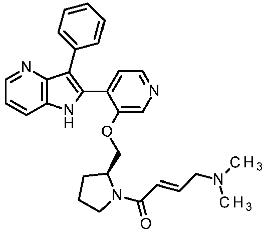

To a solution of 3-phenyl-2-{3-[(2S)-pyrrolidin-2-ylmethoxy]pyridin-4-yl}-1H-pyrrolo[3,2-b]pyridine hydrochloride (1:1) (74.0 mg, 182 µmol) in DMF (1 ml), (2E)-4-(dimethylamino)but-2-enoic acid hydrochloride (1:1) (30.1 mg, 182 µmol) and N,N-diisopropylethylamine (130 µl, 730 µmol) were added at rt. T3P (160 µl, 50 % purity in DMF, 270 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 5.30 mg (100 % purity, 6 % yield) of the desired product.
LC-MS (method 2): Rₜ = 0.73 min; MS (ESlpos): m/z = 480 [M+H]⁺
¹H-NMR (400 MHz, DMSO-d6) δ [ppm]: 11.80-11.70 (m, 1H), 8.61-8.56 (m, 1H), 8.50-8.39 (m, 1H), 8.34-8.22 (m, 1H), 7.86-7.77 (m, 1H), 7.59-7.49 (m, 2H), 7.46-7.12 (m, 6H), 6.66-6.40 (m, 1H), 6.25-6.10 (m, 1H), 4.26-3.95 (m, 3H), 3.75-3.64 (m, 1H), 3.24-3.12 (m, 2H), 3.02-2.92 (m, 2H), 2.64-2.57 (m, 1H), 2.15-1.93 (m, 6H), 1.73-1.46 (m, 5H).

### Example 38

### 2-(3-{[(2S)-1-(ethenesulfonyl)pyrrolidin-2-yl]methoxy}pyridin-4-yl)-3-[3-(trifluoromethyl)phenyl]-1H-pyrrolo[3,2-b]pyridine

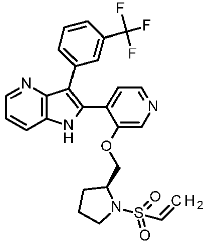

To a solution of 2-{3-[(2S)-pyrrolidin-2-ylmethoxy]pyridin-4-yl}-3-[3-(trifluoromethyl)phenyl]-1H-pyrrolo[3,2-b]pyridine hydrochloride (1:1) (100 mg, 211 µmol) and triethylamine (130 µl, 950 µmol) in DCM (2 ml), 2-chloroethane-1-sulfonyl chloride (26 µl, 250 µmol) was added at 0°C. After stirring at rt for 16 h, water (0.5 ml) was added. The reaction mixture was concentrated under reduced pressure. The residue was purified by reverse phase preparative HPLC (method 3) yielding 20.6 mg (100 % purity, 19 % yield) of the desired product.
LC-MS (method 1): Rₜ = 1.50 min; MS (ESlpos): m/z = 529 [M+H]⁺
¹H-NMR (400 MHz, DMSO-d6) δ [ppm]: 11.94 (br s, 1H), 8.58-8.51 (m, 1H), 8.51-8.45 (m, 1H), 8.40-8.34 (m, 1H), 8.10-8.01 (m, 1H), 7.92-7.73 (m, 2H), 7.60-7.44 (m, 3H), 7.31-7.19 (m, 1H), 6.84-6.67 (m, 1H), 6.11-5.95 (m, 2H), 4.09-3.96 (m, 1H), 3.94-3.84 (m, 1H), 3.56-3.41 (m, 1H), 3.01-2.87 (m, 2H), 1.57-1.36 (m, 3H), 1.34-1.18 (m, 1H).

### Example 39

### 1-[(2S)-2-{[(4-{3-[3-(trifluoromethyl)phenyl]-1H-pyrrolo[3,2-b]pyridin-2-yl}pyridin-3-yl)oxy]methyl}pyrrolidin-1-yl]prop-2-en-1-one

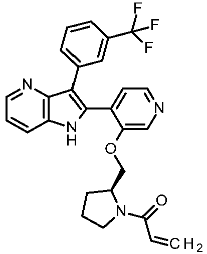

To a solution of 2-{3-[(2S)-pyrrolidin-2-ylmethoxy]pyridin-4-yl}-3-[3-(trifluoromethyl)phenyl]-1H-pyrrolo[3,2-b]pyridine hydrochloride (1:1) (75.0 mg, 158 µmol) in DMF (1 ml), N,N-diisopropylethylamine (110 µl, 630 µmol) and prop-2-enoic acid (11 µl, 160 µmol) were added at rt. T3P (140 µl, 50 % purity in DMF, 240 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 34.1 mg (100 % purity, 44 % yield) of the desired product as a mixture of rotamers.
LC-MS (method 1): Rₜ = 1.37 min; MS (ESlpos): m/z = 493 [M+H]⁺
¹H-NMR (400 MHz, DMSO-d6) δ [ppm]: 12.07-11.89 (m, 1H), 8.70-8.57 (m, 1H), 8.54-8.27 (m, 2H), 8.10-7.95 (m, 1H), 7.93-7.74 (m, 2H), 7.61-7.39 (m, 3H), 7.35-7.20 (m, 1H), 6.47-6.15 (m, 1H), 6.13-5.77 (m, 1H), 5.65-4.88 (m, 1H), 4.23-3.64 (m, 3H), 3.23-3.07 (m, 1H), 1.79-1.40 (m, 4H), 1.37-0.87 (m, 2H).

### Example 40

### 3-(3-chlorophenyl)-2-(3-{[(2S)-1-(ethenesulfonyl)pyrrolidin-2-yl]methoxy}pyridin-4-yl)-1H-pyrrolo[3,2-b]pyridine

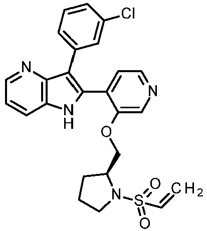

To a solution of 3-(3-chlorophenyl)-2-{3-[(2S)-pyrrolidin-2-ylmethoxy]pyridin-4-yl}-1H-pyrrolo[3,2-b]pyridine hydrochloride (1:1) (100 mg, 227 µmol) and triethylamine (140 µl, 1.0 mmol) in DCM (2 ml), 2-chloroethane-1-sulfonyl chloride (28 µl, 270 µmol) was added at 0°C. After stirring at rt for 16 h, water (0.5 ml) was added. The reaction mixture was concentrated under reduced pressure. The residue was purified by reverse phase preparative HPLC (method 3) yielding 20.9 mg (90 % purity, 17 % yield) of the desired product.
LC-MS (method 1): Rₜ = 1.33 min; MS (ESlpos): m/z = 495 [M+H]⁺
¹H-NMR (400 MHz, DMSO-d6) δ [ppm]: 11.87 (br s, 1H), 8.54 (s, 1H), 8.49-8.44 (m, 1H), 8.40-8.31 (m, 1H), 7.88-7.79 (m, 2H), 7.52-7.42 (m, 1H), 7.37-7.19 (m, 4H), 6.85-6.72 (m, 1H), 6.12-5.99 (m, 2H), 4.09-3.86 (m, 2H), 3.58-3.47 (m, 1H), 3.03-2.90 (m, 2H), 1.65-1.33 (m, 4H).

### Example 41

### 1-{(2S)-2-[({4-[3-(3-chlorophenyl)-1H-pyrrolo[3,2-b]pyridin-2-yl]pyridin-3-yl}oxy)methyl]pyrrolidin-1-yl}prop-2-en-1-one

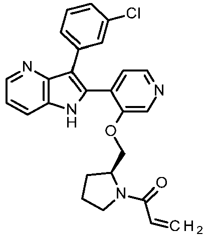

To a solution of 3-(3-chlorophenyl)-2-{3-[(2S)-pyrrolidin-2-ylmethoxy]pyridin-4-yl}-1H-pyrrolo[3,2-b]pyridine hydrochloride (1:1) (75.0 mg, 170 µmol) in DMF (1 ml), N,N-diisopropylethylamine (120 µl, 680 µmol) and prop-2-enoic acid (12 µl, 170 µmol) were added at rt. T3P (150 µl, 50 % purity in DMF, 250 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 34.5 mg (100 % purity, 44 % yield) of the desired product as a mixture of rotamers.
LC-MS (method 1): Rₜ = 1.21 min; MS (ESlpos): m/z = 459 [M+H]⁺
¹H-NMR (400 MHz, DMSO-d6) δ [ppm]: 11.97-11.83 (m, 1H), 8.70-8.57 (m, 1H), 8.55-8.42 (m, 1H), 8.39-8.27 (m, 1H), 7.91-7.72 (m, 2H), 7.48-7.16 (m, 5H), 6.48-6.20 (m, 1H), 6.14-5.79 (m, 1H), 5.65-4.90 (m, 1H), 4.26-3.74 (m, 3H), 3.25-3.09 (m, 2H), 1.80-1.37 (m, 5H), 1.03-0.80 (m, 1H).

### Example 42

### 1-{(2S)-2-[({4-[3-(5-chloro-2-fluorophenyl)-1H-pyrrolo[3,2-b]pyridin-2-yl]pyridin-3-yl}oxy)methyl]pyrrolidin-1-yl}prop-2-en-1-one

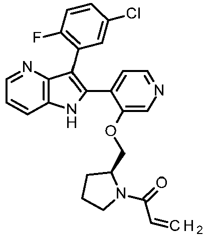

To a solution of 3-(5-chloro-2-fluorophenyl)-2-{3-[(2S)-pyrrolidin-2-ylmethoxy]pyridin-4-yl}-1H-pyrrolo[3,2-b]pyridine hydrochloride (1:1) (46.0 mg, 100 µmol) in DMF (1 ml), N,N-diisopropylethylamine (70 µl, 400 µmol) and prop-2-enoic acid (6.9 µl, 100 µmol) were added at rt. T3P (89 µl, 50 % purity in DMF, 150 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 23.0 mg (90 % purity, 43 % yield) of the desired product as a mixture of rotamers.
LC-MS (method 2): Rₜ = 1.28 min; MS (ESlpos): m/z = 477 [M+H]⁺
¹H-NMR (400 MHz, DMSO-d6) δ [ppm]: 12.05-11.97 (m, 1H), 8.62-8.53 (m, 1H), 8.51-8.40 (m, 2H), 8.33-8.20 (m, 1H), 7.94-7.85 (m, 1H), 7.78-7.70 (m, 1H), 7.56-7.31 (m, 3H), 7.29-7.16 (m, 3H), 6.54-6.26 (m, 1H), 6.18-5.86 (m, 1H), 5.69-5.02 (m, 1H), 4.25-3.93 (m, 4H), 3.91-3.75 (m, 1H), 3.27-3.18 (m, 1H), 1.86-1.43 (m, 8H).

### Example 43

### N-[2-({4-[3-(5-chloro-2-fluorophenyl)-1H-pyrrolo[3,2-b]pyridin-2-yl]pyridin-3-yl}oxy)ethyl]-N-methylprop-2-enamide

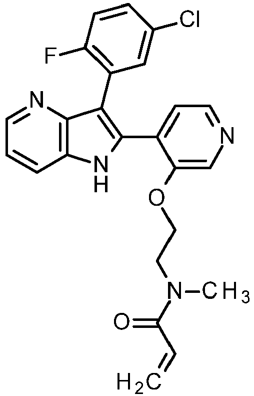

To a solution of 2-({4-[3-(5-chloro-2-fluorophenyl)-1H-pyrrolo[3,2-b]pyridin-2-yl]pyridin-3-yl}oxy)-N-methylethan-1-amine (36.0 mg, 90.7 µmol) in THF (1 ml), prop-2-enoic acid (6.2 µl, 91 µmol) and N,N-diisopropylethylamine (47 µl, 270 µmol) were added at rt. T3P (80 µl, 50 % purity in EtOAc, 140 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 16.0 mg (100 % purity, 39 % yield) of the desired product.
LC-MS (method 2): Rₜ = 1.14 min; MS (ESlpos): m/z = 451 [M+H]⁺
¹H-NMR (400 MHz, DMSO-d6) δ [ppm]: 2.365 (0.60), 2.709 (0.65), 2.762 (14.44), 2.904 (16.00), 3.159 (2.96), 3.172 (3.06), 3.224 (0.65), 3.241 (0.83), 3.284 (1.60), 3.294 (2.03), 3.309 (3.24), 3.320 (2.53), 3.329 (5.21), 3.409 (4.47), 3.416 (3.55), 3.431 (1.64), 3.441 (1.14), 3.463 (1.23), 3.503 (2.48), 3.516 (3.73), 3.529 (2.69), 3.539 (2.64), 3.553 (4.48), 3.566 (2.32), 4.108 (2.35), 4.118 (3.86), 4.132 (2.00), 4.188 (2.28), 4.202 (4.13), 4.215 (2.11), 4.984 (1.45), 4.990 (1.44), 5.016 (1.63), 5.619 (1.65), 5.625 (1.66), 5.646 (1.68), 5.651 (1.84), 5.746 (3.80), 5.789 (1.55), 5.794 (1.62), 6.064 (1.48), 6.070 (1.60), 6.106 (1.79), 6.111 (1.83), 6.391 (1.21), 6.417 (1.33), 6.433 (1.25), 6.459 (1.12), 6.625 (1.56), 6.652 (1.59), 6.667 (1.46), 6.693 (1.33), 7.174 (1.09), 7.198 (3.26), 7.221 (3.78), 7.242 (6.04), 7.253 (5.18), 7.263 (4.61), 7.274 (5.29), 7.290 (2.60), 7.384 (1.32), 7.395 (1.92), 7.406 (2.34), 7.413 (1.99), 7.424 (1.20), 7.707 (1.52), 7.713 (1.76), 7.728 (2.72), 7.741 (1.68), 7.860 (2.05), 7.880 (1.95), 7.933 (2.30), 7.953 (2.06), 8.205 (2.12), 8.217 (2.16), 8.255 (2.00), 8.266 (1.99), 8.412 (2.46), 8.423 (4.25), 8.434 (2.32), 8.488 (3.23), 8.524 (3.51), 11.861 (2.89), 11.963 (2.69).

### Example 44

### N-[2-({4-[3-(1-benzothiophen-6-yl)-1H-pyrrolo[3,2-b]pyridin-2-yl]pyridin-3-yl}oxy)ethyl]-N-methylprop-2-enamide

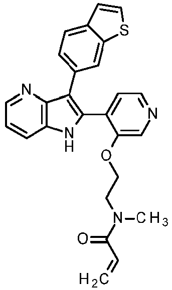

To a solution of 2-({4-[3-(1-benzothiophen-6-yl)-1H-pyrrolo[3,2-b]pyridin-2-yl]pyridin-3-yl}oxy)-N-methylethan-1-amine (59.0 mg, 147 µmol) in THF (1 ml), prop-2-enoic acid (10 µl, 150 µmol) and N,N-diisopropylethylamine (77 µl, 440 µmol) were added at rt. T3P (130 µl, 50 % purity in EtOAc, 220 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 44.0 mg (98 % purity, 64 % yield) of the desired product.
LC-MS (method 1): Rₜ = 1.02 min; MS (ESlpos): m/z = 455 [M+H]⁺
¹H-NMR (400 MHz, DMSO-d6) δ [ppm]: 11.79 and 11.71 (s, 1H), 8.56-8.48 (m, 1H), 8.48-8.43 (m, 1H), 8.34-8.21 (m, 2H), 7.92-7.82 (m, 1H), 7.81-7.68 (m, 2H), 7.46-7.29 (m, 3H), 7.27-7.22 (m, 1H), 6.64-6.25 (m, 1H), 6.09-5.69 (m, 1H), 5.63-5.58 and 4.95-4.89 (m, 1H), 4.19-3.97 (m, 3H), 3.42-3.37 (m, 1H), 3.31-3.27 (m, 1H), 3.19-3.15 (m, 1H), 2.77 and 2.66 (s, 3H)

### Example 45

### N-[2-({4-[3-(3-ethylphenyl)-1H-pyrrolo[3,2-b]pyridin-2-yl]pyridin-3-yl}oxy)ethyl]-N-methylprop-2-enamide

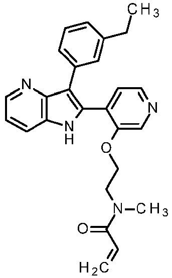

To a solution of 2-({4-[3-(3-ethylphenyl)-1H-pyrrolo[3,2-b]pyridin-2-yl]pyridin-3-yl}oxy)-N-methylethan-1-amine (50.0 mg, 134 µmol) in THF (1 ml), prop-2-enoic acid (9.2 µl, 130 µmol) and N,N-diisopropylethylamine (70 µl, 400 µmol) were added at rt. T3P (120 µl, 50 % purity in EtOAc, 200 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 30.5 mg (100 % purity, 53 % yield) of the desired product.
LC-MS (method 1): Rₜ = 1.11 min; MS (ESlpos): m/z = 427 [M+H]⁺
¹H-NMR (400 MHz, DMSO-d6) δ [ppm]: 11.73 and 11.65 (2s, 1H), 8.54-8.39 (m, 2H), 8.34-8.21 (m, 1H), 7.89-7.79 (m, 1H), 7.47-7.29 (m, 3H), 7.27-7.18 (m, 2H), 7.09-7.03 (m, 1H), 6.65-6.32 (m, 1H), 6.10-5.71 (m, 1H), 5.63-5.60 and 4.91-4.87 (2m, 1H), 4.16-4.10 (m, 1H), 4.03-3.98 (m, 1H), 3.45-3.39 (m, 1H), 2.82 and 2.72 (2s, 3H), 2.54-2.50 (m, 2H), 1.11-1.02 (m, 3H)

### Example 46

### N-[2-({4-[3-(2,5-difluorophenyl)-1H-pyrrolo[3,2-b]pyridin-2-yl]pyridin-3-yl}oxy)ethyl]-N-methylprop-2-enamide

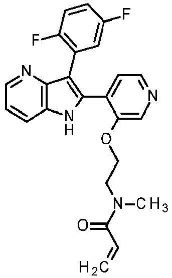

To a solution of 2-({4-[3-(2,5-difluorophenyl)-1H-pyrrolo[3,2-b]pyridin-2-yl]pyridin-3-yl}oxy)-N-methylethan-1-amine (33.0 mg, 86.8 µmol) in THF (1 ml), prop-2-enoic acid (5.9 µl, 87 µmol) and N,N-diisopropylethylamine (45 µl, 260 µmol) were added at rt. T3P (77 µl, 50 % purity in EtOAc, 130 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 12.8 mg (100 % purity, 34 % yield) of the desired product.
LC-MS (method 1): Rₜ = 0.91 min; MS (ESlpos): m/z = 435 [M+H]⁺
¹H-NMR (700 MHz, DMSO-d6) δ [ppm]: 0.921 (0.42), 0.931 (1.07), 0.949 (1.30), 1.289 (1.38), 1.360 (0.57), 1.485 (0.51), 1.522 (0.49), 2.766 (14.06), 2.913 (16.00), 3.170 (1.05), 3.365 (1.54), 3.575 (5.46), 3.582 (3.34), 4.134 (4.52), 4.207 (2.99), 4.215 (5.12), 4.998 (1.75), 5.012 (1.84), 5.631 (2.09), 5.646 (2.16), 5.761 (1.68), 5.784 (1.77), 6.083 (1.97), 6.107 (2.15), 6.417 (1.26), 6.431 (1.38), 6.440 (1.28), 6.455 (1.11), 6.646 (1.49), 6.661 (1.68), 6.670 (1.71), 6.685 (1.52), 7.191 (3.51), 7.208 (3.62), 7.214 (3.43), 7.240 (2.99), 7.247 (2.94), 7.259 (3.35), 7.266 (3.84), 7.270 (4.12), 7.277 (5.18), 7.482 (3.18), 7.882 (1.79), 7.893 (1.76), 7.956 (2.11), 7.968 (2.05), 8.215 (1.99), 8.264 (1.87), 8.417 (2.88), 8.422 (3.29), 8.430 (3.03), 8.501 (2.55), 8.537 (2.78), 11.873 (1.98), 11.983 (1.53).

### Example 47

### N-[2-({4-[3-(2-fluoro-5-methylphenyl)-1H-pyrrolo[3,2-b]pyridin-2-yl]pyridin-3-yl}oxy)ethyl]-N-methylprop-2-enamide

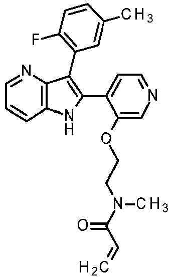

To a solution of 2-({4-[3-(2-fluoro-5-methylphenyl)-1H-pyrrolo[3,2-b]pyridin-2-yl]pyridin-3-yl}oxy)-N-methylethan-1-amine (42.0 mg, 112 µmol) in THF (1 ml), prop-2-enoic acid (7.7 µl, 110 µmol) and N,N-diisopropylethylamine (58 µl, 330 µmol) were added at rt. T3P (99 µl, 50 % purity in EtOAc, 170 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 23.7 mg (100 % purity, 49 % yield) of the desired product.
LC-MS (method 1): Rₜ = 0.91 min; MS (ESlpos): m/z = 431 [M+H]⁺
¹H-NMR (400 MHz, DMSO-d6) δ [ppm]: 2.296 (10.07), 2.311 (12.20), 2.669 (0.42), 2.771 (13.01), 2.936 (16.00), 3.160 (4.33), 3.173 (4.42), 3.271 (0.79), 3.288 (0.76), 3.309 (1.82), 3.317 (1.91), 3.332 (3.60), 3.397 (1.53), 3.424 (0.81), 3.449 (0.58), 3.463 (0.61), 3.477 (0.41), 3.537 (1.45), 3.551 (3.12), 3.564 (1.82), 3.613 (1.96), 3.626 (3.90), 3.640 (2.13), 4.113 (3.53), 4.126 (1.79), 4.218 (2.10), 4.231 (3.93), 4.244 (1.96), 5.001 (1.27), 5.007 (1.25), 5.027 (1.24), 5.033 (1.35), 5.639 (1.58), 5.644 (1.61), 5.665 (1.60), 5.670 (1.76), 5.773 (1.17), 5.779 (1.22), 5.815 (1.31), 5.820 (1.35), 6.095 (1.49), 6.101 (1.52), 6.137 (1.68), 6.143 (1.73), 6.431 (1.11), 6.457 (1.15), 6.472 (1.05), 6.498 (0.93), 6.654 (1.54), 6.680 (1.54), 6.696 (1.45), 6.722 (1.26), 6.983 (0.85), 7.004 (1.57), 7.019 (1.30), 7.029 (1.44), 7.040 (1.99), 7.064 (1.49), 7.124 (1.19), 7.144 (1.63), 7.167 (3.25), 7.178 (3.12), 7.207 (4.41), 7.219 (4.72), 7.228 (3.03), 7.240 (2.87), 7.320 (0.55), 7.339 (0.45), 7.391 (1.68), 7.413 (1.69), 7.439 (1.26), 7.767 (0.46), 7.784 (0.47), 7.834 (1.70), 7.854 (1.59), 7.937 (2.03), 7.955 (1.94), 8.028 (1.16), 8.143 (1.86), 8.155 (1.86), 8.210 (1.58), 8.222 (1.49), 8.372 (2.31), 8.383 (3.83), 8.394 (2.00), 8.467 (2.44), 8.510 (2.90), 11.678 (2.78), 11.792 (2.40).

### Example 48

### N-[2-({4-[3-(5-ethyl-2-fluorophenyl)-1H-pyrrolo[3,2-b]pyridin-2-yl]pyridin-3-yl}oxy)ethyl]-N-methylprop-2-enamide

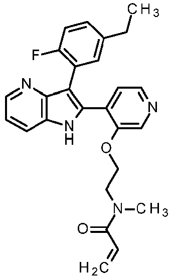

To a solution of 2-({4-[3-(5-ethyl-2-fluorophenyl)-1H-pyrrolo[3,2-b]pyridin-2-yl]pyridin-3-yl}oxy)-N-methylethan-1-amine (33.0 mg, 84.5 µmol) in THF (1 ml), prop-2-enoic acid (5.8 µl, 85 µmol) and N,N-diisopropylethylamine (44 µl, 250 µmol) were added at rt. T3P (75 µl, 50 % purity in EtOAc, 130 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 18.4 mg (100 % purity, 49 % yield) of the desired product.
LC-MS (method 1): Rₜ = 1.10 min; MS (ESlpos): m/z = 445 [M+H]⁺
¹H-NMR (400 MHz, DMSO-d6) δ [ppm]: 11.82 and 11.70 (2s, 1H), 8.63-8.07 (m, 3H), 8.02-7.80 (m, 1H), 7.46-6.98 (m, 5H), 6.77-6.37 (m, 1H), 6.21-5.74 (m, 1H), 5.70-4.97 (m, 1H), 4.28-4.00 (m, 2H), 3.68-3.44 (m, 2H), 2.93 and 2.77 (2s, 3H), 2.65-2.56 (m, 2H), 1.23-1.05 (m, 3H)

### Example 49

### N-methyl-N-{2-[(4-{3-[3-(trifluoromethyl)phenyl]-1H-pyrrolo[3,2-b]pyridin-2-yl}pyridin-3-yl)oxy]ethyl}prop-2-enamide

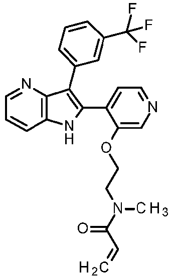

To a solution of N-methyl-2-[(4-{3-[3-(trifluoromethyl)phenyl]-1H-pyrrolo[3,2-b]pyridin-2-yl}pyridin-3-yl)oxy]ethan-1-amine (45.0 mg, 109 µmol) in THF (1 ml), prop-2-enoic acid (7.5 µl, 110 µmol) and N,N-diisopropylethylamine (57 µl, 330 µmol) were added at rt. T3P (96 µl, 50 % purity in EtOAc, 160 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 37.5 mg (99 % purity, 73 % yield) of the desired product.
LC-MS (method 1): Rₜ = 1.20 min; MS (ESlpos): m/z = 467 [M+H]⁺
¹H-NMR (400 MHz, DMSO-d6) δ [ppm]: 11.95 and 11.90 (2s, 1H), 8.58-8.45 (m, 2H), 8.39-8.28 (m, 1H), 8.00-7.94 (m, 1H), 7.91-7.79 (m, 2H), 7.60-7.50 (m, 2H), 7.48-7.37 (m, 1H), 7.30-7.23 (m, 1H), 6.61-6.25 (m, 1H), 6.07-6.00, 5.74-5.67, 5.62-5.56 and 4.91-4.83 (4m, 2H), 4.15-4.00 (m, 2H), 3.38-3.27 (m, 2H), 2.76 and 2.69 (2s, 3H)

### Example 50

### N-[2-({4-[3-(3-fluoro-5-methylphenyl)-1H-pyrrolo[3,2-b]pyridin-2-yl]pyridin-3-yl}oxy)ethyl]-N-methylprop-2-enamide

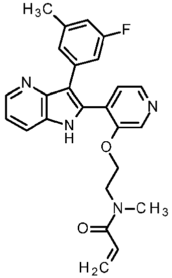

To a solution of 2-({4-[3-(3-fluoro-5-methylphenyl)-1H-pyrrolo[3,2-b]pyridin-2-yl]pyridin-3-yl}oxy)-N-methylethan-1-amine (65.0 mg, 173 µmol) in THF (1 ml), prop-2-enoic acid (12 µl, 170 µmol) and N,N-diisopropylethylamine (90 µl, 520 µmol) were added at rt. T3P (150 µl, 50 % purity in EtOAc, 260 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 52.0 mg (100 % purity, 70 % yield) of the desired product.
LC-MS (method 1): Rₜ = 1.02 min; MS (ESlpos): m/z = 431 [M+H]⁺
¹H-NMR (400 MHz, DMSO-d6) δ [ppm]: 11.83 and 11.76 (2s, 1H), 8.59-8.24 (m, 3H), 7.89-7.78 (m, 1H), 7.43-7.11 (m, 4H), 6.91-6.82 (m, 1H), 6.63-6.29 (m, 1H), 6.09-6.01, 5.74-5.68 5.64-5.58 and 4.90-4.82 (4m, 2H), 5.77-5.74 (m, 1H), 4.21-4.06 (m, 2H), 3.50-3.39 (m, 2H), 2.80 and 2.72 (2s, 3H), 2.28-2.20 (m, 3H)

### Example 51

### N-{2-[(4-{3-[2-fluoro-5-(trifluoromethyl)phenyl]-1H-pyrrolo[3,2-b]pyridin-2-yl}pyridin-3-yl)oxy]ethyl}-N-methylprop-2-enamide

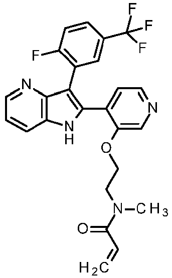

To a solution of 2-[(4-{3-[2-fluoro-5-(trifluoromethyl)phenyl]-1H-pyrrolo[3,2-b]pyridin-2-yl}pyridin-3-yl)oxy]-N-methylethan-1-amine (30.0 mg, 69.7 µmol) in THF (1 ml), prop-2-enoic acid (4.8 µl, 70 µmol) and N,N-diisopropylethylamine (36 µl, 210 µmol) were added at rt. T3P (62 µl, 50 % purity in EtOAc, 100 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 21.0 mg (100 % purity, 62 % yield) of the desired product.
LC-MS (method 1): Rₜ = 1.22 min; MS (ESlpos): m/z = 485 [M+H]⁺
¹H-NMR (400 MHz, DMSO-d6) δ [ppm]: 12.03 and 11.94 (2s, 1H), 8.55-8.47 (m, 1H), 8.46-8.41 (m, 1H), 8.30-8.21 (m, 1H), 8.12-8.03 (m, 1H), 7.98-7.87 (m, 1H), 7.78-7.71 (m, 1H), 7.47-7.37 (m, 1H), 7.35-7.24 (m, 2H), 6.68-6.36 (m, 1H), 6.10-6.05, 5.79-5.74, 5.65-5.60 and 5.01-4.96 (4m, 2H), 5.76 (s, 1H), 4.19-4.06 (m, 2H), 3.51-3.41 (m, 2H), 2.88 and 2.74 (2s, 3H)

### Example 52

### N-[2-({4-[3-(3,5-difluorophenyl)-1H-pyrrolo[3,2-b]pyridin-2-yl]pyridin-3-yl}oxy)ethyl]-N-methylprop-2-enamide

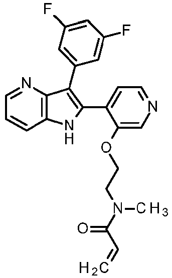

To a solution of 2-({4-[3-(3,5-difluorophenyl)-1H-pyrrolo[3,2-b]pyridin-2-yl]pyridin-3-yl}oxy)-N-methylethan-1-amine (50.0 mg, 131 µmol) in THF (1 ml), prop-2-enoic acid (9.0 µl, 130 µmol) and N,N-diisopropylethylamine (69 µl, 390 µmol) were added at rt. T3P (120 µl, 50 % purity in EtOAc, 200 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 29.8 mg (100 % purity, 52 % yield) of the desired product.
LC-MS (method 1): Rₜ = 1.08 min; MS (ESlpos): m/z = 435 [M+H]⁺
¹H-NMR (400 MHz, DMSO-d6) δ [ppm]: 12.75 and 12.59 (2s br, 1H), 8.68-8.54 (m, 2H), 8.42-8.33 (m, 1H), 8.29-8.20 (m, 1H), 7.55-7.13 (m, 6H), 6.62-6.27 (m, 1H), 6.04-5.95, 5.65-5.54 and 4.87-4.79 (3m, 2H), 4.29-4.14 (m, 2H), 3.60-3.48 (m, 3H), 2.84 and 2.73 (2s, 3H)

### Example 53

### N-[2-({4-[3-(2-ethylphenyl)-1H-pyrrolo[3,2-b]pyridin-2-yl]pyridin-3-yl}oxy)ethyl]-N-methylprop-2-enamide

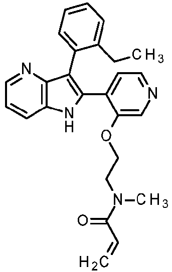

To a solution of 2-({4-[3-(2-ethylphenyl)-1H-pyrrolo[3,2-b]pyridin-2-yl]pyridin-3-yl}oxy)-N-methylethan-1-amine (20.0 mg, 53.7 µmol) in THF (1 ml), prop-2-enoic acid (3.7 µl, 54 µmol) and N,N-diisopropylethylamine (28 µl, 160 µmol) were added at rt. T3P (47 µl, 50 % purity in EtOAc, 81 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 5.00 mg (88 % purity, 19 % yield) of the desired product.
LC-MS (method 1): Rₜ = 1.00 min; MS (ESlpos): m/z = 427 [M+H]⁺
¹H-NMR (500 MHz, CHLOROFORM-d) δ [ppm]: 0.943 (5.25), 0.958 (11.11), 0.973 (5.42), 1.465 (1.43), 1.794 (0.41), 2.420 (0.48), 2.434 (0.85), 2.449 (1.40), 2.464 (1.48), 2.481 (1.45), 2.496 (1.35), 2.511 (0.82), 2.526 (0.48), 3.196 (0.61), 3.236 (16.00), 4.147 (0.88), 4.152 (0.98), 4.159 (1.16), 4.166 (1.48), 4.172 (1.13), 4.179 (1.02), 4.184 (1.03), 4.364 (0.46), 4.378 (1.12), 4.383 (1.01), 4.391 (1.13), 4.397 (1.49), 4.403 (1.17), 4.410 (0.98), 4.416 (1.02), 5.760 (1.72), 5.764 (1.74), 5.780 (1.73), 5.784 (1.88), 6.432 (1.40), 6.435 (1.47), 6.465 (1.96), 6.468 (1.97), 6.633 (1.81), 6.654 (1.81), 6.666 (1.40), 6.687 (1.28), 7.102 (1.52), 7.112 (1.57), 7.149 (1.78), 7.158 (1.84), 7.165 (1.87), 7.174 (1.91), 7.223 (1.14), 7.235 (2.34), 7.238 (2.44), 7.252 (1.36), 7.254 (1.46), 7.262 (11.69), 7.268 (2.04), 7.280 (0.88), 7.283 (0.87), 7.357 (0.81), 7.360 (0.83), 7.372 (1.79), 7.375 (1.71), 7.386 (1.45), 7.389 (1.48), 7.400 (2.41), 7.414 (1.07), 7.444 (0.40), 7.446 (0.47), 7.450 (0.40), 7.452 (0.47), 7.460 (0.98), 7.463 (0.79), 7.466 (1.01), 7.476 (0.70), 7.478 (0.52), 7.481 (0.70), 7.531 (0.47), 7.534 (0.50), 7.545 (0.56), 7.549 (0.55), 7.649 (0.70), 7.652 (0.82), 7.665 (0.71), 7.668 (0.59), 7.673 (0.76), 7.675 (0.86), 7.690 (0.70), 7.692 (0.56), 7.941 (1.11), 7.951 (1.12), 8.292 (1.72), 8.294 (1.87), 8.308 (1.74), 8.311 (1.81), 8.361 (1.78), 8.465 (1.83), 8.468 (1.96), 8.474 (1.96), 8.476 (1.91), 10.966 (1.01).

### Example 54

### N-[2-({4-[3-(3-methoxyphenyl)-1H-pyrrolo[3,2-b]pyridin-2-yl]pyridin-3-yl}oxy)ethyl]-N-methylprop-2-enamide

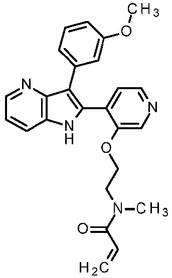

To a solution of 2-({4-[3-(3-methoxyphenyl)-1H-pyrrolo[3,2-b]pyridin-2-yl]pyridin-3-yl}oxy)-N-methylethan-1-amine (65.0 mg, 174 µmol) in THF (1 ml), prop-2-enoic acid (12 µl, 170 µmol) and N,N-diisopropylethylamine (91 µl, 520 µmol) were added at rt. T3P (150 µl, 50 % purity in EtOAc, 260 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 28.0 mg (100 % purity, 38 % yield) of the desired product.
LC-MS (method 1): Rₜ = 0.91 min; MS (ESlpos): m/z = 429 [M+H]⁺
¹H-NMR (400 MHz, DMSO-d6) δ [ppm]: 11.77 and 11.70 (2s, 1H), 8.56-8.47 (m, 1H), 8.46-8.40 (m, 1H), 8.35-8.23 (m, 1H), 7.88-7.79 (m, 1H), 7.44-7.30 (m, 1H), 7.26-7.18 (m, 2H), 7.17-7.10 (m, 2H), 6.82-6.76 (m, 1H), 6.64-6.32 (m, 1H), 6.09-6.03, 5.76-5.68, 5.64-5.58 and 4.89-4.84 (4m, 2H), 4.20-4.02 (m, 2H), 3.65 and 3.63 (2s, 3H), 3.48-3.38 (m, 2H), 2.81 and 2.72 (2s, 3H)

### Example 55

### N-methyl-N-{2-[(4-{3-[3-(propan-2-yl)phenyl]-1H-pyrrolo[3,2-b]pyridin-2-yl}pyridin-3-yl)oxy]ethyl}prop-2-enamide

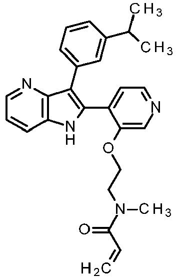

To a solution of N-methyl-2-[(4-{3-[3-(propan-2-yl)phenyl]-1H-pyrrolo[3,2-b]pyridin-2-yl}pyridin-3-yl)oxy]ethan-1-amine (50.0 mg, 129 µmol) in THF (1 ml), prop-2-enoic acid (8.9 µl, 130 µmol) and N,N-diisopropylethylamine (68 µl, 390 µmol) were added at rt. T3P (110 µl, 50 % purity in EtOAc, 190 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 40.8 mg (100 % purity, 72 % yield) of the desired product.
LC-MS (method 1): Rₜ = 1.19 min; MS (ESlpos): m/z = 441 [M+H]⁺
¹H-NMR (400 MHz, DMSO-d6) δ [ppm]: 11.74 and 11.67 (2s, 1H), 8.54-8.46 (m, 1H), 8.46-8.40 (m, 1H), 8.35-8.22 (m, 1H), 7.88-7.79 (m, 1H), 7.59-7.52 (m, 1H), 7.45-7.31 (m, 1H), 7.29-7.18 (m, 3H), 7.11-7.05 (m, 1H), 6.63-6.31 (m, 1H), 6.09-6.03, 5.77-5.71, 5.64-5.59 and 4.90-4.85 (4m, 2H), 4.14-3.95 (m, 2H), 3.42-3.36 (m, 1H), 3.33-3.29 (m, 1H), 2.80 and 2.71 (2s, 3H), 2.78-2.73 (m, 1H), 1.09-1.03 (m, 6H)

### Example 56

### N-[2-({4-[3-(2-fluoro-5-methoxyphenyl)-1H-pyrrolo[3,2-b]pyridin-2-yl]pyridin-3-yl}oxy)ethyl]-N-methylprop-2-enamide

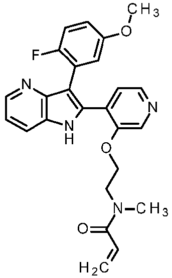

To a solution of 2-({4-[3-(2-fluoro-5-methoxyphenyl)-1H-pyrrolo[3,2-b]pyridin-2-yl]pyridin-3-yl}oxy)-N-methylethan-1-amine (30.0 mg, 76.4 µmol) in THF (1 ml), prop-2-enoic acid (5.2 µl, 76 µmol) and N,N-diisopropylethylamine (40 µl, 230 µmol) were added at rt. T3P (68 µl, 50 % purity in EtOAc, 110 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 18.2 mg (100 % purity, 53 % yield) of the desired product.
LC-MS (method 1): Rₜ = 0.92 min; MS (ESlpos): m/z = 447 [M+H]⁺
¹H-NMR (400 MHz, DMSO-d6) δ [ppm]: 11.83 and 11.72 (2s br, 1H), 8.62-8.31 (m, 2H), 8.28-8.12 (m, 1H), 8.00-7.80 (m, 1H), 7.35-7.00 (m, 4H), 6.96-6.83 (m, 1H), 6.74-6.38 (m, 1H), 6.19-6.04, 5.86-5.71, 5.69-5.59 and 5.07-4.94 (4m, 2H), 4.35-4.04 (m, 2H), 3.81-3.67 (m, 3H), 3.66-3.49 (m, 2H), 2.93 and 2.77 (2s, 3H)

### Example 57

### N-[2-({4-[3-(2,5-dichlorophenyl)-1H-pyrrolo[3,2-b]pyridin-2-yl]pyridin-3-yl}oxy)ethyl]-N-methylprop-2-enamide

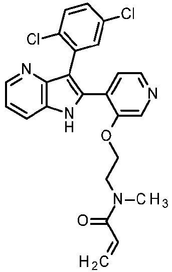

To a solution of 2-({4-[3-(2,5-dichlorophenyl)-1H-pyrrolo[3,2-b]pyridin-2-yl]pyridin-3-yl}oxy)-N-methylethan-1-amine (15.0 mg, 36.3 µmol) in THF (1 ml), prop-2-enoic acid (2.5 µl, 36 µmol) and N,N-diisopropylethylamine (19 µl, 110 µmol) were added at rt. T3P (32 µl, 50 % purity in EtOAc, 54 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding the desired product in insufficient purity. This material was further purified by thin layer chromatography (eluent: DCM / MeOH 10:1) to yield 2.70 mg (99 % purity, 16 % yield) of the desired product.
LC-MS (method 1): Rₜ = 1.05 min; MS (ESlpos): m/z = 467 [M+H]⁺
¹H-NMR (500 MHz, METHANOL-d4) δ [ppm]: 0.894 (0.42), 1.283 (1.84), 1.978 (1.56), 2.926 (9.63), 3.017 (16.00), 3.343 (9.71), 3.629 (0.40), 3.808 (0.88), 3.817 (1.53), 3.827 (0.97), 3.898 (0.73), 4.317 (1.05), 4.383 (0.87), 4.926 (0.94), 4.930 (0.92), 4.947 (0.92), 4.951 (0.97), 5.644 (1.43), 5.648 (1.42), 5.665 (1.46), 5.669 (1.52), 5.745 (0.88), 5.749 (0.89), 5.778 (0.98), 5.782 (0.97), 6.145 (1.28), 6.149 (1.30), 6.178 (1.51), 6.182 (1.49), 6.413 (0.77), 6.434 (0.79), 6.446 (0.75), 6.467 (0.68), 6.599 (1.36), 6.620 (1.36), 6.633 (1.23), 6.654 (1.12), 7.119 (2.01), 7.128 (2.11), 7.140 (1.48), 7.150 (1.47), 7.284 (1.90), 7.293 (1.96), 7.301 (2.04), 7.310 (2.02), 7.337 (0.82), 7.343 (0.88), 7.355 (1.13), 7.360 (1.23), 7.384 (1.10), 7.390 (1.21), 7.402 (1.62), 7.407 (1.79), 7.425 (2.02), 7.442 (1.51), 7.448 (1.94), 7.453 (1.79), 7.470 (2.86), 7.481 (2.80), 7.486 (3.76), 7.918 (1.06), 7.920 (1.12), 7.934 (1.04), 7.937 (1.04), 8.060 (1.61), 8.070 (1.61), 8.083 (1.62), 8.086 (1.68), 8.099 (1.55), 8.102 (1.56), 8.129 (1.24), 8.139 (1.21), 8.322 (1.67), 8.324 (1.81), 8.331 (2.79), 8.334 (2.80), 8.341 (1.27), 8.343 (1.16), 8.440 (2.09), 8.455 (2.66).

### Example 58

### N-methyl-N-{2-[(4-{3-[3-(trifluoromethoxy)phenyl]-1H-pyrrolo[3,2-b]pyridin-2-yl}pyridin-3-yl)oxy]ethyl}prop-2-enamide

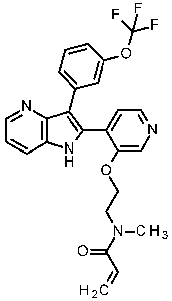

To a solution of N-methyl-2-[(4-{3-[3-(trifluoromethoxy)phenyl]-1H-pyrrolo[3,2-b]pyridin-2-yl}pyridin-3-yl)oxy]ethan-1-amine (50.0 mg, 117 µmol) in THF (1 ml), prop-2-enoic acid (8.0 µl, 120 µmol) and N,N-diisopropylethylamine (61 µl, 350 µmol) were added at rt. T3P (100 µl, 50 % purity in EtOAc, 180 µmol) was added and stirring at rt was continued for 16 h. Water (1 ml) was added and the mixture was extracted with EtOAc twice. The combined organic layers were dried over sodium sulfate, filtered and concentrated under reduced pressure. The residue was purified by reverse phase preparative HPLC (method 3) yielding 33.4 mg (100 % purity, 59 % yield) of the desired product.
LC-MS (method 1): Rₜ = 1.27 min; MS (ESlpos): m/z = 483 [M+H]⁺
¹H-NMR (400 MHz, DMSO-d6) δ [ppm]: 11.96 and 11.91 (2s, 1H), 8.60-8.53 (m, 1H), 8.53-8.48 (m, 1H), 8.41-8.32 (m, 1H), 7.93-7.86 (m, 1H), 7.69-7.64 (m, 1H), 7.59-7.55 (m, 1H), 7.51-7.41 (m, 2H), 7.32-7.27 (m, 1H), 7.25-7.21 (m, 1H), 6.64-6.30 (m, 1H), 6.10-6.04, 5.77-5.71, 5.65-5.61 and 4.91-4.85 (4m, 2H), 4.18-4.05 (m, 2H), 3.46-3.31 (m, 2H), 2.79-2.73 (2s, 3H)

### Example 59

### N-{2-[(4-{3-[2-fluoro-5-(trifluoromethyl)phenyl]-1H-pyrrolo[3,2-b]pyridin-2-yl}pyridin-3-yl)oxy]ethyl}-N-methylethenesulfonamide

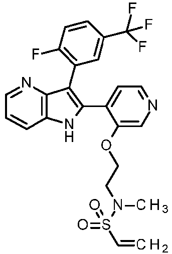

To a solution of 2-[(4-{3-[2-fluoro-5-(trifluoromethyl)phenyl]-1H-pyrrolo[3,2-b]pyridin-2-yl}pyridin-3-yl)oxy]-N-methylethan-1-amine (30.0 mg, 69.7 µmol) and triethylamine (34 µl, 240 µmol) in DCM (1 ml), 2-chloroethane-1-sulfonyl chloride (7.3 µl, 70 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 2 fractions with insufficient purity. The combined frations were further purified by thin layer chromatograpy (Eluent: DCM / MeOH 10:1) to yield 5.30 mg (100 % purity, 15 % yield) of the title compound.
LC-MS (method 1): Rₜ = 1.40 min; MS (ESlpos): m/z = 521 [M+H]⁺
¹H-NMR (500 MHz, CHLOROFORM-d) δ [ppm]: 1.255 (1.08), 2.002 (2.41), 2.919 (16.00), 3.653 (1.87), 3.662 (2.87), 3.671 (1.94), 4.362 (2.14), 4.371 (3.16), 4.380 (2.04), 6.060 (3.02), 6.080 (3.35), 6.312 (2.46), 6.345 (3.90), 6.448 (1.93), 6.468 (1.85), 6.482 (1.29), 6.501 (1.13), 7.118 (2.19), 7.128 (2.24), 7.186 (1.61), 7.195 (1.63), 7.202 (1.66), 7.211 (1.72), 7.218 (0.83), 7.236 (1.60), 7.253 (0.88), 7.264 (4.34), 7.621 (0.51), 7.626 (0.61), 7.629 (0.62), 7.637 (0.66), 7.643 (0.59), 7.647 (0.56), 7.652 (0.49), 7.948 (0.92), 7.952 (0.96), 7.961 (0.97), 7.965 (0.91), 8.009 (1.69), 8.011 (1.78), 8.025 (1.67), 8.028 (1.65), 8.104 (2.16), 8.114 (2.12), 8.451 (3.38), 8.511 (1.72), 8.513 (1.80), 8.520 (1.75), 8.522 (1.70), 10.421 (1.16).

### Example 60

### N-[2-({4-[3-(5-chloro-2-fluorophenyl)-1H-pyrrolo[3,2-b]pyridin-2-yl]pyridin-3-yl}oxy)ethyl]-N-methylethenesulfonamide

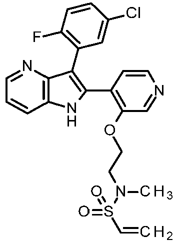

To a solution of 2-({4-[3-(5-chloro-2-fluorophenyl)-1H-pyrrolo[3,2-b]pyridin-2-yl]pyridin-3-yl}oxy)-N-methylethan-1-amine (30.0 mg, 75.6 µmol) and triethylamine (37 µl, 260 µmol) in DCM (1 ml), 2-chloroethane-1-sulfonyl chloride (3.9 µl, 38 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 9.00 mg (100 % purity, 24 % yield) of the title compound.
LC-MS (method 1): Rₜ = 1.20 min; MS (ESlpos): m/z = 487 [M+H]⁺
¹H-NMR (400 MHz, DMSO-d6) δ [ppm]: 2.587 (16.00), 3.149 (2.16), 3.163 (4.48), 3.176 (2.44), 4.120 (2.26), 4.134 (4.18), 4.148 (2.06), 5.914 (2.77), 5.939 (3.00), 5.962 (2.67), 6.004 (3.04), 6.557 (1.56), 6.581 (1.52), 6.598 (1.42), 6.623 (1.24), 7.177 (1.20), 7.200 (2.33), 7.223 (1.50), 7.240 (1.55), 7.252 (1.61), 7.261 (1.65), 7.272 (1.60), 7.313 (2.72), 7.325 (2.76), 7.385 (1.34), 7.393 (1.17), 7.405 (1.09), 7.415 (0.77), 7.750 (1.52), 7.756 (1.61), 7.765 (1.64), 7.772 (1.46), 7.873 (2.21), 7.892 (2.05), 8.265 (2.58), 8.277 (2.50), 8.420 (2.26), 8.430 (2.22), 8.515 (4.22), 11.910 (1.75).

### Example 61

### N-[2-({4-[3-(2,5-difluorophenyl)-1H-pyrrolo[3,2-b]pyridin-2-yl]pyridin-3-yl}oxy)ethyl]-N-methylethenesulfonamide

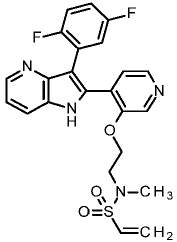

To a solution of 2-({4-[3-(2,5-difluorophenyl)-1H-pyrrolo[3,2-b]pyridin-2-yl]pyridin-3-yl}oxy)-N-methylethan-1-amine (33.0 mg, 86.8 µmol) and triethylamine (42 µl, 300 µmol) in DCM (1 ml), 2-chloroethane-1-sulfonyl chloride (9.1 µl, 87 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding the title compound with insufficient purity. Further purification by thin layer chromatograpy (Eluent: DCM / MeOH 10:1) yielded 1.70 mg (96 % purity, 4 % yield) of the title compound.
LC-MS (method 1): Rₜ = 1.02 min; MS (ESlpos): m/z = 471 [M+H]⁺
¹H-NMR (500 MHz, CHLOROFORM-d) δ [ppm]: 2.913 (16.00), 2.992 (0.41), 3.481 (0.54), 3.656 (2.48), 3.664 (3.89), 3.673 (2.57), 4.352 (2.76), 4.361 (4.16), 4.370 (2.56), 6.048 (2.82), 6.068 (3.09), 6.303 (2.27), 6.336 (3.57), 6.437 (1.82), 6.457 (1.69), 6.470 (1.19), 6.490 (1.05), 7.033 (0.87), 7.039 (1.27), 7.047 (2.32), 7.056 (1.88), 7.063 (1.65), 7.074 (1.18), 7.082 (0.47), 7.173 (1.57), 7.182 (1.75), 7.189 (1.85), 7.199 (3.94), 7.210 (2.70), 7.373 (0.72), 7.379 (0.84), 7.384 (0.92), 7.390 (1.36), 7.396 (0.91), 7.401 (0.85), 7.407 (0.72), 7.997 (1.98), 7.999 (2.01), 8.013 (1.94), 8.015 (1.91), 8.118 (2.91), 8.128 (2.84), 8.439 (4.89), 8.517 (2.06), 8.519 (2.13), 8.526 (2.13), 10.372 (1.61).

### Example 62

### N-[2-({4-[3-(1-benzothiophen-6-yl)-1H-pyrrolo[3,2-b]pyridin-2-yl]pyridin-3-yl}oxy)ethyl]-N-methylethenesulfonamide

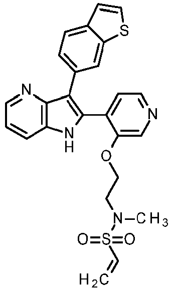

To a solution of 2-({4-[3-(1-benzothiophen-6-yl)-1H-pyrrolo[3,2-b]pyridin-2-yl]pyridin-3-yl}oxy)-N-methylethan-1-amine (55.0 mg, 137 µmol) and triethylamine (67 µl, 480 µmol) in DCM (1 ml), 2-chloroethane-1-sulfonyl chloride (14 µl, 140 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 27.5 mg (100 % purity, 41 % yield) of the title compound.
LC-MS (method 1): Rₜ = 1.17 min; MS (ESlpos): m/z = 491 [M+H]⁺
¹H-NMR (400 MHz, DMSO-d6) δ [ppm]: 11.75 (s br, 1H), 8.53 (s, 1H), 8.47-8.42 (m, 1H), 8.32-8.24 (m, 2H), 7.89-7.81 (m, 1H), 7.80-7.74 (m, 1H), 7.73-7.68 (m, 1H), 7.46-7.35 (m, 3H), 7.29-7.19 (m, 1H), 6.57-6.46 (m, 1H), 5.97-5.85 (m, 2H), 4.13-4.03 (m, 2H), 3.02-2.94 (m, 2H), 2.43 (s, 3H)

### Example 63

### N-[2-({4-[3-(3-ethylphenyl)-1H-pyrrolo[3,2-b]pyridin-2-yl]pyridin-3-yl}oxy)ethyl]-N-methylethenesulfonamide

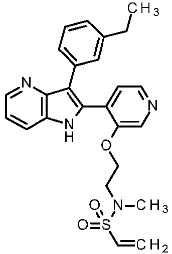

To a solution of 2-({4-[3-(3-ethylphenyl)-1H-pyrrolo[3,2-b]pyridin-2-yl]pyridin-3-yl}oxy)-N-methylethan-1-amine (76.0 mg, 204 µmol) and triethylamine (100 µl, 710 µmol) in DCM (1 ml), 2-chloroethane-1-sulfonyl chloride (21 µl, 200 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 36.3 mg (93 % purity, 36 % yield) of the title compound.
LC-MS (method 1): Rₜ = 1.22 min; MS (ESlpos): m/z = 463 [M+H]⁺
¹H-NMR (400 MHz, DMSO-d6) δ [ppm]: 11.88 (s br, 1H), 8.51 (s, 1H), 8.44-8.41 (m, 1H), 8.31-8.29 (m, 1H), 7.84-7.81 (m, 1H), 7.43-7.31 (m, 3H), 7.25-7.19 (m, 2H), 7.07-7.04 (m, 1H), 6.61-6.54 (m, 1H), 5.98-5.87 (m, 2H), 4.09-4.05 (m, 2H), 3.07-3.03 (m, 2H), 2.53-2.50 (m, 2H), 2.51 (s, 3H), 1.09-1.05 (m, 3H)

### Example 64

### N-[2-({4-[3-(2-fluoro-5-methylphenyl)-1H-pyrrolo[3,2-b]pyridin-2-yl]pyridin-3-yl}oxy)ethyl]-N-methylethenesulfonamide

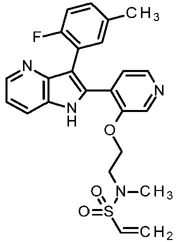

To a solution of 2-({4-[3-(2-fluoro-5-methylphenyl)-1H-pyrrolo[3,2-b]pyridin-2-yl]pyridin-3-yl}oxy)-N-methylethan-1-amine (42.0 mg, 112 µmol) and triethylamine (54 µl, 390 µmol) in DCM (1 ml), 2-chloroethane-1-sulfonyl chloride (10 µl, 100 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 19.0 mg (99 % purity, 36 % yield) of the title compound.
LC-MS (method 2): Rₜ = 1.16 min; MS (ESlpos): m/z = 467 [M+H]⁺
¹H-NMR (400 MHz, DMSO-d6) δ [ppm]: 2.312 (10.19), 2.604 (16.00), 3.160 (0.60), 3.173 (0.64), 3.212 (1.75), 3.226 (3.65), 3.240 (1.91), 3.261 (0.41), 3.279 (0.51), 3.302 (0.79), 3.317 (0.81), 3.406 (1.29), 4.140 (1.77), 4.154 (3.59), 4.168 (1.72), 5.915 (2.84), 5.940 (3.04), 5.970 (2.71), 6.011 (3.06), 6.588 (1.57), 6.613 (1.57), 6.629 (1.47), 6.654 (1.29), 6.995 (1.02), 7.016 (1.63), 7.040 (1.41), 7.122 (0.84), 7.134 (1.04), 7.204 (1.55), 7.216 (1.63), 7.228 (3.05), 7.240 (2.88), 7.442 (1.26), 7.454 (1.23), 7.848 (1.83), 7.851 (1.94), 7.868 (1.76), 7.871 (1.75), 8.208 (2.25), 8.220 (2.14), 8.377 (1.87), 8.380 (1.99), 8.388 (1.89), 8.391 (1.81), 8.504 (3.56), 11.708 (2.50).

### Example 65

### N-[2-({4-[3-(3-fluoro-5-methylphenyl)-1H-pyrrolo[3,2-b]pyridin-2-yl]pyridin-3-yl}oxy)ethyl]-N-methylethenesulfonamide

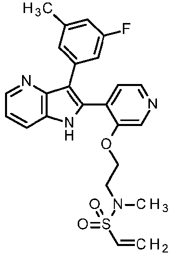

To a solution of 2-({4-[3-(3-fluoro-5-methylphenyl)-1H-pyrrolo[3,2-b]pyridin-2-yl]pyridin-3-yl}oxy)-N-methylethan-1-amine (65.0 mg, 173 µmol) and triethylamine (84 µl, 600 µmol) in DCM (1 ml), 2-chloroethane-1-sulfonyl chloride (18 µl, 170 µmol) was added at rt. After stirring at rt for 3 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 41.6 mg (100 % purity, 52 % yield) of the title compound.
LC-MS (method 1): Rₜ = 1.18 min; MS (ESlpos): m/z = 467 [M+H]⁺
¹H-NMR (400 MHz, DMSO-d6) δ [ppm]: 11.80 (s br, 1H), 8.55 (s, 1H), 8.47-8.42 (m, 1H), 8.36-8.30 (m, 1H), 7.87-7.81 (m, 1H), 7.43-7.37 (m, 1H), 7.27-7.21 (m, 1H), 7.20-7.17 (m, 1H), 7.17-7.12 (m, 1H), 6.90-6.84 (m, 1H), 6.60-6.53 (m, 1H), 6.00-5.85 (m, 2H), 4.16-4.09 (m, 2H), 3.12-3.05 (m, 2H), 2.25 (s, 3H)

### Example 66

### N-methyl-N-{2-[(4-{3-[3-(trifluoromethyl)phenyl]-1H-pyrrolo[3,2-b]pyridin-2-yl}pyridin-3-yl)oxy]ethyl}ethenesulfonamide

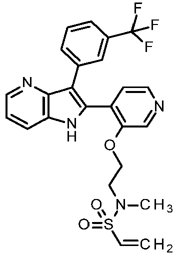

To a solution of N-methyl-2-[(4-{3-[3-(trifluoromethyl)phenyl]-1H-pyrrolo[3,2-b]pyridin-2-yl}pyridin-3-yl)oxy]ethan-1-amine (79.0 mg, 192 µmol) and triethylamine (93 µl, 670 µmol) in DCM (1 ml), 2-chloroethane-1-sulfonyl chloride (20 µl, 190 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 44.0 mg (100 % purity, 46 % yield) of the title compound.
LC-MS (method 1): Rₜ = 1.39 min; MS (ESlpos): m/z = 503 [M+H]⁺
¹H-NMR (400 MHz, DMSO-d6) δ [ppm]: 11.93 (s br, 1H), 8.56-8.52 (m, 1H), 8.49-8.45 (m, 1H), 8.38-8.33 (m, 1H), 7.99-7.94 (m, 1H), 7.90-7.85 (m, 1H), 7.81-7.76 (m, 1H), 7.58-7.51 (m, 2H), 7.48-7.46 (m, 1H), 7.29-7.24 (m, 1H), 6.56-6.48 (m, 1H), 5.96-5.85 (m, 2H), 4.08-4.04 (m, 2H), 3.00-2.94 (m, 2H), 2.47 (s, 3H)

### Example 67

### N-[2-({4-[3-(5-ethyl-2-fluorophenyl)-1H-pyrrolo[3,2-b]pyridin-2-yl]pyridin-3-yl}oxy)ethyl]-N-methylethenesulfonamide

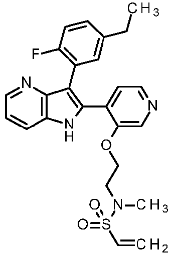

To a solution of 2-({4-[3-(5-ethyl-2-fluorophenyl)-1H-pyrrolo[3,2-b]pyridin-2-yl]pyridin-3-yl}oxy)-N-methylethan-1-amine (59.0 mg, 151 µmol) and triethylamine (74 µl, 530 µmol) in DCM (1 ml), 2-chloroethane-1-sulfonyl chloride (16 µl, 150 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) to yield the desired compound with insufficient purity. Further purification by thin layer chromatograpy (Eluent: DCM / MeOH 10:1) yielded 12.5 mg (100 % purity, 17 % yield) of the title compound.
LC-MS (method 1): Rₜ = 1.22 min; MS (ESlpos): m/z = 481 [M+H]⁺
¹H-NMR (400 MHz, DMSO-d6) δ [ppm]: 11.72 (s br, 1H), 8.50 (s, 1H), 8.41-8.36 (m, 1H), 8.25-8.20 (m, 1H), 7.89-7.84 (m, 1H), 7.43-7.38 (m, 1H), 7.27-7.24 (m, 1H), 7.23-7.20 (m, 1H), 7.19-7.15 (m, 1H), 7.09-7.03 (m, 1H), 6.65-6.59 (m, 1H), 6.02-5.91 (m, 2H), 4.16-4.12 (m, 2H), 3.23-3.19 (m, 2H), 2.63-2.57 (m, 5H), 1.17-1.13 (m, 3H)

### Example 68

### N-[2-({4-[3-(3,5-difluorophenyl)-1H-pyrrolo[3,2-b]pyridin-2-yl]pyridin-3-yl)oxy)ethyl]-N-methylethenesulfonamide

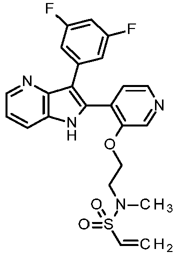

To a solution of 2-({4-[3-(3,5-difluorophenyl)-1H-pyrrolo[3,2-b]pyridin-2-yl]pyridin-3-yl}oxy)-N-methylethan-1-amine (50.0 mg, 131 µmol) and triethylamine (64 µl, 460 µmol) in DCM (1 ml), 2-chloroethane-1-sulfonyl chloride (14 µl, 130 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 28.0 mg (100 % purity, 45 % yield) of the title compound.
LC-MS (method 1): Rₜ = 1.28 min; MS (ESlpos): m/z = 471 [M+H]⁺
¹H-NMR (400 MHz, DMSO-d6) δ [ppm]: 12.01 (s, 1H), 8.62 (s, 1H), 8.54-8.48 (m, 1H), 8.43-8.39 (m, 1H), 7.93-7.86 (m, 1H), 7.54-7.47 (m, 1H), 7.34-7.25 (m, 3H), 7.14-7.06 (m, 1H), 6.63-6.53 (m, 1H), 6.02-5.87 (m, 2H), 4.23-4.15 (m, 2H), 3.17-3.10 (m, 2H), 2.58 (s, 3H)

### Example 69

### N-[2-({4-[3-(3-methoxyphenyl)-1H-pyrrolo[3,2-b]pyridin-2-yl]pyridin-3-yl}oxy)ethyl]-N-methylethenesulfonamide

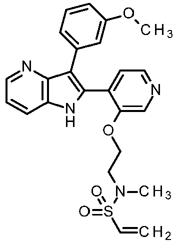

To a solution of 2-({4-[3-(3-methoxyphenyl)-1H-pyrrolo[3,2-b]pyridin-2-yl]pyridin-3-yl}oxy)-N-methylethan-1-amine (66.0 mg, 176 µmol) and triethylamine (86 µl, 620 µmol) in DCM (1 ml), 2-chloroethane-1-sulfonyl chloride (18 µl, 180 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 19.3 mg (100 % purity, 24 % yield) of the title compound.
LC-MS (method 1): Rₜ = 1.04 min; MS (ESlpos): m/z = 465 [M+H]⁺
¹H-NMR (400 MHz, DMSO-d6) δ [ppm]: 11.72 (s, 1H), 8.53 (s, 1H), 8.45-8.41 (m, 1H), 8.33-8.29 (m, 1H), 7.85-7.80 (m, 1H), 7.40-7.36 (m, 1H), 7.24-7.19 (m, 2H), 7.16-7.13 (m, 1H), 7.12-7.08 (m, 1H), 6.81-6.77 (m, 1H), 6.59-6.53 (m, 1H), 5.98-5.87 (m, 2H), 4.14-4.09 (m, 2H), 3.65 (s, 3H), 3.12-3.07 (m, 2H), 2.54 (3, 3H)

### Example 70

### N-methyl-N-{2-[(4-{3-[3-(propan-2-yl)phenyl]-1H-pyrrolo[3,2-b]pyridin-2-yl}pyridin-3-yl)oxy]ethyl}ethenesulfonamide

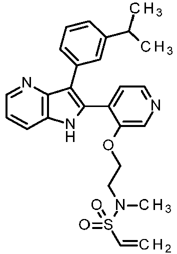

To a solution of N-methyl-2-[(4-{3-[3-(propan-2-yl)phenyl]-1H-pyrrolo[3,2-b]pyridin-2-yl}pyridin-3-yl)oxy]ethan-1-amine (50.0 mg, 129 µmol) and triethylamine (63 µl, 450 µmol) in DCM (1 ml), 2-chloroethane-1-sulfonyl chloride (14 µl, 130 µmol) was added at rt. After stirring at rt for 3 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 20.0 mg (96 % purity, 31 % yield) of the title compound.
LC-MS (method 1): Rₜ = 1.33 min; MS (ESlpos): m/z = 477 [M+H]⁺
¹H-NMR (400 MHz, DMSO-d6) δ [ppm]: 11.70 (s, 1H), 8.51 (s, 1H), 8.45-8.41 (m, 1H), 8.33-8.29 (m, 1H), 7.85-7.81 (m, 1H), 7.56-7.51 (m, 1H), 7.42-7.38 (m, 1H), 7.29 (s, 3H), 7.09-7.05 (m, 1H), 6.59-6.53 (m, 1H), 5.97-5.86 (m, 2H), 4.08-4.03 (m, 2H), 3.06-3.00 (m, 2H), 2.81-2.72 (m, 1H), 2.54 (s, 3H), 1.08-1.04 (m, 6H)

### Example 71

### N-[2-({4-[3-(2,5-dichlorophenyl)-1H-pyrrolo[3,2-b]pyridin-2-yl]pyridin-3-yl}oxy)ethyl]-N-methylethenesulfonamide

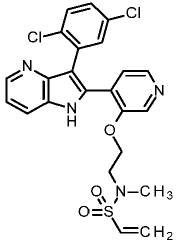

To a solution of 2-({4-[3-(2,5-dichlorophenyl)-1H-pyrrolo[3,2-b]pyridin-2-yl]pyridin-3-yl}oxy)-N-methylethan-1-amine (23.0 mg, 55.6 µmol) and triethylamine (27 µl, 190 µmol) in DCM (1 ml), 2-chloroethane-1-sulfonyl chloride (5.8 µl, 56 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 7.20 mg (93 % purity, 24 % yield) of the title compound.
LC-MS (method 2): Rₜ = 1.35 min; MS (ESlpos): m/z = 503 [M+H]⁺
¹H-NMR (400 MHz, DMSO-d6) δ [ppm]: 12.86-11.98 (m, 1H), 8.60-8.54 (m, 1H), 8.53-8.47 (m, 1H), 8.31-8.12 (m, 2H), 7.61-7.40 (m, 4H), 7.23-7.16 (m, 1H), 6.71-6.59 (m, 1H), 6.06-5.94 (m, 2H), 4.29-4.17 (m, 2H), 3.43-3.26 (m, 2H), 2.62 (s, 3H)

### Example 72

### N-[2-({4-[3-(2-ethylphenyl)-1H-pyrrolo[3,2-b]pyridin-2-yl]pyridin-3-yl}oxy)ethyl]-N-methylethenesulfonamide

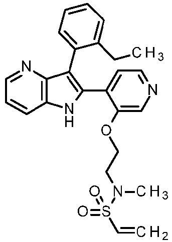

To a solution of 2-({4-[3-(2-ethylphenyl)-1H-pyrrolo[3,2-b]pyridin-2-yl]pyridin-3-yl}oxy)-N-methylethan-1-amine (17.0 mg, 45.6 µmol) and triethylamine (22 µl, 160 µmol) in DCM (1 ml), 2-chloroethane-1-sulfonyl chloride (4.8 µl, 46 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 7.10 mg (100 % purity, 34 % yield) of the title compound.
LC-MS (method 1): Rₜ = 1.13 min; MS (ESlpos): m/z = 463 [M+H]⁺
¹H-NMR (500 MHz, CHLOROFORM-d) δ [ppm]: 0.954 (4.61), 0.969 (8.69), 0.984 (4.76), 1.965 (1.21), 1.993 (1.20), 2.166 (1.53), 2.431 (0.72), 2.446 (1.25), 2.460 (1.89), 2.476 (2.00), 2.494 (2.01), 2.509 (1.87), 2.524 (1.24), 2.538 (0.72), 2.602 (0.77), 2.939 (16.00), 3.677 (0.67), 3.721 (2.46), 3.766 (0.69), 4.343 (1.92), 4.347 (1.92), 4.354 (1.87), 4.383 (1.83), 4.395 (1.93), 6.084 (2.79), 6.103 (3.04), 6.346 (2.24), 6.379 (3.37), 6.493 (1.75), 6.513 (1.73), 6.526 (1.33), 6.546 (1.09), 7.094 (2.96), 7.104 (3.07), 7.129 (1.89), 7.138 (2.16), 7.145 (2.18), 7.154 (1.92), 7.224 (1.68), 7.262 (3.33), 7.358 (1.15), 7.372 (2.55), 7.387 (2.38), 7.398 (3.78), 7.413 (1.74), 7.976 (2.72), 7.985 (2.72), 8.042 (2.64), 8.058 (2.58), 8.386 (4.25), 8.461 (2.94), 8.468 (2.96), 10.510 (2.58).

### Example 73

### N-[2-({4-[3-(2-fluoro-5-methoxyphenyl)-1H-pyrrolo[3,2-b]pyridin-2-yl]pyridin-3-yl}oxy)ethyl]-N-methylethenesulfonamide

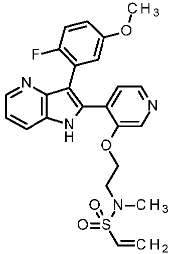

To a solution of 2-({4-[3-(2-fluoro-5-methoxyphenyl)-1H-pyrrolo[3,2-b]pyridin-2-yl]pyridin-3-yl}oxy)-N-methylethan-1-amine (43.0 mg, 110 µmol) and triethylamine (53 µl, 380 µmol) in DCM (1 ml), 2-chloroethane-1-sulfonyl chloride (11 µl, 110 µmol) was added at rt. After stirring at rt for 16 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 13.2 mg (96 % purity, 24 % yield) of the title compound.
LC-MS (method 1): Rₜ = 1.05 min; MS (ESlpos): m/z = 483 [M+H]⁺
¹H-NMR (400 MHz, DMSO-d6) δ [ppm]: 11.79 (s br, 1H), 8.55 (s, 1H), 8.45-8.40 (m, 1H), 8.29-8.25 (m, 1H), 7.92-7.88 (m, 1H), 7.32-7.29 (m, 1H), 7.28-7.24 (m, 1H), 7.22-7.17 (m, 1H), 7.14-7.09 (m, 1H), 6.95-6.90 (m, 1H), 6.68-6.61 (m, 1H), 6.06-5.95 (m, 2H), 4.24-4.17 (m, 2H), 3.77 (s, 3H), 3.30-3.25 (m, 2H), 2.64 (s, 3H)

### Example 74

### N-methyl-N-{2-[(4-{3-[3-(trifluoromethoxy)phenyl]-1H-pyrrolo[3,2-b]pyridin-2-yl}pyridin-3-yl)oxy]ethyl}ethenesulfonamide

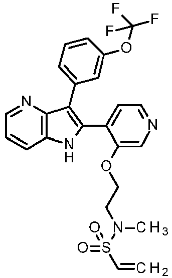

To a solution of N-methyl-2-[(4-{3-[3-(trifluoromethoxy)phenyl]-1H-pyrrolo[3,2-b]pyridin-2-yl}pyridin-3-yl)oxy]ethan-1-amine (70.0 mg, 163 µmol) and triethylamine (80 µl, 570 µmol;) in DCM (1 ml), 2-chloroethane-1-sulfonyl chloride (17 µl, 160 µmol) was added at rt. After stirring at rt for 3 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding the desired compound with insufficient purity. Further purification was done by thin layer chromatography (Eluent DCM / MeOH 10:1) to yield 23.8 mg (100 % purity, 28 % yield) of the title compound.
LC-MS (method 1): Rₜ = 1.44 min; MS (ESlpos): m/z = 519 [M+H]⁺
¹H-NMR (400 MHz, DMSO-d6) δ [ppm]: 11.91 (s, 1H), 8.54 (s, 1H), 8.49-8.43 (m, 1H), 8.39-8.32 (m, 1H), 7.90-7.82 (m, 1H), 7.67-7.59 (m, 1H), 7.56-7.41 (m, 3H), 7.30-7.15 (m, 2H), 6.58-6.49 (m, 1H), 5.98-5.84 (m, 2H), 4.12-4.04 (m, 2H), 3.05-2.98 (m, 2H), 2.47 (s, 3H)

### Example 75

### N-[2-({4-[3-(3,5-dichlorophenyl)-1H-pyrrolo[3,2-b]pyridin-2-yl]pyridin-3-yl}oxy)ethyl]-N-methylethenesulfonamide

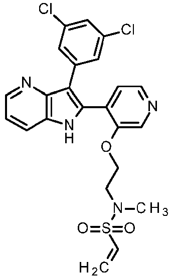

To a solution of 2-({4-[3-(3,5-dichlorophenyl)-1H-pyrrolo[3,2-b]pyridin-2-yl]pyridin-3-yl}oxy)-N-methylethan-1-amine (54.0 mg, 131 µmol) and triethylamine (64 µl, 460 µmol) in DCM (1 ml), 2-chloroethane-1-sulfonyl chloride (14 µl, 130 µmol) was added at rt. After stirring at rt for 3 h, water (0.5 ml) and saturated NaHCO₃ solution (aqueous, 0.5 mL) were added. 2 ml of DCM were added, and the mixture was dried over a water-repellent filter. After concentration under reduced pressure the residue was purified by reverse phase preparative HPLC (method 3) yielding 29.7 mg (100 % purity, 45 % yield) of the title compound.
LC-MS (method 1): Rₜ = 1.59 min; MS (ESlpos): m/z = 503 [M+H]⁺
¹H-NMR (400 MHz, DMSO-d6) δ [ppm]: 12.01 (s, 1H), 8.56 (s, 1H), 8.53-8.46 (m, 1H), 8.42-8.37 (m, 1H), 7.90-7.85 (m, 1H), 7.63-7.55 (m, 2H), 7.52-7.47 (m, 1H), 7.45-7.41 (m, 1H), 7.31-7.24 (m, 1H), 6.59-6.50 (m, 1H), 6.00-5.86 (m, 2H), 4.16-4.09 (m, 2H), 3.09-3.02 (m, 2H), 2.50 (s, 3H)

### Example 76

### 1-[(2S)-2-{[(4-{3-[2-fluoro-5-(trifluoromethyl)phenyl]-1H-pyrrolo[3,2-b]pyridin-2-yl}pyridin-3-yl)oxy]methyl}pyrrolidin-1-yl]prop-2-en-1-one

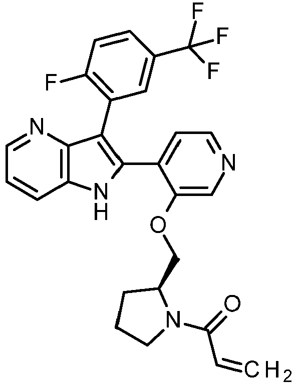

To a solution of 3-[2-fluoro-5-(trifluoromethyl)phenyl]-2-(3-{[(2S)-pyrrolidin-2-yl]methoxy}pyridin-4-yl)-1H-pyrrolo[3,2-b]pyridine (37.0 mg, 81.1 µmol) in DMF (1 ml), prop-2-enoic acid (5.6 µl, 81 µmol) and N,N-diisopropylethylamine (42 µl, 240 µmol) were added at rt. T3P (72 µl, 50 % purity in DMF, 120 µmol) was added and stirring at rt was continued for 3 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 18.9 mg (97 % purity, 44 % yield) of the desired product.
LC-MS (method 1): Rₜ = 1.35 min; MS (ESlpos): m/z = 511 [M+H]⁺
¹H-NMR (400 MHz, DMSO-d6) δ [ppm]: -0.011 (1.57), 0.005 (2.88), 1.525 (2.74), 1.662 (10.25), 1.680 (7.00), 1.717 (2.61), 1.737 (1.62), 2.365 (0.86), 2.709 (0.80), 3.196 (1.22), 3.218 (1.28), 3.271 (0.95), 3.385 (3.91), 3.788 (0.89), 3.804 (1.78), 3.824 (3.33), 3.923 (3.16), 3.941 (4.56), 3.946 (5.11), 3.964 (5.44), 4.114 (3.33), 4.164 (4.47), 4.174 (3.60), 4.188 (3.64), 4.197 (3.03), 5.011 (1.97), 5.018 (1.87), 5.037 (1.93), 5.043 (2.24), 5.642 (5.04), 5.647 (5.02), 5.667 (4.96), 5.673 (5.71), 5.752 (10.02), 5.849 (1.64), 5.855 (1.92), 5.890 (2.20), 5.896 (2.15), 6.115 (4.37), 6.121 (4.64), 6.157 (5.66), 6.162 (5.85), 6.211 (1.85), 6.237 (1.85), 6.253 (1.65), 6.279 (1.49), 6.451 (5.20), 6.477 (5.26), 6.493 (4.39), 6.518 (3.81), 7.253 (5.76), 7.264 (8.05), 7.273 (7.20), 7.285 (8.90), 7.292 (11.00), 7.304 (10.44), 7.361 (4.16), 7.373 (6.40), 7.393 (6.64), 7.416 (5.44), 7.438 (1.56), 7.717 (4.19), 7.725 (3.96), 7.891 (3.68), 7.898 (7.24), 7.901 (7.52), 7.912 (3.66), 7.918 (6.85), 7.922 (6.61), 8.072 (4.18), 8.083 (4.26), 8.108 (2.03), 8.118 (1.98), 8.239 (10.95), 8.251 (10.56), 8.292 (4.38), 8.304 (4.25), 8.431 (6.66), 8.434 (7.32), 8.443 (7.25), 8.446 (7.49), 8.465 (3.46), 8.472 (6.94), 8.577 (16.00), 12.052 (3.56).

### Example 77

### 1-{(2S)-2-[({4-[3-(2-fluoro-5-methylphenyl)-1H-pyrrolo[3,2-b]pyridin-2-yl]pyridin-3-yl}oxy)methyl]pyrrolidin-1-yl}prop-2-en-1-one

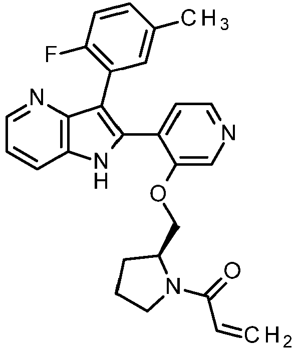

To a solution of 3-(2-fluoro-5-methylphenyl)-2-(3-{[(2S)-pyrrolidin-2-yl]methoxy}pyridin-4-yl)-1H-pyrrolo[3,2-b]pyridine (36.0 mg, 89.4 µmol) in DMF (1 ml), prop-2-enoic acid (6.1 µl, 89 µmol) and N,N-diisopropylethylamine (47 µl, 270 µmol) were added at rt. T3P (79 µl, 50 % purity in DMF, 130 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 14.1 mg (100 % purity, 35 % yield) of the desired product.
LC-MS (method 1): Rₜ = 1.06 min; MS (ESlpos): m/z = 457 [M+H]⁺
¹H-NMR (600 MHz, DMSO-d6) δ [ppm]: 1.671 (0.93), 1.690 (1.84), 1.697 (2.11), 1.709 (2.04), 1.714 (1.89), 1.726 (1.44), 1.732 (1.39), 1.745 (0.99), 1.757 (0.83), 1.778 (0.91), 1.792 (0.92), 1.806 (0.63), 1.827 (0.48), 2.298 (16.00), 2.516 (0.92), 2.519 (0.86), 2.523 (0.69), 3.229 (0.51), 3.361 (1.00), 3.367 (1.11), 3.380 (1.33), 3.390 (1.21), 3.400 (1.13), 3.412 (1.45), 3.425 (0.88), 3.831 (0.46), 3.842 (0.60), 3.846 (0.66), 3.858 (0.65), 3.957 (0.54), 3.966 (0.72), 3.972 (0.44), 3.982 (0.59), 4.021 (0.66), 4.031 (0.61), 4.044 (1.39), 4.054 (1.44), 4.060 (1.49), 4.070 (1.48), 4.224 (1.38), 4.231 (1.65), 4.240 (1.14), 4.247 (1.39), 4.292 (1.08), 5.026 (0.76), 5.030 (0.71), 5.043 (0.74), 5.048 (0.79), 5.672 (1.94), 5.676 (1.84), 5.689 (1.86), 5.692 (2.01), 5.756 (5.87), 5.877 (0.73), 5.882 (0.73), 5.905 (0.83), 5.909 (0.80), 6.158 (1.77), 6.161 (1.77), 6.186 (2.07), 6.190 (2.04), 6.300 (0.70), 6.317 (0.73), 6.327 (0.66), 6.345 (0.61), 6.507 (1.88), 6.524 (1.87), 6.535 (1.65), 6.552 (1.55), 7.001 (1.75), 7.015 (2.97), 7.031 (2.22), 7.122 (1.66), 7.126 (1.68), 7.165 (2.95), 7.173 (2.98), 7.210 (2.01), 7.218 (2.22), 7.224 (2.21), 7.228 (1.22), 7.232 (2.36), 7.241 (0.96), 7.247 (1.32), 7.255 (1.31), 7.392 (1.49), 7.404 (1.52), 7.443 (0.68), 7.455 (0.68), 7.844 (1.03), 7.858 (1.02), 7.879 (2.36), 7.881 (2.33), 7.892 (2.27), 7.894 (2.17), 8.161 (2.35), 8.169 (2.27), 8.235 (1.06), 8.243 (1.02), 8.378 (2.40), 8.380 (2.40), 8.385 (2.43), 8.387 (2.29), 8.399 (1.08), 8.407 (1.07), 8.456 (1.81), 8.560 (3.94), 11.824 (3.32), 11.839 (1.56).

### Example 78

### 1-{(2S)-2-[({4-[3-(3-ethylphenyl)-1H-pyrrolo[3,2-b]pyridin-2-yl]pyridin-3-yl}oxy)methyl]pyrrolidin-1-yl}prop-2-en-1-one

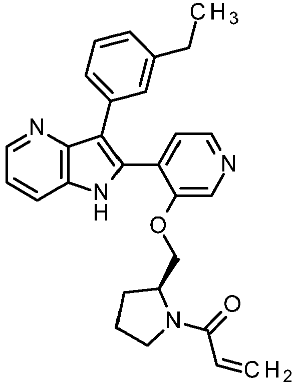

To a solution of 3-(3-ethylphenyl)-2-(3-{[(2S)-pyrrolidin-2-yl]methoxy}pyridin-4-yl)-1H-pyrrolo[3,2-b]pyridine (35.0 mg, 87.8 µmol) in DMF (1 ml), prop-2-enoic acid (6.0 µl, 88 µmol) and N,N-diisopropylethylamine (46 µl, 260 µmol) were added at rt. T3P (78 µl, 50 % purity in DMF, 130 µmol;) was added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 15.0 mg (100 % purity, 38 % yield) of the desired product.
LC-MS (method 1): Rₜ = 1.16 min; MS (ESlpos): m/z = 453 [M+H]⁺
¹H-NMR (400 MHz, DMSO-d6) δ [ppm]: -0.011 (1.88), 1.029 (7.21), 1.048 (16.00), 1.067 (7.87), 1.088 (2.60), 1.105 (1.26), 1.230 (0.40), 1.510 (1.17), 1.548 (2.15), 1.568 (2.81), 1.582 (2.07), 1.601 (1.55), 1.632 (1.90), 1.659 (1.56), 2.366 (0.62), 2.473 (2.26), 2.709 (0.62), 3.166 (1.96), 3.205 (1.25), 3.227 (1.45), 3.244 (1.21), 3.289 (2.13), 3.300 (4.16), 3.305 (4.56), 3.378 (5.53), 3.389 (4.14), 3.407 (1.91), 3.427 (1.23), 3.455 (0.61), 3.675 (0.66), 3.698 (0.99), 3.714 (1.00), 3.835 (0.86), 3.928 (0.89), 3.951 (0.89), 4.021 (0.78), 4.036 (1.59), 4.044 (1.62), 4.058 (1.28), 4.174 (2.92), 4.194 (1.56), 4.203 (1.23), 4.886 (1.02), 4.912 (1.15), 5.610 (2.12), 5.616 (2.06), 5.635 (2.09), 5.641 (2.42), 5.805 (0.91), 5.810 (0.93), 5.846 (1.12), 5.852 (1.17), 6.082 (1.84), 6.088 (1.88), 6.124 (2.32), 6.130 (2.43), 6.233 (1.06), 6.259 (1.09), 6.274 (0.88), 6.299 (0.84), 6.386 (2.16), 6.412 (2.16), 6.428 (1.78), 6.454 (1.53), 7.008 (1.91), 7.027 (2.42), 7.059 (1.37), 7.178 (2.12), 7.197 (5.49), 7.207 (3.33), 7.217 (5.08), 7.228 (3.30), 7.241 (1.79), 7.310 (3.02), 7.322 (5.73), 7.367 (2.30), 7.400 (1.49), 7.415 (2.44), 7.426 (4.52), 7.445 (2.07), 7.815 (4.18), 7.835 (3.85), 8.251 (1.76), 8.262 (1.82), 8.319 (1.12), 8.330 (1.11), 8.419 (2.67), 8.422 (3.02), 8.430 (3.07), 8.434 (3.16), 8.453 (2.76), 8.586 (2.65), 11.725 (3.22), 11.761 (1.87).

### Example 79

### 1-{(2S)-2-[({4-[3-(quinolin-7-yl)-1H-pyrrolo[3,2-b]pyridin-2-yl]pyridin-3-yl}oxy)methyl]pyrrolidin-1-yl}prop-2-en-1-one

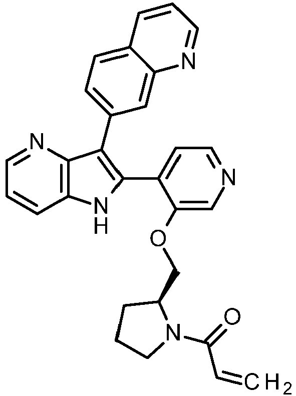

To a solution of 7-[2-(3-{[(2S)-pyrrolidin-2-yl]methoxy}pyridin-4-yl)-1H-pyrrolo[3,2-b]pyridin-3-yl]quinoline (35.0 mg, 83.0 µmol) in DMF (1 ml), prop-2-enoic acid (5.7 µl, 83 µmol) and N,N-diisopropylethylamine (43 µl, 250 µmol) were added at rt. T3P (73 µl, 50 % purity in DMF, 120 µmol) was added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 19.0 mg (100 % purity, 48 % yield) of the desired product.
LC-MS (method 1): Rₜ = 0.92 min; MS (ESlpos): m/z = 476 [M+H]⁺
¹H-NMR (400 MHz, DMSO-d6) δ [ppm]: -0.149 (0.70), 0.146 (0.67), 1.331 (2.40), 1.353 (1.80), 1.440 (5.18), 1.458 (5.64), 1.472 (5.22), 1.488 (4.63), 1.507 (4.04), 1.527 (3.08), 1.546 (3.19), 2.369 (0.77), 2.713 (0.76), 3.146 (1.90), 3.169 (2.82), 3.180 (2.96), 3.199 (2.42), 3.222 (2.15), 3.242 (5.21), 3.266 (3.68), 3.288 (2.61), 3.305 (2.49), 3.315 (4.22), 3.391 (3.08), 3.402 (1.97), 3.417 (1.40), 3.441 (1.29), 3.467 (0.69), 3.601 (0.48), 3.691 (3.97), 3.711 (2.70), 3.726 (1.50), 3.895 (1.43), 3.908 (1.97), 3.916 (1.92), 3.929 (1.17), 4.020 (2.37), 4.035 (3.89), 4.043 (2.98), 4.059 (5.10), 4.101 (3.18), 4.165 (4.41), 4.173 (3.95), 4.188 (3.40), 4.195 (2.78), 4.858 (2.42), 4.865 (2.26), 4.884 (2.29), 4.890 (2.49), 5.551 (4.88), 5.557 (4.67), 5.576 (4.82), 5.582 (5.45), 5.769 (1.95), 5.776 (2.06), 5.811 (2.91), 5.818 (2.86), 6.021 (2.67), 6.030 (4.38), 6.036 (4.36), 6.047 (2.63), 6.063 (2.27), 6.071 (5.84), 6.077 (5.82), 6.088 (1.87), 6.307 (5.20), 6.333 (5.14), 6.349 (4.21), 6.375 (3.62), 7.263 (5.46), 7.274 (8.12), 7.284 (7.03), 7.295 (8.65), 7.306 (3.04), 7.335 (0.63), 7.413 (9.84), 7.425 (11.05), 7.439 (6.72), 7.449 (6.53), 7.460 (7.02), 7.472 (3.11), 7.490 (5.10), 7.502 (5.14), 7.791 (3.54), 7.795 (3.52), 7.813 (7.83), 7.816 (8.08), 7.843 (12.30), 7.854 (4.89), 7.858 (4.98), 7.864 (6.00), 7.870 (7.43), 7.874 (7.79), 7.885 (7.84), 7.891 (7.25), 7.894 (7.36), 8.261 (5.27), 8.281 (10.90), 8.297 (12.39), 8.309 (12.68), 8.336 (10.44), 8.352 (5.84), 8.364 (5.51), 8.498 (15.05), 8.509 (10.42), 8.512 (9.92), 8.520 (4.06), 8.604 (0.85), 8.632 (16.00), 8.816 (6.13), 8.820 (7.31), 8.826 (9.33), 11.942 (1.99).

### Example 80

### 1-{(2S)-2-[({4-[3-(naphthalen-2-yl)-1H-pyrrolo[3,2-b]pyridin-2-yl]pyridin-3-yl}oxy)methyl]pyrrolidin-1-yl}prop-2-en-1-one

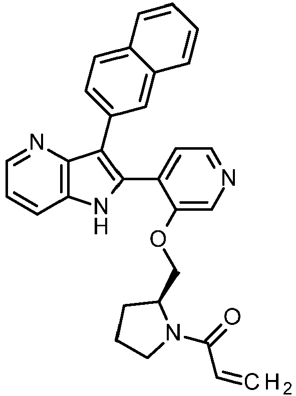

To a solution of 3-(naphthalen-2-yl)-2-(3-{[(2S)-pyrrolidin-2-yl]methoxy}pyridin-4-yl)-1H-pyrrolo[3,2-b]pyridine (35.0 mg, 83.2 µmol) in DMF (1 ml), prop-2-enoic acid (5.7 µl, 83 µmol) and N,N-diisopropylethylamine (43 µl, 250 µmol) were added at rt. T3P (74 µl, 50 % purity, 120 µmol) was added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 23.0 mg (100 % purity, 58 % yield) of the desired product.
LC-MS (method 1): Rₜ = 1.27 min; MS (ESlpos): m/z = 475 [M+H]⁺
¹H-NMR (400 MHz, DMSO-d6) δ [ppm]: -0.150 (0.56), 0.146 (0.62), 1.073 (0.53), 1.090 (1.02), 1.108 (0.56), 1.233 (0.47), 1.445 (3.13), 1.470 (6.09), 1.486 (5.42), 1.510 (4.92), 1.525 (5.21), 1.543 (4.95), 1.560 (5.42), 1.578 (4.49), 2.369 (0.75), 2.712 (0.76), 3.125 (1.33), 3.177 (1.89), 3.219 (2.91), 3.232 (2.58), 3.247 (2.53), 3.256 (3.20), 3.274 (4.99), 3.298 (4.71), 3.409 (2.48), 3.684 (4.44), 3.701 (2.92), 3.717 (1.34), 3.875 (2.22), 3.884 (2.09), 4.052 (2.24), 4.066 (3.65), 4.074 (3.67), 4.089 (3.87), 4.176 (3.36), 4.195 (6.63), 4.217 (3.55), 4.225 (2.67), 4.861 (2.47), 4.868 (2.40), 4.887 (2.47), 4.893 (2.74), 5.571 (4.81), 5.577 (4.87), 5.597 (4.67), 5.603 (5.55), 5.772 (1.95), 5.779 (2.20), 5.814 (2.91), 5.821 (2.97), 6.002 (2.46), 6.028 (2.58), 6.044 (1.96), 6.060 (4.26), 6.066 (4.94), 6.102 (5.63), 6.108 (5.79), 6.347 (5.12), 6.373 (5.07), 6.389 (4.12), 6.415 (3.57), 7.241 (5.57), 7.252 (7.43), 7.261 (6.54), 7.272 (8.01), 7.285 (3.29), 7.329 (9.66), 7.342 (9.87), 7.428 (1.81), 7.446 (13.77), 7.455 (14.67), 7.464 (14.31), 7.470 (8.96), 7.557 (4.84), 7.561 (5.37), 7.579 (5.95), 7.583 (6.76), 7.612 (3.56), 7.777 (14.72), 7.798 (12.93), 7.808 (5.98), 7.830 (7.79), 7.853 (6.94), 7.862 (12.96), 7.865 (13.26), 7.882 (10.46), 7.886 (10.65), 8.189 (5.80), 8.213 (10.02), 8.236 (11.21), 8.248 (10.72), 8.320 (5.90), 8.331 (5.62), 8.468 (9.77), 8.471 (9.54), 8.475 (8.29), 8.483 (7.24), 8.486 (8.02), 8.500 (4.06), 8.504 (3.76), 8.614 (16.00), 11.832 (1.46).

### Example 81

### 1-{(2S)-2-[({4-[3-(1-benzothiophen-6-yl)-1H-pyrrolo[3,2-b]pyridin-2-yl]pyridin-3-yl}oxy)methyl]pyrrolidin-1-yl}prop-2-en-1-one

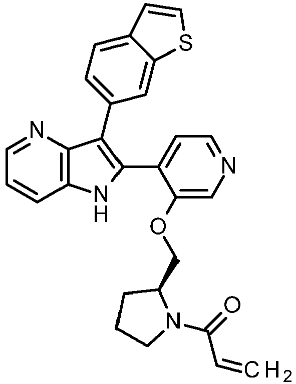

To a solution of 3-(1-benzothiophen-6-yl)-2-(3-{[(2S)-pyrrolidin-2-yl]methoxy}pyridin-4-yl)-1H-pyrrolo[3,2-b]pyridine (35.0 mg, 82.1 µmol) in DMF (1 ml), prop-2-enoic acid (5.6 µl, 82 µmol) and N,N-diisopropylethylamine (43 µl, 250 µmol) were added at rt. T3P (73 µl, 50 % purity in DMF, 120 µmol) was added and stirring at rt was continued for 1.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 20.0 mg (91 % purity, 46 % yield) of the desired product.
LC-MS (method 1): Rₜ = 1.19 min; MS (ESlpos): m/z = 481 [M+H]⁺
¹H-NMR (400 MHz, DMSO-d6) δ [ppm]: 11.83 and 11.79 (2s, 1H), 8.61 and 8.47 (2s, 1H), 8.50-8.44 (m, 1H), 8.35-8.23 (m, 2H), 7.89-7.82 (m, 1H), 7.80-7.73 (m, 1H), 7.71-7.67 (m, 1H), 7.46-7.21 (m, 4H), 6.43-6.36, 6.14-6.07, 5.86-5.79, 5.63-5.58 and 4.96-4.91 (5m, 3H), 4.23-4.02 (m, 2H), 3.94-3.87 and 3.75-3.69 (2m, 1H), 3.31-3.11 (m, 2H), 1.68-1.42 (m, 4H)

### Example 82

### 1-{(2S)-2-[({4-[3-(1-benzofuran-6-yl)-1H-pyrrolo[3,2-b]pyridin-2-yl]pyridin-3-yl}oxy)methyl]pyrrolidin-1-yl}prop-2-en-1-one

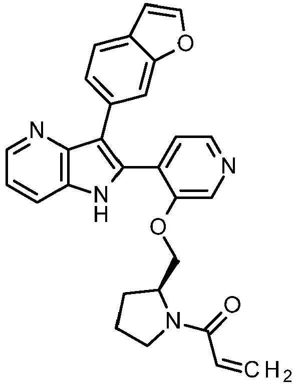

To a solution of 3-(1-benzofuran-6-yl)-2-(3-{[(2S)-pyrrolidin-2-yl]methoxy}pyridin-4-yl)-1H-pyrrolo[3,2-b]pyridine (50.0 mg, 122 µmol) in THF (1 ml), prop-2-enoic acid (8.4 µl, 120 µmol) and N,N-diisopropylethylamine (64 µl, 370 µmol) were added at rt. T3P (110 µl, 50 % purity in EtOAc, 180 µmol) was added and stirring at rt was continued for 3 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 32.8 mg (97 % purity, 56 % yield) of the desired product.
LC-MS (method 2): Rₜ = 1.12 min; MS (ESlpos): m/z = 465 [M+H]⁺
¹H-NMR (400 MHz, DMSO-d6) δ [ppm]: 11.80 and 11.77 (2s, 1H), 8.61 and 8.48 (2s, 1H), 8.48 - 8.43 (m, 1H), 8.34-8.24 (m, 1H), 7.96-7.81 (m, 3H), 7.58-7.50 (m, 1H), 7.45-7.31 (m, 2H), 7.27-7.21 (m, 1H), 6.95-6.89 (m, 1H), 6.43-6.36 and 6.21-6.14 (2m, 1H), 6.12-6.07 and 5.86-5.80 (2m, 1H), 5.63-5.58 and 4.96-4.92 (2m, 1H), 4.23-3.72 (m, 3H), 3.32-3.25 (m, 1H), 3.23-3.12 (m, 1H), 1.70-1.42 (m, 4H)

### Example 83

### 1-{(2S)-2-[({4-[3-(3-methoxyphenyl)-1H-pyrrolo[3,2-b]pyridin-2-yl]pyridin-3-yl}oxy)methyl]pyrrolidin-1-yl}prop-2-en-1-one

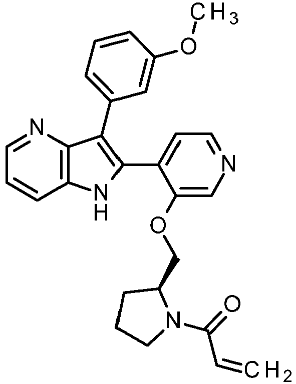

To a solution of 3-(3-methoxyphenyl)-2-(3-{[(2S)-pyrrolidin-2-yl]methoxy}pyridin-4-yl)-1H-pyrrolo[3,2-b]pyridine (50.0 mg, 125 µmol) in THF (1 ml), prop-2-enoic acid (8.6 µl, 120 µmol) and N,N-diisopropylethylamine (65 µl, 370 µmol) were added at rt. T3P (110 µl, 50 % purity in EtOAc, 190 µmol) was added and stirring at rt was continued for 3 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 37.0 mg (100 % purity, 65 % yield) of the desired product.
LC-MS (method 2): Rₜ = 1.05 min; MS (ESlpos): m/z = 455 [M+H]⁺
¹H-NMR (400 MHz, DMSO-d6) δ [ppm]: 11.79 and 11.76 (2s, 1H), 8.60 and 8.49 (2s, 1H), 8.46-8.41 (m, 1H), 8.35-8.25 (m, 1H), 7.86-7.80 (m, 1H), 7.44-7.31 (m, 1H), 7.26-7.11 (m, 4H), 6.82-6.73 (m, 1H), 6.45-6.24 (m, 1H), 6.14-5.80 (m, 1H), 5.65-5.59 and 4.93-4.89 (2m, 1H), 4.23-3.75 (m, 3H), 3.63 and 3.62 (2s, 3H), 3.32-3.26 (m, 1H), 3.23-3.13 (m, 1H), 1.80-1.49 (m, 4H)

### Example 84

### 1-{(2S)-2-[({4-[3-(1H-indol-6-yl)-1H-pyrrolo[3,2-b]pyridin-2-yl]pyridin-3-yl}oxy)methyl]pyrrolidin-1-yl}prop-2-en-1-one

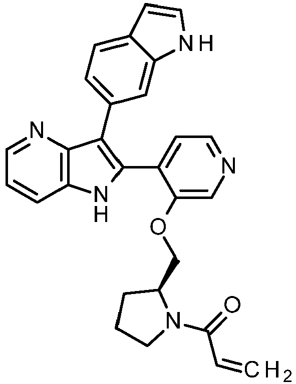

To a solution of 3-(1H-indol-6-yl)-2-(3-{[(2S)-pyrrolidin-2-yl]methoxy}pyridin-4-yl)-1H-pyrrolo[3,2-b]pyridine (35.0 mg, 85.5 µmol) in DMF (1 ml), prop-2-enoic acid (5.9 µl, 85 µmol) and N,N-diisopropylethylamine (45 µl, 260 µmol) were added at rt. T3P (76 µl, 50 % purity in DMF, 130 µmol) was added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding the title compound with insufficient purity. Further purification by thin layer chromatograpy (Eluent: DCM / MeOH 10:1) gave 1.40 mg (96 % purity, 3 % yield) of the desired product.
LC-MS (method 1): Rₜ = 1.11 min; MS (ESlpos): m/z = 464 [M+H]⁺
¹H-NMR (400 MHz, MeOD) δ [ppm]: 8.51-8.04 (m, 4H), 8.00-7.85 (m, 2H), 7.59-7.03 (m, 6H), 6.81-6.73 (m, 1H), 6.46-6.34 (m, 1H), 6.23-6.12 (m, 1H), 5.67-5.56 (m, 1H), 4.63-4.53 (m, 1H), 4.44-4.20 (m, 2H), 3.51-3.11 (m, 2H), 1.80-1.49 (m, 4H)

### Example 85

### 1-{(2S)-2-[({4-[3-(2,3-dichlorophenyl)-1H-pyrrolo[3,2-b]pyridin-2-yl]pyridin-3-yl}oxy)methyl]pyrrolidin-1-yl}prop-2-en-1-one

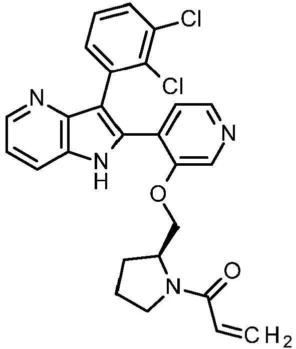

To a solution of 3-(2,3-dichlorophenyl)-2-(3-{[(2S)-pyrrolidin-2-yl]methoxy}pyridin-4-yl)-1H-pyrrolo[3,2-b]pyridine (35.0 mg, 89 % purity, 70.9 µmol) in DMF (1 ml), prop-2-enoic acid (4.9 µl, 71 µmol) and N,N-diisopropylethylamine (37 µl, 210 µmol) were added at rt. T3P (63 µl, 50 % purity in DMF, 110 µmol) was added and stirring at rt was continued for 1.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 21.0 mg (100 % purity, 60 % yield) of the desired product.
LC-MS (method 1): Rₜ = 1.22 min; MS (ESlpos): m/z = 493 [M+H]⁺
¹H-NMR (600 MHz, DMSO-d6) δ [ppm]: 11.94-11.86 (m, 1H), 8.62-8.44 (m, 1H), 8.38-8.29 (m, 1H), 8.23-8.11 (m, 1H), 7.94-7.82 (m, 1H), 7.65-7.55 (m, 1H), 7.40-7.29 (m, 2H), 7.27-7.19 (m, 1H), 7.16-7.02 (m, 1H), 6.61-6.32 (m, 1H), 6.24-6.14 and 5.93-5.87 (2m,1H), 5.72-5.66 and 5.09-5.01 (2m, 1H), 4.42-3.92 (m, 3H), 3.51-3.38 (m, 1H), 3.29-3.19 (m, 1H), 1.97-1.64 (m, 4H)

### Example 86

### 1-{(2S)-2-[({4-[3-(2,3-difluorophenyl)-1H-pyrrolo[3,2-b]pyridin-2-yl]pyridin-3-yl}oxy)methyl]pyrrolidin-1-yl}prop-2-en-1-one

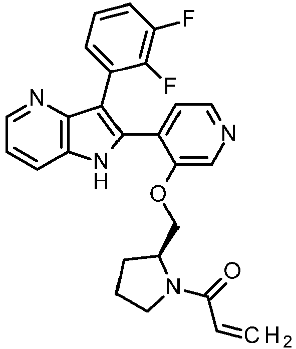

To a solution of 3-(2,3-difluorophenyl)-2-(3-{[(2S)-pyrrolidin-2-yl]methoxy}pyridin-4-yl)-1H-pyrrolo[3,2-b]pyridine (35.0 mg, 86.1 µmol) in DMF (1 ml), prop-2-enoic acid (5.9 µl, 86 µmol) and N,N-diisopropylethylamine (45 µl, 260 µmol) were added at rt. T3P (76 µl, 50 % purity in DMF, 130 µmol) was added and stirring at rt was continued for 1.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 19.0 mg (100 % purity, 48 % yield) of the desired product.
LC-MS (method 1): Rₜ = 1.08 min; MS (ESlpos): m/z = 461 [M+H]⁺
¹H-NMR (600 MHz, DMSO-d6) δ [ppm]: 1.398 (0.47), 1.663 (10.03), 1.676 (8.66), 1.690 (6.46), 1.719 (5.78), 1.733 (5.97), 1.758 (4.05), 1.825 (1.86), 2.383 (1.42), 2.423 (0.70), 2.612 (1.80), 2.652 (0.97), 3.883 (2.75), 3.894 (2.26), 3.985 (2.83), 4.029 (8.79), 4.208 (12.06), 4.218 (9.21), 4.996 (2.64), 5.013 (2.54), 5.663 (6.04), 5.680 (6.24), 5.753 (12.50), 5.757 (6.35), 5.861 (2.56), 5.888 (2.94), 6.143 (5.49), 6.171 (6.38), 6.280 (1.77), 6.297 (1.99), 6.307 (1.72), 6.324 (1.47), 6.487 (4.16), 6.504 (4.41), 6.515 (3.87), 6.532 (3.34), 7.211 (7.08), 7.235 (16.00), 7.242 (15.56), 7.257 (8.15), 7.287 (4.37), 7.331 (11.30), 7.871 (3.49), 7.888 (8.52), 7.902 (6.93), 8.215 (10.07), 8.223 (8.70), 8.266 (4.58), 8.273 (3.97), 8.396 (9.36), 8.401 (8.97), 8.482 (6.00), 8.576 (13.76), 11.981 (9.82), 12.006 (4.24).

### Example 87

### 3-[2-(3-{[(2S)-1-(prop-2-enoyl)pyrrolidin-2-yl]methoxy}pyridin-4-yl)-1H-pyrrolo[3,2-b]pyridin-3-yl]benzonitrile

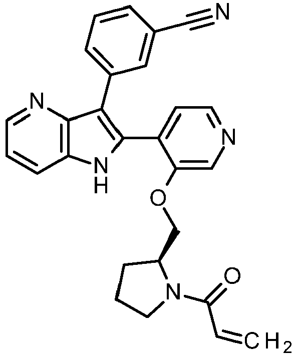

To a solution of 3-[2-(3-{[(2S)-pyrrolidin-2-yl]methoxy}pyridin-4-yl)-1H-pyrrolo[3,2-b]pyridin-3-yl]benzonitrile (37.0 mg, 86 % purity, 80.5 µmol) in THF (1 ml), prop-2-enoic acid (5.5 µl, 80 µmol) and N,N-diisopropylethylamine (42 µl, 240 µmol) were added at rt. T3P (71 µl, 50 % purity in DMF, 120 µmol) was added and stirring at rt was continued for 1.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 12.0 mg (100 % purity, 33 % yield) of the desired product.
LC-MS (method 1): Rₜ = 1.09 min; MS (ESlpos): m/z = 450 [M+H]⁺
¹H-NMR (400 MHz, DMSO-d6) δ [ppm]: -0.150 (0.58), 0.146 (0.65), 1.175 (0.69), 1.415 (2.16), 1.556 (5.83), 1.573 (4.92), 1.617 (3.70), 1.989 (1.07), 3.174 (2.70), 3.198 (3.16), 3.810 (1.71), 3.829 (1.65), 3.851 (1.70), 3.897 (1.40), 3.971 (1.49), 3.988 (1.54), 4.016 (2.33), 4.032 (3.55), 4.054 (4.22), 4.090 (2.91), 4.167 (3.72), 4.190 (2.92), 4.947 (1.71), 4.979 (1.72), 5.601 (4.00), 5.607 (3.61), 5.627 (4.00), 5.633 (4.29), 5.756 (16.00), 5.809 (1.60), 5.850 (1.88), 5.857 (1.88), 6.067 (3.61), 6.073 (3.53), 6.109 (4.64), 6.115 (4.45), 6.213 (1.48), 6.239 (1.48), 6.255 (1.46), 6.280 (1.15), 6.362 (3.55), 6.388 (3.69), 6.404 (2.85), 6.430 (2.54), 7.257 (4.28), 7.269 (5.41), 7.277 (5.98), 7.289 (5.10), 7.409 (6.74), 7.421 (7.03), 7.449 (3.30), 7.461 (5.73), 7.481 (6.68), 7.501 (4.84), 7.508 (3.74), 7.527 (1.86), 7.634 (4.95), 7.654 (4.52), 7.685 (1.84), 7.729 (4.66), 7.748 (6.06), 7.768 (2.01), 7.862 (7.87), 7.882 (7.10), 8.109 (3.82), 8.153 (8.21), 8.329 (7.51), 8.341 (7.44), 8.361 (3.68), 8.373 (3.30), 8.485 (6.88), 8.493 (8.08), 8.531 (5.00), 8.633 (10.75), 11.984 (6.69), 12.007 (3.21).

### Example 88

### 1-{(2S)-2-[({4-[3-(2-chlorophenyl)-1H-pyrrolo[3,2-b]pyridin-2-yl]pyridin-3-yl}oxy)methyl]pyrrolidin-1-yl}prop-2-en-1-one

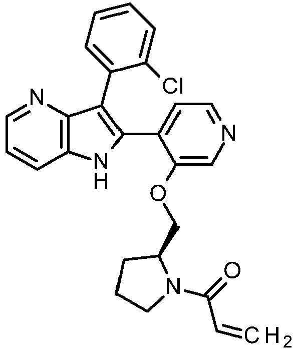

To a solution of 3-(2-chlorophenyl)-2-(3-{[(2S)-pyrrolidin-2-yl]methoxy}pyridin-4-yl)-1H-pyrrolo[3,2-b]pyridine (35.0 mg, 86.4 µmol) in DMF (1 ml), prop-2-enoic acid (5.9 µl, 86 µmol) and N,N-diisopropylethylamine (45 µl, 260 µmol) were added at rt. T3P (76 µl, 50 % purity in DMF, 130 µmol) was added and stirring at rt was continued for 1.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 19.0 mg (91 % purity, 44 % yield) of the desired product.
LC-MS (method 1): Rₜ = 1.05 min; MS (ESlpos): m/z = 459 [M+H]⁺
¹H-NMR (400 MHz, DMSO-d6) δ [ppm]: 11.87-11.77 (m, 1H), 8.57 and 8.47 (2s, 1H), 8.37-8.22 (m, 1H), 8.20-8.07 (m, 1H), 7.94-7.81 (m, 1H), 7.52-7.31 (m, 4H), 7.25-7.18 (m, 1H), 7.10-6.99 (m, 1H), 6.62-6.49 and 6.46-6.33 (2m, 1H), 6.24-6.14 and 5.94-5.85 (2m, 1H), 5.73-5.66 and 5.09-4.99 (2m, 1H), 4.47-3.93 (m, 3H), 3.54-3.18 (m, 2H), 1.98-1.63 (m, 4H)

### Example 89

### 1-{(2S)-2-[({4-[3-(2-fluorophenyl)-1H-pyrrolo[3,2-b]pyridin-2-yl]pyridin-3-yl}oxy)methyl]pyrrolidin-1-yl}prop-2-en-1-one

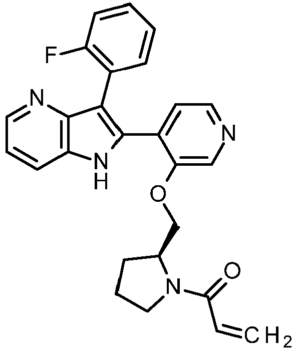

To a solution of 3-(2-fluorophenyl)-2-(3-{[(2S)-pyrrolidin-2-yl]methoxy}pyridin-4-yl)-1H-pyrrolo[3,2-b]pyridine (35.0 mg, 86 % purity, 77.5 µmol) in DMF (1 ml), prop-2-enoic acid (5.3 µl, 77 µmol) and N,N-diisopropylethylamine (40 µl, 230 µmol) were added at rt. T3P (68 µl, 50 % purity in DMF, 120 µmol) was added and stirring at rt was continued for 1.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 18.5 mg (100 % purity, 54 % yield) of the desired product.
LC-MS (method 1): Rₜ = 0.98 min; MS (ESlpos): m/z = 443 [M+H]⁺
¹H-NMR (400 MHz, DMSO-d6) δ [ppm]: -0.150 (0.92), 0.008 (16.00), 0.146 (0.98), 1.233 (0.43), 1.688 (7.87), 1.702 (7.59), 1.718 (6.02), 1.738 (4.69), 3.384 (8.29), 3.400 (7.00), 3.819 (1.03), 3.842 (1.86), 3.857 (1.71), 3.938 (1.36), 3.952 (2.19), 3.974 (2.81), 3.990 (1.94), 4.006 (1.65), 4.022 (3.22), 4.037 (3.64), 4.045 (3.84), 4.060 (3.66), 4.207 (3.35), 4.217 (4.64), 4.241 (5.93), 4.257 (3.33), 5.004 (1.90), 5.036 (2.06), 5.665 (4.57), 5.671 (4.49), 5.690 (4.54), 5.696 (4.94), 5.755 (5.48), 5.857 (1.91), 5.899 (2.26), 5.905 (2.12), 6.145 (4.00), 6.151 (4.20), 6.187 (5.13), 6.193 (5.05), 6.274 (1.86), 6.300 (1.76), 6.316 (1.62), 6.341 (1.45), 6.493 (4.53), 6.519 (4.51), 6.535 (3.80), 6.560 (3.33), 7.132 (4.08), 7.154 (6.91), 7.172 (10.06), 7.184 (10.36), 7.203 (3.75), 7.210 (7.24), 7.221 (13.82), 7.231 (10.35), 7.241 (10.89), 7.257 (4.44), 7.270 (3.92), 7.315 (2.85), 7.329 (5.15), 7.348 (4.41), 7.362 (1.78), 7.545 (2.80), 7.564 (5.13), 7.583 (2.68), 7.614 (2.28), 7.633 (1.26), 7.847 (2.85), 7.876 (6.62), 7.896 (5.79), 8.165 (8.39), 8.177 (8.16), 8.238 (3.68), 8.250 (3.67), 8.374 (6.57), 8.382 (6.82), 8.391 (3.79), 8.403 (3.10), 8.456 (5.89), 8.562 (13.07), 11.858 (8.62), 11.879 (4.15).

### Example 90

### 4-fluoro-3-[2-(3-{[(2S)-1-(prop-2-enoyl)pyrrolidin-2-yl]methoxy}pyridin-4-yl)-1H-pyrrolo[3,2-b]pyridin-3-yl]benzonitrile

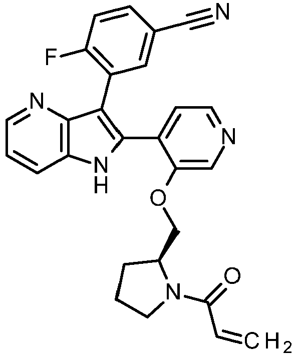

To a solution of 4-fluoro-3-[2-(3-{[(2S)-pyrrolidin-2-yl]methoxy}pyridin-4-yl)-1H-pyrrolo[3,2-b]pyridin-3-yl]benzonitrile (19.0 mg, 86 % purity, 39.5 µmol) in DMF (1 ml), prop-2-enoic acid (3.0 µl, 43 µmol) and N,N-diisopropylethylamine (21 µl, 120 µmol) were added at rt. T3P (35 µl, 50 % purity in DMF, 59 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 8.50 mg (95 % purity, 44 % yield) of the desired product.
LC-MS (method 1): Rₜ = 1.06 min; MS (ESlpos): m/z = 468 [M+H]⁺
¹H-NMR (400 MHz, DMSO-d6) δ [ppm]: -0.149 (0.95), 0.146 (1.07), 1.233 (1.40), 1.369 (1.26), 1.568 (2.72), 1.701 (8.74), 2.367 (0.95), 2.711 (1.01), 3.850 (1.24), 3.893 (1.82), 3.915 (1.45), 3.959 (4.42), 3.981 (6.04), 3.998 (4.18), 4.139 (3.31), 4.177 (4.18), 4.200 (3.40), 4.210 (2.84), 5.053 (1.74), 5.079 (1.82), 5.651 (4.46), 5.657 (4.32), 5.677 (4.36), 5.683 (4.91), 5.755 (16.00), 5.855 (1.70), 5.902 (2.12), 6.126 (3.69), 6.132 (3.95), 6.168 (4.96), 6.174 (4.97), 6.249 (1.63), 6.274 (1.64), 6.289 (1.31), 6.316 (1.26), 6.468 (4.25), 6.494 (4.33), 6.510 (3.67), 6.536 (3.30), 7.263 (5.16), 7.275 (6.52), 7.283 (13.00), 7.295 (12.72), 7.324 (3.07), 7.337 (2.97), 7.386 (3.94), 7.410 (6.20), 7.432 (4.35), 7.862 (4.48), 7.868 (4.24), 7.890 (3.62), 7.909 (7.90), 7.926 (6.10), 8.169 (6.46), 8.180 (5.69), 8.185 (6.53), 8.239 (5.97), 8.251 (6.00), 8.281 (2.74), 8.293 (2.62), 8.439 (6.56), 8.450 (8.46), 8.461 (3.29), 8.495 (3.79), 8.583 (9.41), 12.081 (5.37).

### Example 91

### 2-fluoro-1-[(2S)-2-({[4-(3-phenyl-1H-pyrrolo[3,2-b]pyridin-2-yl)pyridin-3-yl]oxy}methyl)pyrrolidin-1-yl]prop-2-en-1-one

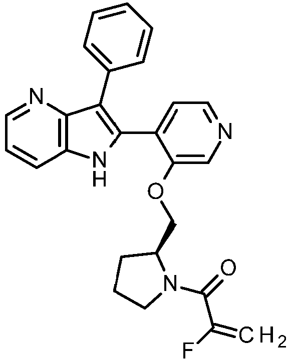

To a solution of 3-phenyl-2-(3-{[(2S)-pyrrolidin-2-yl]methoxy}pyridin-4-yl)-1H-pyrrolo[3,2-b]pyridine hydrogen chloride (1/1) (65.0 mg, 160 µmol) in DMF (1 ml), 2-fluoroprop-2-enoic acid (15.8 mg, 176 µmol) and N,N-diisopropylethylamine (140 µl, 800 µmol) were added at rt. T3P (140 µl, 50 % purity in DMF, 240 µmol) was added and stirring at rt was continued for 15 min. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 37.0 mg (97 % purity, 51 % yield) of the desired product.
LC-MS (method 1): Rₜ = 1.06 min; MS (ESlpos): m/z = 443 [M+H]⁺
¹H-NMR (400 MHz, DMSO-d6) δ [ppm]: 1.521 (2.25), 1.541 (2.27), 1.555 (1.63), 1.718 (0.97), 2.523 (1.10), 3.091 (0.92), 3.287 (0.78), 3.427 (0.61), 4.157 (2.43), 4.177 (2.56), 5.078 (1.14), 5.155 (1.21), 5.199 (2.97), 5.749 (16.00), 7.162 (1.52), 7.165 (1.01), 7.175 (1.13), 7.180 (3.91), 7.185 (1.60), 7.199 (5.72), 7.211 (3.73), 7.220 (3.93), 7.232 (3.88), 7.269 (4.64), 7.288 (7.89), 7.306 (3.96), 7.320 (2.37), 7.332 (2.71), 7.552 (4.24), 7.571 (3.38), 7.791 (2.78), 7.794 (3.07), 7.812 (2.69), 7.815 (2.82), 8.265 (2.40), 8.276 (2.56), 8.415 (4.30), 8.419 (4.63), 8.426 (4.29), 8.430 (4.16), 8.543 (3.41), 11.717 (4.67).

### Example 92

### (2E)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one

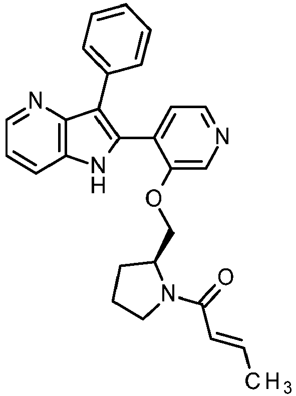

To a solution of 3-phenyl-2-(3-{[(2S)-pyrrolidin-2-yl]methoxy}pyridin-4-yl)-1H-pyrrolo[3,2-b]pyridine (40.0 mg, 108 µmol) in DMF (1 ml), (2E)-but-2-enoic acid (9.30 mg, 108 µmol) and N,N-diisopropylethylamine (56 µl, 320 µmol) were added at rt. T3P (95 µl, 50 % purity in DMF, 160 µmol) was added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 30.0 mg (100 % purity, 63 % yield) of the desired product.
LC-MS (method 1): Rₜ = 1.04 min; MS (ESlpos): m/z = 439 [M+H]⁺
¹H-NMR (400 MHz, DMSO-d6) δ [ppm]: -0.150 (0.47), 0.146 (0.47), 1.073 (0.96), 1.091 (2.00), 1.108 (0.99), 1.502 (6.58), 1.516 (6.98), 1.536 (5.26), 1.553 (5.28), 1.575 (6.12), 1.591 (3.96), 1.624 (2.11), 1.643 (2.63), 1.664 (2.59), 1.780 (12.71), 1.783 (13.83), 1.797 (13.02), 1.800 (13.64), 2.369 (0.52), 2.713 (0.50), 3.132 (1.02), 3.164 (6.25), 3.177 (6.86), 3.195 (2.46), 3.209 (2.69), 3.255 (2.38), 3.274 (4.85), 3.298 (4.01), 3.374 (4.61), 3.392 (2.39), 3.406 (1.94), 3.438 (0.56), 3.468 (0.60), 3.750 (2.27), 3.764 (2.79), 3.935 (0.57), 3.958 (1.50), 3.967 (1.39), 4.027 (1.53), 4.041 (3.26), 4.049 (3.26), 4.064 (2.64), 4.100 (1.19), 4.114 (1.14), 4.158 (3.30), 4.170 (6.27), 4.191 (3.24), 4.199 (2.27), 6.012 (1.32), 6.049 (1.56), 6.073 (3.59), 6.077 (3.85), 6.111 (4.07), 6.114 (4.29), 6.494 (1.10), 6.511 (1.20), 6.532 (1.06), 6.549 (0.95), 6.618 (0.93), 6.635 (3.48), 6.652 (3.58), 6.672 (3.33), 6.689 (3.03), 6.706 (0.80), 7.166 (2.04), 7.185 (5.91), 7.206 (13.12), 7.217 (9.33), 7.226 (10.22), 7.238 (9.34), 7.269 (6.93), 7.288 (12.37), 7.294 (11.15), 7.306 (16.00), 7.325 (4.32), 7.344 (2.19), 7.401 (2.72), 7.413 (2.84), 7.542 (11.34), 7.560 (13.60), 7.578 (3.70), 7.816 (6.95), 7.820 (6.66), 7.837 (6.66), 7.840 (5.96), 8.240 (7.83), 8.252 (7.58), 8.311 (2.69), 8.322 (2.54), 8.418 (6.40), 8.421 (8.76), 8.429 (6.94), 8.432 (8.34), 8.489 (4.19), 8.585 (11.95), 11.744 (7.11).

### Example 93

### 1-[(2S)-2-({[4-(3-phenyl-1H-pyrrolo[3,2-b]pyridin-2-yl)pyridin-3-yl]oxy}methyl)pyrrolidin-1-yl]prop-2-yn-1-one

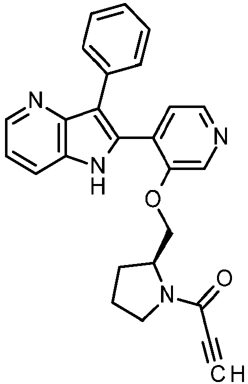

To a solution of 3-phenyl-2-(3-{[(2S)-pyrrolidin-2-yl]methoxy}pyridin-4-yl)-1H-pyrrolo[3,2-b]pyridine (40.0 mg, 108 µmol) in DMF (1 ml), prop-2-ynoic acid (6.7 µl, 110 µmol) and N,N-diisopropylethylamine (56 µl, 320 µmol) were added at rt. T3P (95 µl, 50 % purity in DMF, 160 µmol) was added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 25.0 mg (100 % purity, 55 % yield) of the desired product.
LC-MS (method 1): Rₜ = 0.95 min; MS (ESlpos): m/z = 423 [M+H]⁺
¹H-NMR (400 MHz, DMSO-d6) δ [ppm]: 1.087 (0.51), 1.232 (0.80), 1.482 (3.01), 1.499 (4.93), 1.515 (8.00), 1.529 (6.40), 1.721 (1.98), 1.741 (2.07), 1.772 (1.24), 2.365 (0.87), 2.709 (0.82), 2.984 (1.21), 3.000 (1.36), 3.108 (1.98), 3.119 (1.87), 3.134 (2.48), 3.166 (7.34), 3.210 (1.90), 3.230 (1.48), 3.289 (3.15), 3.390 (5.82), 3.399 (4.28), 3.411 (3.29), 3.417 (3.56), 3.436 (2.18), 3.505 (0.83), 3.612 (0.63), 3.664 (2.27), 3.994 (3.26), 4.011 (3.37), 4.055 (2.48), 4.065 (2.48), 4.096 (1.79), 4.124 (6.79), 4.132 (7.61), 4.147 (2.63), 4.330 (7.82), 4.400 (14.09), 4.832 (0.43), 7.164 (2.85), 7.182 (6.05), 7.187 (5.12), 7.196 (6.72), 7.203 (6.69), 7.207 (6.88), 7.216 (6.16), 7.223 (4.10), 7.228 (5.48), 7.235 (3.62), 7.271 (8.66), 7.291 (13.12), 7.311 (12.29), 7.323 (7.64), 7.379 (3.77), 7.391 (3.90), 7.542 (10.13), 7.557 (7.07), 7.562 (9.02), 7.799 (5.87), 7.802 (6.08), 7.819 (5.78), 8.258 (7.44), 8.270 (7.04), 8.306 (4.24), 8.318 (3.97), 8.411 (5.20), 8.415 (5.88), 8.422 (6.35), 8.426 (5.30), 8.524 (16.00), 8.602 (0.85), 11.734 (1.82).

### Example 94

### 1-[(2S)-2-({[4-(3-phenyl-1H-pyrrolo[3,2-b]pyridin-2-yl)pyridin-3-yl]oxy}methyl)pyrrolidin-1-yl]but-2-yn-1-one

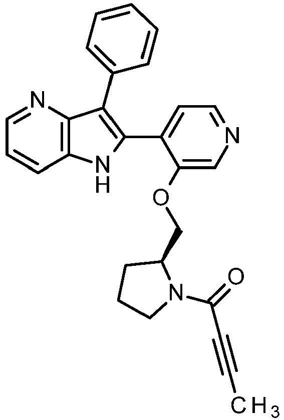

To a solution of 3-phenyl-2-(3-{[(2S)-pyrrolidin-2-yl]methoxy}pyridin-4-yl)-1H-pyrrolo[3,2-b]pyridine hydrogen chloride (1/1) (65.0 mg, 160 µmol) in DMF (1 ml), but-2-ynoic acid (13.4 mg, 160 µmol) and N,N-diisopropylethylamine (140 µl, 800 µmol) were added at rt. T3P (140 µl, 50 % purity in DMF, 240 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 49.0 mg (98 % purity, 69 % yield) of the desired product.
LC-MS (method 1): Rₜ = 1.01 min; MS (ESlpos): m/z = 437 [M+H]⁺
¹H-NMR (400 MHz, DMSO-d6) δ [ppm]: -0.012 (0.71), 1.477 (0.99), 1.494 (1.78), 1.510 (2.92), 1.520 (2.35), 1.530 (2.01), 1.545 (1.46), 1.696 (0.51), 1.712 (0.73), 1.732 (0.82), 1.744 (0.60), 1.763 (0.48), 1.879 (9.49), 1.931 (16.00), 2.728 (10.58), 2.887 (12.34), 2.975 (0.43), 2.990 (0.48), 3.061 (0.76), 3.071 (0.68), 3.087 (0.94), 3.103 (0.46), 3.171 (0.66), 3.201 (0.56), 3.285 (0.63), 3.304 (1.08), 3.402 (1.91), 3.421 (0.77), 3.439 (0.44), 3.463 (0.48), 3.988 (0.73), 4.011 (1.06), 4.024 (1.02), 4.036 (1.27), 4.044 (1.28), 4.084 (0.53), 4.109 (2.22), 4.120 (2.98), 4.135 (0.87), 7.166 (0.72), 7.185 (2.03), 7.191 (1.55), 7.202 (4.00), 7.213 (3.31), 7.222 (2.84), 7.234 (2.85), 7.271 (2.55), 7.275 (2.27), 7.291 (4.49), 7.296 (3.49), 7.302 (3.65), 7.309 (2.66), 7.314 (4.53), 7.366 (1.69), 7.378 (1.74), 7.538 (2.68), 7.544 (4.08), 7.562 (3.61), 7.803 (3.00), 7.806 (2.41), 7.823 (2.80), 7.826 (2.20), 7.947 (1.73), 8.251 (3.37), 8.263 (3.21), 8.298 (2.02), 8.310 (1.87), 8.415 (2.93), 8.426 (2.81), 8.524 (4.82), 8.560 (2.84), 11.726 (0.55).

### Example 95

### 3-(1-methyl-1H-pyrazol-3-yl)-1-[(2S)-2-({[4-(3-phenyl-1H-pyrrolo[3,2-b]pyridin-2-yl)pyridin-3-yl]oxy}methyl)pyrrolidin-1-yl]prop-2-yn-1-one

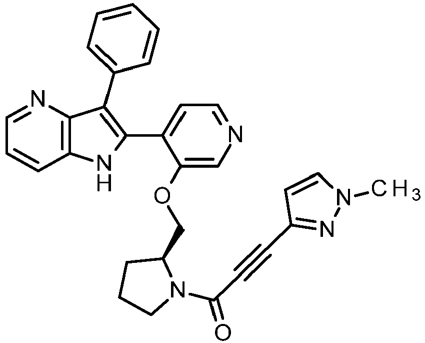

To a solution of 3-phenyl-2-(3-{[(2S)-pyrrolidin-2-yl]methoxy}pyridin-4-yl)-1H-pyrrolo[3,2-b]pyridine (70.0 mg, 189 µmol) in DMF (1.5 ml), lithium 3-(1-methyl-1H-pyrazol-3-yl)prop-2-ynoate (29.5 mg, 189 µmol) and N,N-diisopropylethylamine (130 µl, 760 µmol) were added at rt. T3P (170 µl, 50 % purity in DMF, 280 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 53.0 mg (100 % purity, 56 % yield) of the desired product.
LC-MS (method 2): Rₜ = 1.06 min; MS (ESlpos): m/z = 503 [M+H]⁺
¹H-NMR (600 MHz, DMSO-d6) δ [ppm]: 1.531 (1.98), 1.543 (2.78), 1.552 (2.18), 1.561 (1.05), 1.568 (0.84), 1.575 (0.73), 1.756 (0.74), 1.770 (0.99), 1.776 (0.90), 1.790 (0.76), 3.016 (0.47), 3.197 (0.70), 3.204 (0.79), 3.214 (0.94), 3.224 (0.70), 3.236 (0.57), 3.256 (0.50), 3.451 (0.46), 3.463 (0.98), 3.475 (0.69), 3.480 (1.04), 3.492 (0.49), 3.853 (7.55), 3.888 (16.00), 4.087 (0.64), 4.103 (0.90), 4.108 (0.78), 4.115 (1.00), 4.122 (1.19), 4.142 (0.96), 4.158 (2.28), 4.163 (2.69), 4.238 (0.57), 4.242 (0.58), 4.253 (0.51), 4.258 (0.44), 5.753 (3.69), 6.489 (1.41), 6.493 (1.49), 6.594 (2.99), 6.598 (3.10), 7.160 (1.64), 7.172 (2.42), 7.182 (2.24), 7.190 (1.75), 7.196 (1.61), 7.203 (1.68), 7.207 (0.97), 7.215 (0.85), 7.221 (0.79), 7.229 (0.78), 7.243 (1.07), 7.256 (1.82), 7.270 (2.66), 7.284 (3.75), 7.296 (2.00), 7.314 (2.60), 7.322 (2.73), 7.347 (1.26), 7.354 (1.28), 7.535 (1.67), 7.549 (1.76), 7.555 (3.55), 7.567 (3.16), 7.776 (1.36), 7.779 (1.38), 7.800 (2.68), 7.814 (2.69), 7.820 (3.14), 7.824 (2.99), 8.262 (2.84), 8.269 (2.79), 8.289 (1.35), 8.296 (1.30), 8.410 (1.84), 8.412 (1.94), 8.419 (2.67), 8.427 (1.01), 8.547 (4.62), 8.557 (2.34), 11.738 (2.81).

### Example 96

### (2E)-4-(dimethylamino)-1-[(2S)-2-{[(4-{3-[3-(trifluoromethyl)phenyl]-1H-pyrrolo[3,2-b]pyridin-2-yl}pyridin-3-yl)oxy]methyl}pyrrolidin-1-yl]but-2-en-1-one

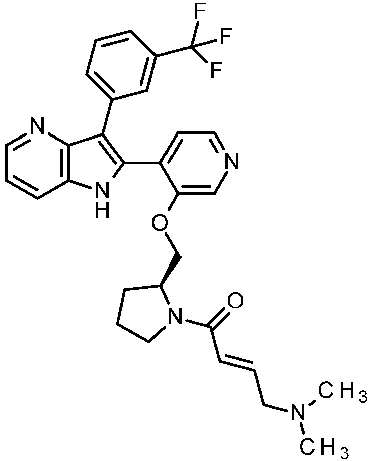

To a solution of 2-(3-{[(2S)-pyrrolidin-2-yl]methoxy}pyridin-4-yl)-3-[3-(trifluoromethyl)phenyl]-1H-pyrrolo[3,2-b]pyridine hydrogen chloride (1/1) (40.0 mg, 84.2 µmol) in DMF (1 ml), (2E)-4-(dimethylamino)but-2-enoic acid hydrogen chloride (1/1) (13.9 mg, 84.2 µmol) and N,N-diisopropylethylamine (73 µl, 420 µmol) were added at rt. T3P (74 µl, 50 % purity in DMF, 130 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 2 fractions containing the desired product. These fractions were combined and purified again by reverse phase preparative HPLC (method 8) to yield 11.8 mg (95 % purity, 24 % yield) of the desired product.
LC-MS (method 1): Rₜ = 1.00 min; MS (ESlneg): m/z = 548 [M-H]⁻
¹H-NMR (400 MHz, DMSO-d6) δ [ppm]: 1.511 (0.57), 1.526 (0.88), 1.543 (0.95), 1.569 (0.64), 1.587 (0.60), 1.969 (5.20), 2.002 (0.43), 2.073 (0.85), 2.103 (16.00), 2.587 (0.47), 2.603 (0.52), 2.942 (1.27), 2.957 (1.37), 3.129 (0.48), 3.142 (0.46), 3.154 (0.54), 3.253 (0.64), 3.276 (0.74), 3.295 (0.64), 3.308 (0.87), 3.375 (0.80), 3.391 (0.49), 3.401 (0.46), 3.701 (0.43), 3.971 (0.42), 3.988 (0.68), 3.995 (0.64), 4.011 (0.78), 4.071 (0.49), 4.154 (0.62), 4.161 (0.61), 4.177 (0.50), 4.184 (0.44), 5.754 (1.55), 6.127 (0.96), 6.164 (0.90), 6.550 (0.76), 6.588 (0.64), 7.251 (0.88), 7.263 (1.08), 7.272 (1.16), 7.283 (1.12), 7.415 (1.42), 7.426 (1.49), 7.499 (1.03), 7.515 (2.37), 7.558 (0.78), 7.793 (0.87), 7.808 (0.61), 7.849 (0.97), 7.853 (1.08), 7.870 (1.03), 7.873 (0.98), 7.883 (0.46), 8.019 (0.57), 8.038 (1.43), 8.322 (1.51), 8.334 (1.47), 8.369 (0.57), 8.380 (0.51), 8.481 (1.17), 8.484 (1.26), 8.492 (1.49), 8.495 (1.24), 8.510 (0.84), 8.627 (2.21), 11.953 (1.31), 11.978 (0.47).

### Example 97

### (2E)-1-{(2S)-2-[({4-[3-(5-chloro-2-fluorophenyl)-1H-pyrrolo[3,2-b]pyridin-2-yl]pyridin-3-yl}oxy)methyl]pyrrolidin-1-yl}-4-(dimethylamino)but-2-en-1-one

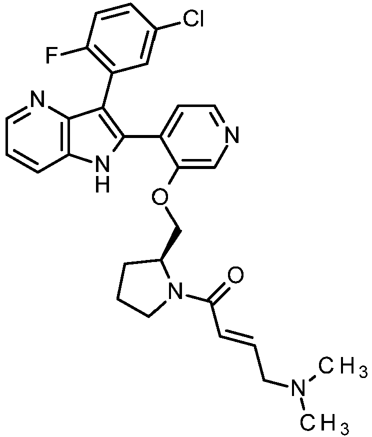

To a solution of 3-(5-chloro-2-fluorophenyl)-2-(3-{[(2S)-pyrrolidin-2-yl]methoxy}pyridin-4-yl)-1H-pyrrolo[3,2-b]pyridine (54.0 mg, 128 µmol) in DMF (1 ml), (2E)-4-(dimethylamino)but-2-enoic acid hydrogen chloride (1/1) (21.1 mg, 128 µmol) and N,N-diisopropylethylamine (89 µl, 510 µmol) were added at rt. T3P (110 µl, 50 % purity in DMF, 190 µmol) was added and stirring at rt was continued for 16 h. Additional (2E)-4-(dimethylamino)but-2-enoic acid hydrogen chloride (1/1) (10.5 mg, 64 µmol) were added and stirring was continued for additional 24h. Additional (2E)-4-(dimethylamino)but-2-enoic acid hydrogen chloride (1/1) (10.5 mg, 64 µmol), N,N-diisopropylethylamine (22 µl, 128 µmol) and T3P (59 µl, 50 % purity in DMF, 101 µmol) were added and stirring at rt was continued for 24h. Water (2 ml) was added, and the mixture was extracted with EtOAc twice. The combined organic layers were dried over sodium sulfate, filtered and concentrated under reduced pressure. The residue was purified by reverse phase preparative HPLC (method 3) but no fraction containing the desired product could be isolated. The aqueous phase that was obtained after work-up was basified with saturated NaHCO3 solution (aqueous, 5 mL) and extracted with EtOAc three times. The combined organic layers were dried over sodium sulfate, filtered and concentrated under reduced pressure. The residue was purified by reverse phase preparative HPLC (method 3) yielding 29.0 mg (100 % purity, 43 % yield) of the desired product.
LC-MS (method 1): Rₜ = 0.89 min; MS (ESlneg): m/z = 532 [M-H]⁻
¹H-NMR (400 MHz, DMSO-d6) δ [ppm]: 1.612 (0.50), 1.640 (0.53), 1.683 (1.17), 1.703 (0.97), 1.716 (0.89), 1.734 (0.78), 1.752 (0.61), 1.986 (5.40), 2.142 (16.00), 2.644 (0.55), 3.016 (1.80), 3.031 (1.81), 3.239 (0.41), 3.392 (0.71), 3.769 (0.44), 3.790 (0.46), 3.828 (0.42), 4.003 (0.88), 4.018 (0.69), 4.200 (1.28), 4.219 (0.72), 5.756 (1.94), 6.269 (0.79), 6.306 (0.96), 6.622 (0.75), 6.659 (0.62), 7.172 (0.59), 7.195 (1.21), 7.218 (0.82), 7.226 (0.55), 7.244 (0.92), 7.255 (2.47), 7.266 (2.47), 7.276 (1.28), 7.359 (0.53), 7.372 (1.15), 7.383 (0.81), 7.395 (0.71), 7.405 (0.59), 7.720 (0.71), 7.727 (0.82), 7.736 (0.83), 7.742 (0.78), 7.873 (0.45), 7.889 (1.37), 7.910 (1.08), 8.170 (3.60), 8.220 (1.48), 8.232 (1.47), 8.289 (0.55), 8.301 (0.52), 8.424 (1.18), 8.435 (1.21), 8.454 (0.46), 8.489 (0.86), 8.574 (2.37), 11.990 (1.46), 12.012 (0.60).

### Example 98

### (2Z)-4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one

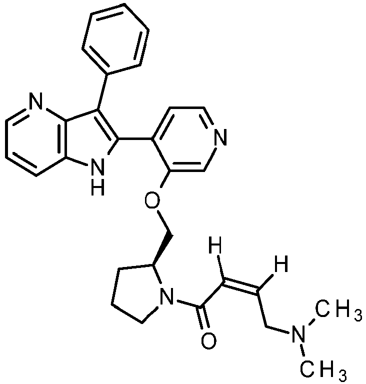

To a solution of 3-phenyl-2-(3-{[(2S)-pyrrolidin-2-yl]methoxy}pyridin-4-yl)-1H-pyrrolo[3,2-b]pyridine (50.0 mg, 135 µmol) in DMF (1 ml), lithium (2Z)-4-(dimethylamino)but-2-enoate (18.2 mg, 135 µmol) and N,N-diisopropylethylamine (94 µl, 540 µmol) were added at rt. T3P (120 µl, 50 % purity in DMF, 200 µmol) was added and stirring at rt was continued for 18 h. 2 drops of water were added, and the mixture was directly purified by reverse phase preparative HPLC (method 3) yielding the desired compound with insufficient purity. Further purification by thin layer chromatograpy (Eluent: DCM / MeOH 10:1) gave 3.00 mg (90 % purity, 4 % yield) of the desired product.
LC-MS (method 1): Rₜ = 0.67 min; MS (ESlneg): m/z = 480 [M-H]⁻
¹H-NMR (400 MHz, DMSO-d6) δ [ppm]: 1.232 (0.88), 1.535 (1.64), 1.550 (1.45), 1.572 (1.65), 1.654 (1.03), 1.674 (1.05), 1.691 (0.75), 1.907 (0.61), 1.985 (0.92), 2.066 (8.26), 2.141 (16.00), 3.006 (0.41), 3.130 (0.93), 3.155 (0.96), 3.183 (1.20), 3.222 (1.45), 3.245 (1.32), 3.725 (0.65), 3.744 (0.98), 3.969 (0.56), 3.979 (0.56), 4.021 (0.66), 4.035 (1.01), 4.043 (0.99), 4.058 (1.08), 4.146 (0.93), 4.167 (1.53), 4.191 (0.92), 4.199 (0.75), 5.429 (0.50), 5.443 (0.49), 5.458 (0.56), 5.752 (13.48), 5.897 (0.59), 5.927 (0.52), 5.992 (0.70), 6.005 (1.77), 6.016 (3.59), 6.046 (0.45), 7.168 (0.64), 7.186 (1.91), 7.200 (2.48), 7.212 (2.44), 7.221 (2.33), 7.232 (2.24), 7.244 (0.90), 7.274 (2.13), 7.293 (4.01), 7.304 (4.17), 7.315 (3.46), 7.325 (1.27), 7.396 (1.18), 7.408 (1.20), 7.546 (3.50), 7.564 (3.34), 7.818 (2.52), 7.821 (2.64), 7.838 (2.39), 7.841 (2.36), 8.247 (2.34), 8.258 (2.26), 8.308 (1.19), 8.320 (1.19), 8.416 (1.96), 8.428 (2.21), 8.441 (1.09), 8.460 (1.80), 8.594 (3.31), 11.761 (1.73), 11.778 (1.17).

### Example 99

### (2E)-1-[(2S)-2-({[4-(3-phenyl-1H-pyrrolo[3,2-b]pyridin-2-yl)pyridin-3-yl]oxy}methyl)pyrrolidin-1-yl]-4-(pyrrolidin-1-yl)but-2-en-1-one

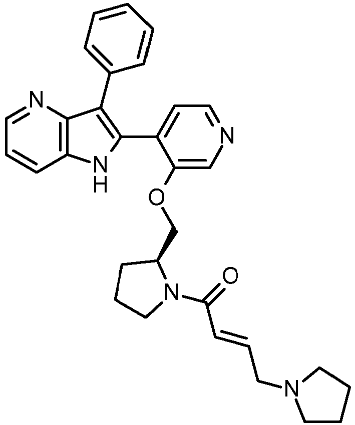

To a solution of 3-phenyl-2-(3-{[(2S)-pyrrolidin-2-yl]methoxy}pyridin-4-yl)-1H-pyrrolo[3,2-b]pyridine (31.0 mg, 83.7 µmol) in DMF (1 ml), (2E)-4-(pyrrolidin-1-yl)but-2-enoic acid hydrogen chloride (1/1) (16.0 mg, 83.7 µmol) and N,N-diisopropylethylamine (58 µl, 330 µmol) were added at rt. T3P (74 µl, 50 % purity in DMF, 130 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 22.0 mg (100 % purity, 52 % yield) of the desired product.
LC-MS (method 1): Rₜ = 0.71 min; MS (ESlneg): m/z = 506 [M-H]⁻
¹H-NMR (400 MHz, DMSO-d6) δ [ppm]: 11.84-11.68 (m, 1H), 8.59 and 8.48 (2s, 1H), 8.46-8.38 (m, 1H), 8.35-8.22 (m, 1H), 8.21-8.14 (m, 1H), 7.87-7.79 (m, 1H), 7.62-7.50 (m, 2H), 7.46-7.13 (m, 4H), 6.72-6.43 (m, 1H), 6.29-6.12 (m, 1H), 4.25-3.96 (m, 3H), 3.36-3.06 (m, 4H), 2.92-2.77 (m, 1H), 2.38-2.24 (m, 1H), 1.87-1.45 (m, 8H)

### Example 100

### 4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-yn-1-one

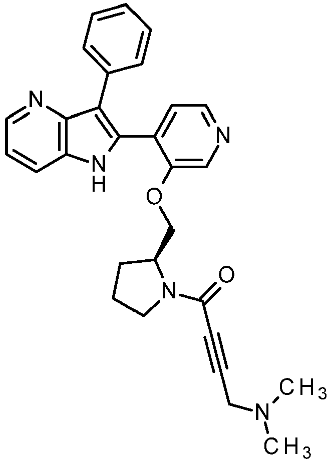

To a solution of 3-phenyl-2-(3-{[(2S)-pyrrolidin-2-yl]methoxy}pyridin-4-yl)-1H-pyrrolo[3,2-b]pyridine (30.0 mg, 81.0 µmol) in DMF (1 ml), 4-(dimethylamino)but-2-ynoic acid (10.3 mg, 81.0 µmol) and N,N-diisopropylethylamine (42 µl, 240 µmol) were added at rt. T3P (72 µl, 50 % purity in DMF, 120 µmol) was added and stirring at rt was continued for 3.5 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 23.0 mg (100 % purity, 59 % yield) of the desired product.
LC-MS (method 1): Rₜ = 0.70 min; MS (ESlneg): m/z = 478 [M-H]⁻
¹H-NMR (500 MHz, DMSO-d6) δ [ppm]: 1.089 (0.70), 1.501 (0.53), 1.515 (0.87), 1.528 (1.54), 1.537 (0.87), 1.555 (0.96), 1.564 (0.67), 1.753 (0.41), 2.038 (8.45), 2.125 (16.00), 3.116 (0.48), 3.315 (1.89), 3.376 (2.49), 3.381 (2.43), 3.388 (0.64), 3.399 (0.51), 3.419 (0.49), 4.055 (0.56), 4.066 (0.47), 4.076 (0.54), 4.082 (0.47), 4.140 (1.99), 4.149 (1.65), 7.167 (0.42), 7.182 (1.09), 7.185 (0.84), 7.198 (1.48), 7.207 (1.28), 7.214 (1.22), 7.223 (1.27), 7.232 (0.50), 7.274 (1.73), 7.290 (2.89), 7.305 (1.56), 7.309 (1.68), 7.319 (1.54), 7.362 (0.76), 7.372 (0.79), 7.542 (0.95), 7.554 (2.21), 7.568 (1.62), 7.793 (0.92), 7.796 (0.97), 7.801 (0.55), 7.804 (0.56), 7.810 (0.91), 7.812 (0.90), 7.818 (0.54), 7.821 (0.50), 8.259 (1.59), 8.269 (1.53), 8.301 (0.86), 8.310 (0.82), 8.410 (0.95), 8.413 (0.97), 8.419 (1.39), 8.422 (1.37), 8.429 (0.53), 8.432 (0.50), 8.536 (2.81), 11.734 (1.73).

### Example 101

### 2-(3-{[(2S)-1-(ethenesulfonyl)pyrrolidin-2-yl]methoxy}pyridin-4-yl)-3-[2-fluoro-5-(trifluoromethyl)phenyl]-1H-pyrrolo[3,2-b]pyridine

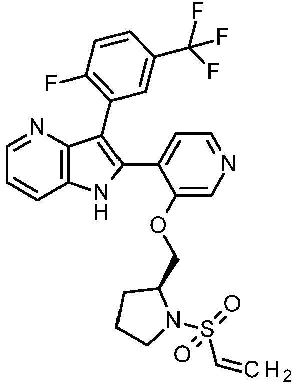

To a solution of 3-[2-fluoro-5-(trifluoromethyl)phenyl]-2-(3-{[(2S)-pyrrolidin-2-yl]methoxy}pyridin-4-yl)-1H-pyrrolo[3,2-b]pyridine (35.0 mg, 76.7 µmol) and triethylamine (37 µl, 270 µmol) in DCM (1 ml), 2-chloroethane-1-sulfonyl chloride (8.0 µl, 77 µmol) was added at rt. After stirring at rt for 4 h, water (0.5 ml) and saturated NaHCO3 solution (aqueous, 0.5 mL) were added. 2 ml of DCM were added, and the mixture was dried over a water-repellent filter. After concentration under reduced pressure the residue was purified by reverse phase preparative HPLC (method 3) yielding 6.20 mg (90 % purity, 13 % yield) of the title compound.
LC-MS (method 1): Rₜ = 1.46 min; MS (ESlpos): m/z = 547 [M+H]⁺
¹H-NMR (400 MHz, DMSO-d6) δ [ppm]: -0.156 (2.25), -0.076 (1.89), -0.025 (2.17), 1.231 (7.77), 1.326 (3.15), 1.438 (6.58), 1.636 (3.26), 1.654 (3.58), 2.136 (2.27), 2.327 (3.17), 2.366 (2.49), 2.669 (4.20), 2.709 (3.96), 2.821 (1.67), 2.914 (5.19), 2.927 (5.48), 2.940 (5.99), 3.026 (3.06), 3.213 (1.18), 3.270 (2.48), 3.401 (7.27), 3.426 (6.11), 3.448 (5.80), 3.498 (3.80), 3.521 (3.23), 3.624 (5.09), 3.756 (2.86), 3.792 (2.82), 3.812 (3.28), 3.837 (2.95), 3.911 (3.52), 4.026 (4.46), 4.038 (4.83), 4.060 (4.39), 5.750 (6.33), 6.015 (3.21), 6.025 (3.25), 6.047 (4.55), 6.056 (4.14), 6.062 (4.36), 6.087 (5.57), 6.753 (2.09), 6.778 (1.96), 6.796 (2.97), 6.821 (2.81), 6.837 (1.85), 6.862 (1.79), 7.205 (3.40), 7.217 (3.20), 7.232 (4.96), 7.240 (5.28), 7.251 (6.14), 7.263 (5.36), 7.336 (12.91), 7.348 (14.17), 7.365 (10.68), 7.376 (8.79), 7.414 (4.49), 7.423 (4.53), 7.438 (4.73), 7.661 (6.30), 7.794 (4.23), 7.815 (2.64), 7.838 (4.20), 7.855 (6.73), 7.875 (3.62), 8.137 (7.29), 8.147 (6.82), 8.160 (5.92), 8.173 (4.86), 8.189 (2.90), 8.207 (4.99), 8.229 (2.89), 8.280 (8.66), 8.289 (5.49), 8.414 (12.94), 8.430 (16.00), 8.438 (10.93), 8.457 (6.76), 8.561 (4.91), 8.572 (4.95), 11.877 (3.99), 11.931 (4.01), 11.974 (5.48), 11.986 (5.03).

### Example 102

### 1-{(2S)-2-[({4-[3-(3-ethynylphenyl)-1H-pyrrolo[3,2-b]pyridin-2-yl]pyridin-3-yl}oxy)methyl]pyrrolidin-1-yl}prop-2-en-1-one

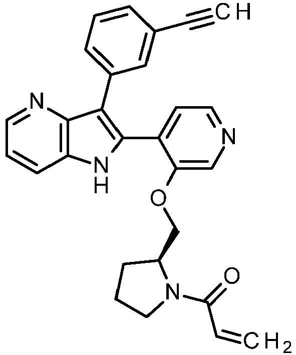

To a solution of 3-(3-ethynylphenyl)-2-(3-{[(2S)-pyrrolidin-2-yl]methoxy}pyridin-4-yl)-1H-pyrrolo[3,2-b]pyridine (26.5 mg, 79 % purity, 53.1 µmol) in THF (1 ml), prop-2-enoic acid (3.6 µl, 53 µmol) and N,N-diisopropylethylamine (28 µl, 160 µmol) were added at rt. T3P (47 µl, 50 % purity in EtOAc, 80 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 9.80 mg (100 % purity, 41 % yield) of the desired product.
LC-MS (method 1): Rₜ = 1.14 min; MS (ESlpos): m/z = 449 [M+H]⁺
¹H-NMR (400 MHz, DMSO-d6) δ [ppm]: -0.149 (0.44), 0.146 (0.43), 1.244 (2.16), 1.259 (2.76), 1.275 (1.43), 1.459 (1.83), 1.553 (3.67), 1.569 (4.82), 1.586 (4.27), 1.620 (3.23), 1.641 (3.82), 1.662 (3.33), 1.691 (2.20), 1.745 (1.18), 3.197 (2.86), 3.221 (3.30), 3.236 (2.21), 3.762 (0.96), 3.777 (1.52), 3.800 (1.63), 3.848 (1.52), 3.865 (1.44), 3.961 (1.55), 3.985 (1.55), 3.999 (1.15), 4.029 (2.08), 4.044 (3.04), 4.051 (2.88), 4.067 (3.42), 4.113 (16.00), 4.118 (10.28), 4.144 (2.76), 4.177 (3.93), 4.201 (2.75), 4.926 (1.84), 4.932 (1.72), 4.951 (1.72), 4.958 (1.87), 5.608 (3.70), 5.614 (3.61), 5.634 (3.77), 5.640 (4.01), 5.755 (9.21), 5.813 (1.70), 5.819 (1.73), 5.854 (1.96), 5.861 (1.99), 6.074 (3.23), 6.080 (3.32), 6.116 (4.24), 6.122 (4.21), 6.235 (1.71), 6.260 (1.76), 6.276 (1.42), 6.302 (1.29), 6.378 (3.62), 6.404 (3.66), 6.420 (3.05), 6.446 (2.65), 7.232 (4.07), 7.243 (5.92), 7.252 (6.27), 7.264 (6.67), 7.270 (6.51), 7.287 (12.55), 7.308 (3.97), 7.322 (6.10), 7.361 (6.94), 7.372 (7.00), 7.429 (3.90), 7.442 (6.92), 7.459 (3.52), 7.507 (1.86), 7.788 (4.08), 7.842 (12.72), 7.861 (5.42), 8.292 (7.26), 8.304 (6.92), 8.340 (3.89), 8.352 (3.60), 8.451 (5.22), 8.454 (5.54), 8.466 (7.37), 8.476 (3.19), 8.500 (5.82), 8.612 (10.94), 11.852 (5.40), 11.885 (2.98).

### Example 103

### 1-[(2S)-2-{[(4-{3-[3-(difluoromethoxy)-4-fluorophenyl]-1H-pyrrolo[3,2-b]pyridin-2-yl}pyridin-3-yl)oxy]methyl}pyrrolidin-1-yl]prop-2-en-1-one

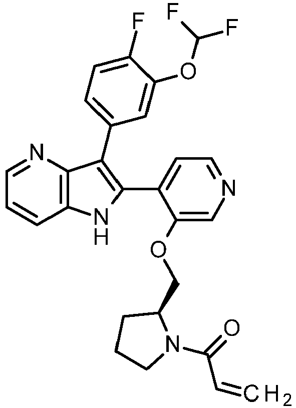

To a solution of 3-[3-(difluoromethoxy)-4-fluorophenyl]-2-(3-{[(2S)-pyrrolidin-2-yl]methoxy}pyridin-4-yl)-1H-pyrrolo[3,2-b]pyridine (30.0 mg, 66.0 µmol) in THF (1.5 ml), prop-2-enoic acid (6.8 µl, 99 µmol) and N,N-diisopropylethylamine (34 µl, 200 µmol) were added at rt. T3P (58 µl, 50 % purity in EtOAc, 99 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 20.0 mg (100 % purity, 60 % yield) of the desired product.
LC-MS (method 1): Rₜ = 1.28 min; MS (ESlpos): m/z = 509 [M+H]⁺
¹H-NMR (400 MHz, DMSO-d6) δ [ppm]: -0.150 (0.68), 0.146 (0.72), 1.412 (2.30), 1.427 (2.41), 1.547 (4.53), 1.562 (6.83), 1.579 (5.55), 1.598 (5.28), 1.616 (4.72), 1.644 (3.70), 1.729 (1.17), 1.752 (1.19), 2.367 (0.84), 2.711 (0.93), 3.133 (1.40), 3.152 (1.40), 3.174 (1.45), 3.187 (2.68), 3.202 (2.82), 3.213 (4.16), 3.228 (2.33), 3.802 (1.26), 3.816 (1.84), 3.825 (1.69), 3.839 (1.88), 3.929 (1.71), 3.945 (1.63), 3.986 (2.28), 4.008 (4.22), 4.024 (5.56), 4.047 (5.51), 4.069 (2.73), 4.087 (3.31), 4.161 (4.64), 4.168 (4.08), 4.184 (3.70), 4.191 (2.94), 4.883 (2.29), 4.890 (2.16), 4.909 (2.20), 4.915 (2.24), 5.604 (5.32), 5.610 (4.88), 5.630 (5.13), 5.636 (5.70), 5.755 (7.10), 5.795 (1.93), 5.802 (2.06), 5.837 (2.59), 5.843 (2.40), 6.069 (4.61), 6.075 (4.48), 6.111 (6.09), 6.117 (5.83), 6.231 (2.09), 6.257 (2.16), 6.273 (1.83), 6.298 (1.69), 6.366 (5.53), 6.392 (5.36), 6.409 (4.36), 6.434 (3.85), 6.919 (4.96), 6.932 (2.26), 7.101 (9.76), 7.115 (4.41), 7.233 (6.33), 7.245 (7.12), 7.254 (8.94), 7.265 (7.40), 7.274 (3.02), 7.284 (4.72), 7.297 (2.35), 7.310 (4.10), 7.333 (6.84), 7.358 (5.47), 7.379 (2.78), 7.398 (10.14), 7.410 (10.24), 7.442 (4.59), 7.454 (4.56), 7.490 (1.73), 7.501 (1.86), 7.512 (1.64), 7.523 (1.65), 7.536 (4.77), 7.548 (8.36), 7.567 (12.23), 7.589 (2.33), 7.838 (10.38), 7.858 (9.28), 8.319 (11.14), 8.330 (10.50), 8.351 (4.92), 8.363 (4.57), 8.457 (7.44), 8.461 (7.68), 8.469 (9.97), 8.479 (3.79), 8.503 (7.33), 8.613 (16.00), 11.879 (9.48), 11.900 (4.52).

### Example 104

### (2E)-1-[(2S)-2-{[(4-{3-[3-(difluoromethoxy)-4-fluorophenyl]-1H-pyrrolo[3,2-b]pyridin-2-yl}pyridin-3-yl)oxy]methyl}pyrrolidin-1-yl]-4-(dimethylamino)but-2-en-1-one

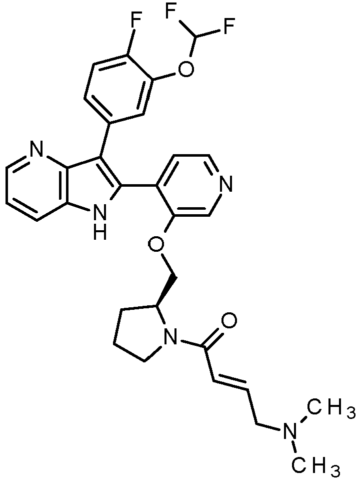

To a solution of 3-[3-(difluoromethoxy)-4-fluorophenyl]-2-(3-{[(2S)-pyrrolidin-2-yl]methoxy}pyridin-4-yl)-1H-pyrrolo[3,2-b]pyridine (40.0 mg, 88.0 µmol) in THF (1.5 ml), (2E)-4-(dimethylamino)but-2-enoic acid hydrogen chloride (1/1) (21.9 mg, 132 µmol) and N,N-diisopropylethylamine (77 µl, 440 µmol) were added at rt. T3P (78 µl, 50 % purity in EtOAc, 130 µmol) was added and stirring at rt was continued for 16 h. Additional (2E)-4-(dimethylamino)but-2-enoic acid hydrogen chloride (1/1) (21.9 mg, 132 µmol), N,N-diisopropylethylamine (77 µl, 440 µmol) and T3P (78 µl, 50 % purity in EtOAc were added and stirring was continued for 20h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 28.0 mg (94 % purity, 53 % yield) of the desired product.
LC-MS (method 1): Rₜ = 0.93 min; MS (ESlneg): m/z = 564 [M-H]⁻
¹H-NMR (400 MHz, DMSO-d6) δ [ppm]: 0.910 (3.52), 0.926 (2.58), 0.948 (1.47), 0.965 (0.84), 1.232 (4.25), 1.251 (15.75), 1.268 (13.76), 1.470 (2.57), 1.547 (3.77), 1.563 (4.57), 1.580 (3.96), 1.644 (1.60), 1.806 (0.77), 2.221 (4.93), 2.275 (1.16), 2.344 (16.00), 2.407 (9.58), 2.671 (1.05), 2.711 (1.04), 2.875 (10.83), 2.975 (1.32), 2.995 (1.67), 3.043 (11.36), 3.127 (2.47), 3.192 (3.54), 3.612 (2.60), 3.671 (1.30), 3.840 (1.08), 3.938 (0.90), 4.004 (1.45), 4.021 (2.57), 4.044 (3.26), 4.160 (2.34), 4.183 (1.98), 6.270 (2.30), 6.309 (2.95), 6.448 (0.67), 6.553 (1.22), 6.572 (2.28), 6.589 (2.10), 6.608 (1.72), 6.669 (1.47), 6.707 (0.88), 6.919 (2.60), 6.946 (0.87), 7.101 (5.06), 7.128 (1.67), 7.237 (3.11), 7.249 (3.46), 7.258 (3.87), 7.269 (3.60), 7.284 (2.59), 7.311 (2.59), 7.334 (3.65), 7.360 (3.49), 7.382 (1.23), 7.397 (5.16), 7.409 (5.22), 7.444 (1.67), 7.456 (1.66), 7.535 (5.82), 7.548 (3.34), 7.553 (4.63), 7.598 (1.08), 7.613 (0.95), 7.840 (3.40), 7.858 (3.27), 8.318 (5.51), 8.330 (5.46), 8.351 (1.68), 8.362 (1.57), 8.459 (4.22), 8.463 (4.36), 8.471 (5.31), 8.517 (2.28), 8.607 (8.15), 11.907 (4.71), 11.951 (1.40).

### Example 105

### 3-[3-(difluoromethoxy)-4-fluorophenyl]-2-(3-{[(2S)-1-(ethenesulfonyl)pyrrolidin-2-yl]methoxy}pyridin-4-yl)-1H-pyrrolo[3,2-b]pyridine

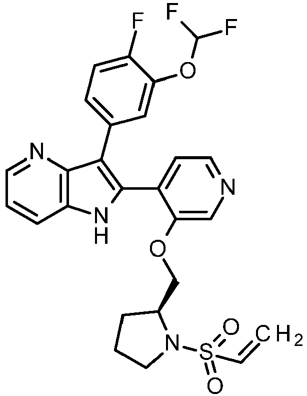

To a solution of 3-[3-(difluoromethoxy)-4-fluorophenyl]-2-(3-{[(2S)-pyrrolidin-2-yl]methoxy}pyridin-4-yl)-1H-pyrrolo[3,2-b]pyridine (40.0 mg, 88.0 µmol) and triethylamine (43 µl, 310 µmol; CAS-RN:[121-44-8]) in DCM (1 ml), 2-chloroethane-1-sulfonyl chloride (9.2 µl, 88 µmol) was added at rt. After stirring at rt for 16 h, water (0.5 ml) and saturated NaHCO3 solution (aqueous, 0.5 mL) were added. 2 ml of DCM were added, and the mixture was dried over a water-repellent filter. After concentration under reduced pressure the residue was purified by reverse phase preparative HPLC (method 3) yielding 17.4 mg (100 % purity, 36 % yield) of the title compound.
LC-MS (method 1): Rₜ = 1.40 min; MS (ESlpos): m/z = 545 [M+H]⁺
¹H-NMR (500 MHz, DMSO-d6) δ [ppm]: -0.009 (1.16), 1.174 (0.47), 1.354 (1.54), 1.360 (2.26), 1.366 (1.92), 1.370 (1.90), 1.381 (1.56), 1.389 (1.15), 1.440 (1.03), 1.484 (1.20), 1.498 (3.84), 1.511 (4.84), 1.523 (5.02), 1.537 (3.20), 1.551 (2.47), 1.568 (1.46), 1.584 (0.43), 2.400 (0.41), 2.674 (0.41), 2.947 (4.66), 2.960 (8.56), 2.972 (4.12), 3.540 (0.85), 3.548 (1.44), 3.556 (2.49), 3.563 (2.93), 3.570 (2.26), 3.578 (1.74), 3.584 (0.76), 3.925 (3.07), 3.940 (3.54), 3.944 (4.51), 3.959 (3.82), 4.026 (3.95), 4.034 (4.26), 4.046 (3.10), 4.053 (2.76), 5.753 (3.08), 6.033 (10.97), 6.051 (11.30), 6.066 (12.33), 6.071 (11.79), 6.763 (5.99), 6.783 (6.15), 6.796 (6.08), 6.816 (5.28), 6.942 (4.60), 7.088 (9.14), 7.233 (9.39), 7.242 (6.44), 7.249 (6.48), 7.258 (6.67), 7.320 (4.14), 7.338 (5.34), 7.342 (4.58), 7.359 (4.93), 7.443 (9.95), 7.452 (10.24), 7.521 (2.41), 7.525 (3.00), 7.530 (2.82), 7.534 (3.00), 7.538 (2.34), 7.542 (3.01), 7.547 (2.28), 7.551 (2.67), 7.567 (3.77), 7.571 (3.28), 7.583 (3.80), 7.831 (6.58), 7.834 (6.95), 7.847 (6.39), 7.850 (6.11), 8.353 (11.98), 8.363 (11.29), 8.455 (7.03), 8.458 (7.27), 8.464 (6.96), 8.467 (6.62), 8.535 (16.00), 11.876 (8.65).

### Example 106

### 1-[(2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidin-1-yl]prop-2-en-1-one

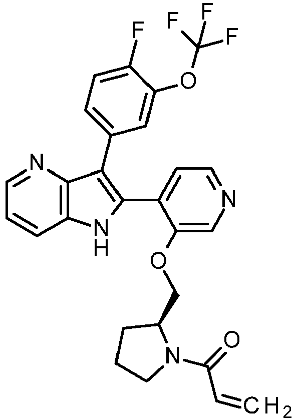

To a solution of 3-[4-fluoro-3-(trifluoromethoxy)phenyl]-2-(3-{[(2S)-pyrrolidin-2-yl]methoxy}pyridin-4-yl)-1H-pyrrolo[3,2-b]pyridine (30.0 mg, 63.5 µmol) in THF (1 ml), prop-2-enoic acid (2.2 µl, 32 µmol) and N,N-diisopropylethylamine (33 µl, 190 µmol) were added at rt. T3P (56 µl, 50 % purity in EtOAc, 95 µmol) was added and stirring at rt was continued for 16 h. Additional prop-2-enoic acid (4.4 µl, 63 µmol) and N,N-diisopropylethylamine (17 µl, 85 µmol) were added at rt. T3P (28 µl, 50 % purity in EtOAc, 48 µmol) were added and stirring at rt was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 13.0 mg (94 % purity, 37 % yield) of the desired product.
LC-MS (method 2): Rₜ = 1.60 min; MS (ESlpos): m/z = 527 [M+H]⁺
¹H-NMR (400 MHz, DMSO-d6) δ [ppm]: -0.149 (0.45), 0.914 (1.27), 0.928 (1.19), 1.266 (5.38), 1.350 (1.65), 1.425 (2.15), 1.536 (4.15), 1.552 (3.55), 1.617 (2.63), 3.159 (2.03), 3.184 (2.39), 3.281 (4.70), 3.615 (0.93), 3.809 (1.30), 3.825 (1.38), 3.909 (1.21), 3.980 (1.29), 4.002 (2.41), 4.018 (2.89), 4.041 (2.80), 4.089 (2.11), 4.155 (2.55), 4.178 (2.07), 4.906 (1.24), 4.932 (1.20), 5.594 (2.46), 5.600 (2.48), 5.619 (2.61), 5.625 (2.82), 5.755 (16.00), 5.809 (1.13), 5.850 (1.42), 6.059 (2.17), 6.064 (2.26), 6.101 (2.95), 6.107 (3.03), 6.218 (1.09), 6.245 (1.15), 6.260 (0.90), 6.287 (0.86), 6.349 (2.48), 6.374 (2.49), 6.390 (2.07), 6.416 (1.84), 7.248 (2.65), 7.259 (3.58), 7.268 (3.85), 7.279 (3.63), 7.426 (2.15), 7.439 (4.60), 7.450 (7.40), 7.475 (4.09), 7.491 (2.43), 7.503 (1.73), 7.693 (6.71), 7.707 (5.59), 7.851 (3.55), 7.871 (3.51), 8.343 (3.87), 8.354 (3.90), 8.372 (2.00), 8.383 (1.88), 8.475 (4.32), 8.484 (5.69), 8.514 (3.36), 8.630 (6.95), 11.959 (4.46), 11.987 (2.29).

### Example 107

### (2E)-4-(dimethylamino)-1-[(2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidin-1-yl]but-2-en-1-one

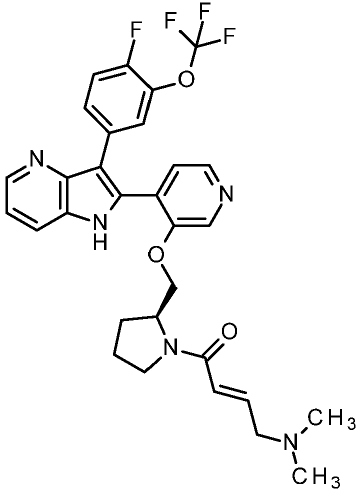

To a solution of 3-[4-fluoro-3-(trifluoromethoxy)phenyl]-2-(3-{[(2S)-pyrrolidin-2-yl]methoxy}pyridin-4-yl)-1H-pyrrolo[3,2-b]pyridine (40.0 mg, 84.7 µmol) in THF (1 ml), (2E)-4-(dimethylamino)but-2-enoic acid hydrogen chloride (1/1) (14.0 mg, 84.7 µmol) and N,N-diisopropylethylamine (74 µl, 420 µmol) were added at rt. T3P (75 µl, 50 % purity in EtOAc, 130 µmol) was added and stirring at rt was continued for 16 h. Additional (2E)-4-(dimethylamino)but-2-enoic acid hydrogen chloride (1/1) (14.0 mg, 84.7 µmol), N,N-diisopropylethylamine (74 µl, 420 µmol) and T3P (75 µl, 50 % purity in EtOAc, 130 µmol) were added and stirring was continued for 2 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 4.90 mg (96 % purity, 10 % yield) of the desired product.
LC-MS (method 2): Rₜ = 1.18 min; MS (ESlneg): m/z = 582 [M-H]⁻
¹H-NMR (400 MHz, DMSO-d6) δ [ppm]: -0.150 (1.22), 0.146 (1.13), 0.913 (15.43), 0.932 (10.80), 0.951 (4.69), 1.157 (3.13), 1.175 (6.32), 1.193 (3.63), 1.366 (4.22), 1.393 (4.56), 1.473 (16.00), 1.535 (10.15), 1.989 (11.81), 2.252 (8.40), 2.330 (6.07), 2.369 (4.95), 2.672 (3.03), 2.712 (3.14), 3.148 (7.86), 3.811 (2.94), 4.003 (4.90), 4.020 (8.45), 4.039 (8.55), 4.081 (5.53), 4.155 (6.03), 4.178 (4.69), 6.222 (2.32), 6.570 (2.04), 7.267 (6.42), 7.433 (8.91), 7.446 (14.10), 7.472 (8.12), 7.680 (8.75), 7.702 (13.22), 7.862 (2.75), 8.143 (9.12), 8.338 (9.19), 8.350 (9.05), 8.475 (11.24), 8.485 (12.47), 8.525 (2.58), 8.624 (15.76), 11.975 (4.40).

### Example 108

### 2-(3-{[(2S)-1-(ethenesulfonyl)pyrrolidin-2-yl]methoxy}pyridin-4-yl)-3-[4-fluoro-3-(trifluoromethoxy)phenyl]-1 H-pyrrolo[3,2-b]pyridine

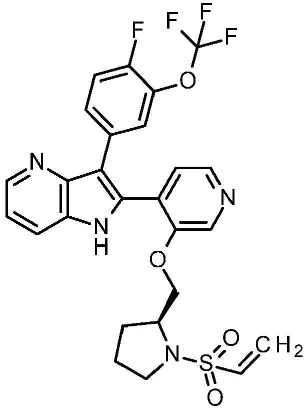

To a solution of 3-[4-fluoro-3-(trifluoromethoxy)phenyl]-2-(3-{[(2S)-pyrrolidin-2-yl]methoxy}pyridin-4-yl)-1H-pyrrolo[3,2-b]pyridine (50.0 mg, 106 µmol) and triethylamine (52 µl, 370 µmol) in DCM (1 ml), 2-chloroethane-1-sulfonyl chloride (11 µl, 110 µmol) was added at rt. After stirring at rt for 16 h, water (0.5 ml) and saturated NaHCO3 solution (aqueous, 0.5 mL) were added. 2 ml of DCM were added, and the mixture was dried over a water-repellent filter. After concentration under reduced pressure the residue was purified by reverse phase preparative HPLC (method 3) yielding 17.0 mg (100 % purity, 29 % yield) of the title compound.
LC-MS (method 2): Rₜ = 1.72 min; MS (ESlpos): m/z = 563 [M+H]⁺
¹H-NMR (400 MHz, DMSO-d6) δ [ppm]: 1.296 (2.60), 1.435 (0.81), 1.481 (4.11), 1.499 (8.34), 1.506 (7.03), 1.512 (6.86), 1.546 (1.16), 2.939 (4.30), 2.949 (6.40), 2.957 (5.77), 2.975 (2.55), 3.403 (0.47), 3.544 (2.64), 3.552 (3.16), 3.561 (2.54), 3.907 (3.05), 3.931 (4.74), 3.950 (3.94), 4.015 (4.34), 4.024 (4.47), 4.039 (3.11), 4.047 (2.76), 5.755 (1.32), 6.017 (9.89), 6.041 (10.19), 6.058 (11.40), 6.066 (10.90), 6.754 (5.63), 6.779 (5.71), 6.795 (5.52), 6.820 (4.77), 7.245 (5.86), 7.257 (5.91), 7.266 (6.06), 7.277 (6.13), 7.433 (3.99), 7.455 (5.53), 7.484 (12.22), 7.495 (10.49), 7.694 (6.84), 7.708 (9.49), 7.715 (4.20), 7.721 (2.71), 7.727 (3.16), 7.733 (2.03), 7.845 (6.58), 7.848 (6.96), 7.865 (6.38), 7.869 (6.20), 8.377 (10.86), 8.388 (10.31), 8.468 (6.73), 8.472 (7.01), 8.480 (6.80), 8.483 (6.44), 8.544 (16.00), 11.949 (9.63).

### Example 109

### 1-[(2S)-2-({[5-fluoro-4-(3-phenyl-1H-pyrrolo[3,2-b]pyridin-2-yl)pyridin-3-yl]oxy}methyl)pyrrolidin-1-yl]prop-2-en-1-one

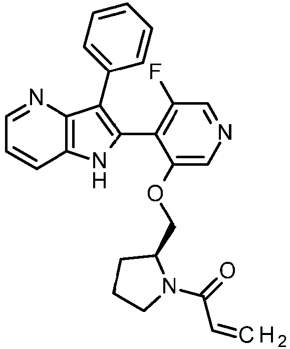

To a solution of 2-(3-fluoro-5-{[(2S)-pyrrolidin-2-yl]methoxy}pyridin-4-yl)-3-phenyl-1H-pyrrolo[3,2-b]pyridine hydrogen chloride (1/1) (60.0 mg, 141 µmol) in DMF (1 ml), prop-2-enoic acid (12 µl, 170 µmol) and N,N-diisopropylethylamine (150 µl, 850 µmol) were added at rt. T3P (120 µl, 50 % purity in DMF, 210 µmol) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 25.3 mg (100 % purity, 40 % yield) of the desired product.
LC-MS (method 1): Rₜ = 1.06 min; MS (ESlpos): m/z = 443 [M+H]⁺
¹H-NMR (400 MHz, DMSO-d6) δ [ppm]: ¹H-NMR (400 MHz, DMSO-d6): d [ppm] = 11.90 and 11.86 (2s br, 1H), 8.54 and 8.39 (2s, 1H), 8.49-8.40 (m, 2H), 7.92-7.82 (m, 1H), 7.60-7.51 (m, 2H), 7.36-7.14 (m, 4H), 6.43-6.33 and 6.30-6.19 (2m, 1H), 6.14-6.04 and 5.83-5.73 (2m, 1H), 5.65-5.59 and 4.86-4.78 (2m, 1H), 4.29-3.80 (m, 3H), 3.28-3.02 (m, 2H), 1.84-1.36 (m, 4H)

### Example 110

### 1-[(2S,4S)-4-methyl-2-({[4-(3-phenyl-1H-pyrrolo[3,2-b]pyridin-2-yl)pyridin-3-yl]oxy}methyl)pyrrolidin-1-yl]prop-2-en-1-one

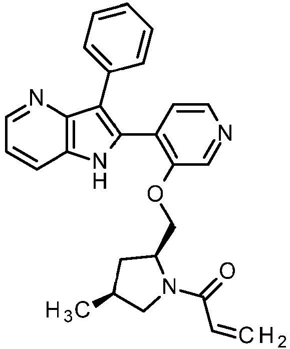

To a solution of 2-(3-{[(2S,4S)-4-methylpyrrolidin-2-yl]methoxy}pyridin-4-yl)-3-phenyl-1H-pyrrolo[3,2-b]pyridine (45.0 mg, 117 µmol) in THF (0.9 ml), prop-2-enoic acid (8.0 µl, 120 µmol) and N,N-diisopropylethylamine (61 µl, 350 µmol) were added at rt. T3P (100 µl, 50 % purity in EtOAc, 180 µmol) was added and stirring at rt was continued for 3 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 39.5 mg (100 % purity, 77 % yield) of the desired product.
LC-MS (method 1): Rₜ = 1.04 min; MS (ESlpos): m/z = 439 [M+H]⁺
¹H-NMR (600 MHz, DMSO-d6) δ [ppm]: 0.538 (16.00), 0.548 (15.44), 0.745 (3.82), 0.756 (3.88), 0.918 (0.63), 0.930 (1.36), 0.942 (0.93), 0.950 (0.74), 0.962 (0.84), 1.176 (0.48), 1.188 (0.43), 1.208 (0.94), 1.226 (2.64), 1.241 (2.73), 1.260 (1.28), 1.288 (1.25), 1.835 (0.58), 1.846 (0.78), 1.857 (1.03), 1.868 (1.22), 1.875 (1.64), 1.886 (1.71), 1.901 (2.20), 1.912 (2.35), 1.918 (2.00), 1.931 (2.08), 1.944 (0.93), 2.061 (0.54), 2.082 (0.54), 2.295 (2.37), 2.312 (4.52), 2.329 (2.40), 2.340 (0.74), 2.359 (1.12), 2.377 (0.68), 2.425 (0.55), 2.519 (1.57), 2.654 (0.40), 3.520 (2.26), 3.535 (2.85), 3.547 (2.22), 3.568 (9.87), 3.828 (0.60), 3.835 (0.67), 3.844 (0.69), 3.852 (0.72), 3.865 (0.61), 3.877 (0.62), 3.884 (0.63), 3.896 (0.55), 3.940 (0.60), 4.023 (0.77), 4.032 (0.71), 4.039 (0.71), 4.048 (0.54), 4.120 (2.13), 4.191 (2.95), 4.195 (2.91), 4.207 (3.52), 4.211 (3.11), 4.382 (3.13), 4.389 (3.23), 4.398 (2.85), 4.405 (2.55), 4.896 (0.79), 4.914 (0.82), 4.918 (0.82), 5.548 (4.01), 5.551 (3.87), 5.565 (3.88), 5.568 (4.24), 5.755 (6.44), 5.803 (0.75), 5.808 (0.76), 5.831 (0.85), 5.835 (0.89), 6.001 (3.59), 6.005 (3.60), 6.029 (4.37), 6.033 (4.31), 6.283 (3.89), 6.300 (4.54), 6.311 (3.44), 6.317 (0.93), 6.328 (3.70), 6.345 (0.64), 7.168 (1.87), 7.180 (4.67), 7.193 (3.24), 7.209 (1.25), 7.220 (3.31), 7.228 (2.92), 7.234 (3.18), 7.241 (3.12), 7.263 (6.36), 7.271 (7.87), 7.276 (11.78), 7.288 (5.27), 7.295 (1.71), 7.308 (2.39), 7.321 (1.27), 7.374 (1.32), 7.381 (1.35), 7.528 (9.01), 7.540 (9.00), 7.556 (2.00), 7.826 (2.88), 7.839 (2.94), 7.858 (0.80), 8.232 (4.06), 8.240 (3.93), 8.303 (1.04), 8.310 (0.98), 8.421 (4.71), 8.427 (4.70), 8.439 (1.30), 8.447 (1.20), 8.474 (1.65), 8.530 (6.47), 11.804 (2.17).

### Example 111

### 2-(3-{[(2S,4S)-1-(ethenesulfonyl)-4-methylpyrrolidin-2-yl]methoxy}pyridin-4-yl)-3-phenyl-1H-pyrrolo[3,2-b]pyridine

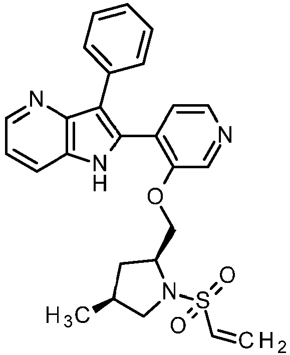

To a solution of 2-(3-{[(2S,4S)-4-methylpyrrolidin-2-yl]methoxy}pyridin-4-yl)-3-phenyl-1H-pyrrolo[3,2-b]pyridine (52.0 mg, 135 µmol) and triethylamine (66 µl, 470 µmol) in DCM (1 ml), 2-chloroethane-1-sulfonyl chloride (13 µl, 120 µmol) was added at rt. After stirring at rt for 20 h, water (0.5 ml) and saturated NaHCO3 solution (aqueous, 0.5 mL) were added. 2 ml of DCM were added, and the mixture was dried over a water-repellent filter. After concentration under reduced pressure the residue was purified by reverse phase preparative HPLC (method 3) yielding 18.9 mg (100 % purity, 29 % yield) of the title compound.
LC-MS (method 1): Rₜ = 1.15 min; MS (ESlpos): m/z = 475 [M+H]⁺
¹H-NMR (600 MHz, DMSO-d6) δ [ppm]: 0.264 (0.71), 0.274 (0.70), 0.385 (0.49), 0.396 (0.49), 0.507 (0.88), 0.517 (0.74), 0.549 (0.63), 0.565 (16.00), 0.575 (15.18), 0.609 (0.63), 0.620 (0.61), 1.098 (0.95), 1.116 (2.07), 1.131 (2.15), 1.151 (1.18), 1.750 (0.73), 1.760 (0.95), 1.768 (1.41), 1.779 (1.58), 1.790 (1.21), 1.797 (1.82), 1.809 (1.84), 1.818 (1.69), 1.829 (1.79), 1.841 (0.82), 2.321 (2.53), 2.339 (4.42), 2.357 (2.51), 2.425 (0.46), 2.516 (1.35), 2.520 (1.32), 2.523 (1.07), 3.171 (2.21), 3.201 (0.52), 3.211 (0.50), 3.228 (1.92), 3.240 (2.25), 3.246 (2.18), 3.258 (2.08), 3.558 (0.97), 3.568 (5.05), 3.580 (1.57), 3.595 (0.61), 4.000 (0.44), 4.072 (2.09), 4.082 (2.19), 4.088 (3.45), 4.098 (3.14), 4.128 (3.66), 4.133 (3.87), 4.144 (2.35), 4.149 (2.01), 5.755 (6.69), 6.009 (0.44), 6.021 (7.61), 6.028 (7.57), 6.037 (7.85), 6.056 (7.94), 6.753 (3.98), 6.770 (4.09), 6.781 (3.95), 6.797 (3.59), 7.154 (0.46), 7.176 (2.27), 7.188 (4.86), 7.199 (6.36), 7.206 (4.87), 7.212 (4.54), 7.220 (4.68), 7.238 (0.79), 7.250 (0.77), 7.259 (0.61), 7.276 (5.95), 7.289 (9.61), 7.301 (4.92), 7.314 (0.56), 7.322 (0.59), 7.340 (0.41), 7.352 (7.23), 7.360 (7.46), 7.442 (0.45), 7.456 (0.55), 7.533 (0.55), 7.546 (0.56), 7.567 (8.47), 7.579 (7.87), 7.581 (6.45), 7.800 (4.96), 7.802 (5.08), 7.813 (4.73), 7.816 (4.60), 8.273 (1.05), 8.281 (1.05), 8.287 (8.71), 8.295 (8.24), 8.395 (0.45), 8.411 (5.00), 8.414 (4.98), 8.419 (4.95), 8.421 (4.60), 8.443 (0.41), 8.450 (0.40), 8.489 (0.81), 8.511 (12.84), 8.555 (0.60), 8.562 (0.40).

### Example 112

### 1-[(2S)-4,4-difluoro-2-({[4-(3-phenyl-1H-pyrrolo[3,2-b]pyridin-2-yl)pyridin-3-yl]oxy}methyl)pyrrolidin-1-yl]prop-2-en-1-one

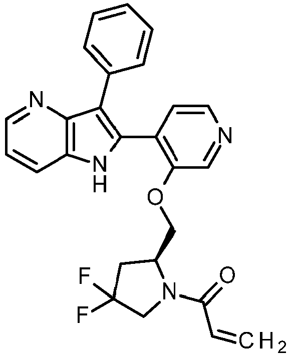

At rt, a mixture of 100 mg 2-(3-{[(2S)-4,4-difluoropyrrolidin-2-yl]methoxy}pyridin-4-yl)-3-phenyl-1H-pyrrolo[3,2-b]pyridine and 2-(3-{[(2S)-4-fluoro-2,3-dihydro-1H-pyrrol-2-yl]methoxy}pyridin-4-yl)-3-phenyl-1H-pyrrolo[3,2-b]pyridine (ratio 60:40) in DMF (1 ml) was treated with N,N-diisopropylethylamine (86 µl, 490 µmol) and prop-2-enoic acid (17 µl, 250 µmol). T3P (220 µl, 50 % purity in DMF, 370 µmol) was added and stirring at rt was continued for 16 h. Saturated NaHCO3 solution (aqueous, 0.5 mL) and water (1 ml) were added, and the mixture was extracted with EtOAc twice. The combined organic layers were dried over sodium sulfate, filtered and concentrated under reduced pressure. The residue was purified by reverse phase preparative HPLC (method 3) yielding 11.6 mg (98 % purity, 10 % yield) of the desired product.
An additional fraction was isolated that is described as **Example 113**
LC-MS (method 1): Rₜ = 0.95 min; MS (ESlpos): m/z = 461 [M+H]⁺
¹H-NMR (400 MHz, DMSO-d6) δ [ppm]: 2.084 (1.92), 2.116 (2.15), 2.166 (1.79), 2.327 (3.23), 2.366 (2.91), 2.386 (1.60), 2.669 (1.64), 2.710 (1.21), 3.221 (1.02), 3.274 (2.18), 3.297 (4.28), 3.401 (2.20), 3.423 (2.81), 3.461 (4.66), 3.493 (5.22), 3.519 (1.73), 3.940 (4.60), 3.976 (4.24), 4.006 (1.68), 4.123 (3.76), 4.140 (4.53), 4.163 (3.31), 4.253 (5.84), 4.277 (4.47), 4.381 (2.94), 4.915 (2.21), 4.945 (2.41), 5.669 (4.43), 5.695 (4.85), 5.820 (2.11), 5.865 (2.54), 6.115 (3.91), 6.156 (5.33), 6.283 (1.74), 6.309 (1.96), 6.337 (4.63), 6.363 (4.36), 6.378 (3.17), 6.404 (2.66), 7.171 (2.36), 7.188 (7.01), 7.207 (11.74), 7.218 (8.71), 7.227 (9.09), 7.238 (6.82), 7.270 (7.44), 7.289 (16.00), 7.307 (13.23), 7.314 (12.20), 7.326 (11.26), 7.365 (4.02), 7.376 (3.86), 7.531 (14.67), 7.549 (13.58), 7.803 (8.64), 7.823 (8.08), 8.132 (7.60), 8.272 (7.87), 8.283 (8.02), 8.308 (4.21), 8.320 (3.78), 8.422 (7.53), 8.433 (10.61), 8.486 (5.98), 8.576 (12.70), 11.736 (8.69), 11.766 (4.79).

### Example 113

### 1-[(2S)-4-fluoro-2-({[4-(3-phenyl-1H-pyrrolo[3,2-b]pyridin-2-yl)pyridin-3-yl]oxy}methyl)-2,3-dihydro-1H-pyrrol-1-yl]prop-2-en-1-one

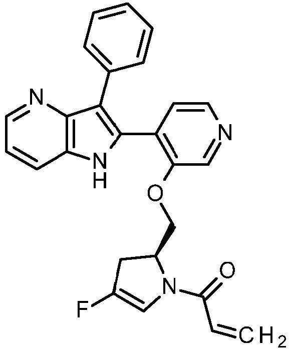

22.0 mg (93 % purity, 19 % yield) of the title compound were isolated from the reaction described in **Example 112.**
LC-MS (method 1): Rₜ = 0.91 min; MS (ESlpos): m/z = 441 [M+H]⁺
¹H-NMR (500 MHz, DMSO-d6) δ [ppm]: 11.76 and 11.69 (2s, 1H), 8.55 and 8.46 (2s, 1H), 8.45-8.40 (m, 1H), 8.33-8.21 (m, 1H), 7.86-7.77 (m, 1H), 7.59-7.49 (m, 2H), 7.40-7.16 (m, 5H), 6.45-6.34 and 6.31-6.23 (2m, 1H), 6.19-6.10 and 5.96-5.87 (2m, 1H), 5.70-5.64 and 5.01-4.96 (2m, 1H), 5.15-5.02 (m, 1H), 5.01-4.96 (m, 1H), 4.84-4.61 (m, 1H), 4.35-4.19 (m, 2H), 4.17-3.93 (m, 1H), 3.84-3.72 (m, 1H)

### Example 114

### 1-[(2S,4S)-4-fluoro-2-({[4-(3-phenyl-1H-pyrrolo[3,2-b]pyridin-2-yl)pyridin-3-yl]oxy}methyl)pyrrolidin-1-yl]prop-2-en-1-one

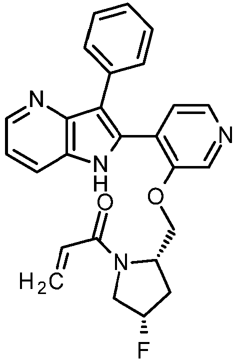

At rt, a mixture of 80 mg 2-(3-{[(2S,4S)-4-fluoropyrrolidin-2-yl]methoxy}pyridin-4-yl)-3-phenyl-1H-pyrrolo[3,2-b]pyridine and 2-(3-{[(2S)-2,5-dihydro-1H-pyrrol-2-yl]methoxy}pyridin-4-yl)-3-phenyl-1H-pyrrolo[3,2-b]pyridine (ratio 76:24) in DMF (1.5 ml) was treated with N,N-diisopropylethylamine (110 µl, 620 µmol) and prop-2-enoic acid (14 µl, 210 µmol). T3P (180 µl, 50 % purity in DMF, 310 µmol) was added and stirring at rt was continued for 45 min. Saturated NaHCO3 solution (aqueous, 0.5 mL) and water (1 ml) were added, and the mixture was extracted with EtOAc twice. The combined organic layers were dried over sodium sulfate, filtered and concentrated under reduced pressure. The residue was purified by reverse phase preparative HPLC (method 3) yielding 21.5 mg (100 % purity, 24 % yield) of the desired product.
An additional fraction was isolated that is described as **Example 115**
LC-MS (method 1): Rₜ = 0.93 min; MS (ESlpos): m/z = 443 [M+H]⁺
¹H-NMR (400 MHz, DMSO-d6) δ [ppm]: 1.757 (1.02), 1.795 (2.29), 1.845 (2.76), 1.903 (1.41), 1.942 (2.52), 1.991 (2.11), 2.073 (2.90), 2.155 (0.86), 2.327 (1.59), 2.331 (1.37), 2.366 (1.38), 2.523 (4.00), 2.669 (1.34), 2.709 (1.17), 3.423 (4.60), 3.460 (3.46), 3.492 (2.49), 3.529 (2.37), 3.669 (1.39), 3.681 (1.53), 3.706 (1.93), 3.737 (1.97), 3.753 (2.88), 3.776 (5.51), 3.800 (5.20), 3.827 (4.39), 3.842 (4.59), 3.872 (2.77), 3.901 (1.90), 3.918 (2.37), 3.937 (2.30), 3.954 (1.16), 4.015 (2.02), 4.038 (2.63), 4.055 (1.25), 4.222 (1.41), 4.243 (2.00), 4.255 (2.28), 4.367 (2.50), 4.379 (2.29), 4.392 (2.40), 4.403 (2.00), 4.771 (2.98), 4.777 (2.92), 4.796 (2.89), 4.803 (3.21), 5.163 (1.95), 5.195 (2.20), 5.299 (1.90), 5.327 (2.19), 5.655 (0.62), 5.680 (0.52), 5.716 (3.62), 5.722 (3.48), 5.742 (3.61), 5.747 (4.18), 5.769 (2.65), 5.775 (2.76), 5.811 (3.14), 5.817 (3.27), 6.126 (0.47), 6.190 (3.40), 6.196 (4.11), 6.226 (3.48), 6.232 (4.70), 6.238 (4.94), 6.267 (2.16), 6.327 (0.47), 6.352 (0.47), 6.504 (3.60), 6.530 (3.60), 6.546 (3.02), 6.572 (2.57), 7.207 (8.93), 7.218 (11.34), 7.228 (9.61), 7.239 (9.20), 7.250 (3.96), 7.264 (1.13), 7.292 (7.73), 7.300 (6.90), 7.311 (12.33), 7.320 (10.46), 7.336 (10.96), 7.348 (7.60), 7.409 (5.83), 7.420 (5.84), 7.515 (1.12), 7.544 (10.04), 7.561 (16.00), 7.579 (7.12), 7.826 (5.11), 7.833 (6.27), 7.846 (4.64), 7.853 (5.53), 8.166 (9.89), 8.216 (0.82), 8.228 (0.86), 8.262 (7.24), 8.274 (6.95), 8.321 (6.16), 8.333 (5.70), 8.417 (5.55), 8.421 (5.58), 8.428 (6.50), 8.432 (6.39), 8.441 (13.19), 8.554 (1.20), 8.642 (10.83), 11.649 (0.73), 11.743 (6.77), 11.792 (5.50).

### Example 115

### 1-[(2S)-2-({[4-(3-phenyl-1H-pyrrolo[3,2-b]pyridin-2-yl)pyridin-3-yl]oxy}methyl)-2,5-dihydro-1H-pyrrol-1-yl]prop-2-en-1-one

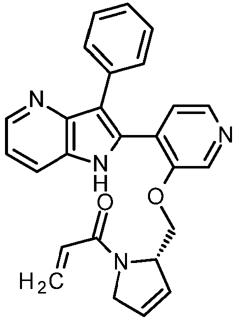

9.00 mg (100 % purity, 10 % yield) of the title compound were isolated from the reaction described in **Example 114.**
LC-MS (method 1): Rₜ = 0.92 min; MS (ESlpos): m/z = 423 [M+H]⁺
¹H-NMR (500 MHz, DMSO-d6) δ [ppm]: 11.74 and 11.66 (2s, 1H), 8.61-8.38 (m, 2H), 8.35-8.20 (m, 1H), 7.88-7.79 (m, 1H), 7.60-7.49 (m, 2H), 7.41-7.15 (m, 5H), 6.43-6.29 (m, 1H), 6.21-4.85 (m, 4H), 4.84-4.51 (m, 1H), 4.40-3.70 (m, 4H)

### Example 116

### 1-[(3RS)-3-{[4-(3-phenyl-1H-pyrrolo[3,2-b]pyridin-2-yl)pyridin-3-yl]oxy}pyrrolidin-1-yl]prop-2-en-1-one

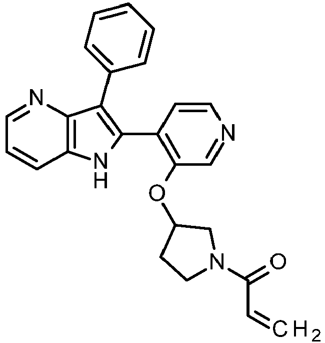

To a solution of 3-phenyl-2-(3-{[(3RS)-pyrrolidin-3-yl]oxy}pyridin-4-yl)-1H-pyrrolo[3,2-b]pyridine (31.0 mg, 87.0 µmol) in THF (1 ml), prop-2-enoic acid (6.0 µl, 87 µmol) and N,N-diisopropylethylamine (45 µl, 260 µmol) were added at rt. T3P (77 µl, 50 % purity in EtOAc, 130 µmol) was added and stirring at rt was continued for 1 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 16.6 mg (98 % purity, 46 % yield) of the desired product.
LC-MS (method 1): Rₜ = 0.79 min; MS (ESlpos): m/z = 411 [M+H]⁺
¹H-NMR (400 MHz, DMSO-d6) δ [ppm]: 1.170 (0.44), 1.187 (0.43), 1.231 (0.40), 1.388 (0.52), 1.627 (1.55), 1.645 (1.47), 1.653 (1.40), 1.686 (1.61), 1.703 (1.79), 1.720 (2.00), 1.734 (1.89), 1.893 (0.83), 1.906 (0.96), 1.916 (1.63), 1.928 (2.23), 1.939 (1.48), 1.950 (2.14), 1.963 (1.95), 1.975 (1.39), 1.987 (1.95), 1.999 (2.33), 2.011 (1.67), 2.023 (2.23), 2.034 (1.35), 2.046 (0.90), 2.057 (0.87), 2.071 (6.04), 2.366 (1.14), 2.709 (1.16), 2.954 (1.54), 2.971 (2.80), 2.979 (3.55), 2.997 (4.69), 3.021 (3.41), 3.040 (1.33), 3.122 (3.04), 3.152 (3.45), 3.160 (4.75), 3.174 (4.13), 3.245 (4.48), 3.279 (6.67), 3.383 (3.92), 3.455 (2.63), 3.479 (6.96), 3.491 (4.81), 3.513 (3.26), 3.525 (2.98), 3.651 (2.64), 3.663 (2.91), 3.680 (2.64), 3.692 (2.46), 4.080 (0.45), 4.092 (1.16), 4.106 (1.08), 4.119 (0.41), 4.985 (4.45), 5.018 (3.20), 5.558 (4.96), 5.564 (4.82), 5.584 (5.03), 5.590 (5.55), 5.604 (4.12), 5.610 (3.93), 5.629 (3.89), 5.635 (4.29), 5.998 (4.41), 6.004 (4.55), 6.023 (3.63), 6.029 (3.73), 6.040 (5.93), 6.046 (5.82), 6.064 (4.68), 6.070 (4.44), 6.315 (5.18), 6.329 (4.34), 6.341 (5.37), 6.356 (6.54), 6.371 (3.56), 6.383 (3.80), 6.397 (3.01), 7.127 (2.37), 7.146 (6.37), 7.164 (5.09), 7.173 (5.38), 7.191 (8.83), 7.195 (6.89), 7.202 (6.39), 7.207 (5.80), 7.211 (6.94), 7.216 (5.61), 7.222 (6.62), 7.227 (5.56), 7.237 (7.97), 7.257 (15.57), 7.278 (13.10), 7.297 (5.12), 7.441 (6.50), 7.453 (8.38), 7.476 (11.36), 7.491 (16.00), 7.508 (10.93), 7.549 (1.33), 7.565 (1.37), 7.572 (1.28), 7.595 (1.67), 7.613 (1.52), 7.625 (1.80), 7.642 (1.12), 7.792 (6.36), 7.795 (7.37), 7.800 (5.95), 7.803 (5.89), 7.812 (6.42), 7.815 (6.82), 7.820 (5.56), 7.824 (5.07), 8.178 (0.50), 8.337 (6.30), 8.347 (6.17), 8.404 (10.58), 8.407 (11.64), 8.415 (10.83), 8.418 (10.96), 8.495 (8.35), 11.708 (7.46).

### Example 117

### 1-[(3RS)-3-{[4-(3-phenyl-1H-pyrrolo[3,2-b]pyridin-2-yl)pyridin-3-yl]oxy}pyrrolidin-1-yl]prop-2-yn-1-one

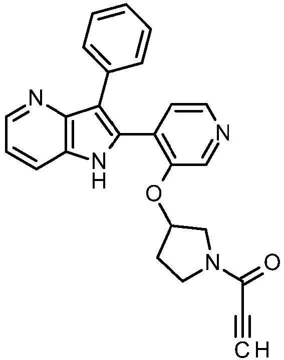

To a solution of 3-phenyl-2-(3-{[(3RS)-pyrrolidin-3-yl]oxy}pyridin-4-yl)-1H-pyrrolo[3,2-b]pyridine (33.0 mg, 92.6 µmol) in THF (1 ml), prop-2-ynoic acid (5.7 µl, 93 µmol) and N,N-diisopropylethylamine (48 µl, 280 µmol) were added at rt. T3P (82 µl, 50 % purity in EtOAc, 140 µmol) was added and stirring at rt was continued for 1 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 15.0 mg (100 % purity, 40 % yield) of the desired product.
LC-MS (method 1): Rₜ = 0.79 min; MS (ESlpos): m/z = 409 [M+H]⁺
¹H-NMR (400 MHz, DMSO-d6) δ [ppm]: 1.234 (0.44), 1.703 (3.90), 2.003 (3.82), 2.024 (3.59), 2.070 (11.35), 2.365 (1.04), 2.709 (0.82), 2.901 (1.50), 2.923 (2.35), 2.951 (2.62), 2.972 (1.57), 3.015 (1.65), 3.040 (3.27), 3.057 (3.21), 3.082 (1.80), 3.108 (4.26), 3.143 (5.09), 3.161 (3.45), 3.173 (3.46), 3.214 (4.58), 3.246 (5.80), 3.466 (3.73), 3.477 (4.01), 3.498 (4.59), 3.512 (5.64), 3.537 (2.13), 3.655 (2.90), 3.666 (3.10), 3.685 (2.76), 3.697 (2.54), 4.093 (0.71), 4.106 (0.66), 4.364 (14.04), 4.434 (13.11), 4.977 (3.97), 5.018 (4.47), 5.751 (0.91), 7.154 (2.56), 7.172 (6.61), 7.185 (7.35), 7.202 (10.12), 7.213 (7.48), 7.221 (7.24), 7.232 (6.79), 7.252 (7.56), 7.270 (15.22), 7.286 (14.14), 7.304 (5.91), 7.454 (7.58), 7.467 (12.93), 7.481 (16.00), 7.496 (15.98), 7.514 (10.48), 7.810 (10.52), 7.830 (9.76), 8.346 (9.00), 8.352 (8.79), 8.415 (11.47), 8.425 (10.93), 8.479 (11.02), 8.490 (10.67), 11.723 (8.52).

### Example 118

### 2-(3-{[(3RS)-1-(ethenesulfonyl)pyrrolidin-3-yl]oxy}pyridin-4-yl)-3-phenyl-1H-pyrrolo[3,2-b]pyridine

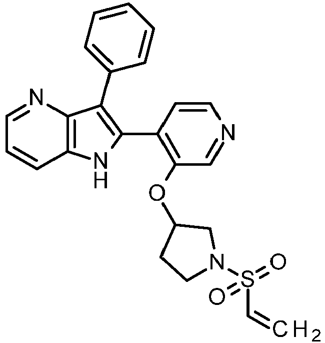

To a solution of 3-phenyl-2-(3-{[(3RS)-pyrrolidin-3-yl]oxy}pyridin-4-yl)-1H-pyrrolo[3,2-b]pyridine (55.0 mg, 154 µmol) and triethylamine (75 µl, 540 µmol) in DCM (1 ml), 2-chloroethane-1-sulfonyl chloride (16 µl, 150 µmol) was added at rt. After stirring at rt for 3 h, water (0.5 ml) and saturated NaHCO3 solution (aqueous, 0.5 mL) were added. 2 ml of DCM were added, and the mixture was dried over a water-repellent filter. After concentration under reduced pressure the residue was purified by reverse phase preparative HPLC (method 3) yielding 7.90 mg (98 % purity, 11 % yield) of the title compound.
LC-MS (method 1): Rₜ = 0.95 min; MS (ESlpos): m/z = 447 [M+H]⁺
¹H-NMR (400 MHz, DMSO-d6) δ [ppm]: 1.663 (1.82), 1.679 (2.01), 1.694 (2.29), 1.711 (2.26), 1.960 (1.07), 1.972 (1.24), 1.983 (1.89), 1.994 (2.67), 2.006 (2.05), 2.018 (2.62), 2.029 (1.61), 2.040 (1.08), 2.052 (1.00), 2.071 (6.62), 2.772 (1.81), 2.796 (4.05), 2.814 (3.94), 2.820 (2.69), 2.838 (1.88), 2.962 (5.18), 2.993 (5.86), 3.120 (2.50), 3.126 (2.73), 3.144 (4.30), 3.165 (3.10), 3.172 (3.41), 3.372 (9.19), 3.391 (6.04), 3.402 (5.02), 5.008 (5.18), 5.305 (9.79), 5.330 (10.41), 5.759 (9.06), 5.801 (11.37), 6.103 (5.83), 6.128 (5.87), 6.144 (4.91), 6.169 (4.37), 7.186 (2.75), 7.205 (6.93), 7.227 (8.28), 7.239 (6.02), 7.248 (6.13), 7.259 (6.14), 7.286 (8.56), 7.305 (14.25), 7.323 (6.76), 7.426 (9.66), 7.438 (9.90), 7.533 (14.03), 7.551 (11.94), 7.595 (0.72), 7.613 (0.64), 7.624 (0.83), 7.642 (0.52), 7.851 (6.86), 7.854 (7.44), 7.871 (6.54), 7.874 (6.70), 8.321 (10.24), 8.333 (9.76), 8.440 (7.07), 8.444 (7.70), 8.452 (7.20), 8.455 (7.17), 8.491 (16.00), 11.743 (1.33).

### Example 119

### 1-[(3RS)-3-({4-[3-(3-chlorophenyl)-1H-pyrrolo[3,2-b]pyridin-2-yl]pyridin-3-yl}oxy)pyrrolidin-1-yl]prop-2-en-1-one

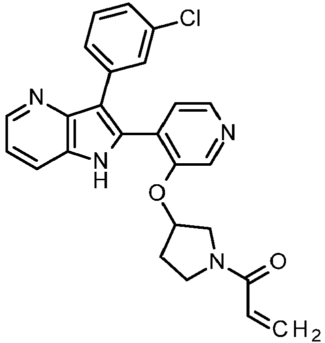

To a solution of 3-(3-chlorophenyl)-2-(3-{[(3RS)-pyrrolidin-3-yl]oxy}pyridin-4-yl)-1H-pyrrolo[3,2-b]pyridine (25.0 mg, 64.0 µmol) in THF (1 ml), prop-2-enoic acid (4.4 µl, 64 µmol) and N,N-diisopropylethylamine (33 µl, 190 µmol) were added at rt. T3P (57 µl, 50 % purity in EtOAc, 96 µmol) was added and stirring at rt was continued for 1 h. Additional prop-2-enoic acid (4.4 µl, 64 µmol) and T3P (57 µl, 50 % purity in EtOAc, 96 µmol) were added and stirring was continued for 1h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 11.0 mg (100 % purity, 39 % yield) of the desired product.
LC-MS (method 1): Rₜ = 1.00 min; MS (ESlpos): m/z = 445 [M+H]⁺
¹H-NMR (400 MHz, DMSO-d6) δ [ppm]: 1.229 (0.48), 1.465 (0.45), 1.723 (1.21), 1.950 (0.94), 1.963 (1.25), 1.985 (1.42), 2.024 (1.45), 2.049 (1.35), 2.925 (1.00), 2.950 (2.21), 2.966 (2.75), 2.993 (2.12), 3.013 (0.83), 3.126 (2.02), 3.167 (16.00), 3.232 (3.08), 3.267 (4.72), 3.477 (1.44), 3.502 (3.79), 3.515 (2.84), 3.537 (1.94), 3.549 (1.77), 3.680 (1.50), 3.693 (1.74), 3.709 (1.58), 3.722 (1.46), 5.018 (2.76), 5.555 (3.07), 5.561 (2.92), 5.580 (3.06), 5.586 (3.41), 5.595 (2.53), 5.601 (2.39), 5.621 (2.42), 5.627 (2.56), 5.991 (2.66), 5.997 (2.71), 6.019 (2.25), 6.025 (2.43), 6.033 (3.72), 6.039 (3.50), 6.061 (2.69), 6.067 (2.63), 6.299 (3.12), 6.325 (3.86), 6.341 (2.59), 6.354 (2.45), 6.367 (2.88), 6.395 (1.65), 7.166 (2.16), 7.187 (3.73), 7.210 (1.97), 7.221 (7.35), 7.226 (6.01), 7.232 (6.38), 7.241 (9.59), 7.247 (4.05), 7.253 (4.70), 7.258 (6.14), 7.276 (4.44), 7.296 (2.29), 7.315 (4.32), 7.334 (3.26), 7.342 (3.67), 7.362 (2.10), 7.497 (3.12), 7.510 (4.63), 7.523 (2.72), 7.681 (4.46), 7.740 (5.75), 7.815 (4.04), 7.819 (4.33), 7.824 (3.54), 7.828 (3.41), 7.836 (3.93), 7.839 (3.89), 7.845 (3.21), 8.386 (2.88), 8.447 (6.67), 8.456 (6.54), 8.529 (3.03), 11.853 (4.72), 11.863 (5.51).

### Example 120

### 1-[(3RS)-3-({4-[3-(3-chlorophenyl)-1H-pyrrolo[3,2-b]pyridin-2-yl]pyridin-3-yl}oxy)pyrrolidin-1-yl]prop-2-yn-1-one

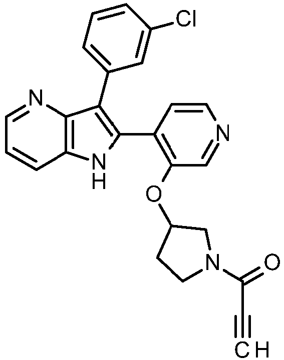

To a solution of 3-(3-chlorophenyl)-2-(3-{[(3RS)-pyrrolidin-3-yl]oxy}pyridin-4-yl)-1H-pyrrolo[3,2-b]pyridine (90.0 mg, 50 % purity, 115 µmol) in THF (1 ml), prop-2-ynoic acid (8.06 mg, 115 µmol) and N,N-diisopropylethylamine (60 µl, 350 µmol) were added at rt. T3P (100 µl, 50 % purity in EtOAc, 170 µmol;) was added and stirring at rt was continued for 16 h. Water (1 ml) was added, and the mixture was extracted with EtOAc twice. The combined organic layers were dried over sodium sulfate, filtered and concentrated under reduced pressure. The residue was purified by reverse phase preparative HPLC (method 3) yielding 13.3 mg (100 % purity, 26 % yield) of the desired product.
LC-MS (method 1): Rₜ = 1.00 min; MS (ESlpos): m/z = 443 [M+H]⁺
¹H-NMR (400 MHz, DMSO-d6) δ [ppm]: 0.907 (3.31), 0.917 (3.10), 0.935 (2.85), 0.952 (1.13), 1.477 (4.70), 1.711 (1.35), 2.003 (0.79), 2.014 (1.07), 2.026 (1.48), 2.037 (1.46), 2.048 (1.48), 2.060 (1.37), 2.369 (0.56), 2.712 (0.55), 2.870 (0.67), 2.891 (1.07), 2.922 (1.03), 2.941 (0.82), 2.982 (0.80), 3.008 (1.41), 3.026 (1.38), 3.052 (0.91), 3.113 (1.89), 3.148 (2.30), 3.244 (1.94), 3.274 (2.27), 3.311 (1.85), 3.332 (2.47), 3.363 (2.20), 3.516 (9.95), 3.736 (6.45), 3.755 (5.28), 3.767 (4.61), 4.360 (7.79), 4.406 (7.04), 4.428 (0.80), 5.029 (1.51), 5.058 (1.64), 7.199 (1.20), 7.221 (3.68), 7.235 (4.66), 7.240 (4.74), 7.244 (3.64), 7.258 (4.59), 7.265 (3.47), 7.274 (2.76), 7.294 (3.44), 7.314 (2.10), 7.343 (3.25), 7.362 (2.40), 7.398 (2.44), 7.418 (1.62), 7.509 (2.99), 7.515 (3.10), 7.520 (3.44), 7.526 (2.97), 7.636 (2.96), 7.744 (3.72), 7.837 (3.69), 7.857 (3.29), 7.982 (0.42), 8.144 (16.00), 8.390 (3.57), 8.400 (3.61), 8.457 (4.33), 8.461 (3.16), 8.464 (2.87), 8.468 (4.18), 8.517 (4.04), 8.534 (3.71), 11.881 (4.21).

### Example 121

### 3-(3-chlorophenyl)-2-(3-{[(3RS)-1-(ethenesulfonyl)pyrrolidin-3-yl]oxy}pyridin-4-yl)-1H-pyrrolo[3,2-b]pyridine

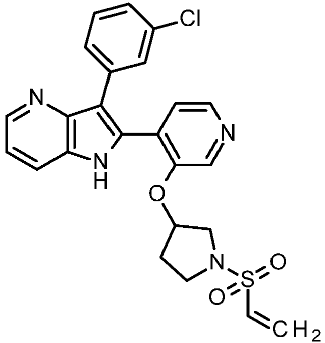

To a solution of 3-(3-chlorophenyl)-2-(3-{[(3RS)-pyrrolidin-3-yl]oxy}pyridin-4-yl)-1H-pyrrolo[3,2-b]pyridine (90.0 mg, 50 % purity, 115 µmol) and triethylamine (56 µl, 400 µmol) in DCM (1 ml), 2-chloroethane-1-sulfonyl chloride (12 µl, 120 µmol) was added at rt. After stirring at rt for 16 h, water (0.5 ml) was added and the solvent was removed under reduced pressure. The crude product was dissolved in a mixture of MeOH/DMSO (5 ml, 3:1) and filtered through fritted glass. After concentration under reduced pressure the DMSO containing residue was purified by reverse phase preparative HPLC (method 8) yielding 5.30 mg (100 % purity, 10 % yield) of the title compound.
LC-MS (method 1): Rₜ = 1.24 min; MS (ESlpos): m/z = 481 [M+H]⁺
¹H-NMR (500 MHz, DMSO-d6) δ [ppm]: 1.234 (0.49), 1.673 (2.17), 1.686 (2.36), 1.700 (2.63), 1.711 (2.56), 2.005 (1.21), 2.015 (1.43), 2.023 (2.14), 2.032 (3.36), 2.042 (2.60), 2.052 (3.33), 2.060 (1.91), 2.069 (1.31), 2.079 (1.14), 2.628 (1.07), 2.771 (2.36), 2.785 (3.15), 2.791 (5.26), 2.805 (5.16), 2.810 (3.32), 2.824 (2.41), 2.943 (6.53), 2.967 (7.08), 3.145 (2.94), 3.150 (3.20), 3.164 (5.06), 3.167 (4.86), 3.181 (2.96), 3.186 (2.62), 3.392 (5.44), 3.401 (6.11), 3.415 (5.50), 3.425 (5.03), 5.042 (6.31), 5.338 (13.51), 5.358 (14.29), 5.781 (12.85), 5.814 (15.61), 6.114 (7.63), 6.134 (7.70), 6.147 (6.64), 6.167 (6.06), 7.256 (4.58), 7.263 (8.61), 7.272 (16.00), 7.279 (9.07), 7.288 (8.56), 7.293 (8.85), 7.309 (12.97), 7.325 (6.05), 7.382 (9.61), 7.397 (6.91), 7.484 (8.88), 7.494 (9.11), 7.741 (8.46), 7.745 (13.37), 7.748 (8.45), 7.878 (8.66), 7.881 (8.99), 7.894 (8.43), 7.897 (8.24), 8.372 (4.76), 8.380 (4.70), 8.485 (8.50), 8.488 (8.82), 8.494 (8.83), 8.497 (8.52), 8.533 (6.19), 11.894 (12.39).

### Example 122

### 1-{(3RS)-3-[(4-{3-[3-(trifluoromethyl)phenyl]-1H-pyrrolo[3,2-b]pyridin-2-yl}pyridin-3-yl)oxy]pyrrolidin-1-yl}prop-2-en-1-one

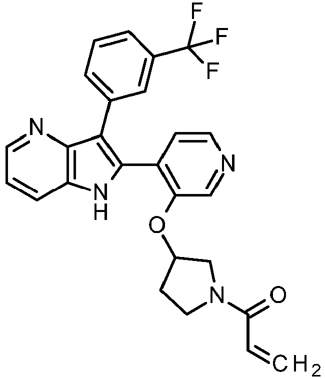

To a solution of 2-(3-{[(3RS)-pyrrolidin-3-yl]oxy}pyridin-4-yl)-3-[3-(trifluoromethyl)phenyl]-1H-pyrrolo[3,2-b]pyridine (42.0 mg, 99.0 µmol) in THF (1 ml), prop-2-enoic acid (6.8 µl, 99 µmol) and N,N-diisopropylethylamine (69 µl, 400 µmol) were added at rt. T3P (87 µl, 50 % purity in EtOAc, 150 µmol) was added and stirring at rt was continued for 16 h. Water (0.5 ml) was added, and the solvent was removed under reduced pressure. The residue was dissolved in 0.1 ml DMSO and the solution was purified by reverse phase preparative HPLC (method 8) yielding 15.0 mg (100 % purity, 32 % yield) of the desired product.
LC-MS (method 1): Rₜ = 1.16 min; MS (ESlpos): m/z = 479 [M+H]⁺
¹H-NMR (400 MHz, DMSO-d6) δ [ppm]: 0.974 (1.87), 0.989 (1.70), 1.509 (1.17), 1.526 (1.45), 1.543 (1.40), 1.552 (1.34), 1.591 (1.57), 1.607 (1.70), 1.625 (1.89), 1.637 (1.80), 1.888 (0.66), 1.900 (0.79), 1.910 (1.44), 1.922 (2.00), 1.933 (1.36), 1.945 (1.99), 1.957 (1.80), 1.970 (1.29), 1.981 (1.85), 1.994 (2.22), 2.005 (1.59), 2.018 (2.16), 2.029 (1.31), 2.041 (0.81), 2.052 (0.73), 2.367 (0.53), 2.711 (0.54), 2.868 (1.48), 2.885 (3.04), 2.892 (3.39), 2.910 (4.42), 2.935 (3.26), 2.955 (1.21), 3.063 (2.98), 3.092 (3.19), 3.178 (4.07), 3.212 (4.87), 3.348 (4.82), 3.370 (1.98), 3.378 (1.68), 3.449 (2.16), 3.475 (5.09), 3.489 (5.39), 3.511 (3.05), 3.523 (2.82), 3.671 (2.42), 3.684 (2.71), 3.700 (2.44), 3.713 (2.26), 4.987 (4.41), 5.020 (3.10), 5.536 (4.60), 5.542 (4.53), 5.562 (4.82), 5.568 (6.04), 5.577 (3.76), 5.597 (3.65), 5.603 (3.93), 5.979 (4.00), 5.985 (4.12), 6.006 (3.33), 6.012 (3.58), 6.021 (5.64), 6.026 (5.43), 6.048 (4.28), 6.054 (4.18), 6.263 (4.86), 6.280 (4.08), 6.289 (5.02), 6.306 (7.55), 6.321 (3.20), 6.331 (3.55), 6.347 (2.67), 7.236 (5.25), 7.241 (4.68), 7.247 (5.71), 7.253 (5.51), 7.256 (6.51), 7.262 (4.99), 7.268 (5.92), 7.273 (4.56), 7.452 (0.73), 7.472 (10.29), 7.485 (13.28), 7.504 (8.28), 7.517 (10.25), 7.530 (9.51), 7.542 (10.98), 7.557 (7.05), 7.757 (3.41), 7.766 (5.91), 7.779 (5.61), 7.836 (6.18), 7.839 (6.96), 7.844 (5.57), 7.848 (5.52), 7.856 (6.15), 7.859 (6.41), 7.865 (5.21), 7.868 (4.79), 7.889 (6.66), 7.950 (8.51), 8.226 (0.42), 8.395 (11.55), 8.398 (10.62), 8.407 (11.28), 8.462 (10.47), 8.465 (11.18), 8.474 (10.63), 8.477 (10.42), 8.524 (12.94), 8.531 (16.00), 11.918 (7.70), 11.927 (9.14).

### Example 123

### 1-{(3RS)-3-[(4-{3-[3-(trifluoromethyl)phenyl]-1H-pyrrolo[3,2-b]pyridin-2-yl}pyridin-3-yl)oxy]pyrrolidin-1-yl}prop-2-yn-1-one

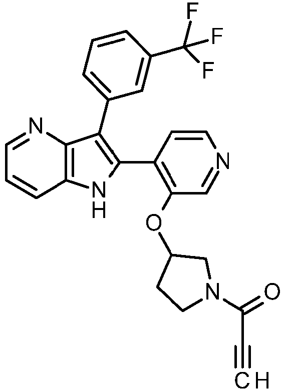

To a solution of 2-(3-{[(3RS)-pyrrolidin-3-yl]oxy}pyridin-4-yl)-3-[3-(trifluoromethyl)phenyl]-1H-pyrrolo[3,2-b]pyridine (40.0 mg, 94.2 µmol) in THF (1 ml), prop-2-ynoic acid (5.8 µl, 94 µmol) and N,N-diisopropylethylamine (66 µl, 380 µmol were added at rt. T3P (83 µl, 50 % purity in EtOAc, 140 µmol) was added and stirring at rt was continued for 16 h. Water (0.5 ml) was added, and the solvent was removed under reduced pressure. The residue was dissolved in 0.1 ml DMSO and the solution was purified by reverse phase preparative HPLC (method 8) yielding 7.20 mg (100 % purity, 16 % yield) of the desired product.
LC-MS (method 1): Rₜ = 1.17 min; MS (ESlpos): m/z = 477 [M+H]⁺
¹H-NMR (500 MHz, DMSO-d6) δ [ppm]: -0.120 (0.65), 0.117 (0.55), 0.959 (4.02), 0.972 (3.67), 1.160 (0.50), 1.175 (0.82), 1.189 (0.44), 1.234 (0.45), 1.599 (3.51), 1.612 (3.46), 1.962 (0.90), 1.980 (2.38), 1.989 (4.53), 1.998 (3.18), 2.007 (4.24), 2.016 (3.03), 2.025 (3.13), 2.034 (2.09), 2.052 (0.85), 2.816 (1.33), 2.834 (2.12), 2.856 (2.25), 2.873 (1.38), 2.914 (1.50), 2.934 (3.24), 2.948 (3.11), 2.968 (1.58), 3.045 (4.34), 3.072 (4.81), 3.154 (3.78), 3.179 (4.11), 3.281 (3.52), 3.474 (3.49), 3.483 (5.42), 3.502 (6.43), 3.520 (2.25), 3.701 (2.76), 3.710 (3.08), 3.725 (2.77), 3.734 (2.57), 4.347 (16.00), 4.394 (13.96), 4.995 (3.86), 5.024 (4.56), 5.757 (2.42), 7.248 (5.52), 7.251 (5.57), 7.257 (6.13), 7.260 (6.18), 7.264 (6.58), 7.267 (6.04), 7.274 (6.21), 7.276 (5.69), 7.471 (1.80), 7.487 (5.52), 7.502 (11.87), 7.520 (12.57), 7.528 (8.66), 7.544 (9.62), 7.553 (11.93), 7.560 (8.33), 7.769 (4.81), 7.783 (4.17), 7.814 (7.43), 7.855 (12.99), 7.871 (9.69), 7.953 (8.52), 8.272 (0.40), 8.402 (10.60), 8.405 (10.63), 8.412 (10.80), 8.414 (10.12), 8.472 (10.03), 8.476 (8.87), 8.482 (9.99), 8.514 (14.30), 8.529 (12.62), 11.939 (15.14).

### Example 124

### 1-[(3RS)-3-({4-[3-(2-fluoro-5-methylphenyl)-1H-pyrrolo[3,2-b]pyridin-2-yl]pyridin-3-yl}oxy)pyrrolidin-1-yl]prop-2-en-1-one

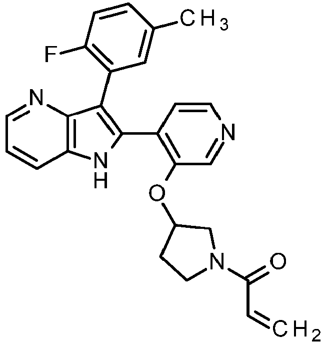

To a solution of 3-(2-fluoro-5-methylphenyl)-2-(3-{[(3RS)-pyrrolidin-3-yl]oxy}pyridin-4-yl)-1H-pyrrolo[3,2-b]pyridine hydrogen chloride (1/2) (54.0 mg, 117 µmol) in THF (1 ml), prop-2-enoic acid (8.0 µl, 120 µmol) and N,N-diisopropylethylamine (82 µl, 470 µmol) were added at rt. T3P (100 µl, 50 % purity in EtOAc, 180 µmol) was added and stirring at rt was continued for 1 h. Water (0.5 ml) was added, and the solvent was removed under reduced pressure. The residue was dissolved in 0.1 ml DMSO and the solution was purified by reverse phase preparative HPLC (method 8) yielding the title compound in insufficient purity. Further purification by thin layer chromatograpy (Eluent: DCM / MeOH 10:1) gave 18.8 mg (100 % purity, 36 % yield) of the desired product.
LC-MS (method 1): Rₜ = 0.88 min; MS (ESlpos): m/z = 443 [M+H]⁺
¹H-NMR (400 MHz, DMSO-d6) δ [ppm]: 1.681 (0.54), 1.780 (0.58), 1.977 (0.58), 1.989 (0.80), 2.000 (0.47), 2.011 (0.77), 2.022 (0.72), 2.033 (0.55), 2.045 (0.82), 2.056 (0.87), 2.074 (1.99), 2.090 (0.47), 2.292 (16.00), 2.328 (0.47), 3.119 (0.71), 3.129 (0.61), 3.150 (0.92), 3.163 (1.56), 3.176 (1.89), 3.202 (1.15), 3.220 (1.94), 3.250 (1.25), 3.271 (1.41), 3.367 (0.78), 3.376 (0.72), 3.389 (0.71), 3.398 (0.97), 3.406 (0.59), 3.418 (0.52), 3.428 (0.45), 3.512 (1.39), 3.524 (1.96), 3.546 (1.97), 3.558 (1.15), 3.570 (0.59), 3.739 (0.92), 3.752 (1.04), 3.768 (0.92), 3.781 (0.82), 5.002 (1.30), 5.058 (1.05), 5.559 (1.82), 5.566 (1.72), 5.585 (1.81), 5.591 (2.20), 5.595 (1.80), 5.601 (1.54), 5.621 (1.50), 5.627 (1.69), 6.018 (1.63), 6.024 (1.72), 6.034 (1.45), 6.039 (1.43), 6.060 (2.11), 6.066 (2.13), 6.075 (1.85), 6.081 (1.74), 6.363 (2.56), 6.389 (2.53), 6.405 (2.11), 6.431 (1.82), 6.940 (1.28), 6.946 (1.19), 6.961 (2.07), 6.966 (2.63), 6.986 (1.86), 6.992 (1.56), 7.061 (1.55), 7.067 (1.69), 7.074 (1.86), 7.088 (1.18), 7.094 (1.04), 7.198 (2.07), 7.203 (1.87), 7.209 (2.19), 7.214 (2.02), 7.219 (2.27), 7.223 (1.94), 7.230 (2.21), 7.235 (1.85), 7.313 (3.32), 7.325 (3.37), 7.339 (2.85), 7.351 (2.82), 7.427 (2.30), 7.440 (2.33), 7.820 (2.25), 7.824 (3.46), 7.828 (2.08), 7.840 (2.17), 7.844 (3.18), 8.273 (3.59), 8.285 (4.27), 8.294 (2.94), 8.385 (3.85), 8.396 (3.68), 8.485 (4.57), 8.495 (5.21), 11.781 (2.44), 11.791 (2.67).

### Example 125

### 1-[(3RS)-3-({4-[3-(2-fluoro-5-methylphenyl)-1H-pyrrolo[3,2-b]pyridin-2-yl]pyridin-3-yl}oxy)pyrrolidin-1-yl]prop-2-yn-1-one

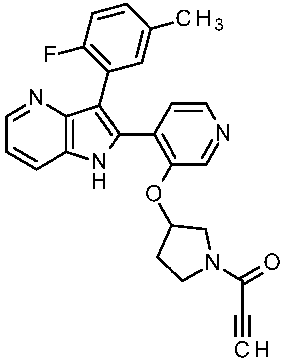

To a solution of 3-(2-fluoro-5-methylphenyl)-2-(3-{[(3RS)-pyrrolidin-3-yl]oxy}pyridin-4-yl)-1H-pyrrolo[3,2-b]pyridine hydrogen chloride (1/2) (54.0 mg, 117 µmol) in THF (1 ml), prop-2-ynoic acid (7.2 µl, 120 µmol) and N,N-diisopropylethylamine (61 µl, 350 µmol) were added at rt. T3P (100 µl, 50 % purity in EtOAc, 180 µmol) was added and stirring at rt was continued for 1 h. Water (0.5 ml) was added, and the solvent was removed under reduced pressure. The residue was dissolved in 0.1 ml DMSO and the solution was purified by reverse phase preparative HPLC (method 8) yielding the title compound in insufficient purity. Further purification by thin layer chromatograpy (Eluent: DCM / MeOH 10:1) gave 13.8 mg (100 % purity, 27 % yield) of the desired product.
LC-MS (method 1): Rₜ = 0.88 min; MS (ESlpos): m/z = 441 [M+H]⁺
¹H-NMR (400 MHz, DMSO-d6) δ [ppm]: 1.729 (1.40), 1.740 (1.43), 1.749 (1.44), 2.007 (0.41), 2.029 (1.14), 2.042 (1.54), 2.051 (1.42), 2.063 (2.09), 2.075 (1.34), 2.085 (1.45), 2.097 (0.95), 2.297 (15.24), 2.307 (16.00), 2.670 (0.59), 3.026 (0.60), 3.046 (1.07), 3.056 (0.91), 3.077 (1.26), 3.096 (0.71), 3.137 (1.80), 3.171 (2.22), 3.200 (0.71), 3.225 (1.43), 3.243 (1.44), 3.267 (0.91), 3.370 (1.00), 3.379 (0.86), 3.500 (1.51), 3.512 (1.67), 3.534 (1.47), 3.546 (2.07), 3.573 (1.46), 3.592 (0.84), 3.600 (0.77), 3.763 (1.30), 3.775 (1.45), 3.794 (1.29), 3.806 (1.18), 4.380 (8.62), 4.430 (7.93), 5.030 (3.34), 6.942 (1.58), 6.951 (1.60), 6.963 (2.57), 6.967 (2.52), 6.972 (2.74), 6.988 (2.27), 6.997 (2.04), 7.080 (2.47), 7.087 (2.73), 7.106 (1.65), 7.207 (3.30), 7.218 (3.47), 7.227 (3.57), 7.238 (3.47), 7.334 (4.09), 7.346 (7.57), 7.358 (3.79), 7.414 (1.73), 7.427 (1.80), 7.451 (1.89), 7.465 (1.88), 7.835 (5.17), 7.855 (4.71), 8.284 (4.62), 8.292 (5.15), 8.295 (5.40), 8.304 (4.06), 8.393 (5.23), 8.403 (5.08), 8.479 (7.10), 8.489 (6.52), 11.808 (5.52).

### Example 126

### 2-(3-{[(3RS)-1-(ethenesulfonyl)pyrrolidin-3-yl]oxy}pyridin-4-yl)-3-(2-fluoro-5-methylphenyl)-1H-pyrrolo[3,2-b]pyridine

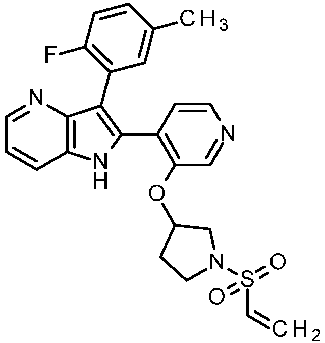

To a solution of 3-(2-fluoro-5-methylphenyl)-2-(3-{[(3RS)-pyrrolidin-3-yl]oxy}pyridin-4-yl)-1H-pyrrolo[3,2-b]pyridine hydrogen chloride (1/2) (54.0 mg, 117 µmol) and triethylamine (57 µl, 410 µmol) in DCM (1 ml), 2-chloroethane-1-sulfonyl chloride (12 µl, 120 µmol) was added at rt. After stirring at rt for 3 h, water (0.5 ml) was added and the solvent was removed under reduced pressure. The crude product was dissolved in a mixture of MeOH/DMSO (5 ml, 3:1) and filtered through fritted glass. After concentration under reduced pressure the DMSO containing residue was purified by reverse phase preparative HPLC (method 8) yielding 10.2 mg (91 % purity, 17 % yield) of the title compound.
LC-MS (method 1): Rₜ = 1.06 min; MS (ESlpos): m/z = 479 [M+H]⁺
¹H-NMR (500 MHz, DMSO-d6) δ [ppm]: 1.754 (1.86), 2.046 (1.28), 2.055 (1.05), 2.064 (1.26), 2.317 (16.00), 2.597 (0.66), 2.760 (0.55), 2.903 (0.73), 2.922 (1.74), 2.937 (1.71), 2.955 (0.82), 3.092 (2.33), 3.116 (2.66), 3.177 (1.31), 3.192 (2.00), 3.209 (1.13), 3.420 (1.89), 3.429 (2.04), 3.444 (1.78), 3.453 (1.67), 5.058 (2.63), 5.352 (3.02), 5.372 (3.18), 5.798 (2.82), 5.831 (3.32), 6.194 (1.66), 6.214 (1.72), 6.227 (1.55), 6.247 (1.36), 6.977 (1.36), 6.995 (2.49), 7.013 (1.74), 7.118 (2.09), 7.235 (1.95), 7.244 (2.20), 7.251 (2.26), 7.260 (2.04), 7.288 (3.70), 7.298 (3.78), 7.481 (2.27), 7.494 (2.43), 7.881 (2.95), 7.897 (2.68), 8.256 (3.47), 8.265 (3.58), 8.417 (3.13), 8.425 (3.02), 8.480 (5.82), 11.805 (3.52).

### Example 127

### 1-[(3RS)-3-({4-[3-(5-chloro-2-fluorophenyl)-1H-pyrrolo[3,2-b]pyridin-2-yl]pyridin-3-yl}oxy)pyrrolidin-1-yl]prop-2-en-1-one

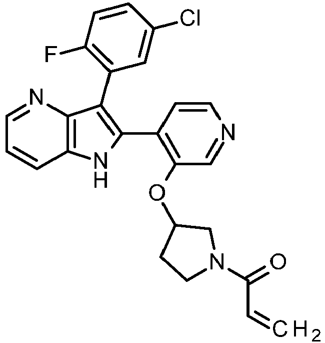

To a solution of 3-(5-chloro-2-fluorophenyl)-2-(3-{[(3RS)-pyrrolidin-3-yl]oxy}pyridin-4-yl)-1H-pyrrolo[3,2-b]pyridinehydrogen chloride (1/2) (42.0 mg, 87.2 µmol) in THF (1 ml), prop-2-enoic acid (6.0 µl, 87 µmol) and N,N-diisopropylethylamine (61 µl, 350 µmol) were added at rt. T3P (77 µl, 50 % purity in EtOAc, 130 µmol) was added and stirring at rt was continued for 16 h. Water (0.5 ml) was added, and the solvent was removed under reduced pressure. The residue was dissolved in 0.1 ml DMSO and the solution was purified by reverse phase preparative HPLC (method 8) yielding 27.7 mg (100 % purity, 69 % yield) of the desired product.
LC-MS (method 1): Rₜ = 0.99 min; MS (ESlpos): m/z = 463 [M+H]⁺
¹H-NMR (400 MHz, DMSO-d6) δ [ppm]: 1.654 (1.48), 1.753 (3.57), 1.987 (1.33), 1.999 (1.83), 2.021 (1.90), 2.070 (4.70), 2.083 (1.93), 2.365 (0.84), 2.709 (0.70), 3.069 (1.09), 3.089 (1.97), 3.119 (2.35), 3.139 (1.73), 3.161 (3.67), 3.173 (5.45), 3.200 (4.39), 3.233 (4.05), 3.273 (6.24), 3.526 (4.19), 3.537 (3.88), 3.554 (3.52), 3.571 (3.48), 3.726 (2.09), 3.739 (2.27), 3.754 (2.01), 3.767 (1.89), 4.095 (0.69), 4.107 (0.65), 5.023 (3.50), 5.071 (2.76), 5.543 (3.17), 5.548 (3.23), 5.574 (3.70), 5.587 (2.95), 5.593 (2.85), 5.614 (2.87), 5.618 (3.01), 5.992 (2.89), 5.997 (2.99), 6.017 (2.71), 6.022 (2.80), 6.034 (3.93), 6.039 (3.79), 6.059 (3.25), 6.063 (3.15), 6.343 (3.18), 6.353 (3.00), 6.369 (3.32), 6.379 (3.37), 6.384 (3.37), 6.395 (2.49), 6.410 (2.52), 6.421 (2.11), 7.124 (2.51), 7.138 (2.74), 7.147 (5.03), 7.160 (4.50), 7.171 (3.60), 7.183 (2.75), 7.230 (3.61), 7.241 (4.46), 7.249 (5.42), 7.256 (4.31), 7.261 (4.57), 7.267 (3.58), 7.315 (2.95), 7.339 (4.39), 7.362 (2.37), 7.378 (6.05), 7.390 (5.93), 7.410 (4.98), 7.422 (4.98), 7.714 (2.76), 7.720 (3.11), 7.735 (5.38), 7.749 (3.55), 7.756 (3.11), 7.847 (6.09), 7.867 (5.69), 8.313 (6.34), 8.324 (10.04), 8.335 (5.45), 8.419 (8.11), 8.429 (7.98), 8.512 (16.00), 11.952 (4.74).

### Example 128

### 1-[(3R)-3-({4-[3-(5-chloro-2-fluorophenyl)-1H-pyrrolo[3,2-b]pyridin-2-yl]pyridin-3-yl}oxy)pyrrolidin-1-yl]prop-2-yn-1-one

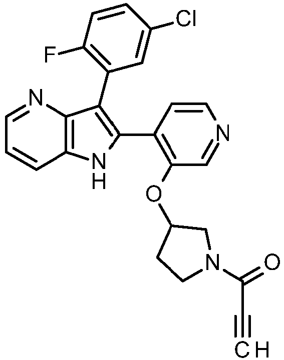

To a solution of 3-(5-chloro-2-fluorophenyl)-2-(3-{[(3R)-pyrrolidin-3-yl]oxy}pyridin-4-yl)-1H-pyrrolo[3,2-b]pyridine hydrogen chloride (1/2) (42.0 mg, 87.2 µmol) in THF (1 ml), prop-2-ynoic acid (5.4 µl, 87 µmol) and N,N-diisopropylethylamine (61 µl, 350 µmol) were added at rt. T3P (77 µl, 50 % purity in EtOAc, 130 µmol) was added and stirring at rt was continued for 1 h. Additional prop-2-ynoic acid (5.4 µl, 87 µmol) and T3P (77 µl, 50 % purity in EtOAc, 130 µmol) were added and stirring was continued for 1 h. Water (0.5 ml) was added, and the solvent was removed under reduced pressure. The residue was dissolved in 0.1 ml DMSO and the solution was purified by reverse phase preparative HPLC (method 8) yielding the desired compound with insufficient purity. Further purification by thin layer chromatograpy (Eluent: DCM / MeOH 10:1) gave 12.3 mg (100 % purity, 31 % yield) of the desired product.
LC-MS (method 1): Rₜ = 0.99 min; MS (ESlpos): m/z = 461 [M+H]⁺
¹H-NMR (400 MHz, DMSO-d6) δ [ppm]: 1.233 (0.67), 1.730 (3.05), 1.884 (0.41), 2.043 (2.18), 2.055 (3.00), 2.064 (2.76), 2.077 (3.83), 2.089 (2.53), 2.097 (2.81), 2.110 (1.77), 2.131 (0.73), 2.367 (0.44), 3.011 (1.24), 3.032 (2.16), 3.040 (1.85), 3.061 (2.51), 3.082 (1.39), 3.163 (5.58), 3.175 (2.43), 3.196 (5.42), 3.219 (3.11), 3.237 (3.09), 3.261 (2.02), 3.353 (5.19), 3.362 (6.20), 3.520 (3.07), 3.532 (3.40), 3.555 (4.07), 3.565 (4.19), 3.584 (3.16), 3.605 (1.72), 3.770 (2.67), 3.781 (3.01), 3.799 (2.59), 3.812 (2.39), 4.084 (0.50), 4.361 (16.00), 4.405 (14.74), 5.061 (6.70), 7.136 (3.52), 7.145 (3.57), 7.159 (6.54), 7.168 (6.20), 7.182 (4.67), 7.191 (4.05), 7.243 (6.29), 7.254 (6.60), 7.263 (6.67), 7.274 (6.44), 7.332 (2.66), 7.341 (5.43), 7.349 (5.88), 7.361 (5.16), 7.370 (4.57), 7.379 (2.77), 7.388 (8.27), 7.401 (9.30), 7.405 (8.96), 7.418 (7.45), 7.699 (3.58), 7.706 (3.75), 7.714 (3.89), 7.721 (3.55), 7.748 (4.06), 7.755 (4.25), 7.764 (4.23), 7.770 (3.83), 7.863 (10.97), 7.883 (9.98), 8.321 (9.11), 8.332 (12.00), 8.342 (7.94), 8.430 (10.89), 8.440 (10.42), 8.510 (13.92), 8.525 (12.95), 11.970 (10.58).

### Example 129

### 1-{(2S)-2-[({4-[3-(5-chloro-2-fluorophenyl)-1H-pyrrolo[3,2-b]pyridin-2-yl]-5-fluoropyridin-3-yl}oxy)methyl]pyrrolidin-1-yl}prop-2-en-1-one

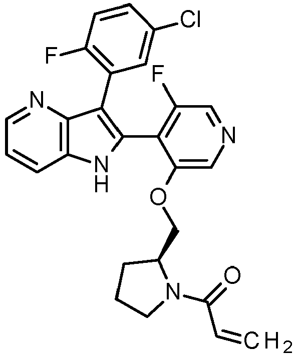

To a solution of 3-(5-chloro-2-fluorophenyl)-2-(3-fluoro-5-{[(2S)-pyrrolidin-2-yl]methoxy}pyridin-4-yl)-1H-pyrrolo[3,2-b]pyridine / hydrogen chloride (1/1) (8.50 mg, 17.8 µmol) in DMF (1 ml), prop-2-enoic acid (6.0 µl, 87 µmol) and N,N-diisopropylethylamine (19 µl, 110 µmol) were added at rt. T3P (16 µl, 50 % purity in DMF, 27 µmol) was added and stirring at rt was continued for 1 h. Water (0.5 ml) was added, and the solvent was removed under reduced pressure. The residue was dissolved in 0.1 ml DMSO and the solution was purified by reverse phase preparative HPLC (method 8) yielding 5.00 mg (91 % purity, 52 % yield) of the desired product.
LC-MS (method 1): Rₜ = 1.31 min; MS (ESlpos): m/z = 495 [M+H]⁺
¹H-NMR (400 MHz, DMSO-d6) δ [ppm]: 0.907 (3.62), 1.170 (4.69), 1.477 (6.09), 1.553 (16.00), 1.656 (8.12), 1.822 (2.94), 2.069 (10.01), 2.329 (5.21), 2.367 (5.04), 2.668 (5.19), 2.708 (3.74), 3.916 (4.51), 4.057 (5.26), 4.127 (13.41), 4.192 (7.20), 4.870 (2.71), 4.895 (2.80), 5.630 (6.08), 5.654 (6.56), 5.831 (2.79), 6.088 (5.26), 6.126 (6.61), 6.395 (4.24), 6.421 (4.68), 6.437 (4.14), 6.462 (3.19), 7.160 (5.11), 7.183 (9.31), 7.205 (7.60), 7.285 (9.28), 7.371 (7.96), 7.707 (10.76), 7.881 (8.64), 7.899 (9.41), 8.131 (15.67), 8.360 (14.90), 8.390 (8.54), 8.406 (8.88), 8.460 (13.40), 8.499 (13.77), 12.087 (10.84), 12.113 (6.25).

### Example 130

### 1-[(2S)-2-({[4-(3-phenyl-1H-pyrrolo[3,2-b]pyridin-2-yl)pyridin-3-yl]oxy}methyl)piperidin-1-yl]prop-2-en-1-one

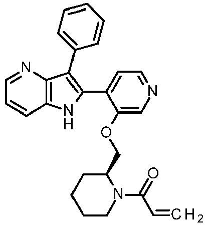

To a solution of 3-phenyl-2-(3-{[(2S)-piperidin-2-yl]methoxy}pyridin-4-yl)-1H-pyrrolo[3,2-b]pyridine (20.0 mg, 86 % purity, 44.7 µmol) in DMF (1 ml), prop-2-enoic acid (3.4 µl, 49 µmol; CAS-RN:[79-10-7]) and N,N-diisopropylethylamine (23 µl, 130 µmol) were added at rt. T3P (40 µl, 50 % purity in DMF, 67 µmol) was added and stirring at rt was continued for 16 h. Water (0.5 ml) was added, and the solvent was removed under reduced pressure. The residue was dissolved in 0.1 ml DMSO and the solution was purified by reverse phase preparative HPLC (method 3) yielding 3.00 mg (95 % purity, 15 % yield) of the desired product.
LC-MS (method 1): Rₜ = 1.05 min; MS (ESlpos): m/z = 439 [M+H]⁺
¹H-NMR (400 MHz, DMSO-d6) δ [ppm]: 0.912 (4.72), 0.929 (10.35), 0.947 (11.11), 0.962 (6.76), 1.235 (2.96), 1.275 (2.20), 1.303 (1.98), 1.500 (6.54), 1.626 (5.01), 1.646 (4.28), 1.823 (1.17), 2.329 (1.83), 2.368 (1.49), 2.671 (1.54), 2.712 (1.04), 2.813 (1.57), 2.847 (1.70), 2.865 (1.66), 3.330 (14.10), 3.592 (4.48), 3.902 (2.09), 3.925 (2.30), 4.632 (1.75), 5.056 (1.75), 5.082 (1.85), 5.703 (2.36), 5.734 (2.73), 5.754 (16.00), 5.814 (1.62), 5.849 (1.95), 6.177 (2.25), 6.214 (2.59), 6.253 (1.57), 6.279 (1.51), 6.295 (1.40), 6.320 (1.26), 6.707 (2.14), 6.733 (2.14), 6.749 (2.10), 6.775 (1.84), 7.197 (2.89), 7.216 (5.70), 7.226 (7.34), 7.246 (5.97), 7.280 (3.52), 7.299 (6.24), 7.308 (6.53), 7.327 (8.19), 7.346 (4.42), 7.383 (3.54), 7.452 (2.59), 7.497 (7.19), 7.517 (9.89), 7.536 (5.23), 7.830 (3.88), 7.850 (3.91), 8.272 (1.88), 8.413 (4.12), 8.423 (4.97), 8.508 (1.38), 8.693 (1.71), 11.678 (3.44), 11.759 (2.58).

### Example 131

### 1-[(2S,4RS)-4-{[2-(dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-one

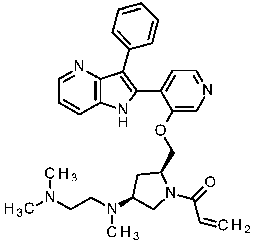

A solution of 1-[(2S,4S)-4-bromo-2-({[4-(3-phenyl-1H-pyrrolo[3,2-b]pyridin-2-yl)pyridin-3-yl]oxy}methyl)pyrrolidin-1-yl]prop-2-en-1-one (54.0 mg, 107 µmol) in ethanol (2.0 ml) was treated with N1,N1,N2-trimethylethane-1,2-diamine (280 µl, 2.1 mmol) at rt. The mixture was stirred at reflux for 18 h. After cooling to rt, the solvent was removed under reduced pressure and the remaing material was purified by reverse phase preparative HPLC (method 3) yielding two isomers of the desired product.
Isomer 1: 36.8 mg (98 % purity, 64 % yield)
LC-MS (method 1): Rₜ = 0.62 min; MS (ESlneg): m/z = 523 [M-H]⁻
¹H-NMR (400 MHz, DMSO-d6) δ [ppm]: 1.929 (1.16), 1.991 (0.52), 2.043 (3.20), 2.060 (6.32), 2.074 (16.00), 2.092 (0.63), 2.142 (0.41), 2.169 (0.46), 2.211 (0.57), 2.226 (1.08), 2.230 (0.82), 2.243 (1.55), 2.259 (0.51), 2.294 (1.35), 2.306 (0.73), 2.313 (0.83), 2.325 (0.43), 2.479 (0.44), 2.523 (0.49), 4.183 (0.46), 4.196 (0.45), 4.292 (0.46), 4.300 (0.51), 5.552 (0.44), 5.557 (0.42), 5.567 (0.59), 5.573 (0.53), 5.636 (0.54), 5.640 (0.52), 5.755 (1.72), 7.192 (0.84), 7.202 (0.83), 7.213 (1.12), 7.222 (0.88), 7.234 (0.80), 7.247 (1.22), 7.259 (1.26), 7.280 (1.00), 7.299 (1.63), 7.317 (0.79), 7.524 (1.45), 7.542 (1.52), 7.545 (1.15), 7.821 (0.82), 7.824 (0.83), 7.841 (0.78), 7.844 (0.70), 8.211 (1.48), 8.223 (1.40), 8.413 (0.86), 8.417 (0.87), 8.424 (0.97), 8.428 (0.79), 8.536 (1.98), 11.678 (0.73).
Isomer 2 is described as **example 132**

### Example 132

### 1-[(2S,4RS)-4-{[2-(dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-one

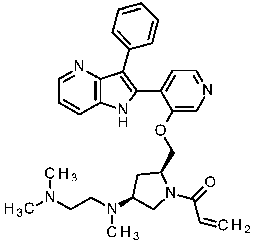

2.90 mg (95 % purity, 5 % yield) of isomer 2 from the reaction described in **example 131** were obtained.
LC-MS (method 1): Rₜ = 0.65 min; MS (ESlneg): m/z = 523 [M-H]⁻
¹H-NMR (500 MHz, CDCl₃) δ [ppm]: 1.258 (0.59), 1.952 (0.90), 2.044 (0.99), 2.147 (2.80), 2.158 (16.00), 2.212 (7.35), 2.242 (1.20), 2.299 (0.52), 2.307 (0.66), 2.314 (1.14), 2.321 (1.17), 2.328 (0.89), 2.333 (0.87), 2.358 (0.64), 2.361 (0.64), 2.393 (0.60), 2.396 (0.60), 2.430 (1.39), 2.444 (2.15), 2.458 (1.00), 2.577 (0.83), 2.591 (1.93), 2.746 (0.53), 2.760 (0.76), 2.771 (0.64), 2.774 (0.59), 2.786 (0.89), 2.800 (0.47), 4.211 (0.57), 4.217 (0.67), 4.230 (0.73), 4.235 (0.70), 4.355 (0.61), 4.370 (0.76), 4.373 (0.69), 4.388 (0.50), 5.083 (0.50), 5.089 (0.49), 5.303 (0.76), 5.307 (0.77), 5.311 (0.77), 6.559 (0.85), 7.163 (0.82), 7.172 (0.92), 7.180 (0.87), 7.188 (0.82), 7.224 (1.34), 7.234 (1.33), 7.309 (0.47), 7.324 (0.99), 7.339 (0.65), 7.405 (1.19), 7.420 (2.05), 7.435 (1.00), 7.543 (1.96), 7.557 (1.75), 7.961 (0.88), 7.964 (0.98), 7.978 (0.87), 7.980 (0.92), 8.027 (1.40), 8.037 (1.40), 8.371 (2.28), 8.525 (0.95), 8.527 (1.15), 8.534 (1.01), 8.536 (1.07), 10.896 (0.82).

### Example 133

### N-methyl-N-[2-{[4-(3-phenyl-1H-pyrrolo[3,2-b]pyridin-2-yl)pyridin-3-yl]oxy}propyl]prop-2-enamide

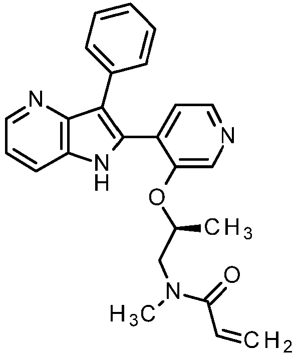

To a solution of N-methyl-2-{[4-(3-phenyl-1H-pyrrolo[3,2-b]pyridin-2-yl)pyridin-3-yl]oxy}propan-1-amine hydrogen chloride (1/1) (150 mg, 0.380 mmol) and prop-2-enoyl chloride (68.8 mg, 0.760 mmol) in dichloromethane (6 ml) was added triethylamine (76.9 mg, 0.760 mmol) at 0 °C. The mixture was stirred at 0 °C for 1 hour. The reaction mixture was concentrated in vacuo to give a residue. The residue was purified by preperative HPLC (column: Phenomenex luna C18 150*25 mm*10 µm; mobile phase: water (trifluoroacetic acid)-acetonitrile; B%:4%-34%, 10 min) to give N-methyl-N-[2-{[4-(3-phenyl-1H-pyrrolo[3,2-b]pyridin-2-yl)pyridin-3-yl]oxy}propyl]prop-2-enamide (28.5 mg, 0.675 mmol, 98% purity, 18% yield) as a yellow solid.

LCMS (Method C): Rₜ = 0.714 min; MS (ESlpos): m/z = 413.2 [M+H]⁺.

¹H NMR (400 MHz, DMSO-d₆), δ [ppm] = 11.40 (br s, 1H), 8.51-8.39 (m, 2H), 8.27-8.19 (m, 1H), 7.83 (d, *J* = 8.0 Hz, 1H), 7.55 (d, *J* = 7.2 Hz, 2H), 7.36 (br s, 1H), 7.30 (t, *J* = 8.0 Hz, 2H), 7.23-7.15 (m, 2H), 6.62-6.20 (m, 1H), 6.09-5.42 (m, 2H), 4.73 (br s, 1H), 3.38 (br s, 2H), 2.73 (br s, 3H), 1.05-0.80 (m, 3H).

### Example 134

### N-methyl-N-[-2-{[4-(3-phenyl-1H-pyrrolo[3,2-b]pyridin-2-yl)pyridin-3-yl]oxy}propyl]ethenesulfonamide

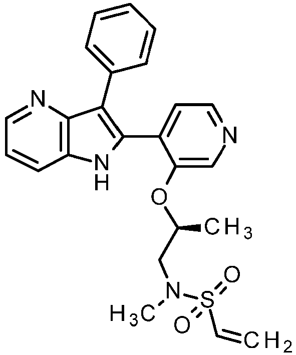

To a solution of N-methyl-2-{[4-(3-phenyl-1H-pyrrolo[3,2-b]pyridin-2-yl)pyridin-3-yl]oxy}propan-1-amine hydrogen chloride (1/1) (150 mg, 0.380 mmol) and ethenesulfonyl chloride (96.1 mg, 0.760 mmol) in dichloromethane (10 ml) was added triethylamine (76.9 mg, 0.760 mmol) at -20 °C. The mixture was stirred at -20 °C for 1 hour. The reaction mixture was concentrated in vacuo to give a residue. The residue was purified by preperative-HPLC (column: Phenomenex luna C18 150*25 mm*10 µm; mobile phase: water (trifluoroacetic acid)- acetonitrile; B%:10%-40%, 10 min) to give N-methyl-N-[2-{[4-(3-phenyl-1H-pyrrolo[3,2-b]pyridin-2-yl)pyridin-3-yl]oxy}propyl]ethenesulfonamide (23.5 mg, 0.0515 mmol, 98% purity, 14% yield) as a yellow solid.

LCMS (Method C): Rₜ = 0.407 min; MS (ESlpos): m/z = 449.1 [M+H]⁺.

¹H NMR (400 MHz, DMSO-d₆), δ [ppm] = 11.67 (s, 1H), 8.49 (s, 1H), 8.41 (dd, *J* = 4.8, 1.2 Hz, 1H), 8.30 (d, *J* = 4.8 Hz, 1H), 7.82 (dd, *J* = 8.0, 1.2 Hz, 1H), 7.49 (d, *J* = 7.2 Hz, 2H), 7.45 (d, *J =* 4.8 Hz, 1H), 7.30 (t, *J =* 7.6 Hz, 2H), 7.24-7.17 (m, 2H), 6.60-6.50 (m, 1H), 5.99-5.92 (m, 2H), 4.72-4.62 (m, 1H), 2.93-2.78 (m, 2H), 2.42 (s, 3H), 0.88 (d, *J* = 6.0 Hz, 3H).

### Example 135

### 1-[(2S)-2-({[4-(7-methyl-3-phenyl-1H-pyrrolo[3,2-b]pyridin-2-yl)pyridin-3-yl]oxy}methyl)pyrrolidin-1-yl]prop-2-en-1-one

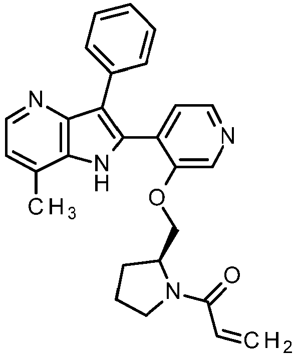

To a solution of 7-methyl-3-phenyl-2-(3-{[(2S)-pyrrolidin-2-yl]methoxy}pyridin-4-yl)-1H-pyrrolo[3,2-b]pyridine hydrogen chloride (1/1) (100 mg, 0.238 mmol) in dichloromethane (6 ml) were slowly added triethylamine (72.1 mg, 0.713 mmol) and a solution of prop-2-enoyl chloride (43.0 mg, 0.475 mmol) in dichloromethane (3 ml) at -20 °C. The mixture was stirred at -20 °C for 1 hours. After adding a few drops of methanol, the reaction mixture was concentrated in vacuo to give a residue. The residue was purified by preparative HPLC [Instrument: ACSWH-GX-Q; Column: Waters xbridge 150*25 mm 10 µm, eluent A: water (0.2% ammonium hydrogen carbonate), eluent B: acetonitrile; gradient: 0-8 min 33%-63% B; flow 25 ml/min; temperature: RT; Detector: UV 220/254 nm] to give 1-[(2S)-2-({[4-(7-methyl-3-phenyl-1H-pyrrolo[3,2-b]pyridin-2-yl)pyridin-3-yl]oxy}methyl)pyrrolidin-1-yl]prop-2-en-1-one (11.4 mg, 0.0253 mmol, 97% purity, 11% yield) as a yellow solid.

LCMS (Method C): Rₜ = 0.624 min; MS (ESlpos): m/z = 439.2 [M+H]⁺.

¹H NMR (400 MHz, DMSO-d₆), δ [ppm] = 11.43 (br s, 1H), 8.53 (br s, 1H), 8.34-8.23 (m, 2H), 7.59 (d, *J* = 7.6 Hz, 2H), 7.32 (d, *J* = 1.2 Hz, 1H), 7.27 (t, *J* = 7.6 Hz, 2H), 7.20-7.13 (m, 1H), 7.02 (d, *J* = 4.8 Hz, 1H), 6.41-6.28 (m, 1H), 6.12-5.91 (m, 1H), 5.64-5.44 (m, 1H), 4.26-3.92 (m, 3H), 3.41-3.25 (m, 1H), 3.19-3.10 (m, 1H), 2.56 (s, 3H), 1.78-1.55 (m, 4H).

### Example 136

### 2-(3-{[(2S)-1-(ethenesulfonyl)pyrrolidin-2-yl]methoxy}pyridin-4-yl)-7-methyl-3-phenyl-1H-pyrrolo[3,2-b]pyridine

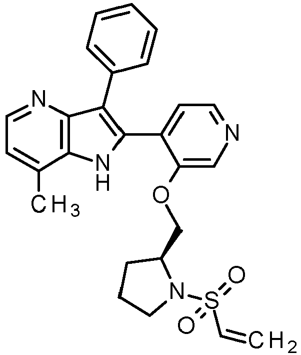

To a solution of 7-methyl-3-phenyl-2-(3-{[(2S)-pyrrolidin-2-yl]methoxy}pyridin-4-yl)-1H-pyrrolo[3,2-b]pyridine hydrogen chloride (1/1) (100 mg, 0.238 mmol) in dichloromethane (3 ml) were slowly added a solution of ethenesulfonyl chloride (60.1 mg, 0.475 mmol) in dichloromethane (2 ml) and triethylamine (72.1 mg, 0.712 mmol) at -10 °C. The mixture was stirred at -10 °C for 1 hour. The reaction mixture was concentrated under reduced pressure to give a residue. The residue was purified by preparative HPLC [Instrument: ACSWH-GX-Q; Column: Phenomenex luna C18 150*25 mm*10 µm, eluent A: water (0.2% formic acid), eluent B: acetonitrile; gradient: 0-10 min 8%-38% B; flow 25 ml/min; temperature: RT; Detector: UV 220/254 nm] to give 2-(3-{[(2S)-1-(ethenesulfonyl)pyrrolidin-2-yl]methoxy}pyridin-4-yl)-7-methyl-3-phenyl-1H-pyrrolo[3,2-b]pyridine (16.6 mg, 0.0349 mmol, 100% purity, 15% yield) as a white solid.

LCMS (Method C): Rₜ = 0.607 min; MS (ESlpos): m/z = 475.2 [M+H]⁺.

¹H NMR (400 MHz, DMSO-d₆), δ [ppm] = 11.67 (s, 1H), 8.52 (s, 1H), 8.30 (dd, *J =* 7.6, 4.8 Hz, 2H), 7.60-7.52 (m, 2H), 7.37 (d, *J* = 4.8 Hz, 1H), 7.28 (t, *J* = 7.6 Hz, 2H), 7.21-7.12 (m, 1H), 7.04 (dd, *J* = 4.4, 0.8 Hz, 1H), 6.80 (dd, *J* = 16.4, 10.0 Hz, 1H), 6.08 (d, *J* = 2.0 Hz, 1H), 6.04 (d, *J* = 8.8 Hz, 1H), 4.09-4.01 (m, 1H), 3.95 (dd, *J* = 9.6, 7.6 Hz, 1H), 3.59 (td, *J* = 7.2, 3.6 Hz, 1H), 3.00-2.91 (m, 2H), 2.53 (s, 3H), 1.61-1.43 (m, 4H).

### Example 137

### 1-{(2S)-2-[({4-[3-(5-chloro-2-fluorophenyl)-7-methyl-1H-pyrrolo[3,2-b]pyridin-2-yl]pyridin-3-yl}oxy)methyl]pyrrolidin-1-yl}prop-2-en-1-one

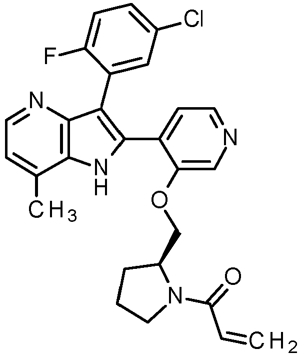

To a solution of 3-(5-chloro-2-fluorophenyl)-7-methyl-2-(3-{[(2S)-pyrrolidin-2-yl]methoxy}pyridin-4-yl)-1H-pyrrolo[3,2-b]pyridine (100 mg, 0.229 mmol) in dichloromethane (3 ml) was added triethylamine(64 µl, 0.460 mmol) and prop-2-enoyl chloride (41.4 mg, 0.458 mmol) at 0 °C. The mixture was stirred at 0 °C for 1 hour. The reaction mixture was concentrated under reduced pressure to give a residue. The residue was purified by flash reversed-phase MPLC (acetonitrile/water, 0.05% ammonia hydroxide, 20%~30%) to give a crude product. The crude product was purified by preparative HPLC [Instrument: ACSWH-GX-Q; Column: Phenomenex luna C18 150*25 mm*10 µm, eluent A: water (0.2% formic acid), eluent B: acetonitrile; gradient: 0-9 min 15%-33% B; flow 25 ml/min; temperature: RT; Detector: UV 220/254 nm] to give 1-{(2S)-2-[({4-[3-(5-chloro-2-fluorophenyl)-7-methyl-1H-pyrrolo[3,2-b]pyridin-2-yl]pyridin-3-yl}oxy)methyl]pyrrolidin-1-yl}prop-2-en-1-one (22.0 mg, 0.445 mmol, 99% purity, 19% yield) as a yellow solid.

LCMS (Method C): Rₜ = 0.678 min; MS (ESlpos): m/z = 491.1 [M+H]⁺.

¹H NMR (400 MHz, DMSO-d₆), δ [ppm] = 11.68 (br s, 1H), 8.51 (br s, 1H), 8.29 (d, *J =* 4.4 Hz, 1H), 8.25 (d, *J* = 4.4 Hz, 1H), 7.69 (dd, *J* = 6.4, 2.8 Hz, 1H), 7.33 (td, *J* = 8.8, 3.6 Hz, 1H), 7.27 (d, *J* = 4.4 Hz, 1H), 7.14 (t, *J* = 9.2 Hz, 1H), 7.06 (d, *J* = 4.8 Hz, 1H), 6.47-6.30 (m, 1H), 6.15-5.91 (m, 1H), 5.70-5.46 (m, 1H), 4.26-3.92 (m, 1H), 4.26-3.91 (m, 2H), 3.44-3.36 (m, 1H), 3.27-3.22 (m, 1H), 2.58 (s, 3H), 1.84-1.62 (m, 4H).

### Example 138

### 3-(5-chloro-2-fluorophenyl)-2-(3-{[(2S)-1-(ethenesulfonyl)pyrrolidin-2-yl]methoxy}pyridin-4-yl)-7-methyl-1H-pyrrolo[3,2-b]pyridine

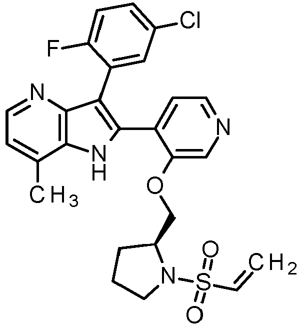

To a solution of 3-(5-chloro-2-fluorophenyl)-7-methyl-2-(3-{[(2S)-pyrrolidin-2-yl]methoxy}pyridin-4-yl)-1H-pyrrolo[3,2-b]pyridine (100 mg, 0.229 mmol) in dichloromethane (3 ml) were added triethylamine (46.3 mg, 0.458 mmol) and ethenesulfonyl chloride (57.9 mg, 0.458 mmol) at 0 °C. The mixture was stirred at 0 °C for 1 hour. The reaction mixture was concentrated under reduced pressure to give a residue. The residue was purified by preparative HPLC [Instrument: ACSWH-GX-A; Column: Waters Xbridge 150*25 mm*5 µm, eluent A: water (0.2% ammonia hydroxide), eluent B: acetonitrile; gradient: 0-9 min 20%-30% B; flow 25 ml/min; temperature: RT; Detector: UV 220/254 nm].to give 3-(5-chloro-2-fluorophenyl)-2-(3-{[(2S)-1-(ethenesulfonyl)pyrrolidin-2-yl]methoxy}pyridin-4-yl)-7-methyl-1H-pyrrolo[3,2-b]pyridine (46.7 mg, 0.866 mmol, 98% purity, 38% yield) as a white solid.

LCMS (Method C): Rₜ = 0.690 min; MS (ESlpos): m/z = 527.1 [M+H]⁺.

¹H NMR (400 MHz, DMSO-d₆), δ [ppm] = 12.00-11.80 (m, 1H), 8.49 (s, 1H), 8.30 (d, *J* = 4.8 Hz, 2H), 7.78 (dd, *J =* 6.4, 2.8 Hz, 1H), 7.41-7.32 (m, 2H), 7.22-7.14 (m, 1H), 7.08 (d, *J* = 4.4 Hz, 1H), 6.84 (dd, *J* = 16.4, 10.0 Hz, 1H), 6.12 (d, *J =* 5.6 Hz, 1H), 6.09 (d, *J =* 12.0 Hz, 1H), 4.03 (dd, *J* = 9.6, 3.6 Hz, 1H), 3.96-3.89 (m, 1H), 3.56-3.49 (m, 1H), 3.11-3.00 (m, 2H), 2.57 (s, 3H), 1.69-1.55 (m, 4H).

### Example 139

### rel-(S)-1-(2-(((4-(3-phenyl-1H-pyrrolo[3,2-b]pyridin-2-yl)pyridin-3-yl)oxy)methyl)azetidin-1-yl)prop-2-en-1-one

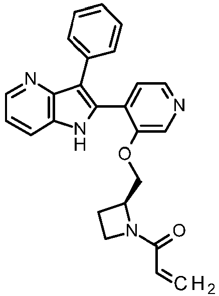

To a solution of a (RS)-2-(3-(azetidin-2-ylmethoxy)pyridin-4-yl)-3-phenyl-1H-pyrrolo[3,2-b]pyridine (50 mg, 140 µmol) in dichloromethane (5 ml) were added triethylamine (42.6 mg, 421 µmol) and acryloyl chloride (14.0 mg, 154 µmol) at 0 °C. The mixture was stirred at 0 °C for 1 hour. The reaction mixture was concentrated in vacuo to give a residue. The residue was purified by preparative HPLC [Instrument: GX-A; Column: Waters Xbridge 150*25mm* 5µm; eluent A: water (0.2% NH3•H2O), eluent B: acetonitrile; gradient: 0-10 min 15-45% B; flow 25 ml/min; temperature: RT; Detector: UV 220/254 nm] to give (RS)-1-(2-(((4-(3-phenyl-1H-pyrrolo[3,2-b]pyridin-2-yl)pyridin-3-yl)oxy)methyl)azetidin-1-yl)prop-2-en-1-one (30 mg, 69.7 µmol, 95% purity) as a yellow solid. The obtained product was seperated with chiral seperation (Column: Chiralpak AD-3 50×4.6mm I.D., 3µm Mobile phase: Phase A for CO2, and Phase B for EtOH(0.05%DEA); Gradient elution:EtOH (0.05% DEA) in CO2 from 5% to 40%; Flow rate: 3mL/min; Detector: PDA; Column Temp: 35C;Back Pressure: 100Bar) to give rel-(S)-1-(2-(((4-(3-phenyl-1H-pyrrolo[3,2-b]pyridin-2-yl)pyridin-3-yl)oxy)methyl)azetidin-1-yl)prop-2-en-1-one (7.70 mg, 17.4 µmol, 93% purity, 12% yield) as a light yellow solid.

LC-MS (Method C): Rₜ = 0.719 min; MS (ESlpos): m/z = 411.2 [M+H]⁺.

¹H NMR (400 MHz, DMSO-d₆), δ [ppm] = 11.99-11.68 (m, 1H), 8.54 (d, *J =* 14.8 Hz, 1H), 8.43 (dd, *J* = 12.8, 3.6 Hz, 1H), 8.36-8.31 (m, 1H), 8.21 (d, *J* = 4.8 Hz, 1H), 7.84 (t, *J* = 6.8 Hz, 1H), 7.55 (d, *J* = 7.6 Hz, 2H), 7.42 (d, *J* = 4.4 Hz, 1H), 7.32 (q, *J* = 7.2 Hz, 2H), 7.27-7.18 (m, 3H), 6.11-5.94 (m, 1H), 5.79-5.53 (m, 1H), 4.61-4.52 (m, 1H), 4.51-4.38 (m, 1H), 4.35-4.23 (m, 1H), 4.12-4.04 (m, 1H), 3.97-3.89 (m, 1H), 3.76-3.66 (m, 1H), 3.64-3.55 (m, 1H), 2.32-2.17 (m, 1H), 1.95-1.84 (m, 1H), 1.82-1.69 (m, 1H).

### Example 140

### rel-(R)-1-(2-(((4-(3-phenyl-1H-pyrrolo[3,2-b]pyridin-2-yl)pyridin-3-yl)oxy)methyl)azetidin-1-yl)prop-2-en-1-one

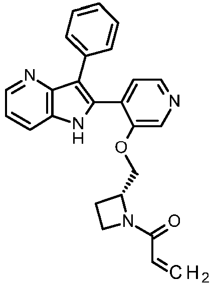

(RS)-1-(2-(((4-(3-phenyl-1H-pyrrolo[3,2-b]pyridin-2-yl)pyridin-3-yl)oxy)methyl)azetidin-1-yl)prop-2-en-1-one (30 mg, 69.7 µmol, 95% purity) was seperated with chiral seperation (Column: Chiralpak AD-3 50×4.6mm I.D., 3µm Mobile phase: Phase A for CO2, and Phase B for EtOH(0.05%DEA); Gradient elution:EtOH (0.05% DEA) in CO2 from 5% to 40%; Flow rate: 3mL/min;Detector: PDA; Column Temp: 35C;Back Pressure: 100Bar) to give rel-(R)-1-(2-(((4-(3-phenyl-1H-pyrrolo[3,2-b]pyridin-2-yl)pyridin-3-yl)oxy)methyl)azetidin-1-yl)prop-2-en-1-one (8.60 mg, 19.3 µmol, 92% purity, 28% yield) as a yellow solid.

LC-MS (Method C): Rₜ = 0.722 min; MS (ESlpos): m/z = 411.2 [M+H]⁺.

¹H NMR (400 MHz, DMSO-d₆), δ [ppm] = 11.94-11.76 (m, 1H), 8.54 (d, *J =* 15.2 Hz, 1H), 8.47-8.40 (m, 1H), 8.34 (d, *J* = 5.2 Hz, 1H), 8.21 (d, *J* = 4.8 Hz, 1H), 7.85 (t, *J* = 7.6 Hz, 1H), 7.58-7.51 (m, 2H), 7.42 (d, *J* = 4.8 Hz, 1H), 7.32 (q, *J* = 7.2 Hz, 2H), 7.27-7.18 (m, 3H), 6.15-5.94 (m, 1H), 5.68 (dd, *J* = 16.8, 2.4 Hz, 1H), 5.60 (dd, *J* = 8.0, 4.0 Hz, 1H), 4.60-4.53 (m, 1H), 4.49 (dd, *J =* 10.4, 3.2 Hz, 1H), 4.43 (dd, *J =* 10.0, 2.8 Hz, 1H), 4.35-4.21 (m, 1H), 4.11-4.03 (m, 1H), 3.97-3.88 (m, 1H), 3.75-3.66 (m, 1H), 3.64-3.55 (m, 1H), 2.31-2.18 (m, 1H), 1.95-1.84 (m, 1H), 1.83-1.68 (m, 1H).

### Example 141

### rel-(S)-3-phenyl-2-(3-((1-(vinylsulfonyl)azetidin-2-yl)methoxy)pyridin-4-yl)-1H-pyrrolo[3,2-b]pyridine

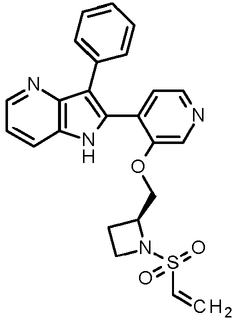

To a solution of a (RS)-2-(3-(azetidin-2-ylmethoxy)pyridin-4-yl)-3-phenyl-1H-pyrrolo[3,2-b]pyridine (320 mg, 0.898 mmol) in dichloromethane (10 ml) were added triethylamine (454 mg, 4.49 mmol) and ethenesulfonyl chloride (227 mg, 1.80 mmol) at -10 °C. The mixture was stirred at -10 °C for 1 hour. The reaction mixture was concentrated in vacuo to give a residue. The residue was purified by preparative TLC (ethyl acetate: NH₃•H₂O = 20: 1) to give (RS)-3-phenyl-2-(3-((1-(vinylsulfonyl)azetidin-2-yl)methoxy)pyridin-4-yl)-1H-pyrrolo[3,2-b]pyridine (40.0 mg, 0.0800 mmol) as a yellow solid. The obtained product was seperated with chiral seperation (Column: Chiralpak AD-3 50×4.6mm I.D., 3µm, Mobile phase: Phase A for CO2, and Phase B for EtOH(0.05%DEA); Gradient elution:EtOH (0.05% DEA) in CO2 from 5% to 40%, Flow rate: 3mL/min;Detector: PDA Column Temp: 35 °C;Back Pressure: 100 Bar) to give rel-(S)-3-phenyl-2-(3-((1-(vinylsulfonyl)azetidin-2-yl)methoxy)pyridin-4-yl)-1H-pyrrolo[3,2-b]pyridine (9.90 mg, 0.0200 mmol, 90% purity, 2% yield) as a yellow solid.

LC-MS (Method C): Rₜ = 0.908 min; MS (ESlpos): m/z = 447.2 [M+H]⁺.

¹H NMR (400 MHz, DMSO-d₆), δ [ppm] = 11.73 (s, 1H), 8.62 (s, 1H), 8.49 (dd, *J* = 4.4, 1.2 Hz, 1H), 8.35 (d, *J =* 4.8 Hz, 1H), 7.89 (dd, *J* = 8.0, 1.2 Hz, 1H), 7.62 (d, *J* = 7.2 Hz, 2H), 7.42 (d, *J* = 4.8 Hz, 1H), 7.37 (t, *J* = 7.6 Hz, 2H), 7.31-7.25 (m, 2H), 6.94 (dd, *J* = 16.4, 10.0 Hz, 1H), 6.23 (d, *J =* 10.0 Hz, 1H), 6.12 (d, *J* = 16.4 Hz, 1H), 4.25-4.12 (m, 3H), 3.69-3.60 (m, 1H), 3.47-3.42 (m, 1H), 2.06-1.92 (m, 2H).

### Example 142

### rel-(R)-3-phenyl-2-(3-((1-(vinylsulfonyl)azetidin-2-yl)methoxy)pyridin-4-yl)-1H-pyrrolo[3,2-b]pyridine

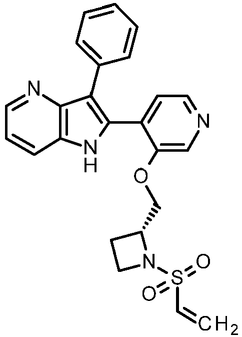

(RS)-3-phenyl-2-(3-((1-(vinylsulfonyl)azetidin-2-yl)methoxy)pyridin-4-yl)-1H-pyrrolo[3,2-b]pyridine (40 mg, 0.0800 mmol) was separated with chiral separation (Column: Chiralpak AD-3 50×4.6mm I.D., 3µm, Mobile phase: Phase A for CO2, and Phase B for EtOH(0.05%DEA); Gradient elution:EtOH (0.05% DEA) in CO2 from 5% to 40%, Flow rate: 3mL/min;Detector: PDA Column Temp: 35 °C;Back Pressure: 100 Bar) to give rel-(R)-3-phenyl-2-(3-((1-(vinylsulfonyl)azetidin-2-yl)methoxy)pyridin-4-yl)-1H-pyrrolo[3,2-b]pyridine (8.80 mg, 0.0189 mmol, 96% purity, 24% yield) as a yellow solid.

LC-MS (Method C): Rₜ = 0.879 min; MS (ESlpos): m/z = 447.2 [M+H]⁺.

¹H NMR (400 MHz, DMSO-d₆), δ [ppm] = 11.67 (s, 1H), 8.56 (s, 1H), 8.42 (dd, *J* = 4.8, 1.2 Hz, 1H), 8.29 (d, *J* = 4.8 Hz, 1H), 7.82 (dd, *J* = 8.4, 1.2 Hz, 1H), 7.58-7.54 (m, 2H), 7.35 (d, *J* = 4.8 Hz, 1H), 7.31 (t, *J* = 7.6 Hz, 2H), 7.25-7.18 (m, 2H), 6.87 (dd, *J* = 16.4, 10.0 Hz, 1H), 6.16 (d, *J* = 10.0 Hz, 1H), 6.06 (d, *J* = 16.4 Hz, 1H), 4.20-4.05 (m, 3H), 3.63-3.53 (m, 1H), 3.39-3.35 (m, 1H), 2.02-1.84 (m, 2H).

### Example 143

### rel-(S)-1-(2-(((4-(3-(3-(trifluoromethyl)phenyl)-1H-pyrrolo[3,2-b]pyridin-2-yl)pyridin-3-yl)oxy)methyl)azetidin-1-yl)prop-2-en-1-one

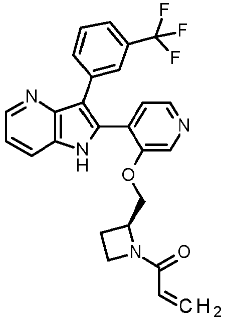

To a solution of 2-{3-[(2RS)-azetidin-2-ylmethoxy]pyridin-4-yl}-3-[3-(trifluoromethyl)phenyl]-1H-pyrrolo[3,2-b]pyridine (100 mg, 0.236 mmol) in dichloromethane (15 ml) was added triethylamine (71.5 mg, 0.707 mmol) at 0 °C. The mixture was stirred at 0 °C for 1 hour. The reaction mixture was concentrated in vacuo to give a crude product. The crude product was purified by preparative HPLC [Instrument: GX-A; Column: Waters Xbridge 150*25mm* 5µm; eluent A: water (0.2% NH3'H2O), eluent B: acetonitrile; gradient: 0-10 min 15-45% B; flow 25 ml/min; temperature: RT; Detector: UV 220/254 nm] to give (SR)-1-(2-(((4-(3-(3-(trifluoromethyl)phenyl)-1H-pyrrolo[3,2-b]pyridin-2-yl)pyridin-3-yl)oxy)methyl)azetidin-1-yl)prop-2-en-1-one (28.0 mg, 0.0543 mmol, 93% purity) as a yellow solid. The obtained product was separated with chiral separation (Column: Chiralpak AD-3 50×4.6mm I.D., 3µm, Mobile phase: Phase A for CO2, and Phase B for EtOH(0.05%DEA); Gradient elution:EtOH (0.05% DEA) in CO2 from 5% to 40%, Flow rate: 3mL/min;Detector: PDA Column Temp: 35 °C;Back Pressure: 100 Bar) to give rel-(S)-1-(2-(((4-(3-(3-(trifluoromethyl)phenyl)-1H-pyrrolo[3,2-b]pyridin-2-yl)pyridin-3-yl)oxy)methyl)azetidin-1-yl)prop-2-en-1-one (12.2 mg, 0.0236 mmol, 93% purity, 10% yield) as a light yellow solid.

LC-MS (Method C): Rₜ = 0.785 min; MS (ESlpos): m/z = 479.2 [M+H]⁺.

¹H NMR (400 MHz, DMSO-d₆), δ [ppm] = 12.15-11.91 (s, 1H), 8.62-8.54 (m, 1H), 8.52-8.45 (m, 1H), 8.42-8.26 (m, 1H), 8.07-7.95 (m, 1H), 7.92-7.76 (m, 2H), 7.61-7.52 (m, 2H), 7.51-7.33 (m, 1H), 7.32-7.20 (m, 1H), 6.09-5.87 (m, 1H), 5.81-5.63 (m, 1H), 5.59-5.42 (m, 1H), 4.52-4.44 (s, 1H), 4.31-4.18 (m, 1H), 4.09-3.82 (m, 1H), 3.74-3.55 (m, 2H), 2.29-2.10 (m, 1H), 1.84-1.64 (m, 1H).

### Example 144

### rel-(S)-3-(3-(trifluoromethyl)phenyl)-2-(3-((1-(vinylsulfonyl)azetidin-2-yl)methoxy)pyridin-4-yl)-1H-pyrrolo[3,2-b]pyridine

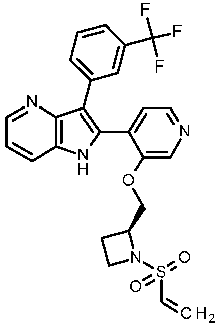

To a solution of 2-{3-[(2RS)-azetidin-2-ylmethoxy]pyridin-4-yl}-3-[3-(trifluoromethyl)phenyl]-1H-pyrrolo[3,2-b]pyridine (500 mg, 1.18 mmol) in dichloromethane (10 ml) were added triethylamine (596 mg, 5.89 mmol) and ethenesulfonyl chloride (298 mg, 2.36 mmol) at -10 °C. The mixture was stirred at -10 °C for 1 hour. The reaction mixture was concentrated in vacuo to give a crude product. The crude product was purified by preparative TLC (ethyl acetate: NH₃•H₂O = 20: 1) to give 3-[3-(trifluoromethyl)phenyl]-2-(3-{[(2RS)-1-(vinylsulfonyl)azetidin-2-yl]methoxy}pyridin-4-yl)-1H-pyrrolo[3,2-b]pyridine (110 mg, 0.205 mmol) as a brown solid. The obtained product was then purified by chiral separation (Column: Chiralcel OJ-3 50×4.6mm I.D., 3µm Mobile phase: Phase A for CO2, and Phase B for EtOH(0.05%DEA); Gradient elution:EtOH (0.05% DEA) in CO2 from 5% to 40%, Flow rate: 3mL/min;Detector: PDA Column Temp: 35 °C;Back Pressure: 100Bar) to give rel-(S)-3-(3-(trifluoromethyl)phenyl)-2-(3-((1-(vinylsulfonyl)azetidin-2-yl)methoxy)pyridin-4-yl)-1H-pyrrolo[3,2-b]pyridine (37.1 mg, 0.0692 mmol, 96% purity, 6% yield) as a yellow solid.

LC-MS (Method C): Rₜ = 0.442 min; MS (ESlpos): m/z = 515.1 [M+H]⁺.

¹H NMR (400 MHz, DMSO-d₆), δ [ppm] = 8.56 (s, 1H), 8.47 (d, *J =* 4.4 Hz, 1H), 8.35 (d, *J* = 4.8 Hz, 1H), 8.02 (s, 1H), 7.86 (dd, *J* = 8.4, 1.2 Hz, 1H), 7.83-7.77 (m, 1H), 7.56-7.50 (m, 2H), 7.46 (d, *J* = 4.8 Hz, 1H), 7.25 (dd, *J* = 8.0, 4.8 Hz, 1H), 6.82 (dd, *J* = 16.4, 10.0 Hz, 1H), 6.12 (d, *J* = 10.0 Hz, 1H), 6.01 (d, *J* = 16.4 Hz, 1H), 4.16-4.09 (m, 1H), 4.07-4.00 (m, 2H), 3.58-3.51 (m, 1H), 3.28 (d, *J* = 4.8 Hz, 1H), 1.93-1.83 (m, 1H), 1.80-1.69 (m, 1H).

### Example 145

### 1-[(2RS)-2-methyl-2-({[4-(3-phenyl-1H-pyrrolo[3,2-b]pyridin-2-yl)pyridin-3-yl]oxy}methyl)azetidin-1-yl]prop-2-en-1-one

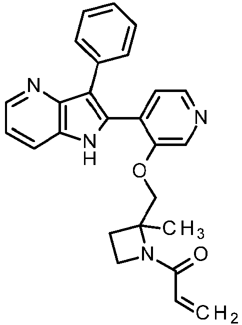

To a solution of 2-(3-{[(2RS)-2-methylazetidin-2-yl]methoxy}pyridin-4-yl)-3-phenyl-1H-pyrrolo[3,2-b]pyridine (180 mg, 0.486 mmol) and acryloyl chloride (88.0 mg, 0.972 mmol) in dichloromethane (6 ml) was added triethylamine (98.3 mg, 0.971 mmol) at 0 °C. The mixture was stirred at 0 °C for 1 hour. The reaction mixture was concentrated in vacuo to give a residue. The residue was purified by preparative-HPLC (column: Phenomenex luna C18 150*25mm* 10µm; mobile phase: water(TFA)-ACN; B%:4%-34%, 10 min) to give 1-[(2RS)-2-methyl-2-({[4-(3-phenyl-1H-pyrrolo[3,2-b]pyridin-2-yl)pyridin-3-yl]oxy}methyl)azetidin-1-yl]prop-2-en-1-one (21.3 mg, 0.0480 mmol, 96% purity, 10% yield) as a yellow solid.

LC-MS (Method C): Rₜ = 0.705 min; MS (ESlpos): m/z = 425.2 [M+H]⁺.

¹H NMR (400 MHz, DMSO-d₆), δ [ppm] = 11.71 (d, *J* = 3.2 Hz, 1H), 8.48 (d, *J* = 3.2 Hz, 1H), 8.44-8.37 (m, 1H), 8.33 (t, *J* = 4.8 Hz, 1H), 7.81 (dd, *J* = 7.2, 6.0 Hz, 1H), 7.49 (d, *J =* 7.2 Hz, 2H), 7.39 (dd, *J* = 12.8, 4.8 Hz, 1H), 7.30 (dt, *J* = 7.6, 3.2 Hz, 2H), 7.24-7.15 (m, 2H), 6.36-6.10 (m, 1H), 5.95 (dt, *J* = 16.8, 2.4 Hz, 1H), 5.59-5.43 (m, 1H), 3.65 (d, *J* = 13.2 Hz, 2H), 3.27-3.14 (m, 2H), 2.05-1.73 (m, 2H), 1.33-1.15 (m, 3H).

### Example 146

### 2-(3-{[(2S)-1-(ethenesulfonyl)-2-methylazetidin-2-yl]methoxy}pyridin-4-yl)-3-phenyl-1H-pyrrolo[3,2-b]pyridine

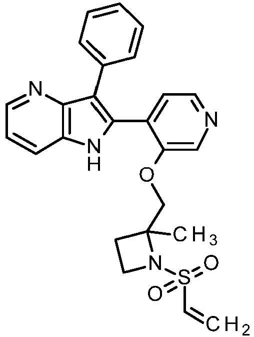

To a solution of 2-(3-{[(2S)-2-methylazetidin-2-yl]methoxy}pyridin-4-yl)-3-phenyl-1H-pyrrolo[3,2-b]pyridine (250 mg, 0.675 mmol) and ethenesulfonyl chloride (171 mg, 1.35 mmol) in dichloromethane (6 ml) was added triethylamine (0.2 ml, 1.30 mmol) at -20 °C. The mixture was stirred at -20 °C for 1 hour. The reaction mixture was concentrated in vacuo to give a residue. The residue was purified by preparative-HPLC (column: Phenomenex luna C18 150*25mm* 10µm; mobile phase: water(TFA)-ACN; B%:5%-35%, 10 min) to give 2-(3-{[(2S)-1-(ethenesulfonyl)-2-methylazetidin-2-yl]methoxy}pyridin-4-yl)-3-phenyl-1H-pyrrolo[3,2-b]pyridine (36.2 mg, 0.0787 mmol, 95% purity, 11% yield) as a yellow solid.

LC-MS (Method C): Rₜ = 0.743 min; MS (ESlpos): m/z = 461.2 [M+H]⁺.

¹H NMR (400 MHz, DMSO-d6), δ [ppm] = 11.76 (br s, 1H), 8.47 (s, 1H), 8.45-8.42 (m, 1H), 8.38-8.32 (m, 1H), 7.87 (d, *J =* 8.4 Hz, 1H), 7.54-7.45 (m, 3H), 7.33 (t, *J =* 7.6 Hz, 2H), 7.27-7.19 (m, 2H), 6.05 (dd, *J =* 16.4, 10.0 Hz, 1H), 5.83 (d, *J =* 16.4 Hz, 1H), 5.60 (d, *J =* 10.0 Hz, 1H), 3.24 (d, *J* = 11.6 Hz, 1H), 3.16-3.09 (m, 2H), 2.97-2.87 (m, 1H), 2.17-2.05 (m, 1H), 1.87-1.74 (m, 1H), 1.10 (s, 3H).

### Example 147

### 1-[(2RS)-2-methyl-2-{[(4-{3-[3-(trifluoromethyl)phenyl]-1H-pyrrolo[3,2-b]pyridin-2-yl}pyridin-3-yl)oxy]methyl}azetidin-1-yl]prop-2-en-1-one

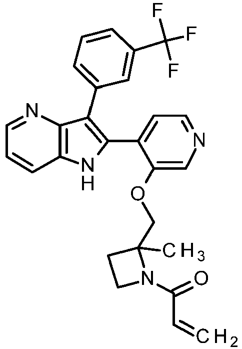

To a solution of 2-(3-{[(2RS)-2-methylazetidin-2-yl]methoxy}pyridin-4-yl)-3-[3-(trifluoromethyl)phenyl]-1H-pyrrolo[3,2-b]pyridine (80.0 mg, 0.182 mmol) and triethylamine (0.05 ml, 0.360 mmol) in dichloromethane (5 ml) was added prop-2-enoyl chloride (24.8 mg, 274 µmol) at 20 °C. The mixture was stirred at 20 °C for 1 hour. The reaction mixture was concentrated in vacuo to give a residue and the residue was purified by silica gel column chromatography (petroleum ether: ethyl acetate = 0: 1) to give a crude product. The crude product was purified by chiral separation [Instrument: ACSWH-PREP-SFC-D; Column: DAICEL CHIRALPAK IF (250mm*30mm,10µm), eluent A: carbon dioxide, eluent B: isopropanol; gradient: 0-5 min 60-60% B; flow 80 ml/min; Detector: UV 220/254 nm; Column: Chiralpak IF-3 50×4.6mm I.D., 3µm; Mobile phase: Phase A for CO2, and Phase B for IPA(0.05%DEA); Gradient elution: 40% IPA (0.05% DEA) in CO2; Flow rate: 3mL/min;Detector: PDA; Column Temp: 35 °C; Back Pressure: 100Bar] to give 1-[(2RS)-2-methyl-2-{[(4-{3-[3-(trifluoromethyl)phenyl]-1H-pyrrolo[3,2-b]pyridin-2-yl}pyridin-3-yl)oxy]methyl}azetidin-1-yl]prop-2-en-1-one (9.80 mg, 0.0197 mmol, 11% yield) as a white solid.

LC-MS (Method **G**): Rₜ = 0.879 min; MS (ESlpos): m/z = 493.1 [M+H]⁺.

¹H NMR (400 MHz, DMSO-d6), δ [ppm] = 8.50 (d, *J* = 4.0 Hz, 1H), 8.47-8.43 (m, 1H), 8.40 (t, *J =* 4.8 Hz, 1H), 7.91 (s, 1H), 7.88-7.81 (m, 1H), 7.73 (d, *J =* 6.4 Hz, 1H), 7.56-7.44 (m, 3H), 7.24 (td, *J* = 8.0, 4.0 Hz, 1H), 6.31-6.07 (m, 1H), 5.93 (dt, *J* = 16.8, 2.4 Hz, 1H), 5.56-5.42 (m, 1H), 3.57 (d, *J =* 13.6 Hz, 1H), 3.50-3.45 (m, 1H), 3.17 (d, *J =* 13.6 Hz, 1H), 3.14-3.04 (m, 1H), 1.92-1.71 (m, 2H), 1.26 (s, 2H), 1.15 (s, 1H).

### Example 148

### 2-(3-{[(2RS)-1-(ethenesulfonyl)-2-methylazetidin-2-yl]methoxy}pyridin-4-yl)-3-[3-(trifluoromethyl)phenyl]-1H-pyrrolo[3,2-b]pyridine

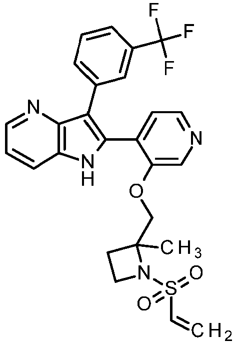

To a solution of 2-(3-{[(2RS)-2-methylazetidin-2-yl]methoxy}pyridin-4-yl)-3-[3-(trifluoromethyl)phenyl]-1H-pyrrolo[3,2-b]pyridine (40.0 mg, 0.0912 mmol) and triethylamine (0.02 ml, 0.140 mmol) in dichloromethane (10 ml) was added ethenesulfonyl chloride (17.3 mg, 137 µmol) at -20 °C. The mixture was stirred at -20 °C for 1 hour. The reaction mixture was concentrated in vacuo to give a residue and the residue was purified by preparative TLC (ethyl acetate: methanol = 10: 1) to give a crude product. The crude product product was purified by preparative MPLC [Column: Spherical C18 (20-45µm, 100 Å), eluent A: acetonitrile, eluent B: water (0.5% formic acid); gradient: 0-20 min 15-45% B; flow 6 ml/min; Detector: UV 220/254 nm] to give 2-(3-{[(2RS)-1-(ethenesulfonyl)-2-methylazetidin-2-yl]methoxy}pyridin-4-yl)-3-[3-(trifluoromethyl)phenyl]-1H-pyrrolo[3,2-b]pyridine (3.50 mg, 0.00636 mmol, 96% purity, 7% yield) as a yellow solid.

LC-MS (Method C): Rₜ = 0.706 min; MS (ESlpos): m/z = 529.1 [M+H]⁺.

¹H NMR (400 MHz, DMSO-d6), δ [ppm] = 12.09 (d, *J =* 8.8 Hz, 1H), 8.51-8.47 (m, 2H), 8.42 (d, *J* = 4.8 Hz, 1H), 7.97-7.85 (m, 2H), 7.82-7.73 (m, 1H), 7.63-7.51 (m, 3H), 7.28 (dd, *J =* 8.4, 4.4 Hz, 1H), 6.05 (dd, *J* = 16.4, 10.0 Hz, 1H), 5.83 (d, *J* = 16.4 Hz, 1H), 5.63 (d, *J =* 10.0 Hz, 1H), 3.22-3.07 (m, 3H), 2.87 (dt, *J* = 9.2, 7.2 Hz, 1H), 2.07-1.95 (m, 1H), 1.88-1.73 (m, 1H), 1.08 (s, 3H).

### Example 149

### 1-[3-({[4-(3-phenyl-1H-pyrrolo[3,2-b]pyridin-2-yl)pyridin-3-yl]oxy}methyl)morpholin-4-yl]prop-2-en-1-one

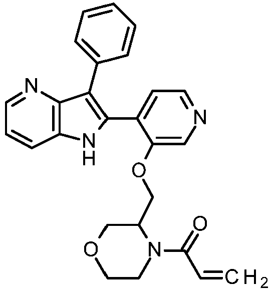

To a solution of 2-(3-{[morpholin-3-yl]methoxy}pyridin-4-yl)-3-phenyl-1H-pyrrolo[3,2-b]pyridine (70.0 mg, 0.181 mmol) in dichloromethane (2 ml) was added prop-2-enoyl chloride (18.0 mg, 0.199 mmol) at 0 °C. The mixture was stirred at 0 °C for 1 hour. The reaction mixture was concentrated under reduced pressure to give a residue. The residue was purified by preparative HPLC [Instrument: ACSWH-GX-Q; Column: Phenomenex luna C18 150*25 mm*10 µm, eluent A: water (0.2% formic acid), eluent B: acetonitrile; gradient: 0-10 min 5%-35% B; flow 25 ml/min; temperature: RT; Detector: UV 220/254 nm] to give 1-[3-({[4-(3-phenyl-1H-pyrrolo[3,2-b]pyridin-2-yl)pyridin-3-yl]oxy}methyl)morpholin-4-yl]prop-2-en-1-one (16.4 mg, 0.322 mmol, 86% purity, 18% yield) as a yellow solid.

LCMS (Method **G**): Rₜ = 0.884 min; MS (ESlpos): m/z = 441.2 [M+H]⁺.

¹H NMR (400 MHz, DMSO-d₆), δ [ppm] = 11.35 (br s, 1H), 8.57 (s, 1H), 8.42 (dd, *J =* 4.4, 1.2 Hz, 1H), 8.29-8.23 (m, 1H), 7.85-7.79 (m, 1H), 7.56 (d, *J* = 7.2 Hz, 2H), 7.34-7.27 (m, 3H), 7.25-7.17 (m, 2H), 6.51-6.29 (m, 1H), 5.94-5.77 (m, 1H), 5.30 (br s, 1H), 4.81-4.58 (m, 1H), 4.28-4.20 (m, 1H), 3.91 (br s, 1H), 3.77 (dd, *J =* 11.2, 3.2 Hz, 1H), 3.67 (d, *J =* 11.8 Hz, 2H), 3.54-3.48 (m, 1H), 3.40-3.28 (m, 2H).

### Example 150

### (2E)-4-(dimethylamino)-1-[3-({[4-(3-phenyl-1H-pyrrolo[3,2-b]pyridin-2-yl)pyridin-3-yl]oxy}methyl)morpholin-4-yl]but-2-en-1-one

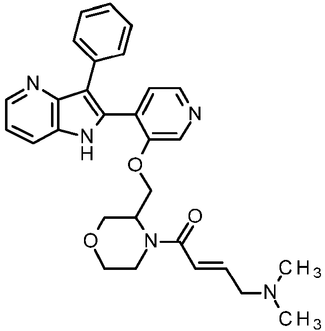

To a solution of 2-(3-{[morpholin-3-yl]methoxy}pyridin-4-yl)-3-phenyl-1H-pyrrolo[3,2-b]pyridine (40.0 mg, 0.104 mmol) and (2E)-4-(dimethylamino)but-2-enoic acid (26.7 mg, 0.207 mmol) in N,N-dimethylformamide (2 ml) were added O-(7-azabenzotriazol-1-yl)-N,N,N,N-tetramethyl uronium hexafluorophosphate (59.0 mg, 0.155 mmol) and N,N-diisopropylethylamine (26.8 mg, 0.210 mmol) at 20 °C. The mixture was stirred at 20 °C for 16 hour. The reaction mixture was concentrated under reduced pressure to give a residue. The residue was purified by preparative HPLC [Instrument: CASWH-GX-D; Column: Waters Xbridge 150*25 mm*5 µm, eluent A: water (0.2% ammonium hydrogencarbonate) eluent B: acetonitrile; gradient: 0-10 min 28%-58% B; flow 25 ml/min; temperature: RT; Detector: UV 220/254 nm] to give (2E)-4-(dimethylamino)-1-[3-({[4-(3-phenyl-1H-pyrrolo[3,2-b]pyridin-2-yl)pyridin-3-yl]oxy}methyl)morpholin-4-yl]but-2-en-1-one (5.00 mg, 0.00992 mmol, 99% purity, 10% yield) as a colorless oil.

LCMS (Method G): Rₜ = 0.850 min; MS (ESlpos): m/z = 498.3 [M+H]⁺.

¹H NMR (400 MHz, DMSO-d6), δ [ppm] = 11.35 (br s, 1H), 8.59 (s, 1H), 8.42 (d, *J* = 3.6 Hz, 1H), 8.26 (t, *J* = 4.8 Hz, 1H), 7.82 (t, *J* = 4.8 Hz, 1H), 7.56 (d, *J =* 7.6 Hz, 2H), 7.31 (t, *J* = 6.0 Hz, 2H), 7.24-7.18 (m, 2H), 6.61-6.25 (m, 2H), 4.76-4.68 (m, 1H), 4.23 (br s, 2H), 4.04-3.84 (m, 1H), 3.82-3.73 (m, 1H), 3.72-3.60 (m, 2H), 3.59-3.45 (m, 1H), 3.38-3.27 (m, 2H), 2.30-2.14 (m, 7H).

### Example 151

### 1-{3-[({4-[3-(5-chloro-2-fluorophenyl)-1H-pyrrolo[3,2-b]pyridin-2-yl]pyridin-3-yl}oxy)methyl]morpholin-4-yl}prop-2-en-1-one

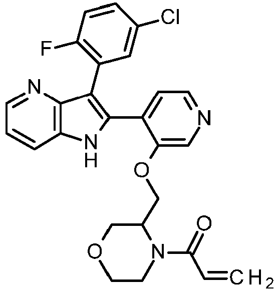

To a solution of 3-(5-chloro-2-fluorophenyl)-2-(3-{[morpholin-3-yl]methoxy}pyridin-4-yl)-1H-pyrrolo[3,2-b]pyridine hydrogen chloride (1/1) (50.0 mg, 0.114 mmol) in dichloromethane (3 ml) were added triethylamine (34.6 mg, 0.342 mmol) and prop-2-enoyl chloride (15.5 mg, 0.171 mmol) at 0 °C. The mixture was stirred at 0 °C for 1 hour. The reaction mixture was concentrated under reduced pressure to give a residue. The residue was purified by preparative HPLC [Instrument: ACSWH-GX-Q; Column: Phenomenex luna C18 150*25 mm*10 µm, eluent A: water (0.2% formic acid), eluent B: acetonitrile; gradient: 0-10 min 13%-32% B; flow 25 ml/min; temperature: RT; Detector: UV 220/254 nm] to give 1-{3-[({4-[3-(5-chloro-2-fluorophenyl)-1H-pyrrolo[3,2-b]pyridin-2-yl]pyridin-3-yl}oxy)methyl]morpholin-4-yl}prop-2-en-1-one (8.00 mg, 0.0156 mmol, 96% purity, 14% yield) as a white solid.

LCMS (Method C): Rₜ = 0.640 min; MS (ESlpos): m/z = 493.1 [M+H]⁺.

¹H NMR (400 MHz, DMSO-d6), δ [ppm] = 11.63 (br s, 1H), 8.57 (s, 1H), 8.42 (dd, *J* = 4.4, 1.2 Hz, 1H), 8.24 (d, *J =* 4.8 Hz, 1H), 7.89 (d, *J =* 8.4 Hz, 1H), 7.68 (dd, *J =* 6.4, 2.8 Hz, 1H), 7.40-7.33 (m, 1H), 7.27-7.21 (m, 2H), 7.17 (t, *J* = 9.2 Hz, 1H), 6.45 (br s, 1H), 5.88 (br s, 1H), 5.35 (br s, 1H), 4.31-4.24 (m, 2H), 3.88 (br s, 1H), 3.84-3.69 (m, 3H), 3.45-3.30 (m, 3H).

### Example 152

### (2E)-1-{3-[({4-[3-(5-chloro-2-fluorophenyl)-1H-pyrrolo[3,2-b]pyridin-2-yl]pyridin-3-yl}oxy)methyl]morpholin-4-yl}-4-(dimethylamino)but-2-en-1-one

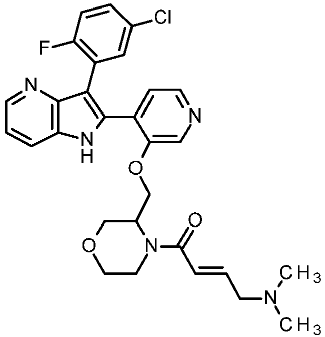

To a solution of 3-(5-chloro-2-fluorophenyl)-2-(3-{[morpholin-3-yl]methoxy}pyridin-4-yl)-1H-pyrrolo[3,2-b]pyridine hydrogen chloride (1/1) (40.0 mg, 0.0841 mmol) and (2E)-4-(dimethylamino)but-2-enoic acid (21.7 mg, 0.168 mmol) in N,N-dimethylformamide (2 ml) were added O-(7-azabenzotriazol-1-yl)-N,N,N,N-tetramethyl uronium hexafluorophosphate (48.0 mg, 0.126 mmol) and N,N-diisopropylethylamine (32.6 mg, 0.250 mmol) at 20 °C. The mixture was stirred at 20 °C for 3 hour. The reaction solution was poiured into water and extracted with ethyl acetate. The combined organic layers were washed with brine, dried over anhydrous sodium sulfate, filtered and concentrated in vacuo to give a residue. The residue was purified by preparative HPLC [Instrument: CASWH-Prep-NPLC-A; Column: Welch Ultimate XB-CN 250*50 mm*10 um, eluent A: Hexane (0.2% ammonia hydroxide), eluent B: ethanol; gradient: 0-15 min 15%-55% B; flow 25 ml/min; temperature: RT; Detector: UV 220/254 nm] to give (2E)-1-{3-[({4-[3-(5-chloro-2-fluorophenyl)-1H-pyrrolo[3,2-b]pyridin-2-yl]pyridin-3-yl}oxy)methyl]morpholin-4-yl}-4-(dimethylamino)but-2-en-1-one (10.0 mg, 0.0179 mmol, 99% purity, 21% yield) as a white solid.

LCMS (Method C): Rₜ = 0.666 min; MS (ESlpos): m/z = 550.4 [M+H]⁺.

1H NMR (400 MHz, DMSO-d6), δ [ppm] = 11.87 (d, *J =* 43.2 Hz, 1H), 8.60 (d, *J =* 10.4 Hz, 1H), 8.45 (br s, 1H), 8.26 (d, *J =* 10.4 Hz, 1H), 7.99-7.81 (m, 1H), 7.73 (br s, 1H), 7.45-7.36 (m, 1H), 7.32-7.18 (m, 3H), 6.69-6.44 (m, 1H), 6.36-6.05 (m, 1H), 4.52-4.11 (m, 3H), 3.92-3.64 (m, 4H), 3.63-3.52 (m, 1H), 3.09-2.98 (m, 1H), 2.16 (br s, 3H), 1.92 (br s, 3H), 1.23 (br s, 2H).

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptides of the invention, and, as such, may be considered in making and practicing the invention. Particularly useful techniques for particular embodiments will be discussed in the sections that follow.

The following examples are put forth so as to provide those of ordinary skill in the art with a complete disclosure and description of how to make and use the assay, screening, and therapeutic methods of the invention, and are not intended to limit the scope of what the inventors regard as their invention.

### EXPERIMENTAL SECTION - BIOLOGICAL ASSAYS

The pharmacological activity of the compounds according to the invention can be assessed using *in vitro-* and/or *in vivo*-assays, as known to the person skilled in the art. The following examples describe the biological activity of the compounds according to the invention, without the invention being limited to said examples.

Example compounds according to the invention were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* activity of the compounds of the present invention can be demonstrated in the following assays:

### Expression and purification of the EGFR proteins used in the biochemical kinase assays

The different EGFR proteins used in the biochemical kinase activity inhibition assays were generated in-house by expression in insect cells using a Baculo Virus system and subsequent purification as described in the following paragraphs.

### Expression constructs:

The cDNAs encoding the various protein sequences from human EGFR human (P00533) were optimized for expression in eukaryotic cells and synthesized by the GeneArt Technology at Life Technologies.

These DNA sequences encoded the following sequence:
Construct EGFR #1 amino acid R669 to A1210
Construct EGFR #2 amino acid R669 to A1210 and the insertion of the amino acids sequence ASV between V769 and D770
Construct EGFR #3 amino acid R669 to A1210 and the insertion of the amino acids sequence SVD between D770 and N771
Additionally all constructs EGFR #1 to #3 encoded: at the N-terminus a TEV (Tobacco etch virus) protease cleavage site (DYDIPTTENLYFQG), at the C-terminus two stop codons and additionally 5' and 3' att-DNA sequences for Gateway Cloning.

Each of the four EFGR constructs was subcloned using the Gateway Technology into the Destination vector pD-Ins1. The vector pD-Ins1 is a Baculovirus transfer vector (based on vector pVL1393, Pharmingen) which provides a N-terminal fusion of a GST-tag to the integrated gene construct. The respective transfer vectors were termed pD-Ins1_ EGFR #1, pD-Ins1_ EGFR #2, pD-Ins1_ EGFR #3.

### EGFR amino acid sequences:

GST-EGFR #1 (Wild Type) (SEQ ID No. 3): 
MSPILGYWKIKGLVQPTRLLLEYLEEKYEEHLYERDEGDKWRNKKFELGLEFPNLPYYID
GDVKLTQSMAIIRYIADKHNMLGGCPKERAEISMLEGAVLDIRYGVSRIAYSKDFETLKVD
FLSKLPEMLKMFEDRLCHKTYLNGDHVTHPDFMLYDALDVVLYMDPMCLDAFPKLVCFK
KRIEAIPQIDKYLKSSKYIAWPLQGWQATFGGGDHPPKSDPITSLYKKAGSDYDIPTTTEN
LYFQGRRRHIVRKRTLRRLLQERELVEPLTPSGEAPNQALLRILKETEFKKIKVLGSGAFG
TVYKGLWIPEGEKVKIPVAIKELREATSPKANKEILDEAYVMASVDNPHVCRLLGICLTSTV
QLITQLMPFGCLLDYVREHKDNIGSQYLLNWCVQIAKGMNYLEDRRLVHRDLAARNVLV
KTPQHVKITDFGLAKLLGAEEKEYHAEGGKVPIKWMALESILHRIYTHQSDVWSYGVTVW
ELMTFGSKPYDGIPASEISSILEKGERLPQPPICTIDVYMIMVKCWMIDADSRPKFRELIIEF
SKMARDPQRYLVIQGDERMHLPSPTDSNFYRALMDEEDMDDVVDADEYLIPQQGFFSS
PSTSRTPLLSSLSATSNNSTVACIDRNGLQSCPIKEDSFLQRYSSDPTGALTEDSIDDTFL
PVPEYINQSVPKRPAGSVQNPVYHNQPLNPAPSRDPHYQDPHSTAVGNPEYLNTVQPT
CVNSTFDSPAHWAQKGSHQISLDNPDYQQDFFPKEAKPNGIFKGSTAENAEYLRVAPQS
SEFIGA
GST-EGFR #2 (ASV between V769 and D770) (SEQ ID No. 4): 
MSPILGYWKIKGLVQPTRLLLEYLEEKYEEHLYERDEGDKWRNKKFELGLEFPNLPYYID
GDVKLTQSMAIIRYIADKHNMLGGCPKERAEISMLEGAVLDIRYGVSRIAYSKDFETLKVD
FLSKLPEMLKMFEDRLCHKTYLNGDHVTHPDFMLYDALDVVLYMDPMCLDAFPKLVCFK
KRIEAIPQIDKYLKSSKYIAWPLQGWQATFGGGDHPPKSDPITSLYKKAGSDYDIPTTTEN
LYFQGRRRHIVRKRTLRRLLQERELVEPLTPSGEAPNQALLRILKETEFKKIKVLGSGAFG
TVYKGLWIPEGEKVKIPVAIKELREATSPKANKEILDEAYVMASVDASVNPHVCRLLGICLT
STVQLITQLMPFGCLLDYVREHKDNIGSQYLLNWCVQIAKGMNYLEDRRLVHRDLAARN
VLVKTPQHVKITDFGLAKLLGAEEKEYHAEGGKVPIKWMALESILHRIYTHQSDVWSYGV
TVWELMTFGSKPYDGIPASEISSILEKGERLPQPPICTIDVYMIMVKCWMIDADSRPKFRE
LIIEFSKMARDPQRYLVIQGDERMHLPSPTDSNFYRALMDEEDMDDVVDADEYLIPQQGF
FSSPSTSRTPLLSSLSATSNNSTVACIDRNGLQSCPIKEDSFLQRYSSDPTGALTEDSIDD
TFLPVPEYINQSVPKRPAGSVQNPVYHNQPLNPAPSRDPHYQDPHSTAVGNPEYLNTVQ
PTCVNSTFDSPAHWAQKGSHQISLDNPDYQQDFFPKEAKPNGIFKGSTAENAEYLRVAP
QSSEFIGA
GST-EGFR #3 (SVD between D770 and N771) (SEQ ID No. 5):

MSPILGYWKIKGLVQPTRLLLEYLEEKYEEHLYERDEGDKWRNKKFELGLEFPNLPYYID
GDVKLTQSMAIIRYIADKHNMLGGCPKERAEISMLEGAVLDIRYGVSRIAYSKDFETLKVD
FLSKLPEMLKMFEDRLCHKTYLNGDHVTHPDFMLYDALDVVLYMDPMCLDAFPKLVCFK
KRIEAIPQIDKYLKSSKYIAWPLQGWQATFGGGDHPPKSDPITSLYKKAGSDYDIPTTTEN
LYFQGRRRHIVRKRTLRRLLQERELVEPLTPSGEAPNQALLRILKETEFKKIKVLGSGAFG
TVYKGLWIPEGEKVKIPVAIKELREATSPKANKEILDEAYVMASVDSVDNPHVCRLLGICLT

STVQLITQLMPFGCLLDYVREHKDNIGSQYLLNWCVQIAKGMNYLEDRRLVHRDLAARN
VLVKTPQHVKITDFGLAKLLGAEEKEYHAEGGKVPIKWMALESILHRIYTHQSDVWSYGV
TVWELMTFGSKPYDGIPASEISSILEKGERLPQPPICTIDVYMIMVKCWMIDADSRPKFRE
LIIEFSKMARDPQRYLVIQGDERMHLPSPTDSNFYRALMDEEDMDDVVDADEYLIPQQGF
FSSPSTSRTPLLSSLSATSNNSTVACIDRNGLQSCPIKEDSFLQRYSSDPTGALTEDSIDD
TFLPVPEYINQSVPKRPAGSVQNPVYHNQPLNPAPSRDPHYQDPHSTAVGNPEYLNTVQ
PTCVNSTFDSPAHWAQKGSHQISLDNPDYQQDFFPKEAKPNGIFKGSTAENAEYLRVAP
QSSEFIGA

### Generation of recombinant Baculovirus:

In separate approaches each of the three transfer vectors was co-transfected in Sf9 cells with Baculovirus DNA (Flashbac Gold DNA, Oxford Expression Technologies) using Fugene HD (Roche). After 5 days, the supernatant of the transfected cells containing the recombinant Baculovirus encoding the various EGFR proteins was used for further infection of Sf9 cells for virus amplification whereby the virus titer was monitored using qPCR.

### EGFR expression in Sf9 cells using bioreactor:

Sf9 cells cultured (Insect-xpress medium, Lonza, 27 °C) in a Wave-bioreactor with a disposable culture bag were infected at a cell density of 106 cells/ml with one of the recombinant Baculovirus stocks at a multiplicity of infection of 1 and incubated for 48 h. Subsequently the cells were harvested by centrifugation and the cell pellet frozen at -80 °C.

### Purification of the GST-EGFR fusion proteins:

Purification of the GST-EGFR fusion proteins was achieved by affinity chromatography using Glutathion Sepharose 4B matrix (GE Healthcare Life Sciences).

The pelleted cells (from 4 I cell culture) were resuspended in Lysis-Buffer (50 mM HEPES pH 7.4, 150 mM NaCl, 5% Glycerol, 1 mM MgCl2, 1 mM MnCl2, 0.5 mM Na3VO4) and lysed by a freeze-thaw cycle followed by an incubation on ice for 60 min. The supernatant was centrifuged at 4000 x g for 30 min. at 4 °C. The supernatant was than incubated with Glutathion Sepharose 4B matrix (in a glass bottle rotating for 16 h, at 4 °C) for binding of the GST EGFR fusion protein, rinsed with Wash-Buffer and finally the bound protein was eluted using Elusion-Buffer (Lysis Buffer plus 25 mM Glutathione) and shock frozen with liquid nitrogen.

### WT-EGFR kinase assay

Inhibitory activity of compounds of the present invention against wild-type Epidermal Growth Factor Receptor (EGFR) was quantified employing the TR-FRET based EGFR assay as described in the following paragraphs.

Recombinant fusion protein of N-terminal Glutathion-S-Transferase (GST) and a fragment of human EGFR (amino acids R669 to A1210), expressed in Sf9 insect cells and purified via affinity chromatography using Glutathion Sepharose as described above, was used as a kinase. As substrate for the kinase reaction the biotinylated peptide biotin-Ahx-AEEEEYFELVAKKK (C-terminus in amide form) (SEQ ID No. 6) was used, which can be purchased e.g. form the company Biosynthan GmbH (Berlin-Buch, Germany).

For the assay, 50 nl of a 100 fold concentrated solution of the test compound in DMSO was pipetted into either a black low volume 384 well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1mM dithiothreitol, 0.5 mM EGTA, 0.3 mM activated sodium ortho-vanadate, 0.005% (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 7.6 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, an terbium-cryptate labelled anti-phospho-tyrosine antibody from Cisbio Bioassays [instead of the PT66 Tb cryptate, PT66 Eu Chelate from Perkin Elmer can also be used]) in an aqueous EDTA-solution (133.3 mM EDTA, 0.2% (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0% inhibition, all other assay components but no enzyme = 100% inhibition). Usually the test compounds were tested on the same microtiter plate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100-fold concentrated solutions in DMSO by serial dilutions, exact concentrations may vary depending pipettors used) in duplicate values for each concentration and IC₅₀ values were calculated using Genedata Screener^{™} software.

### Exon20-mutant-EGFR(D770_N771insSVD) kinase assay

Inhibitory activity of compounds of the present invention against an Epidermal Growth Factor Receptor (EGFR) with an insertion of the amino acids sequence SVD between D770 and N771 (SEQ ID No. 5) was quantified employing the TR-FRET based kinase activity assay as described in the following paragraphs.

A recombinant fusion protein of N-terminal Glutathion-S-Transferase (GST) and a fragment of human EGFR variant (amino acids R669 to A1210 with insertion of the amino acids sequence SVD between D770 and N771 (SEQ ID No. 5) ("**EGFR ins SVD**"), expressed in Sf9 insect cells and purified via affinity chromatography using Glutathion Sepharose as described above, was used as a kinase. As substrate for the kinase reaction the biotinylated peptide biotin-Ahx-AEEEEYFELVAKKK (C-terminus in amide form) (SEQ ID No. 6) was used which can be purchased e.g. form the company Biosynthan GmbH (Berlin-Buch, Germany).

For the assay 50 nl of a 100-fold concentrated solution of the test compound in DMSO was pipetted into either a black low volume 384 well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1 mM dithiothreitol, 0.5 mM EGTA, 0.3 mM activated sodium ortho-vanadate, 0.005% (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 15 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, a terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2% (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently, the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0% inhibition, all other assay components but no enzyme = 100% inhibition). Usually the test compounds were tested on the same microtiter plate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC₅₀ values were calculated using Genedata Screener^{™} software.

### Exon20-mutant-EGFR(V769_D770insASV) kinase assay

Inhibitory activity of compounds of the present invention against an Epidermal Growth Factor Receptor (EGFR) with an insertion of the amino acids sequence ASV between V769 and D770 (SEQ ID No. 4) was quantified employing the TR-FRET based kinase activity assay as described in the following paragraphs.

A recombinant fusion protein of N-terminal Glutathion-S-Transferase (GST) and a fragment of human EGFR variant (amino acids R669 to A1210 with insertion of the amino acids sequence ASV between V769 and D770 (SEQ ID No. 4); ("**EGFR ins ASV**"), expressed in Sf9 insect cells and purified via affinity chromatography using Glutathion Sepharose as described above, was used as kinase. As substrate for the kinase reaction the biotinylated peptide biotin-Ahx-AEEEEYFELVAKKK (C-terminus in amide form) (SEQ ID No. 6) was used which can be purchased e.g. form the company Biosynthan GmbH (Berlin-Buch, Germany).

For the assay, 50 nl of a 100-fold concentrated solution of the test compound in DMSO was pipetted into either a black low volume 384well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1mM dithiothreitol, 0.5 mM EGTA, 0.3 mM activated sodium ortho-vanadate, 0.005% (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 2.5 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, an terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2% (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665.

Therefore, the fluorescence emissions at 620 nm and 665 nm after excitation at 337 nm were measured in a HTRF reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0% inhibition, all other assay components but no enzyme = 100% inhibition). Usually the test compounds were tested on the same microtiter plate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100-fold concentrated solutions in DMSO by serial dilutions, exact concentrations may vary depending pipettors used) in duplicate values for each concentration and IC₅₀ values were calculated using Genedata Screener^{™} software. Table 2 shows the results of the inhibition in mutant EGFR biochemical assay.

**Table 2:**

| **Example No.** | **mutEGFR (D770_N771insSVD) kinase assay** | **mutEGFR (D770_N771insNPG, T790) kinase assay** |
|---|---|---|
| | **IC₅₀** | **IC₅₀** |
| | **[mol/l]** | **[mol/l]** |
| 1 | 4,14E-07 | 6,06E-07 |
| 2 | 4,61E-07 | 7,51E-07 |
| 3 | 2,84E-06 | 3,41E-06 |
| 4 | 7,58E-09 | 1,71E-08 |
| 5 | 1,13E-05 | > 2.00E-5 |
| 6 | 3,85E-06 | 5,51E-06 |
| 7 | 1,47E-07 | 3,16E-07 |
| 8 | 1,98E-07 | 5,20E-07 |
| 9 | 6,01E-09 | 1,35E-08 |
| 10 | 7,31E-08 | 9,06E-08 |
| 11 | 1,34E-08 | 4,62E-08 |
| 12 | 8,68E-09 | 1,42E-08 |
| 13 | 7,20E-07 | 1,39E-06 |
| 14 | 1,64E-08 | 4,63E-08 |
| 15 | 1,54E-06 | 4,85E-06 |
| 16 | 7,65E-09 | 3,23E-08 |
| 17 | 5,69E-07 | 1,70E-06 |
| 18 | 5,49E-07 | 1,67E-06 |
| 19 | 1,78E-07 | 7,97E-07 |
| 20 | 3,65E-07 | 7,79E-07 |
| 21 | 2,90E-07 | 2,26E-06 |
| 22 | 6,80E-09 | 1,22E-08 |
| 23 | 3,68E-08 | 4,93E-08 |
| 24 | 1,19E-08 | 2,09E-08 |
| 25 | 5,01E-10 | 2,08E-09 |
| 26 | 6,59E-08 | 3,33E-07 |
| 27 | 1,97E-08 | 6,98E-08 |
| 28 | 6,50E-06 | ! 2.00E-5 |
| 29 | 3,49E-10 | 8,01E-10 |
| 30 | 5,13E-10 | 1,82E-09 |
| 31 | 9,31E-07 | 3,91E-07 |
| 32 | 1,51E-08 | 2,99E-08 |
| 33 | 3,57E-10 | 1,92E-09 |
| 34 | 3,67E-10 | 2,06E-09 |
| 35 | 5,36E-08 | 3,49E-07 |
| 36 | 1,04E-10 | 3,08E-10 |
| 37 | 3,70E-09 | 1,12E-08 |
| 38 | 4,04E-10 | 1,05E-09 |
| 39 | 1,19E-09 | 5,22E-09 |
| 40 | 1,93E-10 | 2,90E-10 |
| 41 | 3,19E-10 | 9,88E-10 |
| 42 | 1,11E-09 | 2,84E-09 |
| 43 | 1,17E-08 | 3,32E-08 |
| 44 | 2,11E-08 | 6,40E-08 |
| 45 | 2,46E-08 | 7,63E-08 |
| 46 | 2,73E-08 | 6,05E-08 |
| 47 | 3,28E-08 | 1,96E-08 |
| 48 | 3,68E-08 | 1,09E-07 |
| 49 | 5,74E-08 | 1,51E-07 |
| 50 | 6,37E-08 | 1,31E-07 |
| 51 | 7,56E-08 | 1,32E-07 |
| 52 | 7,71E-08 | 2,04E-07 |
| 53 | 1,12E-07 | 7,44E-07 |
| 54 | 1,18E-07 | 2,40E-07 |
| 55 | 1,18E-07 | 5,98E-07 |
| 56 | 1,52E-07 | 3,95E-07 |
| 57 | 1,56E-07 | 4,50E-07 |
| 58 | 2,49E-07 | 1,16E-06 |
| 59 | 4,99E-10 | 1,60E-09 |
| 60 | 8,09E-10 | 2,02E-09 |
| 61 | 8,72E-10 | 2,82E-09 |
| 62 | 1,14E-09 | 3,30E-09 |
| 63 | 1,44E-09 | 5,88E-09 |
| 64 | 1,47E-09 | 2,21E-09 |
| 65 | 2,48E-09 | 7,22E-09 |
| 66 | 2,61E-09 | 6,66E-09 |
| 67 | 3,01E-09 | 1,46E-08 |
| 68 | 3,64E-09 | 8,76E-09 |
| 69 | 3,65E-09 | 8,70E-09 |
| 70 | 5,33E-09 | 4,66E-08 |
| 71 | 5,37E-09 | 8,59E-09 |
| 72 | 5,82E-09 | 7,88E-08 |
| 73 | 8,70E-09 | 2,79E-08 |
| 74 | 1,12E-08 | 5,88E-08 |
| 75 | 4,50E-08 | 2,61E-08 |
| 76 | 2,78E-09 | 9,46E-09 |
| 77 | 8,53E-10 | 1,97E-09 |
| 78 | 1,04E-09 | 2,92E-09 |
| 79 | 4,41E-09 | 9,38E-09 |
| 80 | 5,83E-10 | 1,86E-09 |
| 81 | 3,91E-10 | 1,29E-09 |
| 82 | 2,67E-10 | 8,37E-10 |
| 83 | 2,09E-09 | 7,63E-09 |
| 84 | 3,25E-08 | 1,54E-07 |
| 85 | 5,00E-10 | 3,64E-09 |
| 86 | 9,11E-10 | 3,99E-09 |
| 87 | 1,52E-08 | 3,38E-08 |
| 88 | 1,89E-09 | 1,07E-08 |
| 89 | 5,47E-10 | 2,31E-09 |
| 90 | 3,43E-08 | 6,25E-08 |
| 91 | 7,73E-08 | 7,69E-07 |
| 92 | 3,75E-06 | ! 2.00E-5 |
| 93 | 1,02E-09 | 3,14E-09 |
| 94 | 3,65E-07 | > 1.63E-6 |
| 95 | 7,12E-08 | 2,46E-07 |
| 96 | 3,45E-09 | 2,21E-08 |
| 97 | 2,03E-09 | 8,62E-09 |
| 98 | 1,46E-08 | 5,91E-08 |
| 99 | 2,32E-09 | 1,08E-08 |
| 100 | 1,11E-09 | 4,67E-09 |
| 101 | 5,22E-08 | 8,98E-08 |
| 102 | 4,17E-10 | 1,29E-09 |
| 103 | 3,56E-09 | 1,69E-08 |
| 104 | 3,69E-08 | 1,49E-07 |
| 105 | 2,07E-10 | 5,37E-10 |
| 106 | 1,33E-08 | 7,74E-08 |
| 107 | 9,45E-08 | 3,42E-07 |
| 108 | 5,57E-10 | 2,41E-09 |
| 109 | 5,73E-10 | 8,37E-08 |
| 110 | 1,27E-09 | 5,97E-09 |
| 111 | 1,35E-10 | 3,30E-10 |
| 112 | 2,15E-10 | 9,79E-10 |
| 113 | 1,14E-10 | 7,24E-10 |
| 114 | 9,60E-10 | 4,35E-09 |
| 115 | 2,81E-10 | 8,75E-10 |
| 116 | 5,24E-08 | 2,15E-07 |
| 117 | 9,02E-08 | 2,96E-07 |
| 118 | 1,68E-08 | 7,99E-08 |
| 119 | 1,40E-08 | 5,80E-08 |
| 120 | 3,85E-08 | 9,87E-08 |
| 121 | 6,91E-09 | 1,80E-08 |
| 122 | 1,23E-07 | 7,75E-07 |
| 123 | 1,73E-07 | 7,01E-07 |
| 124 | 2,62E-08 | 1,21E-07 |
| 125 | 8,08E-08 | 2,19E-07 |
| 126 | 1,38E-08 | 3,98E-08 |
| 127 | 2,54E-08 | 9,76E-08 |
| 128 | 6,30E-08 | 2,08E-07 |
| 129 | 1,00E-09 | 9,26E-08 |
| 130 | 3,37E-07 | |
| 131 | 1,17E-09 | 3,58E-09 |
| 132 | 9,32E-09 | 2,75E-08 |
| 133 | 3,47E-06 | 5,59E-06 |
| 134 | 2,20E-08 | 1,01E-07 |
| 135 | 5,89E-10 | 1,83E-09 |
| 136 | 2,14E-10 | 7,06E-10 |
| 137 | 7,24E-10 | 1,55E-09 |
| 138 | 5,82E-10 | 1,36E-09 |
| 139 | 1,20E-07 | 4,92E-07 |
| 140 | 3,00E-10 | 1,33E-09 |
| 141 | 5,96E-10 | 2,21E-09 |
| 142 | < 7.25E-11 | 2,62E-10 |
| 143 | 1,29E-07 | 6,78E-07 |
| 144 | 5,12E-09 | 1,90E-08 |
| 145 | 3,51E-06 | 1,74E-05 |
| 146 | 3,27E-08 | 2,65E-07 |
| 147 | 6,99E-07 | 3,68E-06 |
| 148 | 1,05E-07 | 6,27E-07 |
| 149 | 7,75E-07 | 1,77E-05 |
| 150 | 4,66E-06 | ! 2.00E-5 |
| 151 | 1,10E-08 | 8,11E-08 |
| 152 | 1,64E-07 | 3,53E-07 |

### Cellular Data Description (WT, insSVD, insSVD T790M)

293T cells from ATCC were transfected with pBABEpuro expression constructs for WT EGFR or EGFR-insSVD, or EGFR-insSVD T790M, and pCL-Eco packaging vector using Fugene-6 transfection reagent from Promega. Plates were incubated at 37°C for 48 h. Retrovirus was harvested by filtering the media supernatant through a 0.45 µm filter.

Ba/F3 cells purchased from DSMZ were grown in RPMI + 10% FBS + 10 ng/mL IL-3 and infected with filtered retroviral supernatant at a 1:2 dilution. Polybrene was added to a concentration of 8 µg/mL, plates were spun for 90 min, and incubated for 16h at 37°C. 2 µg/mL puromycin was added to the infected cells 24 h after infection and cells were continually grown in the presence of puromycin and 10 ng/mL IL-3. Following stably expressing Ba/F3 cell lines were generated: Ba/F3-EGFR-WT, Ba/F3-EGFR-insSVD, Ba/F3-EGFR-insSVD T790M, (Ba/F3- vector-control).

For cell survival assays, Ba/F3 cells were grown to a density of 1-2 million cells per mL, spun down and resuspended in media without IL-3, and replated at a concentration of 200,000-500,000 cells per mL. The cells ectopically expressing WT EGFR, EGFR-insSVD, or EGFR-insSVD T790M were plated with 10 ng/mL Millipore Culture grade EGF. The cells ectopically expressing pBABEpuro empty vector were plated with 10 ng/mL IL-3.

2 days later, cells were plated in 50 µL in a 384 well plate at a concentration of 4000 cells per well for cells assayed in the absence of IL-3 and 2000 cells per well for cells assayed in the presence of IL-3. 100 nL of compound was added to each well using a 100 nL pin head, and plates were incubated at 37°C for 48 h.

Cell viability was measured by adding 20 µL of Cell Titer-Glo Luminescent Cell Viability Reagent diluted 1:3 in PBS. Plates were sealed with Perkin Elmer Top-Seal, inverted several times to mix, and immediately centrifuged at 1000 rpm for 2 min. Plates were incubated in low light conditions for 8-10 min and luminescence was measured. The IC₅₀ values for the examples are shown in Table 3.

**Table 3:**

| **Example No.** | **BA/F3 (insSVD) IC₅₀ [mol/l]** | **BA/F3 (EGFR D770_N771 insSVD T790M) IC₅₀ [mol/l]** |
|---|---|---|
| 1 | 4,47E-06 | 9,98E-06 |
| 2 | 5,02E-06 | 9,98E-06 |
| 3 | 9,98E-06 | 9,98E-06 |
| 4 | 3,46E-07 | 9,98E-06 |
| 5 | 9,98E-06 | 9,98E-06 |
| 6 | 7,08E-06 | 9,98E-06 |
| 7 | 4,15E-06 | 6,99E-06 |
| 8 | 5,27E-06 | 8,93E-06 |
| 9 | 2,93E-07 | 8,72E-06 |
| 10 | 2,93E-06 | 9,98E-06 |
| 11 | 1,12E-06 | 9,98E-06 |
| 12 | 5,38E-07 | 9,10E-06 |
| 13 | 6,19E-06 | 9,98E-06 |
| 14 | 8,33E-07 | 9,98E-06 |
| 15 | 9,24E-06 | 9,98E-06 |
| 16 | 5,84E-07 | |
| 17 | 8,16E-06 | 9,98E-06 |
| 18 | 7,98E-06 | 9,98E-06 |
| 19 | 6,10E-06 | 9,98E-06 |
| 20 | 4,65E-06 | 8,99E-06 |
| 21 | 4,16E-06 | 9,98E-06 |
| 22 | 2,62E-07 | 7,64E-06 |
| 23 | 2,15E-06 | 8,86E-06 |
| 24 | 7,26E-07 | 8,32E-06 |
| 25 | 3,88E-08 | 3,86E-06 |
| 26 | 1,53E-06 | 9,98E-06 |
| 27 | 5,32E-07 | 8,41E-06 |
| 28 | 9,98E-06 | 9,98E-06 |
| 29 | 5,24E-08 | 3,13E-06 |
| 30 | 6,22E-08 | 8,31E-07 |
| 31 | 5,53E-06 | 9,98E-06 |
| 32 | 3,64E-07 | 9,65E-06 |
| 33 | 3,07E-08 | 2,53E-07 |
| 34 | 1,69E-08 | 7,50E-07 |
| 35 | 1,40E-06 | 9,98E-06 |
| 36 | 3,27E-09 | 4,46E-08 |
| 37 | 2,29E-07 | 9,13E-06 |
| 38 | 2,01E-08 | 1,47E-07 |
| 39 | 9,14E-08 | 5,40E-06 |
| 40 | 8,54E-09 | 6,79E-08 |
| 41 | 2,59E-08 | 7,82E-07 |
| 42 | 4,47E-08 | 3,64E-06 |
| 43 | 5,38E-07 | |
| 44 | 9,99E-07 | |
| 45 | 1,28E-06 | |
| 46 | 1,15E-06 | |
| 47 | 4,93E-07 | |
| 48 | 9,62E-07 | |
| 49 | 1,93E-06 | |
| 50 | 1,77E-06 | |
| 51 | 1,92E-06 | |
| 52 | 3,10E-06 | |
| 53 | 3,27E-06 | |
| 54 | 3,96E-06 | |
| 55 | 3,05E-06 | |
| 56 | 4,18E-06 | |
| 57 | 4,59E-06 | |
| 58 | 6,28E-06 | |
| 59 | 7,64E-08 | |
| 60 | 1,54E-07 | |
| 61 | 1,20E-07 | |
| 62 | 3,26E-07 | |
| 63 | 4,55E-07 | |
| 64 | 9,29E-08 | |
| 65 | 2,67E-07 | |
| 66 | 4,21E-07 | |
| 67 | 4,18E-07 | |
| 68 | 2,96E-07 | |
| 69 | 2,87E-07 | |
| 70 | 7,49E-07 | |
| 71 | 4,04E-07 | |
| 72 | 4,63E-07 | |
| 73 | 5,55E-07 | |
| 74 | 9,64E-07 | |
| 75 | 2,02E-06 | |
| 76 | 9,79E-08 | |
| 77 | 2,74E-08 | |
| 78 | 8,02E-08 | |
| 79 | 3,67E-07 | |
| 80 | 1,62E-07 | |
| 81 | 5,74E-08 | |
| 82 | 3,68E-08 | |
| 83 | 1,32E-07 | |
| 84 | 3,56E-06 | |
| 85 | 5,57E-08 | |
| 86 | 6,70E-08 | |
| 87 | 7,59E-07 | |
| 88 | 1,66E-07 | |
| 89 | 2,42E-08 | |
| 90 | 9,99E-07 | |
| 91 | 1,47E-06 | |
| 92 | 7,05E-06 | |
| 93 | 5,53E-08 | |
| 94 | 2,44E-06 | |
| 95 | 6,82E-07 | |
| 96 | 3,73E-07 | |
| 97 | 1,79E-07 | |
| 98 | 7,47E-07 | |
| 99 | 1,15E-07 | |
| 100 | 5,75E-08 | |
| 101 | 9,57E-07 | |
| 102 | 1,35E-07 | |
| 103 | 6,50E-07 | |
| 104 | 1,38E-06 | |
| 105 | 3,11E-08 | |
| 106 | 1,63E-06 | |
| 107 | 1,51E-06 | |
| 108 | 7,04E-08 | |
| 109 | 4,66E-08 | |
| 110 | 3,75E-08 | |
| 111 | 1,09E-08 | 7,77E-08 |
| 112 | 1,75E-08 | |
| 113 | 9,99E-09 | |
| 114 | 3,89E-08 | |
| 115 | 1,22E-08 | |
| 116 | 1,52E-06 | |
| 117 | 4,43E-06 | |
| 118 | 7,23E-07 | |
| 119 | 5,49E-07 | |
| 120 | 1,19E-06 | |
| 121 | 4,97E-07 | |
| 122 | 2,59E-06 | |
| 123 | 8,32E-07 | |
| 124 | 9,04E-07 | |
| 125 | 1,03E-06 | |
| 126 | 8,07E-07 | |
| 127 | 6,91E-07 | |
| 128 | 7,65E-07 | |
| 129 | 1,10E-07 | |
| 130 | 1,62E-06 | |
| 131 | 5,46E-08 | |
| 132 | 1,84E-07 | |
| 133 | 9,00E-06 | |
| 134 | 6,22E-07 | |
| 135 | 1,61E-08 | |
| 136 | 8,43E-09 | |
| 137 | 2,51E-08 | |
| 138 | 2,70E-08 | |
| 139 | 2,52E-06 | |
| 140 | 9,73E-09 | |
| 141 | 2,31E-07 | |
| 142 | 1,60E-08 | |
| 143 | 3,84E-06 | |
| 144 | 1,52E-06 | |
| 145 | 9,98E-06 | |
| 146 | 1,24E-06 | |
| 147 | 6,53E-06 | |
| 148 | 2,78E-06 | |
| 149 | 1,29E-06 | |
| 150 | 9,81E-06 | |
| 151 | 2,82E-07 | |
| 152 | 1,88E-06 | |

### Cellular Data Description (L858R, E746_A750del, L858R T790M, E746_A750del T790M₎

293T cells from ATCC were transfected with pBABEpuro expression constructs for EGFR-L858R, EGFR-E746_A750del, EGFR-L858R T790M, or EGFR-E746_A750del T790M, and pCL-Eco packaging vector using Fugene-6 transfection reagent from Promega. Plates were incubated at at 37°C for 48 h. Retrovirus was harvested by filtering the media supernatant through a 0.45 µm filter.

Ba/F3 cells purchased from DSMZ were grown in RPMI + 10% FBS + 10 ng/mL IL-3 and infected with filtered retroviral supernatant at a 1:2 dilution. Polybrene was added to a concentration of 8 µg/mL, plates were spun for 90 min, and incubated for 16h at 37°C. 2 µg/mL puromycin was added to the infected cells 24 h after infection and cells were continually grown in the presence of puromycin and 10 ng/mL IL-3. Following stably expressing Ba/F3 cell lines were generated: Ba/F3-EGFR-L858R, Ba/F3-EGFR-E746_A750del, Ba/F3-EGFR-L858R T790M, or Ba/F3-EGFR-E746_A750del T790M.

For cell survival assays, Ba/F3 cells were grown to a density of 1-2 million cells per mL, spun down and resuspended in media without IL-3, and replated at a concentration 200,000-500,000 cells per mL. The cells ectopically expressing EGFR-L858R, EGFR-E746_A750del, EGFR-L858R T790M, or EGFR-E746_A750del T790M were plated with 10 ng/mL Millipore Culture grade EGF. The cells ectopically expressing pBABEpuro empty vector were plated with 10 ng/mL IL-3.

2 days later, cells were plated in 50 µL in a 384 well plate at a concentration of 4000 cells per well for cells assayed in the absence of IL-3 and 2000 cells per well for cells assayed in the presence of IL-3. 100 nL of compound was added to each well using a 100 nL pin head, and plates were incubated at 37°C for 48 h.

Cell viability was measured by adding 20 µL of Cell Titer-Glo Luminescent Cell Viability Reagent diluted 1:3 in PBS. Plates were sealed with Perkin Elmer Top-Seal, inverted several times to mix, and immediately centrifuged at 1000 rpm for 2 min. Plates were incubated in low light conditions for 8-10 min and luminescence was measured.

### References:

Arcila et al., 2012: Arcila et al., Clin Cancer Res. 2012 Sep 15;18(18):4910-8.
Chen et al., 2016: Chen et al., Onco Targets Ther. 2016 Jul 8;9:4181-6
Chiu et al., 2015 : Chiu et al., J Thorac Oncol. 2015;10: 793-799
Friedlaender et al., 2022: Friedlaender et al., Nat Rev Clin Oncol. 2022 Jan;19(1):51-69
Gridelli et al., 2015: Gridelli et al., Nat Rev Dis Primers. 2015 May 21;1:15009.
Mok et al., 2009 : Mok et al., N Engl J Med. 2009 Sep 3;361(10):947-57
Mok et al., 2017: Mok et al., N Engl J Med. 2017 Feb 16;376(7):629-640
Paez et al., 2004 : Paez et al., Science. 2004 Jun 4;304(5676):1497-500
Pao et al., 2005: Pao et al., PLoS Med. 2005 Mar;2(3):e73
Pao et al., 2010: Pao and Chmielecki, Nat Rev Cancer. 2010 Nov;10(11):760-74
Rangachari et al., 2015: Rangachari et al., Lung Cancer. 2015 Apr;88(1):108-11
Reungwetwattana et al., 2018: Reungwetwattana et al., J Clin Oncol. 2018 Nov 20; 36(33): 3290-3297
Sequist et al., 2013: Sequist et al., J Clin Oncol. 2013 Sep 20;31(27):3327-34
Soria et al., 2018: Soria et al., N Engl J Med. 2018 Jan 11;378(2):113-125.
Yang et al., 2015: Yang et al., Lancet Oncol. 2015 Jul;16(7):830-8
Yasuda, 2013: Yasuda, Sci Transl Med. 2013 Dec 18;5(216):216ra177.

## Claims

1. A compound of formula (I) 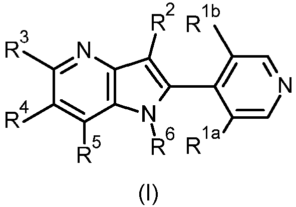 in which:
R^{1a} represents a group selected from the group: -O-(C₂-C₆-alkanediyl)-NR⁷R⁸, -O-CH₂-(C₁-C₅-haloalkanediyl)-NR⁷R⁸, -O-(C₁-C₅-alkanediyl)-R⁹, or -O-R⁹;
R^{1b} represents a hydrogen atom or fluoro;
R² represents phenyl or heteroaryl, wherein said groups are substituted, one or more times, independently of each other, with R¹⁰;
R³ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R⁴ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R⁶ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R⁶ represents a hydrogen atom or methyl;
R⁷ represents -(CO)-R¹¹, -(CO)-C≡C-R¹², -(SO₂)-CH=CH₂, or oxirane-2-carbonyl;
R⁸ represents a hydrogen atom or C₁-C₃-alkyl;
R⁹ represents a group selected from the group: 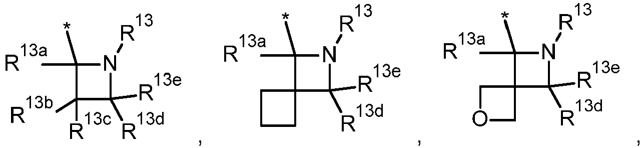 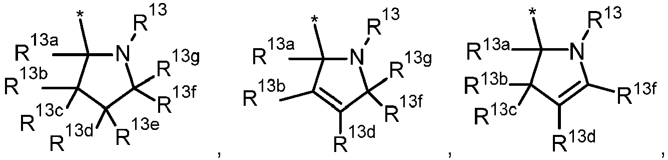 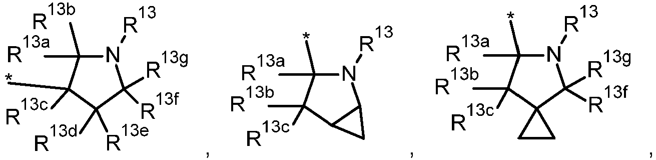 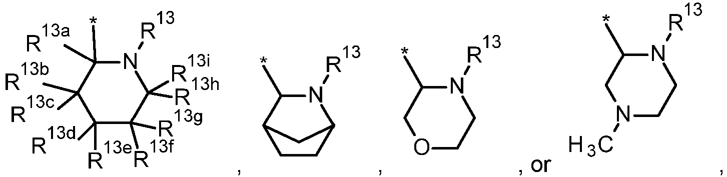 wherein * indicates the point of attachment of said group with the rest of the molecule;
R¹⁰ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R¹¹ represents -CH₂-C≡CH, or -C(R¹⁴)-R¹⁵;
R¹² represents a hydrogen atom, methyl, -CH₂₋N(CH₃)-CHR¹⁶R¹⁷, or 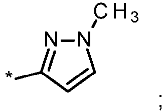
R¹³ represents -(CO)-R¹¹, -(CO)-C≡C-R¹², -(SO₂)-CH=CH₂, or oxirane-2-carbonyl;
R^{13a} represents a hydrogen atom, methyl, or fluoro;
R^{13b} represents a hydrogen atom, methyl, or fluoro;
R^{13c} represents a hydrogen atom, methyl, or fluoro;
R^{13d} represents a hydrogen atom, methyl, fluoro, or -N(CH₃)-CH₂-CH₂-N(CH₃)-CHR¹⁶R¹⁷;
R^{13e} represents a hydrogen atom, methyl, or fluoro;
R^{13f} represents a hydrogen atom, methyl, or fluoro;
R^{13g} represents a hydrogen atom, methyl, or fluoro;
R^{13h} represents a hydrogen atom, methyl, or fluoro;
R¹³ⁱ represents a hydrogen atom, methyl, or fluoro;
R¹⁴ represents =CH₂, =CH-CH₃, =CH-CH₂₋N(CH₃)-CHR¹⁶R¹⁷, or =CH-CH₂₋R¹⁸;
R¹⁶ represents a hydrogen atom, C₁-C₃-alkyl, or fluoro;
R¹⁶ represents a hydrogen atom or methyl;
R¹⁷ represents a hydrogen atom or methyl;
R¹⁸ represents a group selected from the group: 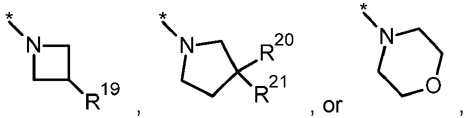 wherein * indicates the point of attachment of said group with the rest of the molecule;
R¹⁹ represents a hydrogen atom, methyl, methoxy, or fluoro;
R²⁰ represents a hydrogen atom, methyl, methoxy, or fluoro;
R²¹ represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

2. The compound of formula (I) according to claim 1, wherein:
R^{1a} represents a group selected from the group: -O-(C₂-C₆-alkanediyl)-NR⁷R⁸, -O-CH₂-(C₁-C₅-haloalkanediyl)-NR⁷R⁸, -O-(C₁-C₅-alkanediyl)-R⁹, or -O-R⁹;
R^{1b} represents a hydrogen atom or fluoro;
R² represents a group selected from the group: 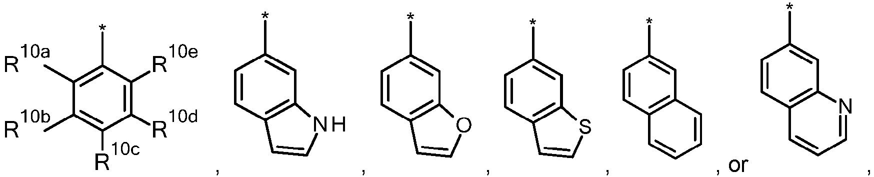 wherein * indicates the point of attachment of said group with the rest of the molecule;
R³ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R⁴ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R⁵ represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R⁶ represents a hydrogen atom or methyl;
R⁷ represents -(CO)-R¹¹, -(CO)-C≡C-R¹², -(SO₂)-CH=CH₂, or oxirane-2-carbonyl;
R⁸ represents a hydrogen atom or C₁-C₃-alkyl;
R⁹ represents a group selected from the group: 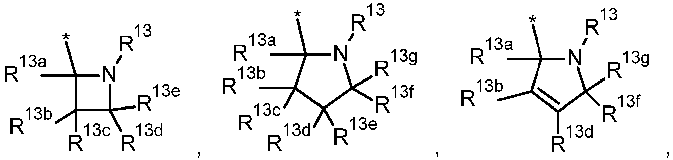 or 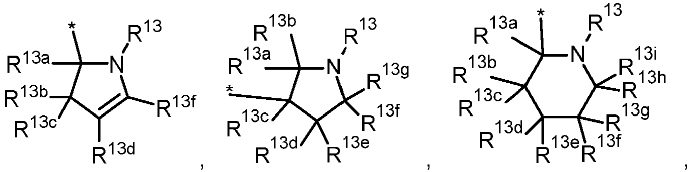 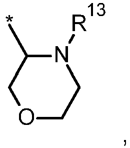 wherein * indicates the point of attachment of said group with the rest of the molecule;
R^{10a} represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R^{10b} represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R^{10c} represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R^{10d} represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁- C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
-R^{10e} represents a hydrogen atom, amino, C₁-C₃-alkyl, C₂-C₃-alkenyl, C₂-C₃-alkinyl, C₁-C₃-haloalkyl, C₁-C₃-hydroxyalkyl, C₁-C₃-alkoxy, C₁-C₃-haloalkoxy, C₁-C₃-alkoxy-C₁-C₃-alkyl, amino-C₁-C₃-alkyl, C₁-C₃-alkylamino-C₁-C₃-alkyl, (C₁-C₃-alkyl)₂amino-C₁-C₃-alkyl, C₁-C₃-alkoxycarbonyl, aminocarbonyl, C₁-C₃-alkylaminocarbonyl, (C₁-C₃-alkyl)₂₋aminocarbonyl, cyano, fluoro, chloro, or bromo;
R¹¹ represents -CH₂-C≡CH, or -C(R¹⁴)-R¹⁵;
R¹² represents a hydrogen atom, methyl, -CH₂₋N(CH₃)-CHR¹⁶R¹⁷, or 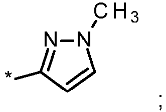
R¹³ represents -(CO)-R¹¹, -(CO)-C≡C-R¹², -(SO₂)-CH=CH₂, or oxirane-2-carbonyl;
R^{13a} represents a hydrogen atom, methyl, or fluoro;
R^{13b} represents a hydrogen atom, methyl, or fluoro;
R^{13c} represents a hydrogen atom, methyl, or fluoro;
R^{13d} represents a hydrogen atom, methyl, fluoro, or -N(CH₃)-CH₂-CH₂-N(CH₃)-CHR¹⁶R¹⁷;
R^{13e} represents a hydrogen atom, methyl, or fluoro;
R^{13f} represents a hydrogen atom, methyl, or fluoro;
R^{13g} represents a hydrogen atom, methyl, or fluoro;
R^{13h} represents a hydrogen atom, methyl, or fluoro;
R¹³ⁱ represents a hydrogen atom, methyl, or fluoro;
R¹⁴ represents =CH₂, =CH-CH₃, =CH-CH₂₋N(CH₃)-CHR¹⁶R¹⁷, or =CH-CH₂₋R¹⁸;
R¹⁵ represents a hydrogen atom, C₁-C₃-alkyl, or fluoro;
R¹⁶ represents a hydrogen atom or methyl;
R¹⁷ represents a hydrogen atom or methyl;
R¹⁸ represents a group selected from the group: 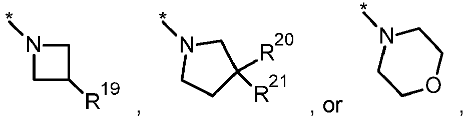 wherein * indicates the point of attachment of said group with the rest of the molecule;
R¹⁹ represents a hydrogen atom, methyl, methoxy, or fluoro;
R²⁰ represents a hydrogen atom, methyl, methoxy, or fluoro;
R²¹ represents a hydrogen atom, methyl, methoxy, or fluoro;
or an N-oxide, a salt, a tautomer, a rotamer, or a stereoisomer of said compound, or a salt of said N-oxide, tautomer, rotamer, or stereoisomer.

3. The compound of formula (I) according any of claims 1 or 2, wherein:
R^{1a} represents a group selected from the group: -O-(C₂-C₄-alkanediyl)-NR⁷R⁸, -O-CH₂-R⁹, or -O-R⁹;
R^{1b} represents a hydrogen atom or fluoro;
R² represents a group selected from the group: 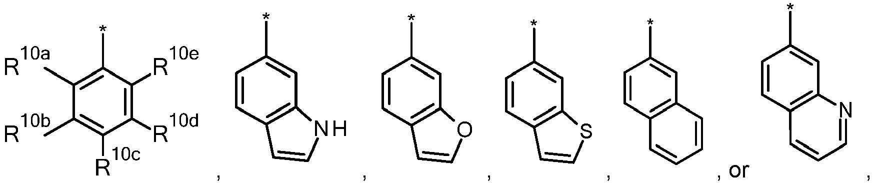 wherein * indicates the point of attachment of said group with the rest of the molecule;
R³ represents a hydrogen atom, or methoxy;
R⁴ represents a hydrogen atom, or fluoro;
R⁵ represents a hydrogen atom or methyl;
R⁶ represents a hydrogen atom;
R⁷ represents -(CO)-R¹¹, -(CO)-C≡C-R¹², or -(SO₂)-CH=CH₂;
R⁸ represents a hydrogen atom, methyl, or ethyl;
R⁹ represents a group selected from the group: 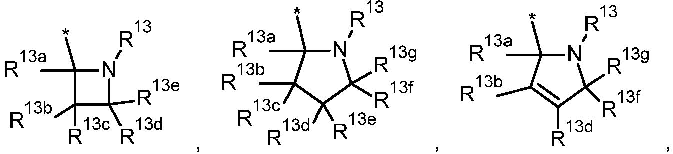 or 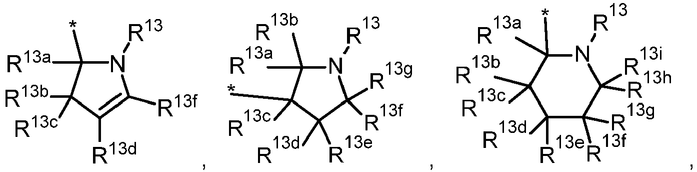 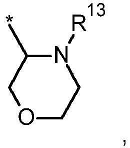 wherein * indicates the point of attachment of said group with the rest of the molecule;
R^{10a} represents a hydrogen atom, ethyl, methoxy, or fluoro;
R^{10b} represents a hydrogen atom, methyl, ethyl, iso-propyl, ethinyl, trifluoromethyl, methoxy, difluoromethoxy, trifluoromethoxy, methoxycarbonyl, cyano, fluoro, or chloro;
R^{10c} represents a hydrogen atom, fluoro, or chloro;
R^{10d} represents a hydrogen atom, methyl, ethyl, fluoro, or chloro;
R^{10e} represents a hydrogen atom, fluoro, or chloro;
R¹¹ represents -C(R¹⁴)-R¹⁵;
R¹² represents a hydrogen atom, or methyl;
R¹³ represents -(CO)-R¹¹, -(CO)-C≡C-R¹², or -(SO₂)-CH=CH₂;
R^{13a} represents a hydrogen atom, or methyl;
R^{13b} represents a hydrogen atom;
R^{13c} represents a hydrogen atom;
R^{13d} represents a hydrogen atom, methyl, fluoro, or -N(CH₃)-CH₂-CH₂-N(CH₃)-CHR¹⁶R¹⁷;
R^{13e} represents a hydrogen atom, or fluoro;
R^{13f} represents a hydrogen atom;
R^{13g} represents a hydrogen atom;
R^{13h} represents a hydrogen atom;
R¹³ⁱ represents a hydrogen atom;
R¹⁴ represents =CH₂, or =CH-CH₂₋N(CH₃)-CHR¹⁶R¹⁷;
R¹⁵ represents a hydrogen atom;
R¹⁶ represents a hydrogen atom;
R¹⁷ represents a hydrogen atom;
or an N-oxide, a salt, a tautomer, a rotamer, or a stereoisomer of said compound, or a salt of said N-oxide, tautomer, rotamer, or stereoisomer.

4. The compound of formula (I) according to any of claims 1 to 3, which is selected from the group consisting of:
N-[2-({4-[3-(4-fluorophenyl)-1H-pyrrolo[3,2-b]pyridin-2-yl]pyridin-3-yl}oxy)ethyl]prop-2-enamide,
N-(2-{[4-(3-phenyl-1H-pyrrolo[3,2-b]pyridin-2-yl)pyridin-3-yl]oxy}ethyl)prop-2-enamide,
N-methyl-N-(3-{[4-(3-phenyl-1H-pyrrolo[3,2-b]pyridin-2-yl)pyridin-3-yl]oxy}propyl)prop-2-enamide,
N-methyl-N-(2-([4-(3-phenyl-1H-pyrrolo[3,2-b]pyridin-2-yl)pyridin-3-yl]oxy}ethyl)prop-2-enamide,
N-(3-{[4-(3-phenyl-1H-pyrrolo[3,2-b]pyridin-2-yl)pyridin-3-yl]oxy}propyl)prop-2-enamide,
N-(2-{[4-(6-fluoro-3-phenyl-1H-pyrrolo[3,2-b]pyridin-2-yl)pyridin-3-yl]oxy}ethyl)prop-2-enamide,
N-(2-{[4-(6-fluoro-3-phenyl-1H-pyrrolo[3,2-b]pyridin-2-yl)pyridin-3-yl]oxy}ethyl)-N-methylprop-2-enamide,
N-(2-{[4-(5-methoxy-3-phenyl-1H-pyrrolo[3,2-b]pyridin-2-yl)pyridin-3-yl]oxy}ethyl)-N-methylprop-2-enamide,
N-methyl-N-[2-({4-[3-(3-methylphenyl)-1H-pyrrolo[3,2-b]pyridin-2-yl]pyridin-3-yl}oxy)ethyl]prop-2-enamide,
N-[2-({4-[3-(4-chloro-3-methylphenyl)-1H-pyrrolo[3,2-b]pyridin-2-yl]pyridin-3-yl}oxy)ethyl]-N-methylprop-2-enamide,
N-[2-({4-[3-(4-chlorophenyl)-1H-pyrrolo[3,2-b]pyridin-2-yl]pyridin-3-yl}oxy)ethyl]-N-methylprop-2-enamide,
N-[2-({4-[3-(4-fluoro-3-methylphenyl)-1H-pyrrolo[3,2-b]pyridin-2-yl]pyridin-3-yl}oxy)ethyl]-N-methylprop-2-enamide,
Methyl 2-chloro-5-[2-(3-{2-[methyl(prop-2-enoyl)amino]ethoxy}pyridin-4-yl)-1H-pyrrolo[3,2-b]pyridin-3-yl]benzoate,
N-[2-({4-[3-(3-fluorophenyl)-1H-pyrrolo[3,2-b]pyridin-2-yl]pyridin-3-yl}oxy)ethyl]-N-methylprop-2-enamide,
N-[2-({4-[3-(4-chloro-3-methoxyphenyl)-1H-pyrrolo[3,2-b]pyridin-2-yl]pyridin-3-yl}oxy)ethyl]-N-methylprop-2-enamide,
N-[2-({4-[3-(1H-indol-6-yl)-1H-pyrrolo[3,2-b]pyridin-2-yl]pyridin-3-yl}oxy)ethyl]-N-methylprop-2-enamide,
N-[2-({4-[3-(2-methoxyphenyl)-1H-pyrrolo[3,2-b]pyridin-2-yl]pyridin-3-yl}oxy)ethyl]-N-methylprop-2-enamide,
N-methyl-N-[2-({4-[3-(quinolin-7-yl)-1H-pyrrolo[3,2-b]pyridin-2-yl]pyridin-3-yl}oxy)ethyl]prop-2-enamide,
N-[2-({4-[3-(3-fluoro-2-methoxyphenyl)-1H-pyrrolo[3,2-b]pyridin-2-yl]pyridin-3-yl}oxy)ethyl]-N-methylprop-2-enamide,
N-[2-({4-[3-(4-chloro-3-ethylphenyl)-1H-pyrrolo[3,2-b]pyridin-2-yl]pyridin-3-yl}oxy)ethyl]-N-methylprop-2-enamide,
N-[2-({4-[3-(3-chloro-2-methoxyphenyl)-1H-pyrrolo[3,2-b]pyridin-2-yl]pyridin-3-yl}oxy)ethyl]-N-methylprop-2-enamide,
N-[2-({4-[3-(3-chlorophenyl)-1H-pyrrolo[3,2-b]pyridin-2-yl]pyridin-3-yl}oxy)ethyl]-N-methylprop-2-enamide,
N-[2-({4-[3-(3,4-dichlorophenyl)-1H-pyrrolo[3,2-b]pyridin-2-yl]pyridin-3-yl}oxy)ethyl]-N-methylprop-2-enamide,
N-[2-({4-[3-(3-chloro-4-fluorophenyl)-1H-pyrrolo[3,2-b]pyridin-2-yl]pyridin-3-yl}oxy)ethyl]-N-methylprop-2-enamide,
N-methyl-N-(2-{[4-(3-phenyl-1H-pyrrolo[3,2-b]pyridin-2-yl)pyridin-3-yl]oxy}ethyl)ethenesulfonamide,
(2E)-4-(dimethylamino)-N-methyl-N-(2-{[4-(3-phenyl-1H-pyrrolo[3,2-b]pyridin-2-yl)pyridin-3-yl]oxy}ethyl)but-2-enamide,
N-methyl-N-(2-{[4-(3-phenyl-1H-pyrrolo[3,2-b]pyridin-2-yl)pyridin-3-yl]oxy}ethyl)prop-2-ynamide,
N-methyl-N-(2-{[4-(3-phenyl-1H-pyrrolo[3,2-b]pyridin-2-yl)pyridin-3-yl]oxy}ethyl)but-2-ynamide,
N-[2-({4-[3-(3-chlorophenyl)-1H-pyrrolo[3,2-b]pyridin-2-yl]pyridin-3-yl}oxy)ethyl]-N-methylethenesulfonamide,
N-[2-({4-[3-(1H-indol-6-yl)-1H-pyrrolo[3,2-b]pyridin-2-yl]pyridin-3-yl}oxy)ethyl]-N-methylethenesulfonamide,
N-[2-({4-[3-(3,5-dichlorophenyl)-1H-pyrrolo[3,2-b]pyridin-2-yl]pyridin-3-yl}oxy)ethyl]-N-methylprop-2-enamide,
N-ethyl-N-(2-{[4-(3-phenyl-1H-pyrrolo[3,2-b]pyridin-2-yl)pyridin-3-yl]oxy}ethyl)prop-2-enamide,
N-ethyl-N-(2-{[4-(3-phenyl-1H-pyrrolo[3,2-b]pyridin-2-yl)pyridin-3-yl]oxy}ethyl)ethenesulfonamide,
1-[(2S)-2-({[4-(3-phenyl-1H-pyrrolo[3,2-b]pyridin-2-yl)pyridin-3-yl]oxy}methyl)pyrrolidin-1-yl]prop-2-en-1-one,
1-[(2R)-2-({[4-(3-phenyl-1H-pyrrolo[3,2-b]pyridin-2-yl)pyridin-3-yl]oxy}methyl)pyrrolidin-1-yl]prop-2-en-1-one,
2-(3-{[(2S)-1-(ethenesulfonyl)pyrrolidin-2-yl]methoxy}pyridin-4-yl)-3-phenyl-1H-pyrrolo[3,2-b]pyridine,
(2E)-4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one,
2-(3-{[(2S)-1-(ethenesulfonyl)pyrrolidin-2-yl]methoxy}pyridin-4-yl)-3-[3-(trifluoromethyl)phenyl]-1H-pyrrolo[3,2-b]pyridine,
1-[(2S)-2-{[(4-{3-[3-(trifluoromethyl)phenyl]-1H-pyrrolo[3,2-b]pyridin-2-yl}pyridin-3-yl)oxy]methyl}pyrrolidin-1-yl]prop-2-en-1-one,
3-(3-chlorophenyl)-2-(3-{[(2S)-1-(ethenesulfonyl)pyrrolidin-2-yl]methoxy}pyridin-4-yl)-1H-pyrrolo[3,2-b]pyridine,
1-{(2S)-2-[({4-[3-(3-chlorophenyl)-1H-pyrrolo[3,2-b]pyridin-2-yl]pyridin-3-yl}oxy)methyl]pyrrolidin-1-yl}prop-2-en-1-one,
1-{(2S)-2-[({4-[3-(5-chloro-2-fluorophenyl)-1H-pyrrolo[3,2-b]pyridin-2-yl]pyridin-3-yl}oxy)methyl]pyrrolidin-1-yl}prop-2-en-1-one,
N-[2-({4-[3-(5-chloro-2-fluorophenyl)-1H-pyrrolo[3,2-b]pyridin-2-yl]pyridin-3-yl}oxy)ethyl]-N-methylprop-2-enamide
N-[2-({4-[3-(1-benzothiophen-6-yl)-1H-pyrrolo[3,2-b]pyridin-2-yl]pyridin-3-yl}oxy)ethyl]-N-methylprop-2-enamide
N-[2-({4-[3-(3-ethylphenyl)-1H-pyrrolo[3,2-b]pyridin-2-yl]pyridin-3-yl}oxy)ethyl]-N-methylprop-2-enamide
N-[2-({4-[3-(2,5-difluorophenyl)-1H-pyrrolo[3,2-b]pyridin-2-yl]pyridin-3-yl}oxy)ethyl]-N-methylprop-2-enamide
N-[2-({4-[3-(2-fluoro-5-methylphenyl)-1H-pyrrolo[3,2-b]pyridin-2-yl]pyridin-3-yl}oxy)ethyl]-N-methylprop-2-enamide
N-[2-({4-[3-(5-ethyl-2-fluorophenyl)-1H-pyrrolo[3,2-b]pyridin-2-yl]pyridin-3-yl}oxy)ethyl]-N-methylprop-2-enamide
N-methyl-N-{2-[(4-{3-[3-(trifluoromethyl)phenyl]-1H-pyrrolo[3,2-b]pyridin-2-yl}pyridin-3-yl)oxy]ethyl}prop-2-enamide
N-[2-({4-[3-(3-fluoro-5-methylphenyl)-1H-pyrrolo[3,2-b]pyridin-2-yl]pyridin-3-yl}oxy)ethyl]-N-methylprop-2-enamide
N-{2-[(4-{3-[2-fluoro-5-(trifluoromethyl)phenyl]-1H-pyrrolo[3,2-b]pyridin-2-yl}pyridin-3-yl)oxy]ethyl}-N-methylprop-2-enamide
N-[2-({4-[3-(3,5-difluorophenyl)-1H-pyrrolo[3,2-b]pyridin-2-yl]pyridin-3-yl}oxy)ethyl]-N-methylprop-2-enamide
N-[2-({4-[3-(2-ethylphenyl)-1H-pyrrolo[3,2-b]pyridin-2-yl]pyridin-3-yl}oxy)ethyl]-N-methylprop-2-enamide
N-[2-({4-[3-(3-methoxyphenyl)-1H-pyrrolo[3,2-b]pyridin-2-yl]pyridin-3-yl}oxy)ethyl]-N-methylprop-2-enamide
N-methyl-N-{2-[(4-{3-[3-(propan-2-yl)phenyl]-1H-pyrrolo[3,2-b]pyridin-2-yl}pyridin-3-yl)oxy]ethyl}prop-2-enamide
N-[2-({4-[3-(2-fluoro-5-methoxyphenyl)-1H-pyrrolo[3,2-b]pyridin-2-yl]pyridin-3-yl}oxy)ethyl]-N-methylprop-2-enamide
N-[2-({4-[3-(2,5-dichlorophenyl)-1H-pyrrolo[3,2-b]pyridin-2-yl]pyridin-3-yl}oxy)ethyl]-N-methylprop-2-enamide
N-methyl-N-{2-[(4-{3-[3-(trifluoromethoxy)phenyl]-1H-pyrrolo[3,2-b]pyridin-2-yl}pyridin-3-yl)oxy]ethyl}prop-2-enamide
N-{2-[(4-{3-[2-fluoro-5-(trifluoromethyl)phenyl]-1H-pyrrolo[3,2-b]pyridin-2-yl}pyridin-3-yl)oxy]ethyl}-N-methylethenesulfonamide
N-[2-({4-[3-(5-chloro-2-fluorophenyl)-1H-pyrrolo[3,2-b]pyridin-2-yl]pyridin-3-yl}oxy)ethyl]-N-methylethenesulfonamide
N-[2-({4-[3-(2,5-difluorophenyl)-1H-pyrrolo[3,2-b]pyridin-2-yl]pyridin-3-yl}oxy)ethyl]-N-methylethenesulfonamide
N-[2-({4-[3-(1-benzothiophen-6-yl)-1H-pyrrolo[3,2-b]pyridin-2-yl]pyridin-3-yl}oxy)ethyl]-N-methylethenesulfonamide
N-[2-({4-[3-(3-ethylphenyl)-1H-pyrrolo[3,2-b]pyridin-2-yl]pyridin-3-yl}oxy)ethyl]-N-methylethenesulfonamide
N-[2-({4-[3-(2-fluoro-5-methylphenyl)-1H-pyrrolo[3,2-b]pyridin-2-yl]pyridin-3-yl}oxy)ethyl]-N-methylethenesulfonamide
N-[2-({4-[3-(3-fluoro-5-methylphenyl)-1H-pyrrolo[3,2-b]pyridin-2-yl]pyridin-3-yl}oxy)ethyl]-N-methylethenesulfonamide
N-methyl-N-{2-[(4-{3-[3-(trifluoromethyl)phenyl]-1H-pyrrolo[3,2-b]pyridin-2-yl}pyridin-3-yl)oxy]ethyl}ethenesulfonamide
N-[2-({4-[3-(5-ethyl-2-fluorophenyl)-1H-pyrrolo[3,2-b]pyridin-2-yl]pyridin-3-yl}oxy)ethyl]-N-methylethenesulfonamide
N-[2-({4-[3-(3,5-difluorophenyl)-1H-pyrrolo[3,2-b]pyridin-2-yl]pyridin-3-yl}oxy)ethyl]-N-methylethenesulfonamide
N-[2-({4-[3-(3-methoxyphenyl)-1H-pyrrolo[3,2-b]pyridin-2-yl]pyridin-3-yl}oxy)ethyl]-N-methylethenesulfonamide
N-methyl-N-{2-[(4-{3-[3-(propan-2-yl)phenyl]-1H-pyrrolo[3,2-b]pyridin-2-yl}pyridin-3-yl)oxy]ethyl}ethenesulfonamide
N-[2-({4-[3-(2,5-dichlorophenyl)-1H-pyrrolo[3,2-b]pyridin-2-yl]pyridin-3-yl}oxy)ethyl]-N-methylethenesulfonamide
N-[2-({4-[3-(2-ethylphenyl)-1H-pyrrolo[3,2-b]pyridin-2-yl]pyridin-3-yl}oxy)ethyl]-N-methylethenesulfonamide
N-[2-({4-[3-(2-fluoro-5-methoxyphenyl)-1H-pyrrolo[3,2-b]pyridin-2-yl]pyridin-3-yl}oxy)ethyl]-N-methylethenesulfonamide
N-methyl-N-{2-[(4-{3-[3-(trifluoromethoxy)phenyl]-1H-pyrrolo[3,2-b]pyridin-2-yl}pyridin-3-yl)oxy]ethyl}ethenesulfonamide
N-[2-({4-[3-(3,5-dichlorophenyl)-1H-pyrrolo[3,2-b]pyridin-2-yl]pyridin-3-yl}oxy)ethyl]-N-methylethenesulfonamide
1-[(2S)-2-{[(4-{3-[2-fluoro-5-(trifluoromethyl)phenyl]-1H-pyrrolo[3,2-b]pyridin-2-yl}pyridin-3-yl)oxy]methyl}pyrrolidin-1-yl]prop-2-en-1-one
1-{(2S)-2-[({4-[3-(2-fluoro-5-methylphenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(3-ethylphenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(quinolin-7-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(naphthalen-2-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(1-benzothiophen-6-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(1-benzofuran-6-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(3-methoxyphenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(1H-indol-6-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(2,3-dichlorophenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(2,3-difluorophenyl)-1H-pyrrolo[3,2-b]pyridin-2-yl]pyridin-3-yl}oxy)methyl]pyrrolidin-1-yl}prop-2-en-1-one
3-[2-(3-{[(2S)-1-(prop-2-enoyl)pyrrolidin-2-yl]methoxy}pyridin-4-yl)-1H-pyrrolo[3,2-b]pyridin-3-yl]benzonitrile
1-{(2S)-2-[({4-[3-(2-chlorophenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(2-fluorophenyl)-1H-pyrrolo[3,2-b]pyridin-2-yl]pyridin-3-yl}oxy)methyl]pyrrolidin-1-yl}prop-2-en-1-one
4-fluoro-3-[2-(3-{[(2S)-1-(prop-2-enoyl)pyrrolidin-2-yl]methoxy}pyridin-4-yl)-1H-pyrrolo[3,2-b]pyridin-3-yl]benzonitrile
2-fluoro-1-[(2S)-2-({[4-(3-phenyl-1H-pyrrolo[3,2-b]pyridin-2-yl)pyridin-3-yl]oxy}methyl)pyrrolidin-1-yl]prop-2-en-1-one
(2E)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one
1-[(2S)-2-({[4-(3-phenyl-1H-pyrrolo[3,2-b]pyridin-2-yl)pyridin-3-yl]oxy}methyl)pyrrolidin-1-yl]prop-2-yn-1-one
1-[(2S)-2-({[4-(3-phenyl-1H-pyrrolo[3,2-b]pyridin-2-yl)pyridin-3-yl]oxy}methyl)pyrrolidin-1-yl]but-2-yn-1-one
3-(1-methyl-1H-pyrazol-3-yl)-1-[(2S)-2-({[4-(3-phenyl-1H-pyrrolo[3,2-b]pyridin-2-yl)pyridin-3-yl]oxy}methyl)pyrrolidin-1-yl]prop-2-yn-1-one
(2E)-4-(dimethylamino)-1-[(2S)-2-{[(4-{3-[3-(trifluoromethyl)phenyl]-1H-pyrrolo[3,2-b]pyridin-2-yl}pyridin-3-yl)oxy]methyl}pyrrolidin-1-yl]but-2-en-1-one
(2E)-1-{(2S)-2-[({4-[3-(5-chloro-2-fluorophenyl)-1H-pyrrolo[3,2-b]pyridin-2-yl]pyridin-3-yl}oxy)methyl]pyrrolidin-1-yl}-4-(dimethylamino)but-2-en-1-one
(2Z)-4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one
(2E)-1-[(2S)-2-({[4-(3-phenyl-1H-pyrrolo[3,2-b]pyridin-2-yl)pyridin-3-yl]oxy}methyl)pyrrolidin-1-yl]-4-(pyrrolidin-1-yl)but-2-en-1-one
4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-yn-1-one
2-(3-{[(2S)-1-(ethenesulfonyl)pyrrolidin-2-yl]methoxy}pyridin-4-yl)-3-[2-fluoro-5-(trifluoromethyl)phenyl]-1H-pyrrolo[3,2-b]pyridine
1-{(2S)-2-[({4-[3-(3-ethynylphenyl)-1H-pyrrolo[3,2-b]pyridin-2-yl]pyridin-3-yl}oxy)methyl]pyrrolidin-1-yl}prop-2-en-1-one
1-[(2S)-2-{[(4-{3-[3-(difluoromethoxy)-4-fluorophenyl]-1H-pyrrolo[3,2-b]pyridin-2-yl}pyridin-3-yl)oxy]methyl}pyrrolidin-1-yl]prop-2-en-1-one
(2E)-1-[(2S)-2-{[(4-{3-[3-(difluoromethoxy)-4-fluorophenyl]-1H-pyrrolo[3,2-b]pyridin-2-yl}pyridin-3-yl)oxy]methyl}pyrrolidin-1-yl]-4-(dimethylamino)but-2-en-1-one
3-[3-(difluoromethoxy)-4-fluorophenyl]-2-(3-{[(2S)-1-(ethenesulfonyl)pyrrolidin-2-yl]methoxy}pyridin-4-yl)-1H-pyrrolo[3,2-b]pyridine
1-[(2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidin-1-yl]prop-2-en-1-one
(2E)-4-(dimethylamino)-1-[(2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidin-1-yl]but-2-en-1-one
2-(3-{[(2S)-1-(ethenesulfonyl)pyrrolidin-2-yl]methoxy}pyridin-4-yl)-3-[4-fluoro-3-(trifluoromethoxy)phenyl]-1H-pyrrolo[3,2-b]pyridine
1-[(2S)-2-({[5-fluoro-4-(3-phenyl-1H-pyrrolo[3,2-b]pyridin-2-yl)pyridin-3-yl]oxy}methyl)pyrrolidin-1-yl]prop-2-en-1-one
1-[(2S,4S)-4-methyl-2-({[4-(3-phenyl-1H-pyrrolo[3,2-b]pyridin-2-yl)pyridin-3-yl]oxy}methyl)pyrrolidin-1-yl]prop-2-en-1-one
2-(3-{[(2S,4S)-1-(ethenesulfonyl)-4-methylpyrrolidin-2-yl]methoxy}pyridin-4-yl)-3-phenyl-1H-pyrrolo[3,2-b]pyridine
1-[(2S)-4,4-difluoro-2-({[4-(3-phenyl-1H-pyrrolo[3,2-b]pyridin-2-yl)pyridin-3-yl]oxy}methyl)pyrrolidin-1-yl]prop-2-en-1-one
1-[(2S)-4-fluoro-2-({[4-(3-phenyl-1H-pyrrolo[3,2-b]pyridin-2-yl)pyridin-3-yl]oxy}methyl)-2,3-dihydro-1H-pyrrol-1-yl]prop-2-en-1-one
1-[(2S,4S)-4-fluoro-2-({[4-(3-phenyl-1H-pyrrolo[3,2-b]pyridin-2-yl)pyridin-3-yl]oxy}methyl)pyrrolidin-1-yl]prop-2-en-1-one
1-[(2S)-2-({[4-(3-phenyl-1H-pyrrolo[3,2-b]pyridin-2-yl)pyridin-3-yl]oxy}methyl)-2,5-dihydro-1H-pyrrol-1-yl]prop-2-en-1-one
1-[(3R)-3-{[4-(3-phenyl-1H-pyrrolo[3,2-b]pyridin-2-yl)pyridin-3-yl]oxy}pyrrolidin-1-yl]prop-2-en-1-one
1-[(3R)-3-{[4-(3-phenyl-1H-pyrrolo[3,2-b]pyridin-2-yl)pyridin-3-yl]oxy}pyrrolidin-1-yl]prop-2-yn-1-one
2-(3-{[(3R)-1-(ethenesulfonyl)pyrrolidin-3-yl]oxy}pyridin-4-yl)-3-phenyl-1H-pyrrolo[3,2-b]pyridine
1-[(3R)-3-({4-[3-(3-chlorophenyl)-1H-pyrrolo[3,2-b]pyridin-2-yl]pyridin-3-yl}oxy)pyrrolidin-1-yl]prop-2-en-1-one
1-[(3R)-3-({4-[3-(3-chlorophenyl)-1H-pyrrolo[3,2-b]pyridin-2-yl]pyridin-3-yl}oxy)pyrrolidin-1-yl]prop-2-yn-1-one
3-(3-chlorophenyl)-2-(3-{[(3R)-1-(ethenesulfonyl)pyrrolidin-3-yl]oxy}pyridin-4-yl)-1H-pyrrolo[3,2-b]pyridine
1-{(3R)-3-[(4-{3-[3-(trifluoromethyl)phenyl]-1H-pyrrolo[3,2-b]pyridin-2-yl}pyridin-3-yl)oxy]pyrrolidin-1-yl}prop-2-en-1-one
1-{(3R)-3-[(4-{3-[3- (trifluoromethyl) phenyl]-1H-pyrrolo[3,2-b]pyridin-2-yl}pyridin-3-yl)oxy]pyrrolidin-1-yl}prop-2-yn-1-one
1-[(3R)-3-({4-[3-(2-fluoro-5-methylphenyl)-1H-pyrrolo[3,2-b]pyridin-2-yl]pyridin-3-yl}oxy)pyrrolidin-1-yl]prop-2-en-1-one
1-[(3R)-3-({4-[3-(2-fluoro-5-methylphenyl)-1H-pyrrolo[3,2-b]pyridin-2-yl]pyridin-3-yl}oxy)pyrrolidin-1-yl]prop-2-yn-1-one
2-(3-{[(3R)-1-(ethenesulfonyl)pyrrolidin-3-yl]oxy}pyridin-4-yl)-3-(2-fluoro-5-methylphenyl)-1H-pyrrolo[3,2-b]pyridine
1-[(3R)-3-({4-[3-(5-chloro-2-fluorophenyl)-1H-pyrrolo[3,2-b]pyridin-2-yl]pyridin-3-yl}oxy)pyrrolidin-1-yl]prop-2-en-1-one
1-[(3R)-3-({4-[3-(5-chloro-2-fluorophenyl)-1H-pyrrolo[3,2-b]pyridin-2-yl]pyridin-3-yl}oxy)pyrrolidin-1-yl]prop-2-yn-1-one
1-{(2S)-2-[({4-[3-(5-chloro-2-fluorophenyl)-1H-pyrrolo[3,2-b]pyridin-2-yl]-5-fluoropyridin-3-yl}oxy)methyl]pyrrolidin-1-yl}prop-2-en-1-one
1-[(2S)-2-({[4-(3-phenyl-1H-pyrrolo[3,2-b]pyridin-2-yl)pyridin-3-yl]oxy}methyl)piperidin-1-yl]prop-2-en-1-one
1-[(2S,4RS)-4-{[2-(dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-one
1-[(2S,4RS)-4-{[2- (dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-one
N-methyl-N-[(2S)-2-{[4-(3-phenyl-1H-pyrrolo[3,2-b]pyridin-2-yl)pyridin-3-yl]oxy}propyl]prop-2-enamide
N-methyl-N-[(2S)-2-{[4-(3-phenyl-1H-pyrrolo[3,2-b]pyridin-2-yl)pyridin-3-yl]oxy}propyl]ethenesulfonamide
1-[(2S)-2-({[4-(7-methyl-3-phenyl-1H-pyrrolo[3,2-b]pyridin-2-yl)pyridin-3-yl]oxy}methyl)pyrrolidin-1-yl]prop-2-en-1-one
2-(3-{[(2S)-1-(ethenesulfonyl)pyrrolidin-2-yl]methoxy}pyridin-4-yl)-7-methyl-3-phenyl-1H-pyrrolo[3,2-b]pyridine
1-{(2S)-2-[({4-[3-(5-chloro-2-fluorophenyl)-7-methyl-1H-pyrrolo[3,2-b]pyridin-2-yl]pyridin-3-yl}oxy)methyl]pyrrolidin-1-yl}prop-2-en-1-one
3-(5-chloro-2-fluorophenyl)-2-(3-{[(2S)-1-(ethenesulfonyl)pyrrolidin-2-yl]methoxy}pyridin-4-yl)-7-methyl-1H-pyrrolo[3,2-b]pyridine
1-[(2R*)-2-({[4-(3-phenyl-1H-pyrrolo[3,2-b]pyridin-2-yl)pyridin-3-yl]oxy}methyl)azetidin-1-yl]prop-2-en-1-one
1-[(2R*)-2-({[4-(3-phenyl-1H-pyrrolo[3,2-b]pyridin-2-yl)pyridin-3-yl]oxy}methyl)azetidin-1-yl]prop-2-en-1-one
2-(3-{[(2R*)-1-(ethenesulfonyl)azetidin-2-yl]methoxy}pyridin-4-yl)-3-phenyl-1H-pyrrolo[3,2-b]pyridine
2-(3-{[(2R*)-1-(ethenesulfonyl)azetidin-2-yl]methoxy}pyridin-4-yl)-3-phenyl-1H-pyrrolo[3,2-b]pyridine
1-[(2R*)-2-{[(4-{3-[3-(trifluoromethyl)phenyl]-1H-pyrrolo[3,2-b]pyridin-2-yl}pyridin-3-yl)oxy]methyl}azetidin-1-yl]prop-2-en-1-one
2-(3-{[(2R*)-1-(ethenesulfonyl)azetidin-2-yl]methoxy}pyridin-4-yl)-3-[3-(trifluoromethyl)phenyl]-1H-pyrrolo[3,2-b]pyridine
1-[(2S)-2-methyl-2-({[4-(3-phenyl-1H-pyrrolo[3,2-b]pyridin-2-yl)pyridin-3-yl]oxy}methyl)azetidin-1-yl]prop-2-en-1-one
2-(3-{[(2S)-1-(ethenesulfonyl)-2-methylazetidin-2-yl]methoxy}pyridin-4-yl)-3-phenyl-1H-pyrrolo[3,2-b]pyridine
1-[(2S)-2-methyl-2-{[(4-{3-[3-(trifluoromethyl)phenyl]-1H-pyrrolo[3,2-b]pyridin-2-yl}pyridin-3-yl)oxy]methyl}azetidin-1-yl]prop-2-en-1-one
2-(3-{[(2S)-1-(ethenesulfonyl)-2-methylazetidin-2-yl]methoxy}pyridin-4-yl)-3-[3-(trifluoromethyl)phenyl]-1H-pyrrolo[3,2-b]pyridine
1-[(3S)-3-({[4-(3-phenyl-1H-pyrrolo[3,2-b]pyridin-2-yl)pyridin-3-yl]oxy}methyl)morpholin-4-yl]prop-2-en-1-one
(2E)-4-(dimethylamino)-1-[(3S)-3-({[4-(3-phenyl-1H-pyrrolo[3,2-b]pyridin-2-yl)pyridin-3-yl]oxy}methyl) morpholin-4-yl]but-2-en-1-one
1-{(3S)-3-[({4-[3-(5-chloro-2-fluorophenyl)-1H-pyrrolo[3,2-b]pyridin-2-yl]pyridin-3-yl}oxy)methyl]morpholin-4-yl}prop-2-en-1-one, and
(2E)-1-{(3S)-3-[({4-[3-(5-chloro-2-fluorophenyl)-1H-pyrrolo[3,2-b]pyridin-2-yl]pyridin-3-yl}oxy)methyl]morpholin-4-yl}-4-(dimethylamino)but-2-en-1-one,
or an N-oxide, a salt, a tautomer, a rotamer, or a stereoisomer of said compound, or a salt of said N-oxide, tautomer, rotamer, or stereoisomer.

5. A compound of formula (I) according to any of claims 1 to 4 for use in the treatment or prophylaxis of diseases.

6. The compound for use according to claim 5, wherein the diseases are hyperproliferative diseases and/or disorders responsive to induction of cell death.

7. The compound for use according to claim 6, wherein the hyperproliferative diseases and/or disorders responsive to induction of cell death are haematological tumours, solid tumours and/or metastases thereof.

8. The compound for use according to claim 7, wherein the tumour harbors a mutant EGFR and/or metastases thereof.

9. The compound for use according to claim 7, wherein the tumour is lung cancer, particularly lung cancer harboring a mutant EGFR with exon 20 insertion mutation, and/or metastases thereof.

10. The compound for use according to claim 7, wherein the tumour is lung cancer, particularly lung cancer harboring a mutant EGFR with in-frame deletions in exon 19 (such as EGFR E746_A750del) or point mutations in exon 21 (e.g. L858R), and/or metastases thereof.

11. The compound for use according to claim 7, wherein the tumour is lung cancer, particularly lung cancer harboring a mutant EGFR with an exon 20 insertion and a T790M mutation, e.g. a D770_N771insSVD T790M mutation, and/or metastases thereof.

12. The compound for use according to claim 7, wherein the tumour is lung cancer, particularly lung cancer harboring a mutant EGFR with inframe deletion in exon 19 such as E746_A750del and a T790M mutation, and/or metastases thereof.

13. The compound for use according to claim 7, wherein the tumour is lung cancer, particularly lung cancer harboring a mutant EGFR with a point mutation in exon 21 such as L858R and a T790M mutation, and/or metastases thereof.

14. The compound for use according to claim 7, wherein the tumour is lung cancer, particularly lung cancer harboring a mutant ERBB2 with exon 20 insertion mutations (such as ERBB2 A775_G776insYVMA), and/or metastases thereof.

15. A pharmaceutical composition comprising at least one compound of formula (I) according to any of claims 1 to 4, together with at least one pharmaceutically acceptable auxiliary.

16. The composition according to claim 15 for use in the treatment of haematological tumours, solid tumours and/or metastases thereof.

17. A combination comprising one or more first active ingredients selected from a compound of formula (I) according to any of claims 1 to 4, and one or more second active ingredients selected from chemotherapeutic anti-cancer agents and target-specific anti-cancer agents.

## Patentansprüche

1. Eine Verbindung der Formel (I) 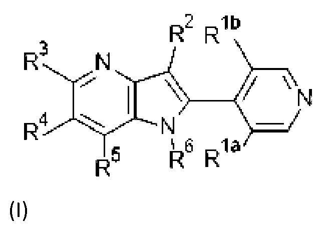 in der:
R^{1a} eine Gruppe ausgewählt aus der Gruppe darstellt: -O-(C₂-C₆-Alkandiyl)-NR⁷R⁸, -O-CH₂-(C₁-C₅-Haloalkandiyl)-NR⁷R⁸, -O-(C₁-C₅-Alkandiyl)-R⁹ oder -O-R⁹;
R^{1b} ein Wasserstoffatom oder Fluor darstellt;
R² Phenyl oder Heteroaryl darstellt, wobei diese Gruppen ein- oder mehrmals unabhängig voneinander mit R¹⁰ substituiert sind;
R³ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R⁴ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R⁵ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R⁶ ein Wasserstoffatom oder Methyl darstellt;
R⁷ -(CO)-R¹¹, -(CO)-C≡C-R¹², -(SO₂)-CH=CH₂ oder Oxiran-2-carbonyl darstellt;
R⁸ ein Wasserstoffatom oder C₁-C₃-Alkyl darstellt;
R⁹ eine Gruppe ausgewählt aus der Gruppe darstellt: 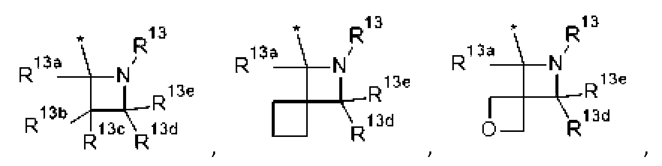 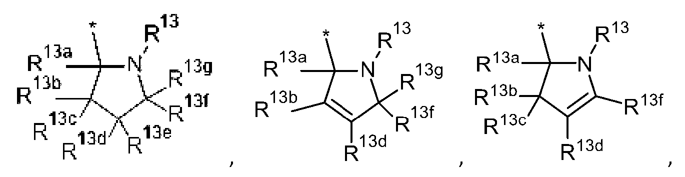 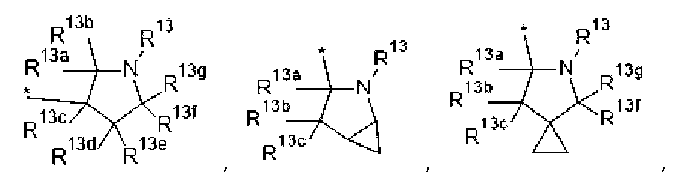 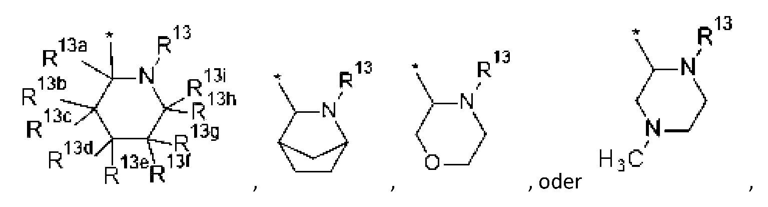 wobei * den Bindungspunkt dieser Gruppe mit dem Rest des Moleküls angibt;
R¹⁰ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R¹¹ -CH₂-C≡CH oder -C(R¹⁴)-R¹⁵ darstellt;
R¹² ein Wasserstoffatom, Methyl, -CH₂₋N(CH₃)-CHR¹⁶R¹⁷ oder 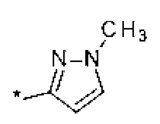 darstellt;
R¹³ -(CO)-R¹¹, -(CO)-C≡C-R¹², -(SO₂)-CH=CH₂ oder Oxiran-2-carbonyl darstellt;
R^{13a} ein Wasserstoffatom, Methyl oder Fluor darstellt;
R^{13b} ein Wasserstoffatom, Methyl oder Fluor darstellt;
R^{13c} ein Wasserstoffatom, Methyl oder Fluor darstellt;
R^{13d} ein Wasserstoffatom, Methyl, Fluor oder -N(CH₃)-CH₂-CH₂-N(CH₃)-CHR¹⁶R¹⁷ darstellt;
R^{13e} ein Wasserstoffatom, Methyl oder Fluor darstellt;
R^{13f} ein Wasserstoffatom, Methyl oder Fluor darstellt;
R^{13g} ein Wasserstoffatom, Methyl oder Fluor darstellt;
R^{13h} ein Wasserstoffatom, Methyl oder Fluor darstellt;
R¹³ⁱ ein Wasserstoffatom, Methyl oder Fluor darstellt;
R¹⁴ =CH₂, =CH-CH₃, =CH-CH₂₋N(CH₃)-CHR¹⁶R¹⁷ oder =CH-CH₂₋R¹⁸ darstellt;
R¹⁵ ein Wasserstoffatom, C₁-C₃-Alkyl oder Fluor darstellt;
R¹⁶ ein Wasserstoffatom oder Methyl darstellt;
R¹⁷ ein Wasserstoffatom oder Methyl darstellt;
R¹⁸ eine Gruppe ausgewählt aus der Gruppe darstellt: 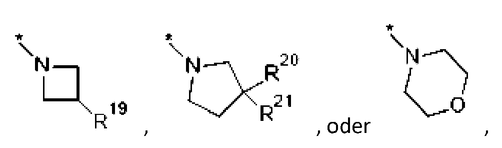 wobei * den Bindungspunkt dieser Gruppe mit dem Rest des Moleküls angibt;
R¹⁹ ein Wasserstoffatom, Methyl, Methoxy oder Fluor darstellt;
R²⁰ ein Wasserstoffatom, Methyl, Methoxy oder Fluor darstellt;
R²¹ ein Wasserstoffatom, Methyl, Methoxy oder Fluor darstellt;
oder ein N-Oxid, ein Salz, ein Tautomer, ein Rotamer oder ein Stereoisomer dieser Verbindung oder ein Salz dieses N-Oxids, Tautomers, Rotamers oder Stereoisomers.

2. Die Verbindung der Formel (I) gemäß Anspruch 1, wobei:
R^{1a} eine Gruppe ausgewählt aus der Gruppe darstellt: -O-(C₂-C₆-Alkandiyl)-NR⁷R⁸, -O-CH₂-(C₁-C₅-Haloalkandiyl)-NR⁷R⁸, -O-(C₁-C₅-Alkandiyl)-R⁹ oder -O-R⁹;
R^{1b} ein Wasserstoffatom oder Fluor darstellt;
R² eine Gruppe ausgewählt aus der Gruppe darstellt: 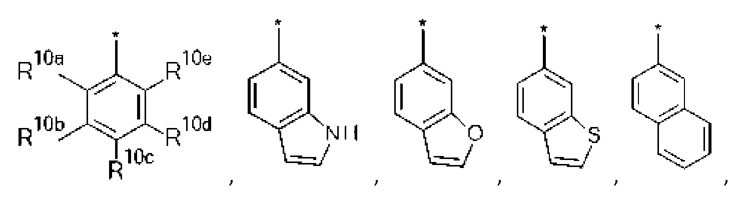 oder 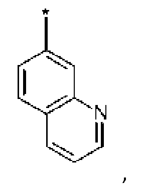
wobei * den Bindungspunkt dieser Gruppe mit dem Rest des Moleküls angibt;
R³ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R⁴ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃- Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R⁵ ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R⁶ ein Wasserstoffatom oder Methyl darstellt;
R⁷ -(CO)-R¹¹, -(CO)-C≡C-R¹², -(SO₂)-CH=CH₂ oder Oxiran-2-carbonyl darstellt;
R⁸ ein Wasserstoffatom oder C₁-C₃-Alkyl darstellt;
R⁹ eine Gruppe ausgewählt aus der Gruppe darstellt: 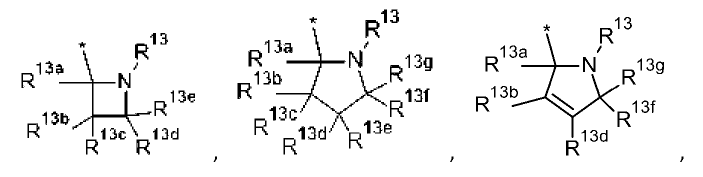 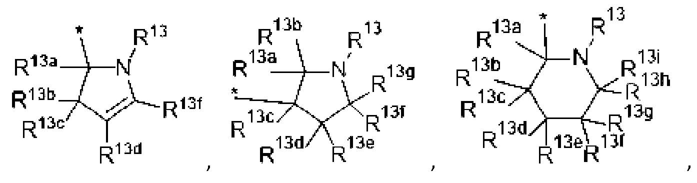 oder 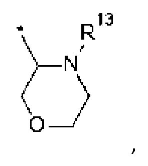
wobei * den Bindungspunkt dieser Gruppe mit dem Rest des Moleküls angibt;
R^{10a} ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R^{10b} ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R^{10c} ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R^{10d} ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R^{10e} ein Wasserstoffatom, Amino, C₁-C₃-Alkyl, C₂-C₃-Alkenyl, C₂-C₃-Alkinyl, C₁-C₃-Haloalkyl, C₁-C₃-Hydroxyalkyl, C₁-C₃-Alkoxy, C₁-C₃-Haloalkoxy, C₁-C₃-Alkoxy-C₁-C₃-Alkyl, Amino-C₁-C₃-Alkyl, C₁-C₃-Alkylamino-C₁-C₃-Alkyl, (C₁-C₃-Alkyl)₂amino-C₁-C₃-Alkyl, C₁-C₃-Alkoxycarbonyl, Aminocarbonyl, C₁-C₃-Alkylaminocarbonyl, (C₁-C₃-Alkyl)₂₋aminocarbonyl, Cyano, Fluor, Chlor oder Brom darstellt;
R¹¹ -CH₂-C≡CH oder -C(R¹⁴)-R¹⁵ darstellt;
R¹² ein Wasserstoffatom, Methyl, -CH₂₋N(CH₃)-CHR¹⁶R¹⁷ oder 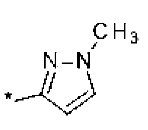 darstellt;
R¹³ -(CO)-R¹¹, -(CO)-C≡C-R¹², -(SO₂)-CH=CH₂ oder Oxiran-2-carbonyl darstellt;
R^{13a} ein Wasserstoffatom, Methyl oder Fluor darstellt;
R^{13b} ein Wasserstoffatom, Methyl oder Fluor darstellt;
R^{13c} ein Wasserstoffatom, Methyl oder Fluor darstellt;
R^{13d} ein Wasserstoffatom, Methyl, Fluor oder -N(CH₃)-CH₂-CH₂-N(CH₃)-CHR¹⁶R¹⁷ darstellt;
R^{13e} ein Wasserstoffatom, Methyl oder Fluor darstellt;
R^{13f} ein Wasserstoffatom, Methyl oder Fluor darstellt;
R^{13g} ein Wasserstoffatom, Methyl oder Fluor darstellt;
R^{13h} ein Wasserstoffatom, Methyl oder Fluor darstellt;
R¹³ⁱ ein Wasserstoffatom, Methyl oder Fluor darstellt;
R¹⁴ =CH₂, =CH-CH₃, =CH-CH₂₋N(CH₃)-CHR¹⁶R¹⁷ oder =CH-CH₂₋R¹⁸ darstellt;
R¹⁵ ein Wasserstoffatom, C₁-C₃-Alkyl oder Fluor darstellt;
R¹⁶ ein Wasserstoffatom oder Methyl darstellt;
R¹⁷ ein Wasserstoffatom oder Methyl darstellt;
R¹⁸ eine Gruppe ausgewählt aus der Gruppe darstellt: 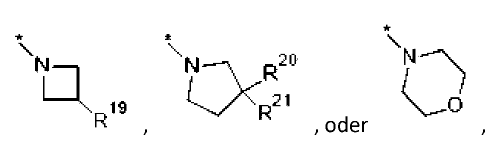
wobei * den Bindungspunkt dieser Gruppe mit dem Rest des Moleküls angibt;
R¹⁹ ein Wasserstoffatom, Methyl, Methoxy oder Fluor darstellt;
R²⁰ ein Wasserstoffatom, Methyl, Methoxy oder Fluor darstellt;
R²¹ ein Wasserstoffatom, Methyl, Methoxy oder Fluor darstellt;
oder ein N-Oxid, ein Salz, ein Tautomer, ein Rotamer oder ein Stereoisomer dieser Verbindung oder ein Salz dieses N-Oxids, Tautomers, Rotamers oder Stereoisomers.

3. Die Verbindung der Formel (I) gemäß einem der Ansprüche 1 oder 2, wobei:
R^{1a} eine Gruppe ausgewählt aus der Gruppe darstellt: -O-(C₂-C₄-Alkandiyl)-NR⁷R⁸, -O-CH₂-R⁹ oder -O-R⁹;
R^{1b} ein Wasserstoffatom oder Fluor darstellt;
R² eine Gruppe ausgewählt aus der Gruppe darstellt: 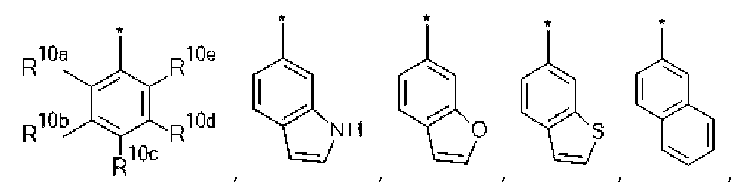 oder 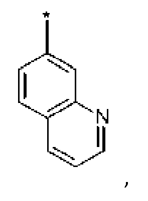
wobei * den Bindungspunkt dieser Gruppe mit dem Rest des Moleküls angibt;
R³ ein Wasserstoffatom oder Methoxy darstellt;
R⁴ ein Wasserstoffatom oder Fluor darstellt;
R⁵ ein Wasserstoffatom oder Methyl darstellt;
R⁶ ein Wasserstoffatom darstellt;
R⁷ -(CO)-R¹¹, -(CO)-C≡C-R¹² oder -(SO₂)-CH=CH₂ darstellt;
R⁸ ein Wasserstoffatom, Methyl oder Ethyl darstellt;
R⁹ eine Gruppe ausgewählt aus der Gruppe darstellt: 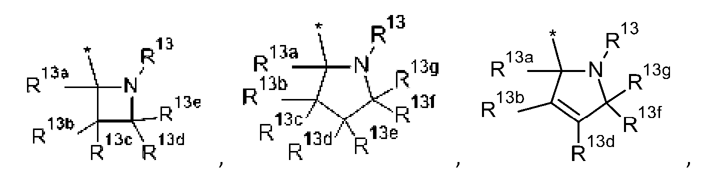 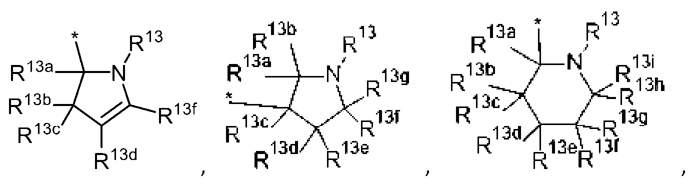 oder 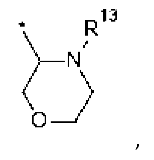
wobei * den Bindungspunkt dieser Gruppe mit dem Rest des Moleküls angibt;
R^{10a} ein Wasserstoffatom, Ethyl, Methoxy oder Fluor darstellt;
R^{10b} ein Wasserstoffatom, Methyl, Ethyl, Iso-propyl, Ethinyl, Trifluormethyl, Methoxy, Difluormethoxy, Trifluormethoxy, Methoxycarbonyl, Cyano, Fluor oder Chlor darstellt;
R^{10c} ein Wasserstoffatom, Fluor oder Chlor darstellt;
R^{10d} ein Wasserstoffatom, Methyl, Ethyl, Fluor oder Chlor darstellt;
R^{10e} ein Wasserstoffatom, Fluor oder Chlor darstellt;
R¹¹ -C(R¹⁴)-R¹⁵ darstellt;
R¹² ein Wasserstoffatom oder Methyl darstellt;
R¹³ -(CO)-R¹¹, -(CO)-C≡C-R¹² oder -(SO₂)-CH=CH₂ darstellt;
R^{13a} ein Wasserstoffatom oder Methyl darstellt;
R^{13b} ein Wasserstoffatom darstellt;
R^{13c} ein Wasserstoffatom darstellt;
R^{13d} ein Wasserstoffatom, Methyl, Fluor oder -N(CH₃)-CH₂-CH₂-N(CH₃)-CHR¹⁶R¹⁷ darstellt;
R^{13e} ein Wasserstoffatom oder Fluor darstellt;
R^{13f} ein Wasserstoffatom darstellt;
R^{13g} ein Wasserstoffatom darstellt;
R^{13h} ein Wasserstoffatom darstellt;
R¹³ⁱ ein Wasserstoffatom darstellt;
R¹⁴ =CH₂ oder =CH-CH₂₋N(CH₃)-CHR¹⁶R¹⁷ darstellt;
R¹⁵ ein Wasserstoffatom darstellt;
R¹⁶ ein Wasserstoffatom darstellt;
R¹⁷ ein Wasserstoffatom darstellt;
oder ein N-Oxid, ein Salz, ein Tautomer, ein Rotamer oder ein Stereoisomer dieser Verbindung oder ein Salz dieses N-Oxids, Tautomers, Rotamers oder Stereoisomers.

4. Die Verbindung der Formel (I) gemäß einem der Ansprüche 1 bis 3, die ausgewählt ist aus der Gruppe bestehend aus:
N-[2-({4-[3-(4-fluorophenyl)-1H-pyrrolo[3,2-b]pyridin-2-yl]pyridin-3-yl}oxy)ethyl]prop-2-enamid,
N-(2-{[4-(3-phenyl-1H-pyrrolo[3,2-b]pyridin-2-yl)pyridin-3-yl]oxy}ethyl)prop-2-enamid,
N-methyl-N-(3-{[4-(3-phenyl-1H-pyrrolo[3,2-b]pyridin-2-yl)pyridin-3-yl]oxy}propyl)prop-2-enamid,
N-methyl-N-(2-{[4-(3-phenyl-1H-pyrrolo[3,2-b]pyridin-2-yl)pyridin-3-yl]oxy}ethyl)prop-2-enamid,
N-(3-{[4-(3-phenyl-1H-pyrrolo[3,2-b]pyridin-2-yl)pyridin-3-yl]oxy}propyl)prop-2-enamid,
N-(2-{[4-(6-fluoro-3-phenyl-1H-pyrrolo[3,2-b]pyridin-2-yl)pyridin-3-yl]oxy}ethyl)prop-2-enamid,
N-(2-{[4-(6-fluoro-3-phenyl-1H-pyrrolo[3,2-b]pyridin-2-yl)pyridin-3-yl]oxy}ethyl)-N-methylprop-2-enamid,
N-(2-{[4-(5-methoxy-3-phenyl-1H-pyrrolo[3,2-b]pyridin-2-yl)pyridin-3-yl]oxy}ethyl)-N-methylprop-2-enamid,
N-methyl-N-[2-({4-[3-(3-methylphenyl)-1H-pyrrolo[3,2-b]pyridin-2-yl]pyridin-3-yl}oxy)ethyl]prop-2-enamid,
N-[2-({4-[3-(4-chlor-3-methylphenyl)-1H-pyrrolo[3,2-b]pyridin-2-yl]pyridin-3-yl}oxy)ethyl]-N-methylprop-2-enamid,
N-[2-({4-[3-(4-chlorophenyl)-1H-pyrrolo[3,2-b]pyridin-2-yl]pyridin-3-yl}oxy)ethyl]-N-methylprop-2-enamid,
N-[2-({4-[3-(4-fluor-3-methylphenyl)-1H-pyrrolo[3,2-b]pyridin-2-yl]pyridin-3-yl}oxy)ethyl]-N-methylprop-2-enamid,
Methyl-2-chlor-5-[2-(3-{2-[methyl(prop-2-enoyl)amino]ethoxy}pyridin-4-yl)-1H-pyrrolo[3,2-b]pyridin-3-yl]benzoat,
N-[2-({4-[3-(3-fluorophenyl)-1H-pyrrolo[3,2-b]pyridin-2-yl]pyridin-3-yl}oxy)ethyl]-N-methylprop-2-enamid,
N-[2-({4-[3-(4-chlor-3-methoxyphenyl)-1H-pyrrolo[3,2-b]pyridin-2-yl]pyridin-3-yl}oxy)ethyl]-N-methylprop-2-enamid,
N-[2-({4-[3-(1H-indol-6-yl)-1H-pyrrolo[3,2-b]pyridin-2-yl]pyridin-3-yl}oxy)ethyl]-N-methylprop-2-enamid,
N-[2-({4-[3-(2-methoxyphenyl)-1H-pyrrolo[3,2-b]pyridin-2-yl]pyridin-3-yl}oxy)ethyl]-N-methylprop-2-enamid,
N-methyl-N-[2-({4-[3-(quinolin-7-yl)-1H-pyrrolo[3,2-b]pyridin-2-yl]pyridin-3-yl}oxy)ethyl]prop-2-enamid,
N-[2-({4-[3-(3-fluor-2-methoxyphenyl)-1H-pyrrolo[3,2-b]pyridin-2-yl]pyridin-3-yl}oxy)ethyl]-N-methylprop-2-enamid,
N-[2-({4-[3-(4-chlor-3-ethylphenyl)-1H-pyrrolo[3,2-b]pyridin-2-yl]pyridin-3-yl}oxy)ethyl]-N-methylprop-2-enamid,
N-[2-({4-[3-(3-chlor-2-methoxyphenyl)-1H-pyrrolo[3,2-b]pyridin-2-yl]pyridin-3-yl}oxy)ethyl]-N-methylprop-2-enamid,
N-[2-({4-[3-(3-chlorophenyl)-1H-pyrrolo[3,2-b]pyridin-2-yl]pyridin-3-yl}oxy)ethyl]-N-methylprop-2-enamid,
N-[2-({4-[3-(3,4-dichlorophenyl)-1H-pyrrolo[3,2-b]pyridin-2-yl]pyridin-3-yl}oxy)ethyl]-N-methylprop-2-enamid,
N-[2-({4-[3-(3-chlor-4-fluorophenyl)-1H-pyrrolo[3,2-b]pyridin-2-yl]pyridin-3-yl}oxy)ethyl]-N-methylprop-2-enamid,
N-methyl-N-(2-{[4-(3-phenyl-1H-pyrrolo[3,2-b]pyridin-2-yl)pyridin-3-yl]oxy}ethyl)Ethensulfonamid,
(2E)-4-(Dimethylamino)-N-methyl-N-(2-{[4-(3-phenyl-1H-pyrrolo[3,2-b]pyridin-2-yl)pyridin-3-yl]oxy}ethyl)but-2-enamid,
N-methyl-N-(2-{[4-(3-phenyl-1H-pyrrolo[3,2-b]pyridin-2-yl)pyridin-3-yl]oxy}ethyl)prop-2-ynamid,
N-methyl-N-(2-{[4-(3-phenyl-1H-pyrrolo[3,2-b]pyridin-2-yl)pyridin-3-yl]oxy}ethyl)but-2-ynamid,
N-[2-({4-[3-(3-chlorophenyl)-1H-pyrrolo[3,2-b]pyridin-2-yl]pyridin-3-yl}oxy)ethyl]-N-methylEthensulfonamid,
N-[2-({4-[3-(1H-indol-6-yl)-1H-pyrrolo[3,2-b]pyridin-2-yl]pyridin-3-yl}oxy)ethyl]-N-methylEthensulfonamid,
N-[2-({4-[3-(3,5-dichlorophenyl)-1H-pyrrolo[3,2-b]pyridin-2-yl]pyridin-3-yl}oxy)ethyl]-N-methylprop-2-enamid,
N-ethyl-N-(2-{[4-(3-phenyl-1H-pyrrolo[3,2-b]pyridin-2-yl)pyridin-3-yl]oxy}ethyl)prop-2-enamid,
N-ethyl-N-(2-{[4-(3-phenyl-1H-pyrrolo[3,2-b]pyridin-2-yl)pyridin-3-yl]oxy}ethyl)Ethensulfonamid,
1-[(2S)-2-({[4-(3-phenyl-1H-pyrrolo[3,2-b]pyridin-2-yl)pyridin-3-yl]oxy}methyl)pyrrolidin-1-yl]prop-2-en-1-on,
1-[(2R)-2-({[4-(3-phenyl-1H-pyrrolo[3,2-b]pyridin-2-yl)pyridin-3-yl]oxy}methyl)pyrrolidin-1-yl]prop-2-en-1-on,
2-(3-{[(2S)-1-(Ethensulfonyl)pyrrolidin-2-yl]methoxy}pyridin-4-yl)-3-phenyl-1H-pyrrolo[3,2-b]pyridin,
(2E)-4-(Dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-on,
2-(3-{[(2S)-1-(Ethensulfonyl)pyrrolidin-2-yl]methoxy}pyridin-4-yl)-3-[3-(Trifluormethyl)phenyl]-1H-pyrrolo[3,2-b]pyridin,
1-[(2S)-2-{[(4-{3-[3-(Trifluormethyl)phenyl]-1H-pyrrolo[3,2-b]pyridin-2-yl}pyridin-3-yl)oxy]methyl}pyrrolidin-1-yl]prop-2-en-1-on,
3-(3-chlorophenyl)-2-(3-{[(2S)-1-(Ethensulfonyl)pyrrolidin-2-yl]methoxy}pyridin-4-yl)-1H-pyrrolo[3,2-b]pyridin,
1-{(2S)-2-[({4-[3-(3-chlorophenyl)-1H-pyrrolo[3,2-b]pyridin-2-yl]pyridin-3-yl}oxy)methyl]pyrrolidin-1-yl}prop-2-en-1-on,
1-{(2S)-2-[({4-[3-(5-chlor-2-fluorophenyl)-1H-pyrrolo[3,2-b]pyridin-2-yl]pyridin-3-yl}oxy)methyl]pyrrolidin-1-yl}prop-2-en-1-on,
N-[2-({4-[3-(5-chlor-2-fluorophenyl)-1H-pyrrolo[3,2-b]pyridin-2-yl]pyridin-3-yl}oxy)ethyl]-N-methylprop-2-enamid
N-[2-({4-[3-(1-Benzothiophen-6-yl)-1H-pyrrolo[3,2-b]pyridin-2-yl]pyridin-3-yl}oxy)ethyl]-N-methylprop-2-enamid
N-[2-({4-[3-(3-ethylphenyl)-1H-pyrrolo[3,2-b]pyridin-2-yl]pyridin-3-yl}oxy)ethyl]-N-methylprop-2-enamid
N-[2-({4-[3-(2,5-difluorophenyl)-1H-pyrrolo[3,2-b]pyridin-2-yl]pyridin-3-yl}oxy)ethyl]-N-methylprop-2-enamid
N-[2-({4-[3-(2-fluor-5-methylphenyl)-1H-pyrrolo[3,2-b]pyridin-2-yl]pyridin-3-yl}oxy)ethyl]-N-methylprop-2-enamid
N-[2-({4-[3-(5-ethyl-2-fluorophenyl)-1H-pyrrolo[3,2-b]pyridin-2-yl]pyridin-3-yl}oxy)ethyl]-N-methylprop-2-enamid
N-methyl-N-{2-[(4-{3-[3-(Trifluormethyl)phenyl]-1H-pyrrolo[3,2-b]pyridin-2-yl}pyridin-3-yl)oxy]ethyl}prop-2-enamid
N-[2-({4-[3-(3-fluor-5-methylphenyl)-1H-pyrrolo[3,2-b]pyridin-2-yl]pyridin-3-yl}oxy)ethyl]-N-methylprop-2-enamid
N-{2-[(4-{3-[2-fluor-5-(Trifluormethyl)phenyl]-1H-pyrrolo[3,2-b]pyridin-2-yl}pyridin-3-yl)oxy]ethyl}-N-methylprop-2-enamid
N-[2-({4-[3-(3,5-difluorophenyl)-1H-pyrrolo[3,2-b]pyridin-2-yl]pyridin-3-yl}oxy)ethyl]-N-methylprop-2-enamid
N-[2-({4-[3-(2-ethylphenyl)-1H-pyrrolo[3,2-b]pyridin-2-yl]pyridin-3-yl}oxy)ethyl]-N-methylprop-2-enamid
N-[2-({4-[3-(3-methoxyphenyl)-1H-pyrrolo[3,2-b]pyridin-2-yl]pyridin-3-yl}oxy)ethyl]-N-methylprop-2-enamid
N-methyl-N-{2-[(4-{3-[3-(propan-2-yl)phenyl]-1H-pyrrolo[3,2-b]pyridin-2-yl}pyridin-3-yl)oxy]ethyl}prop-2-enamid
N-[2-({4-[3-(2-fluor-5-methoxyphenyl)-1H-pyrrolo[3,2-b]pyridin-2-yl]pyridin-3-yl}oxy)ethyl]-N-methylprop-2-enamid
N-[2-({4-[3-(2,5-dichlorophenyl)-1H-pyrrolo[3,2-b]pyridin-2-yl]pyridin-3-yl}oxy)ethyl]-N-methylprop-2-enamid
N-methyl-N-{2-[(4-{3-[3-(Trifluormethoxy)phenyl]-1H-pyrrolo[3,2-b]pyridin-2-yl}pyridin-3-yl)oxy]ethyl}prop-2-enamid
N-{2-[(4-{3-[2-fluor-5-(Trifluormethyl)phenyl]-1H-pyrrolo[3,2-b]pyridin-2-yl}pyridin-3-yl)oxy]ethyl}-N-methylEthensulfonamid
N-[2-({4-[3-(5-chlor-2-fluorophenyl)-1H-pyrrolo[3,2-b]pyridin-2-yl]pyridin-3-yl}oxy)ethyl]-N-methylEthensulfonamid
N-[2-({4-[3-(2,5-difluorophenyl)-1H-pyrrolo[3,2-b]pyridin-2-yl]pyridin-3-yl}oxy)ethyl]-N-methylEthensulfonamid
N-[2-({4-[3-(1-Benzothiophen-6-yl)-1H-pyrrolo[3,2-b]pyridin-2-yl]pyridin-3-yl}oxy)ethyl]-N-methylEthensulfonamid
N-[2-({4-[3-(3-ethylphenyl)-1H-pyrrolo[3,2-b]pyridin-2-yl]pyridin-3-yl}oxy)ethyl]-N-methylEthensulfonamid
N-[2-({4-[3-(2-fluor-5-methylphenyl)-1H-pyrrolo[3,2-b]pyridin-2-yl]pyridin-3-yl}oxy)ethyl]-N-methylEthensulfonamid
N-[2-({4-[3-(3-fluor-5-methylphenyl)-1H-pyrrolo[3,2-b]pyridin-2-yl]pyridin-3-yl}oxy)ethyl]-N-methylEthensulfonamid
N-methyl-N-{2-[(4-{3-[3-(Trifluormethyl)phenyl]-1H-pyrrolo[3,2-b]pyridin-2-yl}pyridin-3-yl)oxy]ethyl}Ethensulfonamid
N-[2-({4-[3-(5-ethyl-2-fluorophenyl)-1H-pyrrolo[3,2-b]pyridin-2-yl]pyridin-3-yl}oxy)ethyl]-N-methylEthensulfonamid
N-[2-({4-[3-(3,5-difluorophenyl)-1H-pyrrolo[3,2-b]pyridin-2-yl]pyridin-3-yl}oxy)ethyl]-N-methylEthensulfonamid
N-[2-({4-[3-(3-methoxyphenyl)-1H-pyrrolo[3,2-b]pyridin-2-yl]pyridin-3-yl}oxy)ethyl]-N-methylEthensulfonamid
N-methyl-N-{2-[(4-{3-[3-(propan-2-yl)phenyl]-1H-pyrrolo[3,2-b]pyridin-2-yl}pyridin-3-yl)oxy]ethyl}Ethensulfonamid
N-[2-({4-[3-(2,5-dichlorophenyl)-1H-pyrrolo[3,2-b]pyridin-2-yl]pyridin-3-yl}oxy)ethyl]-N-methylEthensulfonamid
N-[2-({4-[3-(2-ethylphenyl)-1H-pyrrolo[3,2-b]pyridin-2-yl]pyridin-3-yl}oxy)ethyl]-N-methylEthensulfonamid
N-[2-({4-[3-(2-fluor-5-methoxyphenyl)-1H-pyrrolo[3,2-b]pyridin-2-yl]pyridin-3-yl}oxy)ethyl]-N-methylEthensulfonamid
N-methyl-N-{2-[(4-{3-[3-(Trifluormethoxy)phenyl]-1H-pyrrolo[3,2-b]pyridin-2-yl}pyridin-3-yl)oxy]ethyl}Ethensulfonamid
N-[2-({4-[3-(3,5-dichlorophenyl)-1H-pyrrolo[3,2-b]pyridin-2-yl]pyridin-3-yl}oxy)ethyl]-N-methylEthensulfonamid
1-[(2S)-2-{[(4-{3-[2-fluor-5-(Trifluormethyl)phenyl]-1H-pyrrolo[3,2-b]pyridin-2-yl}pyridin-3-yl)oxy]methyl}pyrrolidin-1-yl]prop-2-en-1-on
1-{(2S)-2-[({4-[3-(2-fluor-5-methylphenyl)-1H-pyrrolo[3,2-b]pyridin-2-yl]pyridin-3-yl}oxy)methyl]pyrrolidin-1-yl}prop-2-en-1-on
1-{(2S)-2-[({4-[3-(3-ethylphenyl)-1H-pyrrolo[3,2-b]pyridin-2-yl]pyridin-3-yl}oxy)methyl]pyrrolidin-1-yl}prop-2-en-1-on
1-{(2S)-2-[({4-[3-(quinolin-7-yl)-1H-pyrrolo[3,2-b]pyridin-2-yl]pyridin-3-yl}oxy)methyl]pyrrolidin-1-yl}prop-2-en-1-on
1-{(2S)-2-[({4-[3-(naphthalen-2-yl)-1H-pyrrolo[3,2-b]pyridin-2-yl]pyridin-3-yl}oxy)methyl]pyrrolidin-1-yl}prop-2-en-1-on
1-{(2S)-2-[({4-[3-(1-Benzothiophen-6-yl)-1H-pyrrolo[3,2-b]pyridin-2-yl]pyridin-3-yl}oxy)methyl]pyrrolidin-1-yl}prop-2-en-1-on
1-{(2S)-2-[({4-[3-(1-Benzofuran-6-yl)-1H-pyrrolo[3,2-b]pyridin-2-yl]pyridin-3-yl}oxy)methyl]pyrrolidin-1-yl}prop-2-en-1-on
1-{(2S)-2-[({4-[3-(3-methoxyphenyl)-1H-pyrrolo[3,2-b]pyridin-2-yl]pyridin-3-yl}oxy)methyl]pyrrolidin-1-yl}prop-2-en-1-on
1-{(2S)-2-[({4-[3-(1H-indol-6-yl)-1H-pyrrolo[3,2-b]pyridin-2-yl]pyridin-3-yl}oxy)methyl]pyrrolidin-1-yl}prop-2-en-1-on
1-{(2S)-2-[({4-[3-(2,3-dichlorophenyl)-1H-pyrrolo[3,2-b]pyridin-2-yl]pyridin-3-yl}oxy)methyl]pyrrolidin-1-yl}prop-2-en-1-on
1-{(2S)-2-[({4-[3-(2,3-difluorophenyl)-1H-pyrrolo[3,2-b]pyridin-2-yl]pyridin-3-yl}oxy)methyl]pyrrolidin-1-yl}prop-2-en-1-on
3-[2-(3-{[(2S)-1-(prop-2-enoyl)pyrrolidin-2-yl]methoxy}pyridin-4-yl)-1H-pyrrolo[3,2-b]pyridin-3-yl]Benzonitril
1-{(2S)-2-[({4-[3-(2-chlorophenyl)-1H-pyrrolo[3,2-b]pyridin-2-yl]pyridin-3-yl}oxy)methyl]pyrrolidin-1-yl}prop-2-en-1-on
1-{(2S)-2-[({4-[3-(2-fluorophenyl)-1H-pyrrolo[3,2-b]pyridin-2-yl]pyridin-3-yl}oxy)methyl]pyrrolidin-1-yl}prop-2-en-1-on
4-Fluor-3-[2-(3-{[(2S)-1-(prop-2-enoyl)pyrrolidin-2-yl]methoxy}pyridin-4-yl)-1H-pyrrolo[3,2-b]pyridin-3-yl]Benzonitril
2-Fluor-1-[(2S)-2-({[4-(3-phenyl-1H-pyrrolo[3,2-b]pyridin-2-yl)pyridin-3-yl]oxy}methyl)pyrrolidin-1-yl]prop-2-en-1-on
(2E)-1-[(2S)-2-({[4-(3-phenyl-1H-pyrrolo[3,2-b]pyridin-2-yl)pyridin-3-yl]oxy}methyl)pyrrolidin-1-yl]but-2-en-1-on
1-[(2S)-2-({[4-(3-phenyl-1H-pyrrolo[3,2-b]pyridin-2-yl)pyridin-3-yl]oxy}methyl)pyrrolidin-1-yl]prop-2-yn-1-on
1-[(2S)-2-({[4-(3-phenyl-1H-pyrrolo[3,2-b]pyridin-2-yl)pyridin-3-yl]oxy}methyl)pyrrolidin-1-yl]but-2-yn-1-on
3-(1-Methyl-1H-pyrazol-3-yl)-1-[(2S)-2-({[4-(3-phenyl-1H-pyrrolo[3,2-b]pyridin-2-yl)pyridin-3-yl]oxy}methyl)pyrrolidin-1-yl]prop-2-yn-1-on
(2E)-4-(Dimethylamino)-1-[(2S)-2-{[(4-{3-[3-(Trifluormethyl)phenyl]-1H-pyrrolo[3,2-b]pyridin-2-yl}pyridin-3-yl)oxy]methyl}pyrrolidin-1-yl]but-2-en-1-on
(2E)-1-{(2S)-2-[({4-[3-(5-chlor-2-fluorophenyl)-1H-pyrrolo[3,2-b]pyridin-2-yl]pyridin-3-yl}oxy)methyl]pyrrolidin-1-yl}-4-(Dimethylamino)but-2-en-1-on
(2Z)-4-(Dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-on
(2E)-1-[(2S)-2-({[4-(3-phenyl-1H-pyrrolo[3,2-b]pyridin-2-yl)pyridin-3-yl]oxy}methyl)pyrrolidin-1-yl]-4-(pyrrolidin-1-yl)but-2-en-1-on
4-(Dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-yn-1-on
2-(3-{[(2S)-1-(Ethensulfonyl)pyrrolidin-2-yl]methoxy}pyridin-4-yl)-3-[2-fluor-5-(Trifluormethyl)phenyl]-1H-pyrrolo[3,2-b]pyridin
1-{(2S)-2-[({4-[3-(3-Ethinylphenyl)-1H-pyrrolo[3,2-b]pyridin-2-yl]pyridin-3-yl}oxy)methyl]pyrrolidin-1-yl}prop-2-en-1-on
1-[(2S)-2-{[(4-{3-[3-(Difluormethoxy)-4-fluorophenyl]-1H-pyrrolo[3,2-b]pyridin-2-yl}pyridin-3-yl)oxy]methyl}pyrrolidin-1-yl]prop-2-en-1-on
(2E)-1-[(2S)-2-{[(4-{3-[3-(Difluormethoxy)-4-fluorophenyl]-1H-pyrrolo[3,2-b]pyridin-2-yl}pyridin-3-yl)oxy]methyl}pyrrolidin-1-yl]-4-(Dimethylamino)but-2-en-1-on
3-[3-(Difluormethoxy)-4-fluorophenyl]-2-(3-{[(2S)-1-(Ethensulfonyl)pyrrolidin-2-yl]methoxy}pyridin-4-yl)-1H-pyrrolo[3,2-b]pyridin
1-[(2S)-2-{[(4-{3-[4-Fluor-3-(Trifluormethoxy)phenyl]-1H-pyrrolo[3,2-b]pyridin-2-yl}pyridin-3-yl)oxy]methyl}pyrrolidin-1-yl]prop-2-en-1-on
(2E)-4-(Dimethylamino)-1-[(2S)-2-{[(4-{3-[4-Fluor-3-(Trifluormethoxy)phenyl]-1H-pyrrolo[3,2-b]pyridin-2-yl}pyridin-3-yl)oxy]methyl}pyrrolidin-1-yl]but-2-en-1-on
2-(3-{[(2S)-1-(Ethensulfonyl)pyrrolidin-2-yl]methoxy}pyridin-4-yl)-3-[4-Fluor-3-(Trifluormethoxy)phenyl]-1H-pyrrolo[3,2-b]pyridin
1-[(2S)-2-({[5-Fluor-4-(3-phenyl-1H-pyrrolo[3,2-b]pyridin-2-yl)pyridin-3-yl]oxy}methyl)pyrrolidin-1-yl]prop-2-en-1-on
1-[(2S,4S)-4-Methyl-2-({[4-(3-phenyl-1H-pyrrolo[3,2-b]pyridin-2-yl)pyridin-3-yl]oxy}methyl)pyrrolidin-1-yl]prop-2-en-1-on
2-(3-{[(2S,4S)-1-(Ethensulfonyl)-4-methylpyrrolidin-2-yl]methoxy}pyridin-4-yl)-3-phenyl-1H-pyrrolo[3,2-b]pyridin
1-[(2S)-4,4-Difluor-2-({[4-(3-phenyl-1H-pyrrolo[3,2-b]pyridin-2-yl)pyridin-3-yl]oxy}methyl)pyrrolidin-1-yl]prop-2-en-1-on
1-[(2S)-4-Fluor-2-({[4-(3-phenyl-1H-pyrrolo[3,2-b]pyridin-2-yl)pyridin-3-yl]oxy}methyl)-2,3-dihydro-1H-pyrrol-1-yl]prop-2-en-1-on
1-[(2S,4S)-4-Fluor-2-({[4-(3-phenyl-1H-pyrrolo[3,2-b]pyridin-2-yl)pyridin-3-yl]oxy}methyl)pyrrolidin-1-yl]prop-2-en-1-on
1-[(2S)-2-({[4-(3-phenyl-1H-pyrrolo[3,2-b]pyridin-2-yl)pyridin-3-yl]oxy}methyl)-2,5-dihydro-1H-pyrrol-1-yl]prop-2-en-1-on
1-[(3R)-3-{[4-(3-phenyl-1H-pyrrolo[3,2-b]pyridin-2-yl)pyridin-3-yl]oxy}pyrrolidin-1-yl]prop-2-en-1-on
1-[(3R)-3-{[4-(3-phenyl-1H-pyrrolo[3,2-b]pyridin-2-yl)pyridin-3-yl]oxy}pyrrolidin-1-yl]prop-2-yn-1-on
2-(3-{[(3R)-1-(Ethensulfonyl)pyrrolidin-3-yl]oxy}pyridin-4-yl)-3-phenyl-1H-pyrrolo[3,2-b]pyridin 1-[(3R)-3-({4-[3-(3-chlorophenyl)-1H-pyrrolo[3,2-b]pyridin-2-yl]pyridin-3-yl}oxy)pyrrolidin-1-yl]prop-2-en-1-on
1-[(3R)-3-({4-[3-(3-chlorophenyl)-1H-pyrrolo[3,2-b]pyridin-2-yl]pyridin-3-yl}oxy)pyrrolidin-1-yl]prop-2-yn-1-on
3-(3-chlorophenyl)-2-(3-{[(3R)-1-(Ethensulfonyl)pyrrolidin-3-yl]oxy}pyridin-4-yl)-1H-pyrrolo[3,2-b]pyridin
1-{(3R)-3-[(4-{3-[3-(Trifluormethyl)phenyl]-1H-pyrrolo[3,2-b]pyridin-2-yl}pyridin-3-yl)oxy]pyrrolidin-1-yl}prop-2-en-1-on
1-{(3R)-3-[(4-{3-[3-(Trifluormethyl)phenyl]-1H-pyrrolo[3,2-b]pyridin-2-yl}pyridin-3-yl)oxy]pyrrolidin-1-yl}prop-2-yn-1-on
1-[(3R)-3-({4-[3-(2-fluor-5-methylphenyl)-1H-pyrrolo[3,2-b]pyridin-2-yl]pyridin-3-yl}oxy)pyrrolidin-1-yl]prop-2-en-1-on
1-[(3R)-3-({4-[3-(2-fluor-5-methylphenyl)-1H-pyrrolo[3,2-b]pyridin-2-yl]pyridin-3-yl}oxy)pyrrolidin-1-yl]prop-2-yn-1-on
2-(3-{[(3R)-1-(Ethensulfonyl)pyrrolidin-3-yl]oxy}pyridin-4-yl)-3-(2-fluor-5-methylphenyl)-1H-pyrrolo[3,2-b]pyridin
1-[(3R)-3-({4-[3-(5-chlor-2-fluorophenyl)-1H-pyrrolo[3,2-b]pyridin-2-yl]pyridin-3-yl}oxy)pyrrolidin-1-yl]prop-2-en-1-on
1-[(3R)-3-({4-[3-(5-chlor-2-fluorophenyl)-1H-pyrrolo[3,2-b]pyridin-2-yl]pyridin-3-yl}oxy)pyrrolidin-1-yl]prop-2-yn-1-on
1-{(2S)-2-[({4-[3-(5-chlor-2-fluorophenyl)-1H-pyrrolo[3,2-b]pyridin-2-yl]-5-fluoropyridin-3-yl}oxy)methyl]pyrrolidin-1-yl}prop-2-en-1-on
1-[(2S)-2-({[4-(3-phenyl-1H-pyrrolo[3,2-b]pyridin-2-yl)pyridin-3-yl]oxy}methyl)piperidin-1-yl]prop-2-en-1-on
1-[(2S,4RS)-4-{[2-(Dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-on
1-[(2S,4RS)-4-{[2-(Dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-on
N-methyl-N-[(2S)-2-{[4-(3-phenyl-1H-pyrrolo[3,2-b]pyridin-2-yl)pyridin-3-yl]oxy}propyl]prop-2-enamid
N-methyl-N-[(2S)-2-{[4-(3-phenyl-1H-pyrrolo[3,2-b]pyridin-2-yl)pyridin-3-yl]oxy}propyl]Ethensulfonamid
1-[(2S)-2-({[4-(7-Methyl-3-phenyl-1H-pyrrolo[3,2-b]pyridin-2-yl)pyridin-3-yl]oxy}methyl)pyrrolidin-1-yl]prop-2-en-1-on
2-(3-{[(2S)-1-(Ethensulfonyl)pyrrolidin-2-yl]methoxy}pyridin-4-yl)-7-methyl-3-phenyl-1H-pyrrolo[3,2-b]pyridin
1-{(2S)-2-[({4-[3-(5-chlor-2-fluorophenyl)-7-methyl-1H-pyrrolo[3,2-b]pyridin-2-yl]pyridin-3-yl}oxy)methyl]pyrrolidin-1-yl}prop-2-en-1-on
3-(5-chlor-2-fluorophenyl)-2-(3-{[(2S)-1-(Ethensulfonyl)pyrrolidin-2-yl]methoxy}pyridin-4-yl)-7-methyl-1H-pyrrolo[3,2-b]pyridin
1-[(2R*)-2-({[4-(3-phenyl-1H-pyrrolo[3,2-b]pyridin-2-yl)pyridin-3-yl]oxy}methyl)azetidin-1-yl]prop-2-en-1-on
1-[(2R*)-2-({[4-(3-phenyl-1H-pyrrolo[3,2-b]pyridin-2-yl)pyridin-3-yl]oxy}methyl)azetidin-1-yl]prop-2-en-1-on
2-(3-{[(2R*)-1-(Ethensulfonyl)azetidin-2-yl]methoxy}pyridin-4-yl)-3-phenyl-1H-pyrrolo[3,2-b]pyridin
2-(3-{[(2R*)-1-(Ethensulfonyl)azetidin-2-yl]methoxy}pyridin-4-yl)-3-phenyl-1H-pyrrolo[3,2-b]pyridin
1-[(2R*)-2-{[(4-{3-[3-(Trifluormethyl)phenyl]-1H-pyrrolo[3,2-b]pyridin-2-yl}pyridin-3-yl)oxy]methyl}azetidin-1-yl]prop-2-en-1-on
2-(3-{[(2R*)-1-(Ethensulfonyl)azetidin-2-yl]methoxy}pyridin-4-yl)-3-[3-(Trifluormethyl)phenyl]-1H-pyrrolo[3,2-b]pyridin
1-[(2S)-2-Methyl-2-({[4-(3-phenyl-1H-pyrrolo[3,2-b]pyridin-2-yl)pyridin-3-yl]oxy}methyl)azetidin-1-yl]prop-2-en-1-on
2-(3-{[(2S)-1-(Ethensulfonyl)-2-methylazetidin-2-yl]methoxy}pyridin-4-yl)-3-phenyl-1H-pyrrolo[3,2-b]pyridin
1-[(2S)-2-Methyl-2-{[(4-{3-[3-(Trifluormethyl)phenyl]-1H-pyrrolo[3,2-b]pyridin-2-yl}pyridin-3-yl)oxy]methyl}azetidin-1-yl]prop-2-en-1-on
2-(3-{[(2S)-1-(Ethensulfonyl)-2-methylazetidin-2-yl]methoxy}pyridin-4-yl)-3-[3-(Trifluormethyl)phenyl]-1H-pyrrolo[3,2-b]pyridin
1-[(3S)-3-({[4-(3-phenyl-1H-pyrrolo[3,2-b]pyridin-2-yl)pyridin-3-yl]oxy}methyl)morpholin-4-yl]prop-2-en-1-on
(2E)-4-(Dimethylamino)-1-[(3S)-3-({[4-(3-phenyl-1H-pyrrolo[3,2-b]pyridin-2-yl)pyridin-3-yl]oxy}methyl)morpholin-4-yl]but-2-en-1-on
1-{(3S)-3-[({4-[3-(5-chlor-2-fluorophenyl)-1H-pyrrolo[3,2-b]pyridin-2-yl]pyridin-3-yl}oxy)methyl]morpholin-4-yl}prop-2-en-1-on, und
(2E)-1-{(3S)-3-[({4-[3-(5-chlor-2-fluorophenyl)-1H-pyrrolo[3,2-b]pyridin-2-yl]pyridin-3-yl}oxy)methyl]morpholin-4-yl}-4-(Dimethylamino)but-2-en-1-on,
oder ein N-Oxid, ein Salz, ein Tautomer, ein Rotamer oder ein Stereoisomer dieser Verbindung oder ein Salz dieses N-Oxids, Tautomers, Rotamers oder Stereoisomers.

5. Eine Verbindung der allgemeinen Formel (I) gemäß einem der Ansprüche 1 bis 4 zur Verwendung in der Behandlung oder Prophylaxe von Krankheiten.

6. Die Verbindung zur Verwendung gemäß Anspruch 5, wobei die Krankheiten hyperproliferative Krankheiten und/oder Störungen sind, die auf die Induktion des Zelltods ansprechen.

7. Die Verbindung zur Verwendung gemäß Anspruch 6, wobei die hyperproliferativen Krankheiten und/oder Störungen, die auf die Induktion des Zelltods ansprechen, hämatologische Tumore, solide Tumore und/oder deren Metastasen sind.

8. Die Verbindung zur Verwendung gemäß Anspruch 7, wobei der Tumor ein mutiertes EGFR und/oder dessen Metastasen aufweist.

9. Die Verbindung zur Verwendung gemäß Anspruch 7, wobei der Tumor Lungenkrebs ist, insbesondere Lungenkrebs mit einem mutierten EGFR mit Exon-20-Insertion-Mutation und/oder deren Metastasen.

10. Die Verbindung zur Verwendung gemäß Anspruch 7, wobei der Tumor Lungenkrebs ist, insbesondere Lungenkrebs mit einem mutierten EGFR mit In-Frame-Streichungen in Exon 19 (wie EGFR E746_A750del) oder Punktmutationen in Exon 21 (z. B. L858R) und/oder deren Metastasen.

11. Die Verbindung zur Verwendung gemäß Anspruch 7, wobei der Tumor Lungenkrebs ist, insbesondere Lungenkrebs mit einem mutierten EGFR mit einer Exon-20-Insertion und einer T790M-Mutation, z. B. einer D770_N771insSVD T790M-Mutation, und/oder deren Metastasen.

12. Die Verbindung zur Verwendung gemäß Anspruch 7, wobei der Tumor Lungenkrebs ist, insbesondere Lungenkrebs mit einer In-Frame-Streichung in Exon 19 wie E746_A750del und einer T790M-Mutation, und/oder deren Metastasen.

13. Die Verbindung zur Verwendung gemäß Anspruch 7, wobei der Tumor Lungenkrebs ist, insbesondere Lungenkrebs mit einem mutierten EGFR mit einer Punktmutation in Exon 21 wie L858R und einer T790M-Mutation, und/oder deren Metastasen.

14. Die Verbindung zur Verwendung gemäß Anspruch 7, wobei der Tumor Lungenkrebs ist, insbesondere Lungenkrebs mit einem mutierten ERBB2 mit Exon-20-Insertion-Mutationen (wie ERBB2 A775_G776insYVMA) und/oder deren Metastasen.

15. Eine pharmazeutische Zusammensetzung umfassend mindestens eine Verbindung der allgemeinen Formel (I) gemäß einem der Ansprüche 1 bis 4, zusammen mit mindestens einem pharmazeutisch akzeptablen Hilfsstoff.

16. Eine Zusammensetzung gemäß Anspruch 15 zur Behandlung von hämatologischen Tumoren, soliden Tumoren und/oder deren Metastasen.

17. Eine Kombination umfassend ein oder mehrere erste Wirkstoffe, ausgewählt aus einer Verbindung der allgemeinen Formel (I) gemäß einem der Ansprüche 1 bis 4, und ein oder mehrere zweite Wirkstoffe, ausgewählt aus chemotherapeutischen Anti-Krebs-Mitteln und zielgerichteten Anti-Krebs-Mitteln.

## Revendications

1. Un composé de formule (I) 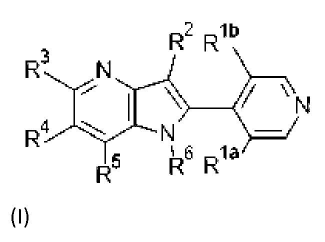 dans lequel :
R^{1a} représente un groupe sélectionné dans le groupe constitué par : -O-(C₂-C₆-alkanediyl)-NR⁷R⁸, -O-CH₂-(C₁-C₅-haloalkanediyl)-NR⁷R⁸, -O-(C₁-C₅-alkanediyl)-R⁹, ou -O-R⁹;
R^{1b} représente un atome d'hydrogène ou fluoro ;
R² représente phényle ou hétéroaryle, lesdits groupes étant substitués, une ou plusieurs fois, indépendamment les uns des autres, par R¹⁰ ;
R³ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R⁴ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R⁵ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R⁶ représente un atome d'hydrogène ou méthyle ;
R⁷ représente -(CO)-R¹¹, -(CO)-C≡C-R¹², -(SO₂)-CH=CH₂, ou oxirane-2-carbonyle ;
R⁸ représente un atome d'hydrogène ou C₁-C₃-alkyle ;
R⁹ représente un groupe sélectionné dans le groupe constitué par : 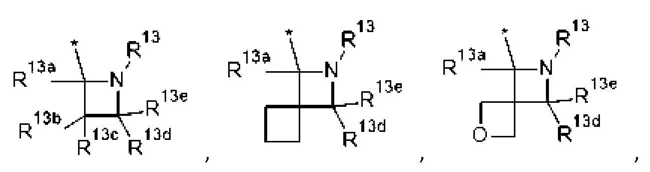 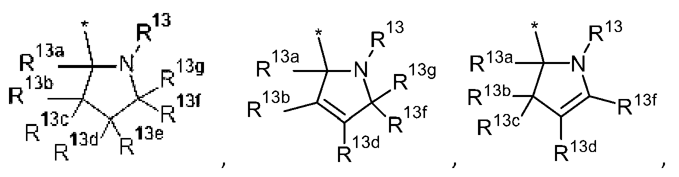 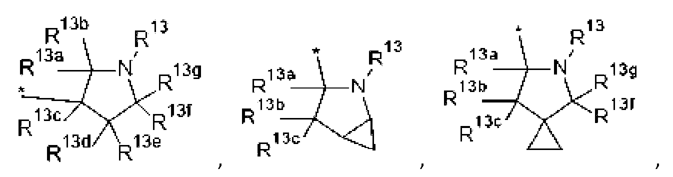 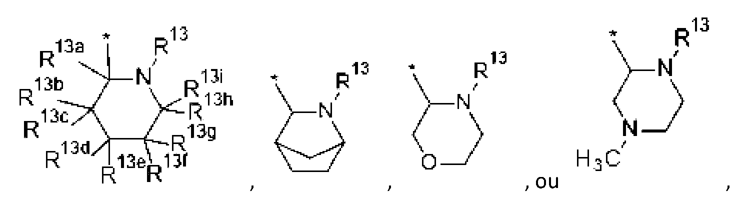 dans lequel * indique le point d'attache dudit groupe avec le reste de la molécule ;
R¹⁰ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R¹¹ représente -CH₂-C≡CH, ou -C(R¹⁴)-R¹⁵ ;
R¹² représente un atome d'hydrogène, méthyle, -CH₂₋N(CH₃)-CHR¹⁶R¹⁷, ou 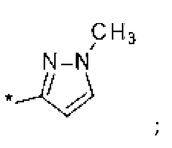
R¹³ représente -(CO)-R¹¹, -(CO)-C≡C-R¹², -(SO₂)-CH=CH₂, ou oxirane-2-carbonyle ;
R^{13a} représente un atome d'hydrogène, méthyle ou fluoro ;
R^{13b} représente un atome d'hydrogène, méthyle ou fluoro ;
R^{13c} représente un atome d'hydrogène, méthyle ou fluoro ;
R^{13d} représente un atome d'hydrogène, méthyle, fluoro, ou -N(CH₃)-CH₂-CH₂-N(CH₃)-CHR¹⁶R¹⁷ ;
R^{13e} représente un atome d'hydrogène, méthyle ou fluoro ;
R^{13f} représente un atome d'hydrogène, méthyle ou fluoro ;
R^{13g} représente un atome d'hydrogène, méthyle ou fluoro ;
R^{13h} représente un atome d'hydrogène, méthyle ou fluoro ;
R¹³ⁱ représente un atome d'hydrogène, méthyle ou fluoro ;
R¹⁴ représente =CH₂, =CH-CH₃, =CH-CH₂-N(CH₃)-CHR¹⁶R¹⁷, ou =CH-CH₂₋R¹⁸ ;
R¹⁵ représente un atome d'hydrogène, C₁-C₃-alkyle ou fluoro ;
R¹⁶ représente un atome d'hydrogène ou méthyle ;
R¹⁷ représente un atome d'hydrogène ou méthyle ;
R¹⁸ représente un groupe sélectionné dans le groupe constitué par : 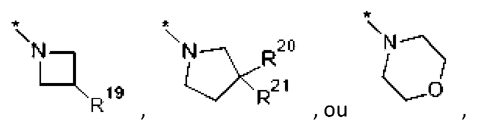 dans lequel * indique le point d'attache dudit groupe avec le reste de la molécule ;
R¹⁹ représente un atome d'hydrogène, méthyle, méthoxy ou fluoro ;
R²⁰ représente un atome d'hydrogène, méthyle, méthoxy ou fluoro ;
R²¹ représente un atome d'hydrogène, méthyle, méthoxy ou fluoro ;
ou un N-oxyde, un sel, un tautomère, un rotamère ou un stéréoisomère dudit composé, ou un sel dudit N-oxyde, tautomère, rotamère ou stéréoisomère.

2. Le composé de formule (I) selon la revendication 1, dans lequel :
R^{1a} représente un groupe sélectionné dans le groupe constitué par : -O-(C₂-C₆-alkanediyl)-NR⁷R⁸, -O-CH₂-(C₁-C₅-haloalkanediyl)-NR⁷R⁸, -O-(C₁-C₅-alkanediyl)-R⁹, ou -O-R⁹;
R^{1b} représente un atome d'hydrogène ou fluoro ;
R² représente un groupe sélectionné dans le groupe constitué par : 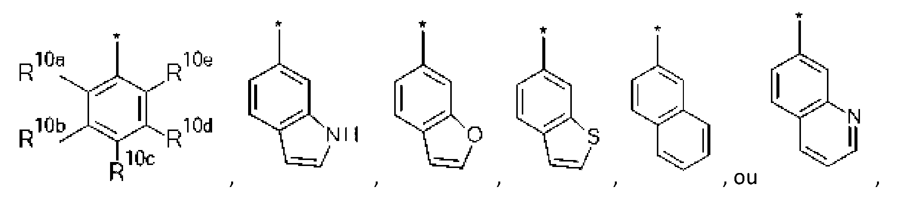
dans lequel * indique le point d'attache dudit groupe avec le reste de la molécule ;
R³ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R⁴ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R⁵ représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R⁶ représente un atome d'hydrogène ou méthyle ;
R⁷ représente -(CO)-R¹¹, -(CO)-C≡C-R¹², -(SO₂)-CH=CH₂, ou oxirane-2-carbonyle ;
R⁸ représente un atome d'hydrogène ou C₁-C₃-alkyle ;
R⁹ représente un groupe sélectionné dans le groupe constitué par : 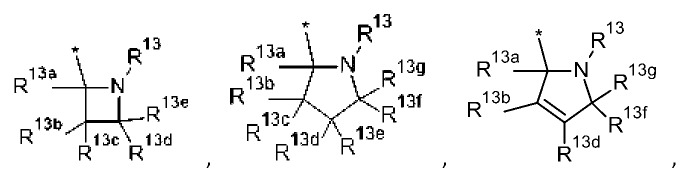 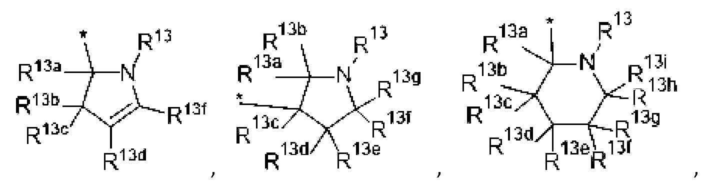 ou 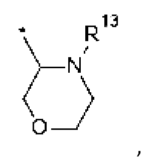
dans lequel * indique le point d'attache dudit groupe avec le reste de la molécule ;
R^{10a} représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R^{10b} représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R^{10c} représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R^{10d} représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R^{10e} représente un atome d'hydrogène, amino, C₁-C₃-alkyle, C₂-C₃-alcényle, C₂-C₃-alcynyle, C₁-C₃-haloalkyle, C₁-C₃-hydroxyalkyle, C₁-C₃-alkoxy, C₁-C₃-haloalkoxy, C₁-C₃-alkoxy-C₁-C₃-alkyle, amino-C₁-C₃-alkyle, C₁-C₃-alkylamino-C₁-C₃-alkyle, (C₁-C₃-alkyl)₂amino-C₁-C₃-alkyle, C₁-C₃-alkoxycarbonyle, aminocarbonyle, C₁-C₃-alkylaminocarbonyle, (C₁-C₃-alkyl)₂₋aminocarbonyle, cyano, fluoro, chloro ou bromo ;
R¹¹ représente -CH₂-C≡CH, ou -C(R¹⁴)-R¹⁵ ;
R¹² représente un atome d'hydrogène, méthyle, -CH₂₋N(CH₃)-CHR¹⁶R¹⁷, ou 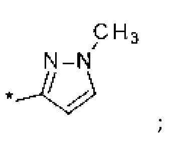
R¹³ représente -(CO)-R¹¹, -(CO)-C≡C-R¹², -(SO₂)-CH=CH₂, ou oxirane-2-carbonyle ;
R^{13a} représente un atome d'hydrogène, méthyle ou fluoro ;
R^{13b} représente un atome d'hydrogène, méthyle ou fluoro ;
R^{13c} représente un atome d'hydrogène, méthyle ou fluoro ;
R^{13d} représente un atome d'hydrogène, méthyle, fluoro, ou -N(CH₃)-CH₂-CH₂-N(CH₃)-CHR¹⁶R¹⁷ ;
R^{13e} représente un atome d'hydrogène, méthyle ou fluoro ;
R^{13f} représente un atome d'hydrogène, méthyle ou fluoro ;
R^{13g} représente un atome d'hydrogène, méthyle ou fluoro ;
R^{13h} représente un atome d'hydrogène, méthyle ou fluoro ;
R¹³ⁱ représente un atome d'hydrogène, méthyle ou fluoro ;
R¹⁴ représente =CH₂, =CH-CH₃, =CH-CH₂₋N(CH₃)-CHR¹⁶R¹⁷, ou =CH-CH₂₋R¹⁸ ;
R¹⁵ représente un atome d'hydrogène, C₁-C₃-alkyle ou fluoro ;
R¹⁶ représente un atome d'hydrogène ou méthyle ;
R¹⁷ représente un atome d'hydrogène ou méthyle ;
R¹⁸ représente un groupe sélectionné dans le groupe constitué par : 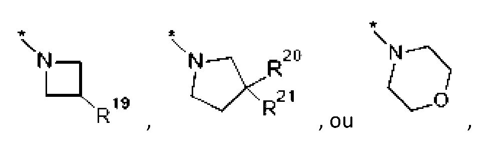
dans lequel * indique le point d'attache dudit groupe avec le reste de la molécule ;
R¹⁹ représente un atome d'hydrogène, méthyle, méthoxy ou fluoro ;
R²⁰ représente un atome d'hydrogène, méthyle, méthoxy ou fluoro ;
R²¹ représente un atome d'hydrogène, méthyle, méthoxy ou fluoro ;
ou un N-oxyde, un sel, un tautomère, un rotamère ou un stéréoisomère dudit composé, ou un sel dudit N-oxyde, tautomère, rotamère ou stéréoisomère.

3. Le composé de formule (I) selon l'une quelconque des revendications 1 ou 2, dans lequel :
R^{1a} représente un groupe sélectionné dans le groupe constitué par : -O-(C₂-C₄-alkanediyl)-NR⁷R⁸, -O-CH₂-R⁹, ou -O-R⁹ ;
R^{1b} représente un atome d'hydrogène ou fluoro ;
R² représente un groupe sélectionné dans le groupe constitué par : 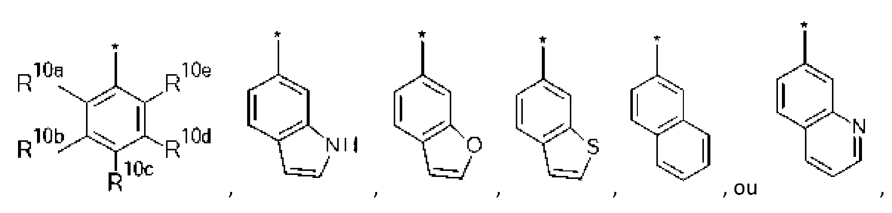
dans lequel * indique le point d'attache dudit groupe avec le reste de la molécule ;
R³ représente un atome d'hydrogène, ou méthoxy ;
R⁴ représente un atome d'hydrogène, ou fluoro ;
R⁵ représente un atome d'hydrogène ou méthyle ;
R⁶ représente un atome d'hydrogène ;
R⁷ représente -(CO)-R¹¹, -(CO)-C≡C-R¹², ou -(SO₂)-CH=CH₂ ;
R⁸ représente un atome d'hydrogène, méthyle, ou éthyle ;
R⁹ représente un groupe sélectionné dans le groupe constitué par : 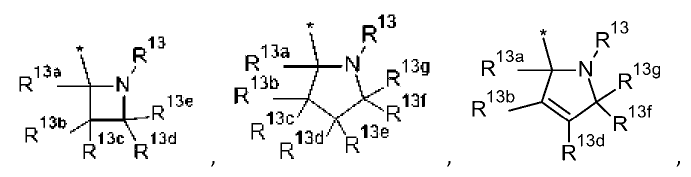 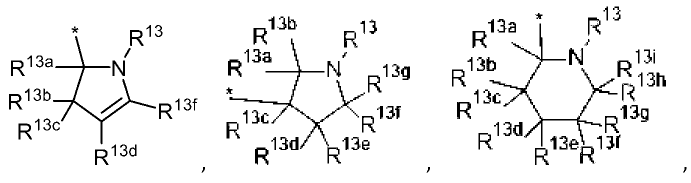 ou 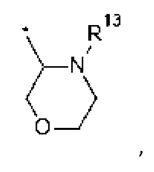
dans lequel * indique le point d'attache dudit groupe avec le reste de la molécule ;
R^{10a} représente un atome d'hydrogène, éthyle, méthoxy, ou fluoro ;
R^{10b} représente un atome d'hydrogène, méthyle, éthyle, iso-propyle, éthinyle, trifluorométhyle, méthoxy, difluorométhoxy, trifluorométhoxy, méthoxycarbonyle, cyano, fluoro, ou chloro ;
R^{10c} représente un atome d'hydrogène, fluoro, ou chloro ;
R^{10d} représente un atome d'hydrogène, méthyle, éthyle, fluoro, ou chloro ;
R^{10e} représente un atome d'hydrogène, fluoro, ou chloro ;
R¹¹ représente -C(R¹⁴)-R¹⁵ ;
R¹² représente un atome d'hydrogène, ou méthyle ;
R¹³ représente -(CO)-R¹¹, -(CO)-C≡C-R¹², ou -(SO₂)-CH=CH₂ ;
R^{13a} représente un atome d'hydrogène, ou méthyle ;
R^{13b} représente un atome d'hydrogène ;
R^{13c} représente un atome d'hydrogène ;
R^{13d} représente un atome d'hydrogène, méthyle, fluoro, ou -N(CH₃)-CH₂-CH₂-N(CH₃)-CHR¹⁶R¹⁷ ;
R^{13e} représente un atome d'hydrogène, ou fluoro ;
R^{13f} représente un atome d'hydrogène ;
R^{13g} représente un atome d'hydrogène ;
R^{13h} représente un atome d'hydrogène ;
R¹³ⁱ représente un atome d'hydrogène ;
R¹⁴ représente =CH₂, ou =CH-CH₂₋N(CH₃)-CHR¹⁶R¹⁷ ;
R¹⁵ représente un atome d'hydrogène ;
R¹⁶ représente un atome d'hydrogène ;
R¹⁷ représente un atome d'hydrogène ;
ou un N-oxyde, un sel, un tautomère, un rotamère, ou un stéréoisomère dudit composé, ou un sel dudit N-oxyde, tautomère, rotamère, ou stéréoisomère.

4. Le composé de formule (I) selon l'une quelconque des revendications 1 à 3, qui est sélectionné dans le groupe constitué de :
N-[2-({4-[3-(4-fluorophenyl)-1H-pyrrolo[3,2-b]pyridin-2-yl]pyridin-3-yl}oxy)ethyl]prop-2-enamide,
N-(2-{[4-(3-phenyl-1H-pyrrolo[3,2-b]pyridin-2-yl)pyridin-3-yl]oxy}ethyl)prop-2-enamide, N-methyl-N-(3-{[4-(3-phenyl-1H-pyrrolo[3,2-b]pyridin-2-yl)pyridin-3-yl]oxy}propyl)prop-2-enamide,
N-methyl-N-(2-{[4-(3-phenyl-1H-pyrrolo[3,2-b]pyridin-2-yl)pyridin-3-yl]oxy}ethyl)prop-2-enamide,
N-(3-{[4-(3-phenyl-1H-pyrrolo[3,2-b]pyridin-2-yl)pyridin-3-yl]oxy}propyl)prop-2-enamide, N-(2-{[4-(6-fluoro-3-phenyl-1H-pyrrolo[3,2-b]pyridin-2-yl)pyridin-3-yl]oxy}ethyl)prop-2-enamide,
N-(2-{[4-(6-fluoro-3-phenyl-1H-pyrrolo[3,2-b]pyridin-2-yl)pyridin-3-yl]oxy}ethyl)-N-methylprop-2-enamide,
N-(2-{[4-(5-methoxy-3-phenyl-1H-pyrrolo[3,2-b]pyridin-2-yl)pyridin-3-yl]oxy}ethyl)-N-methylprop-2-enamide,
N-methyl-N-[2-({4-[3-(3-methylphenyl)-1H-pyrrolo[3,2-b]pyridin-2-yl]pyridin-3-yl}oxy)ethyl]prop-2-enamide,
N-[2-({4-[3-(4-chloro-3-methylphenyl)-1H-pyrrolo[3,2-b]pyridin-2-yl]pyridin-3-yl}oxy)ethyl]-N-methylprop-2-enamide,
N-[2-({4-[3-(4-chlorophenyl)-1H-pyrrolo[3,2-b]pyridin-2-yl]pyridin-3-yl}oxy)ethyl]-N-methylprop-2-enamide,
N-[2-({4-[3-(4-fluoro-3-methylphenyl)-1H-pyrrolo[3,2-b]pyridin-2-yl]pyridin-3-yl}oxy)ethyl]-N-methylprop-2-enamide,
Methyl 2-chloro-5-[2-(3-{2-[methyl(prop-2-enoyl)amino]ethoxy}pyridin-4-yl)-1H-pyrrolo[3,2-b]pyridin-3-yl]benzoate,
N-[2-({4-[3-(3-fluorophenyl)-1H-pyrrolo[3,2-b]pyridin-2-yl]pyridin-3-yl}oxy)ethyl]-N-methylprop-2-enamide,
N-[2-({4-[3-(4-chloro-3-methoxyphenyl)-1H-pyrrolo[3,2-b]pyridin-2-yl]pyridin-3-yl}oxy)ethyl]-N-methylprop-2-enamide,
N-[2-({4-[3-(1H-indol-6-yl)-1H-pyrrolo[3,2-b]pyridin-2-yl]pyridin-3-yl}oxy)ethyl]-N-methylprop-2-enamide,
N-[2-({4-[3-(2-methoxyphenyl)-1H-pyrrolo[3,2-b]pyridin-2-yl]pyridin-3-yl}oxy)ethyl]-N-methylprop-2-enamide,
N-methyl-N-[2-({4-[3-(quinolin-7-yl)-1H-pyrrolo[3,2-b]pyridin-2-yl]pyridin-3-yl}oxy)ethyl]prop-2-enamide,
N-[2-({4-[3-(3-fluoro-2-methoxyphenyl)-1H-pyrrolo[3,2-b]pyridin-2-yl]pyridin-3-yl}oxy)ethyl]-N-methylprop-2-enamide,
N-[2-({4-[3-(4-chloro-3-ethylphenyl)-1H-pyrrolo[3,2-b]pyridin-2-yl]pyridin-3-yl}oxy)ethyl]-N-methylprop-2-enamide,
N-[2-({4-[3-(3-chloro-2-methoxyphenyl)-1H-pyrrolo[3,2-b]pyridin-2-yl]pyridin-3-yl}oxy)ethyl]-N-methylprop-2-enamide,
N-[2-({4-[3-(3-chlorophenyl)-1H-pyrrolo[3,2-b]pyridin-2-yl]pyridin-3-yl}oxy)ethyl]-N-methylprop-2-enamide,
N-[2-({4-[3-(3,4-dichlorophenyl)-1H-pyrrolo[3,2-b]pyridin-2-yl]pyridin-3-yl}oxy)ethyl]-N-methylprop-2-enamide,
N-[2-({4-[3-(3-chloro-4-fluorophenyl)-1H-pyrrolo[3,2-b]pyridin-2-yl]pyridin-3-yl}oxy)ethyl]-N-methylprop-2-enamide,
N-methyl-N-(2-{[4-(3-phenyl-1H-pyrrolo[3,2-b]pyridin-2-yl)pyridin-3-yl]oxy}ethyl)ethenesulfonamide,
(2E)-4-(dimethylamino)-N-methyl-N-(2-{[4-(3-phenyl-1H-pyrrolo[3,2-b]pyridin-2-yl)pyridin-3-yl]oxy}ethyl)but-2-enamide,
N-methyl-N-(2-{[4-(3-phenyl-1H-pyrrolo[3,2-b]pyridin-2-yl)pyridin-3-yl]oxy}ethyl)prop-2-ynamide,
N-methyl-N-(2-{[4-(3-phenyl-1H-pyrrolo[3,2-b]pyridin-2-yl)pyridin-3-yl]oxy}ethyl)but-2-ynamide,
N-[2-({4-[3-(3-chlorophenyl)-1H-pyrrolo[3,2-b]pyridin-2-yl]pyridin-3-yl}oxy)ethyl]-N-methylethenesulfonamide,
N-[2-({4-[3-(1H-indol-6-yl)-1H-pyrrolo[3,2-b]pyridin-2-yl]pyridin-3-yl}oxy)ethyl]-N-methylethenesulfonamide,
N-[2-({4-[3-(3,5-dichlorophenyl)-1H-pyrrolo[3,2-b]pyridin-2-yl]pyridin-3-yl}oxy)ethyl]-N-methylprop-2-enamide,
N-ethyl-N-(2-{[4-(3-phenyl-1H-pyrrolo[3,2-b]pyridin-2-yl)pyridin-3-yl]oxy}ethyl)prop-2-enamide,
N-ethyl-N-(2-{[4-(3-phenyl-1H-pyrrolo[3,2-b]pyridin-2-yl)pyridin-3-yl]oxy}ethyl)ethenesulfonamide,
1-[(2S)-2-({[4-(3-phenyl-1H-pyrrolo[3,2-b]pyridin-2-yl)pyridin-3-yl]oxy}methyl)pyrrolidin-1-yl]prop-2-en-1-one,
1-[(2R)-2-({[4-(3-phenyl-1H-pyrrolo[3,2-b]pyridin-2-yl)pyridin-3-yl]oxy}methyl)pyrrolidin-1-yl]prop-2-en-1-one,
2-(3-{[(2S)-1-(ethenesulfonyl)pyrrolidin-2-yl]methoxy}pyridin-4-yl)-3-phenyl-1H-pyrrolo[3,2-b]pyridine,
(2E)-4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one,
2-(3-{[(2S)-1-(ethenesulfonyl)pyrrolidin-2-yl]methoxy}pyridin-4-yl)-3-[3-(trifluoromethyl)phenyl]-1H-pyrrolo[3,2-b]pyridine,
1-[(2S)-2-{[(4-{3-[3-(trifluoromethyl)phenyl]-1H-pyrrolo[3,2-b]pyridin-2-yl}pyridin-3-yl)oxy]methyl}pyrrolidin-1-yl]prop-2-en-1-one,
3-(3-chlorophenyl)-2-(3-{[(2S)-1-(ethenesulfonyl)pyrrolidin-2-yl]methoxy}pyridin-4-yl)-1H-pyrrolo[3,2-b]pyridine,
1-{(2S)-2-[({4-[3-(3-chlorophenyl)-1H-pyrrolo[3,2-b]pyridin-2-yl]pyridin-3-yl}oxy)methyl]pyrrolidin-1-yl}prop-2-en-1-one,
1-{(2S)-2-[({4-[3-(5-chloro-2-fluorophenyl)-1H-pyrrolo[3,2-b]pyridin-2-yl]pyridin-3-yl}oxy)methyl]pyrrolidin-1-yl}prop-2-en-1-one,
N-[2-({4-[3-(5-chloro-2-fluorophenyl)-1H-pyrrolo[3,2-b]pyridin-2-yl]pyridin-3-yl}oxy)ethyl]-N-methylprop-2-enamide
N-[2-({4-[3-(1-benzothiophen-6-yl)-1H-pyrrolo[3,2-b]pyridin-2-yl]pyridin-3-yl}oxy)ethyl]-N-methylprop-2-enamide
N-[2-({4-[3-(3-ethylphenyl)-1H-pyrrolo[3,2-b]pyridin-2-yl]pyridin-3-yl}oxy)ethyl]-N-methylprop-2-enamide
N-[2-({4-[3-(2,5-difluorophenyl)-1H-pyrrolo[3,2-b]pyridin-2-yl]pyridin-3-yl}oxy)ethyl]-N-methylprop-2-enamide
N-[2-({4-[3-(2-fluoro-5-methylphenyl)-1H-pyrrolo[3,2-b]pyridin-2-yl]pyridin-3-yl}oxy)ethyl]-N-methylprop-2-enamide
N-[2-({4-[3-(5-ethyl-2-fluorophenyl)-1H-pyrrolo[3,2-b]pyridin-2-yl]pyridin-3-yl}oxy)ethyl]-N-methylprop-2-enamide
N-methyl-N-{2-[(4-{3-[3-(trifluoromethyl)phenyl]-1H-pyrrolo[3,2-b]pyridin-2-yl}pyridin-3-yl)oxy]ethyl}prop-2-enamide
N-[2-({4-[3-(3-fluoro-5-methylphenyl)-1H-pyrrolo[3,2-b]pyridin-2-yl]pyridin-3-yl}oxy)ethyl]-N-methylprop-2-enamide
N-{2-[(4-{3-[2-fluoro-5-(trifluoromethyl)phenyl]-1H-pyrrolo[3,2-b]pyridin-2-yl}pyridin-3-yl)oxy]ethyl}-N-methylprop-2-enamide
N-[2-({4-[3-(3,5-difluorophenyl)-1H-pyrrolo[3,2-b]pyridin-2-yl]pyridin-3-yl}oxy)ethyl]-N-methylprop-2-enamide
N-[2-({4-[3-(2-ethylphenyl)-1H-pyrrolo[3,2-b]pyridin-2-yl]pyridin-3-yl}oxy)ethyl]-N-methylprop-2-enamide
N-[2-({4-[3-(3-methoxyphenyl)-1H-pyrrolo[3,2-b]pyridin-2-yl]pyridin-3-yl}oxy)ethyl]-N-methylprop-2-enamide
N-methyl-N-{2-[(4-{3-[3-(propan-2-yl)phenyl]-1H-pyrrolo[3,2-b]pyridin-2-yl}pyridin-3-yl)oxy]ethyl}prop-2-enamide
N-[2-({4-[3-(2-fluoro-5-methoxyphenyl)-1H-pyrrolo[3,2-b]pyridin-2-yl]pyridin-3-yl}oxy)ethyl]-N-methylprop-2-enamide
N-[2-({4-[3-(2,5-dichlorophenyl)-1H-pyrrolo[3,2-b]pyridin-2-yl]pyridin-3-yl}oxy)ethyl]-N-methylprop-2-enamide
N-methyl-N-{2-[(4-{3-[3-(trifluoromethoxy)phenyl]-1H-pyrrolo[3,2-b]pyridin-2-yl}pyridin-3-yl)oxy]ethyl}prop-2-enamide
N-{2-[(4-{3-[2-fluoro-5-(trifluoromethyl)phenyl]-1H-pyrrolo[3,2-b]pyridin-2-yl}pyridin-3-yl)oxy]ethyl}-N-methylethenesulfonamide
N-[2-({4-[3-(5-chloro-2-fluorophenyl)-1H-pyrrolo[3,2-b]pyridin-2-yl]pyridin-3-yl}oxy)ethyl]-N-methylethenesulfonamide
N-[2-({4-[3-(2,5-difluorophenyl)-1H-pyrrolo[3,2-b]pyridin-2-yl]pyridin-3-yl}oxy)ethyl]-N-methylethenesulfonamide
N-[2-({4-[3-(1-benzothiophen-6-yl)-1H-pyrrolo[3,2-b]pyridin-2-yl]pyridin-3-yl}oxy)ethyl]-N-methylethenesulfonamide
N-[2-({4-[3-(3-ethylphenyl)-1H-pyrrolo[3,2-b]pyridin-2-yl]pyridin-3-yl}oxy)ethyl]-N-methylethenesulfonamide
N-[2-({4-[3-(2-fluoro-5-methylphenyl)-1H-pyrrolo[3,2-b]pyridin-2-yl]pyridin-3-yl}oxy)ethyl]-N-methylethenesulfonamide
N-[2-({4-[3-(3-fluoro-5-methylphenyl)-1H-pyrrolo[3,2-b]pyridin-2-yl]pyridin-3-yl}oxy)ethyl]-N-methylethenesulfonamide
N-methyl-N-{2-[(4-{3-[3-(trifluoromethyl)phenyl]-1H-pyrrolo[3,2-b]pyridin-2-yl}pyridin-3-yl)oxy]ethyl}ethenesulfonamide
N-[2-({4-[3-(5-ethyl-2-fluorophenyl)-1H-pyrrolo[3,2-b]pyridin-2-yl]pyridin-3-yl}oxy)ethyl]-N-methylethenesulfonamide
N-[2-({4-[3-(3,5-difluorophenyl)-1H-pyrrolo[3,2-b]pyridin-2-yl]pyridin-3-yl}oxy)ethyl]-N-methylethenesulfonamide
N-[2-({4-[3-(3-methoxyphenyl)-1H-pyrrolo[3,2-b]pyridin-2-yl]pyridin-3-yl}oxy)ethyl]-N-methylethenesulfonamide
N-methyl-N-{2-[(4-{3-[3-(propan-2-yl)phenyl]-1H-pyrrolo[3,2-b]pyridin-2-yl}pyridin-3-yl)oxy]ethyl}ethenesulfonamide
N-[2-({4-[3-(2,5-dichlorophenyl)-1H-pyrrolo[3,2-b]pyridin-2-yl]pyridin-3-yl}oxy)ethyl]-N-methylethenesulfonamide
N-[2-({4-[3-(2-ethylphenyl)-1H-pyrrolo[3,2-b]pyridin-2-yl]pyridin-3-yl}oxy)ethyl]-N-methylethenesulfonamide
N-[2-({4-[3-(2-fluoro-5-methoxyphenyl)-1H-pyrrolo[3,2-b]pyridin-2-yl]pyridin-3-yl}oxy)ethyl]-N-methylethenesulfonamide
N-methyl-N-{2-[(4-{3-[3-(trifluoromethoxy)phenyl]-1H-pyrrolo[3,2-b]pyridin-2-yl}pyridin-3-yl)oxy]ethyl}ethenesulfonamide
N-[2-({4-[3-(3,5-dichlorophenyl)-1H-pyrrolo[3,2-b]pyridin-2-yl]pyridin-3-yl}oxy)ethyl]-N-methylethenesulfonamide
1-[(2S)-2-{[(4-{3-[2-fluoro-5-(trifluoromethyl)phenyl]-1H-pyrrolo[3,2-b]pyridin-2-yl}pyridin-3-yl)oxy]methyl}pyrrolidin-1-yl]prop-2-en-1-one
1-{(2S)-2-[({4-[3-(2-fluoro-5-methylphenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(3-ethylphenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(quinolin-7-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(naphthalen-2-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(1-benzothiophen-6-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(1-benzofuran-6-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(3-methoxyphenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(1H-indol-6-yl)-1H-pyrrolo[3,2-b]pyridin-2-yl]pyridin-3-yl}oxy)methyl]pyrrolidin-1-yl}prop-2-en-1-one
1-{(2S)-2-[({4-[3-(2,3-dichlorophenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(2,3-difluorophenyl)-1H-pyrrolo[3,2-b]pyridin-2-yl]pyridin-3-yl}oxy)methyl]pyrrolidin-1-yl}prop-2-en-1-one
3-[2-(3-{[(2S)-1-(prop-2-enoyl)pyrrolidin-2-yl]methoxy}pyridin-4-yl)-1H-pyrrolo[3,2-b]pyridin-3-yl]benzonitrile
1-{(2S)-2-[({4-[3-(2-chlorophenyl)-1H-pyrrolo[3,2-b]pyridin-2-yl]pyridin-3-yl}oxy)methyl]pyrrolidin-1-yl}prop-2-en-1-one
1-{(2S)-2-[({4-[3-(2-fluorophenyl)-1H-pyrrolo[3,2-b]pyridin-2-yl]pyridin-3-yl}oxy)methyl]pyrrolidin-1-yl}prop-2-en-1-one
4-fluoro-3-[2-(3-{[(2S)-1-(prop-2-enoyl)pyrrolidin-2-yl]methoxy}pyridin-4-yl)-1H-pyrrolo[3,2-b]pyridin-3-yl]benzonitrile
2-fluoro-1-[(2S)-2-({[4-(3-phenyl-1H-pyrrolo[3,2-b]pyridin-2-yl)pyridin-3-yl]oxy}methyl)pyrrolidin-1-yl]prop-2-en-1-one
(2E)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one
1-[(2S)-2-({[4-(3-phenyl-1H-pyrrolo[3,2-b]pyridin-2-yl)pyridin-3-yl]oxy}methyl)pyrrolidin-1-yl]prop-2-yn-1-one
1-[(2S)-2-({[4-(3-phenyl-1H-pyrrolo[3,2-b]pyridin-2-yl)pyridin-3-yl]oxy}methyl)pyrrolidin-1-yl]but-2-yn-1-one
3-(1-methyl-1H-pyrazol-3-yl)-1-[(2S)-2-({[4-(3-phenyl-1H-pyrrolo[3,2-b]pyridin-2-yl)pyridin-3-yl]oxy}methyl)pyrrolidin-1-yl]prop-2-yn-1-one
(2E)-4-(dimethylamino)-1-[(2S)-2-{[(4-{3-[3-(trifluoromethyl)phenyl]-1H-pyrrolo[3,2-b]pyridin-2-yl}pyridin-3-yl)oxy]methyl}pyrrolidin-1-yl]but-2-en-1-one
(2E)-1-{(2S)-2-[({4-[3-(5-chloro-2-fluorophenyl)-1H-pyrrolo[3,2-b]pyridin-2-yl]pyridin-3-yl}oxy)methyl]pyrrolidin-1-yl}-4-(dimethylamino)but-2-en-1-one
(2Z)-4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-en-1-one
(2E)-1-[(2S)-2-({[4-(3-phenyl-1H-pyrrolo[3,2-b]pyridin-2-yl)pyridin-3-yl]oxy}methyl)pyrrolidin-1-yl]-4-(pyrrolidin-1-yl)but-2-en-1-one
4-(dimethylamino)-1-[(2S)-2-({[4-(3-phenyl-1H-pyrrolo[3,2-b]pyridin-2-yl)pyridin-3-yl]oxy}methyl)pyrrolidin-1-yl]but-2-yn-1-one
2-(3-{[(2S)-1-(ethenesulfonyl)pyrrolidin-2-yl]methoxy}pyridin-4-yl)-3-[2-fluoro-5-(trifluoromethyl)phenyl]-1H-pyrrolo[3,2-b]pyridine
1-{(2S)-2-[({4-[3-(3-ethynylphenyl)-1H-pyrrolo[3,2-b]pyridin-2-yl]pyridin-3-yl}oxy)methyl]pyrrolidin-1-yl}prop-2-en-1-one
1-[(2S)-2-{[(4-{3-[3-(difluoromethoxy)-4-fluorophenyl]-1H-pyrrolo[3,2-b]pyridin-2-yl}pyridin-3-yl)oxy]methyl}pyrrolidin-1-yl]prop-2-en-1-one
(2E)-1-[(2S)-2-{[(4-{3-[3-(difluoromethoxy)-4-fluorophenyl]-1H-pyrrolo[3,2-b]pyridin-2-yl}pyridin-3-yl)oxy]methyl}pyrrolidin-1-yl]-4-(dimethylamino)but-2-en-1-one
3-[3-(difluoromethoxy)-4-fluorophenyl]-2-(3-{[(2S)-1-(ethenesulfonyl)pyrrolidin-2-yl]methoxy}pyridin-4-yl)-1H-pyrrolo[3,2-b]pyridine
1-[(2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidin-1-yl]prop-2-en-1-one
(2E)-4-(dimethylamino)-1-[(2S)-2-{[(4-{3-[4-fluoro-3-(trifluoromethoxy)phenyl]-1H-pyrrolo[3,2-b]pyridin-2-yl}pyridin-3-yl)oxy]methyl}pyrrolidin-1-yl]but-2-en-1-one
2-(3-{[(2S)-1-(ethenesulfonyl)pyrrolidin-2-yl]methoxy}pyridin-4-yl)-3-[4-fluoro-3-(trifluoromethoxy)phenyl]-1H-pyrrolo[3,2-b]pyridine
1-[(2S)-2-({[5-fluoro-4-(3-phenyl-1H-pyrrolo[3,2-b]pyridin-2-yl)pyridin-3-yl]oxy}methyl)pyrrolidin-1-yl]prop-2-en-1-one
1-[(2S,4S)-4-methyl-2-({[4-(3-phenyl-1H-pyrrolo[3,2-b]pyridin-2-yl)pyridin-3-yl]oxy}methyl)pyrrolidin-1-yl]prop-2-en-1-one
2-(3-{[(2S,4S)-1-(ethenesulfonyl)-4-methylpyrrolidin-2-yl]methoxy}pyridin-4-yl)-3-phenyl-1H-pyrrolo[3,2-b]pyridine
1-[(2S)-4,4-difluoro-2-({[4-(3-phenyl-1H-pyrrolo[3,2-b]pyridin-2-yl)pyridin-3-yl]oxy}methyl)pyrrolidin-1-yl]prop-2-en-1-one
1-[(2S)-4-fluoro-2-({[4-(3-phenyl-1H-pyrrolo[3,2-b]pyridin-2-yl)pyridin-3-yl]oxy}methyl)-2,3-dihydro-1H-pyrrol-1-yl]prop-2-en-1-one
1-[(2S,4S)-4-fluoro-2-({[4-(3-phenyl-1H-pyrrolo[3,2-b]pyridin-2-yl)pyridin-3-yl]oxy}methyl)pyrrolidin-1-yl]prop-2-en-1-one
1-[(2S)-2-({[4-(3-phenyl-1H-pyrrolo[3,2-b]pyridin-2-yl)pyridin-3-yl]oxy}methyl)-2,5-dihydro-1H-pyrrol-1-yl]prop-2-en-1-one
1-[(3R)-3-{[4-(3-phenyl-1H-pyrrolo[3,2-b]pyridin-2-yl)pyridin-3-yl]oxy}pyrrolidin-1-yl]prop-2-en-1-one
1-[(3R)-3-{[4-(3-phenyl-1H-pyrrolo[3,2-b]pyridin-2-yl)pyridin-3-yl]oxy}pyrrolidin-1-yl]prop-2-yn-1-one
2-(3-{[(3R)-1-(ethenesulfonyl)pyrrolidin-3-yl]oxy}pyridin-4-yl)-3-phenyl-1H-pyrrolo[3,2-b]pyridine
1-[(3R)-3-({4-[3-(3-chlorophenyl)-1H-pyrrolo[3,2-b]pyridin-2-yl]pyridin-3-yl}oxy)pyrrolidin-1-yl]prop-2-en-1-one
1-[(3R)-3-({4-[3-(3-chlorophenyl)-1H-pyrrolo[3,2-b]pyridin-2-yl]pyridin-3-yl}oxy)pyrrolidin-1-yl]prop-2-yn-1-one
3-(3-chlorophenyl)-2-(3-{[(3R)-1-(ethenesulfonyl)pyrrolidin-3-yl]oxy}pyridin-4-yl)-1H-pyrrolo[3,2-b]pyridine
1-{(3R)-3-[(4-{3-[3-(trifluoromethyl)phenyl]-1H-pyrrolo[3,2-b]pyridin-2-yl}pyridin-3-yl)oxy]pyrrolidin-1-yl}prop-2-en-1-one
1-{(3R)-3-[(4-{3-[3-(trifluoromethyl)phenyl]-1H-pyrrolo[3,2-b]pyridin-2-yl}pyridin-3-yl)oxy]pyrrolidin-1-yl}prop-2-yn-1-one
1-[(3R)-3-({4-[3-(2-fluoro-5-methylphenyl)-1H-pyrrolo[3,2-b]pyridin-2-yl]pyridin-3-yl}oxy)pyrrolidin-1-yl]prop-2-en-1-one
1-[(3R)-3-({4-[3-(2-fluoro-5-methylphenyl)-1H-pyrrolo[3,2-b]pyridin-2-yl]pyridin-3-yl}oxy)pyrrolidin-1-yl]prop-2-yn-1-one
2-(3-{[(3R)-1-(ethenesulfonyl)pyrrolidin-3-yl]oxy}pyridin-4-yl)-3-(2-fluoro-5-methylphenyl)-1H-pyrrolo[3,2-b]pyridine
1-[(3R)-3-({4-[3-(5-chloro-2-fluorophenyl)-1H-pyrrolo[3,2-b]pyridin-2-yl]pyridin-3-yl}oxy)pyrrolidin-1-yl]prop-2-en-1-one
1-[(3R)-3-({4-[3-(5-chloro-2-fluorophenyl)-1H-pyrrolo[3,2-b]pyridin-2-yl]pyridin-3-yl}oxy)pyrrolidin-1-yl]prop-2-yn-1-one
1-{(2S)-2-[({4-[3-(5-chloro-2-fluorophenyl)-1H-pyrrolo[3,2-b]pyridin-2-yl]-5-fluoropyridin-3-yl}oxy)methyl]pyrrolidin-1-yl}prop-2-en-1-one
1-[(2S)-2-({[4-(3-phenyl-1H-pyrrolo[3,2-b]pyridin-2-yl)pyridin-3-yl]oxy}methyl)piperidin-1-yl]prop-2-en-1-one
1-[(2S,4RS)-4-{[2-(dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-one
1-[(2S,4RS)-4-{[2-(dimethylamino)ethyl](methyl)amino}-2-({[4-(3-phenyl-1H-pyrrolo[3,2-b]pyridin-2-yl)pyridin-3-yl]oxy}methyl)pyrrolidin-1-yl]prop-2-en-1-one
N-methyl-N-[(2S)-2-{[4-(3-phenyl-1H-pyrrolo[3,2-b]pyridin-2-yl)pyridin-3-yl]oxy}propyl]prop-2-enamide
N-methyl-N-[(2S)-2-{[4-(3-phenyl-1H-pyrrolo[3,2-b]pyridin-2-yl)pyridin-3-yl]oxy}propyl]ethenesulfonamide
1-[(2S)-2-({[4-(7-methyl-3-phenyl-1H-pyrrolo[3,2-b]pyridin-2-yl)pyridin-3-yl]oxy}methyl)pyrrolidin-1-yl]prop-2-en-1-one
2-(3-{[(2S)-1-(ethenesulfonyl)pyrrolidin-2-yl]methoxy}pyridin-4-yl)-7-methyl-3-phenyl-1H-pyrrolo[3,2-b]pyridine
1-{(2S)-2-[({4-[3-(5-chloro-2-fluorophenyl)-7-methyl-1H-pyrrolo[3,2-b]pyridin-2-yl]pyridin-3-yl}oxy)methyl]pyrrolidin-1-yl}prop-2-en-1-one
3-(5-chloro-2-fluorophenyl)-2-(3-{[(2S)-1-(ethenesulfonyl)pyrrolidin-2-yl]methoxy}pyridin-4-yl)-7-methyl-1H-pyrrolo[3,2-b]pyridine
1-[(2R*)-2-({[4-(3-phenyl-1H-pyrrolo[3,2-b]pyridin-2-yl)pyridin-3-yl]oxy}methyl)azetidin-1-yl]prop-2-en-1-one
1-[(2R*)-2-({[4-(3-phenyl-1H-pyrrolo[3,2-b]pyridin-2-yl)pyridin-3-yl]oxy}methyl)azetidin-1-yl]prop-2-en-1-one
2-(3-{[(2R*)-1-(ethenesulfonyl)azetidin-2-yl]methoxy}pyridin-4-yl)-3-phenyl-1H-pyrrolo[3,2-b]pyridine
2-(3-{[(2R*)-1-(ethenesulfonyl)azetidin-2-yl]methoxy}pyridin-4-yl)-3-phenyl-1H-pyrrolo[3,2-b]pyridine
1-[(2R*)-2-{[(4-{3-[3-(trifluoromethyl)phenyl]-1H-pyrrolo[3,2-b]pyridin-2-yl}pyridin-3-yl)oxy]methyl}azetidin-1-yl]prop-2-en-1-one
2-(3-{[(2R*)-1-(ethenesulfonyl)azetidin-2-yl]methoxy}pyridin-4-yl)-3-[3-(trifluoromethyl)phenyl]-1H-pyrrolo[3,2-b]pyridine
1-[(2S)-2-methyl-2-({[4-(3-phenyl-1H-pyrrolo[3,2-b]pyridin-2-yl)pyridin-3-yl]oxy}methyl)azetidin-1-yl]prop-2-en-1-one
2-(3-{[(2S)-1-(ethenesulfonyl)-2-methylazetidin-2-yl]methoxy}pyridin-4-yl)-3-phenyl-1H-pyrrolo[3,2-b]pyridine
1-[(2S)-2-methyl-2-{[(4-{3-[3-(trifluoromethyl)phenyl]-1H-pyrrolo[3,2-b]pyridin-2-yl}pyridin-3-yl)oxy]methyl}azetidin-1-yl]prop-2-en-1-one
2-(3-{[(2S)-1-(ethenesulfonyl)-2-methylazetidin-2-yl]methoxy}pyridin-4-yl)-3-[3-(trifluoromethyl)phenyl]-1H-pyrrolo[3,2-b]pyridine
1-[(3S)-3-({[4-(3-phenyl-1H-pyrrolo[3,2-b]pyridin-2-yl)pyridin-3-yl]oxy}methyl)morpholin-4-yl]prop-2-en-1-one
(2E)-4-(dimethylamino)-1-[(3S)-3-({[4-(3-phenyl-1H-pyrrolo[3,2-b]pyridin-2-yl)pyridin-3-yl]oxy}methyl)morpholin-4-yl]but-2-en-1-one
1-{(3S)-3-[({4-[3-(5-chloro-2-fluorophenyl)-1H-pyrrolo[3,2-b]pyridin-2-yl]pyridin-3-yl}oxy)methyl]morpholin-4-yl}prop-2-en-1-one, et
(2E)-1-{(3S)-3-[({4-[3-(5-chloro-2-fluorophenyl)-1H-pyrrolo[3,2-b]pyridin-2-yl]pyridin-3-yl}oxy)methyl]morpholin-4-yl}-4-(dimethylamino)but-2-en-1-one,
ou un N-oxyde, un sel, un tautomère, un rotamère, ou un stéréoisomère dudit composé, ou un sel dudit N-oxyde, tautomère, rotamère, ou stéréoisomère.

5. Un composé de formule (I) selon l'une quelconque des revendications 1 à 4 pour une utilisation dans le traitement ou la prophylaxie de maladies.

6. Le composé pour une utilisation selon la revendication 5, dans laquelle les maladies sont des maladies hyperprolifératives et/ou des troubles répondant à l'induction de la mort cellulaire.

7. Le composé pour une utilisation selon la revendication 6, dans laquelle les maladies hyperprolifératives et/ou les troubles répondant à l'induction de la mort cellulaire sont des tumeurs hématologiques, des tumeurs solides et/ou leurs métastases.

8. Le composé pour une utilisation selon la revendication 7, dans laquelle la tumeur présente un EGFR mutant et/ou des métastases de celle-ci.

9. Le composé pour une utilisation selon la revendication 7, dans laquelle la tumeur est un cancer du poumon, en particulier un cancer du poumon présentant un EGFR mutant avec une mutation d'insertion dans l'exon 20, et/ou des métastases de celui-ci.

10. Le composé pour une utilisation selon la revendication 7, dans laquelle la tumeur est un cancer du poumon, en particulier un cancer du poumon présentant un EGFR mutant avec des délétions en phase dans l'exon 19 (telles que EGFR E746_A750del) ou des mutations ponctuelles dans l'exon 21 (par exemple L858R), et/ou des métastases de celui-ci.

11. Le composé pour une utilisation selon la revendication 7, dans laquelle la tumeur est un cancer du poumon, en particulier un cancer du poumon présentant un EGFR mutant avec une insertion dans l'exon 20 et une mutation T790M, par exemple une mutation D770_N771insSVD T790M, et/ou des métastases de celui-ci.

12. Le composé pour une utilisation selon la revendication 7, dans laquelle la tumeur est un cancer du poumon, en particulier un cancer du poumon présentant un EGFR mutant avec une délétion en phase dans l'exon 19 telle que E746_A750del et une mutation T790M, et/ou des métastases de celui-ci.

13. Le composé pour une utilisation selon la revendication 7, dans laquelle la tumeur est un cancer du poumon, en particulier un cancer du poumon présentant un EGFR mutant avec une mutation ponctuelle dans l'exon 21 telle que L858R et une mutation T790M, et/ou des métastases de celui-ci.

14. Le composé pour une utilisation selon la revendication 7, dans laquelle la tumeur est un cancer du poumon, en particulier un cancer du poumon présentant un ERBB2 mutant avec des mutations d'insertion dans l'exon 20 (telles que ERBB2 A775_G776insYVMA), et/ou des métastases de celui-ci.

15. Une composition pharmaceutique comprenant au moins un composé de formule générale (I) selon l'une quelconque des revendications 1 à 4, avec au moins un auxiliaire pharmaceutiquement acceptable.

16. Une composition selon la revendication 15 pour le traitement de tumeurs hématologiques, de tumeurs solides et/ou de leurs métastases.

17. Une combinaison comprenant un ou plusieurs premiers principes actifs choisis dans un composé de formule générale (I) selon l'une quelconque des revendications 1 à 4, et un ou plusieurs seconds principes actifs choisis dans des agents anticancéreux chimiothérapeutiques et des agents anticancéreux spécifiques de cible.
